# EUROPEAN PATENT APPLICATION

(11) **EP 3 042 655 A1**
(43) Date of publication of application: **13.07.2016**
(21) Application number: 15195683.6
(22) Date of filing: 01.10.2009
(51) Int. Cl.: A61K 31/437, A61K 31/519, A61P 27/00

(54) **JANUS KINASE INHIBITORS FOR TREATMENT OF DRY EYE AND OTHER EYE RELATED DISEASES**

(30) Priority: 02.10.2008 US 102242 P
(62) Divisional of application: 09737257.7
(71) Applicant: Incyte Holdings Corporation, Wilmington, DE 19803 (US)
(72) Inventor: FRIEDMAN, Paul A., Villanova, PA Pennsylvania 19085 (US); FRIDMAN, Jordan S., Newark, DE Delaware 19711 (US); LUCHI, Monica E., Mount Laurel, NJ New Jersey 08054 (US); WILLIAMS, William V., Havertown, PA Pennsylvania 19104 (US)
(74) Representative: Carpmaels & Ransford LLP

(57) **Abstract**

Methods, kits, and compositions for treating dry eye disorders and other related eye diseases are provided, wherein the methods, kits, and compositions utilize a JAK inhibitor.

## Description

### FIELD OF THE INVENTION

The present invention provides methods, kits, and compositions for the treatment of dry eye and other eye related diseases using compounds which inhibit one or more of the Janus kinases (JAKs).

### BACKGROUND OF THE INVENTION

Dry eye syndrome (DES, also known as keratoconjunctivitis sicca) is one of the most common problems treated by eye physicians. A recent official report of the Dry Eye Workshop (DEWS) defined dry eye as "a multifactorial disease of the tears and ocular surface that results in symptoms of discomfort, visual disturbance, and tear film instability with potential damage to the ocular surface. It is accompanied by increased osmolarity of the tear film and inflammation of the ocular surface." DES affects up to 10% of the population between the ages of 20 to 45 years, with this percentage increasing with age. Although a wide variety of artificial tear products are available, these products provide only transitory relief of symptoms. As such, there is a need for agents, compositions and therapeutic methods to treat dry eye. This invention addresses this need and others.

### SUMMARY OF THE INVENTION

The present invention provides, *inter alia*, a method of treating a dry eye disorder in a patient in need thereof, comprising administering to the patient a therapeutically effective amount of an agent. In some embodiments, the agent used in the methods of the present invention is a compound which can inhibit the activity of one or more Janus kinases (JAKs). The Janus kinase family of protein tyrosine kinases, as well as the Signal Transducers and Activators of Transcription (STATs), are engaged in the signaling of a wide range of cytokines. Generally, cytokine receptors do not have intrinsic tyrosine kinase activity, and thus require receptor-associated kinases to propagate a phosphorylation cascade. JAKs fulfill this function. Cytokines bind to their receptors, causing receptor dimerization, and this enables JAKs to phosphorylate each other as well as specific tyrosine motifs within the cytokine receptors. STATs that recognize these phosphotyrosine motifs are recruited to the receptor, and are then themselves activated by a JAK-dependent tyrosine phosphorylation event. Upon activation, STATs dissociate from the receptors, dimerize, and translocate to the nucleus to bind to specific DNA sites and alter transcription (Scott, M. J., C. J. Godshall, et al. (2002). "Jaks, STATs, Cytokines, and Sepsis." Clin Diagn Lab Immunol 9(6): 1153-9).

The JAK family plays a role in the cytokine-dependent regulation of proliferation and function of cells involved in immune response. Currently, there are four known mammalian JAK family members: JAK1 (also known as Janus kinase-1), JAK2 (also known as Janus kinase-2), JAK3 (also known as Janus kinase, leukocyte; JAKL; L-JAK and Janus kinase-3) and TYK2 (also known as protein-tyrosine kinase 2). The JAK proteins range in size from 120 to 140 kDa and comprise seven conserved JAK homology (JH) domains; one of these is a functional catalytic kinase domain, and another is a pseudokinase domain potentially serving a regulatory function and/or serving as a docking site for STATs (Scott, Godshall *et al.* 2002, *supra*).

While JAK1, JAK2 and TYK2 are ubiquitously expressed, JAK3 is reported to be preferentially expressed in natural killer (NK) cells and not resting T cells, suggesting a role in lymphoid activation (Kawamura, M., D. W. McVicar, et al. (1994). "Molecular cloning of L-JAK, a Janus family protein-tyrosine kinase expressed in natural killer cells and activated leukocytes." Proc Natl Acad Sci U S A 91(14): 6374-8).

Not only do the cytokine-stimulated immune and inflammatory responses contribute to normal host defense, they also play roles in the pathogenesis of diseases: pathologies such as severe combined immunodeficiency (SCID) arise from hypoactivity and suppression of the immune system, and a hyperactive or inappropriate immune / inflammatory response contributes to the pathology of autoimmune diseases such as rheumatoid and psoriatic arthritis, asthma and systemic lupus erythematosus, inflammatory bowel disease, multiple sclerosis, type I diabetes mellitus, myasthenia gravis, thyroiditis, immunoglobulin nephropathies, myocarditis as well as illnesses such as scleroderma and osteoarthritis (Ortmann, R. A., T. Cheng, et al. (2000). "Janus kinases and signal transducers and activators of transcription: their roles in cytokine signaling, development and immunoregulation." Arthritis Res 2(1): 16-32).

Pharmacological targeting of Janus kinase 3 (JAK3) has been employed successfully to control allograft rejection and graft versus host disease (GVHD). In addition to its involvement in signaling of cytokine receptors, JAK3 is also engaged in the CD40 signaling pathway of peripheral blood monocytes. During CD40-induced maturation of myeloid dendritic cells (DCs), JAK3 activity is induced, and increases in costimulatory molecule expression, IL-12 production, and potent allogeneic stimulatory capacity are observed. A rationally designed JAK3 inhibitor WHI-P-154 prevented these effects arresting the DCs at an immature level, suggesting that immunosuppressive therapies targeting the tyrosine kinase JAK3 may also affect the function of myeloid cells (Saemann, M. D., C. Diakos, et al. (2003). "Prevention of CD40-triggered dendritic cell maturation and induction of T-cell hyporeactivity by targeting of Janus kinase 3." Am J transplant 3(11): 1341-9). In the mouse model system, JAK3 was also shown to be an important molecular target for treatment of autoimmune insulin-dependent (type 1) diabetes mellitus. The rationally designed JAK3 inhibitor JANEX-1 exhibited potent immunomodulatory activity and delayed the onset of diabetes in the NOD mouse model of autoimmune type 1 diabetes (Cetkovic-Cvrlje, M., A. L. Dragt, et al. (2003). "Targeting JAK3 with JANEX-1 for prevention of autoimmune type 1 diabetes in NOD mice." Clin Immunol 106(3): 213-25).

Deficiencies in expression of JAK family members are associated with disease states. Jak1-/- mice are runted at birth, fail to nurse, and die perinatally (Rodig, S. J., M. A. Meraz, et al. (1998). "Disruption of the Jak1 gene demonstrates obligatory and nonredundant roles of the Jaks in cytokine-induced biologic responses." Cell 93(3): 373-83). Jak2-/- mouse embryos are anemic and die around day 12.5 postcoitum due to the absence of definitive erythropoiesis. JAK2-deficient fibroblasts do not respond to IFN gamma, although responses to IFNalpha/beta and IL-6 are unaffected. JAK2 functions in signal transduction of a specific group of cytokine receptors required in definitive erythropoiesis (Neubauer, H., A. Cumano, et al. (1998). Cell 93(3): 397-409; Parganas, E., D. Wang, et al. (1998). Cell 93(3): 385-95.). JAK3 appears to play a role in normal development and function of B and T lymphocytes. Mutations of JAK3 are reported to be responsible for autosomal recessive severe combined immunodeficiency (SCID) in humans (Candotti, F., S. A. Oakes, et al. (1997). "Structural and functional basis for JAK3-deficient severe combined immunodeficiency." Blood 90(10): 3996-4003).

Consistent with the role of Janus kinases in inflammation and autoimmune disorders, the present invention provides, *inter alia,* a method of treating dry eye disorders comprising administering to a patient a JAK inhibitor. In a further aspect, the present invention provides a method of treating conjunctivitis, uveitis, chorioditis, retinitis, cyclitis, sclieritis, episcleritis, or iritis; treating inflammation or pain related to corneal transplant, LASIK (laser assisted in situ keratomileusis), photorefractive keratectomy, or LASEK (laser assisted sub-epithelial keratomileusis); inhibiting loss of visual acuity related to corneal transplant, LASIK, photorefractive keratectomy, or LASEK; or inhibiting transplant rejection in a patient in need thereof, comprising administering to the patient a JAK inhibitor. In some embodiments, the agent is administered postoperatively to the patient.
In a further aspect, the present invention provides an ophthalmic insert comprising a JAK inhibitor. In a still further aspect, the present invention provides a kit for treating a dry eye disorder comprising a pharmaceutical composition or ophthalmic composition comprising a JAK inhibitor and instructions comprising a direction to administer the JAK inhibitor to a patient in need of treatment of a dry eye disorder.

### DETAILED DESCRIPTION

The present invention provides, *inter alia,* a method of treating a dry eye disorder in a patient in need thereof, comprising administering to the patient a therapeutically effective amount of an agent. JAKs to which the agent can bind and inhibit includes any member of the JAK family. In some embodiments, the JAK is JAK1, JAK2, JAK3 or TYK2. In some embodiments, the JAK is JAK1. In some embodiments, the agent is selective for JAK1. In some embodiments, the JAK is JAK1 or JAK2. In some embodiments, the JAK is JAK2. In some embodiments, the JAK is JAK3. In some embodiments, the agent is selective. By "selective" is meant that the compound inhibits a JAK with greater affinity or potency, respectively, compared to at least one other JAK. In some embodiments, the agent is a selective inhibitor of JAK1 or JAK2 over JAK3 and/or TYK2. In some embodiments, the agent is a selective inhibitor of JAK2 (e.g., over JAK1, JAK3 and TYK2). Selectivity can be at least about 5-fold, 10-fold, at least about 20-fold, at least about 50-fold, at least about 100-fold, at least about 200-fold, at least about 500-fold or at least about 1000-fold. Selectivity can be measured by methods routine in the art. In some embodiments, selectivity can be tested at the Km of each enzyme. In some embodiments, selectivity of the agent for JAK2 over JAK3 can be determined by the cellular ATP concentration.

In some embodiments, the agents for use in the method of the invention include the JAK inhibitors in U.S. Patent Publ. No. US 20070135461, published June 14, 2007 (Appl. Ser. No. 11/637,545, filed December 12, 2006); U.S. Patent Publ. No. US 20060106020, published May 18, 2006 (Appl. Ser. No. 11/115,702 filed April 27, 2005); U.S. Patent Publ. No. US 20060183906, published August 17, 2006 (Appl. Ser. No. 11/313,394, filed December 21, 2005); U.S. Patent Publ. No. US 20070149506, published June 28, 2007 (Appl. Ser. No. 11/524,641, filed September 21, 2006); U.S. Patent Publ. No. US 20080188500, published August 7, 2008 (Appl. Ser. No. 11/961,424, filed December 20, 2007); U.S. Prov. Appl. No. 61/035,662, filed March 11, 2008; U.S. Prov. Appl. No. 60/988,606, filed November 16, 2007; U.S. Patent Appl. Scr. No. 12/137,883, filed June 12, 2008; and U.S. Patent Appl. Ser. No. 12/137,892, filed June 12,2008; U.S. Patent Appl. No. 12/138,082, filed June 12, 2008, each of which is incorporated herein by reference in its entirety.

As used herein, "dry eye disorder" is intended to encompass the disease states summarized in a recent official report of the Dry Eye Workshop (DEWS), which defined dry eye as "a multifactorial disease of the tears and ocular surface that results in symptoms of discomfort, visual disturbance, and tear film instability with potential damage to the ocular surface. It is accompanied by increased osmolarity of the tear film and inflammation of the ocular surface." Lemp, "The Definition and Classification of Dry Eye Disease: Report of the Definition and Classification Subcommittee of the International Dry Eye WorkShop", The Ocular Surface, 5(2), 75-92 April 2007, which is incorporated herein by reference in its entirety. Dry eye is also sometimes referred to as keratoconjunctivitis sicca. In some embodiments, the treatment of the dry eye disorder involves ameliorating a particular symptom of dry eye disorder, such as eye discomfort, visual disturbance, tear film instability, tear hyperosmolarity, and inflammation of the ocular surface.

As summarized in the DEWS report, dry eye can be classified into two different classes: aqueous tear-deficient dry eye and evaporative dry eye, which in turn encompass various subclasses. Accordingly, in some embodiments, the dry eye disorder is aqueous tear-deficient dry eye (ADDE). In further embodiments, the dry eye disorder is evaporative dry eye. In further embodiments, the dry eye disorder is selected from any of the subclasses of ADDE or evaporative dry eye disorder, or appropriate combinations thereof. As noted by the author of the DEWS report, however, the various classes and subclasses are not mutually exclusive. Hence, dry eye can occur via different mechanism in different subclasses or a dry eye disease state originating in one subclass can lead to events that cause dry eye by a mechanism in another subclass.

The first class of dry eye, aqueous tear-deficient dry eye (ADDE), is also known as tear deficient dry eye and lacrimal tear deficiency. In ADDE, dry eye is believed to be due to a failure of lacrimal tear secretion. While not wishing to be bound by any theory, it is believed that dryness results from reduced lacrimal tear secretion and volume, causing tear hyperosmolarity. Tear film hyperosmolarity can cause hyperosmolarity of the ocular surface epithelial cells, stimulating inflammatory events involving various kinases and signaling pathways.

Two subclasses of ADDE are Sjogren syndrome dry eye (SSDE), where the lacrimal glands are targeted by an autoimmune process, and non-Sjogren syndrome dry eye (NSSDE) . Accordingly, in some embodiments, the eye disorder is SSDE. In other embodiments, dry eye disorder is non-Sjogren syndrome dry eye. In SSDE, it is believed that activated T-cells can infiltrate the lacrimal glands, causing cell death of acinar and ductular cells and hyposecretion of tears. The effects of locally released cytokines or circulating antibodies can amplify the effects of hyposecretion. The two major forms of SSDE are primary and secondary forms. Primary SS can occur in combination with dry mouth (xerostomia). Secondary SSDE occurs with the symptoms of primary SSDE together with an autoimmune connective disease such as rheumatoid arthritis (RA), systemic lupus erythematosis, polyarteritis nodosa, Wegener's granulomatosis, systemic sclerosis, primary bilary sclerosis, or mixed connective tissue disease. Diagnostic criteria for each of these connective diseases is known in the art. Further, primary SSDE may be associated with systemic manifestations of disease which may involve the lungs, kdneys, liver, blood vessels and joints.

In NSSDE, the systemic autoimmune characteristics of Sjogren syndrome dry eye are excluded. Forms of NSSDE include primary lacrimal gland deficiencies (including age-related dry eye, congenital alacrima, and familial dysautonomia), secondary lacrimal deficiencies (including inflammatory infiltration of the lacrimal gland by sarcoid granulomata, lymphomatous cells, and AIDS related T-cells; that associated with graft vs. host disease; and that resulting from lacrimal gland ablation or lacrimal gland denervation), obstruction of the lacrimal gland ducts (including that caused by cicatrizing conjunctivitis including trachoma, cicatricial pemphigoid and mucous membrane pemphigoid, erythema multiforme, and chemical or thermal burns), and reflex hyposecretion (including reflex sensory block, such as that associated with contact lens wear, diabetes mellitus, and neurotrophic keratitis, and reflex motor block, including that associated with VII cranial nerve damage, multiple neuromatosis, and exposure to systemic drugs such as antihistamines, beta blockers, antispasmodics, diuretics, tricyclic antidepressants, selective serotonin reuptake inhibitors, and other psychotropic drugs).

The second major class of dry eye disorder is evaporative dry eye, which is caused by excessive water loss from the exposed ocular surface in the presence of normal lacrimal secretory function. Intrinsic causes of evaporative dry eye include Meibomian gland dysfunction (MGD) (including that caused by a reduced number of glands due to congenital deficiency acquired-MGD; MGD associated with dystichiasis, dystichiasis lymphedema syndrome, and metaplasia; hypersecretory MGD associated with Meibomian seborrhea, hypersecretory MGD associated with retinoid therapy, primary and secondary obstructive MGD, focal or diffuse obstructive MGD, simple or cicatricial obstructive MGD, atrophic or inflammatory obstructive MGD; Simple MGD primary or secondary to anterior blepharitis, acne rosacea, seborrhoeic dermatitis, ectrodactyly syndrome, Turner syndrome, systemic toxicity from 13-cis retinoic acid, polychlorinated biphenyls, and epinephrine; and cicatricial MGD primary or secondary to chemical burns, pemphigoid, acne rosacea, erythema multiforms, VKC and AKC), disorders of the lid aperture and lid/globe congruity or dynamic (such as that occurring with craniostenosis, endocrine and other forms of proptosis, myopia, and after plastic surgery on the lids), and low blink rate (including that caused by an extrapyramidal disorder such as Parkinson's disease). Extrinsic causes of evaporative dry eye include ocular surface disorders (including xerophthalmia caused by vitamin A deficiency; and that associated with topical drugs and preservatives such as topical anesthesia and benzalkonium chloride), contact lens wear, ocular surface disease (including allergic eye disease), allergic conjunctivitis (including aseasonal allergic conjunctivitis, vernal keratoconjunctivitis, and atopic keratoconjunctivitis), and the use of antihistamines.

Patients in need of treatment of a dry eye disorder can be identified by a variety of diagnostic methods known in the art, including the diagnostic methods summarized in Bron, et al., "Methodologies to Diagnose and Monitor Dry Eye Disease: Report of the Diagnostic Methodology Subcommittee of the International Dry Eye Workshop (2007)", The Ocular Surface, 5(2), 108-152 (April 2007), which is hereby incorporated herein by reference in its entirety. These include, but are not limited to: (1) symptom questionnaires (e.g., Begley, et al., "Use of the dry eye questionnaire to measure symptoms of ocular irritation in patients with aqueous tear deficient dry eye", Cornea, 2002:21:664-70); (2) staining of the ocular surface to check for surface damage (e.g., Rose Bengal or fluorescein staining or other staining method such as those techniques summarized in Barr et al., "Corneal scarring in the Collaborative Longitudinal Evaluation of Keratoconus (CLEK) Study: baseline prevalence and repeatability of detection", Cornea 1999;18(1):34-46; Lemp, "Report of the National Eye Institute/Industry Workshop on clinical trials in dry eyes", CLAO J 1995;21(4):221-31; Nichols, et al., "The repeatability of clinical measurements of dry eye", Cornea 2004;23:272-85; Bron, et al., "Grading of corneal and conjunctival staining in the context of other dry eye tests", Cornea 2003;22(7):640-50); (3) measurement of tear film break-up time to test for tear film stability (e.g., Abelson, et al., "Alternate reference values for tear film break-up time in normal and dry eye populations", Adv Exp Med Biol 2002;506,Part B:1121-1125; Bron AJ, et al., "Grading of corneal and conjunctival staining in the context of other dry eye tests", Cornea 2003;22:640-50; Cho et al, "Review of the tear break-up time and a closer look at the tear break-up time of Hong Kong Chinese", Optom Vis Sci 1993;70(1):30-8; Craig et al. "Tear lipid layer structure and stability following expression of the meibomian glands. Ophthalmic Physiol Opt 1995, 15(6):569-74; Eliason, et al., "Staining of the conjunctiva and conjunctival tear film", Br J Ophthalmol 1990;74:519-22; Farrell et al., "A classification for dry eyes following comparison of tear thinning time with Schirmer tear test", Acta Ophthalmol (Copenh) 1992; 70(3):357-60; Johnson et al., "The effect of instilled fluorescein solution volume on the values and repeatability of TBUT measurements", Cornea 2005;24:811-7; Lemp et al., "Corneal desiccation despite normal tear volume", Ann Ophthalmol 1970;284:258-261; Lemp "Report of National Eye Institute/Industry Workshop on clinical trials in dry eyes", CLAO J 1995;21:221-232; Madden et al. Comparative study of two non-invasive tear film stability techniques. Curr Eye Res 1994; 13(4):263-9; Marquardt et al., "Modification of tear film break-up time test for increased reliability" in Holly ed. The Preocular Tear Film in Health, Disease and Contact Lens Wear. Lubbock, Texas: Dry Eye Institute, 1986:57-63; Mengher et al., "Non-invasive tear film break-up time: sensitivity and specificity", Acta Ophthalmol (Copenh) 1986; 64(4):441-4; Nichols et al., "The repeatability of clinical measurements of dry eye" Cornea 2004;23:272-85; Pflugfelder et al. "Evaluation of subjective assessments and objective diagnostic tests for diagnosing tear-film disorders known to cause ocular irritation. Cornea 1998; 17(1):38-56; Vitali ct al. "The European Community Study Group on diagnostic criteria for Sjogren's syndrome. Sensitivity and specificity of tests for ocular and oral involvement in Sjogren's syndrome." 1992; Ann Rheum Dis 53(10):637-47; Welch et al., "An approach to a more standardized method of evaluating tear film break-up time" Invent Ophthalmol Vis Sci 2003; 2485/B324.); (4) the Schirmer test (an estimation of tear flow stimulated reflexly by insertion of a filter paper into the
conjunctival sac) (e.g., van Bijsterveld, "Diagnostic tests in the sicca syndrome" Arch Ophthalmol 1969;82:10-14; Holly et al., "Lacrimation kinetics as determined by a novel technique", in Holly FJ (ed). The preocular tear film. Lubbock TX, Lubbock Dry Eye Institute, 1986, pp 76-88); (5) measurement of tear osmolarity (e.g., Farris, "Tear osmolarity--a new gold standard?" Adv Exp Med Biol 350:495-503, 1994; Nelson et al., "Tear film osmolality determination: an evaluation of potential errors in measurement" Curr Eye Res Sep;5(9):677-81, 1986; Sullivan et al., "4th International Conference on the Lacrimal Gland, Tear Film & Ocular Surface and Dry Eye Syndromes, 11/20/04"; White et al., "Human basic tear fluid osmolality. I. Importance of sample collection strategy", Acta Ophthalmol (Copenh) Aug;71(4):524-9, 1993; (6) measurement of tear meniscus radius, height and cross sectional area to diagnose aqueous tear deficiency (e.g., Cennak et al, "Is complete androgen insensitivity syndrome associated with alterations in the meibomium gland and ocular surface", Cornea 2003;22:516-521; Farrell et al., "A clinical procedure to predict the value of temporary occlusion therapy in keratoconjunctivitis sicca" Ophthal Physiol Opt 2003;23:1-8; Glasson et al., "Differences in clinical parameters and tear film of tolerant and intolerant contact lens wearers", Invest Ophthalmol Vis Sci 2003;44:5116-5124; Mainstone et al., "Tear meniscus measurement in the diagnosis of dry eye", Curr Eye Res 1996; 15:653-661; Nichols et al., "The repeatability of clinical measurements of dry eye", Cornea 2004a; 23:272-285; Nichols et al., "The lack of association between signs and symptoms in patients with dry eye disease", Cornea 2004b; 23:762-770; Oguz et al., "The height and radius of the tear meniscus and methods for examining these parameters", Cornea 2000;19:497-500; Yokoi et al., "Non-invasive methods of assessing the tear film", Exp Eye Res 2004;78:399-407); (7) tear film lipid layer interferometry to diagnose aqueous tear deficient dry eye (ATD) or precorneal lipid tear deficiency (Danjo et al., "Observation of precorneal tear film in patients with Sjogren's syndrome", Acta Ophthalmol Scand 1995;73:501-5; Doane, "An instrument for in vivo tear film interferometry", Optom Vis Sci 1989; 66: 383-8; Goto et al., "Computer-synthesis of an interference color chart of human tear lipid layer by a colorimetric approach",Invest Ophthalmol Vis Sci 2003;44:4693-7; Goto et al., "Differentiation of lipid tear deficiency dry eye by kinetic analysis of tear interference images", Arch Ophthalmol 2003; 121:173-80; Goto E, et al., "Kinetic analysis of tear interference images in aqueous tear deficiency dry eye before and after punctal occlusion. Invest Ophthalmol Vis Sci 2003;44:1897-905; Goto et al., "Color mapping of tear lipid layer thickness distribution from the image analysis in DR-1 tear lipid layer interference images (ARVO abstract). ARVO 2004; Guillon, "Tear film photography and contact lens wear", JBr Contact Lens Assoc 1982;5:84-7; King-Smith et al., "Three interferometric methods for measuring the thickness of layers of the tear film", Optom Vis Sci 1999;76:19-32; Korb, et al., "Increase in tear film lipid layer thickness following treatment of meibomian gland dysfunction", Adv Exp Med Biol 1994;350:293-8; Korb et al., "The effect of two novel lubricant eye drops on tear film lipid layer thickness in subjects with dry eye symptoms", Optom Vis Sci 2005; 82: 594-601; Mathers et al., "Assessment of the tear film with tandem scanning confocal microscopy", Cornea 1997;16:162-8; Maruyama et al., "Effect of environmental conditions on tear dynamics in soft contact lens wearers", Invest Ophthalmol Vis Sci 2004;45(8):2563-8; Tiffany, "Refractive index of meibomian and other lipids", Curr Eye Res 1986;5:887-9; Tiffany et al., "Meniscometry using the Tearscope-plus (ARVO abstract). Invest Ophthalmol Vis Sci 2001;42, s37; Yokoi et al., "Correlation of tear lipid layer interference patterns with the diagnosis and severity of dry eye", Am J Ophthalmol 1996;122:818-24; Yokoi et al., "Assessment of meibomian gland function in dry eye using meibometry", Arch Ophthalmol 1999;117:723-9); (8) Tear Stability Analyses System (TSAS) to diagnose tear instability (e.g., Goto et al., "Tear Film Stability Analysis System: Introducing a new application for videokeratography", Cornea 2004a; Nov;23(8):S65-S70; Goto et al., "Evaluation of the tear film stability after laser in situ keratomileusis using the tear film stability analysis system", Am J Ophthalmol 2004b Jan; 137(1):116-20; Kojima et al., "A new noninvasive tear stability analysis system for the assessment of dry eyes" Invest Ophthalmol Vis Sci 2004;May;45(5):1369-74); (9) meibometry to assess Meibomian gland dysfunction (e.g., Chew et al., "An instrument for quantifying meibomian lipid on the lid margin: the Meibometer", Curr Eye Res 1993a;12:247-254; Chew et al., "The casual level of meibomian lipids in humans", Current Eye Research 1993b;12:255-259; Komuro et al., "Assessment of meibomian gland function by a newly developed laser meibometer", Adv Exp Med Biol 2002;506:517-520; Yokoi et al., "Assessment of meibomian gland function in dry eye using meibometry" Arch Ophthalmol 1999;117:723-729); (10) meibography or meiboscopy to measure Meibomian gland dysfunction (e.g., Kaercher, "Ocular symptoms and signs in patients with ectodermal dysplasia symdromes", Grafes Arch Clin Exp Ophthalmol 2004;495-500; Jester et al., "In vivo biomcroscopy and photography of meibomian glands in a rabbit model of meibomian gland dysfunction", Invent Ophthalmol Vis Sci 1982;22:660-7; Mathers et al., "Video imaging of the meibomian gland", Arch Ophthalmol 1994;112:448-9; Pflugfelder, et al., "Evaluation of subjective assessments and objective diagnostic tests for diagnosing tear-film disorders known to cause ocular irritation", Cornea 1998;17(1):38-56; Robin et al., "In vivo transillumination biomicroscopy and photography of meibomian gland dysfunction. Ophthalmology 1985;92:1423-6; Shimazaki et al., "Meibomian gland dysfunction in patients with Sjogren syndrome", Ophthalmology 1998;105(8):1485-8; Yokoi et al., "A newly developed video-meibography system featuring a newly designed probe", Jpn J Ophthalmol 2007; 51: 53-6); (11) Brush Cytology Technique (e.g., Fukagawa et al., "Histological evaluation of brush cytology of rabbit conjunctiva", Nippon Ganka Gakkai Zasshi 1993;97:1173-8; Fujihara et al., "Evaluation of human conjunctival epithelium by a combination of brush cytology and flow cytometry: an approach to the quantitative technique", Diagn Cytopathol 1997;17:456-60; Miyoshi ct al., "Intcrlcukin-8 concentrations in conjunctival epithelium brush cytology samples correlate with neutrophil, eosinophil infiltration, and corneal damage", Cornea 2001;20:743-7; Takano et al., "Inflammatory cells in brush cytology samples correlate with the severity of corneal lesions in atopic keratoconjunctivitis", Br J Ophthalmol 2004;88:1504-5; Tsubota et al., "Brush cytology for the evaluation of dry-eye", Nippon Ganka Gakkai Zasshi 1990a ;94:224-30; Tsubota et al., "Conjunctival brush cytology", Acta Cytol 1990 b;34:233-5; Tsubota et al., "Detection by brush cytology of mast cells and eosinophils in allergic and vernal conjunctivitis"; Cornea 1991;10:525-31); (12) Flow cytometry in impression cytology to detect conjuctivial inflammation (e.g., Baudouin et al., "Flow cytometry in impression cytology specimens. A new method for evaluation of conjunctival Inflammation", Invest Ophthalmol Vis Sci 1997a;38:1458-1464; Bourcier et al., "Expression of CD40 and CD40 ligand in the human conjunctival epithelium", Invest Ophthalmol Vis Sci 2000;41:120-126; Brignole et al., "Expression of Fas antigen (CD95) in the human conjunctival epithelium. Positive correlation with class II HLA DR expression in inflammatory conditions", Exp Eye Res 1998;67:687-697; Brignole et al., "Flow cytometric analysis of inflammatory markers in conjunctival epithelial cells of patients with dry eyes" Invest Ophthalmol Vis Sci 2000; 41:1356-1363; Brignole et al., "Flow cytometric analysis of inflammatory markers in KCS: 6-month treatment with topical cyclosporin A", Invest Ophthalmol Vis Sci 2001;42:90-95; Brignole et al., "Flow cytometry in conjunctival impression cytology: a new tool for exploring ocular surface pathologies", Exp Eye Res 2004;78:473-481; Fujihara et al., "Evaluation of human conjunctival epithelium by a combination of brush cytology and flow cytometry: an approach to the quantitative technique" Diagn Cytopathol 1997;17:456-460; Pisella et al., "Flow cytometric analysis of conjunctival epithelium in ocular rosacea and keratoconjunctivitis sicca. Ophthalmology 2000;107:1841-1849; Pisella, et al., "Conjunctival proinflammatory and proapoptotic effects of latanoprost, preserved timolol and unpreserved timolol: an ex vivo and in vitro study. Invest Ophthalmol Vis Sci 2004;45:1360-1368); (13) the Ferning test to diagnose the quality of tears (electrolyte concentration), KCS, and hyperosmolarity (e.g., Albach et al., "Diagnosis of keratoconjunctivitis sicca in rheumatoid arthritis. The value of various tests", Ophthalmologe 1994 Apr;91(2):229-34; Golding et al., "X-ray and scanning electron microscopic analysis of the structural composition oftear ferns", Cornea 1994 Jan;13(1):58-66; Norn, "Quantitative tear ferning. Clinical investigations", Acta Ophthalmol (Copenh) 1994 Jun;72(3):369-72; Pearce et al., "Spatial location studies on the chemical composition of human tear ferns", Ophthalmic Physiol Opt 2000;Jul;20(4):306-13; Pensyl et al., "The repeatability of tear mucus ferning grading", Optom Vis Sci 1998 Aug;75(8):600-4; Rolando, "Tear mucus ferning test in normal and keratoconjunctivitis sicca eyes. Chibret Int J Ophthalmol 1984;2(4):32-41; Rolando et al., "Tear mucus ferning test in keratoconjunctivitis sicca",in: Holly FJ, Lamberts DW, MacKeen DL (eds.): The preocular tear film in health, disease, and contact lens wear,. 1st Intern Tear Film Symposium. Lubbok (Texas, USA), Dry Eye Institute, 1986, 203-210; Rolando et al., "The effect of hyperosmolarity on tear mucus ferning", Fortschr Ophthalmol 1986;83:644-646; Rolando et al., "Tear mucus crystallization in children with cystic fibrosis", Ophthalmologica 1988;197(4):202-6); (14) Ocular Protection Index (OPT) to assess ocular surface protection and risk of ocular surface damage (e.g., Ousler et al., "Factors that influence the inter-blink interval (IBI) as measured by the ocular protection index (OPI)", (Poster presentation) ARVO 2002; Nally et al., "Ocular discomfort and tear film break-up time in dry eye patients: A correlation", Invest Ophthalmol Vis Sci 2000;41:4:1436; Abelson et al., "Alternate reference values for tear film break-up time in normal and dry eye populations", Lacrimal Gland, Tear Film, and Dry Eye Syndromes 3 Part B", Adv Exp Med Biol 2002; 506:1121-1125; Abelson et al., "Dry eye syndrome: diagnosis, clinical trials, and pharmaceutical treatment-'improving clinical trials'. Lacrimal Gland, Tear Film, and Dry Eye Syndromes 3 Part B", Adv Exp Med Biol 2002; 506:1079-86); (15) fluorophotometry (fluorimetry) of tear flow to assess changes in tear flow in aqueous tear deficiency (ATD) (e.g., Gobbels et al., "Tear secretion in dry eyes as assessed by objective fluorophotometry. Ger J Ophthalmol 1992; 1:350-353; Kuppens et al., "Basal tear turnover and topical timolol in glaucoma patients and healthy controls by Fluorophotometry", Invest Ophthalmol Vis Sci 1992; 33:3442-3448; Mishima, "Some physiological aspects of the precorneal tear film", Arch Ophthalmol 1965;73:233-241; Mishima S, "Determination of tear volume and tear flow", Invest Ophthalmol 1966; 5:264-275; Mathers et al., "Tear film and evaporation in patients with and without dry eye", Ophthalmology 1996; 103:664-669; Mathers et al., "Tear film changes associated with normal aging", Cornea 1996; 15:229-334; Mathers, "Evaporation from the ocular surface", Exp Eye Res 2004; 78:389-394; Van Best et al., "Measurement of basal tear turnover using a standardized protocol", Graefe's Arch Clin Exp Ophthalmol 1995; 233:1-7; McNamara et al., "Fluorometry in contact lens research: The next step", Optom Vis Sci 1998; 75:316-322; Pearce, "An improved fluorophotometric method for tear turnover assessment", Optom Vis Sci 2001; 78:30-36), and combinations of these diagnostic tests, the disclosures of each reference are incorporated herein by reference in their entireties. These methods can also be used to assess the clinical efficacy of the agents of the invention in treating dry eye disorders.

In a further aspect, the present invention provides a method of treating conjunctivitis, uveitis (including chronic uveitis), chorioditis, retinitis, cyclitis, sclieritis, episcleritis, or iritis; treating inflammation or pain related to corneal transplant, LASIK (laser assisted in situ keratomileusis), photorefractive keratectomy, or LASEK (laser assisted sub-epithelial keratomileusis); inhibiting loss of visual acuity related to corneal transplant, LASIK, photorefractive keratectomy, or LASEK; or inhibiting transplant rejection in a patient in need thereof, comprising administering to the patient a therapeutically effective amount of an agent, or pharmaceutically acceptable salt thereof. In some embodiments, the agent is administered preoperatively to a patient about to undergo a procedure selected from corneal transplant, LASIK, photorefractive keratectomy, and LASEK. In some embodiments, the agent suppresses or lessens inflammation or pain during and after the procedure. In some embodiments, the agent is administered about 1 day to about 2 days prior to the procedure. In some embodiments, the agent is administered postoperatively to a patient who has undergone a procedure selected from corneal transplant, LASIK, photorefractive keratectomy, and LASEK. In some embodiments, inhibiting loss of visual acuity means lessening the loss of visual acuity. In some embodiments, the postoperative or preoperative treatment lessens the amount of scarring and fibrous deposits following the procedure. In some embodiments, inhibiting loss of visual acuity means that the patient retains visual acuity. In some embodiments, inhibiting transplant rejection means that the agent is immunosuppressive, thereby preventing total rejection of the corneal transplant.

In some embodiments, one or more additional therapeutic agents, or other agents, can be used in combination with the agent in the methods of the present invention. The one or more additional therapeutic agents can be administered to a patient simultaneously or sequentially. In some embodiments, the amount of additional therapeutic agent, when administered in a compositions, is from about 0.01% to 5% by weight, from about 0.1% to 2% by weight, or from 0.5% to 50% by weight.

In some embodiments, the additional therapeutic agent is fluocinolone acetonide (Retisert®), or rimexolone (AL-2178, Vexol, Alcon).

In some embodiments, the additional therapeutic agent is cyclosporine (Restasis®).

In some embodiments, the additional therapeutic agent is a corticosteroid. In some embodiments, the corticosteroid is triaminolone, dexamethasone, fluocinolone, cortisone, prednisolone, or flumetholone.

In some embodiments, the additional therapeutic agent is selected from Dehydrex™ (Holles Labs), Civamide (Opko), sodium hyaluonate (Vismed, Lantibio/TRB Chemedia), cyclosporine (ST-603, Sirion Therapeutics), ARG101(T) (testosterone, Argentis), AGR1012(P) (Argentis), ecabet sodium (Senju-Ista), gefarnate (Santen), 15-(s)-hydroxyeicosatetraenoic acid (15(S)-HETE), cevilemine, doxycline (ALTY-0501, Alacrity), minocycline, iDestrin™ (NP50301, Nascent Pharmaceuticals), cyclosporine A (Nova22007, Novagali), oxytetracycline (Duramycin, MOLI1901, Lantibio), CF101 (2S,3S,4R,5R)-3,4-dihydroxy-5-[6-[(3-iodophenyl)methylamino]purin-9-yl]-N-m ethyl-oxolane-2-carboxamide, Can-Fite Biopharma), voclosporin (LX212 or LX214, Lux Biosciences), ARG103 (Agentis), RX-10045 (synthetic resolvin analog, Resolvyx), DYN15 (Dyanmis Therapeutics), rivoglitazone (DE011, Daiichi Sanko), TB4 (RegeneRx), OPH-01 (Ophtalmis Monaco), PCS101 (Pericor Science), REV 1-31 (Evolutec), Lacritin (Senju), rebamipide (Otsuka-Novartis), OT-551 (Othera), PAI-2 (University of Pennsylvania and Temple University), pilocarpine, tacrolimus, pimecrolimus (AMS981, Novartis), loteprednol etabonate, rituximab, diquafosol tetrasodium (INS365, Inspire), KLS-0611 (Kissei Pharmaceuticals), dehydroepiandrosterone, anakinra, efalizumab, mycophenolate sodium, etanercept (Embrel®), hydroxychloroquine, NGX267 (TorreyPines Therapeutics), or thalidomide.

In some embodiments, the additional therapeutic agent is an anti-angiogenic agent, cholinergic agonist, TRP-1 receptor modulator, a calcium channel blocker, a mucin secretagogue, MUC1 stimulant, a calcincurin inhibitor, a corticosteroid, a P2Y2 receptor agonist, a muscarinic receptor agonist, another JAK inhibitor, Bcr-Abl kinase inhibitor, Flt-3 kinase inhibitor, RAF kinase inhibitor, and FAK kinase inhibitor such as, for example, those described in WO 2006/056399. In some embodiments, the additional therapeutic agent is a tetracycline derivative (e.g., minocycline or doxycline).

In some embodiments, the additional therapeutic agent(s) are demulcent eye drops (also known as "artificial tears"), which include, but are not limited to, compositions containing polyvinylalcohol, hydroxypropyl methylcellulose, glycerin, polyethylene glycol (e.g. PEG400), or carboxymethyl cellulose. Artificial tears can help in the treatment dry eye by compensating for reduced moistening and lubricating capacity of the tear film. In some embodiments, the additional therapeutic agent is a mucolytic drug, such as N-acetyl-cysteine, which can interact with the mucoproteins and, therefore, to decrease the viscosity of the tear film.

In some embodiments, the additional therapeutic agent includes an antibiotic, antiviral, antifungal, anesthetic, anti-inflammatory agents including steroidal and non-steroidal antiinflammatories, and anti-allergic agents. Examples of suitable medicaments include aminoglycosides such as amikacin, gentamycin, tobramycin, streptomycin, netilmycin, and kanamycin; fluoroquinolones such as ciprofloxacin, norfloxacin, ofloxacin, trovafloxacin, lomefloxacin, levofloxacin, and enoxacin; naphthyridine; sulfonamides; polymyxin; chloramphenicol; neomycin; paramomomycin; colistimethate; bacitracin; vancomycin; tetracyclines; rifampin and its derivatives ("rifampins"); cycloserine; beta-lactams; cephalosporins; amphotericins; fluconazole; flucytosine; natamycin; miconazole; ketoconazole; corticosteroids; diclofenac; flurbiprofen; ketorolac; suprofen; comolyn; lodoxamide; levocabastin; naphazoling; antazoline; pheniramimane; or azalide antibiotic.

Example Bcr-Abl inhibitors include the compounds, and pharmaceutically acceptable salts thereof, of the genera and species disclosed in U.S. Pat. No. 5,521,184, WO 04/005281, EP2005/009967, EP2005/010408, and U.S. Ser. No. 60/578,491.

Example suitable Flt-3 inhibitors include compounds, and their pharmaceutically acceptable salts, as disclosed in WO 03/037347, WO 03/099771, and WO 04/046120.

Example suitable RAF inhibitors include compounds, and their pharmaceutically acceptable salts, as disclosed in WO 00/09495 and WO 05/028444.

Example suitable FAK inhibitors include compounds, and their pharmaceutically acceptable salts, as disclosed in WO 04/080980, WO 04/056786, WO 03/024967, WO 01/064655, WO 00/053595, and WO 01/014402.

When employed as pharmaceuticals, the agents can be administered in the form of pharmaceutical compositions. These compositions can be prepared in a manner well known in the pharmaceutical art, and can be administered by a variety of routes, depending upon whether local or systemic treatment is desired and upon the area to be treated. Administration may be topical (including transdermal, epidermal, ophthalmic and to mucous membranes including intranasal, vaginal and rectal delivery), pulmonary (e.g., by inhalation or insufflation of powders or aerosols, including by nebulizer; intratracheal or intranasal), oral or parenteral. Parenteral administration includes intravenous, intraarterial, subcutaneous, intraperitoneal intramuscular or injection or infusion; or intracranial, e.g., intrathecal or intraventricular, administration. Parenteral administration can be in the form of a single bolus dose, or may be, for example, by a continuous perfusion pump. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

In some embodiments, the agent is administered as an ophthalmic composition. Accordingly, in some embodiments, the methods comprise administration of the agent and an ophthalmically acceptable carrier. In some embodiments, the ophthalmic composition is a liquid composition, semi-solid composition, insert, film, microparticles or nanooparticles.

In some embodiments, the ophthalmic composition is a liquid composition. In some embodiments, the ophthalmic composition is a semi-solid composition. In some embodiments, the ophthalmic composition is an topical composition. The topical compositions include, but are not limited to liquid and semi-solid compositions. In some embodiments, the ophthalmic composition is a topical composition. In some embodiments, the topical composition comprises aqueous solution, an aqueous suspension, an ointment or a gel. In some embodiments, the ophthalmic composition is topically applied to the front of the eye, under the upper eyelid, on the lower eyelid and in the cul-de-sac. In some embodiments, the ophthalmic composition is sterilized. The sterilization can be accomplished by known techniques like sterilizing filtration of the solution or by heating of the solution in the ampoule ready for use. The ophthalmic compositions of the invention can further contain pharmaceutical excipients suitable for the preparation of ophthalmic formulations. Examples of such excipients are preserving agents, buffering agents, chelating agents, antioxidant agents and salts for regulating the osmotic pressure.

As used herein, the term "ophthalmically acceptable carrier" refers to any material that can contain and release the agent and that is compatible with the eye. In some embodiments, the ophthalmically acceptable carrier is water or an aqueous solution or suspension, but also includes oils such as those used to make ointments and polymer matrices such as used in ocular inserts. In some embodiments, the composition may be an aqueous suspension comprising the agent. Liquid ophthalmic compositions, including both ointments and suspensions, may have a viscosity that is suited for the selected route of administration. In some embodiments, the ophthalmic composition has a viscosity in the range of from about 1,000 to about 30,000 centipoise.

In some embodiments, the liquid composition further comprises a polymer. These polymers may be used to improve the bioavailability, raise viscosity, or reduce drainage from the eye for a liquid formulation. In some embodiments, the polymers include, but are not limited to, those described in Wagh, ct al., "Polymers used in ocular dosage form and drug delivery systems", Asian J. Pharm., pages 12-17 (Jan. 2008), which is incorporated herein by reference in its entirety. In some embodiments, the polymer is sodium hyaluronase, chitosan, a cyclodextrin (e.g., hydroxypropyl β-cyclodextrin), polygalactoronic acid, xyloglucan, xanthan gum, gellan gum, a thiomer, a poly(ortho ester) (e.g., as described in Einmahl, Adv. Drug. Deliv. Rev. 53:45-73 (2001), which is incorporated herein by reference in its entirety), or a tamarind seed polysaccharide (e.g., as described in Ghelardi, et al., Antimicrob. Agents Chemother. 48:3396-3401 (2004), which is incorporated herein by reference in its entirety).

In some embodiments, the ophthalmic compositions may further comprise one or more of surfactants, adjuvants, buffers, antioxidants, tonicity adjusters, preservatives (e.g., EDTA, BAK (benzalkonium chloride), sodium chlorite, sodium perborate, polyquaterium-1), thickeners or viscosity modifiers (e.g., carboxymethyl cellulose, hydroxymethyl cellulose, polyvinyl alcohol, polyethylene glycol, glycol 400, propylene glycol hydroxymethyl cellulose, hydroxpropyl-guar, hyaluronic acid, and hydroxypropyl cellulose) and the like. Additives in the formulation may include, but are not limited to, sodium chloride, sodium bicarbonate, sorbic acid, methyl paraben, propyl paraben, chlorhexidine, castor oil, and sodium perborate.

Aqueous ophthalmic compositions (solutions or suspensions) generally do not contain physiologically or ophthalmically harmful constituents. In some embodiments, purified or deionized water is used in the composition. The pH may be adjusted by adding any physiologically and ophthalmically acceptable pH adjusting acids, bases or buffers to within the range of about 5.0 to 8.5. Ophthalmically acceptable examples of acids include acetic, boric, citric, lactic, phosphoric, hydrochloric, and the like, and examples of bases include sodium hydroxide, sodium phosphate, sodium borate, sodium citrate, sodium acetate, sodium lactate, tromethamine, trishydroxymethylamino-methane, and the like. Salts and buffers include citrate/dextrose, sodium bicarbonate, ammonium chloride and mixtures of the aforementioned acids and bases.

In some embodiments, the osmotic pressure of the ophthalmic composition may be from about 10 milliosmolar (mOsM) to about 400 mOsM, or from 260 to about 340 mOsM. In some embodiments, the osmotic pressure can be adjusted by using appropriate amounts of physiologically and ophthalmically acceptable salts or excipients. In further embodiments, sodium chloride may be used to approximate physiologic fluid. In other embodiments, the composition comprises sodium chloride ranging from about 0.01% to about 1% by weight, or from about 0.05% to about 0.45% by weight, based on the total weight of the composition. Equivalent amounts of one or more salts made up of cations such as potassium, ammonium and the like and anions such as chloride, citrate, ascorbate, borate, phosphate, bicarbonate, sulfate, thiosulfate, bisulfate, sodium bisulfate, ammonium sulfate, and the like can also be used in addition to or instead of sodium chloride to achieve osmolalities within the above stated range. Similarly, a sugar such as mannitol, dextrose, sorbitol, glucose and the like can also be used to adjust osmolality.

In some embodiments, the methods involve forming or supplying a depot of the agent in contact with the external surface of the eye. A depot refers to a source of agent that is not rapidly removed by tears or other eye clearance mechanisms. This allows for continued, sustained high concentrations of agent be present in the fluid on the external surface of the eye by a single application. Without wishing to be bound by any theory, it is believed that absorption and penetration may be dependent on both the dissolved drug concentration and the contact duration of the external tissue with the drug containing fluid. As the drug is removed by clearance of the ocular fluid and/or absorption into the eye tissue, more drug is provided, e.g. dissolved, into the replenished ocular fluid from the depot. Accordingly, the use of a depot may more easily facilitate loading of the ocular tissue for more insoluble agents. In some embodiments, the depot can remain for up to eight hours or more. In some embodiments, the ophthalmic depot forms includes, but is not limited to, aqueous polymeric suspensions, ointments, and solid inserts.

In some embodiments, a semi-solid composition is a liquid formulation which increases in viscosity upon application to the eye, usually because of a polymer in the liquid formulation. This viscosity increase may be triggered by a change in temperature, pH, or electrolyte concentration. In some embodiments, the polymer include, but are not limited to, those described for semi-solid dosage forms in Wagh, et al., "Polymers used in ocular dosage form and drug delivery systems", Asian J. Pharm., pages 12-17 (Jan. 2008), which is incorporated herein by reference in its entirety. In some embodiments, the polymer is celluloseacetophthalate, polyacrylic acid, gellan gum, hyaluronase, chitosan, salts of alginic acid (e.g., sodium alginate), or a block copolymer of ethylene oxide and propylene oxide (e.g., Pluronic®, BASF; poloxamer). In some embodiment, the polyacrylic acid is crosslinked acrylic acid (e.g., Carbopol®). In some embodiments, the semi-solid composition comprises a mixture of carbopol and a block copolymer of ethylene oxide and propylene oxide; a mixture of methyl cellulose and hydroxyethyl cellulose; or a mixture of polyethylene glycol and a block copolymer of ethylene oxide and propylene oxide.

In some embodiments, the ophthalmic composition is an ointment or gel. In some embodiment, the ophthalmic composition is an oil-based delivery vehicle. In some embodiments, the composition comprises a petroleum or lanolin base to which is added the active ingredient, usually as 0.1 to 2%, and excipients. Common bases may include, but are not limited to, mineral oil, petrolatum and combinations thereof. In some embodiments, the ointment is applied as a ribbon onto the lower eyelid.

In some embodiment, the ophthalmic composition is an ophthalmic insert. In some embodiments, the ophthalmic insert is biologically inert, soft, bio-erodible, viscoelastic, stable to sterilization after exposure to therapeutic agents, resistant to infections from air borne bacteria, bio-erodible, biocompatible, and/or viscoelastic. In some embodiments, the insert comprises an ophthalmically acceptable matrix, e.g., a polymer matrix. The matrix is typically a polymer and the agent is generally dispersed therein or bonded to the polymer matrix. In some embodiments, the agent may slowly released from the matrix through dissolution or hydrolysis of the covalcnt bond. In some embodiments, the polymer is bioerodible (soluble) and the dissolution rate thereof can control the release rate of the agent dispersed therein. In another form, the polymer matrix is a biodegradable polymer that breaks down such as by hydrolysis to thereby release the agent bonded thereto or dispersed therein. In further embodiments, the matrix and agent can be surrounded with an additional polymeric coating to further control release. In some embodiments, the insert comprises a biodegradable polymer such as polycaprolactone (PCL), an ethylene/vinyl acetate copolymer (EVA), polyalkyl cyanoacrylate, polyurethane, a nylon, or poly (dl-lactide-co-glycolide) (PLGA), or a copolymer of any of these. In some embodiments, the agent is dispersed into the matrix material or dispersed amongst the monomer composition used to make the matrix material prior to polymerization. In some embodiments, the amount of agent is from about 0.1 to about 50%, or from about 2 to about 20%. In further embodiments, the biodegradable or bioerodible polymer matrix is used so that the spent insert does not have to be removed. As the biodegradable or bioerodible polymer is degraded or dissolved, the agent is released.

In further embodiments, the ophthalmic insert comprises a polymer, including, but are not limited to, those described in Wagh, et al., "Polymers used in ocular dosage form and drug delivery systems", Asian J. Pharm., pages 12-17 (Jan. 2008), which is incorporated herein by reference in its entirety. In some embodiments, the insert comprises a polymer selected from polyvinylpyrrolidone (PVP), an acrylate or methacrylate polymer or copolymer (e.g., Eudragit® family of polymers from Rohm or Degussa), hydroxymethyl cellulose, polyacrylic acid, poly(amidoamine) dendrimers, poly(dimethyl siloxane), polyethylene oxide, poly(lactide-co-glycolide), poly(2-hydroxyethylmethacrylate), poly(vinyl alcohol), or poly(propylene fumarate). In some embodiments, the insert comprises Gelfoam® R. In some embodiments, the insert is a polyacrylic acid of 450 kDa-cysteine conjugante.

In some embodiments, the ophthalmic composition is a ophthalmic film. Polymers suitable for such films include, but are not limited to, those described in Wagh, et al., "Polymers used in ocular dosage form and drug delivery systems", Asian J. Pharm., pages 12-17 (Jan. 2008), In some embodiments, the film is a soft-contract lense, such as ones made from copolymers of N,N-diethylacrylamide and methacrylic acid crosslinked with ethyleneglycol dimethacrylate.

In some embodiments, the insert comprises a core comprising the agent and an outer tube (see e.g., U.S. Patent Pub. No. 20040009222, which is incorporated herein by reference in its entirety). In some embodiments, the outer tube may be permeable, semi-permeable, or impermeable to the drug. In some embodiments, the drug core may include a polymer matrix which does not significantly affect the release rate of the drug. In some embodiments, the outer tube, the polymer matrix of the drug core, or both may be bioerodible. In some embodiments, the co-extruded product can be segmented into drug delivery devices. In some embodiments, the devices may be left uncoated so that their respective ends are open, or the devices may be coated with, for example, a layer that is permeable to the agent, semi-permeable to the agent, or biocrodible. In certain embodiments, the agent and at least one polymer are admixed in powder form. In some embodiments, the insert is formed by forwarding a polymeric material to a first extrusion device, forwarding an agent to a second extrusion device, coextruding a mass including the polymeric material and the agent, and forming the mass into at least one co-extruded drug delivery device which comprises a core including the agent and an outer layer including the polymeric material. In certain embodiments, the agent forwarded to the second extrusion device is in admixture with at least one polymer. In certain embodiments, the agent and the at least one polymer are admixed in powder form. In certain embodiments, this act includes forwarding more than one drug to the second extrusion device. In certain embodiments, the polymeric material is one of impermeable, semi-permeable, or permeable to the agent. The polymeric material may be bioerodible and/or radiation curable. In latter instances, the insert may be irradiated,

In certain embodiments, the insert is in a tubular form, and may be segmented into a plurality of shorter products. In certain embodiments, the insert further comprises a coating of the plurality of shorter products with one or more layers including at least one of a layer that is permeable to the agent, a layer that is semi-permeable to the agent, and a layer that is bioerodible. The polymeric material may include any biocompatible polymer, such as polycaprolactone (PCL), an ethylene/vinyl acetate copolymer (EVA), polyalkyl cyanoacrylate, polyurethane, a nylon, or poly (dl-lactide-co-glycolide) (PLGA), or a copolymer of any of these.

In some embodiments, the insert comprises a therapeutically effective amount of at least one agent coated by or dispersed in a polymer matrix, wherein the agent is in granular or particulate form. In some embodiments, the agent is released from the formulation as drug from the granules dissolves into or within the matrix, diffuses through the matrix, and is released into the surrounding physiological fluid. In some embodiments, the rate of release is limited primarily by the rate of dissolution of the agent from the granules/particles into the matrix; the steps of diffusion through the matrix and dispersion into the surrounding fluid are primarily not release-rate-limiting. In certain embodiments, the polymer matrix is non-bioerodible, while in other embodiments it is bioerodible. Exemplary non-bioerodible polymer matrices can be formed from polyurethane, polysilicone, poly(ethylene-co-vinyl acetate) (EVA), polyvinyl alcohol, and derivatives and copolymers thereof. Exemplary bioerodible polymer matrices can be formed from polyanhydride, polylactic acid, polyglycolic acid, polyorthoester, polyalkylcyanoacrylate, and derivatives and copolymers thereof.

In some embodiments, the insert comprises a collagenous material. In some embodiments, the insert may be a soluble ophthalmic drug insert (SODI, e.g., a polymeric oval film that can be introduced in the upper conjuctival sac for drug delivery; an elliptical insert such as OCUSERT® (Pilocarpine ocular therapeutic system, developed by Alza Corporation) which is made of ethylene vinyl acetate; OCUFIT® (developed by Escalon Ophthalmics Inc., Skillman, NS), which is a rod shaped silicone elastomer; Lacrisert®, a rod shaped insert made of cellulose; New Ophthalmic Drug Delivery Systems (NODS), made of poly (vinyl alcohol); and the inserts described in Fabrizio, Advanced Drug Delivery Reviews 16: 95 -106, 1998, which is incorporated herein by reference in its entirety. In further embodiments, the insert can be placed, depending on the location and the mechanism used to hold the insert in position, by either the patient or the doctor. In further embodiments, the insert comprises collagen, gelatin, or a polymer, wherein the polymer is selected from polycaprolactone (PCL), an ethylene/vinyl acetate copolymer (EVA), polyalkyl cyanoacralate, polyurethane, a nylon, poly(dl-lactide-co-glycolide) (PLGA), or a copolymer of any of the aforementioned. In some embodiments, the insert is implanted under the upper eyelid. In some embodiments, the insert is implanted in the posterior segment of the eye, in the chroidal space, or in the sclera. In some embodiments, the insert is implanted intravitreally or sub-retinally. In some embodiments, the insert is injected sub-retinally. Methods of administration and techniques for their preparation are set forth in Remington's Pharmaceutical Sciences, which is incorporated herein by reference in it entirety.

In other embodiments, the insert provides a sustained release of the agent to the vitreous of the eye. As used herein, "sustained release" means that the composition releases the agent over an extended period of time in a controlled fashion. In some embodiments, the insert releases the agent at a rate such that the aqueous agent concentration remains less than the vitreous agent concentration during the release. In some embodiments, the aqueous agent concentration is from about 0.002 µg/mL to about 0.01 µg/mL, or from about 0.01 µg/mL to about 0.05 µg/mL, or less than about 0.05 µg/mL. In some embodiments, the agent is released at a rate of about 1 µg/day to about 50 µg/day, or from about 1 µg/day to about 10 µg/day. In some embodiments, the insert further comprises an additional therapeutic agent, as detailed above, e.g., fluocinolone acetonide (such as that found in the ophthalmic insert Retisert®).

In some embodiments, the ophthalmic compositon comprises microspheres or nanoparticles. In some embodiment, the microspheres comprise gelatin. In some embodiments, the microspheres are injected to the posterior segment of the eye, in the chroidal space, in the sclera, intravitreally or sub-retinally. In some embodiments, the micospheres or nanoparticles comprises a polymer including, but not limited to, those described in Wagh, et al., "Polymers used in ocular dosage form and drug delivery systems", Asian J. Pharm., pages 12-17 (Jan. 2008), which is incorporated herein by reference in its entirety. In some embodiments, the polymer is chitosan, a polycarboxylic acid such as polyacrylic acid, albumin particles, hyaluronic acid esters, polyitaconic acid, poly(butyl)cyanoacrylate, polycaprolactone, poly(isobutyl)caprolactone, poly(lactic acid-co-glycolic acid), or poly(lactic acid). In some embodiments, the microspheres or nanoparticles comprise solid lipid particles.

In some embodiments, the ophthalmic composition comprises an ion-exchange resin. In some embodiments, the ion-exchange resin is an inorganic zeolite or synthetic organic resin. In some embodiments, the ion-exchange resin includes, but is not limited to, those described in Wagh, et al., "Polymers used in ocular dosage form and drug delivery systems", Asian J. Pharm., pages 12-17 (Jan. 2008), which is incorporated herein by reference in its entirety. In some embodiments, the ion-exhange resin is a partially neutralized polyacrylic acid.

In some embodiments, the ophthalmic composition is an aqueous polymeric suspension. In some embodiments, the agent or a polymeric suspending agent is suspended in an aqueous medium (e.g., having the properties as described above). In some embodiment, the agent is suspended. In some embodiments, the agent is in solution. In further embodiments, the suspending agent serves to provide stability to the suspension, to increase the residence time of the dosage form on the eye, or to enhance the sustained release of the drug in terms of both longer release times and a more uniform release curve. Examples of polymeric suspending agents include, but are not limited to, dextrans, polyethylene glycols, polyvinylpyrolidone, polysaccharide gels, Gelrite®, cellulosic polymers like hydroxypropyl methylcellulose, and carboxy-containing polymers such as polymers or copolymers of acrylic acid, as well as other polymeric demulcents. In some embodiments, the polymeric suspending agent is a water swellable, water insoluble polymer, especially a crosslinked carboxy-containing polymer. In some embodiments, the polymeric suspending agent comprises from at least about 90% to about 99.9%, or from about 95% to about 99.9%, by weight based on the total weight of monomers present, of one or more carboxy-containing monoethylenically unsaturated monomers. In some embodiments, the carboxy-containing monoethylenically unsaturated monomer includes acrylic acid, methacrylic acid, ethacrylic acid, methylacrylic acid (crotonic acid), cis- α-methylcrotonic acid (angelic acid), trans-α-methylcrotonic acid (tiglic acid), α-butylcrotonic acid, α-phenylacrylic acid, α-benzylacrylic acid, α-cyclohexylacrylic acid, phenylacrylic acid (cinnamic acid), coumaric acid (o-hydroxycinnamic acid), and umbellic acid (p-hydroxycoumaric acid). In some embodiments, the polymers may be crosslinked by a polyfunctional crosslinking agent (e.g., a difunctional crosslinking agent). In further embodiments, the amount of crosslinking should be sufficient to form insoluble polymer particles, but not so great as to unduly interfere with sustained release of the agent. In some embodiment, the polymers are only lightly crosslinked. In some embodiments, the crosslinking agent is contained in an amount of from about 0.01% to about 5%, or from about 0.1% to about 5.0%, or from about 0.2% to about 1%, based on the total weight of monomers present. In some embodiments, the crosslinking agents are nonpolyalkenyl polyether difunctional crosslinking monomers such as divinyl glycol, 2,3-dihydroxyhexa-1,5-diene, 2,5-dimethyl-1,5-hexadiene, divinylbenzene, N,N-diallylacrylamide, N,N-diallymethacrylamide; polyalkenyl polyether crosslinking agents containing two or more alkenyl ether groupings per molecule, e.g., alkenyl ether groupings containing terminal H₂C=C<groups, prepared by etherifying a polyhydric alcohol containing at least four carbon atoms and at least three hydroxyl groups with an alkenyl halide such as allyl bromide or the like, e.g., polyallyl sucrose, polyallyl pentaerythritol, or the like; diolefinic non-hydrophilic macromeric crosslinking agents having molecular weights of from about 400 to about 8,000, such as insoluble diacrylates and polyacrylates and methacrylates of diols and polyols, diisocyanate hydroxyalkyl acrylate or methacrylate reaction products of isocyanate terminated prepolymers derived from polyester diols, polyether diols or polysiloxane diols with hydroxyalkylmethacrylates, and the like.

In some embodiments, the crosslinked polymers may be made from a carboxy-containing monoethylenically unsaturated monomer or monomers as the sole monoethylenically unsaturated monomer present, together with a crosslinking agent or agents. In some embodiments, the polymers are ones in which up to about 40%, and preferably from about 0% to about 20% by weight, of the carboxy-containing monoethylenically unsaturated monomer or monomers has been replaced by one or more non-carboxyl-containing monoethylenically unsaturated monomer or monomers containing only physiologically and ophthalmically innocuous substituents, including acrylic and methacrylic acid esters such as methyl methacrylate, ethyl acrylate, butyl acrylate, 2-ethylhexylacrylate, octyl methacrylate, 2-hydroxyethylmethacrylate, 3-hydroxypropylacrylate, and the like, vinyl acetate, N-vinylpyrrolidone, and the like (see Mueller et al. U.S. Pat. No. 4,548,990, the entire contents of which are incorporated herein by reference, for a more extensive listing of such additional monoethylenically unsaturated monomers). In some embodiments, the polymers include polycarbophil (Noveon AA-1), Carbopo®, and DuraSite®. In some embodiments, the crosslinked polymers are prepared by suspension or emulsion polymerizing the monomers, using conventional free radical polymerization catalysts, to a dry particle size of not more than about 50 µm in equivalent spherical diameter. In some embodiments, the average dry particle size is from about 1 to about 30 µm, or from about 3 to about 20 µm in equivalent spherical diameter. In some embodiments, the polymer particles are obtained by mechanically milling larger polymer particles. In further embodiments, such polymers will have a molecular weight from about 250,000 to about 4,000,000, and from 3,000,000,000 to 4,000,000,000. In other embodiments, the particles of crosslinked polymer are monodisperse, meaning that they have a particle size distribution such that at least about 80%, about 90% or about 95%, of the particles fall within a µm band of major particle size distribution. In further embodiments, the monodisperse particle size means that there is no more than about 20%, about 10%, or about 5% particles of a size below 1 µm. In some embodiments, the aqueous polymeric suspension comprises from about 0.05 to about 1%, from about 0.1 to about 0.5%, or from about 0.1 to about 0.5%, of the agent and from about 0.1 to about 10%, from about 0.5 to about 6.5%, from about 0.5 to about 2.0%, from about 0.5% to about 1.2%, from about 0.6 to about 0.9%, or from about 0.6 to about 0.8% of a polymeric suspending agent. Although referred to in the singular, it should be understood that one or more species of polymeric suspending agent can be used with the total amount falling within the stated ranges. In one embodiment, the amount of insoluble lightly crosslinked polymer particles, the pH, and the osmotic pressure can be correlated with each other and with the degree of crosslinking to give a composition having a viscosity in the range of from about 500 to about 100,000 centipoise, and preferably from about 1,000 to about 30,000 or about 1,000 to about 10,000 centipoise, as measured at room temperature (about 25° C.) using a Brookfield Digital LVT Viscometer equipped with a number 25 spindle and a 13R small sample adapter at 12 rpm. In some embodiments, the viscosity is from about 10 to about 400 centipoise, from about 10 to about 200 centipoises or from about 10 to about 25 centipoise.

In some embodiments, the aqueous polymeric suspensions may be formulated so that they retain the same or substantially the same viscosity in the eye that they had prior to administration to the eye. In some embodiments, they may be formulated so that there is increased gelation upon contact with tear fluid. For instance, when a formulation containing DuraSite® or other similar polyacrylic acid-type polymer is administered to the eye at a pH of less than about 6.7, the polymer may swell upon contact with tear fluid since it has a higher pH (around 7). This gelation or increase in gelation may lead to entrapment of the suspended particles, thereby extending the residence time of the composition in the eye. In some embodiments, the agent is released slowly as the suspended particles dissolve over time. In some embodiments, this delivery route increases patient comfort and increased agent contact time with the eye tissues, thereby increasing the extent of drug absorption and duration of action of the formulation in the eye. The agents contained in these drug delivery systems will be released from the gels at rates that depend on such factors as the drug itself and its physical form, the extent of drug loading and the pH of the system, as well as on any drug delivery adjuvants, such as ion exchange resins compatible with the ocular surface, which may also be present.

In some embodiment, the treating comprises administering a pharmaceutical composition to the patient, the composition comprising the agent and a pharmaceutically acceptable carrier. In some embodiments, the pharmaceutical composition is an oral dosage form. In some embodiments, the pharmaceutical compositions comprise, as the active ingredient, one or more of the agents above in combination with one or more pharmaceutically acceptable carriers (excipients). In making the compositions of the invention, the agent is typically mixed with an excipient, diluted by an excipient or enclosed within such a carrier in the form of, for example, a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, the agent can be milled to provide the appropriate particle size prior to combining with the other ingredients. If the agent is substantially insoluble, it can be milled to a particle size of less than 200 mesh. If the agent is substantially water soluble, the particle size can be adjusted by milling to provide a substantially uniform distribution in the formulation, *e.g.* about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions can be formulated so as to provide quick, sustained or delayed release of the active ingredient after administration to the patient by employing procedures known in the art.

The compositions can be formulated in a unit dosage form, each dosage containing from about 5 to about 1000 mg (1 g), more usually about 100 to about 500 mg, of the agent. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The agent can be effective over a wide dosage range and is generally administered in a pharmaceutically effective amount. It will be understood, however, that the amount of the agent actually administered will usually be determined by a physician, according to the relevant circumstances, including the condition to be treated, the chosen route of administration, the actual compound administered, the age, weight, and response of the individual patient, the severity of the patient's symptoms, and the like.

For preparing solid compositions such as tablets, the agent is mixed with a pharmaceutical excipient to form a solid preformulation composition containing a homogeneous mixture of a compound of the present invention. When referring to these preformulation compositions as homogeneous, the agent is typically dispersed evenly throughout the composition so that the composition can be readily subdivided into equally effective unit dosage forms such as tablets, pills and capsules. This solid preformulation is then subdivided into unit dosage forms of the type described above containing from, for example, about 0.1 to about 1000 mg of the agent.

The tablets or pills of the present invention can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the agent and compositions of the present invention can be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* In some embodiments, the compositions are administered by the oral or nasal respiratory route for local or systemic effect. Compositions in can be nebulized by use of inert gases. Nebulized solutions may be breathed directly from the nebulizing device or the nebulizing device can be attached to a face masks tent, or intermittent positive pressure breathing machine. Solution, suspension, or powder compositions can be administered orally or nasally from devices which deliver the formulation in an appropriate manner.

The amount of agent or composition administered to a patient will vary depending upon what is being administered, the purpose of the administration, such as prophylaxis or therapy, the state of the patient, the manner of administration, and the like. In therapeutic applications, compositions can be administered to a patient already suffering from a disease in an amount sufficient to cure or at least partially arrest the symptoms of the disease and its complications. Effective doses will depend on the disease condition being treated as well as by the judgment of the attending clinician depending upon factors such as the severity of the disease, the age, weight and general condition of the patient, and the like.

The compositions administered to a patient can be in the form of pharmaceutical compositions described above. These compositions can be sterilized by conventional sterilization techniques, or may be sterile filtered. Aqueous solutions can be packaged for use as is, or lyophilized, the lyophilized preparation being combined with a sterile aqueous carrier prior to administration. The pH of the agent preparations typically will be between 3 and 11, more preferably from 5 to 9 and most preferably from 7 to 8. It will be understood that use of certain of the foregoing excipients, carriers, or stabilizers will result in the formation of pharmaceutical salts.

The therapeutic dosage of the agents can vary according to, for example, the particular use for which the treatment is made, the manner of administration of the compound, the health and condition of the patient, and the judgment of the prescribing physician. The proportion or concentration of an agent in a pharmaceutical composition can vary depending upon a number of factors including dosage, chemical characteristics *(e.g.,* hydrophobicity), and the route of administration. For example, the agents can be provided in an aqueous physiological buffer solution containing about 0.1 to about 10% w/v of the compound for parenteral administration. Some typical dose ranges are from about 1 µg/kg to about 1 g/kg of body weight per day. In some embodiments, the dose range is from about 0.01 mg/kg to about 100 mg/kg of body weight per day. The dosage is likely to depend on such variables as the type and extent of progression of the disease or disorder, the overall health status of the particular patient, the relative biological efficacy of the compound selected, formulation of the excipient, and its route of administration. Effective doses can be extrapolated from dose-response curves derived from *in vitro* or animal model test systems.

The compositions can further include one or more additional pharmaceutical agents, examples of which are listed hereinabove.

As used herein, the term "individual" or "patient," used interchangeably, refers to any animal, including mammals, preferably mice, rats, other rodents, rabbits, dogs, cats, swine, cattle, sheep, horses, or primates, and most preferably humans.

As used herein, the phrase "therapeutically effective amount" refers to the amount of active compound or pharmaceutical agent that elicits the biological or medicinal response in a tissue, system, animal, individual or human that is being sought by a researcher, veterinarian, medical doctor or other clinician, which includes one or more of the following:
(1) preventing the disease; for example, preventing a disease, condition or disorder in an individual who may be predisposed to the disease, condition or disorder but does not yet experience or display the pathology or symptomatology of the disease;
(2) inhibiting the disease; for example, inhibiting a disease, condition or disorder in an individual who is experiencing or displaying the pathology or symptomatology of the disease, condition or disorder *(i.e.,* arresting further development of the pathology and/or symptomatology), and
(3) ameliorating the disease; for example, ameliorating a disease, condition or disorder in an individual who is experiencing or displaying the pathology or symptomatology of the disease, condition or disorder (*i.e.,* reversing the pathology and/or symptomatology).

### JAK Inhibitors for Use as Agents in the Methods of the Invention

The next section details eleven different classes of agents for use in the methods of the invention. Some of the agents are defined generically in Formulas I-IX. The embodiments following each generic formula relate to the preceding generic formula.

In a first aspect, the agent is selected from a compound of Formula I: 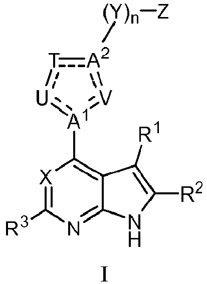 and pharmaceutically acceptable salt forms or prodrugs thereof, wherein:
A¹ and A² are independently selected from C and N;
T, U, and V are independently selected from O, S, N, CR⁵, and NR⁶;
wherein the 5-membered ring formed by A¹, A², U, T, and V is aromatic;
X is N or CR⁴;
Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR11RI2)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein the C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein the C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a} SR^{a} C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(-NRⁱ)NR^{c}R^{d} S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
wherein when Z is H, n is 1;
or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein the 4- to 20-membered aryl, cycloalkyl, heteroaryl, or heterocycloalkyl ring is optionally substituted by 1, 2, 3, 4, or 5 substituents independently selected from -(W)ₘ-Q;
W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c'}, NR^{c'}, NR^{c'}C(O)NR^{d'}, S(O), S(O)NR^{c'}, S(O)₂, or S(O)₂NR^{c'};
Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein the C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
R⁶ is H, C₁₋₄ alkyl, C₂₋₄ alkynyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁹ and R¹⁰ are independently selected from H, C₁₋₁₀ alkyl, C₁₋₆ haloalkyl, C₂₋₆ alkenyl, C₂₋₆ alkynyl, C₁₋₆ alkylcarbonyl, arylcarbonyl, C₁₋₆ alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
or R⁹ and R¹⁰ together with the N atom to which they are attached form a 4-, 5-, 6- or 7-membered heterocycloalkyl group;
R¹¹ and R¹² are independently selected from H and -E**¹**-E**²-**E**³-**E**⁴**;
D¹ and E¹ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D² and E² are independently absent or independently selected from C₁₋₆ alkylene, C₂₋₆ alkenylene, C₂₋₆ alkynylene, (C₁₋₆ alkylene)ᵣ-O-(C₁₋₆ alkylene)ₛ, (C₁₋₆ alkylene)ᵣ-S-(C₁₋₆ alkylene)ₛ, (C₁₋₆ alkylene)ᵣ-NR^{e}-(C₁₋₆ alkylene)ₛ, (C₁₋₆ alkylene)ᵣ-CO-(C₁₋₆ alkylene)ₛ, (C₁₋₆ alkylene)ᵣ-COO-(C₁₋₆ alkylene)ₛ, (C₁₋₆ alkylene)ᵣ-CONR^{e}-(C₁₋₆ alkylene)ₛ, (C₁₋₆ alkylene)ᵣ-SO-(C₁₋₆ alkylene)ₛ, (C₁₋₆ alkylene)ᵣ-SO₂-(C₁₋₆ alkylene)ₛ, (C₁₋₆ alkylene)ᵣSONR^{e}-(C₁₋₆ alkylene)ₛ, and (C₁₋₆ alkylene)ᵣ-NR^{e}CONR^{f}-(C₁₋₆ alkylene)ₛ, wherein each of the C₁₋₆ alkylene, C₂₋₆ alkenylene, and C₂₋₆ alkynylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂-₈ dialkylamino;
D³ and E³ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c} C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein the C₁₋₈ alkyl, C₂₋₈ alkenyl, or C₂₋₈ alkenyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
R^{a} is H, Cy¹, -(C₁₋₆ alkyl)-Cy¹, C₁₋₆ alkyl, C₁₋₆ haloalkyl, C₂₋₆ alkenyl, C₂₋₆ alkynyl, wherein the C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{b} is H, Cy¹, -(C₁₋₆ alkyl)-Cy¹, C₁₋₆ alkyl, C₁₋₆ haloalkyl, C₂₋₆ alkenyl, C₂₋₆ alkynyl, wherein the C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein the C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein the C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C1₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{c} and R^{d} are independently selected from H, Cy¹, -(C₁₋₆ alkyl)-Cy¹, C₁₋₁₀ alkyl, C₁₋₆ haloalkyl, C₂₋₆ alkenyl, C₂₋₆ alkynyl, wherein the C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆ alkyl, C₁₋₆ haloalkyl, C₁₋₆haloalkyl, and halosulfanyl;
or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆ alkyl)-Cy¹, OH, CN, amino, halo, C₋₆ alkyl, C₁₋₆ haloalkyl, C₁₋₆ haloalkyl, and halosulfanyl;
R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein the C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein the C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, C₁₋₆ haloalkyl, aryl, arylalkyl, heteroaryl, heteroarylalkyl, cycloalkyl and heterocycloalkyl;
or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
Rⁱ is H, CN, NO₂, or C₁₋₆ alkyl;
R^{e} and R^{f} are independently selected from H and C₁₋₆ alkyl;
Rⁱ is H, CN, or NO₂;
m is 0 or 1;
n is 0 or 1;
p is 0, 1, 2, 3, 4, 5, or 6;
q is 0, 1, 2, 3, 4, 5 or 6;
r is 0 or 1; and
s is 0 or 1.

In some embodiments, the compound of Formula I is not selected from:
4-[5-(2-isopropyl-5-methylcyclohexyloxymethyl)-1,2-4-oxadiazol-3-yl]-1H-pyrrolo[2,3-b]pyridine; 4-[5-(4-tert-butylphenoxymethyl)-1,2-4-oxadiazol-3-yl]-1H-pyrrolo[2,3-b]pyridine; 4-[5-cyclopentylethyl-1,2-4-oxadiazol-3-yl]-1H-pyrrolo[2,3-b]pyridine; 4-[5-(2,6-difluorophenyl)-1,2-4-oxadiazol-3-yl]-1H-pyrrolo[2,3-b]pyridine; 4-[5-(1-tert-butyl-3-methyl-1H-pyrazol-5-yl)-1,2-4-oxadiazol-3-yl]-1H-pyrrolo[2,3-b]pyridine; 4-[5-(benzyloxymethyl)-1,2-4-oxadiazol-3-yl]-1H-pyrrolo[2,3-b]pyridine; 4-[5-(3-fluorophenyl)-1,2-4-oxadiazol-3-yl]-1H-pyrrolo[2,3-b]pyridine; 4-[5-(phenoxymethyl)-1,2-4-oxadiazol-3-yl]-1H-pyrrolo[2,3-b]pyridine; 4-[5-(4-methoxybenzyl)-1,2-4-oxadiazol-3-yl]-1H-pyrrolo[2,3-b]pyridine; 4-[5-(phenylthiomethyl)-1,2-4-oxadiazol-3-yl]-1H-pyrrolo[2,3-b]pyridine; 4-[5-(3-methylbutyl)-1,2-4-oxadiazol-3-yl]-1H-pyrrolo[2,3-b]pyridine; 4-[5-benzyl-1,2-4-oxadiazol-3-yl]-1H-pyrrolo[2,3-b]pyridine; 4-[5-(2,2-dimethylpropyl)-1,2-4-oxadiazol-3-yl]-1H-pyrrolo[2,3-b]pyridine; 4-[5-methyl-1,2-4-oxadiazol-3-yl]-1H-pyrrolo[2,3-b]pyridine; 4-[5-(formyl)-1,2-4-oxadiazol-3-yl]-1H-pyrrolo[2,3-b]pyridine; 4-[5-(furan-2-yl)-1,2-4-oxadiazol-3-yl]-1H-pyrrolo[2,3-b]pyridine; 4-[5-(1-methyl-1H-pyrrol-2-yl)-1,2-4-oxadiazol-3-yl]-1H-pyrrolo[2,3-b]pyridine; 4-[5-(sec-butyl)-1,2-4-oxadiazol-3-yl]-1H-pyrrolo[2,3-b]pyridine; 4-[5-cyclopropyl-1,2-4-oxadiazol-3-yl]-1H-pyrrolo[2,3-b]pyridine; and pharmaceutically acceptable salts of any of the aforementioned.

In some embodiments, the moiety formed by T, U, V, A¹, and A², is not a 1,2,4-oxadiazol-3-yl ring. In some embodiments, the moiety formed by T, U, V, A¹, and A², is not an oxadiazole ring. wherein the moiety formed by T, U, V, A¹, and A² is not the following moiety:

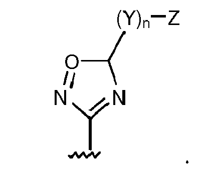

In some embodiments, when X is N, n is 1, and the moiety formed by A¹, A², U, T, V, and -(Y)ₙ-Z has the formula: 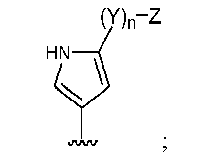 then Y is other than (CR¹¹R¹²)ₚC(O)NR^{c}(CR¹¹R¹²)_{q}.

In some embodiments, when X is N, the 5-membered ring formed by A¹, A², U, T, and V is other than pyrrolyl.

In some embodiments, when X is CH, n is 1, and the moiety formed by A¹, A², U, T, V, and -(Y)ₙ-Z has the formula: 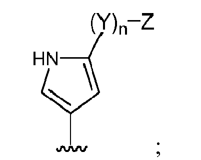 then -(Y)ₙ-Z is other than COOH.

In some embodiments, when X is CH or C-halo, R¹, R², and R³ are each H, n is 1, and the moiety formed by A¹, A², U, T, V, and -(Y)ₙ-Z has the formula: 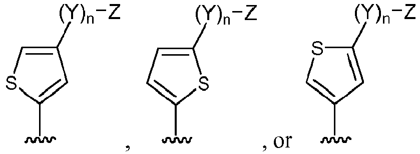 then Y is other than (CR¹¹R¹²)ₚC(O)NR^{c}(CR¹¹R¹²)_{q} or (CR¹¹R¹²)ₚC(O)(CR¹¹R¹²)_{q}.

In some embodiments, when X is CH or C-halo, R¹, R², and R³ are each H, n is 0, and the moiety formed by A¹, A², U, T, V, and -(Y)ₙ-Z has the formula: 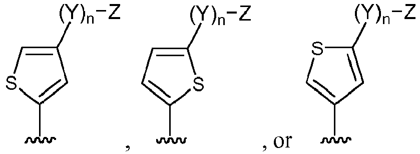 then Z is other than CN, halo, or C₁₋₄ alkyl.

In some embodiments, when X is CH or C-halo, R¹, R², and R³ are each H, n is 1, and the moiety formed by A¹, A², U, T, V, and -(Y)ₙ-Z has the formula: 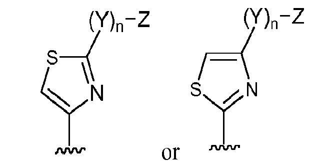 then Y is other than (CR¹¹R¹²)ₚC(O)NR^{c}(CR¹¹R¹²)_{q} or (CR¹¹R¹²)ₚC(O)(CR¹¹R¹²)_{q.}

In some embodiments, when X is CH or C-halo, R¹, R², and R³ arc each H, n is 1, and the moiety formed by A¹, A², U, T, V, and -(Y)ₙ-Z has the formula: 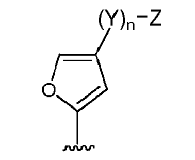 then Y is other than (CR¹¹R¹²)ₚNR^{c}(CR¹¹R¹²)_{q}.

In some embodiments, when X is CH or C-halo and R¹, R², and R³ are each H, then the moiety formed by A¹, A², U, T, V, and -(Y)ₙ-Z has a formula other than:

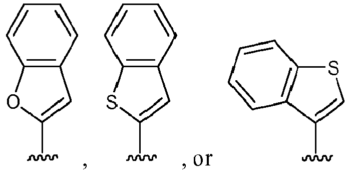

In some embodiments:
Z is H, halo, CN, NO₂, C₁₋₈ alkyl, C₂₋₈ alkenyl, C₂₋₈ alkynyl, C₁₋₈ haloalkyl, aryl, cycloalkyl, heteroaryl, or heterocycloalkyl, wherein the C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d};
Q is H, halo, CN, NO₂, C₁₋₈ alkyl, C₂₋₈ alkenyl, C₂₋₈ alkynyl, C₁₋₈ haloalkyl, aryl, cycloalkyl, heteroaryl, or heterocycloalkyl, wherein the C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, C₁₋₄ hydroxy-alkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, CC(C)NR^{c'}R^{d'}, NR^{c'}R^{d'} , NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
R¹, R², R³, and R⁴ are independently selected from H, halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
R⁵ is H, halo, C₁₋₄ alkyl, C₂₋₄ alkenyl, C₂₋₄ alkynyl, C₁₋₄ haloalkyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁹ and R¹⁰ are independently selected from H, C₁₋₁₀ alkyl, C₁₋₆ haloalkyl, C₂₋₆ alkenyl, C₂₋₆ alkynyl, C₁₋₆ alkylcarbonyl, arylcarbonyl, C₁₋₆ alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
or R⁹ and R¹⁰ together with the N atom to which they are attached form a 4-, 5-, 6- or 7-membered heterocycloalkyl group;
R¹¹ and R¹² are independently selected from H, halo, OH, CN, C₁₋₄ alkyl, C₁₋₄ haloalkyl, C₂₋₄ alkenyl, C₂₋₄ alkynyl, C₁₋₄ hydroxyalkyl, C₁₋₄ cyanoalkyl, aryl, heteroaryl, cycloalkyl, and heterocycloalkyl;
R^{a}, R^{a'}, and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein the C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, aryl, arylalkyl, heteroaryl, heteroarylalkyl, cycloalkyl and heterocycloalkyl;
R^{b}, R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein the C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl;
R^{c} and R^{d} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein the C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl or heterocycloalkyl;
or R^{c} and R^{d} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl;
R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein the C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl;
or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl;
R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein the C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl; and
or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, aryl, arylalkyl, heteroaryl, heteroarylalkyl, cycloalkyl and heterocycloalkyl.

In some embodiments, X is N.

In some embodiments, X is CR⁴.

In some embodiments, X is N or CR⁴.

In some embodiments, A¹ is C.

In some embodiments, A¹ is N.

In some embodiments, A² is C.

In some embodiments, A² is N.

In some embodiments, at least one of A¹, A², U, T, and V is N.

In some embodiments, the 5-membered ring formed by A¹, A², U, T, and V is pyrrolyl, pyrazolyl, imidazolyl, oxazolyl, thiazolyl, or oxadiazolyl.

In some embodiments, the 5-membered ring formed by A¹, A², U, T, and V is selected from: 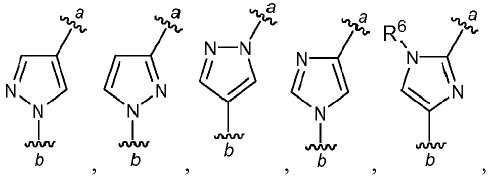 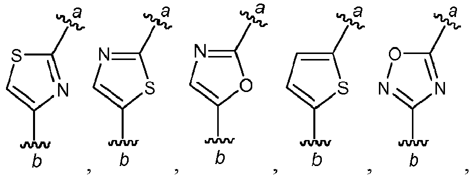 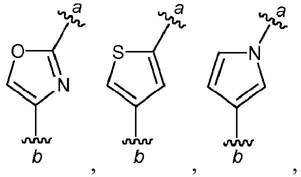 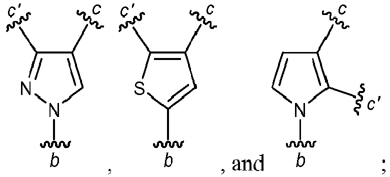 wherein:
*a* designates the site of attachment of moiety -(Y)ₙ-Z;
*b* designates the site of attachment to the core moiety: 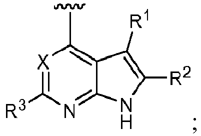 and
c and *c'* designate the two sites of attachment of the fused 4- to 20-membered aryl, cycloalkyl, heteroaryl, or heterocycloalkyl ring.

In some embodiments, the 5-membered ring formed by A¹, A², U, T, and V is selected from: 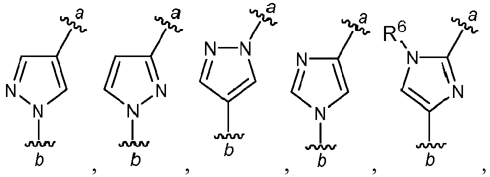 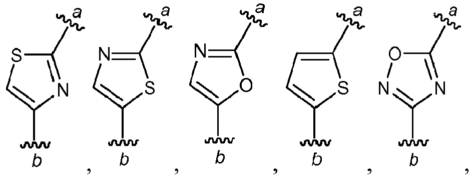 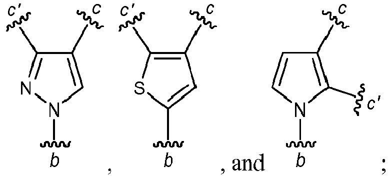 wherein:
*a* designates the site of attachment of moiety -(Y)ₙ-Z;
*b* designates the site of attachment to the core moiety: 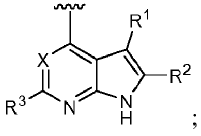 and
*c* and *c'* designate the two sites of attachment of the fused 4- to 20-membered aryl, cycloalkyl, heteroaryl, or heterocycloalkyl ring.

In some embodiments, the 5-membered ring formed by A¹, A², U, T, and V is selected from: 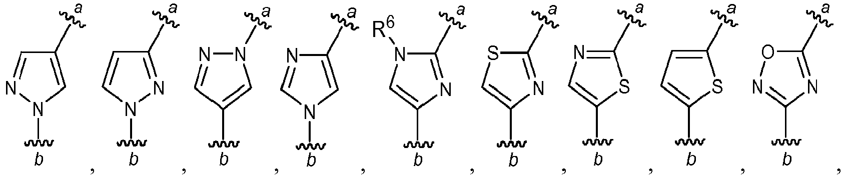 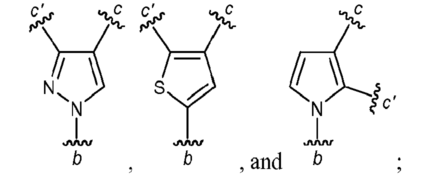 wherein:
*a* designates the site of attachment of moiety -(Y)ₙ-Z;
*b* designates the site of attachment to the core moiety: 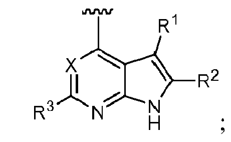 and
*c* and *c'* designate the two sites of attachment of the fused 4- to 20-membered aryl, cycloalkyl, heteroaryl, or heterocycloalkyl ring.

In some embodiments, the 5-membered ring formed by A¹, A², U, T, and V is selected from: 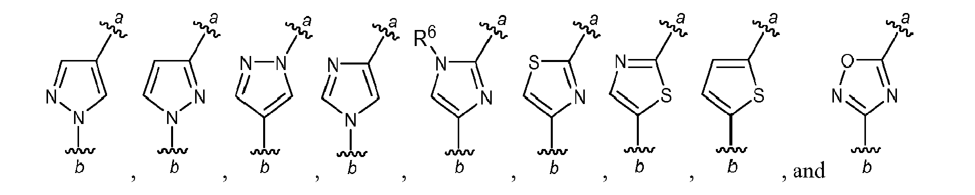 wherein:
*a* designates the site of attachment of moiety -(Y)ₙ-Z;
*b* designates the site of attachment to the core moiety:

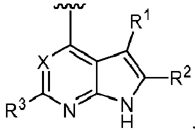

In some embodiments, the 5-membered ring formed by A¹, A², U, T, and V is selected from: 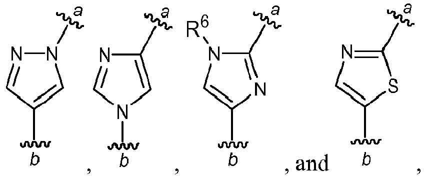 wherein:
*a* designates the site of attachment of moiety -(Y)ₙ-Z;
*b* designates the site of attachment to the core moiety:

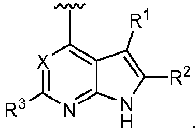

In some embodiments, the 5-membered ring formed by A¹, A², U, T, and V is selected from: 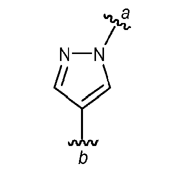 wherein:
*a* designates the site of attachment of moiety -(Y)ₙ-Z;
*b* designates the site of attachment to the core moiety:

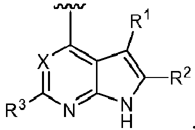

In some embodiments, the 5-membered ring formed by A¹, A², U, T, and V is selected from:

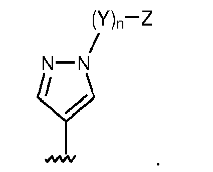

In some embodiments, n is 0.

In some embodiments, n is 1.

In some embodiments, n is 1 and Y is C₁₋₈ alkylene, C₂₋₈ alkenylene, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, wherein the C₁₋₈ alkylene or C₂₋₈ alkenylene, is optionally substituted with 1, 2, or 3 halo, OH, CN, amino, C₁₋₄ alkylamino, or C₂₋₈ dialkylamino.

In some embodiments, n is 1 and Y is C₁₋₈ alkylene, (CR¹¹R¹²)ₚC(O)(CR¹¹R¹²)_{q}, (CR¹¹R¹²)ₚC(O)(CR¹¹R¹²)_{q}, (CR¹¹R¹²)ₚC(O)O(CR¹¹R¹²)_{q}, wherein the C₁₋₈ alkylene is optionally substituted with 1, 2, or 3 halo, OH, CN, amino, C₁₋₄ alkylamino, or C₂₋₈ dialkylamino.

In some embodiments, n is 1 and Y is C₁₋₈ alkylene optionally substituted with 1, 2, or 3 halo, OH, CN, amino, C₁₋₄ alkylamino, or C₂₋₈ dialkylamino.

In some embodiments, n is 1 and Y is ethylene optionally substituted with 1, 2, or 3 halo, OH, CN, amino, C₁₋₄ alkylamino, or C₂₋₈ dialkylamino.

In some embodiments, n is 1 and Y is (CR¹¹R¹²)ₚC(O)(CR¹¹R¹²)_{q}, (CR¹¹R¹²)ₚC(O)NR^{c}-(CR¹¹R¹²)_{q}, or (CR¹¹R¹²)ₚC(O)O(CR¹¹R¹²)_{q}.

In some embodiments, Y is C₁₋₈ alkylene, C₂₋₈ alkenylene, C₂₋₈ alkynylene, (CR¹¹R¹²)ₚ-(C₃₋₁₀ cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀ heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, or (CR¹¹R¹²)ₚS(CR¹¹R¹²)_{q}, wherein the C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴.

In some embodiments, Y is C₁₋₈ alkylene, C₂₋₈ alkenylene, C₂₋₈ alkynylene, (CR¹¹R¹²)ₚ-(C₃₋₁₀ cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀ heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, or (CR¹¹R¹²)ₚS(CR¹¹R¹²)_{q}, wherein the C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from D⁴.

In some embodiments, Y is C₁₋₈ alkylene, C₂₋₈ alkenylene, C₂₋₈ alkynylene, or (CR¹¹R¹²)ₚ-(C₃-₁₀ cycloalkylene)-(CR¹¹R¹²)_{q}, wherein the C₁₋₈ alkylene, C₂₋₈ alkenylene, C₂₋₈ alkynylene, or cycloalkylene, is optionally substituted with 1, 2, or 3 substituents independently selected from -D¹-D²-_{D}³_{-D}⁴.

In some embodiments, Y is C₁₋₈ alkylene, C₂₋₈ alkenylene, C₂₋₈ alkynylene, or (CR¹¹R¹²)ₚ-(C₃₋₁₀ cycloalkylene)-(CR¹¹R¹²)_{q}, wherein the C₁₋₈ alkylene, C₂₋₈ alkenylene, C₂₋₈ alkynylene, or cycloalkylene, is optionally substituted with 1, 2, or 3 substituents independently selected from D⁴.

In some embodiments, Y is C₁₋₈ alkylene, C₂₋₈ alkenylene, or C₂₋₈ alkynylene, each optionally substituted with 1, 2, or 3 substituents independently selected from -D¹- D²-D³-D⁴.

In some embodiments, Y is C₁₋₈ alkylene optionally substituted with 1, 2, or 3 substituents independently selected from-D¹-D²-D³-D⁴.

In some embodiments, Y is C₁₋₈ alkylene optionally substituted with 1, 2, or 3 substituents independently selected from D⁴.

In some embodiments, Y is C₁₋₈ alkylene, C₂₋₈ alkenylene, C₂₋₈ alkynylene, (CR¹¹R¹²)ₚO-(CR¹¹R¹²)_{q}, (CR¹¹R¹²)ₚS(CR¹¹R¹²)_{q}, (CR¹¹R¹²)ₚC(O)(CR¹¹R¹²)q, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q,} wherein the C₁₋₈ alkylene, C₂₋₈ alkenylene, C₂₋₈ alkynylene is optionally substituted with 1, 2, or 3 substituents independently selected from halo, OH, CN, amino, C₁₋₄ alkylamino, and C₂₋₈ dialkylamino.

In some embodiments, Y is C₁₋₈ alkylene, C₂₋₈ alkenylene, C₂₋₈ alkynylene, (CR¹¹R¹²)ₚ-(C₃₋₁₀ cycloalkylene)-(CR¹¹R¹²)_{q}, (CR¹¹R¹²)ₚ-(arylene)-(CR¹¹R¹²)_{q}, (CR¹¹R¹²)_{q}-(C₁₋₁₀ heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NRCR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂NR^{d}(CR¹¹R¹²)_{q}, or (CR¹¹R¹²)ₚS(O)₂NR^{c}(CR¹¹R¹²)_{q}, wherein the C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from halo, OH, CN, amino, C₁₋₄ alkylamino, and C₂₋₈ dialkylamino.

In some embodiments, n is 1 and Y is C₁₋₈ alkylene optionally substituted with 1, 2, or 3 substituents independently selected from D⁴. In some embodiments, n is 1 and Y is C₁₋₈ alkylene optionally substituted with 1, 2, or 3 halo, OH, CN, amino, C₁₋₄ alkylamino, or C₂₋₈ dialkylamino. In some embodiments, n is 1 and Y is C₁₋₈ alkylene optionally substituted with cyano.

In some embodiments, p is 0.

In some embodiments, p is 1.

In some embodiments, p is 2.

In some embodiments, q is 0.

In some embodiments, q is 1.

In some embodiments, q is 2.

In some embodiments, one of p and q is 0 and the other of p and q is 1, 2, or 3.

In some embodiments, Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d} wherein the C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d}.

In some embodiments, Z is aryl, cycloalkyl, heteroaryl, or heterocycloalkyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl, cycloalkyl, heteroaryl, or heterocycloalkyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl or heteroaryl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl or heteroaryl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a} C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl or 5- or 6-membered heteroaryl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl or 5- or 6-membered heteroaryl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is cycloalkyl or heterocycloalkyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is cycloalkyl or heterocycloalkyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, CCO)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is cyclopropyl, cyclobutyl, cyclopentyl, cyclohexyl, or cycloheptyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, or C₂₋₈ alkynyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, or C₂₋₈ alkynyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl, cycloalkyl, heteroaryl, or heterocycloalkyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, CCO)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl, cycloalkyl, heteroaryl, or heterocycloalkyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, CCO)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl or heteroaryl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, CCO)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is aryl or heteroaryl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl or 5- or 6-membered heteroaryl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl or 5- or 6-membered heteroaryl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is phenyl optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is cycloalkyl or heterocycloalkyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is cycloalkyl or heterocycloalkyl, each optionally substituted with 1, 2, 3, 4, 5, or 6 substituents independently selected from halo, C₁₋₄ alkyl, C₂₋₄ alkynyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, or C₂₋₈ alkynyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, CCO)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, or C₂₋₈ alkynyl, each optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, C₂₋₈ alkynyl, aryl, cycloalkyl, heteroaryl, or heterocycloalkyl, each optionally substituted with 1, 2, 3, 4, 5, or 6 substituents independently selected from halo, C₁₋₄ alkyl, C₁₋₄ haloalkyl, halosulfanyl, C₁₋₄ hydroxyalkyl, C₁₋₄ cyanoalkyl, Cy¹, CN, NO₂, OR^{a}, C(O)NR^{c}R^{d}, C(O)OR^{a}, NR^{c}R^{d}, NR^{c}C(O)R^{b}, and S(O)₂R^{b}.

In some embodiments, Z is C₁₋₈ alkyl, C₂₋₈ alkenyl, C₂₋₈ alkynyl, aryl, cycloalkyl, heteroaryl, or heterocycloalkyl, each optionally substituted with 1, 2, 3, 4, 5, or 6 substituents independently selected from halo, C₁₋₄ alkyl, C₁₋₄ haloalkyl, C₁₋₄ hydroxyalkyl, C₁₋₄ cyanoalkyl, Cy¹, CN, NO₂, OR^{a}, C(O)NR^{c}R^{d}, C(O)OR^{a}, NR^{c}R^{d}, NR^{c}C(O)R^{b}, and S(O)₂R^{b}.

In some embodiments, Z is C₁₋₈ alkyl, C₂₋₈ alkenyl, C₂₋₈ alkynyl, aryl, cycloalkyl, heteroaryl, or heterocycloalkyl, each optionally substituted with 1, 2, or 3 substituents independently selected from halo, C₁₋₄ alkyl, C₁₋₄ haloalkyl, halosulfanyl, C₁₋₄ hydroxyalkyl, C₁₋₄ cyanoalkyl, Cy¹, CN, NO₂, OR^{a}, C(O)NR^{c}R^{d}, C(O)OR^{a}, NR^{c}R^{d}, NR^{c}C(O)R^{b}, and S(O)₂R^{b}.

In some embodiments, Z is C₁₋₈ alkyl, C₂₋₈ alkenyl, C₂₋₈ alkynyl, aryl, cycloalkyl, heteroaryl, or heterocycloalkyl, each optionally substituted with 1, 2, or 3 substituents independently selected from halo, C₁₋₄ alkyl, C₁₋₄ haloalkyl, C₁₋₄ hydroxyalkyl, C₁₋₄ cyanoalkyl, Cy¹, CN, NO₂, OR^{a}, C(O)NR^{c}R^{d}, C(O)OR^{a}, NR^{c}R^{d}, NR^{c}C(O)R^{b}, and S(O)₂R^{b}.

In some embodiments, Z is substituted with at least one substituent comprising at least one CN group.

In some embodiments, Z is C₁₋₈ alkyl, C₂₋₈ alkenyl, C₂₋₈ alkynyl, aryl, cycloalkyl, heteroaryl, or heterocycloalkyl, each substituted with at least one CN or C₁₋₄ cyanoalkyl and optionally substituted with 1, 2, 3, 4, or 5 further substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, CCO)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is C₁₋₈ alkyl, C₂₋₈ alkenyl, C₂₋₈ alkynyl, aryl, cycloalkyl, heteroaryl, or heterocycloalkyl, each substituted with at least one CN or C₁₋₄ cyanoalkyl and optionally substituted with 1, 2, 3, 4, or 5 further substituents selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, S(O)R^{b}, S(O)NR^{c}R^{d}, S(O)₂R^{b}, NR^{c}S(O)₂R^{b}, and S(O)₂NR^{c}R^{d}.

In some embodiments, Z is cyclopentyl, which is optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}. In some embodiments, Z is cyclopentyl.

In some embodiments, X is N. In some embodiments, A¹ is C. In some embodiments, As is N. In some embodiments, T is N. In some embodiments, U and V are independently CR⁵.

In some embodiments, wherein the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein the 4- to 20-membered aryl, cycloalkyl, heteroaryl, or hctcrocycloalkyl ring is optionally substituted by 1, 2, 3, 4, or 5 substituents independently selected from -(W)ₘ-Q.

In some embodiments, wherein the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 8-membered aryl, cycloalkyl, heteroaryl, or heterocycloalkyl ring fused to the 5-membered ring formed by A¹, A², U, T, and V, wherein the 4- to 8-membered aryl, cycloalkyl, heteroaryl, or heterocycloalkyl ring is optionally substituted by 1, 2, 3, 4, or 5 substituents independently selected from -(W)ₘ-Q.

In some embodiments, the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 6-membered aryl, cycloalkyl, heteroaryl, or heterocycloalkyl ring fused to the 5-membered ring formed by A¹, A², U, T, and V, wherein the 6-membered aryl, cycloalkyl, heteroaryl, or heterocycloalkyl ring is optionally substituted by 1, 2, or 3 substituents independently selected from halo, CN, NO₂, C₁₋₈ alkyl, C₂₋₈ alkenyl, C₂₋₈ alkynyl, C₁₋₈ haloalkyl, aryl, cycloalkyl, heteroaryl, or heterocycloalkyl wherein the C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted by 1, 2 or 3 CN.

In some embodiments, Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, S(O)R^{b"}, S(O)NR^{c"}R^{d"}, S(O)₂R^{b"}, and S(O)₂NR^{c"}R^{d"}.

In some embodiments, Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, S(O)R^{b"}, S(O)NR^{c"}R^{d"}, S(O)₂R^{b"}, and S(O)₂NR^{c"}R^{d"}.

In some embodiments, Cy¹ and Cy² are independently selected from cycloalkyl and heterocycloalkyl, each optionally substituted by 1, 2, 3, 4 or 5 substituents independently selected from halo, C₁₋₄ alkyl, C₁₋₄ alkenyl, C₂₋₄ alkynyl, C₁₋₄ haloalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, S(O)R^{b"}, S(O)NR^{c"}R^{d"}, S(O)₂R^{b"}, and S(O)₂NR^{c"}R^{d"}.

In some embodiments, Cy¹ and Cy² are independently selected from cycloalkyl optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, S(O)R^{b"}, S(O)NR^{c"}R^{d"}, S(O)₂R^{b"}, and S(O)₂NR^{c"}R^{d"}.

In some embodiments, R¹, R², R³, and R⁴ arc independently selected from H, halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰.

In some embodiments, R¹, R², R³, and R⁴ are independently selected from H, halo, and C₁₋₄ alkyl.

In some embodiments, R¹, R², R³, and R⁴ are each H.

In some embodiments, R¹ is H, halo, or C₁₋₄ alkyl.

In some embodiments, R⁵ is H, halo, C₁₋₄ alkyl, C₂₋₄ alkenyl, C₂₋₄ alkynyl, C₁₋₄ haloalkyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰.

In some embodiments, R⁵ is H, halo, C₁₋₄ alkyl, C₁₋₄ haloalkyl, halosulfanyl, CN, or NR⁹R¹⁰.

In some embodiments, R⁵ is H, halo, C₁₋₄ alkyl, C₁₋₄ haloalkyl, CN, or NR⁹R¹⁰.

In some embodiments, R⁵ is H.

In some embodiments, R⁶ is H or C₁₋₄ alkyl.

In some embodiments, R⁶ is H.

In some embodiments, R¹¹ and R¹² are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein the C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d}.

In some embodiments, R¹¹ and R¹² are independently selected from H, halo, OH, CN, C₁₋₄ alkyl, C₁₋₄ haloalkyl, halosulfanyl, SCN, C₂₋₄ alkenyl, C₂₋₄ alkynyl, C₁₋₄ hydroxyalkyl, C₁₋₄ cyanoalkyl, aryl, heteroaryl, cycloalkyl, and heterocycloalkyl.

In some embodiments, R¹¹ and R¹² are independently selected from H, halo, OH, CN, C₁₋₄ alkyl, C₁₋₄ haloalkyl, C₂₋₄ alkenyl, C₂₋₄ alkynyl, C₁₋₄ hydroxyalkyl, C₁₋₄ cyanoalkyl, aryl, heteroaryl, cycloalkyl, and heterocycloalkyl.

In some embodiments, the agent is selected from compounds of Formula Ia or Ib: 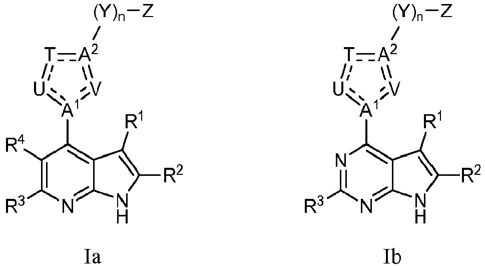 and pharmaceutically acceptable salts thereof.

In some embodiments, the agent is selected from compounds of Formula Ic: 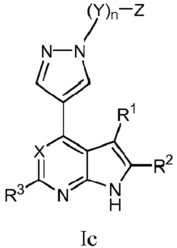 and pharmaceutically acceptable salts thereof.

In some embodiments, the agent is selected from compounds of Formula Id or Ie: 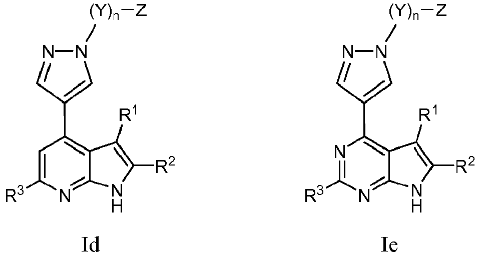 and pharmaceutically acceptable salts thereof.

In some embodiments, the agent is selected from compounds of Formula Ie: 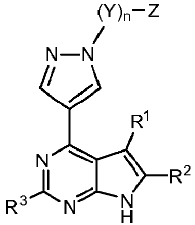 and pharmaceutically acceptable salts thereof.

In some embodiments, the agent is selected from compounds of Formula Ih: 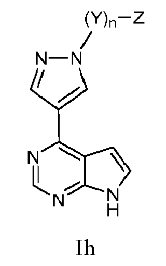 and pharmaceutically acceptable salts thereof.

In some embodiments, the agent is selected from compounds of Formula Ik: 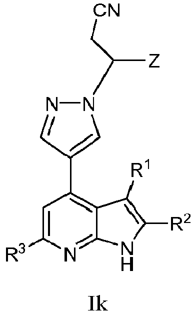 and pharmaceutically acceptable salts thereof.

In some embodiments, the agent is selected from compounds of Formula Il: 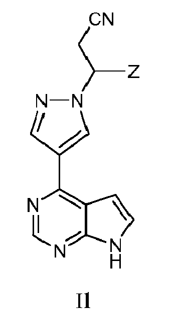 and pharmaceutically acceptable salts thereof.

In some embodiments, the agent is selected from compounds of Formula Im: 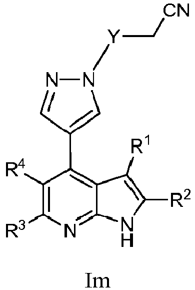 and pharmaceutically acceptable salts thereof.

In some embodiments, the agent is selected from compounds of Formula In: 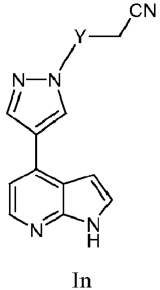 and pharmaceutically acceptable salts thereof.

In some embodiments, the agent is selected from:
3-[3-methyl-1-(1H-pyrrolo[2,3-b]pyridin-4-yl)-1H-pyrazol-4-yl]benzonitrile;
3-[3-methyl-1-(1H-pyrrolo[2,3-b]pyridin-4-yl)-1H-pyrazol-4-yl]acrylonitrile;
3-[3-methyl-1-(1H-pyrrolo[2,3-b]pyridin-4-yl)-1H-pyrazol-4-yl]propanenitrile;
4-(4-phenyl-1H-imidazol-1-yl)-1H-pyrrolo[2,3-b]pyridine;
[3-methyl-1-(1H-pyrrolo[2,3-b]pyridin-4-yl)-1H-pyrazol-4-yl]-piperidin-1-yl-methanone;
[3-methyl-1-(1H-pyrrolo[2,3-b]pyridin-4-yl)-1H-pyrazol-4-ylmethyl]-phenyl-amine;
3-[3-methyl-1-(1H-pyrrolo[2,3-b]pyridin-4-yl)-1H-pyrazol-4-yl]-cyclohexanol;
4-[1-(3-methoxy-1-methyl-propyl)-1H-pyrazol-4-yl]-1H-pyrrolo[2,3-b]pyridine;
4-[1-(1-methyl-3-pyrazol-1-yl-propyl)-1H-pyrazol-4-yl]-1H-pyrrolo[2,3-b]pyridine;
1-(1H-pyrrolo[2,3-b]pyridin-4-yl)-1H-pyrazole-4-carboxylic acid ethyl ester;
4-(3-methyl-4-phenyl-pyrazol-1-yl)-1H-pyrrolo[2,3-b]pyridine;
4-(3-phenyl-pyrazol-1-yl)-1H-pyrrolo[2,3-b]pyridine;
4-(4-bromo-imidazol-1-yl)-1H-pyrrolo[2,3-b]pyridine;
4-(4-bromo-3-methyl-pyrazol-1-yl)-1H-pyrrolo[2,3-b]pyridine;
3-[3-methyl-1-(1H-pyrrolo[2,3-b]pyridin-4-yl)-1H-pyrazol-4-yl]-benzonitrile;
4-[3-methyl-1-(1H-pyrrolo[2,3-b]pyridin-4-yl)-1H-pyrazol-4-yl]-benzonitrile;
4-[4-(3-fluoro-phenyl)-3-methyl-pyrazol-1-yl]-1H-pyrrolo[2,3-b]pyridine;
4-[4-(3,5-bis-trifluoromethyl-phenyl)-3-methyl-pyrazol-1-yl]-1H-pyrrolo[2,3-b]pyridine;
4-[4-(3,5-difluoro-phenyl)-3-methyl-pyrazol-1-yl]-1H-pyrrolo[2,3-b]pyridine;
{3-[3-methyl-1-(1H-pyrrolo[2,3-b]pyridin-4-yl)-1H-pyrazol-4-yl]-phenyl}-methanol;
4-(3-methyl-4-pyrimidin-5-yl-pyrazol-1-yl)-1H-pyrrolo[2,3-b]pyridine;
4-[3-methyl-4-(1-methyl-1H-mdol-5-yl)-pyrazol-1-yl]-1H-pyrrolo[2,3-b]pyridine;
4-(3-methyl-4-thiophen-3-yl-pyrazol-1-yl)-1H-pyrrolo[2,3-b]pyridine;
N,N-dimethyl-4-[3-methyl-1-(1H-pyrrolo[2,3-b]pyridin-4-yl)-1H-pyrazol-4-yl]-benzene-sulfonamide:
N-{4-[3-methyl-1-(1H-pyrrolo[2,3-b]pyridin-4-yl)-1H-pyrazol-4-yl]-phenyl}-acetamide;
3-*tert*-butyl-1-(1H-pyrrolo[2,3-b]pyridin-4-yl)-1H-pyrazole-4-carbonitrile;
4-bromo-1-(1H-pyrrolo[2,3-b]pyridin-4-yl)-1H-pyrazole-3-carbonitrile;
4-(3-cyano-phenyl)-1-(1H-pyrrolo[2,3-b]pyridin-4-yl)-1H-pyrazole-3-carbonitrile;
3-[1-(1H-pyrrolo[2,3-b]pyridin-4-yl)-3-trifluoromethyl-1H-pyrazol-4-yl]-propan-1-ol;
3-[3-methyl-1-(1H-pyrrolo[2,3-b]pyridin-4-yl)-1H-pyrazol-4-yl]-prop-2-en-1-ol;
2-[4-bromo-1-(1H-pyrrolo[2,3-b]pyridin-4-yl)-1H-pyrazol-3-yl]-isoindole-1,3-dione;
4-[4-(2,6-dimethyl-phenyl)-3-methyl-pyrazol-1-yl]-1H-pyrrolo[2,3-b]pyridine;
3-[3-amino-1-(1H-pyrrolo[2,3-b]pyridin-4-yl)-1H-pyrazol-4-yl]-benzonitrile;
3-[3-benzylamino-1-(1H-pyrrolo[2,3-b]pyridin-4-yl)-1H-pyrazol-4-yl]-benzonitrile;
N-[4-(3-cyano-phenyl)-1-(1H-pyrrolo[2,3-b]pyridin-4-yl)-1H-pyrazol-3-yl]-acetamide;
3-[4-(1H-pyrrolo[2,3-b]pyridin-4-yl)-pyrazol-1-yl]-propan-1-ol;
3-[4-(1H-pyrrolo[2,3-b]pyridin-4-yl)-pyrazol-1-yl]-butan-1-ol;
4-[4-(1H-pyrrolo[2,3-b]pyridin-4-yl)-pyrazol-1-yl]-pentanenitrile;
4-[4-(1H-pyrrolo[2,3-b]pyridin-4-yl)-pyrazol-1-yl]-pentanoic acid amide;
4-[1-(3-imidazol-1-yl-1-methyl-propyl)-1H-pyrazol-4-yl]-1H-pyrrolo[2,3-b]pyridine;
4-cyclopentyl-4-[4-(1H-pyrrolo[2,3-b]pyridin-4-yl)-pyrazol-1-yl]-butyronitrile;
4-cyclopentyl-4-[4-(1H-pyrrolo[2,3-b]pyridin-4-yl)-pyrazol-1-yl]-butyramide;
3-cyclopropyl-3-[4-(7H-pyrrolo[2,3-d]pyrimidin-4-yl)-pyrazol-1-yl]-propionitrile;
4-(2-tert-butyl-1-methyl-1H-imidazol-4-yl)-1H-pyrrolo[2,3-b]pyridine;
4-(2-phenyl-1H-imidazol-5-yl)-1H-pyrrolo[2,3-b]pyridine;
4-(2-benzyl-1H-imidazol-5-yl)-1H-pyrrolo[2,3-b]pyridine;
4-[2-(1-phenylethyl)-1H-imidazol-5-yl]-1H-pyrrolo[2,3-b]pyridine;
4-(2-phenyl-1,3-thiazol-4-yl)-1H-pyrrolo[2,3-b]pyridine;
N-methyl-N-propyl-4-(1H-pyrrolo[2,3-b]pyridin-4-yl)-1,3-thiazol-2-amine;
N-phenyl-4-(1H-pyrrolo[2,3-b]pyridin-4-yl)-1,3-thiazol-2-amine;
N-methyl-N-phenyl-4-(1H-pyrrolo[2,3-b]pyridin-4-yl)-1,3-thiazol-2-amine;
4-(2-phenyl-1,3-thiazol-5-yl)-1H-pyrrolo[2,3-b]pyridine;
ethyl 2-methyl-2-[4-(1H-pyrrolo[2,3-b]pyridin-4-yl)-1H-pyrazol-1-yl]propanoate;
2-methyl-2-[4-(1H-pyrrolo[2,3-b]pyridin-4-yl)-1H-pyrazol-1-yl]propanoic acid;
2-methyl-2-[4-(1H-pyrrolo[2,3-b]pyridin-4-yl)-1H-pyrazol-1-yl]propanamide;
ethyl 3-methyl-3-[4-(1H-pyrrolo[2,3-b]pyridin-4-yl)-1H-pyrazol-1-yl]butanoate;
3-methyl-3-[4-(1H-pyrrolo[2,3-b]pyridin-4-yl)-1H-pyrazol-1-yl]butan-1-ol;
4-methyl-4-[4-(1H-pyrrolo[2,3-b]pyridin-4-yl)-1H-pyrazol-1-yl]pentanenitrile;
4-methyl-4-[4-(1H-pyrrolo[2,3-b]pyridin-4-yl)-1H-pyrazol-1-yl]pentanamide;
3-[4-(1H-pyrrolo[2,3-b]pyridin-4-yl)-1H-pyrazol-1-yl]butanemtrile;
3-[4-(1H-pyrrolo[2,3-b]pyridin-4-yl)-1H-pyrazol-1-yl]propanenitrile;
3-[4-(1H-pyrrolo[2,3-b]pyridin-4-yl)-1H-pyrazol-1-yl]hexanenitrile;
3-cyclopentyl-3-[4-(1H-pyrrolo[2,3-b]pyridin-4-yl)-1H-pyrazol-1-yl]propanenitrile;
3-cyclohexyl-3-[4-(1H-pyrrolo[2,3-b]pyridin-4-yl)-1H-pyrazol-1-yl]propanenitrile;
3-[4-(7H-pyrrolo[2,3-d]pyrimidin-4-yl)-1H-pyrazol-1-yl]butanenitrile;
3-cyclopentyl-3-[4-(7H-pyrrolo[2,3-d]pyrimidin-4-yl)-1H-pyrazol-1-yl]propanenitrile;
2-methyl-3-[4-(7H-pyrrolo[2,3-d]pyrimidin-4-yl)-1H-pyrazol-1-yl]propanenitrile;
3-[4-(7H-pyrrolo[2,3-d]pyrimidin-4-yl)-1H-pyrazol-1-yl]pentanenitrile;
5-methyl-3-[4-(7H-pyrrolo[2,3-d]pyrimidin-4-yl)-1H-pyrazol-1-yl]hexanenitrile;
3-cyclohexyl-3-[4-(7H-pyrrolo[2,3-d]pyrimidin-4-yl)-1H-pyrazol-1-yl]propanenitrile;
4-cyclopropyl-3-[4-(7H-pyrrolo[2,3-d]pyrimidin-4-yl)-1H-pyrazol-1-yl]butanenitrile;
4-{1-[1-methylbutyl]-1H-pyrazol-4-yl}-7H-pyrrolo[2,3-d]pyrimidine;
4-methyl-4-[4-(7H-pyrrolo[2,3-d]pyrimidin-4-yl)-1H-pyrazol-1-yl]pentanenitrile;
3-1-[4-(7H-pyrrolo[2,3-d]pyrimidin-4-yl)-1H-pyrazol-1-yl]cyclopropylpropanenitrile;
4-[4-(7H-pyrrolo[2,3-d]pyrimidin-4-yl)-1H-pyrazol-1-yl]pentanenitrile;
3-methyl-3-[4-(7H-pyrrolo[2,3-d]pyrimidin-4-yl)-1H-pyrazol-1-yl]butanenitrile;
3-ethyl-3-[4-(7H-pyrrolo[2,3-d]pyrimidin-4-yl)-1H-pyrazol-1-yl]pentallenitrile;
1-[4-(7H-pyrrolo[2,3-d]pyrimidin-4-yl)-1H-pyrazol-1-yl]cyclopropylacetonitrile;
4-1-[(pyrrolidin-2-ylmethyl]-1H-pyrazol-4-yl-7H-pyrrolo[2,3-d]pyrimidine;
4-(1-[1-(methylsulfonyl)pyrrolidin-2-yl]methyl-1H-pyrazol-4-72yl)-7H-pyrrolo[2,3-d]-pyrimidine;
ethyl 2-methyl-2-[4-(7H-pyrrolo[2,3-d]pyrimidin-4-yl)-1H-pyrazol-1-yl]propanoate;
3-cyclopentyl-3-[4-(7H-pyrrolo[2,3-d]pyrimidin-4-yl)-1H-pyrazol-1-yl]acrylonitrile;
3-cyclopentylidene-3-[4-(7H-pyrrolo[2,3-d]pyrimidin-4-yl)-1H-pyrazol-1-yl]propanenitrile;
3-(methyl[5-(7H-pyrrolo[2,3-d]pyrimidin-4-yl)-1,3-thiazol-2-yl]amino)propanenitrile;
3-[5-(7H-pyrrolo[2,3-d]pyrimidin-4-yl)-1,3-thiazol-2-yl]hexanenitrile;
3-cyclopentyl-3-[5-(7H-pyrrolo[2,3-d]pyrimidin-4-yl)-1,3-thiazol-2-yl]propanenitrile;
5-methyl-3-[5-(7H-pyrrolo[2,3-d]pyrimidin-4-yl)-1,3-thiazol-2-yl]hexanenitrile;
3-pyridin-3-yl-3-[5-(7H-pyrrolo[2,3-d]pyrimidin-4-yl)-1,3-thiazol-2-yl]propanenitrile;
3-(5-bromopyridin-3-yl)-3-[5-(7H-pyrrolo[2,3-d]pyrimidin-4-yl)-1,3-thiazol-2-yl]propanenitrile;
5-(2-cyano-1-[5-(7H-pyrrolo[2,3-d]pyrimidin-4-yl)-1,3-thiazol-2-yl]ethyl)nicotinonitrile;
3-[5-(7H-pyrrolo[2,3-d]pyrimidin-4-yl)-1,3-thiazol-2-yl]butanenitrile;
2-[5-(7H-pyrrolo[2,3-d]pyrimidin-4-yl)-1,3-thiazol-2-yl]pentanenitrile;
4-[5-(7H-pyrrolo[2,3-d]pyrimidin-4-yl)-1,3-thiazol-2-yl]heptanenitrile;
3-[5-(7H-pyrrolo [2,3-d]pyrimidin-4-yl)-1,3-thiazol-2-yl]pentanedinitrile;
3-cyclopentyl-3-[5-(7H-pyrrolo[2,3-d]pyrimidin-4-yl)-1,3-oxazol-2-yl]propanenitrile;
3-[5-(7H-pyrrolo[2,3-d]pyrimidin-4-yl)-1,3-oxazol-2-yl]hexanenitrile;
5-(methylthio)-3-[4-(7H-pyrrolo[2,3-d]pyrimidin-4-yl)-1H-pyrazol-1-yl]pentanenitrile;
5-(methylsulfinyl)-3-[4-(7H-pyrrolo[2,3-d]pyrimidin-4-yl)-1H-pyrazol-1-yl]-pentanenitrile;
5-(methylsulfonyl)-3-[4-(7H-pyrrolo[2,3-d]pyrimidin-4-yl)-1H-pyrazol-1-yl]-pentanenitrile;
4,4,4-trifluoro-3-[4-(7H-pyrrolo[2,3-d]pyrimidin-4-yl)-pyrazol-1-yl]-butyronitrile;
5,5-dimethyl-3-[4-(7H-pyrrolo[2,3-d]pyrimidin-4-yl)-pyrazol-1-yl]-hexanenitrile;
4-[1-(2-methanesulfonyl-ethyl)-1H-pyrazol-4-yl]-7H-pyrrolo[2,3-d]pyrimidine;
5,5,5-trifluoro-4-[4-(7H-pyrrolo[2,3-d]pyrimidin-4-yl)-pyrazol-1-yl]-pentanenitrile;
3-(2-cyano-1-[4-(7H-pyrrolo[2,3-d]pyrimidin-4-yl)-1H-pyrazol-1-yl]ethyl)-cyclopentane-carbonitrile;
3-[3-(hydroxymethyl)cyclopentyl]-3-[4-(7H-pyrrolo[2,3-d]pyrimidin-4-yl)-1H-pyrazol-1-yl]-propanenitrile;
1-(1H-pyrrolo[2,3-b]pyridin-4-yl)-1H-indazole;
2-(1H-pyrrolo[2,3-b]pyridin-4-yl)-2H-indazole;
3-[3-(1H-pyrrolo[2,3-b]pyridin-4-yl)-1,2,4-oxadiazol-5-yl]benzonitrile;
4-(1-benzothien-2-yl)-1H-pyrrolo[2,3-b]pyridine;
4-fluoro-2-[1-(1H-pyrrolo[2,3-b]pyridin-4-yl)-1H-pyrazol-3-yl]phenol ;
4-3-[3-(trifluoromethyl)phenyl]-1H-pyrazol-1-yl-1H-pyrrolo[2,3-b]pyridine;
3-[1-(1H-pyrrolo[2,3-b]pyridin-4-yl)-1H-pyrazol-3-yl]benzonitrile;
3-[1-(1H-pyrrolo[2,3-b]pyridin-4-yl)-1H-pyrazol-4-yl]benzonitrile;
2-[1-(1H-pyrrolo[2,3-b]pyridin-4-yl)-1H-pyrazol-4-yl]-1,3-benzoxazole;
cyclohexyl[1-(1H-pyrrolo[2,3-b]pyridin-4-yl)-1H-pyrazol-4-yl]methanol;
4-[4-(1-phenylvinyl)-1H-pyrazol-1-yl]-1H-pyrrolo[2,3-b]pyridine;
4-(1-benzyl-1H-pyrazol-4-yl)-1H-pyrrolo[2,3-b]pyridine;
4-[1-(2-naphthylmethyl)-1H-pyrazol-4-yl]-1H-pyrrolo[2,3-b]pyridine;
4-(1-phenyl-1H-pyrazol-4-yl)-1H-pyrrolo[2,3-b]pyridine;
3-[4-(1H-pyrrolo[2,3-b]pyridin-4-yl)-1H-pyrazol-1-yl]benzonitrile;
4-{1-[1-methylbutyl]-1H-pyrazol-4-yl}-1H-pyrrolo[2,3-b]pyridine ;
4-methyl-3-[4-(1H-pyrrolo[2,3-b]pyridin-4-yl)-1H-pyrazol-1-yl]benzonitrile;
2-(1H-pyrrolo[2,3-b]pyridin-4-yl)-4,5,6,7-tetrahydro-2H-indazole;
5-nitro-2-(1H-pyrrolo[2,3-b]pyridin-4-yl)-2H-indazole;
6-nitro-2-(1H-pyrrolo[2,3-b]pyridin-4-yl)-2H-indazole;
3-[1-(1H-pyrrolo[2,3-b]pyridin-4-yl)-1H-imidazol-4-yl]benzonitrile;
4-[4-(3-methoxyphenyl)-1H-imidazol-1-yl]-1H-pyrrolo[2,3-b]pyridine;
4-(5-phenyl-2-thienyl)-1H-pyrrolo[2,3-b]pyridine;
4-[3-(4-fluorophenyl)-1H-pyrazol-1-yl]-1H-pyrrolo[2,3-b]pyridine;
4-[3-(3-nitrophenyl)-1H-pyrazol-1-yl]-1H-pyrrolo[2,3-b]pyridine;
4-[3-(4-chlorophenyl)-1H-pyrazol-1-yl]-1H-pyrrolo[2,3-b]pyridine;
4-[3-(4-methoxyphenyl)-1H-pyrazol-1-yl]-1H-pyrrolo[2,3-b]pyridine;
4-[1-(1H-pyrrolo[2,3-b]pyridin-4-yl)-1H-pyrazol-3-yl]benzonitrile;
3-[1-(1H-pyrrolo[2,3-b]pyridin-4-yl)-1H-pyrazol-3-yl]aniline;
4-[3-(3-methoxyphenyl)-1H-pyrazol-1-yl]-1H-pyrrolo[2,3-b]pyridine;
{3-[1-(1H-pyrrolo[2,3-b]pyridin-4-yl)-1H-pyrazol-3-yl]phenoxy}acetonitrile;
2-cyano-N-{3-[1-(1H-pyrrolo[2,3-b]pyridin-4-yl)-1H-pyrazol-3-yl]phenyl}acetamide;
3-cyano-N-{3-[1-(1H-pyrrolo[2,3-b]pyridin-4-yl)-1H-pyrazol-3-yl]phenyl}benzamide;
4-[4-(4-nitrophenyl)-1H-pyrazol-1-yl]-1H-pyrrolo[2,3-b]pyridine;
4-[1-(1H-pyrrolo[2,3-b]pyridin-4-yl)-1H-pyrazol-4-yl]aniline;
4-(4-phenyl-1H-pyrazol-1-yl)-1H-pyrrolo[2,3-b]pyridine;
4-(4-pyridin-3-yl-1H-pyrazol-1-yl)-1H-pyrrolo[2,3-b]pyridine;
2-[1-(1H-pyrrolo[2,3-b]pyridin-4-yl)-1H-pyrazol-4-yl]benzonitrile;
{2-[1-(1H-pyrrolo[2,3-b]pyndm-4-yl)-1H-pyrazol-4-yl]phenyl}acetonitrile;
4-[4-(3-nitrophenyl)-1H-pyrazol-1-yl]-1H-pyrrolo[2,3-b]pyridine;
3-[1-(1H-pyrrolo[2,3-b]pyridin-4-yl)-1H-pyrazol-4-yl]aniline;
{3-[1-(1H-pyrrolo[2,3-b]pyridin-4-yl)-1H-pyrazol-4-yl]phenyl}acetonitrile;
4-[1-(1H-pyrrolo[2,3-b]pyridin-4-yl)-1H-pyrazol-4-yl]benzonitrile;
3-[1-(1H-pyrrolo[2,3-b]pyridin-4-yl)-1H-pyrazol-4-yl]phenol;
methyl 3-[1-(1H-pyrrolo[2,3-b]pyridin-4-yl)-1H-pyrazol-4-yl]benzoate;
{4-[1-(1H-pyrrolo[2,3-b]pyridin-4-yl)-1H-pyrazol-4-yl]phenyl}acetonitrile;
2-cyano-N-{3-[1-(1H-pyrrolo[2,3-b]pyridin-4-yl)-1H-pyrazol-4-yl]phenyl}acetamide;
4-[1-(1H-pyrrolo[2,3-b]pyridin-4-yl)-1H-pyrazol-4-yl]phenol;
5-[1-(1H-pyrrolo[2,3-b]pyridin-4-yl)-1H-pyrazol-4-yl]nicotinonitrile;
{4-[1-(1H-pyrrolo[2,3-b]pyridin-4-yl)-1H-pyrazol-4-yl]phenoxy}acetonitrile;
4-(4-cyclohex-1-en-1-yl-1H-pyrazol-1-yl)-1H-pyrrolo[2,3-b]pyridine;
4-[4-(4-methoxyphenyl)-1H-pyrazol-1-yl]-1H-pyrrolo[2,3-b]pyridine;
4-(4-pyrimidin-4-yl-1H-pyrazol-1-yl)-1H-pyrrolo[2,3-b]pyridine;
3-{hydroxy[1-(1H-pyrrolo[2,3-b]pyridin-4-yl)-1H-pyrazol-4-yl]methyl}benzonitrile;
4-[4-(cyclohex-1-en-1-ylmethyl)-1H-pyrazol-1-yl]-1H-pyrrolo[2,3-b]pyridine;
4-[1-(3,5-dimethoxybenzyl)-1H-pyrazol-4-yl]-1H-pyrrolo[2,3-b]pyridine;
4-[1-(1-phenylethyl)-1H-pyrazol-4-yl]-1H-pyrrolo[2,3-b]pyridine;
4-[1-(cyclohexylmethyl)-1H-pyrazol-4-yl]-1H-pyrrolo[2,3-b]pyridine;
3-{[4-(1H-pyrrolo[2,3-b]pyridin-4-yl)-1H-pyrazol-1-yl]methyl}benzonitrile;
2-{[4-(1H-pyrrolo[2,3-b]pyridin-4-yl)-1H-pyrazol-1-yl]methyl}benzonitrile;
4-{[4-(1H-pyrrolo[2,3-b]pyridin-4-yl)-1H-pyrazol-1-yl]methyl}benzonitrile;
1-phenyl-2-[4-(1H-pyrrolo[2,3-b]pyridin-4-yl)-1H-pyrazol-1-yl]ethanone;
3,3-dimethyl-1-[4-(1H-pyrrolo[2,3-b]pyridin-4-yl)-1H-pyrazol-1-yl]butan-2-one;
4-{1-[(5-methylisoxazol-3-yl)methyl]-1H-pyrazol-4-yl}-1H-pyrrolo[2,3-b]pyridine;
4-[1-(tetrahydro-2H-pyran-2-ylmethyl)-1H-pyrazol-4-yl]-1H-pyrrolo[2,3-b]pyridine;
4-(1-cyclohex-2-en-1-yl-1H-pyrazol-4-yl)-1H-pyrrolo[2,3-b]pyridine;
4-[1-(1-ethylpropyl)-1H-pyrazol-4-yl]-1H-pyrrolo[2,3-b]pyridine;
4-(1-cyclohexyl-1H-pyrazol-4-yl)-1H-pyrrolo[2,3-b]pyridine;
2-[4-(1H-pyrrolo[2,3-b]pyridin-4-yl)-1H-pyrazol-1-yl]acetamide;
4'-{[4-(1H-pyrrolo[2,3-b]pyridin-4-yl)-1H-pyrazol-1-yl]methyl}biphenyl-2-carbonitrile;
4-[1-(2-nitrobenzyl)-1H-pyrazol-4-yl]-1H-pyrrolo[2,3-b]pyridine;
4-{1-[2,6-dichloro-4-(trifluoromethyl)phenyl]-1H-pyrazol-4-yl}-1H-pyrrolo[2,3-b]pyridine;
4-[1-(3-nitrobenzyl)-1H-pyrazol-4-yl]-1H-pyrrolo[2,3-b]pyridine;
4-[1-(2-bromobenzyl)-1H-pyrazol-4-yl]-1H-pyrrolo[2,3-b]pyridine;
N-phenyl-2-[4-(1H-pyrrolo[2,3-b]pyridin-4-yl)-1H-pyrazol-1-yl]propanamide;
4-{1-[3-(trifluoromethoxy)benzyl]-1H-pyrazol-4-yl}-1H-pyrrolo[2,3-b]pyridine;
4-{1-[2-fluoro-5-(trifluoromethyl)benzyl]-1H-pyrazol-4-yl}-1H-pyrrolo[2,3-b]pyridine;
4-{1-[3-(trifluoromethyl)benzyl]-1H-pyrazol-4-yl}-1H-pyrrolo[2,3-b]pyridine;
4-[1-(pyridin-3-ylmethyl)-1H-pyrazol-4-yl]-1H-pyrrolo[2,3-b]pyridine;
4-{1-[1-phenylbutyl]-1H-pyrazol-4-yl}-1H-pyrrolo[2,3-b]pyridine:
1-phenyl-2-[4-(1H-pyrrolo[2,3-b]pyridin-4-yl)-1H-pyrazol-1-yl]propan-1-one;
4-[1-(2,6-dichlorobenzyl)-1H-pyrazol-4-yl]-1H-pyrrolo[2,3-b]pyridine;
4-[1-(2,6-dimethylphenyl)-1H-pyrazol-4-yl]-1H-pyrrolo[2,3-b]pyridine;
2-[4-(1H-pyrrolo[2,3-b]pyridin-4-yl)-1H-pyrazol-1-yl]-5-(trifluoromethyl)-benzonitrile;
4-[1-(4-bromo-3,5,6-trifluoropyridin-2-yl)-1H-pyrazol-4-yl]-1H-pyrrolo[2,3-b]pyridine;
4-[1-(cyclopropylmethyl)-1H-pyrazol-4-yl]-1H-pyrrolo[2,3-b]pyridine;
4-[1-(2,5-dimethylphenyl)-1H-pyrazol-4-yl]-1H-pyrrolo[2,3-b]pyridine;
4-[1-(2-methylphenyl)-1H-pyrazol-4-yl]-1H-pyrrolo[2,3-b]pyridine;
4-[1-(2-methoxyphenyl)-1H-pyrazol-4-yl]-1H-pyrrolo[2,3-b]pyridine;
3-{1-[4-(1H-pyrrolo[2,3-b]pyridin-4-yl)-1H-pyrazol-1-yl]ethyl}benzonitrile;
3-chloro-4-[4-(1H-pyrrolo [2,3-b]pyridin-4-yl)-1H-pyrazol-1-yl]benzonitrile;
4-[1-(1-cyclohexylethyl)-1H-pyrazol-4-yl]-1H-pyrrolo [2,3-b]pyridine;
4-fluoro-2-[4-(1H-pyrrolo[2,3-b]pyridin-4-yl)-1H-pyrazol-1-yl]benzonitrile;
2-fluoro-4-[4-(1H-pyrrolo[2,3-b]pyridin-4-yl)-1H-pyrazol-1-yl]benzonitrile;
3-fluoro-4-[4-(1H-pyrrolo[2,3-b]pyridin-4-yl)-1H-pyrazol-1-yl]benzonitrile;
4-(1-{1-[3-(trifluoromethyl)phenyl]ethyl}-1H-pyrazol-4-yl)-1H-pyrrolo[2,3-b]pyridine;
4-[1-(3,5-dimethylphenyl)-1H-pyrazol-4-yl]-1H-pyrrolo[2,3-b]pyridine;
4-[4-(1H-pyrrolo[2,3-b]pyridin-4-yl)-1H-pyrazol-1-yl]benzonitrile;
14-[4-(1H-pyrrolo[2,3-b]pyridin-4-yl)-1H-pyrazol-1-yl]phenyl}acetonitrile;
4-[1-(1-methylhexyl)-1H-pyrazol-4-yl]-1H-pyrrolo[2,3-b]pyridine;
4-(1-sec-butyl-1H-pyrazol-4-yl)-1H-pyrrolo[2,3-b]pyridine;
4-[1-(1-phenylpropyl)-1H-pyrazol-4-yl]-1H-pyrrolo[2,3-b]pyridine;
4-(1-{1-[4-(methylsulfonyl)phenyl]ethyl}-1H-pyrazol-4-yl)-1H-pyrrolo[2,3-b]pyridine;
4-{1-[1-(3-fluoro-4-methoxyphenyl)ethyl]-1H-pyrazol-4-yl}-1H-pyrrolo[2,3-b]pyridine;
4-(1-(1-[2-(trifluoromethyl)phenyl]ethyl}-1H-pyrazol-4-yl)-1H-pyrrolo[2,3-b]pyridine;
4-(1-{1-[3,5-bis(trifluoromethyl)phenyl]ethyl}-1H-pyrazol-4-yl)-1H-pyrrolo[2,3-b]pyridine;
4-{1-[4-(1H-pyrrolo[2,3-b]pyridin-4-yl)-1H-pyrazol-1-yl]ethyl}benzonitrile;
4-{1-[4-nitro-2-(trifluoromethyl)phenyl]-1H-pyrazol-4-yl}-1H-pyrrolo[2,3-b]pyridine;
3-methyl-4-[4-(1H-pyrrolo[2,3-b]pyridin-4-yl)-1H-pyrazol-1-yl]benzonitrile;
4-[1-(2-chlorophenyl)-1H-pyrazol-4-yl]-1H-pyrrolo[2,3-b]pyridine;
3-bromo-4-[4-(1H-pyrrolo[2,3-b]pyridin-4-yl)-1H-pyrazol-1-yl]benzonitrile;
ethyl 4-[4-(1H-pyrrolo[2,3-b]pyridin-4-yl)-1H-pyrazol-1-yl]benzoate;
4-{1-[2-chloro-6-nitro-4-(trifluoromethyl)phenyl]-1H-pyrazol-4-yl}-1H-pyrrolo[2,3-b]-pyridine;
4-(1-{1-[4-(trifluoromethyl)phenyl]ethyl}-1H-pyrazol-4-yl)-1H-pyrrolo[2,3-b]pyridine;
4-[1-(2,3-dihydro-1H-inden-1-yl)-1H-pyrazol-4-yl]-1H-pyrrolo[2,3-b]pyridine;
4-[1-(1,2,3,4-tetrahydronaphthalen-1-yl)-1H-pyrazol-4-yl]-1H-pyrrolo[2,3-b]pyridine;
4-(1-{1-[2-chloro-5-(trifluoromethyl)phenyl]ethyl}-1H-pyrazol-4-yl)-1H-pyrrolo[2,3-b]-pyridine;
4-{1-[1-(2,4-dichloro-5-fluorophenyl)ethyl]-1H-pyrazol-4-yl}-1H-pyrrolo[2,3-b]pyridine;
4-[1-(1-cyclopentylethyl)-1H-pyrazol-4-yl]-1H-pyrrolo[2,3-b]pyridine;
4-[1-(1-methyl-3-phenylpropyl)-1H-pyrazol-4-yl]-1H-pyrrolo[2,3-b]pyridine;
4-[1-(1-cyclobutylethyl)-1H-pyrazol-4-yl]-1H-pyrrolo[2,3-b]pyridine;
[2-[4-(1H-pyrrolo[2,3-b]pyridin-4-yl)-1H-pyrazol-1-yl]-5-(trifluoromethyl)phenyl] acetonitrile;
[5-[4-(1H-pyrrolo[2,3-b]pyridin-4-yl)-1H-pyrazol-1-yl]-2-(trifluoromethyl)phenyl]-acetonitrile;
4-{1-[pent-3-en-1-yl]-1H-pyrazol-4-yl}-1H-pyrrolo[2,3-b]pyridine;
2-[4-(1H-pyrrolo[2,3-b]pyridin-4-yl)-1H-pyrazol-1-yl]propanenitrile;
4-{1-[4-phenylbut-3-en-1-yl]-1H-pyrazol-4-yl}-1H-pyrrolo[2,3-b]pyridine;
6-[4-(1H-pyrrolo[2,3-b]pyridin-4-yl)-1H-pyrazol-1-yl]hexanenitrile;
ethyl 3-amino-2-1{[4-(1H-pyrrolo[2,3-b]pyridin-4-yl)-1H-pyrazol-1-yl]methyl}-propanoate;
ethyl 2-[4-(1H-pyrrolo[2,3-b]pyridin-4-yl)-1H-pyrazol-1-yl]propanoate;
4-[1-(1-propylbutyl)-1H-pyrazol-4-yl]-1H-pyrrolo[2,3-b]pyridine;
4-[4-(1H-pyrrolo[2,3-b]pyridin-4-yl)-1H-pyrazol-1-yl]butanenitrile;
[3-chloro-2-[4-(1H-pyrrolo[2,3-b]pyridin-4-yl)-1H-pyrazol-1-yl]-5-(trifluoromethyl)phenyl]-acetonitrile:
5-[4-(1H-pyrrolo[2,3-b]pyridin-4-yl)-1H-pyrazol-1-yl]-2-(trifluoromethyl)-benzonitrile;
4-{1-[2-chloro-4-(trifluoromethyl)phenyl]-1H-pyrazol-4-yl}-1H-pyrrolo[2,3-b]pyridine;
4-[4-(1H-pyrrolo[2,3-b]pyridin-4-yl)-1H-pyrazol-1-yl]-2-(trifluoromethyl)-benzonitrile;
2-[4-(1H-pyrrolo[2,3-b]pyridin-4-yl)-1H-pyrazol-1-yl]benzonitrile;
3-chloro-2-[4-(1H-pyrrolo[2,3-b]pyridin-4-yl)-1H-pyrazol-1-yl]benzonitrile;
4-amino-5,6-difluoro-2-[4-(1H-pyrrolo[2,3-b]pyridin-4-yl)-1H-pyrazol-1-yl]isophthalonitrile;
1-{[4-(1H-pyrrolo[2,3-b]pyridin-4-yl)-1H-pyrazol-1-yl]methyl}-cyclopropanecarbonitrile;
5-[4-(1H-pyrrolo[2,3-b]pyridin-4-yl)-1H-pyrazol-1-yl]hexanenitrile;
2,2-dimethyl-6-[4-(1H-pyrrolo[2,3-b]pyridin-4-yl)-1H-pyrazol-1-yl]hexanenitrile;
4-[1-(1-ethyl-2-methylpropyl)-1H-pyrazol-4-yl]-1H-pyrrolo[2,3-b]pyridine;
5-bromo-2-[4-(1H-pyrrolo[2,3-b]pyridin-4-yl)-1H-pyrazol-1-yl]benzonitrile;
3-[4-(1H-pyrrolo[2,3-b]pyridin-4-yl)-1H-pyrazol-1-yl]-4-(trifluoromethyl)-benzonitrile;
2-[4-(1H-pyrrolo[2,3-b]pyridin-4-yl)-1H-pyrazol-1-yl]-3-(trifluoromethyl)-benzonitrile;
3-[4-(1H-pyrrolo[2,3-b]pyridin-4-yl)-1H-pyrazol-1-yl]-4-(trifluoromethyl)benzamide;
3-[4-(1H-pyrrolo[2,3-b]pyridin-4-yl)-1H-pyrazol-1-yl]cyclohexanone;
2-[4-(1H-pyrrolo[2,3-b]pyridin-4-yl)-1H-pyrazol-1-yl]cyclohexanol;
4-(1-{[1-(methylsulfonyl)piperidin-4-yl]methyl}-1H-pyrazol-4-yl)-1H-pyrrolo[2,3-b]-pyridine;
2-[4-(1H-pyrrolo[2,3-b]pyridin-4-yl)-1H-pyrazol-1-yl]cyclohexanecarbonitrile;
4-{1-[2-(trifluoromethyl)phenyl]-1H-pyrazol-4-yl}-1H-pyrrolo[2,3-b]pyridine;
4-[1-(2,6-dichlorophenyl)-1H-pyrazol-4-yl]-1H-pyrrolo[2,3-b]pyridine;
(4-{[4-(1H-pyrrolo[2,3-b]pyridin-4-yl)-1H-pyrazol-1-yl]methyl}cyclohexyl)methanol;
4-[1-(tetrahydrofuran-2-ylmethyl)-1H-pyrazol-4-yl]-1H-pyrrolo[2,3-b]pyridine;
4-[1-(1-cyclopentylpropyl)-1H-pyrazol-4-yl]-1H-pyrrolo[2,3-b]pyridine;
4-[1-(tetrahydrofuran-3-ylmethyl)-1H-pyrazol-4-yl]-1H-pyrrolo[2,3-b]pyridine;
2-chloro-3-[4-(1H-pyrrolo[2,3-b]pyridin-4-yl)-1H-pyrazol-1-yl]benzonitrile;
3-[4-(1H-pyrrolo[2,3-b]pyridin-4-yl)-1H-pyrazol-1-yl]-3-(1,3-thiazol-5-yl)propanenitrile;
1-benzyl-4-{[4-(1H-pyrrolo[2,3-b]pyridin-4-yl)-1H-pyrazol-1-yl]methyl}pyrrolidin-2-one;
3-(1-methyl-1H-imidazol-5-yl)-3-[4-(1H-pyrrolo[2,3-b]pyridin-4-yl)-1H-pyrazol-1-yl]-propanenitrile;
3-[4-(1H-pyrrolo[2,3-b]pyridin-4-yl)-1H-pyrazol-1-yl]-3-(3-thienyl)propanenitrile;
{1-[4-(1H-pyrrolo[2,3-b]pyridin-4-yl)-1H-pyrazol-1-yl]cyclopentyl}acetonitrile;
4-chloro-3-[4-(1H-pyrrolo[2,3-b]pyridin-4-yl)-1H-pyrazol-1-yl]benzonitrile;
4-[4-(1H-pyrrolo[2,3-b]pyridin-4-yl)-1H-pyrazol-1-yl]phthalonitrile;
3-methyl-4-[4-(1H-pyrrolo[2,3-b]pyridin-4-yl)-1H-pyrazol-1-yl]benzaldehyde;
4-[1-(2-methyl-4-nitrophenyl)-1H-pyrazol-4-yl]-1H-pyrrolo[2,3-b]pyridine;
3-[4-(1H-pyrrolo[2,3-b]pyridin-4-yl)-1H-pyrazol-1-yl]cyclopentanone;
4-[1-(3-furylmethyl)-1H-pyrazol-4-yl]-1H-pyrrolo[2,3-b]pyridine;
4-[1-(2-furylmethyl)-1H-pyrazol-4-yl]-1H-pyrrolo[2,3-b]pyridine;
3-{2-cyano-1-[4-(1H-pyrrolo[2,3-b]pyridin-4-yl)-1H-pyrazol-1-yl]ethyl}benzonitrile;
{3-methyl-4-[4-(1H-pyrrolo[2,3-b]pyridin-4-yl)-1H-pyrazol-1-yl]phenyl}methanol;
4-methyl-4-[4-(1H-pyrrolo[2,3-b]pyridin-4-yl)-1H-pyrazol-1-yl]pentan-2-one;
3-(1-benzofuran-2-yl)-3-[4-(1H-pyrrolo[2,3-b]pyridin-4-yl)-1H-pyrazol-1-yl]propanenitrile;
3-(3-furyl)-3-[4-(1H-pyrrolo[2,3-b]pyridin-4-yl)-1H-pyrazol-1-yl]propanenitrile;
{3-methyl-4-[4-(1H-pyrrolo[2,3-b]pyridin-4-yl)-1H-pyrazol-1-yl]phenyl}acetonitrile;
4-methyl-3-[4-(7H-pyrrolo[2,3-d]pyrimidin-4-yl)-1H-pyrazol-1-yl]benzonitrile;
4-[1-(1-cyclopentylpropyl)-1H-pyrazol-4-yl]-7H-pyrrolo[2,3-d]pyrimidine;
{1-[4-(7H-pyrrolo[2,3-d]pyrimidin-4-yl)-1H-pyrazol-1-yl]cyclopentyl}acetonitrile;
3-{(2-cyano-1-[4-(7H-pyrrolo[2,3-d]pyrimidin-4-yl)-1H-pyrazol-1-yl]ethyl}benzonitrile;
3-[4-(7H-pyrrolo[2,3-d]pyrimidin-4-yl)-1H-pyrazol-1-yl]-3-(3-thienyl)propanenitrile;
4-chloro-3-[4-(7H-pyrrolo[2,3-d]pyrimidin-4-yl)-1H-pyrazol-1-yl]benzonitrile;
3-(3-furyl)-3-[4-(7H-pyrrolo[2,3-d]pyrimidin-4-yl)-1H-pyrazol-1-yl]propanenitrile;
3-[4-(7H-pyrrolo[2,3-d]pyrimidin-4-yl)-1H-pyrazol-1-yl]pentanedinitrile;
3-{1-[4-(7H-pyrrolo[2,3-d]pyrimidin-4-yl)-1H-pyrazol-1-yl]cyclopentyl}-propanenitrile;
{1-[4-(7H-pyrrolo[2,3-d]pyrimidin-4-yl)-1H-pyrazol-1-yl]cyclohexyl}acetonitrile;
{3-methyl-4-[4-(7H-pyrrolo[2,3-d]pyrimidin-4-yl)-1H-pyrazol-1-yl]phenyl} methanol;
3-pyridin-4-yl-3-[4-(7H-pyrrolo[2,3-d]pyrimidin-4-yl)-1H-pyrazol-1-yl]propanenitrile;
3-pyridin-3-yl-3-[4-(7H-pyrrolo[2,3-d]pyrimidin-4-yl)-1H-pyrazol-1-yl]propanenitrile;
3-[4-(methylthio)phenyl]-3-[4-(7H-pyrrolo[2,3-d]pyrimidin-4-yl)-1H-pyrazol-1-yl]propanenitrile;
3-(3-methoxyphenyl)-3-[4-(7H-pyrrolo[2,3-d]pyrimidin-4-yl)-1H-pyrazol-1-yl]propanenitrile;
3-(4-methoxyphenyl)-3-[4-(7H-pyrrolo[2,3-d]pyrimidin-4-yl)-1H-pyrazol-1-yl]propanenitrile;
{3-methyl-4-[4-(7H-pyrrolo[2,3-d]pyrimidin-4-yl)-1H-pyrazol-1-yl]phenyl}acetonitrile;
3-[4-(methylsulfinyl)phenyl]-3-[4-(7H-pyrrolo[2,3-d]pyrimidin-4-yl)-1H-pyrazol-1-yl]-propanenitrile;
3-[4-(methylsulfonyl)phenyl]-3-[4-(7H-pyrrolo[2,3-d]pyrimidin-4-yl)-1H-pyrazol-1-yl]-propanenitrile;
3-[3-(cyanomethoxy)phenyl]-3-[4-(7H-pyrrolo[2,3-d]pyrimidin-4-yl)-1H-pyrazol-1-yl]-propanenitrile;
3-(6-chloropyridin-3-yl)-3-[4-(7H-pyrrolo[2,3-d]pyrimidin-4-yl)-1H-pyrazol-1-yl]-propanenitrile;
5-{2-cyano-1-[4-(7H-pyrrolo[2,3-d]pyrimidin-4-yl)-1H-pyrazol-1-yl]ethyl}pyridine-2-carbonitrile:
3-(3, 5-dimethylisoxazol-4-yl)-3-[4-(7H-pyrrolo[2,3-d]pyrimidin-4-yl)-1H-pyrazol-1-yl]-propanenitrile;
3-[4-(7H-pyrrolo[2,3-d]pyrimidin-4-yl)-1H-pyrazol-1-yl]-3-[6-(trifluoromethyl)pyridin-3-yl]-propanenitrile;
3-(6-methoxypyridin-3-yl)-3-[4-(7H-pyrrolo[2,3-d]pyrimidin-4-yl)-1-pyrazol-1-yl]propanenitrile;
3-pyridin-2-yl-3-[4-(7H-pyrrolo[2,3-d]pyrimidin-4-yl)-1H-pyrazol-1-yl]propanenitrile;
3-(6-bromopyridin-2-yl)-3-[4-(7H-pyrrolo[2,3-d]pyrimidin-4-yl)-1H-pyrazol-1-yl]propanenitrile;
6-{2-cyano-1-[4-(7H-pyrrolo[2,3-d]pyrimidin-4-yl)-1H-pyrazol-1-yl]ethyl}pyridine-2-carbonitrile;
4-[4-(7H-pyrrolo[2,3-d]pyrimidin-4-yl)-1H-pyrazol-1-yl]heptanedinitrile;
3-(5-bromopyridin-3-yl)-3-[4-(7H-pyrrolo[2,3-d]pyrimidin-4-yl)-1H-pyrazol-1-yl]propanenitrile;
3-[4-(7H-Pyrrolo[2,3-d]pyrimidin-4-yl)-1H-pyrazol-1-yl]pentane-1,5-diol
5-{2-cyano-1-[4-(7H-pyrrolo[2,3-d]pyrimidin-4-yl)-1H-pyrazol-1-yl]ethyl}nicotinonitrile;
3-(2-methoxypyridin-3-yl)-3-[4-(7H-pyrrolo[2,3-d]pyrimidin-4-yl)-1H-pyrazol-1-yl]propanenitrile;
3-[4-(cyanomethoxy)phenyl]-3-[4-(7H-pyrrolo[2,3-d]pyrimidin-4-yl)-1H-pyrazol-1-yl]-propanenitrile;
3-[2-(cyanomethoxy)phenyl]-3-[4-(7H-pyrrolo[2,3-d]pyrimidin-4-yl)-1H-pyrazol-1-yl]-propanenitrile;
3-(3,5-dibromophenyl)-3-[4-(7H-pyrrolo[2,3-d]pyrimidin-4-yl)-1H-pyrazol-1-yl]propanenitrile;
5-{2-cyano-1-[4-(7H-pyrrolo[2,3-d]pyrimidin-4-yl)-1H-pyrazol-1-yl]ethyl}isophthalomtrile;
3-[6-(dimethylamino)pyridin-2-yl]-3-[4-(7H-pyrrolo[2,3-d]pyrimidin-4-yl)-1H-pyrazol-1-yl]-propanenitrile;
3-(4-bromo-2-thienyl)-3-[4-(7H-pyrrolo[2,3-d]pyrimidin-4-yl)-1H-pyrazol-1-yl]propanenitrile;
5-{2-cyano-1-[4-(7H-pyrrolo[2,3-d]pyrimidin-4-yl)-1H-pyrazol-1-yl]ethyl}thiophene-3-carbonitrile;
3-(5-bromo-2-fluorophenyl)-3-[4-(7H-pyrrolo[2,3-d]pyrimidin-4-yl)-1H-pyrazol-1-yl]-propanenitrile;
3-(3-nitrophenyl)-3-[4-(7H-pyrrolo[2,3-d]pyrimidin-4-yl)-1H-pyrazol-1-yl]propanenitrile;
3-(5-bromo-2-methoxyphenyl)-3-[4-(7H-pyrrolo[2,3-d]pyrimidin-4-yl)-1H-pyrazol-1-yl]-propanenitrile;
3-{2-cyano-1-[4-(7H-pyrrolo[2,3-d]pyrimidin-4-yl)-1H-pyrazol-1-yl]ethyl}-4-methoxybenzonitrile;
3-(3-bromophenyl)-3-[4-(7H-pyrrolo[2,3-d]pyrimidin-4-yl)-1H-pyrazol-1-yl]propanenitrile;
3-{2-cyano-1-[4-(7H-pyrrolo[2,3-d]pyrimidin-4-yl)-1H-pyrazol-1-yl]ethyl}-4-fluorobenzonitrile;
3-[5-bromo-2-(cyanomethoxy)phenyl]-3-[4-(7H-pyrrolo[2,3-d]pyrimidin-4-yl)-1H-pyrazol-1-yl]propancnitrilc;
3-(4-bromo-2-furyl)-3-[4-(7H-pyrrolo[2,3-d]pyrimidin-4-yl)-1H-pyrazol-1-yl]propanenitrile;
4-(cyanomethoxy)-3-{2-cyano-1-[4-(7H-pyrrolo[2,3-d]pyrimidin-4-yl)-1H-pyrazol-1-yl]-ethyl}benzonitrile;
3-(4-bromopyridin-2-yl)-3-[4-(7H-pyrrolo[2,3-d]pyrimidin-4-yl)-1H-pyrazol-1-yl]propanenitrile;
2-{2-cyano-1-[4-(7H-pyrrolo[2,3-d]pyrimidin-4-yl)-1H-pyrazol-1-yl]ethyl}isonicotinonitrile;
5-{2-cyano-1-[4-(7H-pyrrolo[2,3-d]pyrimidin-4-yl)-1H-pyrazol-1-yl]ethyl}-3-furonitrile;
3-[2-bromo-5-(cyanomethoxy)phenyl]-3-[4-(7H-pyrrolo[2,3-d]pyrimidin-4-yl)-1H-pyrazol-1-yl]propanenitrile;
4-(cyanomethoxy)-2-{2-cyano-1-[4-(7H-pyrrolo[2,3-d]pyrimidin-4-yl)-1H-pyrazol-1-yl]-ethyl}benzonitrile;
3-pyrimidin-5-yl-3-[4-(7H-pyrrolo[2,3-d]pyrimidin-4-yl)-1H-pyrazol-1-yl]propanenitrile;
3-(2-bromopyridin-4-yl)-3-[4-(7H-pyrrolo[2,3-d]pyrimidin-4-yl)-1H-pyrazol-1-yl]propanenitrile;
4-{2-cyano-1-[4-(7H-pyrrolo[2,3-d]pyrimidin-4-yl)-1H-pyrazol-1-yl]ethyl}pyridine-2-carbonitrile;
3-(5-methoxypyridin-3-yl)-3-[4-(7H-pyrrolo[2,3-d]pyrimidin-4-yl)-1H-pyrazol-1-yl]propanenitrile;
3-(3-chlorophenyl)-3-[4-(7H-pyrrolo[2,3-d]pyrimidin-4-yl)-1H-pyrazol-1-yl]propanenitrile;
3-[4-(7H-pyrrolo[2,3-d]pyrimidin-4-yl)-1H-pyrazol-1-yl]-3-[3-(trifluoromethyl)phenyl]-propanenitrile;
3-(3-phenoxyphenyl)-3-[4-(7H-pyrrolo[2,3-d]pyrimidin-4-yl)-1H-pyrazol-1-yl]propanenitrile;
3-[4-(7H-pyrrolo[2,3-d]pyrimidin-4-yl)-1H-pyrazol-1-yl]-3-[3-(trifluoromethoxy)-phenyl]-propanenitrile;
methyl 3-{2-cyano-1-[4-(7H-pyrrolo[2,3-d]pyrimidin-4-yl)-1H-pyrazol-1-yl]ethyl}benzoate;
3-{2-cyano-1-[4-(7H-pyrrolo[2,3-d]pyrimidin-4-yl)-1H-pyrazol-1-yl]ethyl}benzoic acid;
3-[3-(1H-pyrazol-4-yl)phenyl]-3-[4-(7H-pyrrolo[2,3-d]pyrimidin-4-yl)-1H-pyrazol-1-yl]-propanenitrile;
3-(3-aminophenyl)-3-[4-(7H-pyrrolo[2,3-d]pyrimidin-4-yl)-1H-pyrazol-1-yl]propanenitrile;
N-(3-{2-cyano-1-[4-(7H-pyrrolo[2,3-d]pyrimidin-4-yl)-1H-pyrazol-1-yl]ethyl}phenyl)-acetamide:
N-(3-{2-cyano-1-[4-(7H-pyrrolo[2,3-d]pyrimidin-4-yl)-1H-pyrazol-1-yl]ethyl}phenyl)-methanesulfonamide;
4-{2-cyano-1-[4-(7H-pyrrolo[2,3-d]pyrimidin-4-yl)-1H-pyrazol-1-yl]ethyl}thiophene-2-carbonitrile;
5-{2-cyano-1-[4-(7H-pyrrolo[2,3-d]pyrimidin-4-yl)-1H-pyrazol-1-yl]ethyl}thiophene-2-carbonitrilc;
3-[3-(morpholin-4-ylcarbonyl)phenyl]-3-[4-(7H-pyrrolo[2,3-d]pyrimidin-4-yl)-1H-pyrazol-1-yl]propanenitrile;
N-(2-aminoethyl)-3-{2-cyano-1-[4-(7H-pyrrolo[2,3-d]pyrimidin-4-yl)-1H-pyrazol-1-yl]-ethyl}benzamide;
3-(5-formyl-3-thienyl)-3-[4-(7H-pyrrolo[2,3-d]pyrimidin-4-yl)-1H-pyrazol-1-yl]propanenitrile;
3-{2-cyano-1-[4-(7H-pyrrolo[2,3-d]pyrimidin-4-yl)-1H-pyrazol-1-yl]ethyl}-N-methylbenzamide;
2-cyano-N-(3-{2-cyano-1-[4-(7H-pyrrolo[2,3-d]pyrimidin-4-yl)-1H-pyrazol-1-yl]ethyl}-phenyl)acetamide;
N-(3-{2-cyano-1-[4-(7H-pyrrolo[2,3-d]pyrimidin-4-yl)-1H-pyrazol-1-yl]ethyl}phenyl)-nicotinamide;
N-(3-{2-cyano-1-[4-(7H-pyrrolo[2,3-d]pyrimidin-4-yl)-1H-pyrazol-1-yl]ethyl}phenyl)-N'-isopropylurea;
isopropyl (3-{2-cyano-1-[4-(7H-pyrrolo[2,3-d]pyrimidin-4-yl)-1H-pyrazol-1-yl]ethyl}-phenyl)carbamate;
3-(5-phenylpyridin-3-yl)-3-[4-(7H-pyrrolo[2,3-d]pyrimidin-4-yl)-1H-pyrazol-1-yl]propanenitrile;
3-(3,3'-bipyridin-5-yl)-3-[4-(7H-pyrrolo[2,3-d]pyrimidin-4-yl)-1H-pyrazol-1-yl]propanenitrile;
3-(5-pyrimidin-5-ylpyridin-3-yl)-3-[4-(7H-pyrrolo[2,3-d]pyrimidin-4-yl)-1H-pyrazol-1-yl]-propanenitrile;
3-[5-(1-methyl-1H-pyrazol-4-yl)pyridin-3-yl]-3-[4-(7H-pyrrolo[2,3-d]pyrimidin-4-yl)-1H-pyrazol-1-yl]propanenitrile;
3-(5-ethynylpyridin-3-yl)-3-[4-(7H-pyrrolo[2,3-d]pyrimidin-4-yl)-1H-pyrazol-1-yl]propanenitrile;
3-[5-(phenylthio)pyridin-3-yl]-3-[4-(7H-pyrrolo[2,3-d]pyrimidin-4-yl)-1H-pyrazol-1-yl]-propanenitrile;
3-(2-bromo-1,3-thiazol-5-yl)-3-[4-(7H-pyrrolo[2,3-d]pyrimidin-4-yl)-1H-pyrazol-1-yl]-propanenitrile;
ethyl 3-[4-(7H-pyrrolo[2,3-d]pyrimidin-4-yl)-1H-pyrazol-1-yl]butanoate;
3-(5-morpholin-4-ylpyridin-3-yl)-3-[4-(7H-pyrrolo[2,3-d]pyrimidin-4-yl)-1H-pyrazol-1-yl]-propanenitrile;
3-(1-methyl-1H-pyrazol-4-yl)-3-[4-(7H-pyrrolo[2,3-d]pyrimidin-4-yl)-1H-pyrazol-1-yl]-propanenitrile;
4-{1-[1-phenyl-2-(1H-1,2,4-triazol-1-yl)ethyl]-1H-pyrazol-4-yl}-7H-pyrrolo[2,3-d]-pyrimidine;
4-{1-[1-phenyl-2-(4H-1,2,4-triazol-4-yl)ethyl]-1H-pyrazol-4-yl}-7H-pyrrolo[2,3-d]-pyrimidine;
3-(3-pyridin-3-ylphenyl)-3-[4-(7H-pyrrolo[2,3-d]pyrimidin-4-yl)-1H-pyrazol-1-yl]-propanenitrile;
3-[5-(phenylsulfinyl)pyridin-3-yl]-3-[4-(7H-pyrrolo[2,3-d]pyrimidin-4-yl)-1H-pyrazol-1-yl]-propanenitrile;
3-[5-(phenylsulfonyl)pyridin-3-yl]-3-[4-(7H-pyrrolo[2,3-d]pyrimidin-4-yl)-1H-pyrazol-1-yl]-propanenitrile;
3-[4-(7H-pyrrolo[2,3-d]pyrimidin-4-yl)-1H-pyrazol-1-yl]pentan-1-ol;
methyl 3-[4-(7H-pyrrolo[2,3-d]pyrimidin-4-yl)-1H-pyrazol-1-yl]pentyl carbonate;
(1E)-3-[4-(7H-pyrrolo[2,3-d]pyrimidin-4-yl)-1H-pyrazol-1-yl]pentanal oxime;
(1E)-3-[4-(7H-pyrrolo[2,3-d]pyrimidin-4-yl)-1H-pyrazol-1-yl]pentanal O-methyloxime;
(1Z)-3-[4-(7H-pyrrolo[2,3-d]pyrimidin-4-yl)-1H-pyrazol-1-yl]pentanal O-methyloxime;
4-[1-(4,4-dibromo-1-ethylbut-3-en-1-yl)-1H-pyrazol-4-yl]-7H-pyrrolo[2,3-d]pyrimidine;
3-[4-(7H-pyrrolo[2,3-d]pyrimidin-4-yl)-1H-pyrazol-1-yl]-3-[5-(1,3-thiazol-2-ylthio)pyridin-3-yl]propanenitrile;
3-[5-(ethylthio)pyridin-3-yl]-3-[4-(7H-pyrrolo[2,3-d]pyrimidin-4-yl)-1H-pyrazol-1-yl]-propanenitrile;
4-[1-(1-ethylbut-3-yn-1-yl)-1H-pyrazol-4-yl]-7H-pyrrolo[2,3-d]pyrimidine trifluoroacetate;
4-{1-[1-methyl-2-(1H-1,2,4-triazol-1-yl)ethyl]-1H-pyrazol-4-yl}-7H-pyrrolo[2,3-d]-pyrimidine;
4-[4-(7H-pyrrolo[2,3-d]pyrimidin-4-yl)-1H-pyrazol-1-yl]pentan-2-one trifluoroacetate;
1-phenyl-2-[4-(7H-pyrrolo[2,3-d]pyrimidin-4-yl)-1H-pyrazol-1-yl]propan-1-one;
3-[5-(ethylsulfinyl)pyridin-3-yl]-3-[4-(7H-pyrrolo[2,3-d]pyrimidin-4-yl)-1H-pyrazol-1-yl]-propanenitrile;
3-[5-(ethylsulfonyl)pyridin-3-yl]-3-[4-(7H-pyrrolo [2,3-d]pyrimidin-4-yl)-1 H-pyrazol-1-yl]-propanenitrile;
3-[5-(cyclohexylthio)pyridin-3-yl]-3-[4-(7H-pyrrolo[2,3-d]pyrimidin-4-yl)-1H-pyrazol-1-yl]-propanenitrile;
1-phenyl-2-[4-(7H-pyrrolo[2,3-d]pyrimidin-4-yl)-1H-pyrazol-1-yl]propan-1-ol;
1-phenyl-2-[4-(7H-pyrrolo[2,3-d]pyrimidin-4-yl)-1H-pyrazol-1-yl]propan-1-ol;
3-[3-(ethylthio)phenyl]-3-[4-(7H-pyrrolo[2,3-d]pyrimidin-4-yl)-1H-pyrazol-1-yl]-propanenitrile;
3-[3-(ethylsulfinyl)phenyl]-3-[4-(7H-pyrrolo[2,3-d]pyrimidin-4-yl)-1H-pyrazol-1-yl]-propanenitrile;
3-[3-(ethylsulfonyl)phenyl]-3-[4-(7H-pyrrolo[2,3-d]pyrimidin-4-yl)-1H-pyrazol-1-yl]-propanenitrile;
3-[5-(cyclohexylsulfonyl)pyridin-3-yl]-3-[4-(7H-pyrrolo[2,3-d]¬pyrimidin-4-yl)-1H-pyrazol-1-yl]propanenitrile;
3-[5-(cyclohexylsulfinyl)pyridin-3-yl]-3-[4-(7H-pyrrolo[2,3-d]pyrimidin-4-yl)-1H-pyrazol-1-yl]propanenitrile;
4-[1-(1-methyl-2-phenylethyl)-1H-pyrazol-4-yl]-7H-pyrrolo[2,3-d]pyrimidine;
4-{1-[1-methyl-2-(3-thienyl)ethyl]-1H-pyrazol-4-yl}-7H-pyrrolo[2,3-d]pyrimidine;
3-{-[4-(7H-pyrrolo[2,3-d]pyrimidin-4-yl)-1H-pyrazol-1-yl]ethyl}benzonitrile;
4-{1-[2-(1H-imidazol-1-yl)-1-methylethyl]-1H-pyrazol-4-yl}-7H-pyrrolo[2,3-d]pyrimidine;
4-{1-[1-methyl-2-(3-methyl-1,2,4-oxadiazol-5-yl)ethyl]-1H-pyrazol-4-yl}-7H-pyrrolo[2,3-d]-pyrimidine;
3-[3-(methylsulfonyl)phenyl]-3-[4-(7H-pyrrolo[2,3-d]pyrimidin-4-yl)-1H-pyrazol-1-yl]-propanenitrile;
3-(3-pyridin-4-ylphenyl)-3-[4-(7H-pyrrolo[2,3-d]pyrimidin-4-yl)-1H-pyrazol-1-yl]propanenitrile;
4-[1-(1-ethylbut-3-en-1-yl)-1H-pyrazol-4-yl]-7H-pyrrolo[2,3-d]pyrimidine;
4-[1-(1,3-dimethylbut-3-en-1-yl)-1H-pyrazol-4-yl]-7H-pyrrolo[2,3-d]pyrimidine;
3-[5-(isopropylthio)pyridin-3-yl]-3-[4-(7H-pyrrolo[2,3-d]pyrimidin-4-yl)-1H-pyrazol-1-yl]-propanenitrile;
3-[5-(isopropylsulfinyl)pyridin-3-yl]-3-[4-(7H-pyrrolo[2,3-d]pyrimidin-4-yl)-1H-pyrazol-1-yl]propanenitrile;
3-[5-(isopropylsulfonyl)pyridin-3-yl]¬-3-[4-(7H-pyrrolo[2,3-d]pyrimidin-4-yl)-1H-pyrazol-1-yl]propanenitrile;
3-[4-(7H-pyrrolo[2,3-d]pyrimidin-4-yl)-1H-pyrazol-1-yl]-3-[5-(trifluoromethyl)pyridin-3-yl]-propanenitrile;
3-[4-(7H-pyrrolo [2,3-d]pyrimidin-4-yl)-1 H-pyrazol-1-yl]-3-[5-(trifluoromethyl)pyridin-3-yl]-propanenitrile;
2-[4-(7H-pyrrolo[2,3-d]pyrimidin-4-yl)-1H-pyrazol-1-yl]-N-[3-(trifluoromethyl)phenyl]-propanamide;
N-2-naphthyl-2-[4-(7H-pyrrolo[2,3-d]pyrimidin-4-yl)-1H-pyrazol-1-yl]propanamide;
N-1-naphthyl-2-[4-(7H-pyrrolo[2,3-d]pyrimidin-4-yl)-1H-pyrazol-1-yl]propanamide;
N-(3-cyanophenyl)-2-[4-(7H-pyrrolo[2,3-d]pyrimidin-4-yl)-1H-pyrazol-1-yl]propanamide;
N-benzyl-2-[4-(7H-pyrrolo[2,3-d]pyrimidin-4-yl)-1H-pyrazol-1-yl]propanamide;
N-phenyl-2-[4-(7H-pyrrolo[2,3-d]-pyrimidin-4-yl)-1H-pyrazol-1-yl]-butanamide;
N-(4-phenoxyphenyl)-2-[4-(7H-pyrrolo[2,3-d]pyrimidin-4-yl)-1H-pyrazol-1-yl]butanamide;
N-2-naphthyl-2-[4-(7H-pyrrolo[2,3-d]pyrimidin-4-yl)-1H-pyrazol-1-yl]butanamide;
N-(3-cyanophenyl)-2-[4-(7H-pyrrolo[2,3-d]pyrimidin-4-yl)-1H-pyrazol-1-yl]butanamide;
N-biphenyl-4-yl-2-[4-(7H-pyrrolo[2,3-d]pyrimidin-4-yl)-1H-pyrazol-1-yl]butanamide;
N-(biphenyl-4-ylmethyl)-2-[4-(7H-pyrrolo[2,3-d]pyrimidin-4-yl)-1H-pyrazol-1-yl]-butanamide;
N-(biphenyl-3-ylmethyl)-2-[4-(7H-pyrrolo[2,3-d]pyrimidin-4-yl)-1H-pyrazol-1-yl]-butanamide:
N-(4-cyanophenyl)-2-[4-(7H-pyrrolo[2,3-d]pyrimidin-4-yl)-1H-pyrazol-1-yl]butanamide;
N-1-naphthyl-2-[4-(7H-pyrrolo[2,3-d]pyrimidin-4-yl)-1H-pyrazol-1-yl]butanamide;
5-{2-cyano-1-[4-(7H-pyrrolo[2,3-d]pyrimidin-4-yl)-1H-pyrazol-1-yl]ethyl}-N-phenylnicotinamide;
N-(3-{2-cyano-1-[4-(7H-pyrrolo[2,3-d]pyrimidin-4-yl)-1H-pyrazol-1-yl]ethyl}phenyl)-3-(trifluoromethyl)benzamide;
N-(3-{[4-(7H-pyrrolo[2,3-d]pyrimidin-4-yl)-1H-pyrazol-1-yl]methyl}phenyl)-3-(trifluoromethyl)benzamide;
3-[3-(methylsulfonyl)phenyl]-3-[4-(7H-pyrrolo[2,3-d]pyrimidin-4-yl)-1H-pyrazol-1-yl]-propanenitrile;
3-[3-(methylsulfonyl)phenyl]-3-[4-(7H-pyrrolo[2,3-d]pyrimidin-4-yl)-1H-pyrazol-1-yl]-propanenitrile;
N-(3-{[4-(7H-pyrrolo[2,3-d]pyrimidin-4-yl)-1H-pyrazol-1-yl]methyl}phenyl)benzenesulfonamide;
3-{[4-(7H-pyrrolo[2,3-d]pyrimidin-4-yl)-1H-pyrazol-1-yl]methyl}-N-[3-(trifluoromethyl)-phenyl]benzamide;
3-{2-cyano-1-[4-(7H-pyrrolo[2,3-d]pyrimidin-4-yl)-1H-pyrazol-1-yl]ethyl}-N,N-dimethylbenzenesulfonamide;
3-{2-cyano-1-[4-(7H-pyrrolo[2,3-d]pyrimidin-4-yl)-1H-pyrazol-1-yl]ethyl}-N,N-dimethylbenzenesulfonamide;
N-benzyl-3-{2-cyano-1-[4-(7H-pyrrolo[2,3-d]pyrimidin-4-yl)-1H-pyrazol-1-yl]ethyl}-benzenesulfonamide;
N-benzyl-3-{2-cyano-1-[4-(7H-pyrrolo[2,3-d]pyrimidin-4-yl)-1H-pyrazol-1-yl]ethyl}-benzamide;
3-{2-cyano-1-[4-(7H-pyrrolo[2,3-d]pyrimidin-4-yl)-1H-pyrazol-1-yl]ethyl}-N-phenylbenzamide;
3-{2-cyano-1-[4-(7H-pyrrolo[2,3-d]pyrimidin-4-yl)-1H-pyrazol-1-yl]ethyl}-N-[3-(trifluoromethyl)phenyl]benzamide;
N-(3-cyanophenyl)-3-{[4-(7H-pyrrolo[2,3-d]pyrimidin-4-yl)-1H-pyrazol-1-yl]methyl}-benzamide;
N-benzyl-3-{[4-(7H-pyrrolo-[2,3-d]pyrimidin-4-yl)-1H-pyrazol-1-yl]methyl}benzamide;
N-1-naphthyl-3-{[4-(7H-pyrrolo[2,3-d]pyrimidin-4-yl)-1H-pyrazol-1-yl]methyl}benzamide;
N-2-naphthyl-3-{[4-(7H-pyrrolo[2,3-d]-pyrimidin-4-yl)-1H-pyrazol-1-yl]methyl}benzamide;
N-(3-{[4-(7H-pyrrolo[2,3-d]pyrimidin-4-yl)-1H-pyrazol-1-yl]methyl}phenyl)-2-naphthamide;
N-(3-{[4-(7H-pyrrolo[2,3-d]pyrimidin-4-yl)-1H-pyrazol-1-yl]methyl}phenyl)-1-naphthamide;
2-phenyl-N-(3-{[4-(7H-pyrrolo[2,3-d]pyrimidin-4-yl)-1H-pyrazol-1-yl]methyl}phenyl)-acetamide:
3-chloro-N-(3-{[4-(7H-pyrrolo[2,3-d]pyrimidin-4-yl)-1H-pyrazol-1-yl]methyl}phenyl)-benzamide;
N-(3-{2-cyano-1-[4-(7H-pyrrolo[2,3-d]pyrimidin-4-yl)-1H-pyrazol-1-yl]ethyl}phenyl)-2-naphthamide;
N-(3-{2-cyano-1-[4-(7H-pyrrolo[2,3-d]pyrimidin-4-yl)-1H-pyrazol-1-yl]ethyl}phenyl)-1-naphthamide;
N-(3-{2-cyano-1-[4-(7H-pyrrolo[2,3-d]pyrimidin-4-yl)-1H-pyrazol-1-yl]-ethyl}phenyl)-2-phenylacetamide;
3-cyano-N-(3-{2-cyano-1-[4-(7H-pyrrolo[2,3-d]pyrimidin-4-yl)-1H-pyrazol-1-yl]ethyl}-phenyl)benzamide;
N-(3-{2-cyano-1-[4-(7H-pyrrolo[2,3-d]pyrimidin-4-yl)-1H-pyrazol-1-yl]ethyl}phenyl)-benzamide;
N-(3-{2-cyano-1-[4-(7H-pyrrolo[2,3-d]pyrimidin-4-yl)-1H-pyrazol-1-yl]ethyl}phenyl)-4-(trifluoromethyl)benzamide;
N-(3-{2-cyano-1-[4-(7H-pyrrolo[2,3-d]pyrimidm-4-yl)-1H-pyrazol-1-yl]ethyl}phenyl)-N'-phenylurea;
3-{2-cyano-1-[4-(7H-pyrrolo[2,3-d]pyrimidin-4-yl)-1H-pyrazol-1-yl]ethyl}-N-[4-(trifluoromethyl)phenyl]benzamide;
3-{2-cyano-1-[4-(7H-pyrrolo[2,3-d]pyrimidin-4-yl)-1H-pyrazol-1-yl]ethyl}-N-(4-methylphenyl)benzamide;
N-(4-cyanophenyl)-3-{2-cyano-1-[4-(7H-pyrrolo[2,3-d]pyrimidin-4-yl)-1H-pyrazol-1-yl]-ethyl}benzamide;
3-{2-cyano-1-[4-(7H-pyrrolo[2,3-d]pyrimidin-4-yl)-1H-pyrazol-1-yl]ethyl}-N-2-naphthyl-benzamide;
3-{2-cyano-1-[4-(7H-pyrrolo[2,3-d]pyrimidin-4-yl)-1H-pyrazol-1-yl]ethyl}-N-1-naphthyl-benzamide;
3-{2-cyano-1-[4-(7H-pyrrolo[2,3-d]pyrimidin-4-yl)-1H-pyrazol-1-yl]ethyl}-N,N-dimethyl-benzamide;
3-{2-cyano-1-[4-(7H-pyrrolo[2,3-d]pyrimidin-4-yl)-1H-pyrazol-1-yl]ethyl}-N-pyridin-3-yl-benzamide;
3-{2-cyano-1-[4-(7H-pyrrolo[2,3-d]pyrimidin-4-yl)-1H-pyrazol-1-yl]ethyl}-N-methyl-N-phenylbenzamide;
3-{2-cyano-1-[4-(7H-pyrrolo[2,3-d]pyrimidin-4-yl)-1H-pyrazol-1-yl]ethyl}-N-cyclohexyl-benzamide;
3-{2-cyano-1-[4-(7H-pyrrolo[2,3-d]pyrimidin-4-yl)-1H-pyrazol-1-yl]ethyl}-N-(4-phenoxyphenyl)benzamide;
N-(3-cyanophenyl)-3-{2-cyano-1-[4-(7H-pyrrolo[2,3-d]pyrimidin-4-yl)-1H-pyrazol-1-yl]-ethyl}benzamide;
N-biphenyl-4-yl-3-{2-cyano-1-[4-(7H-pyrrolo[2,3-d]pyrimidin-4-yl)-1H-pyrazol-1-yl]-ethyl}benzamide;
N-(4-chlorophenyl)-3-{2-cyano-1-[4-(7H-pyrrolo[2,3-d]pyrimidin-4-yl)-1H-pyrazol-1-yl]-ethyl}benzamide;
3-{2-cyano-1-[4-(7H-pyrrolo[2,3-d]pyrimidin-4-yl)-1H-pyrazol-1-yl]ethyl}-N-(3,4-dimethylphenyl)benzamide;
3-{2-cyano-1-[4-(7H-pyrrolo[2,3-d]pyrimidin-4-yl)-1H-pyrazol-1-yl]ethyl}-N-(3-methoxyphenyl)benzamide;
3-{2-cyano-1-[4-(7H-pyrrolo[2,3-d]pyrimidin-4-yl)-1H-pyrazol-1-yl]ethyl}-N-(4-methoxyphenyl)benzamide;
3-{2-cyano-1-[4-(7H-pyrrolo[2,3-d]pyrimidin-4-yl)-1H-pyrazol-1-yl]ethyl}-N-isoxazol-3-ylbenzamide;
3-{2-cyano-1-[4-(7H-pyrrolo[2,3-d]pyrimidin-4-yl)-1H-pyrazol-1-yl]ethyl}-N-methyl-N-phenylbenzenesulfonamide;
3-{2-cyano-1-[4-(7H-pyrrolo[2,3-d]pyrimidin-4-yl)-1H-pyrazol-1-yl]ethyl}-N-propylbenzenesulfonamide;
3-{2-cyano-1-[4-(7H-pyrrolo[2,3-d]pyrimidin-4-yl)-1H-pyrazol-1-yl]ethyl}-N-phenylbenzenesulfonamide;
3-{2-cyano-1-[4-(7H-pyrrolo[2,3-d]pyrimidin-4-yl)-1H-pyrazol-1-yl]ethyl]-N-2-naphthylbenzenesulfonamide;
3-{2-cyano-1-[4-(7H-pyrrolo[2,3-d]pyrimidin-4-yl)-1H-pyrazol-1-yl]ethyl}-N-cyclopropylbenzenesulfonamide:
3-[3-(piperidin-1-ylsulfonyl)phenyl]-3-[4-(7H-pyrrolo[2,3-d]pyrimidin-4-yl)-1H-pyrazol-1-yl]propanenitrile;
3-[3-(morpholin-4-ylsulfonyl)phenyl]-3-[4-(7H-pyrrolo[2,3-d]pyrimidin-4-yl)-1H-pyrazol-1-yl]propanenitrile;
3-{2-cyano-1-[4-(7H-pyrrolo[2,3-d]pyrimidin-4-yl)-1H-pyrazol-1-yl]ethyl}-N-(4-methylphenyl)benzenesulfonamide;
3-{2-cyano-1-[4-(7H-pyrrolo[2,3-d]pyrimidin-4-yl)-1H-pyrazol-1-yl]ethyl}-N-(3,4-dimethylphenyl)benzenesulfonamide;
3-{2-cyano-1-[4-(7H-pyrrolo[2,3-d]pyrimidin-4-yl)-1H-pyrazol-1-yl]ethyl}-N-(3-methoxyphenyl)benzenesulfonamide;
3-{2-cyano-1-[4-(7H-pyrrolo[2,3-d]pyrimidin-4-yl)-1H-pyrazol-1-yl]ethyl}-N-(4-methoxyphenyl)benzenesulfonamide;
3-{2-cyano-1-[4-(7H-pyrrolo[2,3-d]pyrimidin-4-yl)-1H-pyrazol-1-yl]ethyl}-N-(3,5-dimethoxyphenyl)benzamide;
3-{2-cyano-1-[4-(7H-pyrrolo[2,3-d]pyrimidin-4-yl)-1H-pyrazol-1-yl]ethyl}-N-[4-(dimethylamino)phenyl]benzamide;
3-[3-(benzylsulfonyl)phenyl]-3-[4-(7H-pyrrolo[2,3-d]pyrimidin-4-yl)-1H-pyrazol-1-yl]-propanenitrile;
3-[3-(benzylthio)phenyl]-3-[4-(7H-pyrrolo[2,3-d]pyrimidin-4-yl)-1H-pyrazol-1-yl]-propanenitrile;
4-{[(3-{2-cyano-1-[4-(7H-pyrrolo[2,3-d]pyrimidin-4-yl)-1H-pyrazol-1-yl]ethyl}phenyl)-sulfonyl]methyl} benzonitrile;
3-{2-cyano-1-[4-(7H-pyrrolo[2,3-d]pyrimidin-4-yl)-1H-pyrazol-1-yl]ethyl}-N-methylbenzenesulfonamide;
3-{2-cyano-1-[4-(7H-pyrrolo[2,3-d]pyrimidin-4-yl)-1H-pyrazol-1-yl]ethyl}-N-1-naphthylbenzenesulfonamide;
N-biphenyl-4-yl-3-{2-cyano-1-[4-(7H-pyrrolo[2,3-d]pyrimidin-4-yl)-1H-pyrazol-1-yl]ethyl}-benzenesulfonamide;
3-{2-cyano-1-[4-(7H-pyrrolo[2,3-d]pyrimidin-4-yl)-1H-pyrazol-1-yl]ethyl}-N-[4-(trifluoromethoxy)phenyl]benzamide;
3-{2-cyano-1-[4-(7H-pyrrolo[2,3-d]pyrimidin-4-yl)-1H-pyrazol-1-yl]ethyl}-N-(2-methoxyphenyl)benzamide;
3-[3-(benzyloxy)phenyl]-3-[4-(7H-pyrrolo[2,3-d]pyrimidin-4-yl)-1H-pyrazol-1-yl]-propanenitrile;
3-{2-cyano-1-[4-(7H-pyrrolo[2,3-d]pyrimidin-4-yl)-1H-pyrazol-1-yl]ethyl}-N-cyclohexylbenzenesulfonamide:
3-[3-(3,4-dihydroisoquinolin-2(1H)-ylsulfonyl)phenyl]-3-[4-(7H-pyrrolo[2,3-d]pyrimidin-4-yl)-1H-pyrazol-1-yl]propanenitrile;
3-{2-cyano-1-[4-(7H-pyrrolo[2,3-d]pyrimidin-4-yl)-1H-pyrazol-1-yl]ethyl}-N-(2-methoxyethyl)benzenesulfonamide;
3-{2-cyano-1-[4-(7H-pyrrolo[2,3-d]pyrimidin-4-yl)-1H-pyrazol-1-yl]ethyl}-N,N-diethyl-benzenesulfonamide;
3-{3-[(4-ethylpiperazin-1-yl)sulfonyl]phenyl}-3-[4-(7H-pyrrolo[2,3-d]-pyrimidin-4-yl)-1H-pyrazol-1-yl]propanenitrile;
N-1,3-benzodioxol-5-yl-3-{2-cyano-1-[4-(7H-pyrrolo[2,3-d]pyrimidin-4-yl)-1H-pyrazol-1-yl]ethyl}benzenesulfonamide;
3-{3-[(3-methoxybenzyl)sulfonyl]phenyl}-3-[4-(7H-pyrrolo[2,3-d]pyrimidin-4-yl)-1H-pyrazol-1-yl]propanenitrile;
3-{3-[(4-methoxybenzyl)sulfonyl]phenyl}-3-[4-(7H-pyrrolo[2,3-d]pyrimidin-4-yl)-1H-pyrazol-1-yl]propanenitrile;
3-{3-[(2,6-dimethylmorpholin-4-yl)sulfonyl]phenyl}-3-[4-(7H-pyrrolo[2,3-d]pyrimidin-4-yl)-1H-pyrazol-1-yl]propanenitrile;
3-{3-[(4-oxopiperidin-1-yl)sulfonyl]phenyl}-3-[4-(7H-pyrrolo[2,3-d]pyrimidin-4-yl)-1H-pyrazol-1-yl]propanenitrile;
3-[3-(isopropylsulfonyl)phenyl]-3-[4-(7H-pyrrolo[2,3-d]pyrimidin-4-yl)-1H-pyrazol-1-yl]-propanenitrile;
3-{3-[(cyclohexylmethyl)sulfonyl]phenyl}-3-[4-(7H-pyrrolo[2,3-d]pyrimidin-4-yl)-1H-pyrazol-1-yl]propanenitrile;
3-[3-(octahydroisoquinolin-2(1H)-ylsulfonyl)phenyl]-3-[4-(7H-pyrrolo[2,3-d]pyrimidin-4-yl)-1H-pyrazol-1-yl]propanenitrile;
3-{3-[(2-phenylethyl)-sulfonyl]phenyl}-3-[4-(7H-pyrrolo[2,3-d]pyrimidin-4-yl)-1H-pyrazol-1-yl]propanenitrile cis-4-[4-(7H-pyrrolo[2,3-d]pyrimidin-4-yl)-1H-pyrazol-1-yl]cyclohexylacetonitrile;
cis-4-[4-(7H-pyrrolo[2,3-d]pyrimidin-4-yl)-1H-pyrazol-1-yl]cyclohexylmethylthiocyanate;
*N*-5-[(cis-4-[4-(7H-pyrrolo[2,3-d]pyrimidin-4-yl)-1H-pyrazol-1-yl]cyclohexylmethyl)thio]-4H-1,2,4-triazol-3-ylpyrimidin-2-amine;
N-5-[(cis-4-[4-(7H-pyrrolo[2,3-d]pyrimidin-4-yl)-1H-pyrazol-1-yl]cyclohexylmethyl)thio]-4H-1,2,4-triazol-3-ylpyrimidin-2-amine;
3-cis-4-[4-(7H-pyrrolo[2,3-d]pyrimidin-4-yl)-1H-pyrazol-1-yl]cyclohexylpropanenitrile;
5-[(2-cis-4-[4-(7H-pyrrolo[2,3-d]pyrimidin-4-yl)-1H-pyrazol-1-yl]cyclohexylethyl)thio]-4H-1,2,4-triazol-3-amine;
4-[4-(7H-pyrrolo[2,3-d]pyrimidin-4-yl)-1H-pyrazol-1-yl]cyclohexylideneacetonitrile;
cis-4-[4-(7H-pyrrolo[2,3-d]pyrimidin-4-yl)-1H-pyrazol-1-yl]cyclohexanecarbonitrile;
2-[(cis-4-[4-(7H-pyrrolo[2,3-d]pyrimidin-4-yl)-1H-pyrazol-1-yl]cyclohexylmethyl)sulfinyl]-benzonitrile;
2-[(cis-4-[4-(7H-pyrrolo[2,3-d]pyrimidin-4-yl)-1H-pyrazol-1-yl]cyclohexylmethyl)sulfonyl]-benzonitrile;
3-[4-(7H-pyrrolo[2,3-d]pyrimidin-4-yl)-1H-pyrazol-1-yl]cyclohexylacetonitrile;
5-({cis-4-[4-(7H-pyrrolo[2,3-d]pyrimidin-4-yl)-1H-pyrazol-1-yl]cyclohcxyl}thio)-1H-1,2,4-triazol-3-amine;
N-{5-[({cis-4-[4-(7H-pyrrolo[2,3-d]pyrimidin-4-yl)-1H-pyrazol-1-yl]cyclohexyl}methyl)-thio]-4H-1,2,4-triazol-3-yl}methanesulfonamide;
[cis-4-[4-(7H-Pyrrolo[2,3-d]pyrimidin-4-yl)-1H-pyrazol-1-yl]-1-(1H-1,2,4-triazol-1-yl)cyclohexyl] acetonitrile;
3-[3-(piperazin-1-ylsulfonyl)phenyl]-3-[4-(7H-pyrrolo[2,3-d]pyrimidin-4-yl)-1H-pyrazol-1-yl]propanenitrile;
3-[4-(7H-pyrrolo[2,3-d]pyrimidin-4-yl)-1H-pyrazol-1-yl]-3-[3-(thiomorpholin-4-ylsulfonyl)-phenyl]propanenitrile;
3-{3-[(4-hydroxypiperidin-1-yl)sulfonyl]phenyl}-3-[4-(7H-pyrrolo[2,3-d]pyrimidin-4-yl)-1H-pyrazol-1-yl]propanenitrile;
3-[3-(isobutylsulfonyl)phenyl]-3-[4-(7H-pyrrolo[2,3-d]pyrimidin-4-yl)-1H-pyrazol-1-yl]-propanenitrile;
3-[4-(7H-pyrrolo[2,3-d]pyrimidin-4-yl)-1H-pyrazol-1-yl]-3-{3-[(tetrahydro-2H-pyran-4-ylmethyl)sulfonyl]phenyl}propanenitrile;
3-{3-[(2-methoxyethyl)sulfonyl]phenyl}-3-[4-(7H-pyrrolo[2,3-d]pyrimidin-4-yl)-1H-pyrazol-1-yl]propanenitrile;
3-{3-[(3-furylmethyl)sulfonyl]phenyl}-3-[4-(7H-pyrrolo[2,3-d]pyrimidin-4-yl)-1H-pyrazol-1-yl]propanenitrile;
3-{3-[(1,1-dioxidothiomorpholin-4-yl)sulfonyl]phe nyl}-3-[4-(7H-pyrrolo[2,3-d]pyrimidin-4-yl)-1H-pyrazol-1-yl]propanenitrile;
3-{3-[(4-acetylpiperazin-1-yl)sulfonyl]phenyl}-3-[4-(7H-pyrrolo[2,3-d]pyrimidin-4-yl)-1H-pyrazol-1-yl]propanenitrile;
3-{3-[(pyridin-4-ylmethyl)sulfonyl]phenyl}-3-[4-(7H-pyrrolo[2,3-d]pyrimidin-4-yl)-1H-pyrazol-1-yl]propanenitrile;
4-[1-(1-phenylbut-3-yn-1-yl)-1H-pyrazol-4-yl]-7H-pyrrolo[2,3-d]pyrimidine;
4-(1-{1-[3-(morpholin-4-ylsulfonyl)phenyl]but-3-yn-1-yl}-1H-pyrazol-4-yl)-7H-pyrrolo[2,3-d]pyrimidine;
3-{1-[4-(7H-pyrrolo[2,3-d]pyrimidin-4-yl)-1H-pyrazol-1-yl]but-3-yn-1-yl}benzonitrile;
3-{1-[4-(7H-pyrrolo[2,3-d]¬pyrimidin-4-yl)-1H-pyrazol-1-yl]but-3-yn-1-yl}benzaldehyde;
methyl3-(3-cyanophenyl)-3-[4-(7H-pyrrolo[2,3-d]pyrimidin-4-yl)-1H-pyrazol-1-yl]-propanoate;
*N,N-*dimethyl-3-{1-[4-(7H-pyrrolo[2,3-d]pyrimidin-4-yl)-1H-pyrazol-1-yl]but-3-yn-1-yl}-benzenesulfonamide;
3-{2-cyano-1-[4-(7H-pyrrolo[2,3-d]pyrimidin-4-yl)-1H-pyrazol-1-yl]ethyl}-*N*-[4-(dimethylamino)phenyl]benzenesulfonamide;
3-{3-methoxy-1-[4-(7H-pyrrolo[2,3-d]pyrimidin-4-y l)-1H-pyrazol-1-yl]propyl}-*N,N-*dimethylbenzenesulfonamide;
*N*-phenyl-3-{1-[4-(7H-pyrrolo[2,3-d]pyrimidin-4-yl)-1H-pyrazol-yl]-yl]but-3-yn-1-yl}-benzamide;
4-[1-(3-methoxy-1-phenylpropyl)-1H-pyrazol-4-yl]- 7H-pyrrolo[2,3-d]pyrimidine;
*N*-[4-(dimethylamino)phenyl]-3-{1-[4-(7H-pyrrolo[2,3-d]¬pyrimidin-4-yl)-1H-pyrazol-1-yl]but-3-yn-1-yl}benzamide;
3-{3-hydroxy-1-[4-(7H-pyrrolo[2,3-d]pyrimidin-4-yl)-1H-pyrazol-1-yl]propyl}-*N,N-*dimethylbenzenesulfonamide;
3-{1-[4-(7H-pyrrolo[2,3-d]pyrimidin-4-yl)-1H-pyrazol-1-yl]but-3-en-1-yl}benzonitrile;
4-{1-[1-(3-bromophenyl)but-3-en-1-yl]-1H-pyrazol-4-yl}-7H-pyrrolo[2,3-d]pyrimidine;
3-{4,4-difluoro-1-[4-(7H-pyrrolo[2,3-d]pyrimidin-4-yl)-1H-pyrazol-1-yl]but-3-en-1-yl}-benzonitrile;
4-(1-{4,4-difluoro-1-[3-(morpholin-4-ylsulfonyl)phenyl]but-3-en-1-yl}-1H-pyrazol-4-yl)-7H-pyrrolo[2,3-d]pyrimidine;
4-(1-{1-[3-(ethylsulfonyl)phenyl]-4,4-difluorobut-3-en-1-yl}-1H-pyrazol-4-yl)-7H-pyrrolo-[2,3-d]pyrimidine;
4-(1-{1-[3-(benzyloxy)phenyl]-4,4-difluorobut-3-en-1-yl}-1H-pyrazol-4-yl)-7H-pyrrolo[2,3-d]pyrimidine;
4-[1-(2-methoxy-1-phenylethyl)-1H-pyrazol-4-yl]-7H-pyrrolo[2,3-d]pyrimidine;
4-(1-{4,4-difluoro-1-[3-(methylsulfonyl)phenyl]but-3-en-1-yl}-1H-pyrazol-4-yl)-7H-pyrrolo[2,3-d]pyrimidine;
3-{[4-(7H-pyrrolo[2,3-d]pyrimidin-4-yl)-1H-pyrazol-1-yl]methyl}benzonitrile;
3-{1-[4-(7H-pyrrolo[2,3-d]pyrimidin-4-yl)-1H-pyrazol-1-yl]¬butyl}benzonitrile;
4-(1-{1-[3-(ethylsulfonyl)phenyl]-4,4-difluorobutyl}-1H-pyrazol-4-yl)-7H-pyrrolo[2,3-d]-pyrimidine;
4-[1-(4,4-difluoro-1-{3-[(2-methoxyethyl)sulfonyl]phenyl}-but-3-en-1-yl)-1H-pyrazol-4-yl]-7H-pyrrolo[2,3-d]pyrimidine;
4-[1-(1-cyclopentylbut-3-en-1-yl)-1H-pyrazol-4-yl]-7H-pyrrolo[2,3-d]pyrimidine;
4-[1-(1-methylbut-3-en-1-yl)-1H-pyrazol-4-yl]-7H-pyrrolo[2,3-d]pyrimidine;
4-[1-(1-cyclopentyl-2-cyclopropylethyl)-1H-pyrazol-4-yl]-7H-pyrrolo[2,3-d]pyrimidine;
4-[1-(1-cyclopentylbut-3-yn-1-yl)-1H-pyrazol-4-yl]-7H-pyrrolo[2,3-d]pyrimidine;
4-[1-(1-cyclopentylbutyl)-1H-pyrazol-4-yl]-7H-pyrrolo[2,3-d]pyrimidine;
4-[1-(1-cyclopentyl-4,4-difluorobut-3-en-1-yl)-1H-pyrazol-4-yl]-7H-pyrrolo[2,3-d]-pyrimidine;
4-{1-[4,4-difluoro-1-(tetrahydrofuran-3-yl)but-3-en-1-yl]-1H-pyrazol-4-yl}-7H-pyrrolo[2,3-d]pyrimidine;
4-(1-(5,5-difluoropent-4-en-2-yl)-1H-pyrazol-4-yl)-7H-pyrrolo[2,3-d]pyrimidine; 4-[1-(1-cyclopropyl-4,4-difluorobut-3-en-1-yl)-1H-pyrazol-4-yl]-7H-pyrrolo[2,3-d]pyrimidine;
4-[1-(1-cyclopentyl-4,4-difluorobutyl)-1H-pyrazol-4-yl]-7H-pyrrolo[2,3-d]pyrimidine;
3-(1-methylcyclopentyl)-3-[4-(7H-pyrrolo[2,3-d]pyrimidin-4-yl)-1H-pyrazol-1-yl]propanenitrile;
4,4-dimethyl-3-[4-(7H-pyrrolo[2,3-d]pyrimidin-4-yl)-1H-pyrazol-1-yl]pentanenitrile;
1-{2-cyano-1-[4-(7H-pyrrolo[2,3-d]pyrimidin-4-yl)-1H-pyrazol-1-yl]ethyl}cyclopropane-carbonitrile:
*N*-[(1-{2-cyano-1-[4-(7H-pyrrolo[2,3-d]pyrimidin-4-yl)-1H-pyrazol-1-yl]ethyl}cyclopentyl)-methyl]benzamide;
3-{1-[(benzyloxy)methyl]cyclopentyl}-3-[4-(7H-pyrrolo[2,3-d]pyrimidin-4-yl)-1H-pyrazol-1-yl]propanenitrile;
3-[1-(methylsulfonyl)pyrrolidin-3-yl]-3-[4-(7H-pyrrolo[2,3-d]pyrimidin-4-yl)-1H-pyrazol-1-yl]propanenitrile;
*N'*-cyano-4-(cyanomethyl)-4-[4-(7H-pyrrolo[2,3-d]pyrimidin-4-yl)-1H-pyrazol-1-yl]-piperidine-1-carboximidamide;
4-{1-[2,2,2-trifluoro-1-(1H-imidazol-2-ylmethyl)ethyl]-1H-pyrazol-4-yl}-7H-pyrrolo[2,3-d]pyrimidine;
4-(1--2,2,2-trifluoro-1-[(4-methyl-1,3-thiazol-2-yl)methyl]ethyl-1H-pyrazol-4-yl)-7H-pyrrolo[2,3-d]pyrimidine;
4-{1-[1-(trifluoromethyl)but-3-yn-1-yl]-1H-pyrazol-4-yl}-7H-pyrrolo[2,3-d]pyrimidine;
4-{1-[1-(trifluoromethyl)but-3-en-1-yl]-1H-pyrazol-4-yl}-7H-pyrrolo[2,3-d]pyrimidine;
4-{1-[1-(trifluoromethyl)butyl]-1H-pyrazol-4-yl-7H-pyrrolo[2,3-d]pyrimidine;
4-{1-[4,4-difluoro-1-(trifluoromethyl)but-3-en-1-yl]-1H-pyrazol-4-yl}-7H-pyrrolo[2,3-d]-pyrimidine;
4-{1-[4,4-difluoro-1-(trifluoromethyl)butyl]-1H-pyrazol-4-yl}-7H-pyrrolo[2,3-d]pyrimidine;
3-pyridin-4-yl-3-[5-(7H-pyrrolo[2,3-d]pyrimidin-4-yl)-1,3-thiazol-2-yl]propanenitrile;
4-{2-cyano-1-[5-(7H-pyrrolo[2,3-d]pyrimidin-4-yl)-1,3-thiazol-2-yl]ethyl}pyridine-2-carbonitrile; and
3-pyridin-2-yl-3-[5-(7H-pyrrolo[2,3-d]pyrimidin-4-yl)-1,3-thiazol-2-yl]propanenitrile;
and pharmaceutically acceptable salts thereof.

In some embodiments, the agent is selected from 3-cyclopentyl-3-[4-(7H-pyrrolo[2,3-d]pyrimidin-4-yl)-1H-pyrazol-1-yl]propanenitrile and pharmaceutically acceptable salts thereof. In some embodiments, the agent is selected from (3R)-3-cyclopentyl-3-[4-(7H-pyrrolo[2,3-d]pyrimidin-4-yl)-1H-pyrazol-1-yl]propanenitrile and pharmaceutically acceptable salts thereof. In some embodiments, the agent is selected from 3-[3-Methyl-1-(1H-pyrrolo[2,3-b]pyridin-4-yl)-1H-pyrazol-4-yl]benzonitrile and pharmaceutically acceptable salts thereof. In some embodiments, the agent is selected from N-phenyl-4-(1H-pyrrolo[2,3-b]pyridin-4-yl)-1,3-thiazol-2-amine and pharmaceutically acceptable salts thereof.

In a second aspect, the agent is selected from compounds of Formula II: 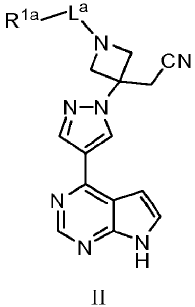 and pharmaceutically acceptable salts thereof, wherein:
L^{a} is SO₂ or CO;
R^{1a} is C₁₋₆ alkyl, C₃₋₇ cycloalkyl, phenyl, 5- or 6-membered heteroaryl, indolyl, NR^{2a}R^{3a}, or OR⁴, wherein the alkyl, cycloalkyl, phenyl, or heteroaryl is optionally substituted with 1, 2, or 3 substituents independently selected from F, CN, and C₁₋₄ alkyl;
R^{2a} and R^{3a} are independently selected from H, C₁₋₄ alkyl, and phenyl; and
R^{4a} is C₁₋₆ alkyl, phenyl, or benzyl.

In some embodiments, when L^{a} is SO₂, then R^{1a} is other than OR^{4a}.

In some embodiments, when L^{a} is SO₂, then R^{1a} is C₁₋₆ alkyl, C₃₋₇ cycloalkyl, phenyl, 5- or 6-membered heteroaryl, or NR^{2a}R^{3a}, wherein the alkyl, cycloalkyl, phenyl, or heteroaryl is optionally substituted with 1, 2, or 3 substituents independently selected from F and C₁₋₄ alkyl.

In some embodiments, when L^{a} is CO, then R^{1a} is C₃₋₇ cycloalkyl, phenyl, 5- or 6-membered heteroaryl, indolyl, NR^{2a}R^{3a}, or OR^{4a}, wherein the cycloalkyl, phenyl, or heteroaryl is optionally substituted with 1, 2, or 3 substituents independently selected from CN and C₁₋₄ alkyl.

In some embodiments, L^{a} is SO₂.

In some embodiments, L^{a} is CO.

In some embodiments, R^{1a} is methyl, ethyl, n-propyl, isopropyl, n-butyl, t-butyl, 2-methylprop-1-yl, 1-methylprop-1-yl, each optionally substituted with 1, 2, or 3 F.

In some embodiments, R^{1a} is C₁₋₄ alkyl.

In some embodiments, R^{1a} is ethyl.

In some embodiments, R^{1a} is C₃₋₇ cycloalkyl optionally substituted by C₁₋₄ alkyl.

In some embodiments, R^{1a} is phenyl optionally substituted with F, methyl, or CN.

In some embodiments, R^{1a} is 5-membered heteroaryl selected from thienyl, pyrazolyl, pyrrolyl, 1,2,4-oxadiazolyl, and isoxazolyl, each optionally substituted with C₁₋₄ alkyl.

In some embodiments, R^{1a} is pyridinyl.

In some embodiments, R^{1a} is NR^{2a}R^{3a} or OR^{4a}.

In some embodiments, L^{a} is SO₂ and R^{1a} is C₁₋₆ alkyl.

In some embodiments, the agent is selected from:
{1-(ethylsulfonyl)-3-[4-(7H-pyrrolo[2,3-d]pyrimidin-4-yl)-1H-pyrazol-1-yl]azetidin-3-yl}acetonitrile;
1-(cyclopropylsulfonyl)-3-[4-(7H-pyrrolo[2,3-d]pyrimidin-4-yl)-1H-pyrazol-1-yl]azetidin-3-ylacetonitrile;
1-[(1-methylcyclopropyl)carbonyl-3-[4-(7H-pyrrolo[2,3-d]pyrimidin-4-yl)-1H-pyrazol-1-yl]azetidin-3-ylacetonitrile;
1-[(1-methylcyclopropyl)sulfonyl]-3-[4-(7H-pyrrolo[2,3-d]pyrimidin-4-yl)-1H-pyrazol-1-yl]azetidin-3-ylacetonitrile;
1-(methylsulfonyl)-3-[4-(7H-pyrrolo[2,3-d]pyrimidin-4-yl)-1H-pyrazol-1-yl]azetidin-3-yl}acetonitrile;
{1-(phenylsulfonyl)-3-[4-(7H-pyrrolo[2,3-d]pyrimidin-4-yl)-1H-pyrazol-1-yl]azetidin-3-yl}acetonitrile;
{1-(isopropylsulfonyl)-3-[4-(7H-pyrrolo[2,3-d]pyrimidin-4-yl)-1H-pyrazol-1-yl]azetidin-3-yl}acetonitrile;
{1-(propylsulfonyl)-3-[4-(7H-pyrrolo[2,3-d]pyrimidin-4-yl)-1H-pyrazol-1-yl]azetidin-3-yl}acetonitrile;
{1-(butylsulfonyl)-3-[4-(7H-pyrrolo[2,3-d]pyrimidin-4-yl)-1H-pyrazol-1-yl]azetidin-3-yl}acetonitrile;
{1-(tert-butylsulfonyl)-3-[4-(7H-pyrrolo[2,3-d]pyrimidin-4-yl)-1H-pyrazol-1-yl]azetidin-3-yl}acetonitrile;
3-(cyanomethyl)-N,N-dimethyl-3-[4-(7H-pyrrolo[2,3-d]pyrimidin-4-yl)-1H-pyrazol-1-yl]azetidine-1-sulfonamide;
{1-[(1-methyl-1H-pyrazol-3-yl)sulfonyl]-3-[4-(7H-pyrrolo[2,3-d]pyrimidin-4-yl)-1H-pyrazol-1-yl]az etidin-3-yl} acetonitrile;
{3-[4-(7H-pyrrolo[2,3-d]pyrimidin-4-yl)-1H-pyrazo 1-1-yl]-1-[(3,3,3-trifluoropropyl)sulfonyl]azetidin-3-yl}acetonitrile;
{1-(isobutylsulfonyl)-3-[4-(7H-pyrrolo[2,3-d]pyrimidin-4-yl)-1H-pyrazol-1-yl]azetidin-3-yl}acetonitrile;
{1-(sec-butylsulfonyl)-3-[4-(7H-pyrrolo[2,3-d]pyrimidin-4-yl)-1H-pyrazol-1-yl]azetidin-3-yl}acetonitrile;
{1-[(5-methyl-2-thienyl)sulfonyl]-3-[4-(7H-pyrrolo[2,3-d]pyrimidin-4-yl)-1H-pyrazol-1-yl]azetidin-3-yl} acetonitrile;
{1-[(4-fluorophenyl)sulfonyl]-3-[4-(7H-pyrrolo[2,3-d]pyrimidin-4-yl)-1H-pyrazol-1-yl]azetidin-3-yl}acetonitrile;
{1-[(3-fluorophenyl)sulfonyl]-3-[4-(7H-pyrrolo[2,3-d]pyrimidin-4-yl)-1H-pyrazol-1-yl]azetidin-3-yl}acetonitrile;
{1-[(2-fluorophenyl)sulfonyl]-3-[4-(7H-pyrrolo[2,3-d]pyrimidin-4-yl)-1H-pyrazol-1-yl]azetidin-3-yl}acetonitrile;
{1-(pyridin-3-ylsulfonyl)-3-[4-(7H-pyrrolo[2,3-d]pyrimidin-4-yl)-1H-pyrazol-1-yl]azetidin-3-yl}acetonitrile;
{1-(pyridin-2-ylsulfonyl)-3-[4-(7H-pyrrolo[2,3-d]pyrimidin-4-yl)-1H-pyrazol-1-yl]azetidin-3-yl}acetonitrile;
{1-(cyclopropylcarbonyl)-3-[4-(7H-pyrrolo[2,3-d]pyrimidin-4-yl)-1H-pyrazol-1-yl]azetidin-3-yl}acetonitrile;
1-[(1-methylcyclopropyl)carbonyl-3-[4-(7H-pyrrolo[2,3-d]pyrimidin-4-yl)-1H-pyrazol-1-yl]azetidin-3-ylacetonitrile;
{1-benzoyl-3-[4-(7H-pyrrolo[2,3-d]pyrimidin-4-yl)-1H-pyrazol-1-yl]azetidin-3-yl}acetonitrile;
{1-[(6-methylpyridin-2-yl)carbonyl]-3-[4-(7H-pyrrolo[2,3-d]pyrimidin-4-yl)-1H-pyrazol-1-yl]azetid in-3-yl}acetonitrile;
{1-(pyridin-3-ylcarbonyl)-3-[4-(7H-pyrrolo[2,3-d]pyrimidin-4-yl)-1H-pyrazol-1-yl]azetidin-3-yl}acetonitrile;
{1-(3-methylbenzoyl)-3-[4-(7H-pyrrolo[2,3-d]pyrimidin-4-yl)-1H-pyrazol-1-yl]azetidin-3-yl}acetonitrile;
{1-(4-methylbenzoyl)-3-[4-(7H-pyrrolo[2,3-d]pyrimidin-4-yl)-1H-pyrazol-1-yl]azetidin-3-yl}acetonitrile;
3-({3-(cyanomethyl)-3-[4-(7H-pyrrolo[2,3-d]pyrimidin-4-yl)-1H-pyrazol-1-yl]azetidin-1-yl}carbonyl)benzonitrile;
[3-[4-(7H-pyrrolo[2,3-d]pyrimidin-4-yl)-1H-pyrazo 1-1-yl]-1-(2-thienylcarbonyl)azetidin-3-yl]acetonitrile;
[3-[4-(7H-pyrrolo[2,3-d]pyrimidin-4-yl)-1H-pyrazol-1-yl]-1-(1H-pyrrol-2-ylcarbonyl)azetidin-3-yl]acetonitrile;
{1-(1H-indol-2-ylcarbonyl)-3-[4-(7H-pyrrolo[2,3-d]pyrimidin-4-yl)-1H-pyrazol-1-yl]azetidin-3-yl}acetonitrile;
{1-(isoxazol-5-ylcarbonyl)-3-[4-(7H-pyrrolo[2,3-d]pyrimidin-4-yl)-1H-pyrazol-1-yl]azetidin-3-yl}acetonitrile;
{1-(1H-pyrazol-3-ylcarbonyl)-3-[4-(7H-pyrrolo[2,3-d]pyrimidin-4-yl)-1H-pyrazol-1-yl]azetidin-3-yl}acctonitrile;
isobutyl 3-(cyanomethyl)-3-[4-(7H-pyrrolo[2,3-d]pyrimidin-4-yl)-1H-pyrazol-1-yl]azetidine-1-carboxylate;
phenyl 3-(cyanomethyl)-3-[4-(7H-pyrrolo[2,3-d]pyrimidin-4-yl)-1H-pyrazol-1-yl]azetidine-1-carboxylate;
benzyl 3-(cyanomethyl)-3-[4-(7H-pyrrolo[2,3-d]pyrimidin-4-yl)-1H-pyrazol-1-yl]azetidine-1-carboxylate;
3-(cyanomethyl)-N-phenyl-3-[4-(7H-pyrrolo[2,3-d]pyrimidin-4-yl)-1H-pyrazol-1-yl]azetidine-1-carboxamide;
and pharmaceutically acceptable salts of any of the aforementioned.

In some embodiments, the agent is selected from {1-(Ethylsulfonyl)-3-[4-(7H-pyrrolo[2,3-d]pyrimidin-4-yl)-1H-pyrazol-1-yl]azetidin-3-yl} acetonitrile and pharmaceutically acceptable salts thereof. In some embodiments, the agent is {1-(Ethylsulfonyl)-3-[4-(7H-pyrrolo[2,3-d]pyrimidin-4-yl)-1H-pyrazol-1-yl]azetidin-3-yl}acetonitrile phosphoric acid salt.

In a third aspect, the agent is selected from compounds of Formula III: 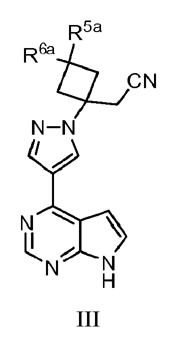 and pharmaceutically acceptable salts thereof, wherein:
R^{5a} and R^{6a} are independently selected from H, F, CN, OH, C₁₋₄ alkyl, benzyloxy, C₂₋₈ dialkylaminosulfonyl, and 5-membered heteroaryl, wherein the alkyl is optionally substituted by 1, 2, or 3 substituents selected from F, OH, CN, and C₁₋₄ alkoxy, and wherein the 5-membered heteroaryl is optionally substituted with C₁₋₄ alkyl.

In some embodiments, when one of R^{5a} and R^{6a} is OH, then the other of R^{5a} and R^{6a} is other than CN or F.

In some embodiments, one of R^{5a} and R^{6a} is H and the other is selected from H, F, CN, OH, C₁₋₄ alkyl, benzyloxy, C₂₋₈ dialkylaminosulfonyl, and 5-membered heteroaryl, wherein the alkyl is optionally substituted by 1, 2, or 3 substituents selected from F, OH, CN, and C₁₋₄ alkoxy, and wherein the 5-membered heteroaryl is optionally substituted with C₁₋₄ alkyl.

In some embodiments, R^{5a} and R^{6a} are independently selected from H, F, CN, OH, and methyl.

In some embodiments, R^{5a} and R^{6a} are independently selected from H and CN.

In some embodiments, the agent is selected from:
3-(cyanomethyl)-N,N-dimethyl-3-[4-(7H-pyrrolo[2,3-d]pyrimidin-4-yl)-1H-pyrazol-1-yl]cyclobutanesulfonamide;
3-isoxazol-3-yl-1-[4-(7H-pyrrolo[2,3-d]pyrimidin-4-yl)-1H-pyrazol-1-yl]cyclobutylacetonitrile;
{3-(3-methyl-1,2,4-oxadiazol-5-yl)-1-[4-(7H-pyrrolo[2,3-d]pyrimidn-4-yl)-1H-pyrazol-1-yl]cyclobutyl acetonitrile;
{3-(3-tert-butyl-1,2,4-oxadiazol-5-yl)-1-[4-(7H-pyrrolo[2,3-d]pyrimidin-4-yl)-1H-pyrazol-1-yl]cyclobutyl} acetonitrile;
1-[4-(7H-pyrrolo[2,3-d]pyrimidin-4-yl)-1H-pyrazol-1-yl]cyclobutylacetonitrile;
3-(hydroxymethyl)-1-[4-(7H-pyrrolo[2,3-d]pyrimidin-4-yl)-1H-pyrazol-1-yl]cyclobutylacetonitrile;
3-(fluoromethyl)-1-[4-(7H-pyrrolo[2,3-d]pyrimidin-4-yl)-1H-pyrazol-1-yl]cyclobutylacetonitrile;
3-(difluoromethyl)-1-[4-(7H-pyrrolo[2,3-d]pyrimidin-4-yl)-1H-pyrazol-1-yl]cyclobutylacetonitrile;
2,2'-[1-[4-(7H-pyrrolo[2,3-d]pyrimidin-4-yl)-1H-pyrazol-1-yl]cyclobutane-1,3-diyl]diacetonitrile;
3-(cyanomethyl)-1-methyl-3-[4-(7H-pyrrolo[2,3-d]pyrimidin-4-yl)-1H-pyrazol-1-yl]cyclobutanecarbonitrile;
3-(cyanomethyl)-1-(methoxymethyl)-3-[4-(7H-pyrrolo[2,3-d]pyrimidin-4-yl)-1H-pyrazol-1-yl]cyclobutanecarbonitrile;
3-(cyanomethyl)-1-(fluoromethyl)-3-[4-(7H-pyrrolo[2,3-d]pyrimidin-4-yl)-1H-pyrazol-1-yl]cyclobutanecarbonitrile;
1,3-bis(cyanomethyl)-3-[4-(7H-pyrrolo[2,3-d]pyrimidin-4-yl)-1H-pyrazol-1-yl]cyclobutanecarbonitrile;
3-(cyanomethyl)-3-[4-(7H-pyrrolo[2,3-d]pyrimidin-4-yl)-1H-pyrazol-1-yl]cyclobutanecarbonitrile;
3,3-bis(hydroxymethyl)-1-[4-(7H-pyrrolo[2,3-d]pyrimidin-4-yl)-1H-pyrazol-1-yl]cyclobutylacetonitrile;
3,3-bis(fluoromethyl)-1-[4-(7-[2-(trimethylsilyl)ethoxy]methyl-7H-pyrrolo[2,3-d]pyrimidin-4-yl)-1H-pyrazol-1-yl]cyclobutylacetonitrile;
2,2',2"-[1-[4-(7H-pyrrolo[2,3-d]pyrimidin-4-yl)-1H-pyrazol-1-yl]cyclobutane-1,3,3-triyl]triacetonitrile;
3-hydroxy-1-[4-(7H-pyrrolo[2,3-d]pyrimidin-4-yl)-1H-pyrazol-1-yl]cyclobutylacetonitrile;
3-fluoro-1-[4-(7H-pyrrolo[2,3-d]pyrimidin-4-yl)-1H-pyrazol-1-yl]cyclobutylacetonitrile;
3-methyl-1-[4-(7H-pyrrolo[2,3-d]pyrimidin-4-yl)-1H-pyrazol-1-yl]cyclobutylacetonitrile;
3,3-dimethyl-1-[4-(7H-pyrrolo[2,3-d]pyrimidin-4-yl)-1H-pyrazol-1-yl]cyclobutylacetonitrile;
3-(benzyloxy)-1-[4-(7H-pyrrolo[2,3-d]pyrimidin-4-yl)-1H-pyrazol-1-yl]cyclobutylacetonitrile;
and pharmaceutically acceptable salts of any of the aforementioned.

In some embodiments, the agent is selected from 3-(cyanomethyl)-3-[4-(7H-pyrrolo[2,3-d]pyrimidin-4-yl)-1H-pyrazol-1-yl]cyclobutanecarbonitrile and pharmaceutically acceptable salts thereof. In some embodiments, the agent is selected from cis-3-(cyanomethyl)-3-[4-(7H-pyrrolo[2,3-d]pyrimidin-4-yl)-1H-pyrazol-1-yl]cyclobutanecarbonitrile and pharmaceutically acceptable salts thereof. In some embodiments, the agent is selected from trans-3-(cyanomethyl)-3-[4-(7H-pyrrolo[2,3-d]pyrimidin-4-yl)-1H-pyrazol-1-yl]cyclobutanecarbonitrile and pharmaceutically acceptable salts thereof.

In a fourth aspect, the agent is selected from compounds of Formula IV: 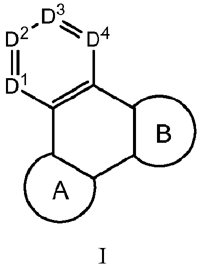 or pharmaceutically acceptable salt or prodrug thereof, wherein:
D¹ is N, NO, or CR^{1a};
D² is N, NO, or CR^{1b};
D³ is N, NO, or CR^{1c};
D⁴ is N, NO or CR^{1d};
Ring A is 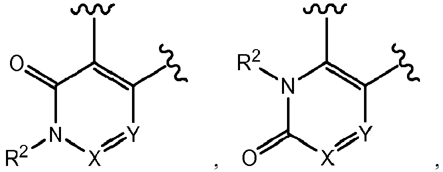 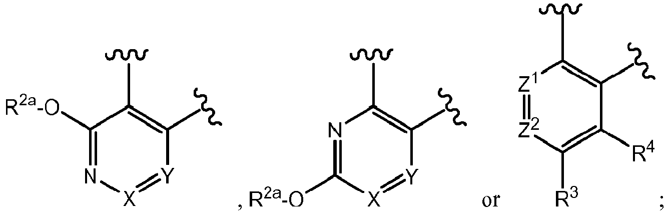
X and Y are each, independently, N or CR⁵;
Z¹ and Z² are each, independently, N, CR⁶, or NO; wherein at least one of Z¹ and Z² is other than CR⁶;
Ring B is 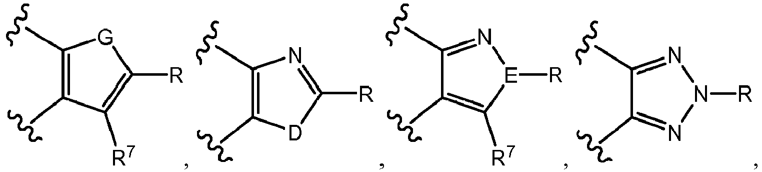 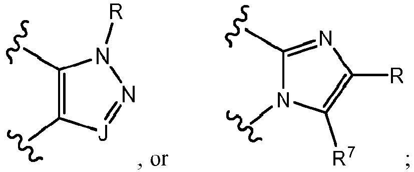
D is O, S, or NR⁸;
E is N or CR⁹;
G is O, S, or NR⁸;
J is N or CR⁷;
R is -W¹-W²-W³-W⁴;
W¹ is absent, C₁₋₆ alkyl, C₂₋₆ alkenyl, C₂₋₆ alkynyl, O, S, NR¹¹, CO, COO, CONR¹¹, SO, SO₂, SONR¹¹, SO₂NR¹¹, or NR¹¹CONR¹², wherein the C₁₋₆ alkyl, C₂₋₆ alkenyl, C₁₋₆ alkynyl are each optionally substituted by 1, 2 or 3 halo, OH, C₁₋₄ alkoxy, C₁₋₄ haloalkoxy, amino, C₁₋₄ alkylamino or C₂₋₈ dialkylamino;
W² is absent, C₁₋₆ alkyl, C₂₋₆ alkenyl, C₂₋₆ alkynyl, aryl, cycloalkyl, heteroaryl or heterocycloalkyl, wherein the C₁₋₆ alkyl, C₂₋₆ alkenyl, C₂₋₆ alkynyl, aryl, cycloalkyl, heteroaryl or heterocycloalkyl is optionally substituted by one or more halo, CN, NO₂, OH, =NH, =NOH, -NO-(C₁₋₄ alkyl), C₁₋₄ haloalkyl, C₁₋₄ alkoxy, C₁₋₄ haloalkoxy, amino, C₁₋₄ alkylamino or C₂₋₈ dialkylamino;
W³ is absent, C₁₋₆ alkyl, C₂₋₆ alkenyl, C₂₋₆ alkynyl, O, S, NR¹⁰, =N-, =N-O-, =N-O-(C₁₋₄ alkyl), O-(C₁₋₄ alkyl), S-(C₁₋₄ alkyl), NR¹⁰-(C₁₋₄ alkyl), (C₁₋₄ alkyl)-O-(C₁₋₄ alkyl), (C₁₋₄ alkyl)-S-(C₁₋₄ alkyl), (C₁₋₄ alkyl)-NR¹⁰-(C₁₋₄ alkyl), CO, COO, C(O)-(C₁₋₄ alkyl), C(O)O-(C₁₋₄ alkyl), C(O)-(C₁₋₄ alkyl)-C(O), NR¹⁰C(O)-(C₁₋₄ alkyl), C(O)NR¹⁰-(C₁₋₄ alkyl), NR¹⁰C(O)O-(C₁₋₄ alkyl), NR¹⁰C(O)O, CONR¹⁰, SO, SO₂, SONR¹⁰, SO₂NR¹⁰, or NR¹⁰CONR¹¹, wherein the C₁₋₆ alkyl, C₂₋₆ alkenyl, C₂₋₆ alkynyl are each optionally substituted by 1, 2 or 3 halo, OH, CN, C₁₋₄ alkoxy, C₁₋₄ haloalkoxy, amino, C₄ alkylamino or C₂₋₈ dialkylamino;
W⁴ is H, NR¹⁰R¹¹, CN, C₁₋₆ alkyl, C₂₋₆ alkenyl, C₂₋₆ alkynyl, aryl, cycloalkyl, heteroaryl or heterocycloalkyl, wherein the C₁₋₆ alkyl, C₂₋₆ alkenyl, C₂₋₆ alkynyl, aryl, cycloalkyl, heteroaryl or heterocycloalkyl is optionally substituted by 1, 2, 3, 4 or 5 halo, OH, CN, C₁₋₄ alkoxy, =NH, =NOH, =NO-(C₁₋₄ alkyl), C₁₋₄ haloalkyl, C₁₋₄ haloalkoxy, COOH, COO-(C₁₋₄ alkyl), amino, C₁₋₄ alkylamino or C₂₋₈ dialkylamino;
R^{1a}, R^{1b}, R^{1c} and R^{1d} are each, independently, H, halo,C₁₋₄ alkyl, C₂₋₄ alkenyl, C₂₋₄ alkynyl, C₁₋₄ haloalkyl, OH, C₁₋₄ alkoxy, C₁₋₄ haloalkoxy, CN, NO₂, C(O)-(C₁₋₄ alkyl), C(O)OH, C(O)O-(C₁₋₄ alkyl), C(O)NH₂, C(O)NH(C₁₋₄ alkyl), C(O)N(C₁₋₄ alkyl)₂, S(O)₂NH₂ S(O)₂NH(C₁₋₄ alkyl), S(O)₂N(C₁₋₄ alkyl)₂, S(O)₂-(C₁₋₄ alkyl), NH₂, NH(C₁₋₄ alkyl), or N(C₁₋₄ alkyl)₂;
R² is H, OH, C₁₋₆ alkyl, C₂₋₆ alkenyl, C₂₋₆ alkynyl, carbocyclyl, heterocyclyl, carbocyclylalkyl or heterocyclylalkyl;
R^{2a} is C₁₋₆ alkyl, C₂₋₆ alkenyl, C₂₋₆ alkynyl, aryl, heteroaryl, arylalkyl, heteroarylalkyl, cycloalkyl, cycloalkylalkyl, heterocycloalkyl or heterocycloalkylalkyl;
R³, R⁴, R⁵, and R⁶ are each, independently, H, C₁₋₆ alkyl, C₂₋₆ alkenyl, C₂₋₆ alkynyl, halo, C₁₋₄ haloalkyl, CN, NO₂, OR¹², SR¹², C(O)R¹³, C(O)OR¹², C(O)NR¹⁴R¹⁵, NR¹⁴R¹⁵, NR¹⁴CONHR¹⁵, NR¹⁴C(O)R¹³, NR¹⁴C(O)OR¹², S(O)R¹³, S(O)₂R¹³, S(O)NR¹⁴R¹⁵, SO₂NR¹⁴R¹⁵;
R⁷ is H, C₁₋₆ alkyl, C₂₋₆ alkenyl, C₂₋₆ alkynyl, halo, C₁₋₄ haloalkyl, OH, C₁₋₄ alkoxy, C₁₋₄ haloalkoxy, CN, NO₂, C(O)-(C₁₋₄ alkyl), C(O)OH, C(O)O-(C₁₋₄ alkyl), C(O)NH₂, C(O)NH(C₁₋₄ alkyl), C(O)N(C₁₋₄ alkyl)₂, S(O)₂NH₂, S(O)₂NH(C₁₋₄ alkyl), S(O)₂N(C₁₋₄ alkyl)₂, S(O)₂-(C₁₋₄ alkyl), NH₂, NH(C₁₋₄ alkyl), or N(C₁₋₄ alkyl)₂;
R⁸ is H, C₁₋₄ alkyl, C₂₋₄ alkenyl, C₂₋₄ alkynyl, OH or C₁₋₄ alkoxy;
R⁹ is H, halo, alkyl, C₁₋₄ haloalkyl, C₂₋₄ alkenyl, C₂₋₄ alkynyl, OH, C₁₋₄ alkoxy or C₁₋₄ haloalkoxy;
R¹⁰ and R¹¹ are each, independently, H, C₁₋₆ alkyl, C₂₋₆ alkenyl, C₂₋₆ alkynyl, aryl, cycloalkyl, arylalkyl, cycloalkylalkyl, COR^{a}, SOR^{a}, or SO₂R^{a} wherein each of the C₁₋₆ alkyl, C₂₋₆ alkenyl, C₂₋₆ alkynyl, aryl, cycloalkyl, arylalkyl, or cycloalkylalkyl is optionally substituted by 1, 2 or 3 substitutents sleeted from halo, C₁₋₄ alkyl, C₁₋₄ haloalkyl, OH, C₁₋₄ alkoxy, C₁₋₄ haloalkoxy, amino, C₁₋₄ alkylamino, C₂₋₈ dialkylamino, aminocarbonyl, C₁₋₄ alkylaminocarbonyl, or C₂₋₈ dialkylaminocarbonyl, CN and NO₂;
or R¹⁰ and R¹¹ together with the N atom to which they are attached form a heterocycloalkyl group optionally substituted by 1, 2 or 3 substitutents slected from halo, C₁₋₄ alkyl, C₁₋₄ haloalkyl, OH, C₁₋₄ alkoxy, C₁₋₄ haloalkoxy, amino, C₁₋₄ alkylamino, C₂₋₈ dialkylamino, aminocarbonyl, C₁₋₄ alkylaminocarbonyl, or C₂₋₈ dialkylaminocarbonyl;
R¹² and R¹³ arc each, independently, H, C₁₋₆ alkyl, C₁₋₆ haloalkyl, C₂₋₆ alkenyl, C₂₋₆ alkynyl, aryl, cycloalkyl, arylalkyl, or cycloalkylalkyl;
R¹⁴ and R¹⁵ are each, independently, H, C₁₋₆ alkyl, C₁₋₆ haloalkyl, C₂₋₆ alkenyl, C₂₋₆ alkynyl, aryl, cycloalkyl, arylalkyl, or cycloalkylalkyl;
or R¹⁴ and R¹⁵ together with the N atom to which they are attached form a heterocyclyl group;
R^{a} is H, C₁₋₆ alkyl, C₁₋₆ haloalkyl, C₂₋₆ alkenyl, C₂₋₆ alkynyl, aryl, cycloalkyl, arylalkyl, cycloalkylalkyl, heteroaryl, heterocycloalkyl, heteroarylalkyl, heterocycloalkylalkyl, NH₂, NH(C₁₋₆ alkyl), N(C₁₋₆ alkyl)₂, NH(carbocyclyl), N(carbocyclyl)₂, NH(carbocyclylalkyl) or N(carbocyclylalkyl)₂;
with the proviso that when Ring A is: 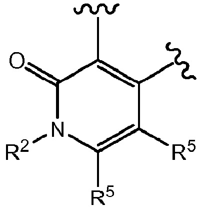
Ring B is: 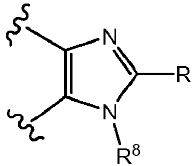
D¹ is CR^{1a};
D² is N or CR^{1b};
D¹ is CR¹⁰; and
D⁴ is CR^{1d};
then W¹ is O, S, NR¹¹, SO, SO₂, SONR¹¹, SO₂NR¹¹, or NR¹¹CONR¹²,

According to some embodiments, Ring A is

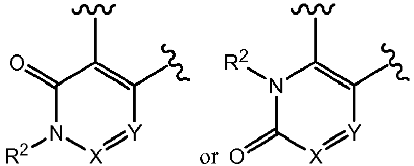

In some embodiments, both X and Y are CR⁵.

In some embodiments, both X and Y are N.

In some embodiments, one of X and Y is N and the other is CR⁵.

In some embodiments, X is CR⁵ and Y is N.

In some embodiments, X is N and Y is CR⁵.

In some embodiments, R² is H.

In some embodiments, R² is H, X is CH and Y is CH.

In some embodiments, Ring A is

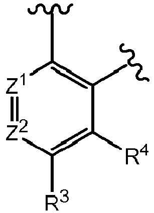

In some embodiments, Z¹ is NO or Z² is NO.

In some embodiments, Z¹ is NO and Z² is CR⁶.

In some embodiments, Z² is NO and Z¹ is CR⁶.

In some embodiments, Ring A is

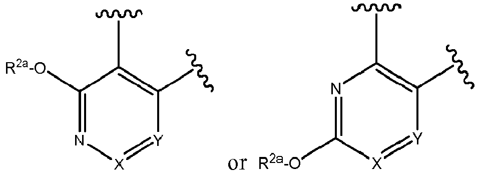

In some embodiments, R^{2a} is C₁₋₆ alkyl.

In some embodiments, R^{2a} is methyl.

In some embodiments, at least one of X and Y is N.

In some embodiments, Ring B is

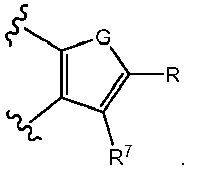

In some embodiments, G is O or S.

In some embodiments, G is NR⁸.

In some embodiments, G is NH.

In some embodiments, R is H, C₁₋₆ alkyl, aryl, heteroaryl, cycloalkyl, heterocycloalkyl, or NR¹⁰R¹¹

In some embodiments, R is H, C₁₋₆ alkyl or NR¹⁰R¹¹.

In some embodiments, R is O-W²-W³-W⁴ , S-W²-W³-W⁴ or NR¹¹-W²-W³-W⁴.

In some embodiments, Ring B is

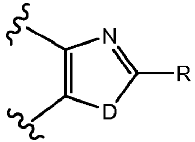

In some embodiments, D is S.

In some embodiments, D is O.

In some embodiments, D is NR⁸.

In some embodiments, R is H, C₁₋₆ alkyl, aryl, heteroaryl, cycloalkyl, heterocycloalkyl, or NR¹⁰R¹¹

In some embodiments, R is H, C₁₋₆ alkyl or NR¹⁰R¹¹.

In some embodiments, R is (C₁₋₆ alkyl)-W²-W³-W⁴, O-W²-W³-W⁴, S-W²-W³-W⁴, NR¹¹-W²-W³-W⁴, or -W²-W³-W⁴.

In some embodiments, D is S or O and R is O-W²-W³-W⁴ , S-W²-W³-W⁴ or NR¹¹-W²-W³-W⁴.

In some embodiments, D is S and R is O-W²-W³-W⁴ , S-W²-W³-W⁴ or NR¹¹-W²-W³-W⁴.

In some embodiments, Ring B is

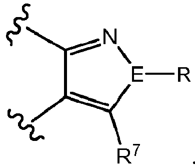

In some embodiments, E is N.

In some embodiments, R⁷ is H.

In some embodiments, R is H, C₁₋₆ alkyl, aryl, heteroaryl, cycloalkyl, heterocycloalkyl, or NR¹⁰R¹¹

In some embodiments, R is H, C₁₋₆ alkyl or NR¹⁰R¹¹.

In some embodiments, E is CR⁹ and R is O-W²-W³-W⁴ , S-W²-W³-W⁴ or NR¹¹-W²-W³-W⁴.

In some embodiments, Ring B is

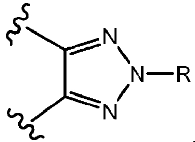

In some embodiments, R is H, C₁₋₆ alkyl, aryl, heteroaryl, cycloalkyl, heterocycloalkyl.

In some embodiments, R is H or C₁₋₆ alkyl.

In some embodiments, R is (C₁₋₆ alkyl)-W²-W³-W⁴, CO-W²-W³-W⁴ , COO-W²-W³-W⁴, CONR¹¹-W²-W³-W⁴ or SO₂-W²-W³-W⁴.

In some embodiments, Ring B is:

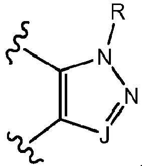

In some embodiments, J is N.

In some embodiments, J is CR⁷.

In some embodiments, R is H, C₁₋₆ alkyl, aryl, heteroaryl, cycloalkyl, heterocycloalkyl.

In some embodiments, R is H or C₁₋₆ alkyl.

In some embodiments, R is (C₁₋₆alkyl)-W²-W³-W⁴, CO-W²-W³-W⁴ , COO-W²-W³-W⁴, CONR¹¹-W²-W³-W⁴ or SO₂-W²-W³-W⁴.

In some embodiments, Ring B is

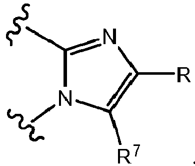

In some embodiments, R is C₁₋₆alkyl, C₂₋₆alkenyl, C₂₋₆alkynyl, cycloalkyl, or heterocycloalkyl, each optionally substituted by 1, 2, 3, 4 or 5 halo, OH, CN, C₁₋₄alkoxy, =NH, =NOH, =NO-(C₁₋₄ alkyl), C₁₋₄ haloalkyl, C₁₋₄ haloalkoxy, COOH, COO-(C₁₋₄ alkyl), amino, C₁₋₄ alkylamino or C₂₋₈ dialkylamino;

In some embodiments, R is cycloalkyl or heterocycloalkyl, each optionally substituted by 1, 2, 3, 4 or 5 halo, OH, CN, C₁₋₄alkoxy, C₁₋₄ haloalkyl, C₁₋₄ haloalkoxy, COOH, COO-(C₁₋₄ alkyl), amino, C₁₋₄ alkylamino or C₂₋₈ dialkylamino.

In some embodiments, R is 5-, 6-, or 7-membered cycloalkyl or 5-, 6-, or 7-membered heterocycloalkyl, each optionally substituted by 1 or 2 halo, OH, CN, C₁₋₄alkoxy, C₁₋₄ halo alkyl, or C₁₋₄ haloalkoxy.

In some embodiments, D¹ is CR^{1a}, D² is CR^{1b}, D³ is CR^{1c} and D⁴ is CR^{1d}.

In some embodiments, D² is CR^{1b}.

In some embodiments, D² is CR^{1b} and CR^{1b} is H, C₁₋₄ alkyl or halo.

In some embodiments, D² is CR^{1b} and CR^{1b} is H or halo.

In some embodiments, D² is CR^{1b} and CR^{1b} is F, Cl, Br or I.

In some embodiments, D² is CR^{1b}; CR^{1b} is F, Cl, Br or I; D¹ is CH, D³ is CH; and D⁴ is CH.

In some embodiments, D² is CF; D¹ is CH, D³ is CH; and D⁴ is CH.

In some embodiments, at least one of D¹, D², D³, and D⁴ is N.

In some embodiments, at least one of D¹, D³, and D⁴ is N.

In some embodiments, not more than 2 of D¹, D², D³, and D⁴ are N.

In some embodiments, at least one of D¹, D², D³, and D⁴ is NO.

In some embodiments, at least one of D¹, D³, and D⁴ is NO.

In some embodiments, the agent is selected from compounds of Formula IVa: 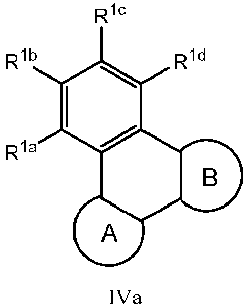 and pharmaceutically acceptable salts thereof.

In some embodiments, R^{1a}, R^{1b}, R^{1c} and R^{1d} are each, independently, H, C₁₋₄ alkyl, C₂₋₄ alkenyl, C₂₋₄ alkynyl, halo, C₁₋₄ haloalkyl, OH, C₁₋₄ alkoxy, C₁₋₄ haloalkoxy, CN, NO₂, NH₂, NH(C₁₋₄ alkyl), or N(C₁₋₄ alkyl)₂.

In some embodiments, R is other than H.

In some embodiments, R is -W¹-W²-W³-W⁴; and W¹ is absent, C₁₋₆ alkyl, O, S, NR¹¹, SO, or SO₂.

In some embodiments, R is -W¹-W²-W³-W⁴; and W¹ is absent, and W² is aryl, cycloalkyl, heteroaryl or heterocycloalkyl, each optionally substituted by 1, 2, 3 or 4 halo, CN, NO₂, OH, =NH, =NOH, =NO-(C₁₋₄ alkyl), C₁₋₄ haloalkyl, C₁₋₄ alkoxy, C₁₋₄ haloalkoxy, amino, C₁₋₄ alkylamino or C₂₋₈ dialkylamino.

In some embodiments:
R is -W¹-W²-W³-W⁴;
W¹ is absent or C₁₋₆ alkyl optionally substituted by 1, 2 or 3 halo, OH, C₁₋₄ alkoxy, C₁₋₄ haloalkoxy, amino, C₁₋₄ alkylamino or C₂₋₈ dialkylamino;
W² is absent; and
W³ is O, S, NR¹⁰, CO, or COO.

In some embodiments, R is H, C₁₋₆ alkyl, C₂₋₆ alkenyl, C₂₋₆ alkynyl, O-W²-W³-W⁴, S-W²-W³-W⁴, or NR¹¹-W²-W³-W⁴, wherein the C₁₋₆ alkyl, C₂₋₆alkenyl, C₂-₆ alkynyl are each optionally substituted by 1, 2 or 3 halo, OH, C₁₋₄ alkoxy, C₁₋₄ haloalkoxy, amino, C₁₋₄ alkylamino or C₂₋₈ dialkylamino.

In some embodiments, R is W⁴.

In some embodiments, R is -W³-W⁴.

In some embodiments, R is -W²-W³-W⁴.

In some embodiments, R is -W¹-W⁴.

In some embodiments, R is -O-W²-W³-W⁴

In some embodiments, R is -S-W²-W³- W⁴

In some embodiments, R is -NR¹¹-W²-W³-W⁴.

In some embodiments, R is NR¹⁰R¹¹.

In some embodiments, R is aryl, cycloalkyl, heteroaryl or heterocycloalkyl each optionally substituted by 1, 2, 3, 4 or 5 halo, OH, CN, C₁₋₄ alkoxy, =NH, =NOH, =NO-(C₁₋₄ alkyl), C₁₋₄ haloalkyl, C₁₋₄ haloalkoxy, COOH, COO-(C₁₋₄ alkyl), amino, C₁₋₄ alkylamino or C₂₋₈ dialkylamino.

In some embodiments, W¹ is O, S, NR¹¹, CO, COO, CONR¹¹, SO, SO₂, SONR¹¹, SO₂NR¹¹, or NR¹¹CONR¹².

In some embodiments, W¹ is C₁₋₆ alkyl optionally substituted by one or more halo, CN, NO₂, OH, =NH, =NOH, =NO-(C₁₋₄ alkyl), C₁₋₄ haloalkyl, C₁₋₄alkoxy, C₁₋₄haloalkoxy, amino, C₁₋₄ alkylamino or C₂₋₈ dialkylamino.

In some embodiments, W¹ is absent.

In some embodiments, W² is aryl, cycloalkyl, heteroaryl or heterocycloalkyl, each optionally substituted by one or more halo, CN, NO₂, OH, -NH, -NOH, =NO-(C₁₋₄ alkyl), C₁₋₄ haloalkyl, C₁₋₄ alkoxy, C₁₋₄ haloalkoxy, amino, C₁₋₄ alkylamino or C₂₋₈ dialkylamino.

In some embodiments, W² is absent.

In some embodiments, W³ is O, S, NR¹⁰, =N-, =N-O-, =N-O-(C₁₋₄ alkyl), O-(C₁₋₄ alkyl), S-(C₁₋₄ alkyl), NR¹⁰-(C₁₋₄ alkyl), (C₁₋₄ alkyl)-O-(C₁₋₄ alkyl), (C₁₋₄ alkyl)-S-(C₁₋₄ alkyl), (C₁₋₄ alkyl)-NR¹⁰-( C₁₋₄ alkyl), CO, COO, C(O)-(C₁₋₄ alkyl), C(O)O-(C₁₋₄ alkyl), C(O)-(C₁₋₄ alkyl)-C(O), NR¹⁰C(O)-(C₁₋₄ alkyl), C(O)NR¹⁰-(C₁₄ alkyl), NR¹⁰C(O)O-(C₁₋₄ alkyl), NR¹⁰C(O)O, CONR¹⁰, SO, SO₂, SONR¹⁰, SO₂NR¹⁰, or NR¹⁰CONR¹¹.

In some embodiments, W³ is C₁₋₆ alkyl optionally substituted by 1, 2 or 3 halo, OH, CN, C₁₋₄ alkoxy, C₁₋₄ haloalkoxy, amino, C₁₋₄ alkylamino or C₂₋₈ dialkylamino.

In some embodiments, W³ is absent.

In some embodiments, W⁴ is aryl, cycloalkyl, heteroaryl or heterocycloalkyl, each optionally substituted by 1, 2, 3, 4 or 5 halo, OH, CN, C₁₋₄ alkoxy, =NH, =NOH, =NO-(C₁₋₄ alkyl), C₁₋₄ haloalkyl, C₁₋₄ haloalkoxy, COOH, COO-(C₁₋₄ alkyl), amino, C₁₋₄ alkylamino or C₂₋₈ dialkylamino.

In some embodiments, W⁴ is C₁₋₆ alkyl optionally substituted by 1, 2, 3, 4 or 5 halo, OH, CN, C₁₋₄alkoxy, =NH, =NOH, =NO-(C₁₋₄ alkyl), C₁₋₄ haloalkyl, C₁₋₄ haloalkoxy, COOH, COO-(C₁₋₄ alkyl), amino, C₁₋₄ alkylamino or C₂₋₈ dialkylamino.

In some embodiments, W⁴ is H, NR¹⁰R¹¹ or CN.

In some embodiments:
Ring B is 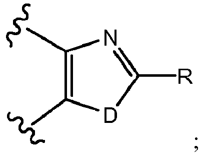 and
R is H, C₁₋₆alkyl, C₂₋₆alkenyl, C₂₋₆ alkynyl, (C₁₋₆ alkyl)-W²-W³-W⁴, O-W²-W³-W⁴, S-W²-W³-W⁴, NR¹¹-W²-W³-W⁴ or -W²-W³-W⁴ wherein the C₁₋₆ alkyl, C₂₋₆ alkenyl, C₂₋₆ alkynyl are each optionally substituted by 1, 2 or 3 halo, OH, C₁₋₄ alkoxy, C₁₋₄ haloalkoxy, amino, C₁₋₄ alkylamino or C₂₋₈ dialkylamino.

In some embodiments:
Ring B is 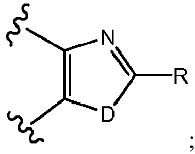 and
R is S-W²-W³-W⁴, S(O)-W²-W³-W⁴ or S(O)₂-W²-W³-W⁴.

In some embodiments:
Ring B is 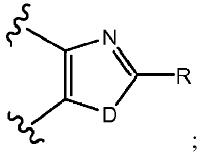
D is NR⁸; and
R is S-W²-W³-W⁴, S(O)-W²-W³-W⁴ or S(O)₂-W²-W³-W⁴.

In some embodiments:
Ring B is 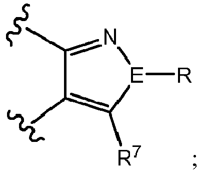
E is N; and
R is H, (C₁₋₆ alkyl)-W²-W³-W⁴, (C₁₋₆ alkenyl)-W²-W³-W⁴ or (C₁₋₆ alkynyl)-W²-W³-W⁴.

In some embodiments:
Ring B is 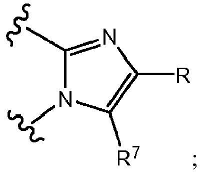
R⁷ is H; and
R is C₁₋₆alkyl, C₂₋₆ alkenyl, C₂₋₆alkynyl, cycloalkyl, or heterocycloalkyl, each optionally substituted by 1, 2, 3, 4 or 5 halo, OH, CN, C₁₋₄ alkoxy, =NH, -NOH, =NO-(C₁₋₄ alkyl), C₁₋₄ haloalkyl, C₁₋₄ haloalkoxy, COOH, COO-(C₁₋₄ alkyl), amino, C₁₋₄ alkylamino or C₂₋₈ dialkylamino.

In some embodiments:
Ring B is 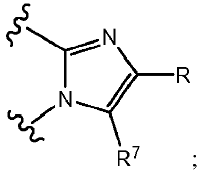
R⁷ is H; and
R is cycloalkyl, or heterocycloalkyl, each optionally substituted by 1, 2, 3, 4 or 5 halo, OH, CN, C₁₋₄ alkoxy, C₁₋₄ haloalkyl, C₁₋₄ haloalkoxy, COOH, COO-(C₁₋₄ alkyl), amino, C₁₋₄ alkylamino or C₂₋₈ dialkylamino.

In some embodiments, the agent is selected from compounds of Formula IVb: 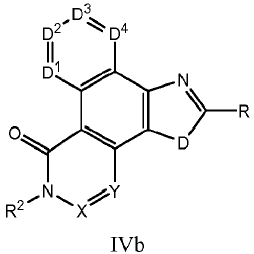 and pharmaceutically acceptable salts thereof.

In some embodiments, the agent is selected from compounds of Formula IVc: 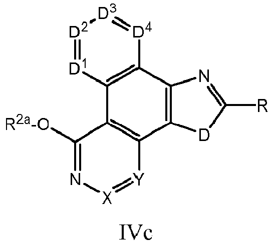 and pharmaceutically acceptable salts thereof.

In some embodiments, the agent is selected from compounds of Formula IVd: 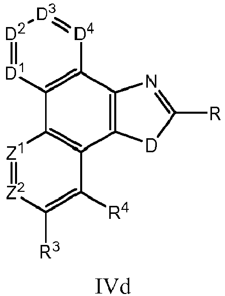 and pharmaceutically acceptable salts thereof.

In some embodiments, the agent is selected from compounds of Formula IVe: 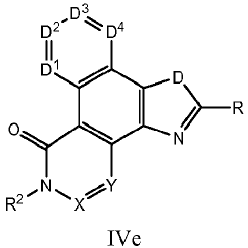 and pharmaceutically acceptable salts thereof.

In some embodiments, the agent is selected from compounds of Formula IVf: 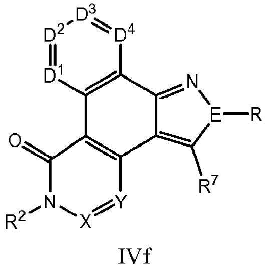 and pharmaceutically acceptable salts thereof

In some embodiments, the agent is selected from compounds of Formula IVg: 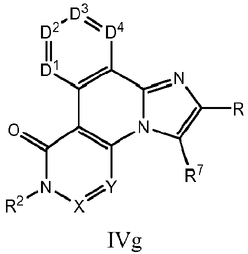 and pharmaceutically acceptable salts thereof.

In some embodiments, the agent is selected from:
9-Fluoro-2-piperidin-1-ylbenzo[h][1,3]thiazolo[5,4-f]isoquinolin-7(6H)-one;
2-(tert-Butylamino)-9-fluorobenzo[h][1,3]thiazolo[5,4-f]isoquinolin-7(6H)-one;
9-Fluoro-2-[(3-methoxypropyl)amino]benzo[h][1,3]thiazolo[5,4-f]isoquillolin-7(6H)-one;
9-Fluoro-2-(4-methylpiperazin-1-yl)benzo[h][1,3]thiazolo[5,4-f]isoquinolin-7(6H)-one;
2-(Dimethylamino)-9-fluorobenzo[h][1,3]thiazolo[5,4-f]isoquinolin-7(6H)-one;
2-(Benzylamino)-9-fluorobenzo[h][1,3]thiazolo[5,4-f]isoquinolin-7(6H)-one;
2-Anilino-9-fluorobenzo[h][1,3]thiazolo[5,4-f]isoquinolin-7(6H)-one;
9-Fluoro-2,6-dihydro-7H-benzo[h]pyrazolo[4,3-f]isoquinolin-7-one;
9-Fluoro-2-piperidin-1-ylbenzo[h][1,3]oxazolo[5,4-f]isoquinolin-7(6H)-one;
2-tert-Butyl-9-fluoro-3H-benzo[h]imidazo[4,5-f]quinoline;
2-tert-Butyl-9-fluoro-3H-benzo[h]imidazo[4,5-f]quinoline 7-oxide;
2-tert-Butyl-9-fluoro-7-methoxy-3H-benzo[f]imidazo[4,5-h]phthalazin-3-ol trifluoroacetic acid;
2-tert-Butyl-9-fluoro-7-methoxy-3H-1,3,5,6-tetraaza-cyclopenta[1]phenanthrene;
2-tert-Butyl-9-fluoro-3,6-dihydro-1,3,5,6-tetraaza-cyclopenta[1]phenanthren-7-one;
2-tert-Butyl-9-fluoro-3H-benzo[f]imidazo[4,5-h]phthalazine-3,7-diol;
2-tert-Butyl-9-fluoro-7-methoxy-3H-benzo[f]imidazo[4,5-h]quinazolin-5-amine;
5-Amino-2-tert-butyl-9-fluoro-3H-benzo[f]imidazo[4,5-h]quinazolin-7-ol;
2-tert-Butyl-9-fluoro-7-methoxy-3H-benzo[f]imidazo[4,5-h]quinazoline;
2-tert-Butyl-9-fluoro-3H-benzo[f]imidazo[4,5-h]quinazolin-7-ol;
2-tert-Butyl-9-fluoro-3H-benzo[h]imidazo[4,5-f]isoquinolin-3-ol;
2-tert-Butyl-9-fluoro-3H-benzo[h]imidazo[4,5-f]isoquinoline;
2-tert-Butyl-9-fluoro-3H-benzo[h]imidazo[4,5-f]isoquinoline 6-oxide;
9-Fluoro-2-[hydroxy(pyridin-3-yl)methyl]benzo[h][1,3]thiazolo[5,4-f]isoquinolin-7(6H)-one;
9-Fluoro-2-[4-(1H-imidazol-4-ylcarbonyl)piperazin-1-yl]benzo[h][l,3]thiazolo[5,4-f]isoquinolin-7(6H)-one;
2-Ethyl-9-fluoro-3,6-dihydro-7H-benzo[f]imidazo [4,5-h]quinazolin-7-one;
trans-2-(9-fluoro-7-oxo-6,7-dihydro-3H-benzo[f]imidazo[4,5-h]quinazolin-2-yl)cyclopropanecarboxamide;
1-(9-fluoro-7-oxo-6,7-dihydro-3H-benzo[f]imidazo[4,5-h]quinazolin-2-yl)cyclopropanecarboxylic acid;
2-[2-(dimethylamino)-1,1-dimethylethyl]-9-fluoro-3,6-dihydro-7H-benzo[f]imidazo[4,5-h]quinazolin-7-one;
4-cthyl-4-(9-fluoro-7-oxo-6,7-dihydro-3H-bcnzo[f]imidazo[4,5-h]quinazolin-2-yl)hexanenitrile;
4-ethyl-4-(9-fluoro-7-oxo-6,7-dihydro-3H-benzo[f]imidazo[4,5-h]quinazolin-2-yl)hexanamide;
2-(4-amino-1,1-dimethylbutyl)-9-fluoro-3,6-dihydro-7H-benzo[f]imidazo[4,5-h]quinazolin-7-one;
benzyl [1-(9-fluoro-7-oxo-6,7-dihydro-3H-benzo[f]imidazo[4,5-h]quinazolin-2-yl)-1-methylethyl]carbamate ;
benzyl [2-(9-fluoro-7-oxo-6,7-dihydro-3H-benzo[f]imidazo[4,5-h]quinazolin-2-yl)-2-methylpropyl]carbamate;
[2-(9-fluoro-7-oxo-6,7-dihydro-3H-benzo[f]imidazo[4,5-h]quinazolin-2-yl)-2-methylpropoxy]acetonitrile;
2-(1-amino-l-methylethyl)-9-fluoro-3,6-dihydro-7H-benzo[f]imidazo[4,5-h]quinazolin-7-one;
4-(9-fluoro-7-oxo-6,7-dihydro-3H-benzo[f]imidazo[4,5-h]quinazolin-2-yl)butanenitrile;
N-[1-(9-fluoro-7-oxo-6,7-dihydro-3H-benzo[f]imidazo[4,5-h]quinazolin-2-yl)-1-methylethyl]acetamide;
benzyl 4-(9-fluoro-7-oxo-6,7-dihydro-3H-benzo[f]imidazo[4,5-h]quinazolin-2-yl)piperidine-1-carboxylate;
3-(9-fluoro-7-oxo-6,7-dihydro-3H-benzo[f]imidazo[4,5-h]quinazolin-2-yl)propanenitrile;
N-[2-(9-fluoro-7-oxo-6,7-dihydro-3H-benzo[f]imidazo[4,5-h]quinazolin-2-yl)-2-methylpropyl]urea;
4-(9-fluoro-7-oxo-6,7-dihydro-3H-benzo[f]imidazo[4,5-h]quinazolin-2-yl)-4-methylpentanenitrile;
2-(1-acetylpiperidin-4-yl)-9-fluoro-3,6-dihydro-7H-benzo[f]imidazo[4,5-h]quinazolin-7-one;
9-fluoro-2-(trans-4-hydroxycyclohexyl)-3,6-dihydro-7H-benzo[f]imidazo[4,5-h]quinazolin-7-one;
9-fluoro-2-(cis-4-hydroxycyclohexyl)-3,6-dihydro-7H-benzo[f]imidazo[4,5-h]quinazolin-7-one;
3-(9-fluoro-7-oxo-6,7-dihydro-3H-benzo[f]imidazo[4,5-h]quinazolin-2-yl)-3-methylbutanenitrile;
2-(Ethylthio)-9-fluoro-3,6-dihydro-7H-benzo[h]imidazo[4,5-f]isoquinolin-7-one;
2-(Ethylsulfinyl)-9-fluoro-3,6-dihydro-7H-benzo[h]imidazo[4,5-f]isoquinolin-7-one;
2-(Ethylsulfonyl)-9-fluoro-3,6-dihydro-7H-benzo[h]imidazo[4,5-f]isoquinolin-7-one;
2-[(9-Fluoro-7-oxo-6,7-dihydro-3H-benzo[h]imidazo[4,5-f]isoquinolin-2-yl)thio]-4-hydroxybutanamide;
9-Fluoro-2-[(2-hydroxycyclohexyl)thio]-3,6-dihydro-7H-benzo[h]imidazo[4,5-f]isoquinolin-7-one;
2-[(3,5-Dimethyl-1H-pyrazol-4-yl)thio]-9-fluoro-3,6-dihydro-7H-benzo[h]imidazo[4,5-f]isoquinolin-7-one;
9-Fluoro-2-{[(5-hydroxy-1H-pyrazol-3-yl)methyl]thio}-3,6-dihydro-7H-benzo[h]imidazo[4,5-f]isoquinolin-7-one;
9-Fluoro-2-[(2,3,5,6-tetrafluoropyridin-4-yl)thio]-3,6-dihydro-7H-benzo[h]imidazo[4,5-f]isoquinolin-7-one;
2-[(2,6-Diamino-3,5-difluoropyridin-4-yl)thio]-9-fluoro-3,6-dihydro-7H-benzo[h]imidazo [4,5-f] isoquinolin-7-one;
2-[(2-Amino-3,5,6-trifluoropyridin-4-yl)thio]-9-fluoro-3,6-dihydro-7H-benzo[h]imidazo[4,5-f]isoquinolin-7-one;
2-(benzylthio)-9-fluoro-3,6-dihydro-7H-benzo[h]imidazo[4,5-f]isoquinolin-7-one;
2-(benzylsulfinyl)-9-fluoro-3,6-dihydro-7H-benzo[h]imidazo[4,5-f]isoquinolin-7-one;
2-(benzylsulfonyl)-9-fluoro-3,6-dihydro-7H-benzo[h]imidazo[4,5-f]isoquinolin-7-one;
3-{[(9-fluoro-7-oxo-6,7-dihydro-3H-benzo[h]imidazo[4,5-f]isoquinolin-2-yl)thio]methyl}benzonitrile;
9-fluoro-2-[(2-methoxyethyl)thio]-3,6-dihydro-7H-benzo[h]imidazo[4,5-f]isoquinolin-7-one;
9-fluoro-2-[(2-oxotetrahydrofuran-3-yl)thio]-3,6-dihydro-7H-benzo[h]imidazo[4,5-f]isoquinolin-7-one ;
2-{2-[(9-fluoro-7-oxo-6,7-dihydro-3H-benzo[h]imidazo[4,5-f]isoquinolin-2-yl)thio]ethyl}-1H-isoindole-1,3(2H)-dione;
9-fluoro-2-[(pyridin-3-ylmethyl)thio]-3,6-dihydro-7H-benzo[h]imidazo[4,5-f]isoquinolin-7-one;
2-[(1-ethylpropyl)thio]-9-fluoro-3,6-dihydro-7H-benzo[h]imidazo[4,5-f]isoquinolin-7-one;
2-[(1-ethylpropyl)sulfinyl]-9-fluoro-3,6-dihydro-7H-benzo[h]imidazo[4,5-f]isoquinolin-7-one;
2-[(1-ethylpropyl)sulfonyl]-9-fluoro-3,6-dihydro-7H-benzo[h]imidazo[4,5-f]isoquinolin-7-one;
2-[(3,5-dimethoxybenzyl)thio]-9-fluoro-3,6-dihydro-7H-benzo [h]imidazo [4,5-f]isoquinolin-7-one;
ethyl [(9-fluoro-7-oxo-6,7-dihydro-3H-benzo[h]imidazo[4,5-f]isoquinolin-2-yl)thio]acetate;
9-fluoro-2-(isopropylthio)-3,6-dihydro-7H-benzo[h]imidazo[4,5-f]isoquinolin-7-one ;
[(9-fluoro-7-oxo-6,7-dihydro-3H-benzo[h]imidazo[4,5-f]isoquinolin-2-yl)thio]acetic acid;
9-fluoro-2-[(l-phenylethyl)thio]-3,6-dihydro-7H-benzo[h]imidazo[4,5-f]isoquinolin-7-one;
[(9-fluoro-7-oxo-6,7-dihydro-3H-benzo[h]imidazo[4,5-flisoquinolin-2-yl)thio]acetonitrile;
[(9-fluoro-7-oxo-6,7-dihydro-3H-benzo[h]imidazo [4,5-f]isoquinolin-2-yl)sulfinyl]acctonitrile;
2-[(9-fluoro-7-oxo-6,7-dihydro-3H-benzo[h]imidazo[4,5-f]isoquinolin-2-yl)thio]-N-phenylpropanamide;
9-fluoro-2-[(3-hydroxypropyl)thio]-3,6-dihydro-7H-benzo[h]imidazo[4,5-f]isoquinolin-7-one;
9-fluoro-2-[(3,3,3-trifluoro-2-oxopropyl)thio]-3,6-dihydro-7H-benzo[h]imidazo[4,5-f]isoquinolin-7-one;
ethyl 4-[(9-fluoro-7-oxo-6,7-dihydro-3H-benzo[h]imidazo[4,5-f]isoquinolin-2-yl)thio]butanoate;
2-[(2-aminoethyl)thio]-9-fluoro-3,6-dihydro-7H-benzo[h]imidazo[4,5-f]isoquinolin-7-one;
2-[(cyclohexylmethyl)thio]-9-fluoro-3,6-dihydro-7H-benzo[h]imidazo[4,5-f]isoquinolin-7-one ;
2-[(9-fluoro-7-oxo-6,7-dihydro-3H-benzo[h]imidazo[4,5-f]isoquinolin-2-yl)thio]acetamide ;
2-(cyclohexylthio)-9-fluoro-3,6-dihydro-7H-benzo[h]imidazo [4,5-f]isoquinolin-7-one;
2-[(2,2-dimethoxyethyl)thio]-9-fluoro-3,6-dihydro-7H-benzo[h]imidazo[4,5-f]isoquinolin-7-one ;
2-[(3,3-dimethyl-2-oxobutyl)thio]-9-fluoro-3,6-dihydro-7H-benzo[h]imidazo[4,5-f]isoquinolin-7-one ;
ethyl2-[(9-fluoro-7-oxo-6,7-dihydro-3H-benzo[h]imidazo[4,5-f]isoquinolin-2-yl)thio]propanoate ;
4-[(9-fluoro-7-oxo-6,7-dihydro-3H-benzo[h]imidazo[4,5-f]isoquinolin-2-yl)thio]butanenitrile;
ethyl 2-[(9-fluoro-7-oxo-6,7-dihydro-3H-benzo[h]imidazo[4,5-f]isoquinolin-2-yl)thio]butanoate;
2-[(9-fluoro-7-oxo-6,7-dihydro-3H-benzo[h]imidazo[4,5-f]isoquinolin-2-yl)thio]-4-hydroxy-N,N-dimethylbutanamide ;
methyl 3-{[(9-fluoro-7-oxo-6,7-dihydro-3H-benzo[h]imidazo [4,5-f]isoquinolin-2-yl)thio]methyl}benzoate ;
9-fluoro-2-[(tetrahydro-2H-pyran-2-ylmethyl)thio]-3,6-dihydro-7H-benzo[h]imidazo[4,5-f]soquinolin-7-one;
4-[(9-fluoro-7-oxo-6,7-dihydro-3H-benzo[h]imidazo[4,5-f]isoquinolin-2-yl)thio]butanoic acid;
4-[(9-fluoro-7-oxo-6,7-dihydro-3H-benzo[h]imidazo[4,5-f]isoquinolin-2-yl)thio]butanamide;
9-fluoro-7-oxo-6,7-dihydro-3H-benzo[h]imidazo[4,5-f]isoquinolin-2-yl thiocyanate;
2-[(9-fluoro-7-oxo-6,7-dihydro-3H-benzo[h]imidazo[4,5-f]isoquinolin-2-yl)thio]propanenitrile;
9-fluoro-2-[(2-hydroxy-2-methylpropyl)thio]-3,6-dihydro-7H-benzo[h]imidazo[4,5-f]isoquinolin-7-one;
9-fluoro-2-[(3-hydroxy-2,2-dimethylpropyl)thio]-3,6-dihydro-7H-benzo[h]imidazo[4,5-f]isoquinolin-7-onc;
9-fluoro-2-[(2-oxocyclopentyl)thio]-3,6-dihydro-7H-benzo[h]imidazo[4,5-f]isoquinolin-7-one;
2-[(1,3-dioxolan-2-ylmethyl)thio]-9-fluoro-3,6-dihydro-7H-benzo[h]imidazo[4,5-f]isoquinolin-7-one;
N-ethyl-2-[(9-fluoro-7-oxo-6,7-dihydro-3H-benzo[h]imidazo[4,5-f]isoquinolin-2-yl)thio]-4-hydroxybutanamide;
9-fluoro-2-[(2-hydroxyethyl)thio]-3,6-dihydro-7H-benzo[h]imidazo[4,5-f]isoquinolin-7-one;
9-fluoro-2-(piperidin-4-ylthio)-3,6-dihydro-7H-benzo[h]imidazo[4,5-f]isoquinolin-7-one;
3-[(9-fluoro-7-oxo-6,7-dihydro-3H-benzo[h]imidazo[4,5-f]isoquinolin-2-yl)thio]propanenitrile ;
9-fluoro-2-[(3-oxocyclohex-1-en-1-yl)thio]-3,6-dihydro-7H-phenanthro[9,10-d]imidazol-7-one ;
9-fluoro-2-(pyridin-4-ylthio)-3,6-diliydro-7H-benzo[h]imidazo[4,5-flisoquinolin-7-one;
9-fluoro-2-(1-pyrazol-4-ylthio)-3,6-dihydro-7H-benzo[h]imidazo[4,5-f]isoquinolin-7-one;
9-fluoro-2-[(2-hydroxy-2-methylpropyl)sulfinyl]-3,6-dihydro-7H-benzo[h]imidazo[4,5-f]isoquinolin-7-one;
3-[(9-fluoro-7-oxo-6,7-dihydro-3H-benzo[h]imidazo[4,5-f]isoquinolin-2-yl)sulfinyl]propanenitrile ;
9-fluoro-2-[(2-hydroxy-3,3-dimethylbutyl)thio]-3,6-dihydro-7H-benzo[h]imidazo[4,5-f]isoquinolin-7-one ;
9-fluoro-2-[(2-oxopropyl)thio]-3,6-dihydro-7H-benzo[h]imidazo[4,5-f]isoquinolin-7-one;
9-fluoro-2-(pyridin-4-ylsulfinyl)-3,6-dihydro-7H-benzo[h]imidazo[4,5-f]isoquinolin-7-one;
4-[(9-fluoro-7-oxo-6,7-dihydro-3H-benzo[h]imidazo[4,5-f]isoquinolin-2-yl)sulfinyl]butanenitrile ;
9-fluoro-2-(pyrimidin-5-ylthio)-3,6-dihydro-7H-benzo[h]imidazo[4,5-f]isoquinolin-7-one;
ethyl 4-[(9-fluoro-7-oxo-6,7-dihydro-3H-benzo[h]imidazo[4,5-f]isoquinolin-2-yl)thio]-3-oxobutanoate;
3-[(9-fluoro-7-oxo-6,7-dihydro-3H-benzo[h]imidazo[4,5-f]isoquinolin-2-yl)thio]-2-methylpropanenitrile ;
3-[(9-fluoro-7-oxo-6,7-dihydro-3H-benzo[h]imidazo[4,5-f]isoquinolin-2-yl)sulfinyl]-2-methylpropanenitrile ;
9-fluoro-2-[(2-hydroxypropyl)thio]-3,3a,6,1b-tetrahydro-7H-benzo[h]imidazo[4,5-f]soquinolin-7-one;
9-fluoro-2-(isoxazol-4-ylthio)-3,6-dihydro-7H-benzo[h]imidazo[4,5-f]isoquinolin-7-one
3-[(9-fluoro-7-oxo-6,7-dihydro-3H-benzo[h]imidazo[4,5-f]isoquinolin-2-yl)thio]propanamide;
2-[(3,5-dichloropyridin-4-yl)thio]-9-fluoro-3,6-dihydro-7H-bcnzo[h]imidazo[4,5-f]isoquinolin-7-one;
2-[(4,6-dimethoxy-1,3,5-triazin-2-yl)thio]-9-fluoro-3,6-dihydro-7H-benzo[h]imidazo[4,5-f]isoquinolin-7-one;
9-fluoro-2-(pyrimidin-2-ylthio)-3,6-dihydro-7H-benzo[h]imidazo[4,5-f]isoquinolin-7-one ;
2-[(2,2-dimethylpropyl)thio]-9-fluoro-3,6-dihydro-7H-benzo[h]imidazo[4,5-f]isoquinolin-7-one ;
9-fluoro-2-(isobutylthio)-3,6-dihydro-7H-benzo[h]imidazo[4,5-f]isoquinolin-7-one;
2-[(2,2-dimethylpropyl)sulfinyl]-9-fluoro-3,6-dihydro-7H-benzo[h]imidazo[4,5-f]isoquinolin-7-one ;
2-(cyclohexylsulfinyl)-9-fluoro-3,6-dihydro-7H-benzo[h]imidazo[4,5-f]isoquinolin-7-one;
9-fluoro-2-(pyrazin-2-ylthio)-3,6-dihydro-7H-benzo[h]imidazo[4,5-f]isoquinolin-7-one;
9-fluoro-2-[(3-fluoropyridin-4-yl)thio]-3,6-dihydro-7H-benzo [h]imidazo [4,5-f]isoquinolin-7-one;
2-[(6-chloropyrimidin-4-yl)thio]-9-fluoro-3,6-dihydro-7H-benzo[h]imidazo[4,5-f]isoquinolin-7-one;
2-[(2,6-dimethoxypyrimidin-4-yl)thio]-9-fluoro-3,6-dihydro-7H-benzo[h]imidazo[4,5-f]isoquinolin-7-one;
2-[(2,6-dichloropyridin-4-yl)thio]-9-fluoro-3,6-dihydro-7H-benzo[h]imidazo[4,5-f]soquinolin-7-one;
2-[(6-chloropyridazin-3-yl)thio]-9-fluoro-3,6-dihydro-7H-benzo[h]imidazo[4,5-f]isoquinolin-7-one;
2-[(3-amino-6-bromopyrazin-2-yl)thio]-9-fluoro-3,6-dihydro-7H-benzo[h]imidazo[4,5-f]isoquinolin-7-one;
9-fluoro-2-(quinolin-4-ylthio)-3,6-dihydro-7H-benzo[h]imidazo[4,5-f]isoquinolin-7-one;
2-[(6-chloropyrazin-2-yl)thio]-9-fluoro-3,6-dihydro-7H-benzo[h]imidazo[4,5-f]isoquinolin-7-one;
2-[(2,6-dichloropyrimidin-4-yl)thio]-9-fluoro-3,6-dihydro-7H-benzo[h]imidazo[4,5-f]isoquinolin-7-one;
9-fluoro-2-[(1-oxidopyridin-4-yl)thio]-3,6-dihydro-7H-benzo[h]imidazo [4,5-f]isoquinolin-7-one ;
2-[(2,6-diaminopyrimidin-4-yl)thio]-9-fluoro-3,6-dihydro-7H-benzo[h]imidazo[4,5-flisoquinolin-7-one ;
9-fluoro-2-[(l-methyl-lH-pyrazol-4-yl)thio]-3,6-dihydro-7H-benzo[h]imidazo[4,5-f]isoquinolin-7-one;
3-[(9-fluoro-7-oxo-6,7-dihydro-3H-benzo[h]imidazo[4,5-f]isoquinolin-2-yl)thio]pyrazine-2-carbonitrile;
9-fluoro-2-[(2-mcthylpyrimidin-5-yl)thio]-3,6-dihydro-7H-bcnzo[h]imidazo[4,5-f]isoquinolin-7-one;
2-[(5-chloro-3-hydroxypyridazin-4-yl)thio]-9-fluoro-3,6-dihydro-7H-benzo[h]midazo[4,5-f]isoquinolin-7-one;
2-[(3,5-dichloropyridazin-4-yl)thio]-9-fluoro-3,6-dihydro-7H-benzo[h]imidazo[4,5-f]isoquinolin-7-one;
9-fluoro-2-[(1-oxidopyridin-2-yl)thio]-3,6-dihydro-7H-benzo [h]imidazo [4,5-f]isoquinolin-7-one:
9-fluoro-2-[(1H-tetrazol-5-ylmethyl)thio]-3,6-dihydro-7H-benzo[h]imidazo[4,5-f]isoquinolin-7-one trifluoroacetate;
9-fluoro-2-[(6-hydroxypyridin-3-yl)thio]-3,6-dihydro-7H-benzo[h]imidazo[4,5-f]isoquinolin-7-one;
2-[(2-amino-6-chloropyridin-4-yl)thio]-9-fluoro-3,6-dihydro-7H-benzo[h]imidazo[4,5-f]isoquinolin-7-one trifluoroacetate;
2-[(6-aminopyrimidin-4-yl)thio]-9-fluoro-3,6-dihydro-7H-benzo[h]imidazo[4,5-f]isoquinolin-7-one;
2-[(6-aminopyrimidin-4-yl)thio]-9-fluoro-3,6-dihydro-7H-benzo[h]imidazo[4,5-f]isoquinolin-7-one;
9-fluoro-2-(pyridin-3-ylthio)-3,6-dihydro-7H-benzo[h]imidazo[4,5-f]isoquinolin-7-one trifluoroacetate;
9-fluoro-2-(phenylthio)-3,6-dihydro-7H-benzo[h]imidazo[4,5-f]isoquinolin-7-one trifluoroacetate;
2-[(2-chloropyrimidin-4-yl)hio]-9-fluoro-3,6-dihydro-7H-benzo[h]imidazo[4,5-f]isoquinolin-7-one;
9-fluoro-2-(1H-tetrazol-5-ylthio)-3,6-dihydro-7H-benzo[h]imidazo[,5-f]isoquinolin-7-one;
2-[(5-bromopyridin-3-yl)thio]-9-fluoro-3,6-dihydro-7H-benzo[h]imidazo[4,5-f]isoquinolin-7-one trifluoroacetate;
2-[(3-aminophenyl)thio]-9-fluoro-3,6-dihydro-7H-benzo[h]imidazo[4,5-f]isoquinolin-7-one trifluoroacetate;
4-[(9-fluoro-7-oxo-6,7-dihydro-3H-benzo[h]imidazo[4,5-f]isoquinolin-2-yl)thio]pyridine-2-carbonitrile;
9-fluoro-2-[(5-methoxypyridin-3-yl)thio]-3,6-dihydro-7H-benzo[h]imidazo[4,5-f]isoquinolin-7-one trifluoroacetate;
2-[(2-aminopyrimidin-4-yl)thio]-9-fluoro-3,6-dihydro-7H-benzo[h]imidazo[4,5-f]isoquinolin-7-one;
5-[(9-fluoro-7-oxo-6,7-dihydro-3H-benzo[h]imidazo[4,5-f]isoquinolin-2-yl)thio]-N-(2-hydroxyethyl)nicotinamide trifluoroacetate (salt);
methyl 4-chloro-5-[(9-fluoro-7-oxo-6,7-dihydro-3H-bcnzo[h]imidazo[4,5-f]isoquinolin-2-yl)thio]pyridine-2-carboxylate;
9-fluoro-2-[(6-fluoropyridin-3-yl)thio]-3,6-dihydro-7H-benzo [h]imidazo [4,5-f]isoquinolin-7-one trifluoroacetate;
2-[(6-aminopyridin-3-yl)thio]-9-fluoro-3,6-dihydro-7H-benzo[h]imidazo[4,5-f]isoquinolin-7-one trifluoroacetate;
9-fluoro-2-[(6-methoxypyridin-3-yl)thio]-3,6-dihydro-7H-benzo[h]imidazo[4,5-f]isoquinolin-7-one trifluoroacetate;
9-fluoro-2-[(2-fluoropyridin-4-yl)thio]-3,6-dihydro-7H-benzo[h]imidazo[4,5-f]isoquinolin-7-one;
9-fluoro-2-(quinolin-3-ylthio)-3,6-dihydro-7H-benzo[h]imidazo[4,5-f]isoquinolin-7-one trifluoroacetate;
5-[(9-fluoro-7-oxo-6,7-dihydro-3H-benzo[h]imidazo[4,5-f]isoquinolin-2-yl)thio]nicotinamide trifluoroacetate;
2-[(2-aminopyridin-4-yl)thio]-9-fluoro-3,6-dihydro-7H-benzo[h]imidazo[4,5-f]isoquinolin-7-one;
9-fluoro-2-[(2-methoxypyridin-4-yl)thio]-3,6-dihydro-7H-benzo[h]imidazo[4,5-f]isoquinolin-7-one;
9-fluoro-2-{[5-(mopholin-4-ylcarbonyl)pyridin-3-yl]thio} -3,6-dihydro-7H-benzo[h]imidazo[4,5-f]isoquinolin-7-one trifluoroacetate;
5-[(9-fluoro-7-oxo-6,7-dihydro-3H-benzo[h]imidazo[4,5-f]isoquinolin-2-yl)thio]-N-[2-(tetrahydro-2H-pyran-4-yl)ethyl]nicotinamide;
9-fluoro-2-({5-[(4-methylpiperazin-l-yl)carbonyl]pyridin-3-yl}thio)-3,6-dihydro-7H-benzo[h]imidazo[4,5-f]isoquinolin-7-one;
9-fluoro-2-({2-[(2-morpholin-4-ylethyl)amino]pyridin-4-yl}thio)-3,6-dihydro-7H-benzo[h]imidazo[4,5-f]isoquinolin-7-one;
2-{[2-(dimethylamino)pyridin-4-yl]thio}-9-fluoro-3,6-dihydro-7H-benzo[h]imidazo[4,5-f]isoquinolin-7-one;
9-fluoro-2-({2-[(2-hydroxyethyl)amino]pyridin-4-yl}thio)-3,6-dihydro-7H-benzo[h]imidazo [4,5-f] isoquinolin-7-one;
methyl 4-[(9-fluoro-7-oxo-6,7-dihydro-3H-benzo [h]imidazo [4,5-f]isoquinolin-2-yl)thio]pyridine-2-carboxylate;
9-fluoro-2-(isoquinolin-4-ylthio)-3,6-dihydro-7H-benzo[h]imidazo[4,5-f]isoquinolin-7-one trifluoroacetate;
9-fluoro-2-({6-[(2-hydroxyethyl)amino]pyridin-3-yl}thio)-3,6-dihydro-7H-benzo[h]imidazo[4,5-f]isoquinolin-7-one trifluoroacetate;
9-fluoro-2-({6-[(2-morpholin-4-ylethyl)amino]pyridin-3-yl}thio)-3,6-dihydro-7H-benzo[h]imidazo[4,5-f]isoquinolin-7-one trifluoroacetate;
4-[(9-fluoro-7-oxo-6,7-dihydro-3H-benzo[h]imidazo[4,5-f]isoquinolin-2-yl)thio]benzamide;
2-[(5-aminopyridin-3-yl)thio]-9-fluoro-3,6-dihydro-7H-benzo[h]imidazo[4,5-f]isoquinolin-7-one;
9-fluoro-2-{[4-(1H-imidazol-1-yl)phenyl]thio}-3,6-dihydro-7H-benzo[h]imidazo[4,5-f]isoquinolin-7-one trifluoroacetate;
9-fluoro-2-[(3-hydroxyphenyl)thio]-3,6-dihydro-7H-benzo[h]imidazo[4,5-f]isoquinolin-7-one trifluoroacetate (salt);
4-[(9-fluoro-7-oxo-6,7-dihydro-3H-benzo[h]imidazo[4,5-f]isoquinolin-2-yl)thio]-N-(2-hydroxyethyl)benzamide trifluoroacetate (salt);
3-[(9-fluoro-7-oxo-6,7-dihydro-3H-benzo[h]imidazo[4,5-f]isoquinolin-2-yl)thio]-N-(2-hydroxyethyl)benzamide trifluoroacetate (salt);
3-[(9-fluoro-7-oxo-6,7-dihydro-3H-benzo[h]imidazo[4,5-f]isoquinolin-2-yl)thio]benzonitrile trifluoroacetate;
9-fluoro-2-[(5-morpholin-4-ylpyridin-3-yl)thio]-3,6-dihydro-7H-benzo[h]imidazo[4,5-f]isoquinolin-7-one trifluoroacetate;
{-[(9-fluoro-7-oxo-6,7-dihydro-3H-benzo[h]imidazo[4,5-f]isoquinolin-2-yl)thio]phenoxy} acetonitrile trifluoroacetate;
N-{3-[(9-fluoro-7-oxo-6,7-dihydro-3H-benzo[h]imidazo[4,5-f]isoquinolin-2-yl)thio]phenyl}ethanesulfonamide trifluoroacetate;
2-cyano-N-{3-[(9-fluoro-7-oxo-6,7-dihydro-3H-benzo[h]imidazo[4,5-f]isoquinolin-2-yl)thio]phenyl}cetamide trifluoroacetate;
N'-{3-[(9-fluoro-7-oxo-6,7-dihydro-3H-benzo[h]imidazo[4,5-f]isoqumolm-2-yl)thio]phenyl}-N,N-dimethylsulfamide;
N-{3-[(9-fluoro-7-oxo-6,7-dihydro-3H-benzo[h]imidazo[4,5-f]isoquinolin-2-yl)thio]phenyl}ethanesulfonamide;
2-(dimethylamino)-N- {3-[(9-fluoro-7-oxo-6,7-dihydro-3H-benzo[h]imidazo[4,5-f]isoquinolin-2-yl)thio]phenyl}acetamide trifluoroacetate;
N-ethyl-N'-{3-[(9-fluoro-7-oxo-6,7-dihydro-3H-benzo[h]imidazo[4,5-f]isoquinolin-2-yl)thio]phenyl}urea trifluoroacetate;
2-tert-butyl-10-fluorobenzo[c]imidazo[1,2-a]-1,6-naphthyridin-8(7H)-one;
10-Fluoro-2-(4-hydroxycyclohexyl)benzo[c]imidazo[1,2-a]-1,6-naphthyridin-8(7H)-one;
trans-4-(10-Fluoro-8-oxo-7,8-dihydrobenzo[c]imidazo[1,2-a]-1,6-naphthyridin-2-yl)cyclohexyl (dimethylamino) acetate;
10-fluoro-2-(1-methylcyclopropyl)benzo[c]imidazo[1,2-a]-1,6-naphthyridin-8(7H)-one;
2-cyclopropyl-10-fluorobenzo[c]imidazo[1,2-a]-1,6-naphthyridin-8(7H)-one;
2-cyclobutyl-10-fluorobenzo[c]imidazo[1,2-a]-1,6-naphthyridin-8(7H)-one;
2-cthyl-10-fluorobcnzo[c]imidazo[1,2-a]-1,6-naphthyridin-8(7H)-onc;
ethyl 2-ethyl-2-(10-fluoro-8-oxo-7,8-dihydrobenzo[c]imidazo[1,2-a]-1,6-naphthyridin-2-yl)butanoate;
2-[1-ethyl-1-(hydroxymethyl)propyl]-10-fluorobenzo[c]imidazo[1,2-a]-1,6-naphthyridin-8(7H)-one;
2-(1-ethylpropyl)-10-fluorobenzo[c]imidazo[1,2-a]-1,6-naphthyridin-8(7H)-one;
10-fluoro-2-(4-oxocyclohexyl)benzo[c]imidazo[1,2-a]-1,6-naphthyridin-8(7H)-one;
10-fluoro-2-methylbenzo[c]imidazo[1,2-a]-1,6-naphthyridin-8(7H)-one;
10-fluoro-2-(cis-4-methoxycyclohexyl)benzo[c]imidazo[1,2-a]-1,6-naphthyridin-8(7H)-one;
10-fluoro-2-(trans-4-methoxycyclohexyl)benzo[c]imidazo[1,2-a]-1,6-naphthyridin-8(7H)-one;
10-fluoro-2-[4-(hydroxyimino)cyclohexyl]benzo[c]imidazo[1,2-a]-1,6-naphthyridin-8(7H)-one ;
2-(1-acetylpiperidin-4-yl)-10-fluorobenzo[c]imidazo[1,2-a]-1,6-naphthyridin-8(7H)-one;
2-amino-9-fluorobenzo[h][1,3]thiazolo[5,4-f]isoquinolin-7(6H)-one;
9-fluoro-2-(4-hydroxypiperidin-1-yl)benzo[h][1,3]thiazolo[5,4-f]isoquinolin-7(6H)-one;
9-fluoro-2-{[(1S)-1-(hydroxymethyl)-3-methylbutyl]amino}benzo[h][1,3]thiazolo[5,4-f]isoquinolin-7(6H)-one;
9-fluoro-2-{[(1R)-2-hydroxy-1-phenylethyl]amino}benzo[h][1,3]thiazolo[5,4-f]isoquinolin-7(6H)-one;
(2S)-2-[(9-fluoro-7-oxo-6,7-dihydrobenzo[h][1,3]thiazolo[5,4-f]isoquinolin-2-yl)amino]-4-methoxy-N-methylbutanamide;
9-fluoro-2-(4-oxopiperidin-1-yl)benzo[h][1,3]thiazolo[5,4-f]isoquinolin-7(6H)-one;
9-fluoro-2-{[(1R)-1-(hydroxymethyl)-3-methylbutyl]amino}benzo[h][1,3]thiazolo[5,4-f]isoquinolin-7(6H)-one;
9-fluoro-2-(3-hydroxypiperidin-1-yl)benzo[h][1,3]thiazolo[5,4-f]isoquinolin-7(6H)-one;
9-fluoro-2-{[(1S)-2-hydroxy-1-phenylethyl]amino}benzo[h][1,3]thiazolo[5,4-f]isoquinolin-7(6H)-one;
9-fluoro-2-(3-hydroxypyrrolidin-1-yl)benzo[h][1,3]thiazolo[5,4-f]isoquinolin-7(6H)-one;
9-fluorobenzo[h][1,3]thiazolo[5,4-f]isoquinolin-7(6H)-one;
9-fluoro-2-(1-hydroxy-4-oxocyclohexyl)benzo[h][1,3]thiazolo[5,4-f]isoquinolin-7(6H)-one;
2-[4-(ethoxyimino)piperidin-1-yl]-9-fluorobenzo[h][1,3]thiazolo[5,4-f]isoquinolin-7(6H)-one;
9-fluoro-2-[4-(hydroxyimino)piperidin-1-yl]benzo[h][1,3]thiazolo[5,4-f]isoquinolin-7(6H)-one;
4-(9-fluoro-7-oxo-6,7-dihydrobenzo[h][1,3]thiazolo[5,4-f]isoquinolin-2-yl)butanenitrile;
2-(1,4-dihydroxycyclohexyl)-9-fluorobenzo[h][1,3]thiazolo[5,4-f]isoquinolin-7(6H)-one;
2-(1-acetyl-4-hydroxypiperidin-4-yl)-9-fluorobenzo[h][1,3]thiazolo[5,4-f]isoquinolin-7(6H)-onc;
9-fluoro-2-(4-hydroxy-1-isobutyrylpiperidin-4-yl)benzo[h][1,3]thiazolo[5,4-f]isoquinolin-7(6H)-one;
9-fluoro-2-isonicotinoylbenzo[h][1,3]thiazolo[5,4-f]isoquinolin-7(6H)-one;
9-fluoro-2-(1-oxidoisonicotinoyl)benzo[h][1,3]thiazolo[5,4-f]isoquinolin-7(6H)-one;
9-fluoro-2-phenylbenzo[h][l,3]thiazolo[5,4-f]isoquinolin-7(6H)-one;
2-ethyl-9-fluorobenzo[h][l,3]thiazolo[5,4-f]isoquinolin-7(6H)-one;
9-fluoro-2-morpholm-4-ylbenzo[h][1,3]thiazolo[5,4-f]isoquinolin-7(6H)-one;
9-fluoro-2-[(pyridin-3-ylmethyl)amino]benzo[h][1,3]thiazolo[5,4-f]isoquinolin-7(6H)-one;
9-fluoro-2-[(3-morpholm-4-ylpropyl)ammo]benzo[h][l,3]thiazolo[5,4-f]isoquinolm-7(6H)-one;
9-fluoro-2-[(3-methoxybenzyl)amino]benzo[h][1,3]thiazolo[5,4-f]isoquinolin-7(6H)-one;
9-fluoro-2-{[(1R)-1-(3-methoxyphenyl)ethyl]amino}benzo[h][1,3]thiazolo[5,4-f]isoquinolin-7(6H)-one;
9-fluoro-2-{[(1S)-1-(3-methoxyphenyl)ethyl]amino}benzo[h][1,3]thiazolo[5,4-f]isoquinolin-7(6H)-one;
9-fluoro-2-[(3-hydroxypropyl)amino]benzo[h][1,3]thiazolo[5,4-f]isoquinolin-7(6H)-one;
9-fluoro-2-[(2-hydroxyethyl)(methyl)amino]benzo[h][1,3]thiazolo[5,4-f]isoquinolin-7(6H)-one;
9-fluoro-2-{[2-(4-hydroxyphenyl)ethyl]amino}benzo[h][1,3]thiazolo[5,4-f]isoquinolin-7(6H)-one;
9-fluoro-2-[(2-methoxy-1-methylethyl)amino]benzo[h][1,3]thiazolo[5,4-f]isoquinolin-7(6H)-one;
N,N-diethyl-1-(9-fluoro-7-oxo-6,7-dihydrobenzo[h][1,3]thiazolo[5,4-f]isoquinolin-2-yl)piperidine-3-carboxamide;
9-fluoro-2-[(3-phenylpropyl)amino]benzo[h][1,3]thiazolo[5,4-f]isoquinolin-7(6H)-one;
9-fluoro-2-{[(1R)-1-(4-methoxyphenyl)ethyl]amino}benzo[h][1,3]thiazolo[5,4-f]isoquinolin-7(6H)-one;
9-fluoro-2-{[(1S)-1-(4-methoxyphenyl)ethyl]amino}benzo[h][1,3]thiazolo[5,4-f]isoquinolin-7(6H)-one;
9-fluoro-2-(pyridin-3-ylamino)benzo[h][1,3]thiazolo[5,4-f]isoquinolin-7(6H)-one;
9-fluoro-2-(4-pyridin-2-ylpiperazin-1-yl)benzo[h][1,3]thiazolo[5,4-f]isoquinolin-7(6H)-one;
9-fluoro-2-{[3-(1H-imidazol-1-yl)propyl]amino}benzo[h][1,3]thiazolo[5,4-f]isoquinolin-7(6H)-one;
9-fluoro-2-[(2-morpholin-4-ylethyl)amino]benzo[h][1,3]thiazolo[5,4-f]isoquinolin-7(6H)-one;
9-fluoro-2-[(2-methoxyethyl)amino]benzo[h][1,3]thiazolo[5,4-f]isoquinolin-7(6H)-one;
9-fluoro-2-[(tctrahydrofuran-2-ylmethyl)amino]benzo[h][1,3]thiazolo[5,4-f]isoquinolin-7(6H)-one;
9-fluoro-2-[(2-pyridin-2-ylethyl)amino]benzo[h][1,3]thiazolo[5,4-f]isoquinolin-7(6H)-one;
9-fluoro-2-{[2-(1H-imidazol-4-yl)ethyl]amino}benzo[h][1,3]thiazolo[5,4-f]isoquinolin-7(6H)-one;
9-fluoro-2-[4-(2-hydroxyethyl)piperazin-1-yl]benzo[h][1,3]thiazolo[5,4-f]isoquinolin-7(6H)-one;
2-{[3-(dimethylamino)propyl]amino}-9-fluorobenzo[h][1,3]thiazolo[5,4-f]isoquinolin-7(6H)-one;
9-fluoro-2-(4-hydroxy-4-pyridin-2-ylpiperidin-1-yl)benzo[h][1,3]thiazolo[5,4-f]isoquinolin-7(6H)-one;
2-[bis(2-methoxyethyl)amino]-9-fluorobenzo[h][l,3]thiazolo[5,4-f]isoquinolin-7(6H)-one;
(9-fluoro-7-oxo-6,7-dihydrobenzo[h][1,3]thiazolo[5,4-f]isoquinolin-2-yl)acetonitrile;
2-{benzyl[2-(dimethylamino)ethyl]amino}-9-fluorobenzo[h][1,3]thiazolo[5,4-f]isoquinolin-7(6H)-one;
9-fluoro-2-{[3-(trifluoromethyl)phenyl]amino}benzo[h][1,3]thiazolo[5,4-f]isoquinolin-7(6H)-one;
9-fluoro-2-[(2-methoxyphenyl)amino]benzo[h][1,3]thiazolo[5,4-f]isoquinolin-7(6H)-one;
9-fluoro-2-(propylamino)benzo[h][1,3]thiazolo[5,4-f]isoquinolin-7(6H)-one;
9-fluoro-2-(isopropylamino)benzo[h][1,3]thiazolo[5,4-f]isoquinolin-7(6H)-one;
3-[(9-fluoro-7-oxo-6,7-dihydrobenzo[h][1,3]thiazolo[5,4-f]isoquinolin-2-yl)amino]benzonitrile;
9-fluoro-2-[(3-hydroxyphenyl)amino]benzo[h][1,3]thiazolo[5,4-f]isoquinolin-7(6H)-one;
9-fluoro-2-[(trans-4-hydroxy-4-pyridin-2-ylcyclohexyl)amino]benzo[h][1,3]thiazolo[5,4-f]isoquinolin-7(6H)-one;
9-fluoro-2-[cis-4-hydroxy-3-(hydroxymethyl)piperidin-1-yl]benzo[h][1,3]thiazolo[5,4-f]isoquinolin-7(6H)-one;
9-fluoro-2-[trans-4-hydroxy-3-(hydroxymethyl)piperidin-1-yl]benzo[h][1,3]thiazolo[5,4-f]isoquinolin-7(6H)-one;
9-fluoro-2-[(2-hydroxyethyl)amino]benzo[h][1,3]thiazolo[5,4-f]isoquinolin-7(6H)-one;
9-fluoro-2-[(4-hydroxybenzyl)amino]benzo[h][1,3]thiazolo[5,4-f]isoquinolin-7(6H)-one;
9-fluoro-2-[4-(hydroxymethyl)piperidin-1-yl]benzo[h][1,3]thiazolo[5,4-f]isoquinolin-7(6H)-one;
9-fluoro-2-[3-(hydroxymethyl)piperidin-1-yl]benzo[h][1,3]thiazolo[5,4-f]isoquinolin-7(6H)-one;
9-fluoro-2-[(2-hydroxy-1-methylethyl)amino]benzo[h][1,3]thiazolo[5,4-f]isoquinolin-7(6H)-one;
9-fluoro-2-[(2S)-2-(hydroxymethyl)pyrrolidin-1-yl]benzo[h][1,3]thiazolo[5,4-f]isoquinolin-7(6H)-one;
9-fluoro-2-[(2R)-2-(hydroxymethyl)pyrrolidin-1-yl]benzo[h][1,3]thiazolo[5,4-f]isoquinolin-7(6H)-one;
2-(4-acetylpiperazin-1-yl)-9-fluorobenzo[h][1,3]thiazolo[5,4-f]isoquinolin-7(6H)-one;
9-fluoro-2-piperazin-1-ylbenzo[h][1,3]thiazolo[5,4-f]isoquinolin-7(6H)-one;
2-{4-[(dimethylamino)acetyl]piperazin-1-yl}-9-fluorobenzo[h][1,3]thiazolo[5,4-f]isoquinolin-7(6H)-one;
9-fluoro-2-[4-(2-hydroxyethyl)piperidin-1-yl]benzo[h][1,3]thiazolo[5,4-f]isoquinolin-7(6H)-one;
9-fluoro-2-{4-[(2R)-2-hydroxypropyl]piperazin-1-yl}benzo[h][1,3]thiazolo[5,4-f]isoquinolin-7(6H)-one;
2-[(2,3-dihydroxypropyl)amino]-9-fluorobenzo[h][1,3]thiazolo[5,4-f]isoquinolin-7(6H)-one;
4-(9-fluoro-7-oxo-6,7-dihydrobenzo[h][1,3]thiazolo[5,4-f]isoquinolin-2-yl)piperazine-1-carboxamide;
methyl 4-(9-fluoro-7-oxo-6,7-dihydrobenzo[h][1,3]thiazolo[5,4-f]isoquinolin-2-yl)piperazine-1-carboxylate;
ethyl 4-(9-fluoro-7-oxo-6,7-dihydrobenzo[h][1,3]thiazolo[5,4-f]isoquinolin-2-yl)piperazine-1-carboxylate;
4-(9-fluoro-7-oxo-6,7-dihydrobenzo[h][1,3]thiazolo[5,4-f]isoquinolin-2-yl)piperazine-1-carbaldehyde;
9-fluoro-2-[4-(1H-tetrazol-5-ylacetyl)piperazin-1-yl]benzo[h][1,3]thiazolo[5,4-f]isoquinolin-7(6H)-one;
9-fluoro-2-(pyridin-2-ylamino)benzo[h][1,3]thiazolo[5,4-f]isoquinolin-7(6H)-one;
9-fluoro-2-(pyridin-4-ylamino)benzo[h][1,3]thiazolo[5,4-f]isoquinolin-7(6H)-one;
9-fluoro-2-[(3R,4R)-3-methoxy-4-(methylamino)pyrrolidin-1-yl]benzo[h][1,3]thiazolo[5,4-f]isoquinolin-7(6H)-one;
9-fluoro-2-[(3S,4R)-3-methoxy-4-(methylamino)pyrrolidin-1-yl]benzo[h][1,3]thiazolo[5,4-f]isoquinolin-7(6H)-one;
benzyl (3R,4R)-3-[(9-fluoro-7-oxo-6,7-dihydrobenzo[h][1,3]thiazolo[5,4-f]isoquinolin-2-yl)(methyl)amino]-4-methoxypyrrolidine-1-carboxylate:
2-(1-ethyl-1-hydroxypropyl)-9-fluorobenzo[h][1,3]thiazolo[5,4-f]isoquinolin-7(6H)-one;
9-fluoro-2-(hydroxymethyl)benzo[h][1,3]thiazolo[5,4-f]isoquinolin-7(6H)-one;
2-(ethoxymethyl)-9-fluorobenzo[h][1,3]thiazolo[5,4-f]isoquinolin-7(6H)-one;
3-(9-fluoro-7-oxo-6,7-dihydrobenzo[h][1,3]thiazolo[5,4-f]isoquinolin-2-yl)propanenitrile;
[1-(9-fluoro-7-oxo-6,7-dihydrobenzo[h][1,3]thiazolo[5,4-f]isoquinolin-2-yl)piperidin-4-yl]acctonitrile;
1-(9-fluoro-7-oxo-6,7-dihydrobenzo[h][1,3]thiazolo[5,4-f]isoquinolin-2-yl)piperidine-4-carbonitrile;
3-[(9-fluoro-7-oxo-6,7-dihydrobenzo[h][1,3]thiazolo[5,4-f]isoquinolin-2-yl)methoxy]propanenitrile;
9-fluoro-2-{4-[2-(1H-1,2,4-triazol-1-yl)ethyl]piperidin-1-yl}benzo[h][1,3]thiazolo[5,4-f]isoquinolin-7(6H)-one;
9-fluoro-2-[4-(1H-1,2,4-triazol-1-ylmethyl)piperidin-1-yl]benzo[h][1,3]thiazolo[5,4-f]isoquinolin-7(6H)-one;
9-fluoro-2-[4-(1H-imidazol-1-ylmethyl)piperidin-1-yl]benzo[h][1,3]thiazolo[5,4-f]isoquinolin-7(6H)-one;
3-[1-(9-fluoro-7-oxo-6,7-dihydrobenzo[h][1,3]thiazolo[5,4-f]isoquinolin-2-yl)piperidin-4-yl]propanenitrile ;
9-fluoro-2-{4-[2-(1H-imidazol-1-yl)ethyl]piperidin-1-yl}benzo[h][1,3]thiazolo[5,4-f]isoquinolin-7(6H)-one ;
2-{4-[(diethylamino)methyl]piperidin-1-yl}-9-fluorobenzo[h][1,3]thiazolo[5,4-f]isoquinolin-7(6H)-one;
9-fluoro-2-(pyridin-3-ylmethyl)benzo[h][1,3]thiazolo[5,4-f]isoquinolin-7(6H)-one;
9-fluoro-2-(pyridin-2-ylmethyl)benzo[h][1,3]thiazolo[5,4-f]isoquinolin-7(6H)-one;
9-fluoro-2-[(1-oxidopyridin-3-yl)methyl]benzo[h][1,3]thiazolo[5,4-f]isoquinolin-7(6H)-one;
9-fluoro-2-(pyridin-4-ylmethyl)benzo[h][1,3]thiazolo[5,4-f]isoquinolin-7(6H)-one;
9-fluoro-2-[hydroxy(6-methoxypyridin-3-yl)methyl]benzo[h][1,3]thiazolo[5,4-f]isoquinolin-7(6H)-one;
9-fluoro-2-[3-hydroxy-1-(pyridin-4-ylmethyl)piperidin-3-yl]benzo[h][1,3]thiazolo[5,4-f]isoquinolin-7(6H)-one;
2-(1-acetyl-3-hydroxypiperidin-3-yl)-9-fluorobenzo[h][1,3]thiazolo[5,4-f]isoquinolin-7(6H)-one;
9-fluoro-2-[(4-hydroxypiperidin-1-yl)methyl]benzo[h][1,3]thiazolo[5,4-f]isoquinolin-7(6H)-one;
2-[(4-acctylpiperazin-1-yl)carbonyl]-9-fluorobenzo[h][1,3]thiazolo[5,4-f]isoquinolin-7(6H)-one;
9-fluoro-2-[3-hydroxy-1-(methylsulfonyl)piperidin-3-yl]benzo[h][1,3]thiazolo[5,4-f]isoquinolin-7(6H)-one;
9-fluoro-2-(1-hydroxy-1-pyridin-3-ylethyljbenzo[h][1,3]thiazolo[5,4-f]isoquinolin-7(6H)-one;
9-fluoro-2-(1-hydroxy-1-pyridin-4-ylethyl)benzo[h][1,3]thiazolo[5,4-f]isoquinolin-7(6H)-onc;
9-fluoro-2-[1-hydroxy-1-(1-oxidopyridin-3-yl)ethyl]benzo[h][1,3]thiazolo[5,4-f]isoquinolin-7(6H)-one;
9-fluoro-2-[1-hydroxy-1-(1-oxidopyridin-4-yl)ethyl]benzo[h][1,3]thiazolo[5,4-f]isoquinolin-7(6H)-one;
2-{4-[(dimethylammo)methyl]piperidm-1-yl}-9-fluorobenzo[h][1,3]thiazolo[5,4-f]isoquinolin-7(6H)-one;
9-fluoro-2-(yridine-3-yloxy)benzo[h][1,3]thiazolo[5,4-f]isoquinolin-7(6H)-one;
9-fluoro-2-(pyridin-4-ylthio)benzo[h][1,3]thiazolo[5,4-f]isoquinolin-7(6H)-one;
9-fluoro-2-[(trans-4-hydroxycyclohexyl)amino]benzo[h][1,3]thiazolo[5,4-f]isoquinolin-7(6H)-one;
9-fluoro-2-(4-pyrazin-2-ylpiperazin-1-yl)benzo[h][1,3]thiazolo[5,4-f]isoquinolin-7(6H)-one;
4-(9-fluoro-7-oxo-6,7-dihydrobenzo[h][l,3]thiazolo[5,4-f]isoquinolin-2-yl)-N-methylpiperazine-1-carboxamide;
9-fluoro-2- [4-(1H-pyrazol-3-ylcarbonyl)piperazin-1-yl]benzo[h][1,3]thiazolo[5,4-f]isoquinolin-7(6H)-one;
9-fluoro-2- [4-(1H-pyrazol-4-ylcarbonyl)piperazin-1-yl]benzo[h][1,3]thiazolo[5,4-f]isoquinolin-7(6H)-one;
9-fluoro-2- [4-(1H-imidazol-2-ylcarbonyl)piperazin-1-yl]benzo[h][1,3]thiazolo[5,4-f]isoquinolin-7(6H)-one;
2-[4-(aminoacetyl)piperazin-1-yl]-9-fluorobenzo[h][1,3]thiazolo[5,4-f]isoquinolin-7(6H)-one;
2-[4-(azetidin-3-ylcarbonyl)piperazin-1-yl]-9-fluorobenzo[h][1,3]thiazolo[5,4-f]isoquinolin-7(6H)-one;
N-(tert-butyl)-4-(9-fluoro-7-oxo-6,7-dihydrobenzo[h][1,3]thiazolo[5,4-f]isoquinolin-2-yl)piperazine-1-carboxamide;
9-fluoro-2-{4-[(2S)-2-hydroxypropyl]piperazin-1-yl}benzo[h][1,3]thiazolo[5,4-f]isoquinolin-7(6H)-one;
3-[4-(9-fluoro-7-oxo-6,7-dihydrobenzo[h][1,3]thiazolo[5,4-f]isoquinolin-2-yl)piperazin-1-yl]-3-oxopropanenitrile;
9-fluoro-2-[4-(methylsulfonyl)piperazin-1-yl]benzo[h][1,3]thiazolo[5,4-f]isoquinolin-7(6H)-one:
4-(9-fluoro-7-oxo-6,7-dihydrobenzo[h][l,3]thiazolo[5,4-f]isoquinolin-2-yl)-N,N-dimethylpiperazine-1-carboxamide;
1-(9-fluoro-7-oxo-6,7-dihydrobenzo[h][1,3]thiazolo[5,4-f]isoquinolin-2-yl)piperidine-4-carboxylic acid;
9-fluoro-2-{4-hydroxy-4-[(methylamino)methyl]piperidin-1-yl}benzo[h][1,3]thiazolo[5,4-f]isoquinolin-7(6H)-one;
9-fluoro-2-[4-hydroxy-4-(1H-1,2,4-triazol-1-ylmethyl)piperidin-1-yl]benzo[h][1,3]thiazolo[5,4-f]isoquinolin-7(6H)-one;
9-fluoro-2-[4-hydroxy-4-(hydroxymethyl)piperidin-1-yl]benzo[h][1,3]thiazolo[5,4-f]isoquinolin-7(6H)-one;
9-fluoro-2-piperidin-3-ylbenzo[h][1,3]thiazolo[5,4-f]isoquinolin-7(6H)-one;
2-(1-acetylpiperidin-3-yl)-9-fluorobenzo[h][1,3]thiazolo[5,4-f]isoquinolin-7(6H)-one;
9-fluoro-2-[1-(methylsulfonyl)piperidin-3-yl]benzo[h][1,3]thiazolo[5,4-f]isoquinolin-7(6H)-one;
9-fluoro-2-[1-(pyridin-4-ylmethyl)piperidin-3-yl]benzo[h][1,3]thiazolo[5,4-f]isoquinolin-7(6H)-one;
3-[3-(9-fluoro-7-oxo-6,7-dihydrobenzo[h][1,3]thiazolo[5,4-f]isoquinolin-2-yl)piperidin-1-yl]-3-oxopropanenitrile;
9-fluoro-2-{1-[(2S)-2-hydroxypropyl]piperidin-3-yl}benzo[h][1,3]thiazolo[5,4-f]isoquinolin-7(6H)-one;
2-{1-[(dimethylamino)acetyl]piperidin-3-yl}-9-fluorobenzo[h][1,3]thiazolo[5,4-f]isoquinolin-7(6H)-one;
9-fluoro-2-[4-(morpholin-4-ylmethyl)piperidin-1-yl]benzo[h][1,3]thiazolo[5,4-f]isoquinolin-7(6H)-one;
9-fluoro-2-[4-(2-morpholin-4-ylethyl)piperidin-1-yl]benzo[h][1,3]thiazolo[5,4-f]isoquinolin-7(6H)-one;
4-(9-fluoro-7-oxo-6,7-dihydrobenzo[h][1,3]oxazolo[5,4-f]isoquinolin-2-yl)butanenitrile;
9-fluoro-2-[3-(1H-1,2,4-triazol-1-yl)propyl]benzo[h][1,3]oxazolo[5,4-f]isoquinolin-7(6H)-one;
9-fluoro-2-{[(1R)-1-phenylethyl]amino}benzo[h][1,3]oxazolo[5,4-f]isoquinolin-7(6H)-one;
9-fluoro-2-{[(1S)-1-phenylethyl]amino}benzo[h][1,3]oxazolo[5,4-f]isoquinolin-7(6H)-one;
9-fluoro-2-[(2-methoxybenzyl)amino]benzo[h][1,3]oxazolo[5,4-f]isoquinolin-7(6H)-one;
9-fluoro-2-[(4-methoxybenzyl)amino]benzo[h][1,3]oxazolo[5,4-f]isoquinolin-7(6H)-one;
9-fluoro-2-[(3-methoxybenzyl)amino]benzo[h][1,3]oxazolo[5,4-f]isoquinolin-7(6H)-one;
9-fluoro-2-(4-oxopiperidin-1-yl)benzo[h][1,3]oxazolo[5,4-f]isoquinolin-7(6H)-one;
9-fluoro-2-(4-hydroxypiperidin-1-yl)benzo[h][1,3]oxazolo[5,4-f]isoquinolin-7(6H)-one;
9-fluoro-2-{[1-(methylsulfonyl)piperidin-4-yl]amino}benzo[h][1,3]oxazolo[5,4-f]isoquinolin-7(6H)-one;
2-[(1-acetylpiperidin-4-yl)amino]-9-fluorobenzo[h][1,3]oxazolo[5,4-f]isoquinolin-7(6H)-one;
9-fluoro-2-[hydroxy(pyridin-3-yl)methyl]benzo[h][1,3]oxazolo[5,4-f]isoquinolin-7(6H)-one;
2-(4-acetylpiperazin-1-yl)-9-fluorobenzo[h][1,3]oxazolo[5,4-f]isoquinolin-7(6H)-one;
4-(9-fluoro-7-oxo-6,7-dihydrobenzo[h][1,3]oxazolo[5,4-f]isoquinolin-2-yl)piperazine-1-carboxamide;
2-[(4-acetylpiperazin-1-yl)methyl]-9-fluorobenzo[h][1,3]oxazolo[5,4-f]isoquinolin-7(6H)-one;
9-fluoro-2-[(trans-4-hydroxycyclohexyl)amino]benzo[h][1,3]oxazolo[5,4-f]isoquinolin-7(6H)-one;
9-fluoro-2-(pyridm-3-yloxy)benzo[h][1,3]oxazolo[5,4-f]isoquinolin-7(6H)-one;
2-[(1-acctylpiperidin-3-yl)amino]-9-fluorobenzo[h][1,3]oxazolo[5,4-f]isoquinolin-7(6H)-one;
9-fluoro-2-{[1-(methylsulfonyl)piperidin-3-yl]amino}benzo[h][1,3]oxazolo[5,4-f]isoquinolin-7(6H)-one;
9-fluoro-2-[4-(1,3-thiazol-2-yl)piperazin-1-yl]benzo[h][1,3]oxazolo[5,4-f]isoquinolin-7(6H)-one;
2-(trans-4-Hydroxycyclohexyl)imidazo[1,2-a]pyrido[4,3-c]-1,6-naphthyridin-8(7H)-one;
2-(cis-4-Hydroxycyclohexyl)imidazo[1,2-a]pyrido[4,3-c]-1,6-naphthyridin-8(7H)-one;
4-(8-Oxo-7,8-dihydroimidazo[1,2-a]pyrido[4,3-c]-1,6-naphthyridin-2-yl)piperidine-1-carbaldehyde;
2-Piperidin-4-ylimidazo[1,2-a]pyrido[4,3-c]-1,6-naphthyridin-8(7H)-one;
2-[1-(Cyclopropylmethyl)piperidin-4-yl]imidazo[1,2-a]pyrido [4,3-c]-1,6-naphthyridin-8(7H)-one;
2-[1-(Propyl)piperidin-4-yl]imidazo[1,2-a]pyrido[4,3-c]-1,6-naphthyridin-8(7H)-one;
2-tert-Butylimidazo[1,2-a]pyrido[4,3-c]-1,6-naphthyridin-8(7H)-one;
2-(4-Hydroxypiperidin-1-yl)-1,9-dihydro-8H-imidazo[4,5-f]-2,8-phenanthrolin-8-one;
2-(4-hydroxypiperidin-1-yl)[1,3]thiazolo[4,5-f]-2,9-phenanthrolin-8(9H)-one;
2-(isopropylamino)[1,3]thiazolo[4,5-f]-2,9-phenanthrolin-8(9H)-one;
2-[(3-methoxypropyl)amino][1,3]thiazolo[4,5-f]-2,9-phenanthrolin-8(9H)-one;
2-{[(1R)-1-(hydroxymethyl)-3-methylbutyl]amino}[1,3]thiazolo[4,5-f]-2,9-phenanthrolin-8(9H)-one;
2-{[1-(methylsulfonyl)piperidin-4-yl]oxy[1,3]thiazolo[4,5-f]-2,9-phenanthrolin-8(9H)-one;
2-{[(1R)-1-(hydroxymethyl)butyl]ammo}[1,3]thiazolo[4,5-f]-2,9-phenanthrolin-8(9H)-one;
2-{[(1R)-1-(hydroxymethyl)propyl]amino}[1,3]thiazolo[4,5-f]-2,9-phenanthrolin-8(9H)-one;
2-{[(1R)-1-(hydroxymethyl)-2-methylpropyl]amino}[1,3]thiazolo[4,5-f]-2,9-phenanthrolin-8(9H)-one;
2-{[(1R,2R)-2-hydroxycyclohexyl]amino}[1,3]thiazolo[4,5-f]-2,9-phenanthrolin-8(9H)-one;
2-{[(1R,2R)-2-hydroxycyclopentyl]amino}[1,3]thiazolo[4,5-f]-2,9-phenanthrolin-8(9H)-one;
2-{[(1R)-1-(hydroxymethyl)-2,2-dimethylpropyl]amino}[1,3]thiazolo[4,5-f]-2,9-phenanthrolin-8(9H)-one;
2-(4-{[3-(2-oxopyrrolidin-1-yl)propyl]amino}piperidin-1-yl)[1,3]thiazolo[4,5-f]-2,9-phenanthrolin-8(9H)-one;
2-{[(1S,2S)-1-(hydroxymcthyl)-2-methylbutyl]amino}[1,3]thiazolo[4,5-f]-2,9-phenanthrolin-8(9H)-one;
2-{4-[(3-morpholin-4-ylpropyl)amino]piperidin-1-yl}[1,3]thiazolo[4,5-f]-2,9-phenanthrolin-8(9H)-one;
2-(dimethylamino)-N-[1-(8-oxo-8,9-dihydro[1,3]thiazolo[4,5-f]-2,9-phenanthrolin-2-yl)piperidin-4-yl]acetamide;
2-(4-hydroxypiperidin-1-yl)[1,3]thiazolo[4,5-f]-2,9-phenanthrolin-8(9H)-one;
2-(propylamino)[1,3]thiazolo[4,5-f]-2,9-phenanthrolin-8(9H)-one;
2-(4-acetylpiperazin-1-yl)[1,3]thiazolo[4,5-f]-2,9-phenanthrolin-8(9H)-one;
2-piperazin-1-yl[1,3]thiazolo[4,5-f]-2,9-phenanthrolin-8(9H)-one;
2-{4-[(2S)-2-hydroxypropyl]piperazin-1-yl}[1,3]thiazolo[4,5-f]-2,9-phenanthrolin-8(9H)-one;
2-[4-(1H-imidazol-5-ylacetyl)piperazin-1-yl][1,3]thiazolo[4,5-f]-2,9-phenanthrolin-8(9H)-one;
2-{4-[(2R)-2-hydroxypropyl]piperazin-1-yl}[1,3]thiazolo[4,5-f]-2,9-phenanthrolin-8(9H)-one;
2-[4-(2-hydroxyethyl)piperazin-1-yl][1,3]thiazolo[4,5-f]-2,9-phenanthrolin-8(9H)-one;
2-{[(2R)-2-amino-3,3-dimethylbutyl]oxy}[1,3]thiazolo[4,5-f]-2,9-phenanthrolin-8(9H)-one;
2-{[(1S)-1-(hydroxymethyl)-2,2-dimethylpropyl]amino}[1,3]thiazolo[4,5-f]-2,9-phenanthrolin-8(9H)-one;
N-[1-(8-oxo-8,9-dihydro[1,3]thiazolo[4,5-f]-2,9-phenanthrolm-2-yl)piperidin-4-yl]-3-pyridin-3-ylpropanamide;
2-[4-(2-morpholin-4-ylethoxy)piperidin-1-yl][1,3]thiazolo[4,5-f]-2,9-phenanthrolin-8(9H)-one;
2-{4-[(2-morpholin-4-ylethoxy)methyl]piperidin-1-yl}[1,3]thiazolo[4,5-f]-2,9-phenanthrolin-8(9H)-one;
2-(4-{methyl[3-(2-oxopyrrolidin-1-yl)propyl]amino}piperidin-1-yl)[1,3]thiazolo[4,5-f]-2 9-phenanthrolin-8(9H)-one;
2-[4-(morpholin-4-ylmethyl)piperidin-1-yl][1,3]thiazolo[4,5-f]-2,9-phenanthrolin-8(9H)-one;
2-[4-(2-morpholin-4-ylethyl)piperidin-1-yl][1,3]thiazolo[4,5-f]-2,9-phenanthrolin-8(9H)-one;
2-(4-hydroxypiperidin-1-yl)[1,3]oxazolo[4,5-f]-2,9-phenanthrolin-8(9H)-one;
2-(4-oxopiperidin-1-yl)[1,3]oxazolo[4,5-f]-2,9-phenanthrolin-8(9H)-one;
2-(4-hydroxy-4-methylpiperidin-1-yl)[1,3]oxazolo[4,5-f]-2,9-phenanthrolin-8(9H)-one;
9-fluoro-2-(pyridin-4-ylmethoxy)-3,6-dihydro-7H-benzo[h]imidazo[4,5-f]isoquinolin-7-one;
9-fluoro-2-[(pyridin-3-ylmethyl)amino]-3,6-dihydro-7H-benzo[h]imidazo[4,5-f]isoquinolin-7-one;
9-fluoro-2-(3-piperidin-1-ylpropoxy)-1,6-dihydro-7H-benzo[h]imidazo[4,5-f]isoquinolin-7-onc;
9-fluoro-2-[(3-morpholin-4-ylpropyl)amino]-1,6-dihydro-7H-benzo[h]imidazo[4,5-f]isoquinolin-7-one;
9-fluoro-2-4-hydroxy-4-[(2-morpholin-4-ylethoxy)methyl]piperidin-1-yl-1,6-dihydro-7H-benzo[h]imidazo[4,5-f]isoquinolin-7-one;
9-fluoro-2-(pyridin-3-yloxy)-3,6-dihydro-7H-benzo[h]imidazo[4,5-f]isoquinolin-7-one;
9-fluoro-2-isopropoxy-3,6-dihydro-7H-benzo[h]imidazo[4,5-f]isoquinolin-7-one;
9-fluoro-2-(pyridin-3-ylmethoxy)-3,6-dihydro-7H-benzo[h]imidazo[4,5-f]isoquinolin-7-one;
9-fluoro-2-(4-oxopyridin-1(4H)-yl)-3,6-dihydro-7H-benzo[h]imidazo[4,5-f]isoquinolin-7-one;
9-fluoro-2-[(cis-4-hydroxycyclohexyl)oxy]-3,6-dihydro-7H-benzo[h]imidazo[4,5-f]isoquinolin-7-one;
9-fluoro-2-[(trans-4-hydroxycyclohexyl)oxy]-3,6-dihydro-7H-benzo[h]imidazo[4,5-f]isoquinolin-7-one;
9-fluoro-2-(pyridin-2-ylmethoxy)-3,6-dihydro-7H-benzo[h]imidazo[4,5-f]isoquinolin-7-one;
9-fluoro-2-[(trans-4-hydroxycyclohexyl)amino]-3,6-dihydro-7H-benzo[h]imidazo[4,5-f]isoquinolin-7-one;
9-fluoro-2-[(pyridin-4-ylmethyl)amino]-3,6-dihydro-7H-benzo[h]imidazo[4,5-f]isoquinolin-7-one;
9-fluoro-2-[(1-oxidopyridin-2-yl)methoxy]-3,6-dihydro-7H-benzo[h]imidazo[4,5-f]isoquinolin-7-one;
9-fluoro-2-[(1-oxidopyridin-3-yl)methoxy]-3,6-dihydro-7H-benzo[h]imidazo[4,5-f]isoquinolin-7-one;
9-fluoro-2-[(1R,3R)-3-hydroxycyclohexyl]oxy-3,6-dihydro-7H-benzo[h]imidazo[4,5-f]isoquinolin-7-one;
cis- and trans-9-fluoro-2-[3-hydroxycyclohexyl]oxy-3,6-dihydro-7H-benzo[h]imidazo[4,5-f]isoquinolin-7-one;
9-fluoro-2-[1-(methylsulfonyl)piperidin-4-yl]oxy-3,6-dihydro-7H-benzo[h]imidazo[4,5-f]isoquinolin-7-one;
2-[(1-acetylpiperidin-4-yl)oxy]-9-fluoro-3,6-dihydro-7H-benzo[h]imidazo[4,5-f]isoquinolin-7-one:
3-4-[(9-fluoro-7-oxo-6,7-dihydro-3H-benzo[h]imidazo[4,5-f]isoquinolin-2-yl)oxy]piperidin-1-yl-3-oxopropanenitrile;
9-fluoro-2-[(trans-4-hydroxycyclohexyl)(methyl)amino]-3,6-dihydro-7H-benzo[h]imidazo[4,5-f]isoquinolin-7-one;
9-fluoro-2-(3-morpholin-4-ylpropoxy)-1,6-dihydro-7H-benzo[h]imidazo[4,5-f]isoquinolin-7-one;
9-fluoro-2-[3-(2-oxopyrrolidin-1-yl)propyl]amino-1,6-dihydro-7H-benzo[h]imidazo[4,5-f]isoquinolin-7-one;
2-(4-{[bis(2-methoxyethyl)amino]methyl}piperidin-1-yl)-9-fluoro-1,6-dihydro-7H-benzo[h]imidazo[4,5-f]isoquinolin-7-one;
9-fluoro-2-{4-[(2-morpholin-4-ylethoxy)methyl]piperidin-1-yl}-1,6-dihydro-7H-benzo[h]imidazo[4,5-f]isoquinolin-7-one;
2-[(cyclopropylmethyl)(3-morpholin-4-ylpropyl)amino]-9-fluoro-1,6-dihydro-7H-benzo[h]imidazo[4,5-f]isoquinolin-7-one;
9-fluoro-2-4-hydroxy-4-[(3-piperidin-1-ylpropoxy)methyl]piperidin-1-yl-1,6-dihydro-7H-benzo[h]imidazo[4,5-f]isoqumolin-7-one;
9-fluoro-2-4-hydroxy-4-[(3-morpholin-4-ylpropoxy)methyl]piperidin-1-yl-1,6-dihydro-7H-benzo[h]imidazo[4,5-f]isoquinolin-7-one;
10-fluoro-2-[4-(hydroxyimino)cyclohexyl]benzo[c]imidazo[1,2-a]-1,6-naphthyridin-8(7H)-one;
9-fluoro-2-4-[(2-morpholin-4-ylethoxy)imino]piperidin-1-yl-1,6-dihydro-7H-benzo[h]imidazo[4,5-f]isoquinolin-7-one;
4-ethyl-4-(9-fluoro-7-hydroxy-3H-benzo[f]imidazo[4,5-h]phthalazin-2-yl)hexanenitrile;
4-ethyl-4-(9-fluoro-7-oxo-6,7-dihydro-3H-benzo[f]imidazo[4,5-h]phthalazin-2-yl)hexanoic acid;
4-ethyl-4-(9-fluoro-7-hydroxy-3H-benzo[f]imidazo[4,5-h]phthalazin-2-yl)hexanamide;
methyl 2-(9-fluoro-7-hydroxy-3H-benzo[f]imidazo[4,5-h]phthalazin-2-yl)-2-methylpropanoate;
2-(9-fluoro-7-hydroxy-3H-benzo[f]imidazo[4,5-h]phthalazin-2-yl)-2-methylpropanoic acid;
9-fluoro-2-(2-hydroxy-1,1-dimethylethyl)-3H-benzo[f]imidazo[4,5-h]phthalazin-7-ol;
4-(9-fluoro-7-oxo-6,7-dihydro-3H-benzo[f]imidazo[4,5-h]phthalazin-2-yl)-4-methylpentanenitrile;
(2E)-4-(9-fluoro-7-oxo-6,7-dihydro-3H-benzo[f]imidazo[4,5-h]phthalazin-2-yl)-4-methylpent-2-enenitrile;
9-fluoro-2-[4-(methylthio)phenyl]-3,6-dihydro-7H-benzo[f]imidazo[4,5-h]phthalazin-7-one;
9-fluoro-2-[4-(methylsulfinyl)phenyl]-3,6-dihydro-7H-benzo[f]imidazo[4,5-h]phthalazin-7-one;
4-(9-fluoro-7-hydroxy-3H-benzo[f]imidazo[4,5-h]phthalazin-2-yl)-4-methylpentanamide;
3-(9-fluoro-7-hydroxy-3H-benzo[f]imidazo[4,5-h]phthalazin-2-yl)propanenitrile;
9-fluoro-2-(2-pyridin-2-ylethyl)-3H-benzo[f]imidazo[4,5-h]phthalazin-7-ol;
9-fluoro-2-(pyridin-4-ylmethyl)-3,6-dihydro-7H-benzo[f]imidazo[4,5-h]phthalazin-7-one;
9-fluoro-2-(pyridin-3-ylmethyl)-3,6-dihydro-7H-benzo[f]imidazo[4,5-h]phthalazin-7-one;
2-[4-(benzyloxy)cyclohexyl]-9-fluoro-3,6-dihydro-7H-benzo[f]imidazo[4,5-h]phthalazin-7-one;
9-fluoro-2-(4-hydroxycyclohexyl)-3,6-dihydro-7H-benzo[f]imidazo[4,5-h]phthalazin-7-one;
methyl 2-(9-fluoro-7-hydroxybenzo[f][1,3]oxazolo[5,4-h]phthalazin-2-yl)-2-methylpropanoate;
benzyl [4-(9-fluoro-7-hydroxybenzo[f][1,3]oxazolo[5,4-h]phthalazin-2-yl)-4-methylpentyl]carbamate;
2-(4-amino-1,1-dimethylbutyl)-9-fluorobenzo[f][1,3]oxazolo[5,4-h]phthalazin-7-ol;
2-(9-fluoro-7-hydroxybenzo[f][1,3]oxazolo[5,4-h]phthalazin-2-yl)-2-methylpropanoic acid;
2-tert-butyl-1,9-dihydro-8H-imidazo[4,5-f]-2,8-phenanthrolin-8-one;
2-tert-butyl-3,6-dihydro-7H-imidazo[4,5-f]-1,9-phenanthrolin-7-one;
2-cyclohexyl-1,9-dihydro-8H-imidazo[4,5-f]-2,8-phenanthrolin-8-one;
3-(8-oxo-8,9-dihydro-1H-imidazo[4,5-f]-2,8-phenanthrolin-2-yl)propanenitrile;
2-(trans-4-hydroxycyclohexyl)-1,9-dihydro-8H-imidazo[4,5-f]-2,8-phenanthrolin-8-one;
2-[1-(methylsulfonyl)piperidin-4-yl]-1,9-dihydro-8H-imidazo[4,5-f]-2,8-phenanthrolin-8-one;
3-oxo-3-[4-(8-oxo-8,9-dihydro-1H-imidazo[4,5-f]-2,8-phenanthrolin-2-yl)piperidin-1-yl]propanenitrile;
3-methyl-3-(8-oxo-8,9-dihydro-1H-imidazo[4,5-f]-2,8-phenanthrolin-2-yl)butanenitrile;
2-tert-butyl-1,9-dihydro-8H-imidazo[4,5-f]-2,7-phenanthrolin-8-one;
2-cyclohexyl-1,9-dihydro-8H-imidazo[4,5-f]-2,7-phenanthrolin-8-one;
2-cyclopentyl-1,9-dihydro-8H-imidazo[4,5-f]-2,7-phenanthrolin-8-one;
2-tert-butyl-1,9-dihydro-8H-imidazo[4,5-f]-2,8-phenanthrolin-8-one 5-oxide; and
3-methyl-3-(5-oxido-8-oxo-8,9-dihydro-1H-imidazo[4,5-f]-2,8-phenanthrolin-2-yl)butanenitrile;
and pharmaceutically acceptable salts of any of the aforementioned.

In a fifth aspect, the agent is selected from compounds of Formula V: 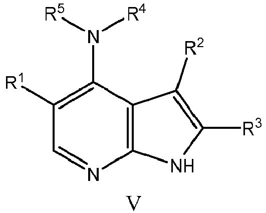 or pharmaceutically acceptable salt forms or prodrug thereof, wherein:
R¹, R², and R³ are each, independently, H, halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR^{a}, SR^{a}, C(O)R^{b}, C(O)NR^{c}R^{d}, C(O)OR³, OC(O)R^{b}, OC(O)NR^{c}R^{d}, NR^{c}R^{d}, NR^{c}C(O)R^{d}, NR^{c}C(O)OR^{a}, S(O)R^{b}, S(O)NR^{c}R^{d}, S(O)₂R^{b}, or S(O)₂NR^{c}R^{d};
R⁴ is H, C₁₋₄ alkyl, C₂₋₄ alkenyl, C₂₋₄ alkynyl, S(O)₂R⁹, SOR⁹, cycloalkyl, or heterocycloalkyl, wherein the C₁₋₄ alkyl, C₂₋₄ alkenyl, C₂₋₄ alkynyl, cycloalkyl, heterocycloalkyl are each optionally substituted with 1, 2 or 3 substituents selected from halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{d}, NR^{c}C(O)OR^{a}, S(O)R^{b}, S(O)NR^{c}R^{d}, S(O)₂R^{b}, and S(O)₂NR^{c}R^{d},
R⁵ is 3-8 membered cycloalkyl, 3-8 membered heterocycloalkyl, -L-(3-8 membered cycloalkyl), -L-(3-8 membered heterocycloalkyl), each substituted by one R⁶ and 0, 1 or 2 R⁷;
L is C₁₋₄ alkylenyl, C₂₋₄ alkenylenyl, C₂₋₄ alkynylenyl, O, S, NR¹⁴, CO, COO, OCO, NR¹⁴C(O)0, CONR¹⁴, SO, SO₂, SONR¹⁴, SO₂NR¹⁴, or NR¹⁴CONR¹⁴;
R⁶ is -W¹-W²-W³-W⁴-W⁵-W⁶-R¹³;
W¹ is absent, C₁₋₄ alkylenyl, C₂₋₄ alkenylenyl, C₂₋₄ alkynylenyl, aryl, heteroaryl, cycloalkyl or heterocycloalkyl, each optionally substituted by 1, 2 or 3 halo, CN, NO₂, OH, =NH, =NOH, =NO-(C₁-₄ alkyl), C₁₋₄ haloalkyl, C₁₋₄ alkoxy, C₁₋₄ haloalkoxy, amino, C₁₋₄ alkylamino or C₂₋₈ dialkylamino;
W² is absent, C₁₋₄ alkylenyl, C₂₋₄ alkenylenyl, C₂₋₄ alkynylenyl, O, S, NR¹², CO, COO, OCO, C(S), C(S)NR¹², -C(=N-CN)-, NR¹²C(O)O, CONR¹², SO, SO₂, SONR¹², SO₂NR¹², or NR¹²CONR¹², wherein the C₁₋₄ alkylenyl, C₂₋₄ alkenylenyl, C₂₋₄ alkynylenyl, are each optionally substituted by 1, 2 or 3 halo, OH, C₁₋₄alkoxy, C₁₋₄haloalkoxy, amino, C₁₋₄alkylamino or C₂₋₈ dialkylamino;
W³ is absent, C₁₋₄ alkylenyl, C₂₋₄alkenylenyl, C₂₋₄ alkynylenyl, aryl, cycloalkyl, heteroaryl or heterocycloalkyl, wherein the C₁₋₄ alkylenyl, C₂₋₄ alkenylenyl, C₂₋₄ alkynylenyl, aryl, cycloalkyl, heteroaryl or heterocycloalkyl is optionally substituted by 1, 2 or 3 halo, CN, NO₂, OH, =NH, =NOH, =NO-(C₁₋₄ alkyl), C₁₋₄ haloalkyl, C₁₋₄ alkoxy, C₁₋₄ haloalkoxy, amino, C₁₋₄ alkylamino or C₂₋₈ dialkylamino;
W⁴ is absent, C₁₋₄ alkylenyl, C₂₋₄ alkenylenyl, C₂₋₄ alkynylenyl, O, S, NR¹², CO, COO, OCO,-C(=N-CN)-, NR¹²C(O)O, CONR¹², SO, SO₂, SONR¹², SO₂NR¹², or NR¹²CONR¹², wherein the C₁₋₄ alkylenyl, C₂₋₄ alkenylenyl, C₂₋₄ alkynylenyl, are each optionally substituted by 1, 2 or 3 halo, CN, NO₂, OH, =NH, =NOH, =NO-(₁₋₄ alkyl), C₁₋₄ haloalkyl, C₁₋₄alkoxy, C₁₋₄haloalkoxy, amino, C₁₋₄ alkylamino or C₂₋₈ dialkylamino;
W⁵ is absent, C₁₋₄ alkylenyl, C₂₋₄ alkenylenyl, C₂₋₄ alkynylenyl, aryl, cycloalkyl, heteroaryl or heterocycloalkyl, wherein the C₁₋₄ alkylenyl, C₂₋₄alkenylenyl, C₂₋₄ alkynylenyl, aryl, cycloalkyl, heteroaryl or heterocycloalkyl is optionally substituted by 1, 2 or 3 halo, CN, NO₂, OH, =NH, =NOH, =NO-(C₁₋₄ alkyl), C₁₋₄ haloalkyl, C₁₋₄ alkoxy, C₁₋₄ haloalkoxy, amino, C₁₋₄ alkylamino or C₂₋₈ dialkylamino;
W⁶ is absent, C₁₋₄ alkyl, C₂₋₄ alkenyl, C₂₋₄ alkynyl, O, S, NR¹², CO, COO, OCO, -C(=N-CN)-, NR¹²C(O)O, CONR¹², SO, SO₂, SONR¹², SO₂NR¹², or NR¹²CONR¹², wherein the C₁₋₄ alkyl, C₂₋₄ alkenyl, C₂₋₄ alkynyl arc each optionally substituted by 1, 2 or 3 CN, NO₂, OH, =NH, =NOH, =NO-(C₁₋₄ alkyl), C₁₋₄haloalkyl, C₁₋₄ alkoxy, C₁₋₄ haloalkoxy, amino, C₁₋₄alkylamino or C₂₋₈ dialkylamino;
R⁷ is halo, C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, CN, NO₂, OR^{a.}, SR^{a.}, C(O)R^{b"}, C(O)NR^{c"}R^{d"} , C(O)OR^{a"} OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{d"}, S(O)₂R^{b"}, S(O)₂NR^{c"}R^{d"}, -(C₁₋₆alkyl)-CN, -(C₁₋₆alkyl)-NO₂, - (C₁₋₆ alkyl)-OR^{a"}, -(C₁₋₆alkyl)-SR^{a"}, -(C₁₋₆ alkyl)-C(O)R^{b"}, -(C₁₋₆ alkyl)-C(O)NR^{c"}R^{d"}, -(C₁₋₆ alkyl)-C(O)OR^{a"}, -(C₁₋₆alkyl)-OC(O)R^{b"}, -(C₁₋₆ alkyl)-OC(O)NR^{c"}Rd^{"}, -(C₁₋₆ alkyl)-NR^{c"}R^{d"}, -(C₁₋₆alkyl)-NR^{c"}C(O)R^{d"}, -(C₁₋₆alkyl)-NR^{c"}C(O)OR^{a"}, -(C₁₋₆alkyl)-S(O)^{b"}, -(C₁₋₆alkyl)-S(O)NR^{c"}R^{d"}, -(C₁₋₆ alkyl)-S(O)₂R^{b"}, or -(C₁₋₆alkyl)-S(O)₂NR^{c"}R^{d"};
R⁹ is C₁₋₄ alkyl, aryl, heteroaryl, cycloalkyl, or heterocycloalkyl, each optionally substituted with 1, 2, or 3 substituents selected from halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR^{a'}, SR^{a'}, C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{d'}, NR^{c'}C(O)OR^{a'}, S(O)R^{b'}, S(O)NR^{c}R^{d'}, S(O)₂R^{b'}, and S(O)₂NR^{c}R^{d};
R¹² and R¹⁴ are each, independently, H or C₁₋₆ alkyl optionally substituted by 1, 2 or 3 substituents selected from OH, CN, NO₂, amino, (C₁₋₄ alkyl)amino, (C₂₋₈ dialkyl)amino, C₁₋₆ haloalkyl, C₁₋₆ acyl, C₁₋₆ acyloxy, C₁₋₆ acylamino, -(C₁₋₆ alkyl)-CN, and -(C₁₋₆ alkyl)-NO₂;
R¹³ is halo, C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{d"}, S(O)₂R^{b"}, S(O)₂NR^{c"}R^{d"}, -(C₁₋₆ alkyl)-CN, -(C₁₋₆ alkyl)-NO₂,-(C₁₋₆alkyl)-OR^{a"}, -(C₁₋₆ alkyl)-SR^{a"}, -(C₁₋₆ alkyl)-C(O)R^{b"}, -(C₁₋₆ alkyl)-C(O)NR^{c"}R^{d"}, -(C₁₋₆ alkyl)-C(O)OR^{a"}, -(C₁₋₆ alkyl)-OC(O)R^{b"}, -(C₁₋₆ alkyl)-OC(O)NR^{c"}R^{d"}, -(C₁₋₆ alkyl)-NR^{c"}R^{d"}, -(C₁₋₆ alkyl)-NR^{c"}C(O)R^{d"}, -(C₁₋₆ alkyl)-NR^{c"}C(O)OR^{a"}, -(C₁₋₆ alkyl)-S(O)R^{b"}, -(C₁₋₆ alkyl)-S(O)NR^{c"}R^{d"}, -(C₁₋₆alkyl)-S(O)₂R^{b"}, or -(C₁₋₆ alkyl)-S(O)₂NR^{c"}R^{d"}, wherein each of the C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl or heterocycloalkylalkyl is optionally substituted by 1, 2, 3, 4 or 5 substituents independently selected from:
C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, halo, CN, NO₂, OR^{a"}, SR^{a"}, C(O)R^{b"}, C(O)NR^{c"}R^{d"} , C(O)OR^{a"}, OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{d"}, S(O)₂R^{b"}, S(O)₂NR^{c"}R^{d"},
   -(C₁₋₆alkyl)-CN, -(C₁₋₆alkyl)-NO₂, -(C₁₋₆alkyl)-OR^{a"}, -(C₁₋₆alkyl)-SR^{a"},
   -(C₁₋₆ alkyl)-C(O)R^{b"}, -(C₁₋₆ alkyl)-C(O)NR^{c"}R^{d"}, -(C₁₋₆ alkyl)-C(O)OR^{a"},
   -(C₁₋₆ alkyl)-OC(O)R^{b"}, -(C₁₋₆ alkyl)-OC(O)NR^{c"}R^{d"}, -(C₁₋₆ alkyl)-NR^{c"}R^{d"},
   -(C₁₋₆ alkyl)-NR^{c"}C(O)R^{d"}, -(C₁₋₆ alkyl)-NR^{c"}C(O)OR^{a"}, -(C₁₋₆ alkyl)-S(O)R^{b"},
   -(C₁₋₆alkyl)-S(O)NR^{c"}R^{d"}, -(C₁₋₆ alkyl)-S(O)₂R^{b"}, and -(C₁₋₆ alkyl)-S(O)₂NR^{c"}R^{d"};
R^{a}, R^{a'} and R^{a"} are each, independently, H, C₁₋₆ alkyl, C₁₋₆ haloalkyl, C₂₋₆ alkenyl, C₂₋₆ alkynyl, aryl, cycloalkyl, heteroaryl or heterocycloalkyl;
R^{b}, R^{b} and R^{b"} are each, independently, H, C₁₋₆ alkyl, C₁₋₆ haloalkyl, C₂₋₆ alkenyl, C₂₋₆ alkynyl, aryl, cycloalkyl, heteroaryl or heterocycloalkyl;
R^{c} and R^{d} are each, independently, H, C₁₋₆ alkyl, C₁₋₆ haloalkyl, C₂₋₆ alkenyl, C₂₋₆ alkynyl, aryl, cycloalkyl, arylalkyl, or cycloalkylalkyl;
or R^{c} and R^{d} together with the N atom to which they are attached form a 4-, 5-, 6- or 7-membered heterocycloalkyl group;
R^{c'} and R^{d'} are each, independently, H, C₁₋₆ alkyl, C₁₋₆ haloalkyl, C₂₋₆ alkenyl, C₂₋₆ alkynyl, aryl, cycloalkyl, arylalkyl, or cycloalkylalkyl;
or R^{c'} and R^{d'} together with the N atom to which they are attached form a 4-, 5-, 6- or 7-membered heterocycloalkyl group; and
R^{c"} and R^{d"} are each, independently, H, C₁₋₆ alkyl, C₁₋₆ haloalkyl, C₂₋₆ alkenyl, C₂₋₆ alkynyl, aryl, cycloalkyl, arylalkyl, or cycloalkylalkyl;
or R^{c"} and R^{d"} together with the N atom to which they are attached form a 4-, 5-, 6- or 7-membered heterocycloalkyl group.

In a sixth aspect, the agent is selected from the compounds of Formula VI: 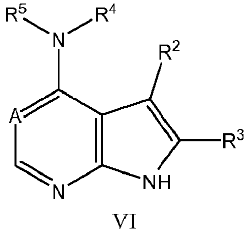 or pharmaceutically acceptable salt forms or prodrugs thereof, wherein:
A is N or CR¹;
R¹, R², and R³ are each, independently, H, halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{d}, NR^{c}C(O)OR^{a}, S(O)R^{b}, S(O)NR^{c}R^{d}, S(O)₂R^{b}, or S(O)₂NR^{c}R^{d};
R⁴ is H, C₁₋₄ alkyl, C₂₋₄ alkynyl, C₂₋₄ alkynyl, S(O)₂R⁹, SOR⁹, cycloalkyl, or heterocycloalkyl, wherein the C₁₋₄ alkyl, C₂₋₄ alkenyl, C₂₋₄ alkynyl, cycloalkyl, heterocycloalkyl are each optionally substituted with 1, 2 or 3 substituents selected from halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{d}, NR^{c}C(O)OR^{a}, S(O)R^{b}, S(O)NR^{c}R^{d}, S(O)₂R^{b}, and S(O)₂NR^{c}R^{d};
R⁵ is 3-8 membered cycloalkyl, 3-8 membered heterocycloalkyl, -L-(3-8 membered cycloalkyl), -L-(3-8 membered heterocycloalkyl), each substituted by one R⁶ and 0, 1 or 2 R⁷;
L is C₁₋₄ alkylenyl, C₂₋₄ alkenylenyl, C₂₋₄ alkynylenyl, O, S, NR¹⁴, CO, COO, OCO, NR¹⁴C(O)O, CONR¹⁴, SO, SO₂, SONR¹⁴, SO₂NR¹⁴, or NR¹⁴CONR¹⁴;
R⁶ is -W¹-W²-W³-W⁴-W⁵-W⁶-R¹³;
W¹ is absent, C₁₋₄ alkylenyl, C₂₋₄ alkenylenyl, C₂₋₄ alkynylenyl, aryl, heteroaryl, cycloalkyl or heterocycloalkyl, each optionally substituted by 1, 2 or 3 halo, CN, NO₂, OH, =NH, =NOH, =NO-(C₁₋₄ (alkyl), C₁₋₄haloalkyl, C₁₋₄alkoxy, C₁₋₄haloalkoxy, amino, C₁₋₄alkylamino or C₂₋₈dialkylamino;
W² is absent, C₁₋₄ alkylenyl, C₂₋₄ alkenylenyl, C₂₋₄ alkynylenyl, O, S, NR¹², CO, COO, OCO, C(S), C(S)NR¹², -C(=N-CN)-, NR¹²C(O)O, CONR¹², SO, SO₂, SONR¹², SO₂NR¹², or NR¹²CONR¹², wherein the C₁₋₄ alkylenyl, C₂₋₄ alkenylenyl, C₂₋₄ alkynylenyl, are each optionally substituted by 1, 2 or 3 halo, OH, C₁₋₄alkoxy, C₁₋₄ haloalkoxy, amino, C₁₋₄ alkylamino or C₂₋₈ dialkylamino;
W³ is absent, C₁₋₄ alkylenyl, C₂₋₄ alkenylenyl, C₂₋₄ alkynylenyl, aryl, cycloalkyl, heteroaryl or heterocycloalkyl, wherein the C₁₋₄ alkylenyl, C₂₋₄ alkenylenyl, C₂₋₄ alkynylenyl, aryl, cycloalkyl, heteroaryl or heterocycloalkyl is optionally substituted by 1, 2 or 3 halo, CN, NO₂, OH, =NH, =NOH, =NO-(C₁₋₄ alkyl), C₁₋₄ haloalkyl, C₁₋₄ alkoxy, C₁₋₄ haloalkoxy, amino, C₁₋₄ alkylamino or C₂₋₈ dialkylamino;
W⁴ is absent, C₁₋₄ alkylenyl, C₂₋₄ alkenylenyl, C₂₋₄ alkynylenyl, O, S, NR¹², CO, COO, OCO,-C(=N-CN)-, NR¹²C(O)O, CONR¹², SO, SO₂, SONR¹², SO₂NR¹², or NR¹²CONR¹², wherein the C₁₋₄ alkylenyl, C₂₋₄ alkenylenyl, C₂₋₄ alkynylenyl, are each optionally substituted by 1, 2 or 3 halo, CN, NO₂, OH, =NH, =NOH, =NO-(C₁₋₄ alkyl), C₁₋₄ haloalkyl, C₁₋₄ alkoxy, C₁₋₄ haloalkoxy, amino, C₁₋₄ alkylamino or C₂₋₈ dialkylamino;
W⁵ is absent, C₁₋₄ alkylenyl, C₂₋₄ alkenylenyl, C₂₋₄ alkynylenyl, aryl, cycloalkyl, heteroaryl or heterocycloalkyl, wherein the C₁₋₄ alkylenyl, C₂₋₄ alkenylenyl, C₂₋₄ alkynylenyl, aryl, cycloalkyl, heteroaryl or heterocycloalkyl is optionally substituted by 1, 2 or 3 halo, CN, NO₂, OH, =NH, =NOH, =NO-(C₁₋₄ alkyl), C₁₋₄ haloalkyl, C₁₋₄ alkoxy, C₁₋₄ haloalkoxy, amino, C₁₋₄ alkylamino or C₂₋₈ dialkylamino;
W⁶ is absent, C₁₋₄ alkyl, C₂₋₄ alkenyl, C₂₋₄ alkynyl, O, S, NR¹², CO, COO, OCO, -C(=N-CN)-, NR¹²C(O)O, CONR¹², SO, SO₂, SONR¹², SO₂NR¹², or NR¹²CONR¹², wherein the C₁₋₄ alkyl, C₂₋₄ alkenyl, C₂₋₄ alkynyl are each optionally substituted by 1, 2 or 3 CN, NO₂, OH, =NH, =NOH, =NO-(C₁₋₄ alkyl), C₁₋₄ haloalkyl, C₁₋₄ alkoxy, C₁₋₄ haloalkoxy, amino, C₁₋₄ alkylamino or C₂₋₈ dialkylamino;
R⁷ is halo, C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, CN, NO₂, OR^{a"}, SR^{a"}, C(O)R^{b"}, C(O)NR^{c"}R^{d"} , C(O)OR^{a}, OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{d"}, S(O)₂R^{b"}, S(O)₂NR^{c"}R^{d"}, -(C₁₋₆ alkyl)-CN, -(C₁₋₆ alkyl)-NO₂,-(C₁₋₆alkyl)-OR^{a"}, -(C₁₋₆alkyl)-SR^{a"}, -(C₁₋₆alkyl)-C(O)R^{b"}, -(C₁₋₆alkyl)-C(O)NR^{c"}R^{d"}, -(C₁₋₆ alkyl)-C(O)OR^{a"}, -(C₁₋₆alkyl)-OC(O)R^{b"}, -(C₁₋₆alkyl)-OC(O)NR^{c"}R^{d"}, -(C₁₋₆alkyl)-NR^{c"}R^{d"}, -(C₁₋₆alkyl)-NR^{c"}C(O)R^{d"}, -(C₁₋₆alkyl)-NR^{c"}C(O)OR^{a"}, -(C₁₋₆alkyl)-S(O)R^{b"} -(C₁₋₆alkyl)-S(O)NR^{c"}R^{d"}, -(C₁₋₆ alkyl)-S(O)₂R^{b"}, or -(C₁₋₆ alkyl)-S(O)₂NR^{c"}R^{d"};
R⁹ is C₁₋₄ alkyl, aryl, heteroaryl, cycloalkyl, or heterocycloalkyl, each optionally substituted with 1, 2, or 3 substituents selected from halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR^{a'}, SR^{a} , C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{d'}, NR^{c'}C(O)OR^{a'}, S(O)R^{b'}, S(O)NR^{c'}R^{d'}, S(O)₂R^{b'}, and S(O)₂NR^{c'}R^{d'};
R¹² and R¹⁴ are each, independently, H or C₁₋₆ alkyl optionally substituted by 1, 2 or 3 substituents selected from OH, CN, NO₂, amino, (C₁₋₄ alkyl)amino, (C₂₋₈ dialkyl)amino, C₁₋₆ haloalkyl, C₁₋₆ acyl, C₁₋₆ acyloxy, C₁₋₆ acylamino, -(C₁₋₆ alkyl)-CN, and -(C₁₋₆ alkyl)-NO₂;
R¹³ is halo, C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{d"},
   NR^{c"}C(O)OR^{a"}, S(O)R^{b"}, S(O)NR^{c"}R^{d"}, S(O)₂R^{b"}, S(O)₂NR^{c"}R^{d"}, -(C₁₋₆ alkyl)-CN, -(C₁₋₆ alkyl)-NO₂,-(C₁₋₆ alkyl)-OR^{a"}, -(C₁₋₆ alkyl)-SR^{a"},
   -(C₁₋₆ alkyl)-C(O)R^{b"}, -(C₁₋₆ alkyl)-C(O)NR^{c"}R^{d"}, -(C₁₋₆ alkyl)-C(O)OR^{a"},
   -(C₁₋₆ alkyl)-OC(O)^{b"}, -(C₁₋₆ alkyl)-OC(O)NR^{c"}R^{d"}, -(C₁₋₆ alkyl)-NR^{c"}R^{d"},
   -(C₁₋₆ alkyl)-NR^{c"}C(O)R^{d"}, -(C₁₋₆alkyl)-NR^{c"}C(O)OR^{a"}, -(C₁₋₆alkyl)-S(O)R^{b"},
   -(C₁₋₆ alkyl)-S(O)NR^{c"}R^{d"}, -(C₁₋₆alkyl)-S(O)₂R^{b"}, or -(C₁₋₆ alkyl)-S(O)₂NR^{c"}R^{d"}, wherein each of the C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl or heterocycloalkylalkyl is optionally substituted by 1, 2, 3, 4 or 5 substituents independently selected from:
C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, halo, CN, NO₂, OR^{a"}, SR^{a"}, C(O)R^{b"}, C(O)NR^{c"}R^{d"} , C(O)OR^{a"}, OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{d"}, S(O)₂R^{b"}, S(O)₂NR^{c"}R^{d"},
   -(C₁₋₆alkyl)-CN, -(C₁₋₆alkyl)-NO₂, -(C₁₋₆alkyl)-OR^{a"}, -(C₁₋₆alkyl)-SR^{a"},
   -(C₁₋₆ alkyl)-C(O)R^{b"}, -(C₁₋₆ alkyl)-C(O)NR^{c"}R^{d"}, -(C₁₋₆ alkyl)-C(O)OR^{a"},
   -(C₁₋₆ alkyl)-OC(O)R^{b"}, -(C₁₋₆ alkyl)-OC(O)NR^{c"}R^{d"}, -(C₁₋₆ alkyl)-NR^{c"}R^{d"},
   -(C₁₋₆ alkyl)-NR^{c"}C(O)R^{d"}, -(C₁₋₆ alkyl)-NR^{c}C(O)OR^{a"}, -(C₁₋₆ alkyl)-S(O)R^{b"},
   -(C₁₋₆alkyl)-S(O)NR^{c"}R^{d"}, -(C₁₋₆alkyl)-S(O)₂R^{b"}, and -(C₁₋₆alkyl)-S(O)₂NR^{c"}R^{d"};
R^{a}, R^{a} and R^{a"} are each, independently, H, C₁₋₆ alkyl, C₁₋₆ haloalkyl, C₂₋₆ alkenyl, C₂₋₆ alkynyl, aryl, cycloalkyl, heteroaryl or heterocycloalkyl;
R^{b}, R^{b} and R^{b} are each, independently, H, C₁₋₆ alkyl, C₁₋₆ haloalkyl, C₂₋₆ alkenyl, C₂₋₆ alkynyl, aryl, cycloalkyl, heteroaryl or heterocycloalkyl;
R^{c} and R^{d} are each, independently, H, C₁₋₆ alkyl, C₁₋₆ haloalkyl, C₂₋₆ alkenyl, C₂₋₆ alkynyl, aryl, cycloalkyl, arylalkyl, or cycloalkylalkyl;
or R^{c} and R^{d} together with the N atom to which they are attached form a 4-, 5-, 6- or 7-membered heterocycloalkyl group;
R^{c'} and R^{d'} are each, independently, H, C₁₋₆ alkyl, C₁₋₆ haloalkyl, C₂₋₆ alkenyl, C₂₋₆ alkynyl, aryl, cycloalkyl, arylalkyl, or cycloalkylalkyl;
or R^{c'} and R^{d'} together with the N atom to which they are attached form a 4-, 5-, 6- or 7-membered heterocycloalkyl group; and
R^{c"} and R^{d"} are each, independently, H, C₁₋₆ alkyl, C₁₋₆ haloalkyl, C₂₋₆ alkenyl, C₂₋₆ alkynyl, aryl, cycloalkyl, arylalkyl, or cycloalkylalkyl;
or R^{c"} and R^{d"} together with the N atom to which they are attached form a 4-, 5-, 6- or 7-membered heterocycloalkyl group.

In some embodiments of the compounds of Formula V or VI, A is CR¹.

In some embodiments of the compounds of Formula V or VI, A is N.

In some embodiments of the compounds of Formula V or VI, R¹ is H.

In some embodiments of the compounds of Formula V or VI, R² is H.

In some embodiments of the compounds of Formula V or VI, R³ is H.

In some embodiments of the compounds of Formula V or VI, R⁴ is H or C₁₋₄ alkyl.

In some embodiments of the compounds of Formula V or VI, R⁴ is methyl.

In some embodiments of the compounds of Formula V or VI, R⁵ is 3-8 membered heterocycloalkyl substituted by one R⁶ and 0, 1 or 2 R⁷.

In some embodiments of the compounds of Formula V or VI, R⁵ is 6-membered heterocycloalkyl substituted by one R⁶ and 0, 1 or 2 R⁷.

In some embodiments of the compounds of Formula V or VI, R⁵ is piperidinyl substituted by one R⁶ and 0, 1 or 2 R⁷.

In some embodiments of the compounds of Formula V or VI, R⁵ is piperidin-3-yl substituted by one R⁶ and 0, 1 or 2 R⁷.

In some further embodiments of the compounds of Formula V or VI, R⁶ is substituted on the piperidinyl N-atom.

In some embodiments of the compounds of Formula V or VI, R⁵ is -L-pyrrolidinyl; L is C₁₋₄ alkylenyl; and the pyrrolidinyl is substituted by one R⁶ and 0, 1 or 2 R⁷.

In some embodiments of the compounds of Formula V or VI, R⁵ is -L-pyrrolidin-2-yl; L is C₁₋₄ alkylenyl and the pyrrolidin-2-yl is substituted by one R⁶ and 0, 1 or 2 R⁷.

In some embodiments of the compounds of Formula V or VI, W² is absent, C₁₋₄ alkylenyl, C₂₋₄ alkenylenyl, C₂₋₄ alkynylenyl, O, S, NR¹², CO, COO, OCO, -C(=N-CN)-, NR¹²C(O)O, CONR¹², SO, SO₂, SONR¹², SO₂NR¹², or NR¹²CONR¹², wherein the C₁₋₄ alkylenyl, C₂₋₄ alkenylenyl, C₂₋₄ alkynylcnyl, arc cach optionally substituted by 1, 2 or 3 substitucnts independently selected from halo, OH, C₁₋₄ alkoxy, C₁₋₄ haloalkoxy, amino, C₁₋₄ alkylamino, and C₂₋₈ dialkylamino.

In some embodiments of the compounds of Formula V or VT, W² is SO₂, CO, COO, C(S)NR¹², or CONR¹².

In some embodiments of the compounds of Formula V or VI, W² is SO₂, CO, COO, C(S)NH, CONH or -CON(C₁₋₄ alkyl)-.

In some embodiments of the compounds of Formula V or VI, W² is SO₂ or CO.

In some embodiments of the compounds of Formula V or VI, W³ is C₁₋₄ alkylenyl or cycloalkyl.

In some embodiments of the compounds of Formula V or VI, R¹³ is halo, C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, CN, NO₂, OR^{a"}, SR^{a"}, C(O)R^{b"} , C(O)NR^{c"}R^{d"}, C(O)OR^{a"}, OC(O)R^{b"}, OC(O)NR^{c"}Rd^{"}, NR^{c"}R^{d"}, NR^{c"}C(O)R^{d"}, NR^{c"}C(O)OR^{a"}, S(O)R^{b"}, S(O)NR^{c"}R^{d"}, S(O)₂R^{b"}, S(O)₂NR^{c"}R^{d"}, -(C₁₋₆alkyl)-CN, -(C₁₋₆ alkyl)-NO₂, -(C₁₋₆ alkyl)-OR^{a"}, -(C₁₋₆ alkyl)-SR^{a"}, -(C₁₋₆ alkyl)-C(O)R^{b"}, -(C₁₋₆ alkyl)-C(O)NR^{c"}R^{d"}, -(C₁₋₆ alkyl)-C(O)OR^{a"}, -(C₁₋₆ alkyl)-OC(O)R^{b"}, -(C₁₋₆ alkyl)-OC(O)NR^{c"}R^{d"}, -(C₁₋₆alkyl)-NR^{c"}R^{d"}, -(C₁₋₆ alkyl)-NR^{c"}C(O)R^{d"}, -(C₁₋₆alkyl)-NR^{c"}C(O)OR^{a"}, - (C₁₋₆ alkyl)-S(O)R^{b"}, -(C₁₋₆ alkyl)-S(O)NR^{c"}R^{d"}, -(C₁₋₆ alkyl)-S(O)₂R^{b"}, or -(C₁₋₆ alkyl)-S(O)₂NR^{c"}R^{d"}.

In some embodiments of the compounds of Formula V or VI, R¹³ is C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from:
C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, halo, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{d"}, S(O)₂R^{b"}, S(O)₂NR^{c"Rd"},
   -(C₁₋₆ alkyl)-CN, -(C₁₋₆ alkyl)-NO₂, -(C₁₋₆ alkyl)-OR^{a"}, -(C₁₋₆ alkyl)-SR^{a"},
   -(C₁₋₆ alkyl)-C(O)R^{b"}, -(C₁₋₆ alkyl)-C(O)NR^{c"}R^{d"}, -(C₁₋₆ alkyl)-C(O)OR^{a"},
   -(C₁₋₆ alkyl)-OC(O)R^{b"}, -(C₁₋₆ alkyl)-OC(O)NR^{c"}R^{d".}, -(C₁₋₆ alkyl)-NR^{c"}R^{d"},
   -(C₁₋₆ alkyl)-NR^{c"}C(O)R^{d"}, -(C₁₋₆ alkyl)-NR^{c"}C(O)OR^{a"}, -(C₁₋₆ alkyl)-S(O)R^{b"},
   -(C₁₋₆ alkyl)-S(O)NR^{c"}R^{d"}, -(C₁₋₆ alkyl)-S(O)₂R^{b"}, and -(C₁₋₆ alkyl)-S(O)₂NR^{c"}R^{d"}.

In some embodiments of the compounds of Formula V or VI, R¹³ is aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from:
C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, halo, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{c"}, S(O)₂R^{b"}, S(O)₂NR^{c"}R^{d"},
   -(C₁₋₆ alkyl)-CN, -(C₁₋₆ alkyl)- NO₂, -(C₁₋₆ alkyl)-OR^{a"}, -(C₁₋₆ alkyl)-SR^{a"},
   -(C₁₋₆ alkyl)-C(O)R^{b"}, -(C₁₋₆ alkyl)-C(O)NR^{c"}R^{d"}, -(C₁₋₆ alkyl)-C(O)OR^{a"},
   -(C₁₋₆ alkyl)-OC(O)R^{b"}, -(C₁₋₆ alkyl)-OC(O)NR^{c"}R^{d"}, -(C₁₋₆ alkyl)-NR^{c"}R^{d"},
   -(C₁₋₆ alkyl)-NR^{c"}C(O)R^{d"}, -(C₁₋₆ alkyl)-NR^{c"}C(O)OR^{a"}, -(C₁₋₆ alkyl)-S(O)R^{b"},
   -(C₁₋₆alkyl)-S(O)NR^{c"}R^{d"}, -(C₁₋₆ alkyl)-S(O)₂R^{b"}, and -(C₁₋₆ alkyl)-S(O)₂NR^{c"}R^{d"}.

In some embodiments of the compounds of Formula V or VI, R¹³ is C₁₋₆ alkyl, C₁₋₆ haloalkyl, aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from:
C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, halo, CN, NO₂, OR^{a"}, Sura, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{c"}, S(O)₂R^{b"}, S(O)₂NR^{c"}R^{d"},
   -(C₁₋₆ alkyl)-CN, -(C₁₋₆ alkyl)-NO₂, -(C₁₋₆ alkyl)-OR^{a"}, -(C₁₋₆ alkyl)-SR^{a"},
   -(C₁₋₆ alkyl)-C(O)R^{b"}, -(C₁₋₆ alkyl)-C(O)NR^{c"}R^{d"}, -(C₁₋₆ alkyl)-C(O)OR^{a"},
   -(C₁₋₆ alkyl)-OC(O)R^{b"}, -(C₁₋₆ alkyl)-OC(O)NR^{c"}R^{d"}, -(C₁₋₆ alkyl)-NR^{c"}R^{d"},
   -(C₁₋₆ alkyl)-NR^{c"}C(O)R^{d"}, -(C₁₋₆ alkyl)-NR^{c"}C(O)OR^{a"}, -(C₁₋₆ alkyl)-S(O)R^{b"},
   -(C₁₋₆ alkyl)-S(O)NR^{c"} R^{d"}, -(C₁₋₆ allyl)-S(O)₂R^{b"}, and -(C₁₋₆ akyl)-S(O)₂NR^{c"}R^{c"}.

In some embodiments of the compounds of Formula V or VI, R¹³ is C₁₋₆ alkyl, C₁₋₆ haloalkyl, aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from C₁₋₆ alkyl, C₁₋₆ haloalkyl, aryl, heteroaryl, halo, CN, OR^{a"}, SR^{a"}, C(O)R^{b"}, C(O)OR^{a"}, NR^{c"}C(O)R^{d"}, S(O)₂R^{b"}, and -(C₁₋₆ alkyl)-CN.

In some embodiments of the compounds of Formula V or VI, R¹³ is aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from C₁₋₆ alkyl, C₁₋₆ haloalkyl, aryl, heteroaryl, halo, CN, OR^{a"}, SR^{a"}, C(O)R^{b"}, C(O)OR^{a"}, NR^{c"}C(O)R^{d"}, S(O)₂R^{b"}, and-(C₁₋₆ alkyl)-CN.

In some embodiments of the compounds of Formula V or VI, R¹³ is C₁₋₆ alkyl, C₁₋₆ haloalkyl, aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from C₁₋₆ alkyl, C₁₋₆ haloalkyl, aryl, heteroaryl, halo, CN, OH, C₁₋₄ alkoxy, C₁₋₄ haloalkoxy, heteroaryloxy, aryloxy, -SC₁₋₆ alkyl, -C(O)-C₁₋₆ alkyl, -C(O)OC₁₋₆ alkyl, -S(O)₂C₁₋₆ alkyl, -NHC(O)-C₁₋₆ alkyl, and -(C₁₋₆ alkyl)-CN.

In some embodiments of the compounds of Formula V or VI, R¹³ is aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from C₁₋₆ alkyl, C₁₋₆ haloalkyl, aryl, heteroaryl, halo, CN, OH, C₁₋₄ alkoxy, C₁₋₄ haloalkoxy, heteroaryloxy, aryloxy, -SC₁₋₆ alkyl, -C(O)-C₁₋₆ alkyl, -C(O)OC₁₋₆ alkyl, -S(O)₂C₁₋₆ alkyl, -NHC(O)-C₁₋₆ alkyl, and -(C₁₋₆ alkyl)-CN.

In some embodiments of the compounds of Formula V or VI, R¹³ is aryl, cycloalkyl, heteroaryl or heterocycloalkyl, each optionally substituted by 1, 2, 3, 4 or 5 substituents independently selected from C₁₋₆ alkyl, C₁₋₆ haloalkyl, aryl, heteroaryl, halo, CN, OH, C₁₋₄ alkoxy, C₁₋₄ haloalkoxy, heteroaryloxy, aryloxy, -SCₗ₋₆ alkyl, -C(O)-C₁₋₆ alkyl, -C(O)OC₁₋₆ alkyl, -S(O)₂C₁₋₆ alkyl, -NHC(O)-C₁₋₆ alkyl, and -(C₁₋₆ alkyl)-CN.

In some embodiments of the compounds of Formula V or VI, R¹³ is arylalkyl, cycloalkylalkyl, heteroarylalkyl or heterocycloalkylalkyl, each optionally substituted by 1, 2, 3, 4 or 5 substituents independently selected from C₁₋₆ alkyl, C₁₋₆ haloalkyl, aryl, heteroaryl, halo, CN, OH, C₁₋₄ alkoxy, C₁₋₄ haloalkoxy, heteroaryloxy, aryloxy, -SC₁_₆ alkyl, -C(O)-C₁₋₆ alkyl, -C(O)OC₁₋₆ alkyl, - S(O)₂C₁₋₆ alkyl, -NHC(O)-C₁₋₆ alkyl, and -(C₁₋₆ alkyl)-CN.

In some embodiments of the compounds of Formula V or VI, R¹³ is OH or CN.

In some embodiments of the compounds of Formula V or VI, W¹ is absent, W² is CO or SO₂, W³ is C₁₋₄ alkyleneyl or cycloalkyl, W⁴ is absent, W⁵ is absent, W⁶ is absent, and R¹³ is CN or OH.

In some embodiments of the compounds of Formula V or VI, R⁶ is -W²-W³-R¹³.

In some embodiments of the compounds of Formula V or VI, R⁶ is -CO-CH₂-CN.

In some embodiments of the compounds of Formula V or VI, R⁶ is -W²-R¹³.

In some embodiments of the compounds of Formula V or VI, R⁶ is R¹³.

In some embodiments of the compounds of Formula V or VI:
R⁶ is -W² -R¹³;
W² is SO₂, CO, COO, C(S)NR¹², or CONR¹²; and
R¹³ is C₁₋₆ alkyl, C₁₋₆ haloalkyl, aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from:
C₁₋₆ alkyl, C₂₋₆ alkenyl, C₂₋₆ alkynyl, C₁₋₆ halo alkyl, aryl, cycloalkyl, heteroaryl, heterocycloalkyl, arylalkyl, cycloalkylalkyl, heteroarylalkyl, heterocycloalkylalkyl, halo, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{c"}, S(O)₂R^{b"}, S(O)₂NR^{c"}R^{d"}, -(C₁₋₆alkyl)-CN, -(C₁₋₆alkyl)-NO₂, - (C₁₋₆ alkyl)-OR^{a} -(C₁₋₆alkyl)-SR^{a"}, -(C₁₋₆alkyl)-C(O)R^{b"},
   -(C₁₋₆alkyl)-C(O)NR^{c"}R^{d"}, -(C₁₋₆ alkyl)-C(O)OR^{a} -(C₁₋₆ alkyl)-OC(O)R^{b"},
   -(C₁₋₆ alkyl)-OC(O)NR^{c"}R^{d"}, -(C₁₋₆ alkyl)-NR^{c"}R^{d"}, -(C₁₋₆ alkyl)-NR^{c"}C(O)R^{d"},
   -(C₁₋₆alkyl)-NR^{c"}C(O)OR^{a"}, -(Ci.6 alkyl)-S(O)R^{b"}, -(C₁₋₆ alkyl)-S(O)NR^{c"}R^{d"},
   -(C₁₋₆ alkyl)-S(O)₂R^{b"}, and -(C₁₋₆ alkyl)-S(O)₂NR^{c"}R^{d"}.

In some embodiments, the compounds of formula V have the structure of Formula Va: 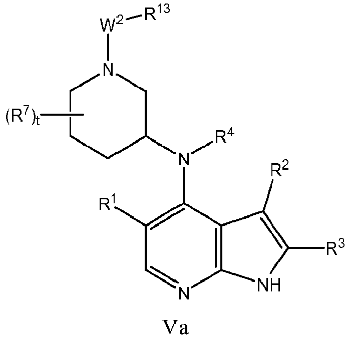 wherein t is 0, 1 or 2.

In some embodiments, the compounds of the invention have Formula Va, wherein:
W² is SO₂, CO, COO, C(S)NR¹², or CONR¹²;
R¹³ is C₁₋₆ alkyl, C₁₋₆ haloalkyl, aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from:
C₁₋₆ alkyl, C₂₋₆ alkenyl, C₂₋₆ alkynyl, C₁₋₆ haloalkyl, aryl, cycloalkyl, heteroaryl, heterocycloalkyl, arylalkyl, cycloalkylalkyl, heteroarylalkyl, heterocycloalkylalkyl, halo, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{d"}, NR^{c"}C(O)OR^{a"}, S(O)R^{b"}, S(O)NR^{c"}R^{c"}, S(O)₂R^{b"}, S(O)₂NR^{c"}R^{d"}, -(C₁₋₆ alkyl)-CN, -(C₁₋₆ alkyl)-NO₂, - (C₁₋₆ alkyl)-OR^{a} -(C₁₋₆ alkyl)-SR^{a"}, -(C₁₋₆ alkyl)-C(O)R^{b"}, -(C₁₋₆alkyl)-C(O)NR^{c"}R^{d"}, -(C₁₋₆ alkyl)-C(O)OR^{a"}, -(C₁₋₆ alkyl)-OC(O)R^{b"}, -(C₁₋₆ alkyl)-OC(O)NR^{c"}R^{d"}, -(C₁₋₆ alkyl)-NR^{c"}R^{d"}, -(C₁₋₆ alkyl)-NR^{c}'C()R^{d"}, -(C₁₋₆ alkyl)-NR^{c"}C(O)OR^{a"}, -(C₁₋₆ alkyl)-S(O)R^{b"}, -(C₁₋₆ alkyl)-S(O)NR^{c"}R^{d"}, -(C₁₋₆ alkyl)-S(O)₂R^{b"}, and -(C₁₋₆ alkyl)-S(O)₂NR^{c"}R^{d"}; and
t is 0, 1 or 2.

In some embodiments, t is 0.

In some embodiments, t is 1.

In some embodiments, t is 2.

In some embodiments, the compounds have the structure of Formula Vb: 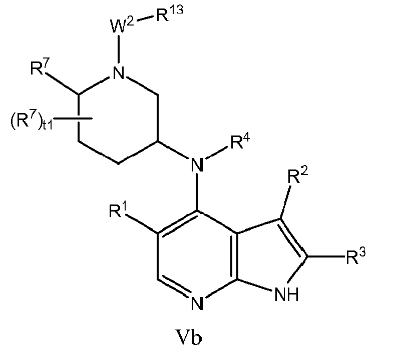 wherein t1 is 0 or 1.

In some further embodiments, the compounds have Formula Vb wherein:
R¹³ is C₁₋₆ alkyl, C₁₋₆ haloalkyl, aryl, cycloalkyl, heteroaryl, heterocycloalkyl, arylalkyl, cycloalkylalkyl, heteroarylalkyl or heterocycloalkylalkyl, each optionally substituted by 1, 2, 3, 4 or 5 substituents independently selected from C₁₋₆ alkyl, C₁₋₆ haloalkyl, aryl, heteroaryl, halo, CN, OR^{3a}, SR^{a"}, C(O)R^{b"}, C(O)OR^{a"}, NR^{c"}C(O)R^{d"}, S(O)₂R^{b"}, and -(C₁₋₆ alkyl)-CN;
W² is SO₂, CO, COO, C(S)NH, CONH or -CON(C₁₋₄ alkyl)-; and
R⁴ is C₁₋₄ alkyl, C₂₋₄ alkenyl, C₂₋₄ alkynyl, S(O)₂R⁹, SOR⁹, cycloalkyl, or heterocycloalkyl, wherein the C₁₋₄ alkyl, C₂₋₄ alkenyl, C₂₋₄ alkynyl, cycloalkyl, heterocycloalkyl are each optionally substituted with 1, 2 or 3 substituents selected from halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR^{a}, SR^{a}, C(O)R^{b}, C(O)NR^{c}R^{d}, C(O)OR³, OC(O)R^{b}, OC(O)NR^{c}R^{d}, NR^{c}R^{d}, NR^{c}C(O)R^{d}, NR^{c}C(O)OR^{a}, S(O)R^{b}, S(O)NR^{c}R^{d}, S(O)₂R^{b}, and S(O)₂NR^{c}R^{d}.

In some embodiments, t1 is 0.

In some embodiments, t1 is 1.

In some embodiments, the therapeutic agent is selected from:
3-{3-[methyl(1H-pyrrolo[2,3-b]pyridin-4-yl)amino]piperidin-1-yl}-3-oxopropanenitrile;
3-{(3R,4R)-4-methyl-3-[methyl(1H-pyrrolo[2,3-b]pyridin-4-yl)amino]piperidin-1-yl}-3-oxopropanenitrile;
N-methyl-N-[(3R,4R)-4-methyl-1-(phenylsulfonyl)piperidin-3-yl]-N-(1H-pyrrolo[2,3-b]pyridin-4-yl)-amine;
N-[(3R,4R)-1-(methoxyacetyl)-4-methylpiperidin-3-yl]-N-methyl-N-(1H-pyrrolo[2,3-b]pyridin-4-yl)-amine;
(3R,4R)-4-methyl-3-[methyl(1H-pyrrolo[2,3-b]pyridin-4-yl)amino]-N-phenylpiperidine-1-carboxamide;
(3R,4R)-N-benzyl-4-methyl-3-[methyl(1H-pyrrolo[2,3-b]pyridin-4-yl)amino]piperidine-1-carboxamide;
(3R,4R)-N-ethyl-4-methyl-3-[methyl(1H-pyrrolo[2,3-b]pyridin-4-yl)amino]piperidine-1-carboxamide;
(3R,4R)-N-isopropyl-4-methyl-3-[methyl(1H-pyrrolo[2,3-b]pyridin-4-yl)amino]piperidine-1-carboxamide;
N-[(3R,4R)-1-isobutyryl-4-methylpiperidin-3-yl]-N-methyl-N-(1H-pyrrolo[2,3-b]pyridin-4-yl)-amine;
N-methyl-N-[(3R,4R)-4-methyl-1-(morpholin-4-ylcarbonyl)piperidin-3-yl]-N-(1H-pyrrolo[2,3-b]pyridin-4-yl)-amine;
N-[(3R,4R)-1-acetyl-4-methylpiperidin-3-yl]-N-methyl-N-(1H-pyrrolo[2,3-b]pyridin-4-yl)-amine;
N-methyl-N-[(3R,4R)-4-methyl-1-(3-methylbutanoyl)piperidin-3-yl]-N-(1H-pyrrolo[2,3-b]pyridin-4-yl)-amine;
N-[(3R,4R)-1-benzoyl-4-methylpiperidin-3-yl]-N-methyl-N-(1H-pyrrolo[2,3-b]pyridin-4-yl)-amine;
(3R,4R)-N,N,4-trimethyl-3-[methyl(1H-pyrrolo [2,3-b]pyridin-4-yl)amino]piperidine-1-carboxamide;
4-({(3R,4R)-4-methyl-3-[methyl(1H-pyrrolo[2,3-b]pyridin-4-yl)amino]piperidin-1-yl}carbonyl)benzonitrile;
N-[(3R,4R)-1-(cyclopropylcarbonyl)-4-methylpiperidin-3-yl]-N-methyl-N-(1H-pyrrolo[2,3-b]pyridin-4-yl)-amine;
N-[(3R,4R)-1-isonicotinoyl-4-methylpiperidin-3-yl]-N-methyl-N-(1H-pyrrolo[2,3-b]pyridin-4-yl)-amine;
N- {(3R,4R)-1-[(1-acetylpiperidin-4-yl)carbonyl]-4-methylpiperidin-3-yl}-N-methyl-N-(1H-pyrrolo[2,3-b]pyridin-4-yl)-amine;
Phenyl (3R,4R)-4-methyl-3-[methyl(1H-pyrrolo[2,3-b]pyridin-4-yl)amino]piperidine-1-carboxylate;
Methyl (3R,4R)-4-methyl-3-[methyl(1H-pyrrolo[2,3-b]pyridin-4-yl)amino]piperidine-1-carboxylate;
N-methyl-N-[(3R,4R)-4-methyl-1-(trifluoroacetyl)piperidin-3-yl]-N-(1H-pyrrolo[2,3-b]pyridin-4-yl)-amine;
N-[(3R,4R)-1-(2-furoyl)-4-methylpiperidin-3-yl]-N-methyl-N-(1H-pyrrolo[2,3-b]pyridin-4-yl)-amine;
(3R,4R)-N-(4-cyanophenyl)-4-methyl-3-[methyl(1H-pyrrolo[2,3-b]pyridin-4-yl)amino]piperidine-1-carboxamide;
(3R,4R)-N-(3-cyanophenyl)-4-methyl-3-[methyl(1H-pyrrolo[2,3-b]pyridin-4-yl)amino]piperidine-1-carboxamide;
(3R,4R)-4-methyl-3-[methyl(1H-pyrrolo[2,3-b]pyridin-4-yl)amino]-N-(2-phenylethyl)piperidine-1-carboxamide;
(3R,4R)-N-(2-furylmethyl)-4-methyl-3-[methyl(1H-pyrrolo[2,3-b]pyridin-4-yl)amino]piperidine-1-carboxamide;
N-methyl-N-[(3R,4R)-4-methyl-1-(propylsulfonyl)piperidin-3-yl]-N-(1H-pyrrolo[2,3-b]pyridin-4-yl)-amine;
N-[(3R,4R)-1-(isopropylsulfonyl)-4-methylpiperidin-3-yl]-N-methyl-N-(1H-pyrrolo[2,3-b]pyridin-4-yl)-amine;
4-({(3R,4R)-4-methyl-3-[methyl(1H-pyrrolo[2,3-b]pyridin-4-yl)amino]piperidin-1-yl}sulfonyl)benzonitrile;
2-({(3R,4R)-4-methyl-3-[methyl(1H-pyrrolo[2,3-b]pyridin-4-yl)amino]piperidin-1-yl}sulfonyl)benzonitrile;
N-methyl-N-[(3R,4R)-4-methyl-1-(methylsulfonyl)piperidin-3-yl]-N-(1H-pyrrolo[2,3-b]pyridin-4-yl)-amine;
N-methyl-N-{(3R,4R)-4-methyl-1-[(trifluoromethyl)sulfonyl]piperidin-3-yl}-N-(1H-pyrrolo[2,3-b]pyridin-4-yl)-amine;
N-methyl-N-[(3R,4R)-4-methyl-1-(pyridin-3-ylsulfonyl)piperidin-3-yl]-N-(1H-pyrrolo[2,3-b]pyridin-4-yl)-amine;
2-fluoro-5-({(3R,4R)-4-methyl-3-[methyl(1H-pyrrolo[2,3-b]pyridin-4-yl)amino]piperidin-1-yl}sulfonyl)benzonitrile;
N-methyl-N-[(3R,4R)-4-methyl-1-(3-pyridin-3-ylpropanoyl)piperidin-3-yl]-N-(1H-pyrrolo[2,3-b]pyridin-4-yl)-amine;
N-methyl-N-[(3R,4R)-4-methyl-1-(3,3,3-trifluoropropanoyl)piperidin-3-yl]-N-(1H-pyrrolo[2,3-b]pyridin-4-yl)-amine;
N-methyl-N-[(3R,4R)-4-methyl-1-(tetrahydrofuran-2-ylcarbonyl)piperidin-3-yl]-N-(1H-pyrrolo[2,3-b]pyridin-4-yl)-amine;
(2R)-1-{(3R,4R)-4-methyl-3-[methyl(1H-pyrrolo[2,3-b]pyridin-4-yl)amino]piperidin-1-yl}-1-oxopropan-2-ol;
(2S)-1-{(3R,4R)-4-methyl-3-[methyl(1H-pyrrolo[2,3-b]pyridin-4-yl)amino]piperidin-1-yl}-1-oxopropan-2-ol;
N-methyl-N-[(3R,4R)-4-methyl-1-(3-phenylpropanoyl)piperidin-3-yl]-N-(1H-pyrrolo[2,3-b]pyridin-4-yl)-amine;
(3R,4R)-N-(4-cyanophenyl)-4-methyl-3-[methyl(1H-pyrrolo[2,3-b]pyridin-4-yl)amino]piperidine-1-carbothioamide;
N-methyl-N-{(3R,4R)-4-methyl-1-[(5-methylisoxazol-4-yl)sulfonyl]piperidin-3-yl}-N-(1H-pyrrolo[2,3-b]pyridin-4-yl)-amine;
N-methyl-N-{(3R,4R)-4-methyl-1-[(1-methyl-1H-imidazol-4-yl)sulfonyl]piperidin-3-yl}--N-(1H-pyrrolo[2,3-b]pyridin-4-yl)-amine;
N-{(3R,4R)-1-[(3,5-dimethylisoxazol-4-yl)carbonyl]-4-methylpiperidin-3-yl}-N-methyl-N-(1H-pyrrolo[2,3-b]pyridin-4-yl)-amine;
(3R,4R)-4-methyl-3-[methyl(1H-pyrrolo [2,3-b]pyridin-4-yl)amino]-N-2-thienylpiperidine-1-carboxamide;
N-[(3R,4R)-1-(isoxazol-5-ylcarbonyl)-4-methylpiperidin-3-yl]-N-methyl-N-(1H-pyrrolo[2,3-b]pyridin-4-yl)-amine;
N-{(3R,4R)-1-[(1,2-dimethyl-1H-imidazol-4-yl)sulfonyl]-4-methylpiperidin-3-yl}-N-methyl-N-(1H-pyrrolo[2,3-b]pyridin-4-yl)-amine;
N-[4-methyl-5-({(3R,4R)-4-methyl-3-[methyl(1H-pyrrolo[2,3-b]pyridin-4-yl)amino]piperidin-1-yl}sulfonyl)-1,3-thiazol-2-yl]acetamide;
N-{(3R,4R)-1-[(2,4-dimethyl-1,3-thiazol-5-yl)sulfonyl]-4-methylpiperidin-3-yl}-N-methyl-N-(1H-pyrrolo[2,3-b]pyridin-4-yl)-amine;
N-methyl-N-{(3R,4R)-4-methyl-1-[(1,3,5-trimethyl-1H-pyrazol-4-yl)sulfonyl]piperidin-3-yl}-N-(1H-pyrrolo[2,3-b]pyridin-4-yl)-amine;
N-{(3R,4R)-1-[(3,5-dimethylisoxazol-4-yl)sulfonyl]-4-methylpiperidin-3-yl}-N-methyl-N-(1H-pyrrolo[2,3-b]pyridin-4-yl)-amine;
N-methyl-N-{(3R,4R)-4-methyl-1-[(pyridin-4-ylmethyl)sulfonyl]piperidin-3-yl}-N-(1H-pyrrolo[2,3-b]pyridin-4-yl)-amine;
N-methyl-N-{(3R,4R)-4-methyl-1-[(pyridin-3-ylmethyl)sulfonyl]piperidin-3-yl}-N-(1H-pyrrolo[2,3-b]pyridin-4-yl)-amine;
N-methyl-N-{(3R,4R)-4-methyl-1-[(pyridin-2-ylmethyl)sulfonyl]piperidin-3-yl}-N-(1H-pyrrolo[2,3-b]pyridin-4-yl)-amine;
4-({(3R,4R)-4-methyl-3-[methyl(1H-pyrrolo[2,3-b]pyridin-4-yl)amino]piperidin-1-yl}sulfonyl)benzonitrile;
(3R,4R)-N-(4-cyanophenyl)-N,4-dimethyl-3-[methyl(1H-pyrrolo[2,3-b]pyridin-4-yl)amino]piperidine-1-carboxamide;
(3R,4R)-N-(4-cyanophenyl)-N-ethyl-4-methyl-3-[methyl(1H-pyrrolo[2,3-b]pyridin-4-yl)amino]piperidine-1-carboxamide;
(3R,4R)-4-methyl-3-[methyl(1H-pyrrolo[2,3-b]pyridin-4-yl)amino]-N-1,3-thiazol-2-ylpiperidine-1-carboxamide;
(3R,4R)-4-methyl-N-(3-methylisoxazol-5-yl)-3-[methyl(1H-pyrrolo[2,3-b]pyridin-4-yl)amino]piperidine-1-carboxamide;
3-chloro-4-({(3R,4R)-4-methyl-3-[methyl(1H-pyrrolo[2,3-b]pyridin-4-yl)amino]piperidin-1-yl}sulfonyl)benzonitrile;
(3R,4R)-4-methyl-N-(5-methylisoxazol-3-yl)-3-[methyl(1H-pyrrolo[2,3-b]pyridin-4-yl)amino]piperidine-1-carboxamide;
(3R,4R)-N-isoxazol-3-yl-4-methyl-3-[methyl(1H-pyrrolo[2,3-b]pyridin-4-yl)amino]piperidine-1-carboxamide;
N-methyl-N-{(3R,4R)-4-methyl-1-[(5-pyridin-3-yl-2-thienyl)sulfonyl]piperidin-3-yl}-N-(1H-pyrrolo[2,3-b]pyridin-4-yl)-amine;
(3R,4R)-N-(3-cyano-2-thienyl)-4-methyl-3-[methyl(1H-pyrrolo[2,3-b]pyridin-4-yl)amino]piperidine-1-carboxamide;
(3R,4R)-N-1,3-benzothiazol-2-yl-4-methyl-3-[methyl(1H-pyrrolo[2,3-b]pyridin-4-yl)amino]piperidine-1-carboxamide;
N-[(3R,4R)-1-(2,3-dihydro-1H-indol-1-ylcarbonyl)-4-methylpiperidin-3-yl]-N-methyl-N-(1H-pyrrolo[2,3-b]pyridin-4-yl)-amine;
N-methyl-N- {(3R,4R)-4-methyl-1-[(methylthio)acetyl]piperidin-3-yl}-N-(1H-pyrrolo[2,3-b]pyridin-4-yl)-amine;
(3R,4R)-N-(4,5-dihydro-1,3-thiazol-2-yl)-4-methyl-3-[methyl(1H-pyrrolo[2,3-b]pyridin-4-yl)amino]piperidine-1-carboxamide;
(3R,4R)-4-methyl-3-[methyl(1H-pyrrolo[2,3-b]pyridin-4-yl)amino]-N-(1,3-thiazol-2-ylmethyl)piperidine-1-carboxamide;
1-({(3R,4R)-4-methyl-3-[methyl(1H-pyrrolo[2,3-b]pyridin-4-yl)amino]piperidin-1-yl}carbonyl)piperidine-4-carbonitrile;
1-({(3R,4R)-4-methyl-3-[methyl(1H-pyrrolo[2,3-b]pyridin-4-yl)amino]piperidin-1-yl}carbonyl)piperidine-3-carbonitrile;
1-({(3R,4R)-4-methyl-3-[methyl(1H-pyrrolo[2,3-b]pyridin-4-yl)amino]piperidin-1-yl}carbonyl)pyrrolidine-3-carbonitrile;
(3R,4R)-4-methyl-3-[methyl(1H-pyrrolo[2,3-b]pyridin-4-yl)amino]-N-(3-thienylmethyl)piperidine-1-carboxamide;
(3R,4R)-N-(2-benzothien-1-ylmethyl)-4-methyl-3-[methyl(1H-pyrrolo[2,3-b]pyridin-4-yl)amino]piperidine-1-carboxamide;
(3R,4R)-N-(1,3-benzothiazol-2-ylmethyl)-4-methyl-3-[methyl(1H-pyrrolo[2,3-b]pyridin-4-yl)amino]piperidine-1-carboxamide;
N-[(3R,4R)-1-(3-furylacetyl)-4-methylpiperidin-3-yl]-N-methyl-N-(1H-pyrrolo[2,3-b]pyridin-4-yl)-amine;
3-(2-{(3R,4R)-4-methyl-3-[methyl(1H-pyrrolo[2,3-b]pyridin-4-yl)amino]piperidin-1-yl}-2-oxoethyl)-1,3-thiazolidine-2,4-dione;
3-(2-{(3R,4R)-4-methyl-3-[methyl(1H-pyrrolo[2,3-b]pyridin-4-yl)amino]piperidin-1-yl}-2-oxoethyl)-1,3-benzothiazol-2(3H)-one;
(3R,4R)-N-[5-(cyanomethyl)-4,5-dihydro,-1,3-thiazol-2-yl]-3-[methyl-(1H-pyrrolo[2,3-b]pyridin-4-yl)amino]-4-methyl-piperidine-1-carboxamide;
(3S)-1-({(3R,4R)-4-methyl-3-[methyl(1H-pyrrolo[2,3-b]pyridin-4-yl)amino]piperidin-1-yl}carbonyl)pyrrolidine-3-carbonitrile;
(3R)-1-({(3R,4R)-4-methyl-3-[methyl(1H-pyrrolo[2,3-b]pyridin-4-yl)amino]piperidin-1-yl}carbonyl)pyrrolidine-3-carbonitrile;
1-({(3R,4R)-4-methyl-3-[methyl(1H-pyrrolo[2,3-b]pyridin-4-yl)amino]piperidin-1-yl}carbonyl)-4-phenylpiperidine-4-carbonitrile;
N-methyl-N-((3R,4R)-4-methyl-1-{[3-(trifluoromethyl)pyrrolidin-1-yl]carbonyl}piperidin-3-yl)-N-(1H-pyrrolo[2,3-b]pyridin-4-yl)-amine;
1-({(3R,4R)-4-methyl-3-[methyl(1H-pyrrolo[2,3-b]pyridin-4-yl)amino]piperidin-1-yl}carbonyl)azetidine-3-carbonitrile;
4-methyl-1-({(3R,4R)-4-methyl-3-[methyl(1H-pyrrolo[2,3-b]pyridin-4-yl)amino]piperidin-1-yl}carbonyl)pyrrolidine-3-carbonitrile;
1-({(3R,4R)-4-methyl-3-[methyl(1H-pyrrolo[2,3-b]pyridin-4-yl)amino]piperidin-1-yl}carbonyl)pyrrolidine-3,4-dicarbonitrile;
3-methyl-1-({(3R,4R)-4-methyl-3-[methyl(1H-pyrrolo[2,3-b]pyridin-4-yl)amino]piperidin-1-yl}carbonyl)pyrrolidine-3-carbonitrile;
(3R,4R)-N-(2-cyanoethyl)-4-methyl-3-[methyl(1H-pyrrolo[2,3-b]pyridin-4-yl)amino]piperidine-1-carboxamide;
4-methoxy-1-({(3R,4R)-4-methyl-3-[methyl(1H-pyrrolo[2,3-b]pyridin-4-yl)amino]piperidin-1-yl}carbonyl)pyrrolidine-3-carbonitrile;
N-{(3R,4R)-1-[(2R)-2-aminopropanoyl]-4-methylpiperidin-3-yl}-N-methyl-N-(1H-pyrrolo[2,3-b]pyridin-4-yl)-amine;
N-[(3R,4R)-1-(aminoacetyl)-4-methylpiperidin-3-yl]-N-methyl-N-(1H-pyrrolo[2,3-b]pyridin-4-yl)-amine;
1-(2-{(3R,4R)-4-methyl-3-[methyl(1H-pyrrolo[2,3-b]pyridin-4-yl)amino]piperidin-1-yl}-2-oxoethyl)piperidine-4-carbonitrile;
N-methyl-N-[(3R,4R)-4-methyl-1-(1,3-thiazol-4-ylcarbonyl)piperidin-3-yl]-N-(1H-pyrrolo[2,3-b]pyridin-4-yl)-amine bis (trifluoroacetate);
4-(2-2S-{[methyl-(1H-pyrrolo[2,3-b]pyridin-4-yl)-amino]-methyl}-pyrrolidin-1-yl-sulfonyl)-benzonitrile trifluoroacetate;
N-[(1-methanesulfonyl-2S-pyrrolidin-2-yl)-methyl]-N-methyl-N-(1H-pyrrolo[2,3-b]pyridin-4-yl)-amine trifluoroacetate;
3-((2S)-2-{[methyl(1H-pyrrolo[2,3-b]pyridin-4-yl)amino]methyl}pyrrolidin-1-yl)-3-oxopropanenitrile trifluoroacetate;
Methyl 3-[({(3R,4R)-4-methyl-3-[methyl(1H-pyrrolo[2,3-b]pyridin-4-yl)amino]piperidine-1-yl}carbonyl)-amino]benzoate;
(3R,4R)-N-(4-trifluoromethoxyphenyl)-4-methyl-3-[methyl-(1H-pyrrolo[2,3-b]pyridin-4-yl)amino]piperidine-1-carboxamide;
(3R,4R)-N-(4-fluorophenyl)-4-methyl-3-[methyl-(1H-pyrrolo[2,3-b]pyridin-4-yl)amino]piperidine-1-carboxamide;
(3R,4R)-N-(3-fluorophenyl)-4-methyl-3-[methyl-(1H-pyrrolo[2,3-b]pyridin-4-yl)amino]piperidine-1-carboxamide;
(3R,4R)-N-(2-fluorophenyl)-4-methyl-3-[methyl-(1H-pyrrolo[2,3-b]pyridin-4-yl)amino]piperidine-1-carboxamide;
(3R,4R)-N-(4-trifluoromethylphenyl)-4-methyl-3-[methyl-(1H-pyrrolo[2,3-b]pyridin-4-yl)amino]piperidine-1-carboxamide;
(3R,4R)-N-(2-methoxyphenyl)-4-methyl-3-[methyl(1H-pyrrolo[2,3-b]pyridin-4-yl)amino]piperidine-1-carboxamide;
(3R,4R)-4-methyl-N-(4-methylphenyl)-3-[methyl(1H-pyrrolo[2,3-b]pyridin-4-yl)-amino]piperidine-1-carboxamide;
N-methyl-N-{(3R,4R)-4-methyl-1-[4-(pyridin-2-yloxy)phenyl]sulfonyl-piperidin-3-yl}-N-(1H-pyrrolo[2,3-b]pyridin-4-yl)-amine;
N-methyl-N-{(3R,4R)-4-methyl-1-[4-(1,3-oxazol-5-yl)phenyl]sulfonyl-piperidin-3-yl}-N-(1H-pyrrolo[2,3-b]pyridin-4-yl)-amine;
N-methyl-N-{(3R,4R)-4-methyl-1-[5-(1,3-oxazol-5-yl)thienyl]sulfonyl-piperidin-3-yl}-N-(1H-pyrrolo[2,3-b]pyridin-4-yl)-amine;
N-methyl-N-{(3R,4R)-4-methyl-1-[(6-phenoxy-pyridin-3-yl)sulfonyl]piperidin-3-yl}-(1H-pyrrolo[2,3-b]pyridin-4-yl)amine;
N-{(3R,4R)-1-[(2,6-dichlorophenyl)sulfonyl]-4-methylpiperidin-3-yl}-N-methyl-(1H-pyrrolo[2,3-b]pyridin-4-yl)-amine;
N-{(3R,4R)-1-[(4-fluorophenyl)sulfonyl]-4-methylpiperidin-3-yl}-N-methyl-(1H-pyrrolo[2,3-b]pyridin-4-yl)-amine;
N-{(3R,4R)-1-[(3-fluorophenyl)sulfonyl]-4-methylpiperidin-3-yl}-N-methyl-(1H-pyrrolo[2,3-b]pyridin-4-yl)-amine;
N-{(3R,4R)-1-[(2-fluorophenyl)sulfonyl]-4-methylpiperidin-3-yl}-N-methyl-(1H-pyrrolo[2,3-b]pyridin-4-yl)-amine;
N-{(3R,4R)-4-methyl-1-[4-(trifluoromethyl)phenyl]sulfonyl-piperidin-3-yl}-N-(1H-pyrrolo[2,3-b]pyridin-4-yl)-amine;
N-{(3R,4R)-4-methyl-1-[3-(trifluoromethyl)phenyl]sulfonyl-piperidin-3-yl}-N-(1H-pyrrolo[2,3-b]pyridin-4-yl)-amine;
N-{(3R,4R)-4-methyl-1-[2-(trifluoromethyl)phenyl]sulfonyl-piperidin-3-yl}-N-(1H-pyrrolo[2,3-b]pyridin-4-yl)-amine;
N- {(3R,4R)-1-[(4-methoxyphenyl)sulfonyl]-4-methylpiperidin-3-yl}-N-methyl-N-(1H-pyrrolo[2,3-b]pyridin-4-yl)-amine;
N-{(3R,4R)-1-[(3-methoxyphenyl)sulfonyl]-4-methylpiperidin-3-yl}-N-methyl-N-(1H-pyrrolo[2,3-b]pyridin-4-yl)-amine;
N-methyl-N-{(3R,4R)-4-methyl-l-[(4-methylphenyl)sulfonyl]piperidin-3-yl}-N-(1H-pyrrolo[2,3-b]pyridin-4-yl)-amine;
N-methyl-N-{(3R,4R)-4-methyl-1-[(3-methylphenyl)sulfonyl]piperidin-3-yl}-N-(1H-pyrrolo[2,3-b]pyridin-4-yl)-amine;
N-methyl-N-{(3R,4R)-4-methyl-1-[(2-methylphenyl)sulfonyl]piperidin-3-yl}-N-(1H-pyrrolo[2,3-b]pyridin-4-yl)-amine;
N-{(3R,4R)-1-[(4-chlorophenyl)sulfonyl]-4-methylpiperidin-3-yl}-N-methyl-N-(1H-pyrrolo[2,3-b]pyridin-4-yl)-amine;
N-{(3R,4R)-1-[(3-chlorophenyl)sulfonyl]-4-methylpiperidin-3-yl}-N-methyl-N-(1H-pyrrolo[2,3-b]pyridin-4-yl)-amine; and
N-{(3R,4R)-1-[(2-chlorophenyl)sulfonyl]-4-methylpiperidin-3-yl}-N-methyl-N-(1H-pyrrolo[2,3-b]pyridin-4-yl)-amine,
and pharmaceutically acceptable salts thereof.

In a seventh aspect, the agent is selected from compounds of Formula VIIa, VIIb, and VIIc: 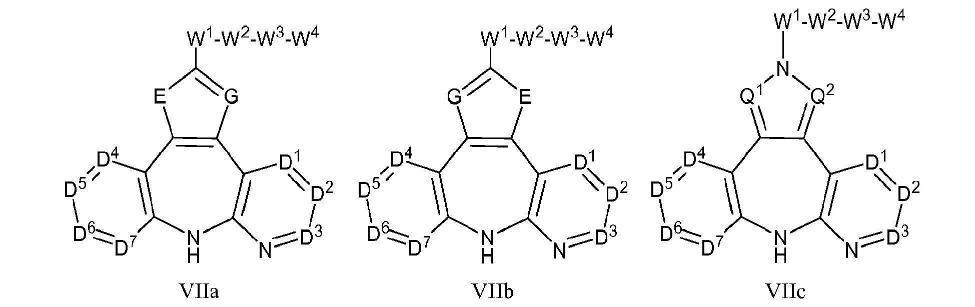 and pharmaceutically acceptable salts thereof, wherein:
D¹, D², D³, D⁴, D⁵, D⁶ and D⁷ are, independently, CR¹ or N;
E is O, S, SO, SO₂, or NR^{2a};
G is N or CR^{2b};
Q¹ and Q² are each, independently, N or CR^{2c};
W¹ is absent, C₁₋₆ alkyl, C₂₋₆ alkenyl, C₂₋₆ alkynyl, O, S, NR³, CO, COO, CONR³, SO, SO₂, SONR³, SO₂NR³, or NR³CONR⁴, wherein the C₁₋₆ alkyl, C₂₋₆ alkenyl or C₂₋₆ alkynyl is optionally substituted by 1, 2 or 3 substituents independently selected from halo, OH, C₁₋₄ alkoxy, C₁₋₄ haloalkoxy, amino, C₁₋₄ alkylamino and C₂₋₈ dialkylamino;
W² is absent, C₁₋₆ alkyl, C₂₋₆ alkenyl, C₂₋₆ alkynyl, aryl, cycloalkyl, heteroaryl or heterocycloalkyl, wherein the C₁₋₆ alkyl, C₂₋₆ alkenyl, C₂₋₆ alkynyl, aryl, cycloalkyl, heteroaryl or heterocycloalkyl is optionally substituted by 1, 2, or 3 substituents independently selected from halo, CN, NO₂, OH, =NH, =NOH, =NO-(C₁₋₄ alkyl), -NR³C(O)O-(C₁₋₄ alkyl), NR³C(O)-(C₁₋₄ alkyl), - C(O)O-(C₁₋₄ alkyl), C₁₋₄ haloalkyl, C₁₋₄ cyanoalkyl, pentahalosulfanyl, C₁₋₄ hydroxylalkyl, C₁₋₄ alkylthio, C₁₋₄ haloalkylthio, C₁₋₄ alkoxy, C₁₋₄ haloalkoxy, amino, C₁₋₄ alkylamino and C₂₋₈ dialkylamino;
W³ is absent, C₁₋₆ alkyl, C₂₋₆ alkenyl, C₂₋₆ alkynyl, O, S, NR³, =N-, =N-O-, =N-O-( C₁₋₄ alkyl)-, O-( C₁₋₄ alkyl), S-(C₁₋₄ alkyl), NR³-( C₁₋₄ alkyl), (C₁₋₄ alkyl)-O-( C₁₋₄ alkyl), (C₁₋₄ alkyl)-S-(C₁₋₄ alkyl), (C₁₋₄ alkyl)-NR³-( C₁₋₄ alkyl), CO, COO, C(O)-(C₁₋₄ alkyl), C(O)O-(C₁₋₄ alkyl), C(O)-(C₁₋₄ alkyl)-C(O), NR³C(O)-(C₁₋₄ alkyl), C(O)NR³-(C₁₋₄ alkyl), NR³C(O)O-(C₁₋₄ alkyl), NR³C(O)O, CONR³, SO, SO₂, SONR³, SO₂NR³, or NR³CONR⁴, wherein the C₁₋₆ alkyl, C₂₋₆ alkenyl or C₂₋₆ alkynyl is optionally substituted by 1, 2 or 3 substituents independently selected from halo, OH, CN, C₁₋₄ alkoxy, C₁₋₄ haloalkoxy, amino, C₁₋₄ alkylamino and C₂₋₈ dialkylamino;
W⁴ is H, NR³R⁴, CN, C₁₋₆ alkyl, C₂₋₆ alkenyl, C₂₋₆ alkynyl, aryl, cycloalkyl, heteroaryl or heterocycloalkyl, wherein each of the C₁₋₆ alkyl, C₂₋₆ alkenyl, C₂₋₆ alkynyl, aryl, cycloalkyl, heteroaryl or heterocycloalkyl is optionally substituted by 1, 2, 3, 4 or 5 substituents independently selected from halo, OH, CN, C₁₋₄ alkoxy, =NH, =NOH, =NO-(C₁₋₄ alkyl), C₁₋₄ haloalkyl, C₁₋₄ haloalkoxy, pentahalosulfanyl, COOH, CONH₂, COO-(C₁₋₄ alkyl), amino, C₁₋₄ alkylamino and C₂₋₈ dialkylamino;
R¹ is, independently, H, halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR^{a}, SR^{a}, C(O)R^{b}, C(O)NR^{c}R^{d}, NR^{c}C(O)NR^{c}R^{d}, C(O)OR³, OC(O)R^{b}, OC(O)NR^{c}R^{d}, NR^{c}R^{d}, NR^{c}C(O)R^{d}, NR^{c}C(O)OR^{a}, S(O)R^{b}, S(O)NR^{c}R^{d}, NR^{c}S(O)NR^{c}R^{d}, pentahalosulfanyl, S(O)₂R^{b}, or S(O)₂NR^{c}R^{d};
R^{2a} is H, C₁₋₄ alkyl, C₂₋₄ alkenyl, C₂-₄ alkynyl, OH, C₁₋₄ alkoxy, C(O)R^{b}, C(O)NR^{c}R^{d}, S(O)R^{b}, S(O)NR^{c}R^{d}, S(O)₂R^{b}, or S(O)₂NR^{c}R^{d};
R^{2b} and R^{2c} are each, independently, H, halo, C₁₋₄ alkyl, C₂₋₄ alkenyl, C₂₋₄ alkynyl, C₁₋₄ haloalkyl, aryl, cycloalkyl, heteroaryl, heterocycloalkyl, CN, NO₂, OR^{a}, SR^{a}, C(O)R^{b}, C(O)NR^{c}R^{d}, C(O)OR³, OC(O)R^{b}, OC(O)NR^{c}R^{d}, NR^{c}C(O)NR^{c}R^{d}, NR^{c}R^{d}, NR^{c}C(O)R^{d}, NR^{c}C(O)OR^{a}, S(O)R^{b}, S(O)NR^{c}R^{d}, NR^{c}S(O)NR^{c}R^{d}, S(O)₂R^{b}, or S(O)₂NR^{c}R^{d};
R³ and R⁴ are each, independently, H, C₁₋₆ alkyl, C₂₋₆ alkenyl, C₂₋₆ alkynyl, aryl, cycloalkyl, arylalkyl, cycloalkylalkyl, COOR^{a} COR^{b'}, SOR^{b'}, or SO₂R^{b}' wherein each of the C₁₋₆ alkyl, C₂₋₆ alkenyl, C₂₋₆ alkynyl, aryl, cycloalkyl, arylalkyl, or cycloalkylalkyl is optionally substituted by 1, 2 or 3 substituents selected from halo, C₁₋₄ alkyl, C₁₋₄ haloalkyl, OH, C₁₋₄ alkoxy, C₁₋₄ haloalkoxy, amino, C₁₋₄ alkylamino, C₂₋₈ dialkylamino, aminocarbonyl, C₁₋₄ alkylaminocarbonyl, C₂₋₈ dialkylaminocarbonyl, CN and NO₂;
or R³ and R⁴ together with the N atom to which they are attached form a heterocycloalkyl group optionally substituted by 1, 2 or 3 substituents selected from halo, C₁₋₄ alkyl, C₁₋₄ haloalkyl, OH, C₁₋₄ alkoxy, C₁₋₄ haloalkoxy, amino, C₁₋₄ alkylamino, C₂ dialkylamino, aminocarbonyl, C₁₋₄ alkylaminocarbonyl, and C₂₋₈ dialkylaminocarbonyl;
R^{a} and R^{a'} are each, independently, H, C₁₋₆ alkyl, C₁₋₆ haloalkyl, C₂₋₆ alkenyl, C₂₋₆ alkynyl, aryl, cycloalkyl, heteroaryl or heterocycloalkyl;
R^{b} and R^{b'} are each, independently, H, C₁₋₆ alkyl, C₁₋₆ haloalkyl, C₂₋₆ alkenyl, C₂₋₆ alkynyl, aryl, cycloalkyl, heteroaryl or heterocycloalkyl; and
R^{c} and R^{d} are each, independently, H, C₁₋₆ alkyl, C₁₋₆ haloalkyl, C₂₋₆ alkenyl, C₂₋₆ alkynyl, aryl, cycloalkyl, arylalkyl, or cycloalkylalkyl;
or R^{c} and R^{d} together with the N atom to which they are attached form a 4-, 5-, 6- or 7-membered heterocycloalkyl group;

In some embodiments, when D⁷ is N, E is O or S, and G is N; then -W¹-W²-W³-W⁴ is other than H.

In some embodiments, no more than two of D¹, D², and D³ are N.

In some embodiments, no more than three of D⁴, D⁵, D⁶, and D⁷ are N.

In some embodiments, no more than two of D⁴, D⁵, D⁶, and D⁷ are N.

In some embodiments, the compound has Formula 1a.

In some embodiments, the compound has Formula Ib.

In some embodiments, the compound has Formula Ic.

In some embodiments, E is NR^{2a}.

In some embodiments, E is NH or NOH.

In some embodiments, G is N.

In some embodiments, E is NH or NOH and G is N.

In some embodiments, E is NH and G is N.

In some embodiments, the compound has Formula Ia, E is NH or NOH, and G is N.

In some embodiments, Q¹ is N and Q² is CR^{2c}.

In some embodiments, Q¹ is CR^{2c} and Q² is N.

In some embodiments, each D¹, D², and D³ is CR¹.

In some embodiments, each D¹, D², and D³ is CH.

In some embodiments, each D⁴, D⁵, D⁶, and D⁷ is CR¹.

In some embodiments, each D⁴, D⁵, and D⁷ is CR¹ and D⁶ is N.

In some embodiments, D⁴ and D⁷ are CH.

In some embodiments, D⁵ is CR¹ and R¹ is H or halo.

In some embodiments, D⁶ is CR¹ and R¹ is halo.

In some embodiments, D⁶ is CF.

In some embodiments, D⁶ is N.

In some embodiments, D⁵ is CH or CF.

In some embodiments, -W¹-W²-W³-W⁴ is C₁₋₆ alkyl, aryl, arylalkyl, cycloalkyl, cycloalkylalkyl, heteroaryl, heteroarylalkyl, heterocycloalkyl or heterocycloalkylalkyl, each optionally substituted by 1, 2, or 3 halo, C₁₋₄ alkyl, C₁₋₄ haloalkyl, C₁₋₄ haloalkylcarbonyl, aryl, cycloalkyl, heteroaryl, heterocycloalkyl, -COOH, -COO-(C₁₋₄alkyl), OH, C₁₋₄alkoxy, C₁₋₄haloalkoxy, hydroxyalkyl, CN, cyanoalkyl, alkylthio, arylthio, haloalkylthio, alkylsulfinyl, alkylsulfonyl, arylsulfinyl, arylsulfonyl, aryloxy, cycloalkyloxy, arylalkyloxy, aminocarbonyl, aminocarbonylalkyl, cyanoalkylcarbonyl, formyl, alkylcarbonyl, amino, alkylamino, alkylcarbonylamino, alkyloxycarbonylamino, or dialkylamino optionally substituted by CN.

In some embodiments, -W¹-W²-W³-W⁴ is C₁₋₆ alkyl, aryl, arylalkyl, cycloalkyl, heteroaryl, heterocycloalkyl, or heterocycloalkylalkyl, each optionally substituted by 1, 2, or 3 halo, C₁₋₄ alkyl, C₁₋₄ haloalkyl, C₁₋₄ haloalkylcarbonyl, aryl, -COO-(C₁₋₄ alkyl), OH, C₁₋₄ alkoxy, C₁₋₄ haloalkoxy, hydroxyalkyl, CN, cyanoalkyl, alkylthio, haloalkylthio, alkylsulfinyl, alkylsulfonyl, arylalkyloxy, aminocarbonyl, aminocarbonylalkyl, cyanoalkylcarbonyl, formyl, alkylcarbonyl, alkyloxycarbonylamino, alkylcarbonylamino, or dialkylamino optionally substituted by CN.

In some embodiments, -W¹-W²-W³-W⁴ is methyl, ethyl, propyl, butyl, pentyl, hexyl, phenyl, naphthyl, biphenyl, benzyl, phenylethyl, phenylpropyl, cyclopropyl, cyclohexyl, cyclohexenyl, bicyclo[2.2.1]hept-2-enyl, cyclopentyl, pyridyl, pyrryl, imidazolyl, isoxazolyl, thiazolyl, quinolinyl, piperidinyl, tetrahydrofuranyl, pyrrolidinyl, benzo[1,3]dioxolyl, (piperidin-4-yl)methyl, (piperidin-3-yl)methyl, (tetrahydropyran-4-yl)-methyl, (tetrahydrothiopyran-4-yl)-methyl, each optionally substituted by 1, 2, or 3 halo, C₁₋₄ alkyl, C₁₋₄ haloalkyl, C₁₋₄ haloalkylcarbonyl, aryl, -COO-(C₁₋₄ alkyl), OH, C₁₋₄ alkoxy, C₁₋₄ haloalkoxy, hydroxyalkyl, CN, cyanoalkyl, cyanoalkylcarbonyl, alkylthio, haloalkylthio, alkylsulfinyl, alkylsulfonyl, arylalkyloxy, aminocarbonyl, aminocarbonylalkyl, cyanoalkylcarbonyl, formyl, alkylcarbonyl, alkyloxycarbonylamino, alkylcarbonylamino, or dialkylamino optionally substituted by CN.

In some embodiments, -W¹-W²-W³-W⁴ is phenyl, pyrazyl, pyrindyl, pyrryl, indolyl, furyl, thienyl, or benzothienyl, each optionally substituted by 1, 2, 3, 4, or 5 halo, C₁₋₄ alkyl, C₁₋₄ haloalkyl, aryl, OH, C₁₋₄ alkoxy, hydroxyalkyl, CN, cyanoalkyl, alkylthio, haloalkylthio, alkylsulfinyl, alkylsulfonyl, cycloalkyl, heterocycloalkyl, cycloalkylakyl, heterocyclyoalkylalkyl, amino, dialkylamino, aminoalkyl, or aminosulfonyl.

In some embodiments, the agent is selected from compounds of Formula VIId or VIIe: 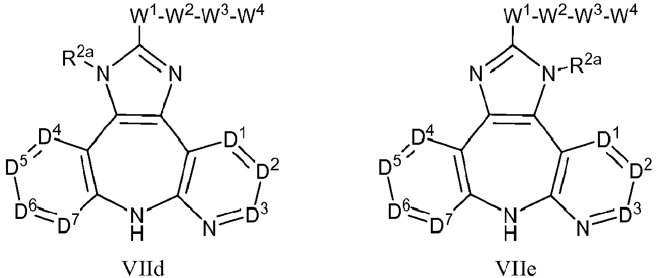 and pharmaceutically acceptable salts thereof.

In some embodiments, the agent is selected from compounds of Formula VIIf or VIIg:

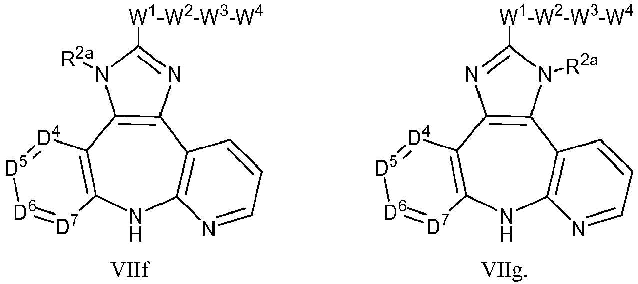

In some embodiments, the agent is selected from compounds of Formula VIIh or VIIi 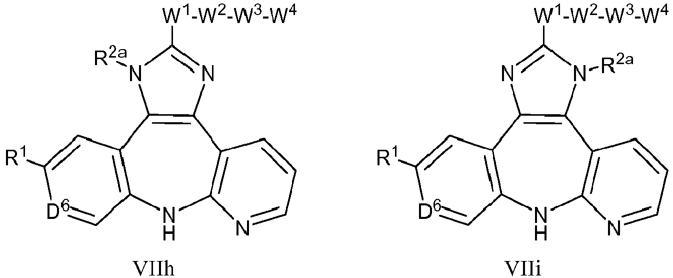 and pharmaceutically acceptable salts thereof.

In some embodiments, the agent is selected from compounds of Formula VIIj or VIIk: 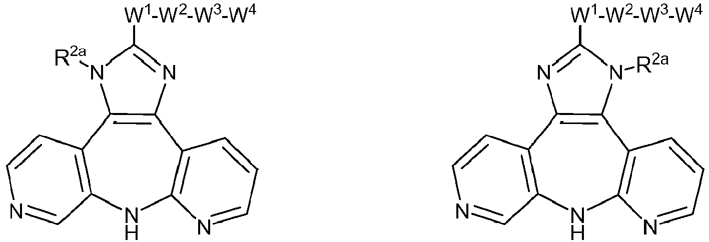 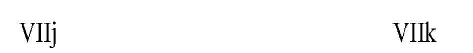 and pharmaceutically acceptable salts thereof.

In some embodiments, the agent is selected from compounds of Formula VIII: 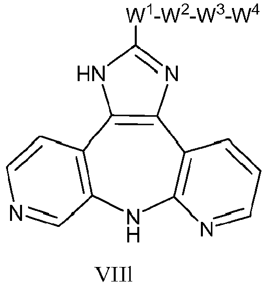 and pharmaceutically acceptable salts thereof.

In some embodiments, the agent is selected from compounds of Formula VIIm or VIIn: 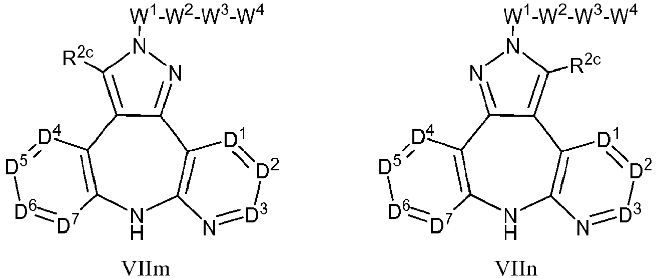 and pharmaceutically acceptable salts thereof.

In some embodiments, the agent is selected from compounds of Formula VIIo or VIIp: 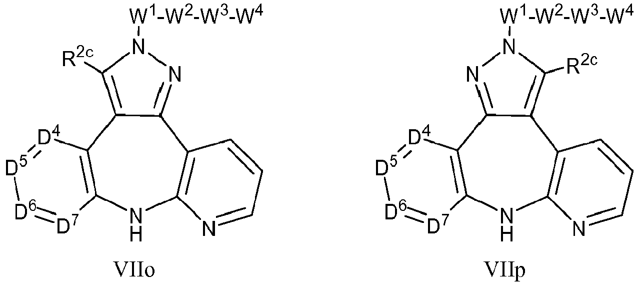 and pharmaceutically acceptable salts thereof.

In some embodiments, the agent is selected from compounds of Formula VIIq or VIIr: 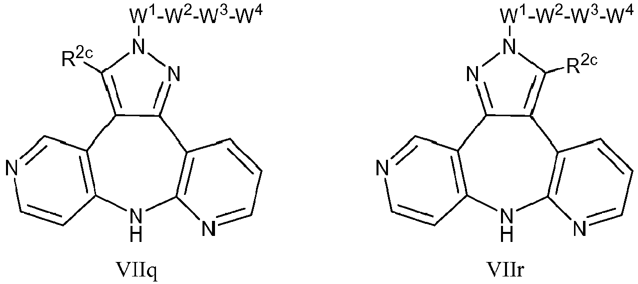 and pharmaceutically acceptable salts thereof.

In some embodiments, the agent is selected from:
2-tert-butyl-10-fluoro-3,8-dihydroimidazo[4,5-d]pyrido[2,3-b][1]benzazepine;
2-cyclopropyl-10-fluoro-3,8-dihydroimidazo[4,5-d]pyrido[2,3-b][1]benzazepine;
2-cyclohexyl-10-fluoro-3,8-dihydroimidazo[4,5-d]pyrido[2,3-b][1]benzazepine;
10-fluoro-2-isobutyl-3,8-dihydroimidazo[4,5-d]pyrido[2,3-b][1]benzazepine;
2-cyclopentyl-10-fluoro-3,8-dihydroimidazo[4,5-d]pyrido[2,3-b][1]benzazepine;
10-fluoro-2-(tetrahydrofuran-3-yl)-3,8-dihydroimidazo[4,5-d]pyrido[2,3-b][1]benzazepine;
2-cyclohex-3-en-1-yl-10-fluoro-3,8-dihydroimidazo[4,5-d]pyrido[2,3-b][1]benzazepine;
Trans ethyl (1R,S)-2-(10-fluoro-3,8-dihydroimidazo[4,5-d]pyrido[2,3-b][1]benzazepin-2-yl)cyclopropanecarboxylate;
2-bicyclo[2.2.1]hept-5-en-2-yl-10-fluoro-3,8-dihydroimidazo[4,5-d]pyrido[2,3-b][1]benzazepine;
[(1S)-2-(10-fluoro-3,8-dihydroimidazo[4,5-d]pyrido[2,3-b][1]benzazepin-2-yl)cyclopropyl]methanol trifluoroacetate;
2-(1-ethylpentyl)-10-fluoro-3,8-dihydroimidazo[4,5-d]pyrido[2,3-b][1]benzazepine;
4-ethyl-4-(10-fluoro-3,8-dihydroimidazo[4,5-d]pyrido[2,3-b][1]benzazepin-2-yl)hexanenitrile;
2-cyclopentyl-10,11-difluoro-3,8-dihydroimidazo[4,5-d]pyrido[2,3-b][1]benzazepine;
2-(1-ethylpentyl)-10,11-difluoro-3,8-dihydroimidazo[4,5-d]pyrido[2,3-b][1]benzazepine;
3-[1-(10-fluoro-3,8-dihydroimidazo[4,5-d]pyrido[2,3-b][1]benzazepin-2-yl)cyclohexyl]propanenitrile;
4-(10-fluoro-3,8-dihydroimidazo[4,5-d]pyrido[2,3-b][1]benzazepin-2-yl)-4-methylpentanenitrile;
2-(1-ethylpropyl)-10-fluoro-3,8-dihydroimidazo[4,5-d]pyrido[2,3-b][1]benzazepine;
10-fluoro-2-[2-(methylthio)ethyl]-3,8-dihydroimidazo[4,5-d]pyrido[2,3-b][1]benzazepine;
10-fluoro-2-[2-(methylsulfinyl)ethyl]-3,8-dihydroimidazo[4,5-d]pyrido[2,3-b][1]benzazepine;
2-[(benzyloxy)methyl]-10-fluoro-3,8-dihydroimidazo[4,5-d]pyrido[2,3-b][1]benzazepine;
Cis-[4-(10-fluoro-3,8-dihydroimidazo[4,5-d]pyrido[2,3-b][1]benzazepin-2-yl)cyclohexyl]methanol;
Trans-[4-(10-fluoro-3,8-dihydroimidazo[4,5-d]pyrido[2,3-b][1]benzazepin-2-yl)cyclohexyl]methanol;
Cis-4-(10-fluoro-3,8-dihydroimidazo[4,5-d]pyrido[2,3-b][1]benzazepin-2-yl)cyclohexanol;
Trans-4-(10-fluoro-3,8-dihydroimidazo[4,5-d]pyrido[2,3-b][1]benzazepin-2-yl)cyclohexanol;
Trans-[4-(10-fluoro-3,8-dihydroimidazo[4,5-d]pyrido[2,3-b][1]benzazepin-2-yl)cyclohexyl]acetonitrile;
2-[4-(10-fluoro-3,8-dihydroimidazo[4,5-d]pyrido[2,3-b][1]benzazepin-2-yl)cyclohexyl]acetamide;
tert-butyl 4-(10-fluoro-3,8-dihydroimidazo[4,5-d]pyrido[2,3-b][1]benzazepin-2-yl)piperidine-1-carboxylate;
10-fluoro-2-piperidin-4-yl-3,8-dihydroimidazo[4,5-d]pyrido[2,3-b][1]benzazepine;
3-[4-(10-fluoro-3,8-dihydroimidazo[4,5-d]pyrido[2,3-b][1]benzazepin-2-yl)piperidin-1-yl]-3-oxopropanenitrile;
2-(1-acetylpiperidin-4-yl)-10-fluoro-3,8-dihydroimidazo[4,5-d]pyrido[2,3-b][1]benzazepine;
10-fluoro-2-[1-(methylsulfonyl)piperidin-4-yl]-3,8-dihydroimidazo[4,5-d]pyrido[2,3-b][1]benzazepine;
[4-(10-fluoro-3,8-dihydroimidazo[4,5-d]pyrido[2,3-b][1]benzazepin-2-yl)pipendin-1-yl]acetonitrile;
ethyl [4-(10-fluoro-3,8-dihydroimidazo[4,5-d]pyrido[2,3-b][1]benzazepin-2-yl)piperidin-1-yl]acetate;
2-[4-(10-fluoro-3,8-dihydroimidazo[4,5-d]pyrido[2,3-b][1]benzazepin-2-yl)piperidin-1-yl]ethanol;
3-[4-(10-fluoro-3,8-dihydroimidazo[4,5-d]pyrido[2,3-b][1]benzazepin-2-yl)piperidin-1-yl]propan-1-ol;
4-[4-(10-fluoro-3,8-dihydroimidazo[4,5-d]pyrido[2,3-b][1]benzazepin-2-yl)piperidin-1-yl]butanenitrile;
10-fluoro-2-[3-methyl-5-(trifluoromethyl)-1H-pyrazol-4-yl]-3,8-dihydroimidazo[4,5-d]pyrido[2,3-b][1]benzazepine;
2-[3-(difluoromethyl)-5-methyl-1H-pyrazol-4-yl]-10-fluoro-3,8-dihydroimidazo[4,5-d]pyrido[2,3-b][1]benzazepine;
2-(3,5-dimethyl-1H-pyrazol-4-yl)-10-fluoro-3,8-dihydroimidazo[4,5-d]pyrido[2,3-b][1]benzazepine;
2-(3,5-diethyl-1H-pyrazol-4-yl)-10-fluoro-3,8-dihydroimidazo[4,5-d]pyrido[2,3-b][1]benzazepine;
[4-(10-fluoro-3,8-dihydroimidazo[4,5-d]pyrido [2,3-b][1]benzazepin-2-yl)-5-methyl-3-(trifluoromethyl)-1H-pyrazol-1 -yl] acetonitrile;
2-(2-chloro-6-methylphenyl)-10-fluoro-3,8-dihydroimidazo[4,5-d]pyrido[2,3-b][1]benzazepine trifluoroacetate;
2-(10-fluoro-1,8-dihydroimidazo[4,5-d]pyrido[2,3-b][1]benzazepin-2-yl)-3-methylbenzonitrile trifluoroacetate;
2-(2,6-dimethylphenyl)-10-fluoro-3,8-dihydroimidazo[4,5-d]pyrido[2,3-b][1]benzazepine;
2-(3,5-dichloropyridin-4-yl)-10-fluoro-3,8-dihydroimidazo[4,5-d]pyrido[2,3-b][1]benzazepine; and
2-(2,4-dimethylpyridin-3-yl)-10-fluoro-3,8-dihydroimidazo[4,5-d]pyrido[2,3-b][1]benzazepine;
or pharmaceutically acceptable salt thereof.

In some embodiments, the agent is selected from:
4-(3,8-dihydroimidazo[4,5-d]dipyrido[2,3-b:4',3'-f]azepin-2-yl)-4-ethylhexanenitrile;
2-[4-(hydroxymethyl)-cyclohexyl]-imidazo[4,5-d]dipyrido[2,3-b:4',3'-f]azepin-3(8H)-ol;
[4-(3,8-dihydroimidazo[4,5-d]dipyrido[2,3-b:4',3'-f]azepin-2-yl)cyclohexyl]methanol;
[4-(3-hydroxy-3,8-dihydroimidazo[4,5-d]dipyrido[2,3-b:4',3'-f]azepin-2-yl)cyclohexyl]acetonitrile;
[4-(3,8-dihydroimidazo[4,5-d]dipyrido[2,3-b:4',3'-f]azepin-2-yl)cyclohexyl]acetonitrile;
3-(3,8-dihydroimidazo[4,5-d]dipyrido[2,3-b:4',3'-f]azepin-2-yl)-3-methylbutanenitrile;
3-(3,8-dihydroimidazo[4,5-d]dipyrido[2,3-b:4',3'-f]azepin-2-yl)-3-ethylpentanenitrile;
[3-(3,8-dihydroimidazo[4,5-d]dipyrido[2,3-b:4',3'-f]azepin-2-yl)cyclopentyl]methanol;
3-(3,8-dihydroimidazo[4,5-d]dipyrido[2,3-b:4',3'-f]azepin-2-yl)cyclopentanecarbonitrile;
2-(3,5-dichloropyridin-4-yl)-3,8-dihydroimidazo[4,5-d]dipyrido[2,3-b:4',3'-f]azepine;
2-(2,6-dichlorophenyl)-3,8-dihydroimidazo[4,5-d]dipyrido[2,3-b:4',3'-f]azepine;
2-(2,6-dimethylphenylj-3,8-dihydroimidazo[4,5-d]dipyrido[2,3-b:4',3'-f]azepine ;
2-(2-fluoro-6-methoxyphenyl)-3,8-dihydroimidazo[4,5-d]dipyrido[2,3-b:4',3'-f]azepine;
2-(6-chloro-2-fluoro-3-methylphenyl)-3,8-dihydroimidazo[4,5-d]dipyrido[2,3-b:4',3'-f]azepine;
2-[2-fluoro-6-(trifluoromethyl)phenyl]-3,8-dihydroimidazo[4,5-d]dipyrido[2,3-b:4',3'-f]azepine;
2-(2-chloro-6-fluoro-3-methylphenyl)-3,8-dihydroimidazo[4,5-d]dipyrido[2,3-b:4',3'-f]azepine;
2-[3-chloro-2-fluoro-6-(trifluoromethyl)phenyl]-3,8-dihydroimidazo[4,5-d]dipyrido[2,3-b:4',3'-f]azepine;
2-(2-chloro-6-fluorophenyl)-3,8-dihydroimidazo[4,5-d]dipyrido[2,3-b:4',3'-f]azepine;
2-[2-chloro-5-(trifluoromethyl)phenyl]-3,8-dihydroimidazo[4,5-d]dipyrido[2,3-b:4',3'-f]azepine;
2-(2,6-difluorophenyl)-3,8-dihydroimidazo[4,5-d]dipyrido[2,3-b:4',3'-f]azepine;
2-(2,5-dichlorophenyl)-3,8-dihydroimidazo[4,5-d]dipyrido[2,3-b:4',3'-f]azepine;
2-(2,6-dibromopyridin-4-yl)-3,8-dihydroimidazo[4,5-d]dipyrido[2,3-b:4',3'-f]azepine;
2-(2-bromophenyl)-3,8-dihydroimidazo[4,5-d]dipyrido[2,3-b:4',3'-f]azepine;
2-(2-methylphenyl)-3,8-dihydroimidazo[4,5-d]dipyrido[2,3-b:4',3'-f]azepine;
2-(2-chlorophenyl)-3,8-dihydroimidazo[4,5-d]dipyrido[2,3-b:4',3'-f]azepine;
2-(2-ethylphenyl)-3,8-dihydroimidazo[4,5-d]dipyrido[2,3-b:4',3'-f]azepine;
2-(2,5-dimethylphenyl)-3,8-dihydroimidazo[4,5-d]dipyrido[2,3-b:4',3'-f]azepine;
2-[2-chloro-3-(trifluoromethyl)phenyl]-3,8-dihydroimidazo[4,5-d]dipyrido[2,3-b:4',3'-f]azepine;
2-[2,5-bis(trifluoromethyl)phenyl]-3,8-dihydroimidazo[4,5-d]dipyrido[2,3-b:4',3'-f]azepine;
2-(3-chloro-2,6-difluorophenyl)-3,8-dihydroimidazo[4,5-d]dipyrido[2,3-b:4',3'-f]azepine;
2-[2-(trifluoromethyl)phenyl]-3,8-dihydroimidazo[4,5-d]dipyrido[2,3-b:4',3'-f]azepine;
2-(2,3-dichlorophenyl)-3,8-dihydroimidazo[4,5-d]dipyrido[2,3-b:4',3'-f]azepine;
3-[[4-(3,8-dihydroimidazo[4,5-d]dipyrido[2,3-b:4',3'-f]azepin-2-ylphenyl](ethyl)amino]propanenitrile;
2-(2-chloro-3,6-difluorophenyl)-3,8-dihydroimidazo[4,5-d]dipyrido[2,3-b:4',3'-f]azepine;
2-(2-bromopyridin-4-yl)-3,8-dihydroimidazo[4,5-d]dipyrido[2,3-b:4',3'-f]azepine;
2-(5-bromo-2,3-dimethoxyphenyl)-3,8-dihydroimidazo[4,5-d]dipyrido[2,3-b:4',3'-f]azepine;
2-[3-chloro-2-fluoro-5-(trifluoromethyl) phenyl]-3,8-dihydroimidazo[4,5-d]dipyrido[2,3-b:4',3'-f]azepine;
2-(2-chloropyridin-4-yl)-3,8-dihydroimidazo[4,5-d]dipyrido[2,3-b:4',3'-f]azepine;
2-(2,3-dimethylphenyl)-3,8-dihydroimidazo[4,5-d]dipyrido[2,3-b:4',3'-f]azepine;
2-[2-fluoro-3-(trifluoromethyl) phenyl]-3,8-dihydroimidazo[4,5-d]dipyrido[2,3-b:4',3'-f]azepine;
2-(3-fluoro-2-methyl phenyl)-3,8-dihydroimidazo[4,5-d]dipyrido[2,3-b:4',3'-f]azepine;
2-(2-fluorophenyl)-3,8-dihydroimidazo[4,5-d]dipyrido[2,3-b:4',3'-f]azepine;
2-(5-bromo-2-methoxyphenyl)-3,8-dihydroimidazo[4,5-d]dipyrido[2,3-b:4',3'-f]azepine;
2-[2-fluoro-5-(trifluoromethyl) phenyl]-3,8-dihydroimidazo[4,5-d]dipyrido[2,3-b:4',3'-f]azepine;
2-(2-fluoro-3-methoxyphenyl)-3,8-dihydroimidazo[4,5-d]dipyrido[2,3-b:4',3'-f]azepine;
2-(2-fluoro-5-methoxyphenyl)-3,8-dihydroimidazo[4,5-d]dipyrido[2,3-b:4',3'-f]azepine;
2-(2,3-difluorophenyl)-3,8-dihydroimidazo[4,5-d]dipyrido[2,3-b:4',3'-f]azepine;
2-quinolin-4-yl-3,8-dihydroimidazo[4,5-d]dipyrido[2,3-b:4',3'-f]azepine;
2-(5-fluoro-2-methoxyphenyl)-3,8-dihydroimidazo[4,5-d]dipyrido[2,3-b:4',3'-f]azepine;
2-(5-bromo-2-fluorophenyl)-3,8-dihydroimidazo[4,5-d]dipyrido[2,3-b:4',3'-f]azepine;
2-(2,5-dimethoxyphenyl)-3,8-dihydroimidazo[4,5-d]dipyrido[2,3-b:4',3'-f]azepine;
2-(2,5-difluorophenyl)-3,8-dihydroimidazo[4,5-d]dipyrido[2,3-b:4',3'-f]azepine;
2-(3,5-dimethyl-1H-pyrrol-2-yl)-3,8-dihydroimidazo[4,5-d]dipyrido[2,3-b:4',3'-f]azepine;
2-(2,6-dimethoxyphenyl)-3,8-dihydroimidazo[4,5-d]dipyrido[2,3-b:4',3'-f]azepine;
2-(4-methyl-1H-imidazol-5yl)-3,8-dihydroimidazo[4,5-d]dipyrido[2,3-b:4',3'-f]azepine;
4-(3,8-dihydroimidazo[4,5-d]dipyrido[2,3-b:4',3'-f]azepin-2-yl)-3,5-dimethoxyphenol;
2-(2-pentafluoroethyl)-3,8-dihydroimidazo[4,5-d]dipyrido[2,3-b:4',3'-f]azepine;
2-(5-bromo-1,3-benzodioxol-4-yl)-3,8-dihydroimidazo[4,5-d]dipyrido[2,3-b:4',3'-f]azepine;
4-(3,8-dihydroimidazo[4,5-d]dipyrido[2,3-b:4',3'-f]azepin-2-yl)-4-phenylpentanenitrile;
2-[2-fluoro-4-(trifluoromethyl)phenyl]-3,8-dihydroimidazo[4,5-d]dipyrido[2,3-b:4',3'-f]azepine;
2-(cyclohexylmethyl)-3,8-dihydroimidazo[4,5-d]dipyrido[2,3-b:4',3'-f]azepine;
3-bromo-2-(3,8-dihydroimidazo[4,5-d]dipyrido[2,3-b:4',3'-f]azepin-2-yl)-6-methoxyphenol;
2-(2-fluoropyridin-4-yl)-3,8-dihydroimidazo[4,5-d]dipyrido[2,3-b:4',3'-f]azepine;
2-biphenyl-2-yl-3,8-dihydroimidazo[4,5-d]dipyrido[2,3-b:4',3'-f]azepine;
methyl-4-(3,8-dihydroimidazo[4,5-d]dipyrido[2,3-b:4',3'-f]azepin-2-yl)benzoate;
2-[2-(ethylthio)phenyl]-3,8-dihydroimidazo[4,5-d]dipyrido[2,3-b:4',3'-f]azepine;
2-(1H-pyrrol-2-yl)-3,8-dihydroimidazo[4,5-d]dipyrido[2,3-b:4',3'-f]azepine;
2-{4-[(trifluoromethyl)thio]phenyl}-3,8-dihydroimidazo[4,5-d]dipyrido[2,3-b:4',3'-f]azepine;
2-(2-naphthyl)-3,8-dihydroimidazo[4,5-d]dipyrido[2,3-b:4',3'-f]azepine;
tert-butyl[1-(3,8-dihydroimidazo[4,5-d]dipyrido[2,3-b:4',3'-f]azepin-2-yl)-2-phenethyl] carbamate;
2-pyrrolidin-2-yl-3,8-dihydroimidazo[4,5-d]dipyrido[2,3-b:4',3'-f]azepine;
2-(2-chloro-6-methoxyquinolin-3-yl)-3,8-dihydroimidazo[4,5-d]dipyrido[2,3-b:4',3'-f]azepine;
2-(1-acetylpyrrolidin-2-yl)-3,8-dihydroimidazo[4,5-d]dipyrido[2,3-b:4',3'-f]azepine;
2-(1,3-thiazol-2-yl)-3,8-dihydroimidazo[4,5-d]dipyrido[2,3-b:4',3'-f]azepine;
1-acetyl-5-(3,8-dihydroimidazo[4,5-d]dipyrido[2,3-b:4',3'-f]azepin-2-yl)pyrrolidin-3-ol;
N-[1-(3,8-dihydroimidazo[4,5-d]dipyrido[2,3-b:4',3'-f]azepin-2-yl)ethyl]acetamide;
tert-butyl 2-(3,8-dihydroimidazo[4,5-d]dipyrido[2,3-b:4',3'-f]azepin-2-yl)-4-hydroxypyrrolidine-1-carboxylate;
N-[4-(3,8-dihydroimidazo[4,5-d]dipyrido[2,3-b:4',3'-f]azepin-2-yl)phenyl]acetamide;
2-[4-(difluoromethoxy)phenyl)-3,8-dihydroimidazo[4,5-d]dipyrido[2,3-b:4',3'-f]azepine;
2-(6-chloropyridin-3-yl)-3,8-dihydroimidazo[4,5-d]dipyrido[2,3-b:4',3'-f]azepine;
6-(3,8-dihydroimidazo[4,5-d]dipyrido[2,3-b:4',3'-f]azepin-2-yl)-2(methylthio)nicotinonitrile;
2-(3-fluoropyridin-4-yl)-3,8-dihydroimidazo[4,5-d]dipyrido[2,3-b:4',3'-f]azepine;
2-(6-methoxypyridin-3-yl)-3,8-dihydroimidazo[4,5-d]dipyrido[2,3-b:4',3'-f]azepine;
2-(6-bromopyridin-3-yl)-3,8-dihydroimidazo[4,5-d]dipyrido[2,3-b:4',3'-f]azepine;
2-(6-bromopyridin-2-yl)-3,8-dihydroimidazo[4,5-d]dipyrido[2,3-b:4',3'-f]azepine;
2-(1H-imidazol-4-yl)-3,8-dihydroimidazo[4,5-d]dipyrido[2,3-b:4',3'-f]azepine;
2-(3-methoxyphenyl)-3,8-dihydroimidazo[4,5-d]dipyrido[2,3-b:4',3'-f]azepine;
2-(4-methoxyphenyl)-3,8-dihydroimidazo[4,5-d]dipyrido[2,3-b:4',3'-f]azepine;
2-[2-(methylthio)ethyl]-3,8-dihydroimidazo[4,5-d]dipyrido[2,3-b:4',3'-f]azepine;
2-(piperidin-4-yl)-3,8-dihydroimidazo[4,5-d]dipyrido[2,3-b:4',3'-f]azepine;
3-[4-(3,8-dihydroimidazo[4,5-d]dipyrido[2,3-b:4',3'-f]azepin-2-yl)piperidin-1-yl]-3-oxopropanenitrile;
3-[3-(3,8-dihydroimidazo[4,5-d]dipyrido[2,3-b:4',3'-f]azepin-2-yl)piperidin-1-yl]-3-oxopropanenitrile;
3-[4-(3,8-dihydroimidazo[4,5-d]dipyrido[2,3-b:4',3'-f]azepin-2-yl)piperidin-1-ylmethyl]-3-oxopropanenitrile;
3-[3-(3,8-dihydroimidazo[4,5-d]dipyrido[2,3-b:4',3'-f]azepin-2-yl)piperidin-1-ylmethyl]-3-oxopropanenitrile;
2-[1-(trifluoroacetyl)piperidin-4-yl] 3,8-dihydroimidazo[4,5-d]dipyrido[2,3-b:4',3'-f]azepine bis(trifluoroacetate);
2-[1-(trifluoroacetyl)piperidin-3-yl] 3,8-dihydroimidazo[4,5-d]dipyrido[2,3-b:4',3'-f]azepine;
2-{[1-(trifluoroacetyl)piperidin-4-yl]methyl}- 3,8-dihydroimidazo[4,5-d]dipyrido[2,3-b:4',3'-f]azepine;
2-{[1-(trifluoroacetyl)piperidin-3-yl]methyl}- 3,8-dihydroimidazo[4,5-d]dipyrido[2,3-b:4',3'-f]azepine;
2-(1-acetylpiperidin-4-yl)-3,8-dihydroimidazo[4,5-d]dipyrido[2,3-b:4',3'-f]azepine;
4-(3,8-dihydroimidazo[4,5-d]dipyrido[2,3-b:4',3'-f]azepin-2-yl)piperidine-1-carbaldehyde;
2-[(1-acetylpiperidin-4-yl)methyl]-3,8-dihydroimidazo[4,5-d]dipyrido[2,3-b:4',3'-f]azepine;
2-[(1-acetylpiperidin-3-yl)methyl]-3,8-dihydroimidazo[4,5-d]dipyrido[2,3-b:4',3'-f]azepine;
2-(1-methylpiperidin-3-yl)-3,8-dihydroimidazo[4,5-d]dipyrido[2,3-b:4',3'-f]azepine;
4-(3,8-dihydroimidazo[4,5-d]dipyrido[2,3-b:4',3'-f]azepin-2-yl)benzonitrile;
4-(3-hydroxy-3,8-dihydroimidazo[4,5-d]dipyrido[2,3-b:4',3'-f]azepin-2-yl)benzonitrile;
3-(3,8-dihydroimidazo[4,5-d]dipyrido[2,3-b:4',3'-f]azepin-2-yl)benzonitrile;
2-pyridin-3-yl-3,8-dihydroimidazo[4,5-d]dipyrido[2,3-b:4',3'-f]azepine;
2-pyridin-3-ylimidazo[4,5-d]dipyrido[2,3-b:4',3'-f]azepin-3(8H)-ol;
2-pyridin-2-ylimidazo[4,5-d]dipyrido[2,3-b:4',3'-f]azepin-3(8H)-ol;
2-pyridin-4-yl-3,8-dihydroimidazo[4,5-d]dipyrido[2,3-b:4',3'-f]azepine;
2-pyridin-4-ylimidazo[4,5-d]dipyrido[2,3-b:4',3'-f]azepin-3(8H)-ol;
2-piperidin-3-yl-3,8-dihydroimidazo[4,5-d]dipyrido[2,3-b:4',3'-f]azepine;
2-[4-(trifluoromethyl)phenyl]-3,8-dihydroimidazo[4,5-d]dipyrido[2,3-b:4',3'-f]azepine;
2-[6-(trifluoromethyl)pyridine-3-yl]-3,8-dihydroimidazo[4,5-d]dipyrido[2,3-b:4',3'-f]azepine;
2-[3-(trifluoromethyl)phenyl]-3,8-dihydroimidazo[4,5-d]dipyrido[2,3-b:4',3'-f]azepine;
2-(3,5-dimethylisoxazol-4-yl)imidazo [4,5-d]dipyrido[2,3-b:4',3'-f]azepin-3(8H)-ol;
2-[4-(methylthio)phenyl]-3,8-dihydroimidazo[4,5-d]dipyrido[2,3-b:4',3'-f]azepine;
2-[4-(methylthio)phenyl]imidazo [4,5-d]dipyrido[2,3-b:4',3'-f]azepin-3(8H)-ol;
2-[4-(methylsulfonyl)phenyl]-3,8-dihydroimidazo[4,5-d]dipyrido[2,3-b:4',3'-f]azepine;
2-(1H-imidazol-2-yl)-3,8-dihydroimidazo[4,5-d]dipyrido[2,3-b:4',3'-f]azepine;
2-(1-methyl-1H-imidazol-2-yl)-3,8-dihydroimidazo[4,5-d]dipyrido[2,3-b:4',3'-f]azepine;
2-phenyl-3,8-dihydroimidazo[4,5-d]dipyrido[2,3-b:4',3'-f]azepine;
2-phenylimidazo [4,5-d]dipyrido[2,3-b:4',3'-f]azepin-3(8H)-ol;
2-benzyl-3,8-dihydroimidazo[4,5-d]dipyrido[2,3-b:4',3'-f]azepine;
2-benzylimidazo[4,5-d]dipyrido[2,3-b:4',3'-f]azepin-3(8H)-ol;
2-(2-phenethyl)-3,8-dihydroimidazo[4,5-d]dipyrido[2,3-b:4',3'-f]azepine;
2-(2-phenethyl)imidazo[4,5-d]dipyrido[2,3-b:4',3'-f]azepin-3(8H)-ol;
2-piperidin-4-yl-3,8-dihydroimidazo[4,5-d]dipyrido[2,3-b:4',3'-f]azepine;
2-piperidin-4-yl imidazo[4,5-d]dipyrido[2,3-b:4',3'-f]azepin-3(8H)-ol;
2-(piperidin-4-ylmethyl)-3,8-dihydroimidazo[4,5-d]dipyrido[2,3-b:4',3'-f]azepine;
2-(piperidin-4-ylmethyl) imidazo[4,5-d]dipyrido[2,3-b:4',3'-f]azepin-3(8H)-ol;
2-(piperidin-3-ylmethyl)-3,8-dihydroimidazo[4,5-d]dipyrido[2,3-b:4',3'-f]azepine;
2-(tetrahydro-2H-pyran-4-ylmethyl)-3,8-dihydroimidazo[4,5-d]dipyrido[2,3-b:4',3'-f]azepine;
2-(tetrahydro-2H-pyran-4-ylmethyl)imidazo[4,5-d]dipyrido[2,3-b:4',3'-f]azepin-3(8H)-ol;
2-(tetrahydro-2H-thiopyran-4-ylmethyl)-3,8-dihydroimidazo[4,5-d]dipyrido[2,3-b:4',3'-f]azepine;
2-(tetrahydro-2H-thiopyran-4-ylmethyl)imidazo[4,5-d]dipyrido[2,3-b:4',3'-f]azepin-3(8H)-ol;
2-[5-methyl-3-(trifluoromethyl)-1H-pyrazol-4-yl]-3,8-dihydroimidazo[4,5-d]dipyrido[2,3-b:4',3'-f]azepine;
2-[3-(difluoromethyl)-5-methyl-1H-pyrazol-4-yl]-3,8-dihydroimidazo[4,5-d]dipyrido[2,3-b:4',3'-f]azepine;
2-[1-(4-methoxybenzyl)-3-methyl-5-(trifluoromethyl)-1H-pyrazol-4-yl]-3,8-dihydroimidazo[4,5-d]dipyrido[2,3-b:4',3'-f]azepine;
2-[1,5-dimethyl-3-(trifluoromethyl)-1H-pyrazol-4-yl]-3,8-dihydroimidazo[4,5-d]dipyrido[2,3-b:4',3'-f]azepine;
2-[1-ethyl-5-methyl-3-(trifluoromethyl)-1H-pyrazol-4-yl]-3,8-dihydroimidazo[4,5-d]dipyrido[2,3-b:4',3'-f]azepine;
2-[1-(cyclopropylmethyl)-5-methyl-3-(trifluoromethyl)-1H-pyrazol-4-yl]-3,8-dihydroimidazo[4,5-d]dipyrido[2,3-b:4',3'-f]azepine;
2-[3-methyl-5-(pentafluoroethyl)-1H-pyrazol-4-yl]-3,8-dihydroimidazo[4,5-d]dipyrido[2,3-b:4',3'-f]azepine;
4-(3,8-dihydroimidazo[4,5-d]dipyrido[2,3-b:4',3'-f]azepin-2-yl)-N,N,5-trimethyl-3-(trifluoromethyl)-1 H-pyrazole-1 -sulfonamide;
2-[3-[chloro(difluoro)methyl]-5-methyl-1-(tetrahydro-2H-pyran-2-yl)-1H-pyrazol-4-yl]-3,8-dihydroimidazo[4,5-d]dipyrido[2,3-b:4',3'-f]azepine;
2-[3-(difluoromethyl)-5-methyl-1-(tetrahydro-2H-pyran-2-yl)-1H-pyrazol-4-yl]-3,8-dihydroimidazo[4,5-d]dipyrido[2,3-b:4',3'-f]azepine;
2-(3,5-dimethyl-1H-pyrazol-4-yl)-3,8-dihydroimidazo[4,5-d]dipyrido[2,3-b:4',3'-f]azepine;
2-(3-isobutyl-5-methyl-1H-pyrazol-4-yl)-3,8-dihydroimidazo[4,5-d]dipyrido[2,3-b:4',3'-f]azepine;
2-(3-ethyl-5-methyl-1H-pyrazol-4-yl)-3,8-dihydroimidazo[4,5-d]dipyrido[2,3-b:4',3'-f]azepine;
2-(3-butyl-5-methyl-1H-pyrazol-4-yl)-3,8-dihydroimidazo[4,5-d]dipyrido[2,3-b:4',3'-f]azepine;
2-(3,5-diethyl-1H-pyrazol-4-yl)-3,8-dihydroimidazo[4,5-d]dipyrido[2,3-b:4',3'-f]azepine;
2-(5-cyclopropyl-3-methyl-1H-pyrazol-4-yl)-3,8-dihydroimidazo[4,5-d]dipyrido[2,3-b:4',3'-f]azepine;
2-(2-chloro-6-methylphenyl)-3,8-dihydroimidazo[4,5-d]dipyrido[2,3-b:4',3'-f]azepine;
2-(3,8-dihydroimidazo[4,5-d]dipyrido[2,3-b:4',3'-f]azepin-2-yl)-3-methylbenzonitrile;
2-(2,4-dimethyl-3-thienyl)-3,8-dihydroimidazo[4,5-d]dipyrido[2,3-b:4',3'-f]azepine;
2-[4-methylsulfinyl)phenyl]-3,8-dihydroimidazo[4,5-d]dipyrido[2,3-b:4',3'-f]azepine;
2-[4-(ethylthio)phenyl]-3,8-dihydroimidazo[4,5-d]dipyrido[2,3-b:4',3'-f]azepine;
2-{4-[2-(5-methyl-2-furyl)propyl]phenyl}-3,8-dihydroimidazo[4,5-d]dipyrido[2,3-b:4',3'-f]azepine;
2-(1-benzothien-5-yl)-3,8-dihydroimidazo[4,5-d]dipyrido[2,3-b:4',3'-f]azepine;
1-(2,4-dimethyl-1,3-thiazol-5-yl)-3,8-dihydroimidazo[4,5-d]dipyrido[2,3-b:4',3'-f]azepine;
2-(3-methyl-5-phenylisoxazol-4-yl)-3,8-dihydroimidazo[4,5-d]dipyrido[2,3-b:4',3'-f]azepine;
2-(4-chloro-1-methyl-1H-pyrazol-3-yl)-3,8-dihydroimidazo[4,5-d]dipyrido[2,3-b:4',3'-f]azepine;
2-(1,3,5-trimethyl-1H-pyrazol-4-yl)-3,8-dihydroimidazo[4,5-d]dipyrido[2,3-b:4',3'-f]azepine;
2-(5-chloro-1,3-dimethyl-1H-pyrazol-4-yl)-3,8-dihydroimidazo[4,5-d]dipyrido[2,3-b:4',3'-f]azepine;
2-(3,5-dimethylisoxazol-4-yl)-3,8-dihydroimidazo[4,5-d]dipyrido[2,3-b:4',3'-f]azepine;
2-[1-(2-methoxyethyl)-2,5-dimethyl-1H-pyrrol-3-yl]-3,8-dihydroimidazo[4,5-d]dipyrido[2,3-b:4',3'-f]azepine;
2-(1-cyclopropyl-2,5-dimethyl-1H-pyrrol-3-yl)-3,8-dihydroimidazo[4,5-d]dipyrido[2,3-b:4',3'-f]azepine;
2-[2,5-dimethoxy-4-(methylthio)phenyl]-3,8-dihydroimidazo[4,5-d]dipyrido[2,3-b:4',3'-f]azepine;
2-(trifluoromethyl)-3,8-dihydroimidazo[4,5-d]dipyrido[2,3-b:4',3'-f]azepine;
2-(2,4-dimethoxy-3-methylphenyl)-3,8-dihydroimidazo[4,5-d]dipyrido[2,3-b:4',3'-f]azepine;
2-[2-(methylthio)phenyl]-3,8-dihydroimidazo[4,5-d]dipyrido[2,3-b:4',3'-f]azepine;
2-(2-ethoxyphenyl)-3,8-dihydroimidazo[4,5-d]dipyrido[2,3-b:4',3'-f]azepine;
2-(2,4-dimethoxyphenyl)-3,8-dihydroimidazo[4,5-d]dipyrido[2,3-b:4',3'-f]azepine;
2-(2,3,4,5,6-pentamethylphenyl)-3,8-dihydroimidazo[4,5-d]dipyrido[2,3-b:4',3'-f]azepine;
2-(2-chloro-4-methoxyphenyl)-3,8-dihydroimidazo[4,5-d]dipyrido[2,3-b:4',3'-f]azepine;
2-(2-methyl-4-methoxyphenyl)-3,8-dihydroimidazo[4,5-d]dipyrido[2,3-b:4',3'-f]azepine;
2-(3,8-dihydroimidazo[4,5-d]dipyrido[2,3-b:4',3'-f]azepin-2-yl)phenol;
2-(2,5-dimethyl-4-methoxyphenyl)-3,8-dihydroimidazo[4,5-d]dipyrido[2,3-b:4',3'-f]azepine;
2-(2-chloro-3,4-dimethoxyphenyl)-3,8-dihydroimidazo[4,5-d]dipyrido[2,3-b:4',3'-f]azepine;
2-(2,3-dimethyl-4-methoxyphenyl)-3,8-dihydroimidazo[4,5-d]dipyrido[2,3-b:4',3'-f]azepine;
2-(2,6-dichloro-3,4-dimethoxyphenyl)-3,8-dihydroimidazo[4,5-d]dipyrido[2,3-b:4',3'-f]azepine;
2,4-dichloro-3-(3,8-dihydroimidazo[4,5-d]dipyrido[2,3-b:4',3'-f]azepin-2-yl)-6-methoxyphenol;
2-(2,4,5-trimethylphenyl)-3,8-dihydroimidazo[4,5-d]dipyrido[2,3-b:4',3'-f]azepine;
2-(2,4-dichlorophenyl)-3,8-dihydroimidazo[4,5-d]dipyrido[2,3-b:4',3'-f]azepine;
2-(2-chloro-4-fluorophenyl)-3,8-dihydroimidazo[4,5-d]dipyrido[2,3-b:4',3'-f]azepine;
2-(2,4-dimethylphenyl)-3,8-dihydroimidazo[4,5-d]dipyrido[2,3-b:4',3'-f]azepine;
2-[2-(trifluoromethoxy)phenyl]-3,8-dihydroimidazo[4,5-d]dipyrido[2,3-b:4',3'-f]azepine;
2-[2-(difluoromethoxy)phenyl]-3,8-dihydroimidazo[4,5-d]dipyrido[2,3-b:4',3'-f]azepine;
2-(2-methoxypyridin-3-yl)-3,8-dihydroimidazo[4,5-d]dipyrido[2,3-b:4',3'-f]azepine;
2-[4-fluoro-2-(trifluoromethyl)phenyl]-3,8-dihydroimidazo[4,5-d]dipyrido[2,3-b:4',3'-f]azepine;
2-(2-methoxyphenyl)-3,8-dihydroimidazo[4,5-d]dipyrido[2,3-b:4',3'-f]azepine;
2-(2,4,6-trimethylphenyl)-3,8-dihydroimidazo[4,5-d]dipyrido[2,3-b:4',3'-f]azepine;
2-chloro-3-(3,8-dihydroimidazo[4,5-d]dipyrido[2,3-b:4',3'-f]azepin-2-yl)-6-methoxyphenol;
2-(3-methylpyridin-2-yl)--3,8-dihydroimidazo[4,5-d]dipyrido[2,3-b:4',3'-f]azepine;
4-(3,8-dihydroimidazo[4,5-d]dipyrido[2,3-b:4',3'-f]azepin-2-yl)-3-ethoxy-N,N-diethylaniline;
2-(2,5-dimethoxy-4-bromophenyl)-3,8-dihydroimidazo[4,5-d]dipyrido[2,3-b:4',3'-f]azepine;
2-(2-isobutoxyphenyl)-3,8-dihydroimidazo[4,5-d]dipyrido[2,3-b:4',3'-f]azepine;
3-(3,8-dihydroimidazo[4,5-d]dipyrido[2,3-b:4',3'-f]azepin-2-yl)pyridine-2-amine;
2-(1H-indol-4-yl)-3,8-dihydroimidazo[4,5-d]dipyrido[2,3-b:4',3'-f]azepine;
2-{2-[(trifluormethyl)thio]phenyl}-3,8-dihydroimidazo[4,5-d]dipyrido[2,3-b:4',3'-f]azepine;
4-bromo-3-(3,8-dihydroimidazo[4,5-d]dipyrido[2,3-b:4',3'-f]azepin-2-yl)phenol;
2-[2-chloro-4-(methylsulfonyl)phenyl]-3,8-dihydroimidazo[4,5-d]dipyrido[2,3-b:4',3'-f]azepine;
2-(2,2-difluoro-1,3-benzodioxol-4-yl)-3,8-dihydroimidazo[4,5-d]dipyrido[2,3-b:4',3'-f]azepine;
4-(3,8-dihydroimidazo[4,5-d]dipyrido[2,3-b:4',3'-f]azepin-2-yl)-3,5-dimethylphenol;
2-(2-chloro-4,6-dimethoxyphenyl)-3,8-dihydroimidazo[4,5-d]dipyrido[2,3-b:4',3'-f]azepine;
2-(3-chlorophenyl)-3,8-dihydroimidazo[4,5-d]dipyrido[2,3-b:4',3'-f]azepine;
2-[3-fluoro-5-(trifluoromethyl)phenyl]-3,8-dihydroimidazo[4,5-d]dipyrido[2,3-b:4',3'-f]azepine;
2-(3,5-dichlorophenyl)-3,8-dihydroimidazo[4,5-d]dipyrido[2,3-b:4',3'-f]azepine;
2-(3-chloro-4-methoxyphenyl)-3,8-dihydroimidazo[4,5-d]dipyrido[2,3-b:4',3'-f]azepine;
2-(6-chloro-1,3-benzodioxol-5-yl)-3,8-dihydroimidazo[4,5-d]dipyrido[2,3-b:4',3'-f]azepine;
2-(4-chloro-2,6-difluorophenyl)-3,8-dihydroimidazo[4,5-d]dipyrido[2,3-b:4',3'-f]azepine;
2-(2-chloro-5,6-difluorophenyl)-3,8-dihydroimidazo[4,5-d]dipyrido[2,3-b:4',3'-f]azepine;
2-(2,6-dichloro-3-fluorophenyl)-3,8-dihydroimidazo[4,5-d]dipyrido[2,3-b:4',3'-f]azepine;
2-(2-chloro-6-fluoro-3-methoxyphenyl)-3,8-dihydroimidazo[4,5-d]dipyrido[2,3-b:4',3'-f]azepine;
2-(2,4,6-trifluorophenyl)-3,8-dihydroimidazo[4,5-d]dipyrido[2,3-b:4',3'-f]azepine;
2-[2-methoxy-4-(trifluoromethoxy)phenyl]-3,8-dihydroimidazo[4,5-d]dipyrido[2,3-b:4',3'-f]azepine;
2-(3-chloro-4-fluorophenyl)-3,8-dihydroimidazo[4,5-d]dipyrido[2,3-b:4',3'-f]azepine;
2-(4-fluoro-2-methylphenyl)-3,8-dihydroimidazo[4,5-d]dipyrido[2,3-b:4',3'-f]azepine;
2-(2,4-dichloro-6-methoxyphenyl)-3,8-dihydroimidazo[4,5-d]dipyrido[2,3-b:4',3'-f]azepine;
2-(2,6-dichloro-3-methoxyphenyl)-3,8-dihydroimidazo[4,5-d]dipyrido[2,3-b:4',3'-f]azepine;
2-(2,6-dichloro-4-methylthiophenyl)-3,8-dihydroimidazo[4,5-d]dipyrido[2,3-b:4',3'-f]azepine;
2-[2,6-dichloro -4-(methylsulfinyl)phenyl]-3,8-dihydroimidazo[4,5-d]dipyrido[2,3-b:4',3'-f]azepine;
2-[2,6-dichloro -4-(methylsulfonyl)phenyl]-3,8-dihydroimidazo[4,5-d]dipyrido[2,3-b:4',3'-f]azepine;
2-(2-chloro-6-fluoro-5-methoxyphenyl)-3,8-dihydroimidazo[4,5-d]dipyrido[2,3-b:4',3'-f]azepine;
2-(2-chloro-6-fluoro-4-methoxyphenyl)-3,8-dihydroimidazo[4,5-d]dipyrido[2,3-b:4',3'-f]azepine;
[3,5-dichloro-4-(3,8-dihydroimidazo[4,5-d]dipyrido[2,3-b:4',3'-f]azepin-2-yl)phenyl]methanol;
[3,5-dichloro-4-(3,8-dihydroimidazo[4,5-d]dipyrido[2,3-b:4',3'-f]azepin-2-yl)phenyl]methanol;
3,5-dichloro-2-(3,8-dihydroimidazo[4,5-d]dipyrido[2,3-b:4',3'-f]azepin-2-yl)phenol
5-chloro-4-(3,8-dihydroimidazo[4,5-d]dipyrido[2,3-b:4',3'-f]azepin-2-yl)nicotinonitrile;
4-(3,8-dihydroimidazo[4,5-d]dipyrido[2,3-b:4',3'-f]azepin-2-yl)pyridine-3,5-dicarbonitrile;
2-(3,8-dihydroimidazo[4,5-d]dipyrido[2,3-b:4',3'-f]azepin-2-yl)-3-fluoro-6-methylbenzonitrile;
4-(3,8-dihydroimidazo[4,5-d]dipyrido[2,3-b:4',3'-f]azepin-2-yl)nicotinonitrile;
2-(3,8-dihydroimidazo[4,5-d]dipyrido[2,3-b:4',3'-f]azepin-2-yl)-3,6-difluorobenzonitrile;
3-(3,8-dihydroimidazo[4,5-d]dipyrido[2,3-b:4',3'-f]azepin-2-yl)-2-fluoro-4-(trifluoromethyl)benzonitrile;
3-(3,8-dihydroimidazo[4,5-d]dipyrido[2,3-b:4',3'-f]azepin-2-yl)- 4-methoxybenzonitrile;
2-(3,8-dihydroimidazo[4,5-d]dipyrido[2,3-b:4',3'-f]azepin-2-yl)-5,6-dimethoxybenzonitrile;
2-(3,8-dihydroimidazo[4,5-d]dipyrido[2,3-b:4',3'-f]azepin-2-yl)-4,5-dimethoxyisophthalonitrile;
2-(3,8-dihydroimidazo[4,5-d]dipyrido[2,3-b:4',3'-f]azepin-2-yl)-4-hydroxy-5-methoxyisophthalonitrile;
4-(3,8-dihydroimidazo[4,5-d]dipyrido[2,3-b:4',3'-f]azepin-2-yl)-2,5-dimethoxybenzonitrile;
2-(3,8-dihydroimidazo[4,5-d]dipyrido[2,3-b:4',3'-f]azepin-2-yl)-5-(methylsulfonyl)benzonitrile;
2-(3,8-dihydroimidazo[4,5-d]dipyrido[2,3-b:4',3'-f]azepin-2-yl)-3,5-dimethoxybenzonitrile;
2-(3,8-dihydroimidazo[4,5-d]dipyrido[2,3-b:4',3'-f]azepin-2-yl)-3,5-difluorobenzonitrile;
2-(3,8-dihydroimidazo[4,5-d]dipyrido[2,3-b:4',3'-f]azepin-2-yl)-3,4-difluorobenzonitrile;
2-(3,8-dihydroimidazo[4,5-d]dipyrido[2,3-b:4',3'-f]azepin-2-yl)-3-fluoro-6-methoxybenzonitrile;
3-(3,8-dihydroimidazo[4,5-d]dipyrido[2,3-b:4',3'-f]azepin-2-yl)-6-fluorobenzonitrile;
2-(3,8-dihydroimidazo[4,5-d]dipyrido[2,3-b:4',3'-f]azepin-2-yl)-3-fluoro-4-methoxybenzonitrile;
2-(3,8-dihydroimidazo[4,5-d]dipyrido[2,3-b:4',3'-f]azepin-2-yl)-3-fluoro-5-methoxybenzonitrile;
2-(3,8-dihydroimidazo[4,5-d]dipyrido[2,3-b:4',3'-f]azepin-2-yl)-5-(methylthio)isophthalonitrile;
2-(3,8-dihydroimidazo[4,5-d]dipyrido[2,3-b:4',3'-f]azepin-2-yl)-5-(hydroxymethyl)isophthalonitrile;
2-(3,8-dihydroimidazo[4,5-d]dipyrido[2,3-b:4',3'-f]azepin-2-yl)-5-(hydroxymethyl)benzonitrile;
[3,5-dichloro-4-(3,8-dihydroimidazo[4,5-d]dipyrido[2,3-b:4',3'-f]azepin-2-yl)phenyl] acetonitrile;
5-(cyanomethyl)-2-(3,8-dihydroimidazo[4,5-d]dipyrido[2,3-b:4',3'-f]azepin-2-yl)isophthalonitrile;
5-(cyanomethyl)-2-(3,8-dihydroimidazo[4,5-d]dipyrido[2,3-b:4',3'-f]azepin-2-yl)benzonitrile;
2-(3,8-dihydroimidazo[4,5-d]dipyrido[2,3-b:4',3'-f]azepin-2-yl)-5-(methylsulfinyl)benzonitrile;
2-(3,8-dihydroimidazo[4,5-d]dipyrido[2,3-b:4',3'-f]azepin-2-yl)5-(methylsulfonyl)isophthalonitrile;
2-(3,8-dihydroimidazo[4,5-d]dipyrido[2,3-b:4',3'-f]azepin-2-yl)-5-hydroxybenzonitrile;
1-[3,5-dichloro-4-(3,8-dihydroimidazo[4,5-d]dipyrido[2,3-b:4',3'-f]azepin-2-yl)-2-phenyl]-N,N-dimethylmethanamine;
2-[2,6-dichloro-4-(morpholin-4-ylmethyl)phenyl]-3,8-dihydroimidazo[4,5-d]dipyrido[2,3-b:4',3'-f] azepine;
2-[2,6-dichloro-4-(thiomorpholin-4-ylmethyl)phenyl]-3,8-dihydroimidazo[4,5-d]dipyrido[2,3-b:4',3'-f]azepine;
2-[2,6-dichloro-4-(methoxymethyl)phenyl]-3,8-dihydroimidazo[4,5-d]dipyrido[2,3-b:4',3'-f]azepine;
2-[2,6-dichloro-4-(ethylthio)phenyl]-3,8-dihydroimidazo[4,5-d]dipyrido[2,3-b:4',3'-f]azepine;
2-[2,6-dichloro-4-(isopropylthio)phenyl]-3,8-dihydroimidazo[4,5-d]dipyrido[2,3-b:4',3'-f]azepine;
2-[2,6-dichloro-4-(ethylsulfinyl)phenyl]-3,8-dihydroimidazo[4,5-d]dipyrido[2,3-b:4',3'-f]azepine;
2-[2,6-dichloro-4-(ethylsulfonyl)phenyl]-3,8-dihydroimidazo[4,5-d]dipyrido[2,3-b:4',3'-f]azepine;
2-[2,6-dichloro-4-(isopropylsulfinyl)phenyl]-3,8-dihydroimidazo[4,5-d]dipyrido[2,3-b:4',3'-f]azepine;
2-[2,6-dichloro-4-(isopropylsulfonyl)phenyl]-3,8-dihydroimidazo[4,5-d]dipyrido[2,3-b:4',3'-f]azepine;
2-(3,8-dihydroimidazo[4,5-d]dipyrido[2,3-b:4',3'-f]azepin-2-yl)-5-(isopropylsulfonyl)benzonitrile;
2-(3,8-dihydroimidazo[4,5-d]dipyrido[2,3-b:4',3'-f]azepin-2-yl)-5-(ethylthio)isophthalonitrile;
3-chloro-2-(3,8-dihydroimidazo[4,5-d]dipyrido[2,3-b:4',3'-f]azepin-2-yl)-5-(ethylthio)benzonitrile;
2-(3,8-dihydroimidazo[4,5-d]dipyrido[2,3-b:4',3'-f]azepin-2-yl)-5-(isopropylthio)isophthalonitrile;
3-chloro-2-(3,8-dihydroimidazo[4,5-d]dipyrido[2,3-b:4',3'-f]azepin-2-yl)-5-(isopropylthio)benzonitrile;
2-(3,8-dihydroimidazo[4,5-d]dipyrido[2,3-b:4',3'-f]azepin-2-yl)-5-(isopropylsulfinyl)isophthalonitrile;
3-chloro-2-(3,8-dihydroimidazo[4,5-d]dipyrido[2,3-b:4',3'-f]azepin-2-yl)-5-(isopropylsulfinyl)benzo-nitrile;
2-[3,5-dichloro-4-(3,8-dihydroimidazo[4,5-d]dipyrido[2,3-b:4',3'-f]azepin-2-yl)phenoxy]acetonitrile;
2-[3,5-dichloro-4-(3,8-dihydroimidazo[4,5-d]dipyrido[2,3-b:4',3'-f]azepin-2-yl)phenoxy]propanenitrile;
5-(1-cyanoethoxy)-2-(3,8-dihydroimidazo[4,5-d]dipyrido[2,3-b:4',3'-f]azepin-2-yl)isophthalonitrile;
5-(1-cyanoethoxy)-2-(3,8-dihydroimidazo[4,5-d]dipyrido[2,3-b:4',3'-f]azepin-2-yl)benzonitrile;
5-(cyanomethoxy)-2-(3,8-dihydroimidazo[4,5-d]dipyrido[2,3-b:4',3'-f]azepin-2-yl)isophthalonitrile;
2-[3,5-dichloro-4-(3,8-dihydroimidazo[4,5-d]dipyrido[2,3-b:4',3'-f]azepin-2-yl)phenoxy]propanamide;
2-[3,5-dicyano-4-(3,8-dihydroimidazo[4,5-d]dipyrido[2,3-b:4',3'-f]azepin-2-yl)phenoxy]propanamide;
5-(2-amino-1-methyl-2-oxoethoxy)-3-cyano-2-(3,8-dihydroimidazo[4,5-d]dipyrido[2,3-b:4',3'-f]azepin-2-yl)benz amide;
5-(2-amino-1-methyl-2-oxoehtoxy)-2-(3,8-dihydroimidazo[4,5-d]dipyrido[2,3-b:4',3'-f] azepin-2-yl)isophthalamide;
2-[3,5-dichloro-4-(3,8-dihydroimidazo[4,5-d]dipyrido[2,3-b:4',3'-f]azepin-2-yl)phenoxy]acetamide;
2-[3,5-dicyano-4-(3,8-dihydroimidazo[4,5-d]dipyrido[2,3-b:4',3'-f]azepin-2-yl)phenoxy] ac etamide;
2-cyano-2-[3,5-dichloro-4-(3,8-dihydroimidazo[4,5-d]dipyrido[2,3-b:4',3'-f]azepin-2-yl)phenoxy]-N,N-dimethylacetamide;
2-(3,8-dihydroimidazo[4,5-d]dipyrido[2,3-b:4',3'-f]azepin-2-yl)isophthalonitrile;
2-(3,8-dihydroimidazo[4,5-d]dipyrido[2,3-b:4',3'-f]azepin-2-yl)benzonitrile;
2-(3,8-dihydroimidazo[4,5-d]dipyrido[2,3-b:4',3'-f]azepin-2-yl)-4-hydroxybenzonitrile;
4-(3,8-dihydroimidazo[4,5-d]dipyrido[2,3-b:4',3'-f]azepin-2-yl)-1,3-benzodioxole-5-carbonitrile;
3-chloro-2-(3,8-dihydroimidazo[4,5-d]dipyrido[2,3-b:4',3'-f]azepin-2-yl)benzonitrile;
3-fluoro-4-methyl-2-(3,8-dihydroimidazo[4,5-d]dipyrido[2,3-b:4',3'-f]azepin-2-yl)benzonitrile;
3-fluoro-2-(3,8-dihydroimidazo[4,5-d]dipyrido[2,3-b:4',3'-f]azepin-2-yl)benzonitrile;
3-methoxy-2-(3,8-dihydroimidazo[4,5-d]dipyrido[2,3-b:4',3'-f]azepin-2-yl)benzonitrile;
5-fluoro-2-(3,8-dihydroimidazo[4,5-d]dipyrido[2,3-b:4',3'-f]azepin-2-yl)benzonitrile;
3-chloro-6-methoxy-2-(3,8-dihydroimidazo[4,5-d]dipyrido[2,3-b:4',3'-f]azepin-2-yl)benzonitrile;
2-(3,8-dihydroimidazo[4,5-d]dipyrido[2,3-b:4',3'-f]azepin-2-yl)-4-methoxyisophthalonitrile;
tert-butyl [4-(3,8-dihydroimidazo[4,5-d]dipyrido[2,3-b:4',3'-f]azepin-2-yl)pyridin-3-yl]carbamate;
tert-butyl [4-(3-hydroxy-3,8-dihydroimidazo[4,5-d]dipyrido[2,3-b:4',3'-f]azepin-2-yl)pyridin-3-yl]carbamate;
2-(3,5-dimethylpyridin-4-yl)-3,8-dihydroimidazo[4,5-d]dipyrido[2,3-b:4',3'--f]azepine;
3,5-dichloro-4-(3,8-dihydroimidazo[4,5-d]dipyrido[2,3-b:4',3'-f]azepin-2-yl)phenol;
2-(2,6-dichloro-4-methoxyphenyl)-3,8-dihydroimidazo[4,5-d]dipyrido[2,3-b:4',3'-f]azepine;
2-(2,6-dichloro-4-ethoxyphenyl)-3,8-dihydroimidazo[4,5-d]dipyrido[2,3-b:4',3'-f]azepine;
2-(2,6-dichloro-4-isopropoxyphenyl)-3,8-dihydroimidazo[4,5-d]dipyrido[2,3-b:4',3'-f]azepine;
2-[2,6-dichloro-4-(trifluoromethoxy)phenyl]-3,8-dihydroimidazo[4,5-d]dipyrido[2,3-b:4',3'-f]azepine;
2-(3-methylpyridin-4-yl)-3,8-dihydroimidazo[4,5-d]dipyrido[2,3-b:4',3'-f]azepine;
2-(2-methylpyridin-3-yl)-3,8-dihydroimidazo[4,5-d]dipyrido[2,3-b:4',3'-f]azepine;
2-(2,4-dimethylpyridin-3-yl)-3,8-dihydroimidazo[4,5-d]dipyrido[2,3-b:4',3'-f]azepine;
2-(5-chloro-2-ethoxyphenyl)-3,8-dihydroimidazo[4,5-d]dipyrido[2,3-b:4',3'-f]azepine;
2-(5-chloro-2-methoxyphenyl)-3,8-dihydroimidazo[4,5-d]dipyrido[2,3-b:4',3'-f]azepine;
2-(5-bromo-2-ethoxyphenyl)-3,8-dihydroimidazo[4,5-d]dipyrido[2,3-b:4',3'-f]azepine;
2-(2,3-difluoro-6-methoxyphenyl)-3,8-dihydroimidazo[4,5-d]dipyrido[2,3-b:4',3'-f]azepine;
2-[2,6-dichloro-4-(trifluoromethyl)phenyl]-3,8-dihydroimidazo[4,5-d]dipyrido[2,3-b:4',3'-f]azepine;
3,5-dichloro-4-(3,8-dihydroimidazo[4,5-d]dipyrido[2,3-b:4',3'-f]azepin-2-yl)-N,N-dimethylaniline;
2-(2,6-dichloro-4-fluorophenyl)-3,8-dihydroimidazo[4,5-d]dipyrido[2,3-b:4',3'-f]azepine;
3-chloro-2-(3,8-dihydroimidazo[4,5-d]dipyrido[2,3-b:4',3'-f]azepin-2-yl)-5-methoxybenzonitrile;
2-(3,8-dihydroimidazo[4,5-d]dipyrido[2,3-b:4',3'-f]azepin-2-yl)-5-methoxyisophthalonitrile;
3-chloro-2-(3,8-dihydroimidazo[4,5-d]dipyrido[2,3-b:4',3'-f]azepin-2-yl)-5-ethoxybenzonitrile;
2-(3,8-dihydroimidazo[4,5-d]dipyrido[2,3-b:4',3'-f]azepin-2-yl)-5-ethoxyisophthalonitrile;
3-chloro-2-(3,8-dihydroimidazo[4,5-d]dipyrido[2,3-b:4',3'-f]azepin-2-yl)-5-isopropoxybenzonitrile;
2-(3,8-dihydroimidazo[4,5-d]dipyrido[2,3-b:4',3'-f]azepin-2-yl)-5-isopropoxyisophthalonitrile;
2-(3,8-dihydroimidazo[4,5-d]dipyrido[2,3-b:4',3'-f]azepin-2-yl)-5-(trifluoromethoxy)isophthalonitrile;
3-chloro-2-(3,8-dihydroimidazo[4,5-d]dipyrido[2,3-b:4',3'-f]azepin-2-yl)-5-(trifluoromethyl)benzonitrile;
3-chloro-2-(3,8-dihydroimidazo[4,5-d]dipyrido[2,3-b:4',3'-f]azepin-2-yl)-5-(dimethylamino)benzonitrile; and
3-chloro-2-(3,8-dihydroimidazo[4,5-d]dipyrido[2,3-b:4',3'-f]azepin-2-yl)-5-fluorobenzonitrile;
and pharmaceutically acceptable salts thereof.

In some embodiments, the agent is selected from:
2-(3,5-dichloropyridin-4-yl)-3,8-dihydroimidazo[4,5-d]dipyrido[2,3-b:3',4'-f]azepine;
2-(2,6-dichlorophenyl)-3,8-dihydroimidazo[4,5-d]dipyrido[2,3-b:3',4'-f]azepine;
2-(2,6-difluorophenyl)-3,8-dihydroimidazo[4,5-d]dipyrido[2,3-b:3',4'-f]azepine;
2-(2-chloro-6-fluorophenyl)-3,8-dihydroimidazo[4,5-d]dipyrido[2,3-b:3',4'-f]azepine;
2-(2,6-dimethylphenyl)-3,8-dihydroimidazo[4,5-d]dipyrido[2,3-b:3',4'-f]azepine;
4-(3,8-dihydroimidazo[4,5-d]dipyrido[2,3-b:3',4'-f]azepin-2-yl)-4-ethylhexanenitrile;
2-[1-(4-methoxybenzyl)-5-methyl-3-(trifluoromethyl)-1H-pyrazol-4-yl]imidazo[4,5-d]dipyrido[2,3-b:3',4'-f]azepin-3(8H)-ol;
2-[1-(4-methoxybenzyl)-5-methyl-3-(trifluoromethyl)-1H-pyrazol-4-yl]-3,8-dihydroimidazo[4,5-d]dipyrido[2,3-b:3',4'-f]azepine;
2-[5-methyl-3-(trifluoromethyl)-1H-pyrazol-4-yl]imidazo[4,5-d]dipyrido[2,3-b:3',4'-f]azepin-3(8H)-ol;
2-[5-methyl-3-(trifluoromethyl)-1H-pyrazol-4-yl]-3,8-dihydroimidazo[4,5-d]dipyrido[2,3-b:3',4'-f] azepine;
2-(2,3-dimethylphenyl)imidazo[4,5-d]dipyrido[2,3-b:3',4'-f]azepin-3(8H)-ol;
2-(2,3-dimethylphenyl)-1,8-dihydroimidazo[4,5-d]dipyrido[2,3-b:3',4'-f]azepine;
2-[2-(dimethylamino)-pyridin-3-yl]imidazo[4,5-d]dipyrido[2,3-b:3',4'-f]azepin-3(8H)-ol;
3-(3,8-dihydroimidazo[4,5-d]dipyrido[2,3-b:3',4'-f]azepin-2-yl)-N,N-dimethylpyridin-2-amine;
2-(2-cyano-6-fluorophenyl)-3,8-dihydroimidazo[4,5-d]dipyrido[2,3-b:3',4'-f]azepine
and pharmaceutically acceptable salts thereof.

In an eight aspect, the agent is selected from compounds of Formula VIII: 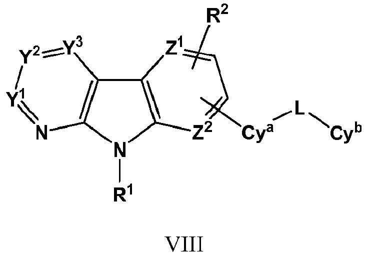 and pharmaceutically acceptable salts thereof; wherein:
Cy^{a} is selected from arylene, heteroarylene, cycloalkylene, and heterocycloalkylene, each optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1} OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1} NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1};
Cy^{b} is selected from aryl, heteroaryl, cycloalkyl, and heterocycloalkyl, each optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, 0C(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2};
L is a divalent moiety selected from C₁₋₆ alkylene, C₂₋₆ alkenylene, C₂₋₆ alkynylene, (C₁₋₆ alkylene)ₚ-(C₃₋₁₀ cycloalkylene)-(C₁₋₆ alkylene)_{q}, (C₁₋₆ alkylene)ₚ-(C₃₋₁₀ heterocycloalkylene)-( C₁₋₆ alkylene)_{q}, (C₁₋₆ alkylene)ₚ-(C₆₋₁₀ arylene)-( C₁₋₆ alkylene)_{q}. (C₁₋₆ alkylene)ₚ-(C₃₋₁₀ heteroarylene)-( C₁₋₆ alkylene)_{q}, (C₁₋₆ alkylene)ₚ-O-( C₁₋₆ alkylene)_{q}, (C₁₋₆ alkylene)ₚ-S-(C₁₋₆ alkylene)_{q}, (C₁₋₆ alkylene)ₚ-NR^{c3}-(C₁₋₆ alkylene)_{q}, (C₁₋₆ alkylene)ₚ-C(O)-(C₁₋₆ alkylene)_{q}, (C₁₋₆ alkylene)ₚ-OC(O)-(C₁₋₆ alkylene)_{q}, (C₁₋₆ alkylene)ₚ-C(O)NR^{c3}-(C₁₋₆ alkylene)_{q}, (C₁₋₆ alkylene)ₚ-OC(O)NR^{c3}-(C₁₋₆ alkylene)_{q}, (C₁₋₆ alkylene)ₚ-SO-(C₁₋₆ alkylene)_{q}, (C₁₋₆ alkylene)ₚ-SO₂-(C₁₋₆ alkylene)_{q}, (C₁₋₆ alkylene)ₚ-SONR^{c3}-(C₁₋₆ alkylene)_{q}, (C₁₋₆ alkylene)ₚ-SO₂NR^{c3}-(C₁₋₆ alkylene)_{q}, (C₁₋₆ alkylene)ₚ-NR^{c3}CONR^{d3}-(C₁₋₆ alkylene)_{q}, (C₁₋₆ alkylene)ₚ-NR^{c3}SONR^{d3}-(C₁₋₆ alkylene)_{q}, and (C₁₋₆ alkylene)ₚ-NR^{c3}SO₂NR^{d3}-(C₁₋₆ alkylene)_{q}, wherein each of the C₁₋₆ alkylene, C₂₋₆ alkenylene, C₂₋₆ alkynylene, cycloalkylene, arylene, heterocycloalkylene, and heteroarylene is optionally substituted by 1, 2 or 3 substituents independently selected from C₁₋₄ alkyl, C₂₋₄ alkenyl, C₂₋₄ alkynyl, C₁₋₄ hydroxyalkyl, C₁₋₄ cyanoalkyl, aryl, heteroaryl, cycloalkyl, heterocycloalkyl, halo, CN, NO₂, SCN, OH, C₁₋₄ haloalkyl, halosulfanyl, C₁₋₄ alkoxy-C₁₋₄ alkyl, C₁₋₄alkoxy, C₁₋₄ haloalkoxy, amino, C₁₋₄ alkylamino, and C₂₋₈ dialkylamino;
wherein L is oriented in either direction with respect to its attachment to Cy^{a} and Cy^{b};
Y¹ is selected from CR³ and N;
Y² is selected from CR⁴ and N;
Y³ is selected from CR⁵ and N;
provided that at least one of Y¹ and Y² is other than N;
Z¹ is selected from CR⁶ and N;
Z² is selected from CR⁷ and N;
R¹ is selected from H, C₁₋₆ alkyl, C(O)C₁₋₆ alkyl, and C(O)aryl;
R², R³, R⁴, R⁵, R⁶, and R⁷ are independently selected from H, halo, C₁₋₆ alkyl, C₂₋₆ alkenyl, C₂₋₆ alkynyl, Cy¹, C₁₋₆ haloalkyl, halosulfanyl, CN, NO₂, OR^{a4}, SR^{a4}, C(O)R^{b4}, C(O)NR^{c4}R^{d4}, C(O)OR^{a4}, OC(O)R^{b4}, OC(O)NR^{c4}R^{d4}, C(=NRⁱ)NR^{c4}R^{d4}, NR^{c4}C(=NRⁱ)NR^{c4}R^{d4}, NR^{c4}R^{d4}, NR^{c4}C(O)R^{b4}, NR^{c4}C(O)OR^{a4}, NR^{c4}C(O)NR^{c4}R^{d4}, NR^{c4}S(O)R^{b4}, NR^{c4}S(O)₂R^{b4}, S(O)R^{b4}, S(O)NR^{c4}R^{d4}, S(O)₂R^{b4}, and S(O)₂NR^{c4}R^{d4}; wherein the C₁₋₆ alkyl, C₂₋₆ alkenyl, or C₂₋₆ alkynyl is optionally substituted with 1, 2, 3, 4, or 5 substituents selected from CN, NO₂, Cy¹, Cy¹-(C₁₋₆ alkyl)-, OR^{a4}, SR^{a4}, C(O)R^{b4}, C(O)NR^{c4}R^{d4}, C(O)OR^{a4}, OC(O)R^{b4}, OC(O)NR^{c4}R^{d4}, NR^{c4}R^{d4}, NR^{c4}C(O)R^{b4}, NR^{c4}C(O)OR^{a4}, C(=NRⁱ)NR^{c4}R^{d4}, NR^{c4}C(=NRⁱ)NR^{c4}R^{d4}, S(O)R^{b4}, S(O)NR^{c4}R^{d4}, S(O)₂R^{b4}, and S(O)₂NR^{c4}R^{d4};
Cy, Cy¹, and Cy² are independently selected from aryl, heteroaryl, cycloalkyl, and heterocycloalkyl, each optionally substituted by 1, 2, 3, 4 or 5 substituents independently selected from halo, C₁₋₆ alkyl, C₂₋₆ alkenyl, C₂₋₆ alkynyl, C₁₋₆ haloalkyl, halosulfanyl, CN, NO₂, N₃, OR^{a5}, SR^{a5}, C(O)R^{b5}, C(O)NR^{c5}R^{d5}, C(O)OR^{a5}, 0C(O)R^{b5}, OC(O)NR^{c5}R^{d5} , NR^{c}5R^{d5}, NR^{c5}C(O)R^{b5}, NR^{c5}C(O)NR^{c5}R^{d5}, NR^{c5}C(O)OR^{a5}, C(=NRⁱ)NR^{c5}R^{d5}, NR^{c5}C(=NRⁱ)NR^{c5}R^{d5}, P(R^{f5})₂, P(OR^{e5})₂, P(O)R^{e5}R^{f5}, P(O)OR^{e5}OR^{f5}, S(O)R^{b5}, S(O)NR^{c5}R^{d5}, S(O)₂R^{b5}, NR^{c5}S(O)₂R^{b5}, and S(O)₂NR^{c5}R^{d5}, wherein the C₁₋₆ alkyl, C₂₋₆ alkenyl, and C₂₋₆ alkynyl are each optionally substituted by 1, 2, or 3 substituents independently selected from halo, C₁₋₆ haloalkyl, halosulfanyl, CN, NO₂, N₃, OR^{a5}, SR^{a5}, C(O)R^{b5}, C(O)NR^{c5}R^{d5}, C(O)OR^{a5}, OC(O)R^{b5}, OC(O)NR^{c5}R^{d5}, NR^{c5}R^{d5}, NR^{c5}C(O)R^{b5}, NR^{c5}C(O)NR^{c5}R^{d5}, NR^{c5}C(O)OR^{a5}, C(=NRⁱ)NR^{c5}R^{d5}, NR^{c5}C(=NRⁱ)NR^{c5}R^{d5}, P(R^{f5})₂, P(OR^{e5})₂, P(O)R^{e5}R^{f5}, P(O)OR^{e5}OR^{f5}, S(O)R^{b5}, S(O)NR^{c5}R^{d5}, S(O)₂R^{b5}, NR^{c5}S(O)₂R^{b5}, and S(O)₂NR^{c5}R^{d5} ;
R^{a1}, R^{a2}, and R^{a4}are independently selected from H, Cy², -(C₁₋₆alkyl)-Cy², C₁₋₆ alkyl, C₁₋₆ haloalkyl, C₂₋₆ alkenyl, C₂₋₆ alkynyl, and C(O)-C₁₋₇ hydrocarbyl, wherein the C₁₋₆ alkyl, C₂₋₆ alkenyl, C₁₋₇ hydrocarbyl, or C₂₋₆ alkynyl is optionally substituted with 1, 2, or 3 substituents independently selected from OH, C₁₋₆ alkoxy, CN, amino, alkylamino, dialkylamino, halo, C₁₋₆ alkyl, C₁₋₆ haloalkyl, halosulfanyl, aryl, arylalkyl, heteroaryl, heteroarylalkyl, cycloalkyl, and heterocycloalkyl;
R^{b1}, R^{b2}, and R^{b4} are independently selected from H, Cy², -(C₁₋₆ alkyl)-Cy², C₁₋₆ alkyl, C₁₋₆ haloalkyl, C₂₋₆ alkenyl, C₂₋₆ alkynyl, wherein the C₁₋₆ alkyl, C₂₋₆ alkenyl, or C₂₋₆ alkynyl is optionally substituted with 1, 2, or 3 substituents independently selected from OH, C₁₋₆ alkoxy, CN, amino, alkylamino, dialkylamino , halo, C₁₋₆ alkyl, C₁₋₆ haloalkyl, halosulfanyl, aryl, arylalkyl, heteroaryl, heteroarylalkyl, cycloalkyl, and heterocycloalkyl;
R^{c1}, R^{c2}, and R^{c4} are independently selected from H, Cy², -(C₁₋₆ alkyl)-Cy², C₁₋₆ alkyl, C₁₋₆ haloalkyl, C₁₋₆ hydroxyalkyl, C₂₋₆ alkenyl, C₂₋₆ alkynyl, wherein the C₁₋₆ alkyl, C₂₋₆ alkenyl, or C₂₋₆ alkynyl, is optionally substituted with 1, 2, or 3 substituents independently selected from OH, C₁₋₆ alkoxy, CN, amino, alkylamino, dialkylamino, halo, C₁₋₆ alkyl, C₁₋₆ haloalkyl, halosulfanyl, aryl, arylalkyl, heteroaryl, heteroarylalkyl, cycloalkyl, and heterocycloalkyl;
R^{d1}, R^{d2}, and R^{d4} are independently selected from H, Cy², -(C₁₋₆ alkyl)-Cy², C₁₋₆ alkyl, C₁₋₆ haloalkyl, C₂₋₆ alkenyl, C₂₋₆ alkynyl, wherein the C₁₋₆ alkyl, C₂₋₆ alkenyl, or C₂₋₆ alkynyl, is optionally substituted with 1, 2, or 3 substituents independently selected from OH, C₁₋₆ alkoxy, CN, amino, alkylamino, dialkylamino, halo, C₁₋₆ alkyl, C₁₋₆ haloalkyl, halosulfanyl, aryl, arylalkyl, heteroaryl, heteroarylalkyl, cycloalkyl, and heterocycloalkyl; or,
one or more of R^{c1} and R^{d1}, R^{c2} and R^{d2}, and R^{c4} and R^{d4} together with the N atom to which they are attached, optionally form a 4-, 5-, 6- or 7-membered heterocycloalkyl group or heteroaryl group, each optionally substituted with 1, 2, or 3 substituents independently selected from OH, C₁₋₆ alkoxy, CN, amino, alkylamino, dialkylamino, halo, C₁₋₆ alkyl, C₁₋₆ haloalkyl, halosulfanyl, aryl, arylalkyl, heteroaryl, heteroarylalkyl, cycloalkyl, and heterocycloalkyl;
R^{c3} and R^{d3} are independently selected from H, Cy², -(C₁₋₆ alkyl)-Cy², C₁₋₆ alkyl, C₁₋₆ haloalkyl, C₂₋₆ alkenyl, C₂₋₆ alkynyl, wherein the C₁₋₆ alkyl, C₂₋₆ alkenyl, or C₂₋₆ alkynyl is optionally substituted with 1, 2, or 3 substituents independently selected from OH, C₁₋₆ alkoxy, CN, amino, alkylamino, dialkylamino, halo, C₁₋₆ alkyl, C₁₋₆ haloalkyl, halosulfanyl, aryl, arylalkyl, heteroaryl, heteroarylalkyl, cycloalkyl, and heterocycloalkyl;
R^{a5} is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl, wherein the C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{b5} is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl, wherein the C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{c5} and R^{d5} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl, and heterocycloalkylalkyl, wherein the C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl, or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy; or
R^{c5} and R^{d5} together with the N atom to which they are attached form a 4-, 5-, 6- or 7-mcmbcrcd heterocycloalkyl group or heteroaryl group, each optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{e5} is H, C₁₋₆ alkyl, C₁₋₆ haloalkyl, C₂₋₆ alkenyl, (C₁₋₆ alkoxy)-C₁₋₆ alkyl, C₂₋₆ alkynyl, aryl, cycloalkyl, heteroaryl, heterocycloalkyl, arylalkyl, cycloalkylalkyl, heteroarylalkyl, or heterocycloalkylalkyl;
R^{f5} is H, C₁₋₆ alkyl, C₁₋₆ haloalkyl, C₂₋₆ alkenyl, C₂₋₆ alkynyl, aryl, cycloalkyl, heteroaryl, or heterocycloalkyl;
Rⁱ is H, CN, NO₂, C(O)NH₂, or C₁₋₆ alkyl;
p is 0 or 1; and
q is 0 or 1.

In some embodiments, when Cy^{a} is a piperazine ring, R² is other than halo.

In some embodiments, Y¹ is N and Y² is CR⁴.

In some embodiments, Y² is N and Y¹ is CR³.

In some embodiments, Y² is N.

In some embodiments, Y³ is N.

In some embodiments, Y³ is CR⁵.

In some embodiments, at least one of Y¹, Y² and Y³ is N.

In some embodiments, both of Y¹ and Y³ are N.

In some embodiments, both of Y² and Y³ are N.

In some embodiments, Y¹ is CR³, Y² is CR⁴, and Y³ is CR⁵.

In some embodiments, Z¹ is N.

In some embodiments, Z¹ is CR⁶.

In some embodiments, Z² is N.

In some embodiments, Z² is CR⁷.

In some embodiments, at least one of Z¹ and Z² is N.

In some embodiments, both of Z¹ and Z² are N.

In some embodiments, Z¹ is CR⁶ and Z² is CR⁷.

In some embodiments, Cy^{a} is aryl or heteroaryl, each optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)-R^{b1}, and S(O)₂NR^{c1}R^{d1}.

In some embodiments, Cy^{a} is aryl optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂k^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}.

In some embodiments, Cy^{a} is aryl optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{e1}R^{d}1, S(O)₂R^{b1}, and S(O)₂NR^{c1}R^{d1}.

In some embodiments, Cy^{a} is aryl optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, CN, NO₂, OR^{a1}, SR^{a1}, and NR^{c1}R^{d1}.

In some embodiments, Cy^{a} is aryl optionally substituted by 1, 2, 3, 4 or 5 substituents independently selected from halo, C₁₋₆ alkyl, C₂₋₆ and OR^{a1}.

In some embodiments, Cy^{a} is phenyl, optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)_{N}R^{c1}R^{d1}, S(O)₂R^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1} SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)-R^{b1}, and S(O)₂NR^{c1}R^{d1}.

In some embodiments, Cy^{a} is heteroaryl, optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{dl}, NR^{c1}C(=NRⁱ)NR^{c1}R^{d1}, NR^{c1}R^{d1}, NR^{c1}C(O)R^{b1}, NR^{c1}C(O)OR^{a1} NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}.

In some embodiments, Cy^{a} is cycloalkyl, optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{al}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1} NR^{c1}C(=NRⁱ)NR^{c1}R^{d1} NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}.

In some embodiments, Cy^{a} is heterocycloalkyl, optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1} NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR³¹, OC(O)R^{b1}, OC(O)NR^{c1}R^{d1}, C(=NR)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}.

In some embodiments, Cy^{a} is a substituted aryl or substituted heteroaryl ring according to Formula IA: 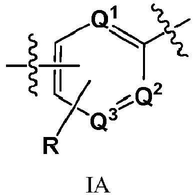 wherein:
R is selected from H, halo, C₁₋₆ alkyl, C₂₋₆ alkenyl, C₂₋₆ alkynyl, C_{y}, C₁₋₆ haloalkyl, halo-sulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1} NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1};
Q¹, Q² and Q³ are independently selected from CR^{Q} and N;
R^{Q} is independently selected from H, halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)k^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}S(O)₂R^{b1}, or S(O)₂NR^{c1}R^{d1}; wherein the C₁₋₆ alkyl, C₂₋₆ alkenyl, and C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}; and
the point of attachment on the right hand side of the ring of Formula IA is attached to L.

In some embodiments, Cy^{a} is a substituted aryl or substituted heteroaryl ring according to Formula IB: 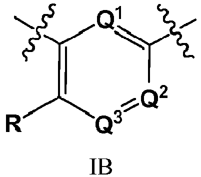 wherein:
R is selected from H, halo, C₁₋₆ alkyl, C₂₋₆ alkenyl, C₂₋₆ alkynyl, Cy, C₁₋₆ haloalkyl, halo-sulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1} NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NRⁱR^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1};
Q¹, Q² and Q³ are independently selected from CR^{Q} and N;
R^{Q} is independently selected from H, halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{bl}, S(O)NR^{c1}R^{d1}, S(O)₂R^{b1}, and S(O)₂NR^{c1}R^{d1}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, C(=NRⁱ)NR^{c1}R^{d1}, NR^{c1}C(=NRⁱ)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, S(O)R^{b1}, S(O)NR^{c1}R^{d1}, S(O)₂R^{b1}, and S(O)₂NR^{c1}R^{d1}; and
the point of attachment on the right hand side of the ring of Formula IB is attached to L.

In some embodiments, R is selected from H, C₁₋₆ alkyl, halo and OR^{a1}.

In some embodiments, R is selected from C₁₋₆ alkyl, halo and OR^{a1}.

In some embodiments, R is selected from C₁₋₆ alkyl and OR^{a1}.

In some embodiments, R is selected from C₁₋₆ alkyl and halo.

In some embodiments, Q¹ is N.

In some embodiments, Q¹ is CR^{Q}.

In some embodiments, Q² is N.

In some embodiments, Q² is CR^{Q}.

In some embodiments, Q³ is N.

In some embodiments, Q³ is CR^{Q}.

In some embodiments, at least one of Q¹, Q² and Q³ is N.

In some embodiments, at least two of Q¹, Q² and Q³ is N.

In some embodiments, all of Q¹, Q² and Q³ are N.

In some embodiments, all of Q¹, Q² and Q³ are CR^{Q}.

In some embodiments, R^{Q} is independently selected from H, halo, C₁₋₆ alkyl, C₁₋₆ haloalkyl, OR^{a1}, halosulfanyl, Cy, NR^{c1}R^{d1}, C(O)R^{b1}, and C(O)NR^{c1}R^{d1}.

In some embodiments, Cy^{a} is a substituted aryl or substituted heteroaryl ring according to Formula IC: 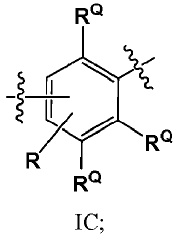 and the point of attachment on the right hand side of the ring of Formula IC is attached to L.

In some embodiments, Cy^{a} is a substituted aryl or substituted heteroaryl ring according to Formula ID: 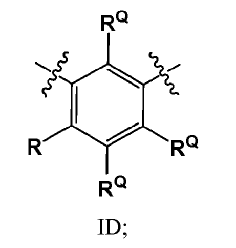 and the point of attachment on the right hand side of the ring of Formula ID is attached to L.

In some embodiments, Cy^{b} is aryl or heteroaryl, each optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}(O)OR^{a2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR³², OC(O)R^{b2}, OC(O)NR^{c2}R^{d2}, Q=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}(O)OR^{a2}, NR^{c2}(O)NR^{c2}R^{d2}, NR^{c2}S(O)R^{b2}, NR^{c2}S(O)₂R^{b2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}.

In some embodiments, Cy^{b} is aryl or heteroaryl, each optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}.

In some embodiments, Cy^{b} is aryl or heteroaryl, each optionally substituted by 1, 2, 3, 4 or 5 substituents independently selected from halo, C₁₋₆ alkyl, Cy, C₁₋₆ haloalkyl, halosulfanyl, CN, NO₂, OR^{a2}, and NR^{c2}R^{d2}.

In some embodiments, Cy^{b} is aryl, optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, or S(O)₂NR^{c2}R^{d2}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}-, C(O)R^{b2}, C(O)NR^{c2}R^{d2}, C(O)OR^{a2}, OC(O)R^{b2}, OC(O)NR^{c2}R^{d2}, Q=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}(O)OR^{a2}, NR^{c2}(O)NR^{c2}R^{d2}, NR^{c2}S(O)R^{b2}, NR^{c2}S(O)₂R^{b2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}.

In some embodiments, Cy^{b} is phenyl, optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, Q=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}(O)OR^{a2}, NR^{c2}(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR³², OC(O)R^{b2}, OC(O)NR^{c2}R^{d2}, Q=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}(O)OR^{a2}, NR^{c2}C(O)NR^{c2}R^{d2}, NR^{c2}S(O)R^{b2}, NR^{c2}S(O)₂R^{b2}, NR^{c2}(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}.

In some embodiments, Cy^{b} is heteroaryl, optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, Q=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}(O)OR^{a2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, CCO)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}.

In some embodiments, Cy^{b} is cycloalkyl, optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, CCO)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, CCO)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}.

In some embodiments, Cy^{b} is heterocycloalkyl, optionally substituted by 1, 2, 3, 4 or 5 substituents independently selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, CCO)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, CCO)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}.

In some embodiments, Cy^{b} is a substituted aryl or substituted heteroaryl ring according to Formula IE: 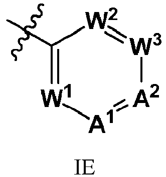 wherein:
W¹, W² and W³ are independently selected from CR^{W} and N;
A¹ and A² are independently selected from CR^{W} and N; or the group, A¹=A², is S, O, or NH; and
each R^{W} is independently selected from H, halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2};
provided that, when A¹ and A² are independently selected from CR^{W} and N; then at least three of W¹, W², W³, A¹ and A² are CR^{W}.

In some embodiments, Cy^{b} is a substituted aryl or substituted hctcroaryl ring according to Formula IF: 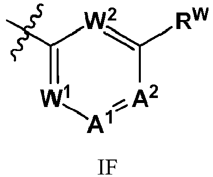 wherein:
W¹ and W² are independently selected from CR^{W} and N;
A¹ and A² are independently selected from CR^{W} and N; or
the group, A¹=A², is S, O, or NH; and
each R^{W} is independently selected from H, halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, and S(O)₂NR^{c2}R^{d2}; wherein the C₁₋₆ alkyl, C₂₋₆ alkenyl, or C₂₋₆ alkynyl is optionally substituted with 1, 2, 3, 4, or 5 substituents selected from halo, C₁₋₆ alkyl, C₂₋₆ alkenyl, C₂₋₆ alkynyl, Cy, C₁₋₆ haloalkyl, halosulfanyl, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, C(=NRⁱ)NR^{c2}R^{d2}, NR^{c2}C(=NRⁱ)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)R^{b2}, NR^{c2}S(O)₂R^{b2}, NR^{c2}C(O)NR^{c2}R^{d2}, NR^{c2}S(O)R^{b2}, NR^{c2}S(O)₂R^{b2}, S(O)R^{b2}, S(O)NR^{c2}R^{d2}, S(O)₂R^{b2}, or S(O)₂NR^{c2}R^{d2}.

In some embodiments, Q¹ is CR^{Q}.

In some embodiments, W¹ is N.

In some embodiments, W¹ is CR^{W}.

In some embodiments, W² is N.

In some embodiments, W² is CR^{W}.

In some embodiments, at least one of W¹ and W² is N.

In some embodiments, both of W¹ and W² are N.

In some embodiments, both of W¹ and W² are CR^{W}.

In some embodiments, A¹ is N.

In some embodiments, A¹ is CR^{W}.

In some embodiments, A² is N.

In some embodiments, A² is CR^{W}.

In some embodiments, at least one of W¹ and W² is N.

In some embodiments, both of W¹ and W² are N.

In some embodiments, both of W¹ and W² are CR^{W}.

In some embodiments, the group A¹=A² is S.

In some embodiments, the group A¹=A² is O.

In some embodiments, the group A¹=A² is NH.

In some embodiments, R^{W} is independently selected from H, halo, C₁₋₆ alkyl, C₁₋₆ haloalkyl, OR^{a1} halosulfanyl, Cy, NR^{c1}R^{d1}, C(O)R^{b1}, and C(O)NR^{c1}R^{d1}.

In some embodiments, Cy^{b} is a substituted aryl or substituted heteroaryl ring according to Formula IG:

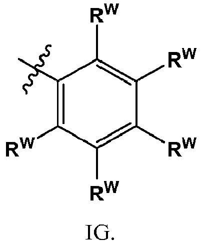

In some embodiments, L is a divalent moiety selected from C₁₋₆alkylene, (C₁₋₆alkylene)ₚ-O-( C₁₋₆alkylene)_{q}, (C₁₋₆alkylene)ₚ-S-(C₁₋₆alkylene)_{q}, (C₁₋₆alkylene)ₚ-NR^{c3}-(C₁₋₆alkylene)_{q}, (C₁₋₆ alkylene)ₚ-C(O)-(C₁₋₆alkylene)_{q}, (C₁₋₆ alkylene)ₚ-OC(O)-(C₁₋₆alkylene)_{q}, (C₁₋₆ alkylene)ₚ-C(O)NR^{c3}-(C₁₋₆ alkylene)_{q}, (C₁₋₆ alkylene)ₚ-OC(O)NR^{c3}-(C₁₋₆alkylene)_{q}, (C₁₋₆ alkylene)ₚ-SO-(C₁₋₆alkylene)_{q}, (C₁₋₆ alkylene)ₚ-SO₂-(C₁₋₆alkylene)_{q}, (C₁₋₆ alkylene)ₚ-SONR^{c3}-(C₁₋₆alkylene)_{q}, (C₁₋₆alkylene)ₚ-SO₂NR^{c3}-(C₁₋₆ alkylene)_{q}, (C₁₋₆ alkylene)ₚ-NR^{c3}CONR^{d3}-(C₁₋₆ alkylene)_{q}, (C₁₋₆alkylene)ₚ-NR^{c3}SONR^{d3}-(C₁₋₆ alkylene)_{q}, and (C₁₋₆ alkylene)ₚ-NR^{c3}SO₂NR^{d3}-(C₁₋₆alkylene)_{q}, wherein the C₁₋₆ alkylene is optionally substituted by 1, 2 or 3 substituents independently selected from C₁₋₄alkyl, C₂₋₄alkenyl, C₂₋₄alkynyl, C₁₋₄hydroxyalkyl, C₁₋₄cyanoalkyl, aryl, heteroaryl, cycloalkyl, heterocycloalkyl, halo, CN, NO₂, SCN, OH, C₁₋₄haloalkyl, halosulfanyl, C₁₋₄ alkoxy-C₁₋₄alkyl, C₁₋₄alkoxy, C₁₋₄ haloalkoxy, amino, C₁₋₄ alkylamino, and C₂₋₈dialkylamino.

In some embodiments, L is a divalent moiety selected from (C₁₋₆alkylene)ₚ-C(O)NR^{c3}-(C₁₋₆ alkylene)_{q} and (C₁₋₆alkylene)ₚ-NR^{c3}CONR^{d3}-(C₁₋₆alkylene)_{q}, wherein the C₁₋₆alkylene is optionally substituted by 1, 2 or 3 substituents independently selected from C₁₋₄ alkyl, C₂₋₄ alkenyl, C₂₋₄ alkynyl, C₁₋₄ hydroxyalkyl, C₁₋₄ cyanoalkyl, aryl, heteroaryl, cycloalkyl, heterocycloalkyl, halo, CN, NO₂, SCN, OH, C₁₋₄haloalkyl, halosulfanyl, C₁₋₄ alkoxy-C₁₋₄alkyl, C₁₋₄alkoxy, C₁₋₄ haloalkoxy, amino, C₁₋₄ alkylamino, and C₂₋₈ dialkylamino.

In some embodiments,L is a divalent moiety selected from (C₁₋₆alkylene)ₚ-C(O)NR^{c3}-(C₁₋₆ alkylene)_{q} and (C₁₋₆alkylene)ₚ-NR^{c3}CONR^{d3}-(C₁₋₆alkylene)_{q}.

In some embodiments, L is a divalent moiety selected from C(O)NH, C(O)NH-(C₁₋₆alkylene) and NHCONH.

In some embodiments, L is C(O)NH.

In some embodiments, L is C(O)NH-(C₁₋₆alkylene).

In some embodiments, L is NHCONH.

In some embodiments, L is C₁₋₆alkylene.

In some embodiments, L is (C₁₋₆alkylene)p-(C₃₋₁₀cycloalkylene)-(C₁₋₆ alkylene)_{q}.

In some embodiments, L is (C₁₋₆alkylene)p-(C₃₋₁₀heterocycloalkylene)-(C₁₋₆ alkylene)_{q}.

In some embodiments, L is (C₁₋₆ alkylene)ₚ-(C₆₋₁₀arylene)-(C₁₋₆ alkylene)_{q}.

In some embodiments, L is (C₁₋₆ alkylene)ₚ-(C₃₋₁₀heteroarylene)-(C₁₋₆ alkylene)_{q}.

In some embodiments, L is (C₁₋₆alkylene)ₚ-O-(C₁₋₆ alkylene)_{q}.

In some embodiments, L is (C₁₋₆alkylene)p-S-(C₁₋₆alkylene)_{q}.

In some embodiments, L is (C₁₋₆alkylene)p-NR^{c3}-(C₁₋₆alkylene)_{q}.

In some embodiments, L is (C₁₋₆alkylene)ₚ-C(O)-(C₁₋₆alkylene)_{q}.

In some embodiments, L is (C₁₋₆ alkylene)ₚ-OC(O)-(C₁₋₆alkylene)_{q}.

In some embodiments, L is (C₁₋₆alkylene)ₚ-C(O)NR^{c3}-(C₁₋₆alkylene)_{q}.

In some embodiments, L is (C₁₋₆alkylene)ₚ-OC(O)NR^{c3}-(C₁₋₆alkylene)_{q}.

In some embodiments, L is (C₁₋₆alkylene)ₚ-SO-(C₁₋₆alkylene)_{q}.

In some embodiments, L is (C₁₋₆alkylene)ₚ-SO₂-(C₁₋₆alkylene)_{q}.

In some embodiments, L is (C₁₋₆alkylene)ₚ-SONR^{c3}-(C₁₋₆alkylene)_{q}.

In some embodiments, L is (C₁₋₆alkylene)ₚ-SO₂NR^{c3}-(C₁₋₆alkylene)_{q}.

In some embodiments, L is (C₁₋₆alkylene)ₚ-NR^{c3}CONR^{d3}-(C₁₋₆alkylene)_{q}.

In some embodiments, L is (C₁₋₆alkylene)ₚ-NR^{c3}SONR^{d3}-(C₁₋₆alkylene)_{q}.

In some embodiments, L is (C₁₋₆alkylene)ₚ-NR^{c3}SO₂NR^{d3}-(C₁₋₆alkylene)_{q}.

In some embodiments, each of the C₁₋₆ alkylene, cycloalkylene, arylene, heterocycloalkylene, and heteroarylene in the above embodiments of L is optionally substituted by 1, 2 or 3 substituents independently selected from C₁₄alkyl, C₂₋₄ alkenyl, C₂₋₄ alkynyl, C₁₋₄ hydroxyalkyl, C₁₋₄ cyanoalkyl, aryl, heteroaryl, cycloalkyl, heterocycloalkyl, halo, CN, NO₂, SCN, OH, C₁₋₄haloalkyl, halosulfanyl, C₁₋₄alkoxy-C₁₋₄ alkyl, C₁₋₄alkoxy, C₁₋₄haloalkoxy, amino, C₁₋₄ alkylamino, and C₂₋₈dialkylamino.

In some embodiments p is 0.

In some embodiments p is 1.

In some embodiments q is 0.

In some embodiments q is 1.

In some embodiments p and q are both 0.

In some embodiments p and q are both 1.

In some embodiments, L is O.

In some embodiments, L is NR^{c3}CONR^{d3}.

In some embodiments, L is NR^{c3}SO₂NR^{d3}.

In some embodiments p is 0.

In some embodiments p is 1.

In some embodiments q is 0.

In some embodiments q is 1.

In some embodiments p and q are both 0.

In some embodiments p and q are both 1.

In some embodiments p and q, when added together, total 1.

In some embodiments, R¹ is selected from H, C₁₋₆ alkyl, or C(O)C₁₋₆ alkyl.

In some embodiments, R¹ is H.

In some embodiments, R¹ is C₁₋₆ alkyl.

In some embodiments, R¹ is C(O)C₁₋₆ alkyl.

In some embodiments, R², R³, R⁴, R⁵, R⁶, and R⁷ are independently selected from H, halo, C₁₋₆ alkyl, Cy, C₁₋₆ haloalkyl, halosulfanyl, CN, NO₂, OR^{a4}, SR^{a4}, C(O)R^{b4}, C(O)NR^{c4}R^{d4}, C(O)OR^{a4}, OC(O)R^{b4}, OC(O)NR^{c4}R^{d4}, NR^{c4}R^{d4}, NR^{c4}C(O)R^{ba}, NR^{c4}C(O)OR^{a4}, NR^{c4}C(O)NR^{c4}R^{d4}, NR^{c4}S(O)R^{ba}, NR^{c4}S(O)₂R^{b4}, S(O)R^{b4}, S(O)NR^{c4}R^{ba}, S(O)₂R^{b4}, and S(O)₂NR^{c4}R^{d4}; wherein the C₁₋₆alkyl is optionally substituted with 1, 2, 3, 4, or 5 substituents selected from CN, NO₂, Cy, Cy-(C₁₋₆ alkyl)-, OR^{a4}, SR^{a4}, C(O)R^{b4}, C(O)NR^{c4}R^{d4}, C(O)OR^{a4}, OC(O)R^{b4}, OC(O)NR^{c4}R^{d4}, NR^{c4}R^{d4}, NR^{c4}C(O)R^{b4}, NR^{c4}C(O)OR^{a4}, C(=NRⁱ)NR^{c4}R^{d4}, NR^{c4}C(=NRⁱ)NR^{c4}R^{d4}, S(O)R^{b4}, S(O)NR^{c4}R^{d4}, S(O)₂R^{b4}, and S(O)₂NR^{c4}R^{d4}.

In some embodiments, R², R³, R⁴, R⁵, R⁶, and R⁷ are independently selected from H, halo, C₁₋₆ alkyl, Cy, C₁₋₆ haloalkyl, halosulfanyl, CN, NO₂, OR^{a4}, SR^{a4}, C(O)R^{b4}, C(O)NR^{c4}R^{d4}, C(O)OR^{a4}, NR^{c4}R^{d4}, NR^{c4}C(O)R^{ba}, NR^{c2}C(O)OR^{a2}, NR^{c4}S(O)₂R^{b4}, S(O)R^{b4}, S(O)₂R^{b4}, and S(O)₂NR^{c4}R^{d4}; wherein the C₁₋₆ alkyl is optionally substituted with 1, 2, 3, 4, or 5 substituents selected from CN, NO₂, Cy, Cy-(C₁₋₆ alkyl)-, OR^{a4}, SR^{a4}, C(O)R^{b4}, C(O)NR^{c4}R^{d4}, C(O)OR^{a4}, OC(O)R^{b2}, OC(O)NR^{c4}R^{d4}, NR^{c4}R^{d4}, NR^{c4}C(O)R^{ba}, NR^{c4}C(O)OR^{a4}, C(=NRⁱ)NR^{c4}R^{d4}, NR^{c4}C(=NRⁱ)NR^{c4}R^{d4}, S(O)R^{b4}, S(O)NR^{c4}R^{d4}, S(O)₂R^{b4}, and S(O)₂NR^{c4}R^{d4}.

In some embodiments, R², R³, R⁴, R⁵, R⁶, and R⁷ are independently selected from H, halo, C₁₋₆ alkyl, Cy, C₁₋₆ haloalkyl, halosulfanyl, CN, NO₂, OR^{a4}, C(O)R^{b4}, C(O)NR^{c4}R^{d4}, C(O)OR^{a4}, NR^{c4}R^{d4}, NR^{c4}C(O)R^{b4}, NR^{c4}S(O)₂R^{b4}, S(O)₂R^{b4}, and S(O)₂NR^{c4}R^{d4}; wherein the C₁₋₆ alkyl is optionally substituted with 1, 2, 3, 4, or 5 substituents selected from CN, NO₂, Cy, Cy-(C₁₋₆ alkyl)-, OR^{a4}, SR^{a4}, C(O)R^{b4}, C(O)NR^{c4}R^{d4}, C(O)OR³⁴, OC(O)R^{b4}, OC(O)NR^{c4}R^{d4}, NR^{c4}R^{d4}, NR^{c4}C(O)R^{b4}, NR^{c2}C(O)OR^{a2}, C(=NRⁱ)NR^{c4}R^{d4}, NR^{c4}C(=NRⁱ)NR^{c4}R^{d4}, S(O)R^{ba}, S(O)NR^{c4}R^{d4}, S(O)₂R^{b4}, and S(O)₂NR^{c4}R^{d4}.

In some embodiments, R², R³, R⁴, R⁵, R⁶, and R⁷ are independently selected from H, halo, C₁₋₆ alkyl, Cy, C₁₋₆ haloalkyl, halosulfanyl, CN, NO₂, OC₁₋₆ alkyl, SC₁₋₆ alkyl, C(O)C₁₋₆ alkyl, NH₂, NR^{c4}C(O)C₁₋₆ alkyl, NR^{c4}S(O)₂C₁₋₆ alkyl, S(O)₂C₁₋₆ alkyl; wherein the C₁₋₆ alkyl is optionally substituted with 1, 2, 3, 4, or 5 substituents selected from CN, NO₂, Cy, Cy-(C₁₋₆ alkyl)-, OR^{a4}, SR^{a4}, C(O)R^{b4}, C(O)NR^{c4}R^{d4}, C(O)OR^{a4}, OC(O)R^{b4}, OC(O)NR^{c4}R^{d4}, NR^{c4}R^{d4}, NR^{c4}C(O)R^{b4}, NR^{c4}C(O)OR^{a4}, C(=NRⁱ)NR^{c4}R^{d4}, NR^{c4}C(=NRⁱ)NR^{c4}R^{d4}, S(O)R^{ba}, S(O)NR^{c4}R^{ba}, S(O)₂R^{b4}, and S(O)₂NR^{c4}R^{d4}.

In some embodiments, at least one of R², R³, R⁴, R⁵, R⁶, and R⁷ is H.

In some embodiments, at least two of R², R³, R⁴, R⁵, R⁶, and R⁷ is H.

In some embodiments, at least three of R², R³, R⁴, R⁵, R⁶, and R⁷ is H.

In some embodiments, at least four of R², R³, R⁴, R⁵, R⁶, and R⁷ is H.

In some embodiments, at least one of R¹ and R² is H.

In some embodiments, R¹ and R² are both H.

In some embodiments, Rⁱ is H.

In some embodiments, Rⁱ is CN.

In some embodiments, Rⁱ is C(O)NH₂.

In some embodiments, R¹ is C₁₋₆ alkyl.

In some embodiments, Rⁱ is H or C₁₋₆ alkyl.

In some embodiments, the agent is selected from compounds of Formula VIIIa: 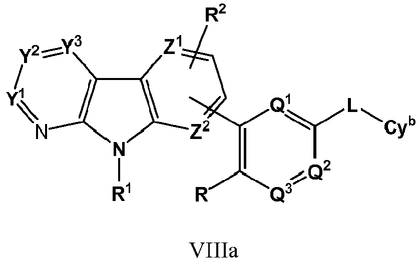 and pharmaceutically acceptable salts thereof.

In some embodiments, the agent is selected from compounds of Formula VIIIb: 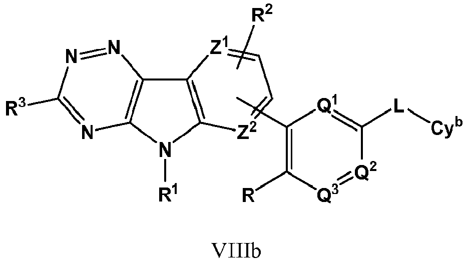 and pharmaceutically acceptable salts thereof.

In some embodiments, the agent is selected from compounds of Formula VIIc: 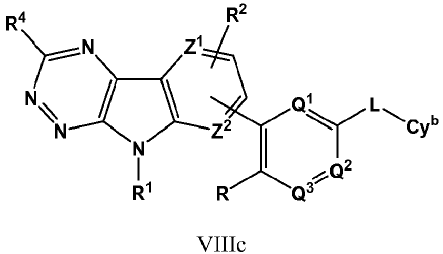 and pharmaceutically acceptable salts thereof.

In some embodiments, the agent is selected from compounds of Formula VIIId: 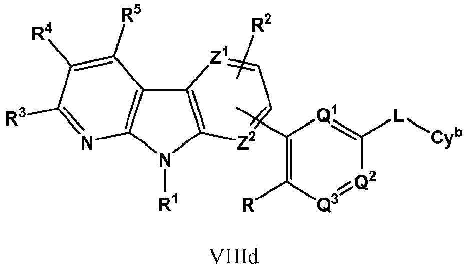 and pharmaceutically acceptable salts thereof.

In some embodiments, the agent is selected from compounds of Formula VIIIe: 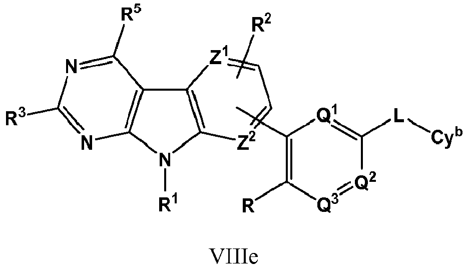 and pharmaceutically acceptable salts thereof.

In some embodiments, the agent is selected from compounds of Formula VIIIf: 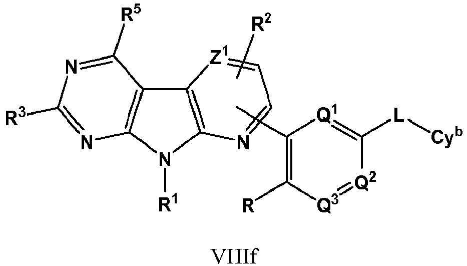 and pharmaceutically acceptable salts thereof.

In some embodiments, the agent is selected from compounds of Formula VTTTg: 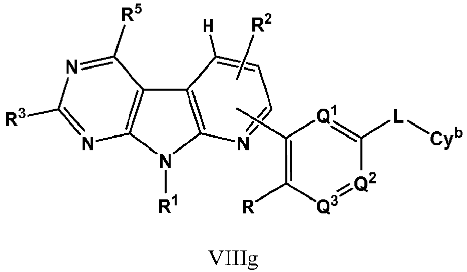 and pharmaceutically acceptable salts thereof.

In some embodiments, the agent is selected from compounds of Formula VIIIh:

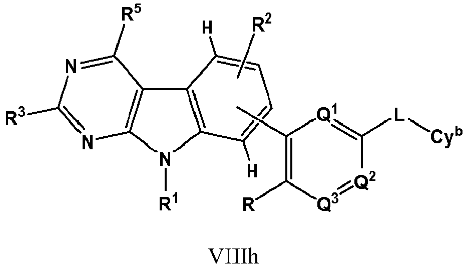

In some embodiments, the agent is selected from compounds of Formula VIIIi: 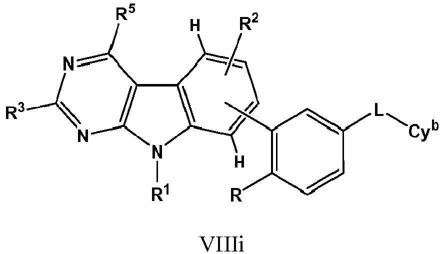 and pharmaceutically acceptable salts thereof.

In some embodiments, the agent is selected from compounds of Formula VIIIj: 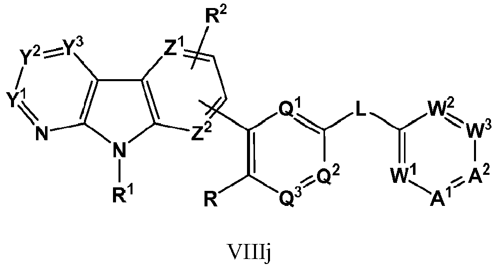 and pharmaceutically acceptable salts thereof.

In some embodiments, the agent is selected from compounds of Formula VIIIk: 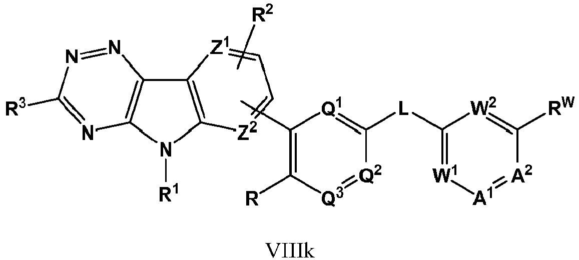 and pharmaceutically acceptable salts thereof.

In some embodiments, the agent is selected from compounds of Formula VIIII: 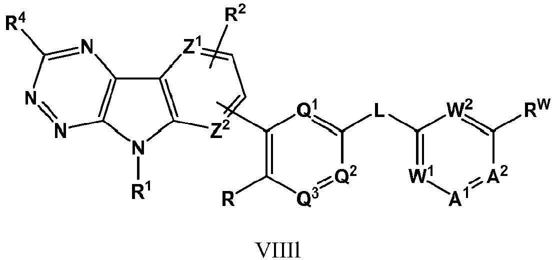 and pharmaceutically acceptable salts thereof.

In some embodiments, the agent is selected from compounds of Formula VIIIm: 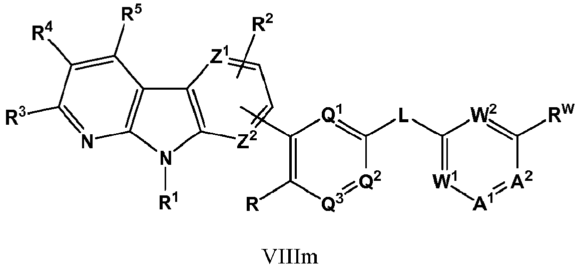 and pharmaceutically acceptable salts thereof.

In some embodiments, the agent is selected from compounds of Formula VIIIn: 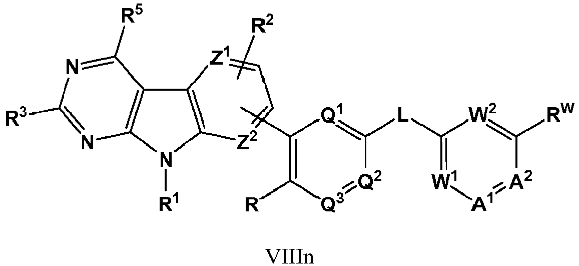 and pharmaceutically acceptable salts thereof.

In some embodiments, the agent is selected from compounds of Formula VIIIo: 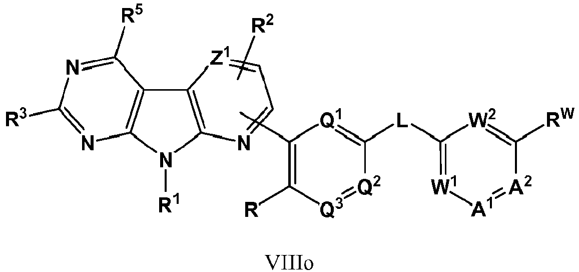 and pharmaceutically acceptable salts thereof.

In some embodiments, the agent is selected from compounds of Formula VIIIp: 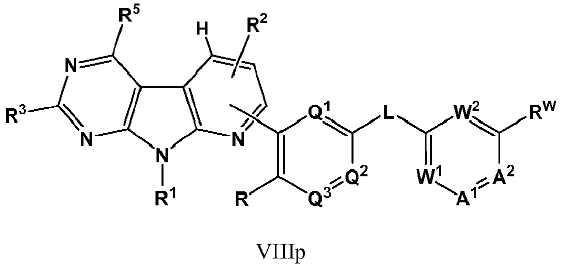 and pharmaceutically acceptable salts thereof.

In some embodiments, the agent is selected from compounds of Formula VIIIq: 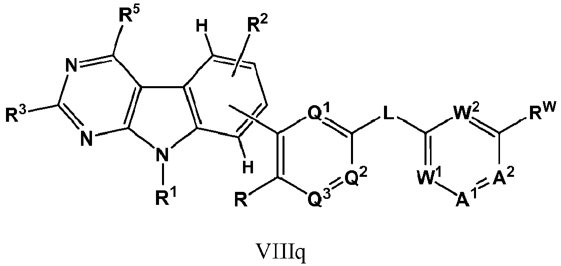 and pharmaceutically acceptable salts thereof.

In some embodiments, the agent is selected from compounds of Formula VIIIr: 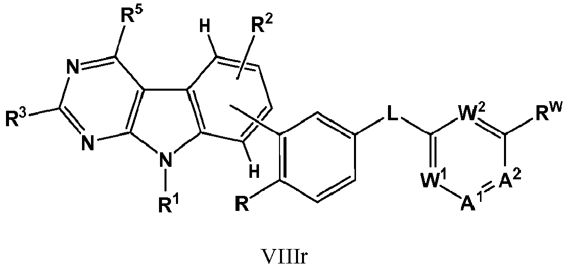 and pharmaceutically acceptable salts thereof.

In some embodiments, the agent is selected from compounds of Formula VIIIs: 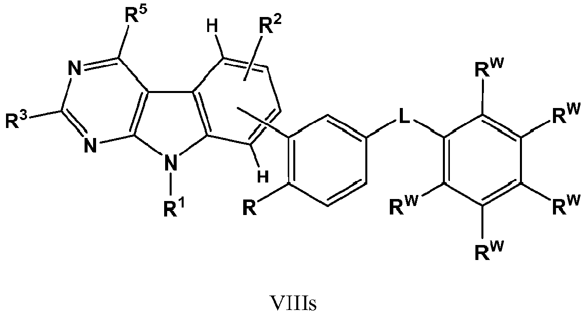 and pharmaceutically acceptable salts thereof.

In some embodiments, the agent is selected from compounds of Formula VIIIt: 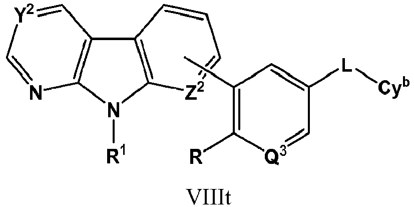 and pharmaceutically acceptable salts thereof.

In some embodiments, the agent is selected from compounds of Formula VIIIu or VIIIv: 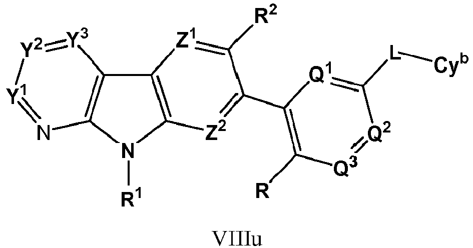 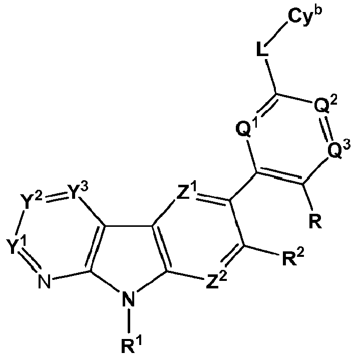 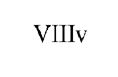 and pharmaceutically acceptable salts thereof.

In some embodiments, the agent is selected from:
*N*-[4-methyl-3-(9H-pyrimido[4,5-b]indol-7-yl)phenyl]-3-(trifluoromethyl)benzamide;
3-(4-Methyl-1H-imidazol-1-yl)-N-[4-methyl-3-(9H-pyrimido[4,5-b]indol-7-yl)phenyl]-5-(trifluoromethyl)benzamide;
3-fluoro-N-[4-methyl-3-(9H-pyrimido[4,5-b]indol-7-yl)phenyl]-5-(trifluoromethyl)-benzamide;
3-(4-formyl-1H-imidazol-1-yl)-N-[4-methyl-3-(9H-pyrimido[4,5-b]indol-7-yl)phenyl]-5-(trifluoromethyl)benzamide;
3-[4-(hydroxymethyl)-1H-imidazol-1-yl]-*N*-[4-methyl-3-(9H-pyrimido[4,5-b]indol-7-yl)-phenyl]-5-(trifluoromethyl)benzamide;
3-4-[(methylamino)methyl]-1H-imidazol-1-yl-*N*-[4-methyl-3-(9H-pyrimido[4,5-b]indol-7-yl)phenyl]-5-(trifluoromethyl)benzamide;
3-(4-methylpiperazin-1-yl)-*N*-[4-methyl-3-(9H-pyri mido[4,5-b]indol-7-yl)phenyl]-5-(trifluoromethyl)benzamide;
*N*-[4-methyl-3-(9H-pyrimido[4,5-b]indol-6-yl)phenyl]-3-(trifluoromethyl) benzamide;
4-fluoro-N-[4-methyl-3-(9H-pyrimido[4,5-b]indol-6-yl)phenyl]-3-(trifluoromethyl)-benzamide;
*N*-[4-methyl-3-(9H-pyrimido[4,5-b]indol-7-yl)phenyl]-*N*'-[3-(trifluoromethyl)phenyl]urea;
*N*-[4-methyl-3-(9H-pyrimido[4,5-b]indol-6-yl)phenyl]-*N*'-[3-(trifluoromethyl)phenyl]urea;
4-methyl-3-(9H-pyrimido[4,5-b]indol-6-yl)-*N*-[3-(trifluoromethyl)phenyl]benzamide;
4-chloro-3-(9H-pyrimido[4,5-b]indol-7-yl)-*N*-[3-(trifluoromethyl)phenyl]benzamide;
4-cyano-3-(9H-pyrimido[4,5-b]indol-7-yl)-*N*-[3-(trifluoromethyl)phenyl]benzamide;
2-fluoro-*N*-[4-methyl-3-(9H-pyrimido[4,5-b]indol-7-yl)phenyl]-3-(trifluoromethyl)-benzamide;
4-fluoro-*N*-[4-methyl-3-(9H-pyrimido[4,5-b]indol-7-yl)phenyl]-3-(trifluoromethyl)-benzamide;
2-fluoro-*N*-[4-methyl-3-(9H-pyrimido[4,5-b]indol-7-yl)phenyl]-5-(trifluoromethyl)-benzamide;
3-fluoro-*N*-[4-methyl-3-(9H-pyrimido[4,5-b]indol-6-yl)phenyl]benzamide;
*N*-[4-methyl-3-(9H-pyrimido[4,5-b]indol-7-yl)phenyl]-2,5-bis(trifluoromethyl)benzamide;
3-chloro-2-fluoro-*N*-[4-methyl-3-(9H-pyrimido[4,5-b]indol-7-yl)phenyl]-5-(trifluoromethyl)-benzamide;
*N*-[4-methyl-3-(9H-pyrimido[4,5-b]indol-7-yl)phenyl]-3,5-bis(trifluoromethyl)benzamide;
4-methoxy-*N*-[4-methyl-3-(9H-pyrimido[4,5-b]indol-7-yl)phenyl]-3-(trifluoromethyl)-benzamide;
3-methoxy-*N*-[4-methyl-3-(9H-pyrimido[4,5-b]indol-7-yl)phenyl]benzamide;
*N*-[4-methyl-3-(9H-pyrimido[4,5-b]indol-7-yl)phenyl]-2-(trifluoromethyl)benzamide;
*N*-[4-fluoro-3-(9H-pyrimido[4,5-b]indol-7-yl)phenyl]-3-(trifluoromethyl)benzamide;
*N*-[3-(9H-pyrimido[4,5-b]-indol-7-yl)phenyl]-3-(trifluoromethyl)benzamide;
3-chloro-*N*-[4-methyl-3-(9H-pyrimido[4,5-b]indol-7-yl)phenyl]benzamide;
*N*-[4-methyl-3-(9H-pyrimido[4,5-b]indol-7-yl)phenyl]-3-(pentafluoro-λ(6)-sulfanyl)-benzamide;
*N*-[4-chloro-3-(9H-pyrimido[4,5-b]indol-7-yl)phenyl]-3-(trifluoromethyl)benzamide,
*N*-[4-methyl-3-(9H-pyrimido[4,5-b]indol-7-yl)phenyl]-4-(trifluoromethyl)pyridine-2-carboxamide;
3-(1H-imidazol-1-yl)-*N*-[4-methyl-3-(9H-pyrimido[4,5-b]indol-7-yl)phenyl]-5-(trifluoromethyl)benzamide;
*N*-[4-methyl-3-(9H-pyrimido[4,5-b]indol-7-yl)phenyl]-3-(1H-1,2,4-triazol-1-yl)-5-(trifluoro-methyl)benzamide;
3-{4-[(dimethylamino)methyl]-1H-imidazol-1-yl}-*N*-[4-methyl-3-(9H-pyrimido[4,5-b]indol-7-yl)phenyl]-5-(trifluoromethyl)benzamide;
*N*-[4-methyl-3-(9H-pyrimido[4,5-b]indol-7-yl)phenyl]-3-morpholin-4-yl-5-(trifluoromethyl)-benzamide;
*N*-[4-methyl-3-(9H-pyrimido[4,5-b]indol-7-yl)phenyl]-3-piperazin-1-yl-5-(trifluoromethyl)-benzamide;
3-(4-hydroxypiperidin-1-yl)-*N*-[4-methyl-3-(9H-pyrimido[4,5-b]indol-7-yl)phenyl]-5-(trifluoromethyl)benzamide;
3-(3-hydroxypiperidin-1-yl)-*N*-[4-methyl-3-(9H-pyrimido[4,5-b]indol-7-yl)phenyl]-5-(trifluoromethyl)benzamide;
*N*-[4-methyl-3-(9H-pyrimido[4,5-b]indol-7-yl)phenyl]-3-[(2-morpholin-4-ylethyl)amino]-5-(trifluoromethyl)benzamide;
3-[4-(2-hydroxyethyl)piperazin-1-yl]-*N*-[4-methyl-3-(9H-pyrimido[4,5-b]indol-7-yl)phenyl]-5-(trifluoromethyl)benzamide;
3-{[3-(dimethylamino)propyl]amino}-*N*-[4-methyl-3-(9H-pyrimido[4,5-b]indol-7-yl)phenyl]-5-(trifluoromethyl)benzamide;
3-(3-hydroxypyrrolidin-1-yl)-*N*-[4-methyl-3-(9H-pyrimido[4,5-b]indol-7-yl)phenyl]-5-(trifluoromethyl)benzamide;
3-{[3-(1H-imidazol-1-yl)-propyl]amino}-*N*-[4-methyl-3-(9H-pyrimido[4,5-b]indol-7-yl)-phenyl]-5-(trifluoromethyl)benzamide;
3-(dimethylamino)-*N*-[4-methyl-3-(9H-pyrimido[4,5-b]indol-7-yl)phenyl]-5-(trifluoromethyl)benzamide;
3-[3-(dimethylamino)-pyrrolidin-1-yl]-*N*-[4-methyl-3-(9H-pyrimido[4,5-b]indol-7-yl)-phenyl]-5-(trifluoromethyl)benzamide;
3-{[2-(dimethylamino)ethyl]amino}-*N*-[4-methyl-3-(9H-pyrimido[4,5-b]indol-7-yl)phenyl]-5-(trifluoromethyl)benzamide;
4-fluoro-*N*-[4-methyl-3-(9H-pyrimido[4,5-b]indol-6-yl)phenyl]-3-(trifluoromethyl)-benzamide;
3-fluoro-*N*-[4-methyl-3-(9H-pyrimido[4,5-b]indol-6-yl)phenyl]-5-(trifluoromethyl)-benzamide;
3-fluoro-*N*-[4-methyl-3-(9H-pyrimido[4,5-b]indol-6-yl)phenyl]benzamide;
*N*-[4-methyl-3-(9H-pyrimido[4,5-b]indol-6-yl)phenyl]-2,5-bis(trifluoromethyl)benzamide;
3-chloro-2-fluoro-*N*-[4-methyl-3-(9H-pyrimido[4,5-b]indol-6-yl)phenyl]-5-(trifluoromethyl)-benzamide;
4-methyl-3-(9H-pyrimido-[4,5-b]indol-7-yl)-*N*-[3-(trifluoromethyl)phenyl]benzamide;
4-methyl-*N*-(3-methyl-phenyl)-3-(9H-pyrimido[4,5-b]indol-7-yl)benzamide;
4-methyl-3-(9H-pyrimido[4,5-b]indol-7-yl)-*N*-[3-(trifluoromethoxy)phenyl]benzamide;
*N*-(2,5-difluorobenzyl)-4-methyl-3-(9H-pyrimido[4,5-b]indol-7-yl)benzamide;
4-methyl-3-(9H-pyrimido[4,5-b]indol-7-yl)-*N*-[3-(trifluoromethyl)benzyl]benzamide;
4-methyl-*N*-(5-methyl-1,3-thiazol-2-yl)-3-(9H-pyrimido[4,5-b]indol-7-yl)benzamide;
4-chloro-3-(9H-pyrimido[4,5-b]indol-7-yl)-*N*-[3-(trifluoromethyl)phenyl]benzamide;
3-(9H-pyrimido[4,5-b]indol-7-yl)-*N*-[3-(trifluoromethyl)phenyl]benzamide;
4-methoxy-3-(9H-pyrimido[4,5-b]indol-7-yl)-*N*-[3-(trifluoromethyl)phenyl]benzamide;
4-methyl-3-(9H-pyrimido[4,5-b]indol-7-yl)-*N*-[5-(trifluoromethyl)-1,3,4-thiadiazol-2-yl]-benzamide;
4-methyl-*N*-[3-(4-methyl-1H-imidazol-1-yl)-5-(trifluoromethyl)phenyl]-3-(9H-pyrimido[4,5-b]indol-7-yl)benzamide;
4-methyl-*N*-[(1R)-1-phenylethyl]-3-(9H-pyrimido[4,5-b]indol-7-yl)benzamide;
4-methyl-*N*-[(1S)-1-phenylethyl]-3-(9H-pyrimido[4,5-b]indol-7-yl)benzamide;
4-methyl-*N*-[3-(pentafluoro-λ(6)-sulfanyl)phenyl]-3-(9H-pyrimido[4,5-b]indol-7-yl)-benzamide;
4-methyl-3-(9H-pyrimido[4,5-b]indol-7-yl)-*N*-[5-(trifluoromethyl)pyridin-3-yl]benzamide;
*N*-[3-(2-amino-9H-pyrimido[4,5-b]indol-7-yl)-4-methylphenyl]-3-(trifluoromethyl)-benzamide;
4-methyl-3-(9H-pyrido[2,3-b]indol-7-yl)-*N*-[3-(trifluoromethyl)phenyl]benzamide;
4-methyl-3-(9H-pyrido[3',2':4,5]pyrrolo[2,3-d]pyrimidin-7-yl)-*N*-[3-(trifluoromethyl)phenyl]-benzamide;
N-[6-methyl-5-(9H-pyrimido[4,5-b]indol-7-yl)pyridin-3-yl]-3-(trifluoromethyl)benzamide;
3-fluoro-N-[6-methyl-5-(9H-pyrimido[4,5-b]indol-7-yl)pyridin-3-yl]-5-(trifluoromethyl)benzamide;
3-(1H-imidazol-1-yl)-N-[6-methyl-5-(9H-pyrimido[4,5-b]indol-7-yl)pyridin-3-yl]-5-(trifluoromethyl)benzamide;
N-[6-methyl-5-(9H-pyrimido[4,5-b]indol-7-yl)pyridin-3-yl]-3-(1H-1,2,4-triazol-1-yl)-5-(trifluoromethyl)benzamide;
3-(4-formyl-1H-imidazol-1-yl)-N-[6-methyl-5-(9H-pyrimido[4,5-b]indol-7-yl)pyridin-3-yl]-5-(trifluoromethyl)benzamide;
3-[4-(hydroxymethyl)-1H-imidazol-1-yl]-N-[6-methyl-5-(9H-pyrimido[4,5-b]indol-7-yl)pyridin-3-yl]-5-(trifluoromethyl)benzamide;
3-[2-(dimethylamino)ethyl]amino-N-[6-methyl-5-(9H-pyrimido[4,5-b]indol-7-yl)pyridin-3-yl]-5-(trifluoromethyl)benzamide;
3-[3-(dimethylamino)propyl]amino-N-[6-methyl-5-(9H-pyrimido[4,5-b]indol-7-yl)pyridin-3-yl]-5-(trifluoromethyl)benzamide;
and pharmaccutically acceptable salts thereof.

In a ninth aspect, the agent is selected from compounds of Formula IX: 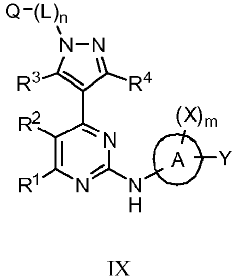 and pharmaceutically acceptable salts forms thereof, wherein:
Ring A is aryl or heteroaryl;
L is C₁₋₈ alkylene, C₂₋₈ alkenylene, C₂₋₈ alkynylene, (CR⁵R⁶)ₚ-(C₃₋₁₀cycloalkylene)-(CR⁵R⁶)_{q}, (CR⁵R⁶)ₚ-(arylene)-(CR⁵R⁶)_{q}, (CR⁵R⁶)ₚ-(C₁₋₁₀ heterocycloalkylene)-(CR⁵R⁶)_{q}, (CR⁵R⁶)ₚ-(heteroarylene)-(CR⁵R⁶)_{q}, (CR⁵R⁶)ₚO(CR⁵R⁶)_{q}, (CR⁵R⁶)ₚS(CR⁵R⁶)_{q}, (CR⁵R⁶)ₚC(O)(CR⁵R⁶)_{q}, (CR⁵R⁶)ₚC(O)NR^{c}(CR⁵R⁶)_{q}, (CR⁵R⁶)ₚC(O)O(CR⁵R⁶)_{q}, (CR⁵R⁶)ₚOC(O)(CR⁵R⁶)_{q}, (CR⁵R⁶)ₚOC(O)NR^{c}(CR⁵R⁶)_{q}, (CR⁵R⁶)ₚNR^{c}(CR⁵R⁶)_{q}, (CR⁵R⁶)ₚNR^{c}C(O)NR^{d}CR⁵R⁶)_{q}, (CR⁵R⁶)ₚS(O)(CR⁵R⁶)_{q}, (CR⁵R ⁶)ₚS(O)NR^{c}(CR⁵R⁶)_{q}, (CR⁵R⁶)ₚS(O)₂(CR⁵R⁶)_{q}, or (CR⁵R⁶)ₚS(O)₂NR^{c}(CR⁵R⁶)_{q}, wherein the C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from halo, C₁₋₆ alkyl, C₂₋₆ alkenyl, C₂₋₆ alkynyl, C₁₋₆ haloalkyl, CN, ORₐ, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, CN, OR^{a}, SR^{a}, C(O)R^{b}, C(O)NR^{c}R^{b}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR^{g})NR^{c}R^{d}, NR^{c}C(=NR^{g})NR^{c}R^{d}, S(O)R^{b}, S(O)NR^{c}R^{a}, S(O)₂R^{b}, NR^{c}S(O)₂R^{b}, and S(O)₂NR^{c}R^{d};
Q is H, Cy¹, halo, C₁₋₆ alkyl, C₂₋₆ alkenyl, C₂₋₆ alkynyl, C₁₋₆ haloalkyl, CN, NO₂, OR^{a1}, SR^{a1}, C(O)R^{b1}, C(O)NR^{c1}R^{a1}, C(O)OR^{a1}, OC(O)R^{b1}, OC(O)NR^{c4}R^{d4}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, or S(O)₂NR^{c1}R^{d1}, wherein the C₁₋₆ alkyl, C₂₋₆ alkenyl, C₂₋₆ or alkynyl, are optionally substituted by 1, 2, 3, 4 or 5 substituents independently selected from halo, C₁₋₆ alkyl, C₂₋₆ alkenyl, C₂₋₆ alkynyl, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c4}R^{d4}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1};
X is H, halo, C₁₋₆ alkyl, C₂₋₆ alkenyl, C₂₋₆ alkynyl, C₁₋₆ haloalkyl, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR^{g})NR^{c}R^{d}, NR^{c}C(=NR^{g})NR^{c}R^{d}, S(O)R^{b}, S(O)NR^{c}R^{d}, S(O)₂R^{b}, NR^{c}S(O)₂R^{b}, or S(O)₂NR^{c}R^{d};
Y is H, Cy², halo, C₁₋₆ alkyl, C₂₋₆ alkenyl, C₂₋₆ alkynyl, C₁₋₆ haloalkyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c4}R^{d4}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, or S(O)₂NR^{c1}R^{d1}, wherein the C₁₋₆ alkyl, C₂₋₆ alkenyl, C₂₋₆ alkynyl, or C₁₋₆ haloalkyl, is optionally substituted by 1, 2, 3, 4 or 5 substituents independently selected from halo, C₁₋₆ alkyl, C₂₋₆ alkenyl, C₂₋₆ alkynyl, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b2}, OC(O)NR^{c4}R^{d4}, NR^{c1}R^{a1}, NR^{1c}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1};
R¹, R², R³, R⁴, R⁵, and R⁶ are independently selected from H, halo, C₁₋₆ alkyl, C₂₋₆ alkenyl, C₂₋₆ alkynyl, C₁₋₆ haloalkyl, CN, (CH₂)ₘCN, NO₂, OR^{a}, (CH₂)ₘOR^{a}, SR^{a}, C(O)R^{b}, C(O)NR^{c}R^{d}, C(O)OR^{a}, NR^{c}R^{d}, (CH₂)ₘNR^{c}R^{d}, NR^{c}C(O)R^{b}, NR^{c}S(O)₂R^{b}, and S(O)₂NR^{c}R^{d};
Cy¹ and Cy² are independently selected from aryl, cycloalkyl, heteroaryl, and heterocycloalkyl, each optionally substituted by 1, 2, 3, 4, or 5 substituents independently selected from halo, C₁₋₆ alkyl, C₂₋₆ alkenyl, C₂₋₆ alkynyl, aryl, cycloalkyl, heteroaryl, and heterocycloalkyl, C₁₋₆ haloalkyl, halosulfanyl, CN, NO₂, OR^{a1}, SR^{a1}, C(O)R^{b1}, C(O)NR^{c1}R^{a1}, C(O)OR^{a1}, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(=NR⁸)NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1}, wherein the C₁₋₆ alkyl, C₂₋₆ alkenyl, C₂₋₆ alkynyl, aryl, cycloalkyl, heteroaryl, or heterocycloalkyl that is substituted on Cy¹ or Cy² is further optionally substituted by 1, 2, or 3 substituents independently selected from halo, C₁₋₆ haloalkyl, halosulfanyl, CN, NO₂, OR^{a1}, SR^{a1}, C(O)R^{b1}, C(O)NR^{c1}R^{a1}, C(O)OR^{a1}, OC(O)R^{b1}, OC(O)NR^{c4}R^{d4}, NR^{c1}R^{d1}, NR^{c1}C(O)R^{b1}, NR^{c1}C(O)NR^{c1}R^{d1}, NR^{c1}C(O)-OR^{a1}, C(=NR^{g})NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1};
R^{a}, R^{b}, R^{c}, and R^{d} are independently selected from H, C₁₋₆ alkyl, C₁₋₆ alkenyl, C₂₋₆ alkynyl, aryl, cycloalkyl, heteroaryl, heterocycloalkyl, arylalkyl, heteroarylalkyl, cycloalkylalkyl, and heterocycloalkylalkyl, wherein the C₁₋₆ alkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl is optionally substituted with 1, 2, 3, 4, or 5 substituents independently selected from OH, CN, amino, halo, C₁₋₆ alkyl, C₁₋₆ alkoxy, C₁₋₆ haloalkyl, and C₁₋₆ haloalkoxy;
or R^{c} and R^{d} together with the N atom to which they are attached form a 4-, 5-, 6- or 7-membered heterocycloalkyl group or heteroaryl group, each optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{a1}, R^{b1}, R^{c1}, and R^{d1} are independently selected from H, C₁₋₆ alkyl, C₂₋₆ alkenyl, C₂₋₆ alkynyl, aryl, cycloalkyl, heteroaryl, heterocycloalkyl, arylalkyl, heteroarylalkyl, cycloalkylalkyl, and heterocycloalkylalkyl, wherein the C₁₋₆ alkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl is optionally substituted with 1, 2, 3, 4, or 5 substituents independently selected from C₁₋₆ alkyl, halo, CN, OR^{a2}, SR^{a2}, C(O)R^{b2}, C(O)NR^{c2}R^{d2}, C(O)OR^{a2}, OC(O)R^{b2}, OC(O)NR^{c2}R^{d2}, NR^{c2}R^{d2}, NR^{c2}C(O)R^{b2}, NR^{c2}C(O)NR^{c2}R^{d2}, NR^{c2}C(O)OR^{a2}, C(=NR^{g})NR^{c2}R^{d2}, NR^{c2}C(=NR^{g})NR^{c2}R^{d2}, S(O)R^{b2}, S(O)NR^{c2}R^{d2}, S(O)₂R^{b2}, NR^{c2}S(O)₂R^{b2}, and S(O)₂NR^{c2}R^{d2};
or R^{c} and R^{d} together with the N atom to which they are attached form a 4-, 5-, 6- or 7-membered heterocycloalkyl group or heteroaryl group, each optionally substituted with 1, 2, or 3 substituents independently selected from C₁₋₆ alkyl, halo, CN, OR^{a2}, SR^{a2}, C(O)R^{b2}, C(O)NR^{c2}R^{d2}, C(O)OR^{a2}, OC(O)R^{b2}, OC(O)NR^{c2}R^{d2}, NR^{c2}R^{d2}, NR^{c2}C(O)R^{b2}, NR^{c2}C(O)NR^{c2}R^{d2}, NR^{c2}C(O)OR^{a2}, C(=NR^{g})NR^{c2}R^{d2}, NR^{c2}C(=NR^{g})NR^{c2}R^{d2}, S(O)R^{b2}, S(O)NR^{c2}R^{d2}, S(O)₂R^{b2}, NR^{c2}S(O)₂R^{b2}, and S(O)₂NR^{c2}R^{d2};
R^{a2}, R^{b2}, R^{c2}, and R^{d2} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, and heterocycloalkylalkyl, wherein the C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl, or heterocycloalkylalkyl is optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
or R^{c2} and R^{d2} together with the N atom to which they are attached form a 4-, 5-, 6- or 7-membered heterocycloalkyl group or heteroaryl group, each optionally substituted with 1, 2, or 3 substituents independently selected from OH, CN, amino, halo, C₁₋₆ alkyl, C₁₋₆ alkoxy, C₁₋₆ haloalkyl, and C₁₋₆ haloalkoxy;
R^{g} is H, CN, and NO₂;
m is 0, 1, 2, or 3;
n is 0 or 1;
p is 0, 1, 2, 3, 4, 5, or 6; and
q is 0, 1, 2, 3, 4, 5 or 6.

In some embodiments, A is aryl.

In some embodiments, A is phenyl.

In some embodiments, A is heteroaryl.

In some embodiments, A is pyrazolyl.

In some embodiments, A is pyridyl.

In some embodiments, L is C₁₋₈ alkylene, C₂₋₈ alkenylene, C₂₋₈ alkynylene, (CR⁵R⁶)ₚ-(C₃₋₁₀ cycloalkylene)-(CR⁵R⁶)_{q}, (CR⁵R⁶)ₚ-(arylene)-(CR⁵R⁶)_{q}, (CR⁵R⁶)ₚ-(C₁₋₁₀ heterocycloalkylene)-(CR⁵R⁶)_{q}, (CR⁵R⁶)ₚ-(heteroarylene)-(CR⁵R⁶)_{q}, (CR⁵R⁶)ₚO(CR⁵R⁶)_{q}, (CR⁵R⁶)ₚS(CR⁵R⁶)_{q}, wherein the C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from halo, C₁₋₆ alkyl, C₂₋₆ alkenyl, C₂₋₆ alkynyl, C₁₋₆ haloalkyl, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR^{g})NR^{c}R^{d}, NR^{c}C(=NR^{g})NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, L is C₁₋₈ alkylene, C₂₋₈ alkenylene, C₂₋₈ alkynylene, (CR⁵R⁶)ₚ-(C₃₋₁₀ cycloalkylene)-(CR⁵R⁶)_{q}, (CR⁵R⁶)ₚ-(arylene)-(CR⁵R⁶)_{q}, (CR⁵R⁶)ₚ-(C₁₋₁₀ heterocycloalkylene)-(CR⁵R⁶)_{q}, (CR⁵R⁶)p-(heteroarylene)-(CR⁵R⁶)q, wherein the C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from halo, C₁₋₆ alkyl, C₂₋₆ alkenyl, C₂₋₆ alkynyl, C₁₋₆ haloalkyl, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR^{g})NR^{c}R^{d}, NR^{c}C(=NR^{g})NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, L is C₁₋₈ alkylene, C₂₋₈ alkenylene, C₂₋₈ alkynylene, (CR⁵R⁶)ₚ-(C₃₋₁₀ cycloalkylene)-(CR⁵R⁶)_{q}, (CR⁵R⁶)ₚ-(C₁₋₁₀heterocycloalkylene)-(CR⁵R⁶)_{q}, wherein the C₁₋₈ alkylene, C₂₋₈ alkenylene, C₂₋₈ alkynylene, cycloalkylene, or heterocycloalkylene, is optionally substituted with 1, 2, or 3 substituents independently selected from halo, C₁₋₆ alkyl, C₂₋₆ alkenyl, C₂₋₆ alkynyl, C₁₋₆ haloalkyl, CN, OR^{a} SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR^{g})NR^{c}R^{d}, NR^{c}C(=NR^{g})NR^{c}R^{d}, S(O)R^{b}, S(O)NR^{c}R^{d}, S(O)₂R^{b}, NR^{c}S(O)₂R^{b}, and S(O)NR^{c}R^{d}.

In some embodiments, L is C₁₋₈ alkylene or (CR⁵R⁶)ₚ-(C₁₋₁₀ heterocycloalkylene)-(CR⁵R⁶)_{q}, wherein the C₁₋₈ alkylene or heterocycloalkylene, is optionally substituted with 1, 2, or 3 substituents independently selected from halo, C₁₋₆ alkyl, C₂₋₆ alkenyl, C₂₋₆ alkynyl, C₁₋₆ haloalkyl, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR^{g})NR^{c}R^{d}, NR^{c}C(=NR^{g})NR^{c}R^{d}, S(O)R^{b}, S(O)NR^{c}R^{d}, S(O)=R^{b}, NR^{c}S(O)₂R^{b}, and S(O)₂NR^{c}R^{d}.

In some embodiments, L is C₁₋₈ alkylene optionally substituted with 1, 2, or 3 substituents independently selected from halo, C₁₋₆ alkyl, C₂₋₆ alkenyl, C₂₋₆ alkynyl, C₁₋₆ haloalkyl, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR^{g})NR^{c}R^{d}, NR^{c}C(=NR^{g})NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, L is (CR⁵R⁶)ₚ-(C₁₋₁₀ heterocycloalkylene)-(CR⁵R⁶)_{q} optionally substituted with 1, 2, or 3 substituents independently selected from halo, C₁₋₆ alkyl, C₂₋₆ alkenyl, C₂₋₆ alkynyl, C₁₋₆ haloalkyl, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR^{g})NR^{c}R^{d}, NR^{c}C(=NR^{g})NR^{c}R^{d}, S(O)R^{b}, S(O)NR^{c}R^{d}, S(O)₂R^{b}, NR^{c}S(O)₂R^{b}, and S(O)₂NR^{c}R^{d}.

In some embodiments, L is (CR⁵R⁶)ₚC(O)(CR⁵R⁶)_{q}, (CR⁵R⁶)ₚC(O)NR^{c}(CR⁵R⁶)_{q}, (CR⁵R⁶)ₚC(O)O(CR⁵R⁶)_{q}, (CR⁵R⁶)ₚC(O)(CR⁵R⁶)_{q} (CR⁵R⁶)ₚOC(O)NR^{c}(CR⁵R⁶)_{q}, (CR⁵R⁶)ₚNR^{c}(CR⁵R⁶)_{q}, (CR⁵R⁶)ₚNR^{c}C(O)NR^{d}(CR⁵R⁶)_{q}, (CR⁵R⁶)ₚS(O)(CR⁵R⁶)_{q}, (CR⁵R⁶)pS(O)NR^{c}(CR⁵R⁶)_{q}, (CR⁵R⁶)ₚS(O)₂(CR⁵R⁶)_{q}, or (CR⁵R⁶)ₚS(O)₂NR^{c}(CR⁵R⁶)_{q}.

In some embodiments, Q is H, Cy¹, halo, C₁₋₆ alkyl, C₂₋₆ alkenyl, C₂₋₆ alkynyl, C₁₋₆ haloalkyl, CN, NO₂, OR^{a1}, or SR^{a1}, wherein the C₁₋₆ alkyl, C₂₋₆ alkenyl, C₂₋₆ or alkynyl, are optionally substituted by 1, 2, 3, 4 or 5 substituents independently selected from halo, C₁₋₆ alkyl, C₂₋₆ alkenyl, C₂₋₆ alkynyl, C₁₋₆ haloalkyl, halosulfanyl, CN, NO₂, OR^{a1}, S^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1}.

In some embodiments, Q is C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(-NR^{g})NR^{c1}R^{d1}, NR^{c1}C(-NR^{g})NR^{c1}R^{d1}, S(O)R^{b1}, S(O)NR^{c1}R^{d1}, S(O)₂R^{b1}, NR^{c1}S(O)2R^{b1}, or S(O)₂NR^{c1}R^{d1}.

In some embodiments, Q is Cy¹, C(O)R^{b1}, S(O)₂R^{b1}, or OR^{a1}.

In some embodiments, Cy¹ is aryl or cycloalkyl, each optionally substituted by 1, 2, 3, 4, or 5 substituents independently selected from halo, C₁₋₆ alkyl, C₂₋₆ alkenyl, C₂₋₆ alkynyl, aryl, cycloalkyl, heteroaryl, and heterocycloalkyl, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1.}

In some embodiments, Cy¹ is heteroaryl or heterocycloalkyl, each optionally substituted by 1, 2, 3, 4, or 5 substituents independently selected from halo, C₁₋₆ alkyl, C₂₋₆ alkenyl, C₂₋₆ alkynyl, aryl, cycloalkyl, heteroaryl, and heterocycloalkyl, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(=NR⁸)NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1}.

In some embodiments, R₁ is H.

In some embodiments, R₂ is H.

In some embodiments, R₂ is C₁₋₆ alkyl.

In some embodiments, R₂ is methyl.

In some embodiments, R₂ is C₁₋₆ alkoxy.

In some embodiments, R₂ is methoxy.

In some embodiments, wherein R₃ is H.

In some embodiments, R₄ is H.

In some embodiments, R₅ is H.

In some embodiments, R₆ is H.

In some embodiments, the agent is selected from compounds of Formula IXa, IXb, IXc, IXd, IXe, or IXf: 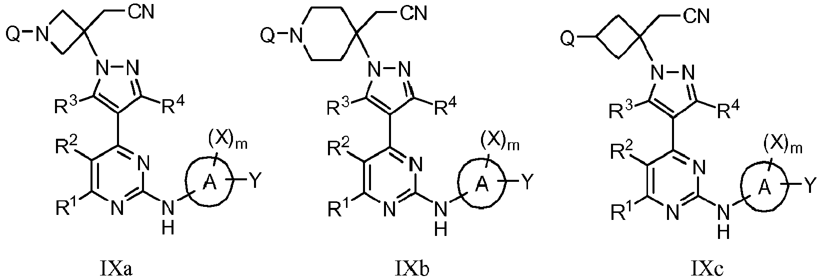 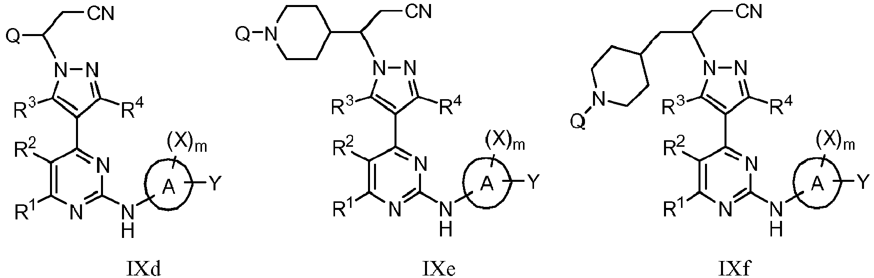 and pharmaceutically acceptable salts thereof.

In some embodiments, X is H, halo, C₁₋₆ alkyl, C₂₋₆ alkenyl, C₂₋₆ alkynyl, C₁₋₆ haloalkyl, CN, SR^{a}, C(O)R^{b}, C(O)OR^{a}, OC(O)R^{b}, OC(O)NR^{c}R^{d}, NR^{c}C(O)NR^{c}R^{d}, C(=NR⁹)NR^{c}R^{d}, NR^{c}C(=NR^{g})NR^{c}R^{d}, S(O)R^{b}, S(O)NR^{c}R^{d}, or S(O)₂R^{b},

In some embodiments, X is NO₂, OR^{a}, C(O)NR^{c}R^{d}, NR^{c}R^{d}, NR^{c}C(O)R^{b}, NR^{c}C(O)OR^{a} NR^{c}S(O)₂R^{b}, or S(O)₂NR^{c}R^{d}.

In some embodiments, X is OCH₃, OC₆H₅, NO₂, NH₂, or N(CH₂CH₃)₂

In some embodiments, X is H.

In some embodiments, Y is C₁₋₆ alkyl, C₂₋₆ alkenyl, C₁₋₆ alkynyl, or C₁₋₆ haloalkyl, wherein the C₁₋₆ alkyl, C₂₋₆ alkenyl, or C₂₋₆ alkynyl is optionally substituted by 1, 2, 3, 4, or 5 substituents independently selected from halo, C₁₋₆ alkyl, C₂₋₆ alkenyl, C₂₋₆ alkynyl, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1}.

In some embodiments, Y is H, Cy², halo, CN, NO₂, OR^{a1}, SR^{a1}, C(O)R^{b1}, C(O)NR^{c1}R^{d1}, C(O)OR³¹, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(=NR^{g})NR^{c1}R^{d1}, S(O)R^{b1}, S(O)NR^{c1}R^{d1}, S(O)₂R^{b1}, NR^{c1}S(O)₂R^{b1}, or S(O)₂NR^{c1}R^{d1}.

In some embodiments, Y is H.

In some embodiments, Cy² is aryl, or cycloalkyl, each optionally substituted by 1, 2, 3, 4, or 5 substituents independently selected from halo, C₁₋₆ alkyl, C₂₋₆ alkenyl, C₂₋₆ alkynyl, aryl, cycloalkyl, heteroaryl, and heterocycloalkyl, C₁₋₆ haloalkyl, halosulfanyl, CN, NO₂, OR^{a1}, SR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{a1}C(O)NR^{c1}R^{d1}, NR^{c1}C(O)OR^{a1}, C(=NR^{g})NR^{c1}R^{d1}, NR^{c1}C(-NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1}.

In some embodiments, Cy² is heteroaryl, or heterocycloalkyl, each optionally substituted by 1, 2, 3, 4, or 5 substituents independently selected from halo, C₁₋₆ alkyl, C₂₋₆ alkenyl, C₂₋₆ alkynyl, aryl, cycloalkyl, heteroaryl, and heterocycloalkyl, C₁₋₆ haloalkyl, halosulfanyl, CN, NO₂, OR^{a1}, SR^{a1}, C(O)R^{b1}, C(O)NR^{c1}R^{d1}, C(O)OR³¹, OC(O)R^{b1}, OC(O)NR^{c1}R^{d1}, NR^{c1}R^{d1}, NR^{c1}C(O)R^{b1}, NR^{c1}C(O)NR^{c1}R^{d1}, NR^{c1}C(O)OR^{a1}, C(=NR^{g})NR^{c1}R^{d1}, NR^{c1}C(-NR^{g})NR^{c1}R^{d1}, S(O)R^{b1}, S(O)NR^{c1}R^{d1}, S(O)₂R^{b1}, NR^{c1}S(O)₂R^{b1}, and S(O)₂NR^{c1}R^{d1}.

In some embodiments, the agent is selected from:
3-(4-(2-(4-(1H-imidazol-1-yl)phenylamino)pyrimidin-4-yl)-1H-pyrazol-1-yl)-4-(1-(2,4-difluorobenzoyl)piperidin-4-yl)butanenitrile;
4-(1-(2,4-difluorobenzoyl)piperidin-4-yl)-3-(4-(2-(4-(piperazin-1-yl)phenylamino)pyrimidin-4-yl)-1H-pyrazol-1-yl)butanenitrile;
4-(1-(2,4-difluorobenzoyl)piperidin-4-yl)-3-(4-(2-(4-methoxyphenylamino)pyrimidin-4-yl)-1H-pyrazol-1-yl)butanenitrile;
4-(1-(2,4-difluorobenzoyl)piperidin-4-yl)-3-(4-(2-(phenylamino)pyrimidin-4-yl)-1H-pyrazol-1-yl)butanenitrile;
4-(1-(2,4-difluorobenzoyl)piperidin-4-yl)-3-(4-(2-(4-morpholinophenylamino)pyrimidin-4-yl)-1H-pyrazol-1-yl)butanenitrile;
3-(4-(2-(4-(1H-pyrazol-1-yl)phenylamino)pyrimidin-4-yl)-1H-pyrazol-1-yl)-4-(1-(2,4-difluorobenzoyl)piperidin-4-yl)butanenitrile;
4-(1-(2,4-difluorobenzoyl)piperidin-4-yl)-3-(4-(2-(3-(oxazol-5-yl)phenylamino)pyrimidin-4-yl)-1H-pyrazol-1-yl)butanenitrile;
4-(1-(2,4-difluorobenzoyl)piperidin-4-yl)-3-(4-(2-(1-methyl-1H-pyrazol-3-ylamino)pyrimidin-4-yl)-1H-pyrazol-1-yl)butanenitrile;
4-(1-(2,4-difluorobenzoyl)piperidin-4-yl)-3-(4-(2-(4-phenoxyphenylamino)pyrimidin-4-yl)-1H-pyrazol-1-yl)butanenitrile;
2-(4-(4-(2-(4-(1H-pyrazol-1-yl)phenylamino)pyrimidin-4-yl)-1H-pyrazol-1-yl)-1-(isoxazole-5-carbonyl)piperidin-4-yl)acetonitrile;
2-(1-(isoxazole-5-carbonyl)-4-(4-(2-(3-(oxazol-5-yl)phenylamino)pyrimidin-4-yl)-1H-pyrazol-1-yl)piperidin-4-yl)acetonitrile;
2-(4-(4-(2-(3-(1H-tetrazol-5-yl)phenylamino)pyrimidin-4-yl)-1H-pyrazol-1-yl)-1-(isoxazole-5-carbonyl)piperidin-4-yl)acetonitrile;
2-(1-(isoxazolc-5-carbonyl)-4-(4-(2-(4-(morpholinosulfonyl)phenylamino)pyrimidin-4-yl)-1H-pyrazol-1-yl)piperidin-4-yl)acetonitrile;
2-(1-(isoxazole-5-carbonyl)-4-(4-(2-(6-methoxypyridin-3-ylamino)pyrimidin-4-yl)-1H-pyrazol-1-yl)piperidin-4-yl)acetonitrile;
2-(3-(4-(2-(4-(1H-pyrazol-1-yl)phenylamino)pyrimidin-4-yl)-1H-pyrazol-1-yl)-1-(cyclopropylsulfonyl)azetidin-3-yl)acetonitrile;
2-(1-(cyclopropylsulfonyl)-3-(4-(2-(3-(oxazol-5-yl)phenylamino)pyrimidin-4-yl)-1H-pyrazol-1-yl)azetidin-3-yl)acetonitrile;
*N*-(4-(4-(1-(3-(cyanomethyl)-1-(cyclopropylsulfonyl)azetidin-3-yl)-1H-pyrazol-4-yl)pyrimidin-2-ylamino)phenyl)acetamide;
2-(1-(cyclopropylsulfonyl)-3-(4-(2-(3-(2-methylpyrimidin-4-yl)phenylamino)pyrimidin-4-yl)-1H-pyrazol-1-yl)azetidin-3-yl)acetonitrile;
2-(1-(cyclopropylsulfonyl)-3-(4-(2-(4-(oxazol-5-yl)phenylamino)pyrimidin-4-yl)-1H-pyrazol-1-yl)azetidin-3-yl)acetonitrile;
3-(4-(2-(4-morpholinophenylamino)pyrimidin-4-yl)-1H-pyrazol-1-yl)-3-(piperidin-4-yl)propanenitrile;
3-(1-(5-fluoropyrimidin-2-yl)piperidin-4-yl)-3-(4-(2-(4-morpholinophenylamino)pyrimidin-4-yl)-1H-pyrazol-1-yl)propanenitrile;
3-(1-(methylsulfonyl)piperidin-4-yl)-3-(4-(2-(4-morpholinophenylamino)pyrimidin-4-yl)-1H-pyrazol-1-yl)propanenitrile;
3-(4-(2-(4-morpholinophenylamino)pyrimidin-4-yl)-1H-pyrazol-1-yl)-3-(1-(phenylsulfonyl)piperidin-4-yl)propanenitrile;
3-(1-acetylpiperidin-4-yl)-3-(4-(2-(4-morpholinophenylamino)pyrimidin-4-yl)-1H-pyrazol-1-yl)propanenitrile;
3-(1-benzoylpiperidin-4-yl)-3-(4-(2-(4-morpholinophenylamino)pyrimidin-4-yl)-1H-pyrazol-1-yl)propanenitrile;
2-(4-(4-(2-(4-(1H-pyrazol-1-yl)phenylamino)pyrimidin-4-yl)-1H-pyrazol-1-yl)-1-(cyclopropylsulfonyl)piperidin-4-yl)acetonitrile;
2-(1-(cyclopropylsulfonyl)-4-(4-(2-(4-morpholinophenylamino)pyrimidin-4-yl)-1H-pyrazol-1-yl)piperidin-4-yl)acetonitrile;
4-(4-(1-(4-(cyanomethyl)-1-(cyclopropylsulfonyl)piperidin-4-yl)-1H-pyrazol-4-yl)pyrimidin-2-ylamino)benzamide;
4-(4-(1-(4-(cyanomethyl)-1-(cyclopropylsulfonyl)piperidin-4-yl)-1H-pyrazol-4-yl)pyrimidin-2-ylamino)-*N*-(2-hydroxyethyl)benzamide;
4-(4-(1-(4-(cyanomethyl)-1-(cyclopropylsulfonyl)piperidin-4-yl)-1H-pyrazol-4-yl)pyrimidin-2-ylamino)-*N,N*-dimethylbenzamide;
4-(4-(1-(2-cyano-1-cyclopentylethyl)-1H-pyrazol-4-yl)pyrimidin-2-ylamino)benzamide;
3-(4-(2-(4-(1H-pyrazol-1-yl)phenylamino)pyrimidin-4-yl)-1H-pyrazol-1-yl)-3-cyclopentylpropanenitrile;
3-cyclopentyl-3-(4-(2-(4-morpholinophenylamino)pyrimidin-4-yl)-1H-pyrazol-1-yl)propanenitrile;
3-cyclopentyl-3-(4-(2-(phenylamino)pyrimidin-4-yl)-1H-pyrazol-1-yl)propanenitrile;
3-cyclopentyl-3-(4-(2-(3-(oxazol-5-yl)phenylamino)pyrimidin-4-yl)-1H-pyrazol-1-yl)propanenitrile;
3-cyclopentyl-3-(4-(2-(4-methoxyphenylamino)pyrimidin-4-yl)-1H-pyrazol-1-yl)propanenitrile;
*N*-(4-(4-(1-(2-cyano-1-cyclopentylethyl)-1H-pyrazol-4-yl)pyrimidin-2-ylamino)phenyl)acetamide;
4-(4-(1-(2-cyano-1-cyclopentylethyl)-1H-pyrazol-4-yl)pyrimidin-2-ylamino)-N,N-dimethylbenzamide;
3-cyclopentyl-3-(4-(2-(4-(piperazin-1-yl)phenylamino)pyrimidin-4-yl)-1H-pyrazol-1-yl)propanenitrile;
4-(1-(ethylsulfonyl)piperidin-4-yl)-3-(4-(2-(4-morpholinophenylamino)pyrimidin-4-yl)-1H-pyrazol-1-yl)butanenitrile;
3-(4-(2-(4-(1H-pyrazol-1-yl)phenylamino)pyrimidin-4-yl)-1H-pyrazol-1-yl)-4-(1-(ethylsulfonyl)piperidin-4-yl)butanenitrile;
4-(1-(ethylsulfonyl)piperidin-4-yl)-3-(4-(2-(phenylamino)pyrimidin-4-yl)-1H-pyrazol-1-yl)butanenitrile;
*N*-(4-(4-(1-(1-cyano-3-(1-(1-methyl-1H-pyrazol-3-ylsulfonyl)piperidin-4-yl)propan-2-yl)-lH-pyrazol-4-yl)pyrimidin-2-ylamino)phenyl)acetamide;
4-(4-(1-(1-cyano-3-(1-(ethylsulfonyl)piperidin-4-yl)propan-2-yl)-1H-pyrazol-4-yl)pyrimidin-2-ylamino)-*N,N*-dimethylbenzamide;
4-(4-(1-(1-cyano-3-(1-(-methyl-1H-pyrazol-3-ylsulfonyl)piperidin-4-yl)propan-2-yl)-1H-pyrazol-4-yl)pyrimidin-2-ylamino)benzamide;
4-(1-(ethylsulfonyl)piperidin-4-yl)-3-(4-(5-methyl-2-(4-morpholinophenylamino)pyrimidin-4-yl)-1H-pyrazol-1-yl)butanenitrile;
3-(4-(2-(4-(1H-pyrazol-1-yl)phenylamino)-5-methylpyrimidin-4-yl)-1H-pyrazol-1-yl)-4-(1-(ethylsulfonyl)piperidin-4-yl)butanenitrile;
4-(1-(ethylsulfonyl)piperidin-4-yl)-3-(4-(5-methyl-2-(phenylamino)pyrimidin-4-yl)-1H-pyrazol-1-yl)butanenitrile;
N-(4-(4-(1-(1-cyano-3-(1-(1-methyl-1H-pyrazol-3-ylsulfonyl)piperidin-4-yl)propan-2-yl)-1H-pyrazol-4-yl)-5-methylpyrimidin-2-ylamino)phenyl)acetamide;
4-(4-(1-(1-cyano-3-(1-(ethylsulfonyl)piperidin-4-yl)propan-2-yl)-1H-pyrazol-4-yl)-5-methylpyrimidin-2-ylamino)-N,N dimethylbenzamide;
4-(4-(1-(1-cyano-3-(1-(-methyl-1H-pyrazol-3-ylsulfonyl)piperidin-4-yl)propan-2-yl)-1H-pyrazol-4-yl)-5-methylpyrimidin-2-ylamino)benzamide;
3-cyclopentyl-3-(4-(2-(4-(4-(methylsulfonyl)piperazin-1-yl)phenylamino)pyrimidin-4-yl)-1H-pyrazol-1-yl)propanenitrile;
4-(1-(1-methyl-1H-pyrazol-3-ylsulfonyl)pipcridin-4-yl)-3-(4-(2-(4-morpholinophenylamino)pyrimidin-4-yl)-1H-pyrazol-1-yl)butanenitrile;
3-(4-(2-(4-(1H-pyrazol-1-yl)phenylamino)pyrimidin-4-yl)-1H-pyrazol-1-yl)-4-(1-(1-methyl-1H-pyrazol-3-ylsulfonyl)piperidin-4-yl)butanenitrile;
4-(1-(1-methyl-1H-pyrazol-3-ylsulfonyl)piperidin-4-yl)-3-(4-(2-(phenylamino)pyrimidin-4-yl)-1H-pyrazol-1-yl)butanenitrile;
4-(4-(1-(1-cyano-3-(1-(-methyl-1H-pyrazol-3-ylsulfonyl)piperidin-4-yl)propan-2-yl)-1H-pyrazol-4-yl)pyrimidin-2-ylamino)*-N,N*-dimethylbenzamide:
4-(4-(1-(1-cyano-3-(1-(2,4-difluorobenzoyl)piperidin-4-yl)propan-2-yl)-1H-pyrazol-4-yl)pyrimidin-2-ylamino)-*N,N*-dimethylbenzamide;
N-(4-(4-(1-(1-cyano-3-(1-(2,4-difluorobenzoyl)piperidin-4-yl)propan-2-yl)-1H-pyrazol-4-yl)pyrimidin-2-ylamino)phenyl)acetamide;
4-(1-(1-methyl-1H-pyrazol-3-ylsulfonyl)piperidin-4-yl)-3-(4-(5-methyl-2-(4-morpholinophenylamino)pyrimidin-4-yl)-1H-pyrazol-1-yl)butanenitrile;
4-(1-(2,4-difluorobenzoyl)piperidin-4-yl)-3-(4-(5-methyl-2-(4-morpholinophenylamino)pyrimidin-4-yl)-1H-pyrazol-1-yl)butanenitrile;
3-(4-(2-(4-(1H-pyrazol-1-yl)phenylamino)-5-methylpyrimidin-4-yl)-1H-pyrazol-1-yl)-4-(1-(1-methyl-1 H-pyrazol-3-ylsulfonyl)piperidin-4-yl)butanenitrile;
3-(4-(2-(4-(1H-pyrazol-1-yl)phenylamino)-5-methylpyrimidin-4-yl)-1H-pyrazol-1-yl)-4-(1-(2,4-difluorobenzoyl)piperidin-4-yl)butanenitrile;
4-(1-(1-methyl-1H-pyrazol-3-ylsulfonyl)piperidin-4-yl)-3-(4-(5-methyl-2-(phenylamino)pyrimidin-4-yl)-1H-pyrazol-1-yl)butanenitrile;
4-(1-(2,4-difluorobenzoyl)piperidin-4-yl)-3-(4-(5-methyl-2-(phenylamino)pyrimidin-4-yl)-1H-pyrazol-1-yl)butanenitrile;
4-(4-(1-(1-cyano-3-(1-(-methyl-1H-pyrazol-3-ylsulfonyl)piperidin-4-yl)propan-2-yl)-1H-pyrazol-4-yl)-5-methylpyrimidin-2-ylamino)-*N*,*N*-dimethylbenzamide;
4-(4-(1-(1-cyano-3-(1-(2,4-difluorobenzoyl)piperidin-4-yl)propan-2-yl)-1H-pyrazol-4-yl)-5-methylpyrimidin-2-ylamino)-*N,N*-dimethylbenzamide;
*N*-(4-(4-(1-(1-cyano-3-(1-(2,4-difluorobenzoyl)piperidin-4-yl)propan-2-yl)-1H-pyrazol-4-yl)-5-methylpyrimidin-2-ylamino)phenyl)acetamide;
4-(4-(1-(2-cyano-1-cyclopentylethyl)-1H-pyrazol-4-yl)-5-methylpyrimidin-2-ylamino)benzamide;
3-(4-(2-(4-(1H-pyrazol-1-yl)phenylamino)-5-methylpyrimidin-4-yl)-1H-pyrazol-1-yl)-3-cyclopentylpropanenitrile;
3-cyclopentyl-3-(4-(5-methyl-2-(4-morpholinophenylamino)pyrimidin-4-yl)-1H-pyrazol-1-yl)propanenitrile;
3-cyclopentyl-3-(4-(5-methyl-2-(phenylamino)pyrimidin-4-yl)-1H-pyrazol-1-yl)propanenitrile;
3-cyclopentyl-3-(4-(5-methyl-2-(4-(oxazol-5-yl)phenylamino)pyrimidin-4-yl)-1H-pyrazol-1-yl)propancnitrilc;
3-cyclopentyl-3-(4-(2-(4-methoxyphenylamino)-5-methylpyrimidin-4-yl)-1H-pyrazol-1-yl)propanenitrile;
3-cyclopentyl-3-(4-(5-methyl-2-(4-(piperazin-1-yl)phenylamino)pyrimidin-4-yl)-1H-pyrazol-1-yl)propanenitrile;
3-cyclopentyl-3-(4-(2-(4-(diethylamino)phenylamino)-5-methylpyrimidin-4-yl)-1H-pyrazol-1-yl)propanenitrile;
3-cyclopentyl-3-(4-(2-(4-(ethyl(3-hydroxypropyl)amino)phenylamino)-5-methylpyrimidin-4-yl)-1H-pyrazol-1-yl)propanenitrile;
4-( 4-(1-(2-cyano-1-cyclopentylethyl)-1H-pyrazol-4-yl)-5-methylpyrimidin-2-ylamino)benzoic acid;
3-cyclopentyl-3-(4-(5-methyl-2-(4-nitrophenylamino)pyrimidin-4-yl)-1H-pyrazol-1-yl)propanenitrile;
4-(4-(1-(2-cyano-1-cyclopentylethyl)-1H-pyrazol-4-yl)-5-methylpyrimidin-2-ylamino)-*N*-(2-hydroxyethyl)benzamide;
3-cyclopentyl-3-(4-(5-methyl-2-(3-(oxazol-5-yl)phenylamino)pyrimidin-4-yl)-1H-pyrazol-1-yl)propanenitrile;
3-(4-(2-(4-aminophenylamino)-5-methylpyrimidin-4-yl)-1H-pyrazol-1-yl)-3-cyclopentylpropanenitrile;
4-(4-(1-(2-cyano-1-cyclopentylethyl)-1H-pyrazol-4-yl)-5-methylpyrimidin-2-ylamino)-N-methylbenzamide;
4-(4-(1-(2-cyano-1-cyclopentylethyl)-1H-pyrazol-4-yl)-5-methylpyrimidin-2-ylamino)-*N*-(1-methoxypropan-2-yl)benzamide;
3-cyclopentyl-3-(4-(2-(4-(4-hydroxypiperidine-1-carbonyl)phenylamino)-5-methylpyrimidin-4-yl)-1H-pyrazol-1-yl)propanenitrile;
*N*-(4-(4-(1-(2-cyano-1-cyclopentylethyl)-1H-pyrazol-4-yl)-5-methylpyrimidin-2-ylamino)phenyl)methanesulfonamide;
Methyl 4-(4-(1-(2-cyano-1-cyclopentylethyl)-1H-pyrazol-4-yl)-5-methylpyrimidin-2-ylamino)phenylcarbamate;
*N*-(4-(4-(1-(2-cyano-1-cyclopentylethyl)-1H-pyrazol-4-yl)-5-methylpyrimidin-2-ylamino)phenyl)-2-(pyrrolidin-1-yl)acetamide;
3-(4-(2-(4-(3-oxomorpholino)phenylamino)pyrimidin-4-yl)-1H-pyrazol-1-yl)-4-(piperidin-4-yl)butanenitrile;
2-(1-(cyclopropylsulfonyl)-3-(4-(2-(4-morpholinophenylamino)pyrimidin-4-yl)-1H-pyrazol-1-yl)azetidin-3-yl)acetonitrile;
2-(1-(isoxazole-5-carbonyl)-4-(4-(2-(4-morpholinophenylamino)pyrimidin-4-yl)-1H-pyrazol-1-yl)piperidin-4-yl)acetonitrile;
4-(1-(methylsulfonyl)piperidin-4-yl)-3-(4-(2-(4-(3-oxomorpholino)phenylamino)pyrimidin-4-yl)-1H-pyrazol-1-yl)butanenitrile;
4-(1-(ethylsulfonyl)piperidin-4-yl)-3-(4-(2-(4-(3-oxomorpholino)phenylamino)pyrimidin-4-yl)-1H-pyrazol-1-yl)butanenitrile;
4-(1-(cyclopropylsulfonyl)piperidin-4-yl)-3-(4-(2-(4-(3-oxomorpholino)phenylamino)pyrimidin-4-yl)-1H-pyrazol-1-yl)butanenitrile;
3-cyclopentyl-3-(4-(2-(4-(3-oxomorpholino)phenylamino)pyrimidin-4-yl)-1H-pyrazol-1-yl)propanenitrile;
3-cyclopentyl-3-(4-(2-(3-(2-methylpyrimidin-4-yl)phenylamino)pyrimidin-4-yl)-1H-pyrazol-1-yl)propanenitrile;
3-(4-(1-(2-cyano-1-cyclopentylethyl)-1H-pyrazol-4-yl)pyrimidin-2-ylamino)benzoic acid;
3-cyclopentyl-3-(4-(5-methoxy-2-(4-morpholinophenylamino)pyrimidin-4-yl)-1H-pyrazol-1-yl)propanenitrile;
3-(4-(2-(4-(1H-pyrazol-1-yl)phenylamino)-5-methoxypyrimidin-4-yl)-1H-pyrazol-1-yl)-3-cyclopentylpropanenitrile;
*N*-(4-(4-(1-(2-cyano-1-cyclopentylethyl)-1H-pyrazol-4-yl)-5-methoxypyrimidin-2-ylamino)phenyl)acetamide;
4-(4-(1-(2-cyano-1-cyclopentylethyl)-1H-pyrazol-4-yl)-5-methoxypyrimidin-2-ylamino)-*N*,*N-*dimethylbenzamide;
3-cyclopentyl-3-(4-(2-(4-(2-oxopiperidin-1-yl)phenylamino)pyrimidin-4-yl)-1H-pyrazol-1-yl)propanenitrile;
3-cyclopentyl-3-(4-(2-(4-(2-oxo-1,3-oxazinan-3-yl)phenylamino)pyrimidin-4-yl)-1H-pyrazol-1-yl)propanenitrile;
3-cyclopentyl-3-(4-(2-(4-(2-oxooxazolidin-3-yl)phenylamino)pyrimidin-4-yl)-1H-pyrazol-1-yl)propanenitrile;
3-(4-(2-(3-aminophenylamino)pyrimidin-4-yl)-1H-pyrazol-1-yl)-3-cyclopentylpropanenitrile;
3-(4-(1-(2-cyano-1-cyclopentylethyl)-1H-pyrazol-4-yl)pyrimidin-2-ylamino)-*N*-methylbenzamide;
3-(4-(1-(2-cyano-1-cyclopentylethyl)-1H-pyrazol-4-yl)pyrimidin-2-ylamino)-*N,N-*dimethylbenzamide;
3-cyclopentyl-3-(4-(2-(3-(4-hydroxypiperidine-1-carbonyl)phenylamino)pyrimidin-4-yl)-1H-pyrazol-1-yl)propanenitrile;
3-(4-(1-(2-cyano-1-cyclopentylethyl)-1H-pyrazol-4-yl)pyrimidin-2-ylamino)-*N*-(2-hydroxyethyl)benzamide;
3-(4-(1-(2-cyano-1-cyclopentylethyl)-1H-pyrazol-4-yl)pyrimidin-2-ylamino)-*N*-(1-methoxypropan-2-yl)benzamide;
*N*-(3-(4-(1-(2-cyano-1-cyclopentylethyl)-1H-pyrazol-4-yl)pyrimidin-2-ylamino)phenyl)ethanesulfonamide;
*N*-(3-(4-(1-(2-cyano-1-cyclopentylethyl)-1H-pyrazol-4-yl)pyrimidin-2-ylamino)phenyl)methanesulfonamide;
methyl 3-(4-(1-(2-cyano-1-cyclopentylethyl)-1H-pyrazol-4-yl)pyrimidin-2-ylamino)phcnylcarbamate;
*N*-(3-(4-(1-(2-cyano-1-cyclopentylethyl)-1H-pyrazol-4-yl)pyrimidin-2-ylamino)phenyl)acetamide;
*N*-(3-(4-(1-(2-cyano-1-cyclopentylethyl)-1H-pyrazol-4-yl)pyrimidin-2-ylamino)phenyl)-2-(pyrrolidin-1-yl)acetamide;
4-(4-(1-(2-cyano-1-cyclopentylethyl)-1H-pyrazol-4-yl)pyrimidin-2-ylamino)benzoicacid;
3-cyclopentyl-3-(4-(2-(4-(4-methylpiperazine-1-carbonyl)phenylamino)pyrimidin-4-yl)-1H-pyrazol-1-yl)propanenitrile;
3-cyclopentyl-3-(4-(2-(4-(4-(2-hydroxyethyl)piperazine-1-carbonyl)phenylamino)pyrimidin-4-yl)-1H-pyrazol-1-yl)propanenitrile;
3-cyclopentyl-3-(4-(2-(4-(pyrrolidine-1-carbonyl)phenylamino)pyrimidin-4-yl)-1H-pyrazol-1-yl)propanenitrile;
3-cyclopentyl-3-(4-(2-(4-(3-oxopiperazine-1-carbonyl)phenylamino)pyrimidin-4-yl)-1H-pyrazol-1-yl)propanenitrile;
3-cyclopentyl-3-(4-(2-(4-(4-hydroxypiperidine-1-carbonyl)phenylamino)pyrimidin-4-yl)-1H-pyrazol-1-yl)propanenitrile;
4-(4-(1-(2-cyano-1-cyclopentylethyl)-1H-pyrazol-4-yl)pyrimidin-2-ylamino)-*N*-(cyclopropylmethyl)-*N*-propylbenzamide;
4-(4-(1-(2-cyano-1-cyclopentylethyl)-1 H-pyrazol-4-yl)pyrimidin-2-ylamino)-*N-*(cyclopropylmethyl)benzamide;
3-cyclopentyl-3-(4-(2-(4-(3-hydroxypyrrolidine-1-carbonyl)phenylamino)pyrimidin-4-yl)-1H-pyrazol-1-yl)propanenitrile;
3-(4-(2-(4-(azetidine-1-carbonyl)phenylamino)pyrimidin-4-yl)-1H-pyrazol-1-yl)-3-cyclopentylpropanenitrile;
3-cyclopentyl-3-(4-(2-(4-(2-oxopyrrolidin-1-yl)phenylamino)pyrimidin-4-yl)-1H-pyrazol-1-yl)propanenitrile;
3-cyclopentyl-3-(4-(5-methoxy-2-(4-(2-oxopyrrolidin-1-yl)phenylamino)pyrimidin-4-yl)-1H-pyrazol-1-yl)propanenitrile;
3-cyclopentyl-3-(4-(5-methoxy-2-(4-(oxazol-5-yl)phenylamino)pyrimidin-4-yl)-1H-pyrazol-1-yl)propanenitrile;
3-cyclopentyl-3-(4-(5-methoxy-2-(3-(oxazol-5-yl)phenylamino)pyrimidin-4-yl)-1H-pyrazol-1-yl)propanenitrile;
3-cyclopentyl-3-(4-(5-methoxy-2-(4-(3-oxomorpholino)phenylamino)pyrimidin-4-yl)-1H-pyrazol-1-yl)propanenitrile;
3-cyclopentyl-3-(4-(5-methoxy-2-(3-(2-methylpyrimidin-4-yl)phenylamino)pyrimidin-4-yl)-1H-pyrazol-1-yl)propanenitrile;
3-cyclopentyl-3-(4-(5-methoxy-2-(4-(2-oxopiperidin-1-yl)phenylamino)pyrimidin-4-yl)-1H-pyrazol-1-yl)propanenitrile;
3-cyclopentyl-3-(4-(5-methoxy-2-(4-(2-oxooxazolidin-3-yl)phenylamino)pyrimidin-4-yl)-1H-pyrazol-1-yl)propanenitrile;
3-cyclopentyl-3-(4-(2-(3-(4-methylpiperazine-1-carbonyl)phenylamino)pyrimidin-4-yl)-1H-pyrazol-1-yl)propanenitrile;
3-cyclopentyl-3-(4-(2-(3-(4-(2-hydroxyethyl)piperazine-1-carbonyl)phenylamino)pyrimidin-4-yl)-1H-pyrazol-1-yl)propanenitrile;
3-cyclopentyl-3-(4-(2-(3-(pyrrolidine-1-carbonyl)phenylamino)pyrimidin-4-yl)-1H-pyrazol-1-yl)propanenitrile;
3-cyclopentyl-3-(4-(2-(3-(3-oxopiperazine-1-carbonyl)phenylamino)pyrimidin-4-yl)-1H-pyrazol-1-yl)propanenitrile;
3-cyclopentyl-3-(4-(2-(3-(3-hydroxypyrrolidine-1-carbonyl)phenylamino)pyrimidin-4-yl)-1H-pyrazol-1-yl)propanenitrile;
3-(4-(2-(3-(azetidine-1-carbonyl)phenylamino)pyrimidin-4-yl)-1H-pyrazol-1-yl)-3-cyclopentylpropanenitrile;
3-(4-(2-(3-(4-acetylpiperazine-1-carbonyl)phenylamino)pyrimidin-4-yl)-1H-pyrazol-1-yl)-3-cyclopentylpropanenitrile;
3-cyclopentyl-3-(4-(2-(3-(4-(pyridin-3-ylmethyl)piperidine-1-carbonyl)phenylamino)pyrimidin-4-yl)-1 H-pyrazol-1-yl)propanenitrile;
3-(4-(1-(2-cyano-1-cyclopentylethyl)-1H-pyrazol-4-yl)pyrimidin-2-ylamino)-*N*-(1-(3-methoxyphenyl)ethyl)benzamide;
3-(4-(1-(2-cyano-1-cyclopentylethyl)-1H-pyrazol-4-yl)pyrimidin-2-ylamino)-N-(pyridin-3-ylmethyl)benzamide;
3-cyclopentyl-3-(4-(2-(3-(morpholine-4-carbonyl)phenylamino)pyrimidin-4-yl)-1H-pyrazol-1-yl)propanenitrile;
3-(4-(1-(2-cyano-1-cyclopentylethyl)-1H-pyrazol-4-yl)pyrimidin-2-ylamino)-*N*-((5-methylisoxazol-3-yl)methyl)benzamide;
3-(4-(1-(2-cyano-1-cyclopentylethyl)-1H-pyrazol-4-yl)pyrimidin-2-ylamino)-*N*-(2-(1-methylpyrrolidin-2-yl)ethyl)benzamide;
3-cyclopentyl-3-(4-(2-(3-(4-hydroxy-4-phenylpiperidine-1-carbonyl)phenylamino)pyrimidin-4-yl)-1 H-pyrazol-1-yl)propanenitrile;
3-(4-(2-(3-(4-benzyl-4-hydroxypiperidine-1-carbonyl)phenylamino)pyrimidin-4-yl)-1H-pyrazol-1-yl)-3-cyclopentylpropanenitrile;
3-cyclopentyl-3-(4-(2-(3-(3-(pyridin-2-yl)pyrrolidine-l-carbonyl)phenylamino)pyrimidin-4-yl)-1H-pyrazol-1-yl)propanenitrile;
3-(4-(1-(2-cyano-1-cyclopentylethyl)-1H-pyrazol-4-yl)pyrimidin-2-ylamino)-*N*-((tetrahydro-2H-pyran-4-yl)methyl)benzamide;
3-(4-(1-(2-cyano-1-cyclopentylethyl)-1H-pyrazol-4-yl)pyrimidin-2-ylamino)-*N*-(1-methylpiperidin-4-yl)benzamide;
3-cyclopentyl-3-(4-(2-(3-(4-phenylpiperidine-1-carbonyl)phenylamino)pyrimidin-4-yl)-1H-pyrazol-1-yl)propanenitrile;
3-(4-(1-(2-cyano-1-cyclopentylethyl)-1H-pyrazol-4-yl)pyrimidin-2-ylamino)-*N*-(1-(pyridin-2-yl)ethyl)benzamide;
3-cyclopentyl-3-(4-(2-(3-(3-(3-fluorophenyl)pyrrolidine-1-carbonyl)phenylamino)pyrimidin-4-yl)-1 H-pyrazol-1-yl)propanenitrile;
*N*-((3*R*)-1-(3-(4-(1-(2-cyano-1-cyclopentylethyl)-1H-pyrazol-4-yl)pyrimidin-2-ylamino)benzoyl)pyrrolidin-3-yl)acetamide;
3-(4-(1-(2-cyano-1-cyclopentylethyl)-1H-pyrazol-4-yl)pyrimidin-2-ylamino)-*N*-(2-(2-oxoimidazolidin-1-yl)ethyl)benzamide;
3-cyclopentyl-3-(4-(2-(3-(4-(pyrimidin-2-yl)piperazine-1-carbonyl)phenylamino)pyrimidin-4-yl)-1*H-*pyrazol-1-yl)propanenitrile;
3-(4-(1-(2-cyano-1-cyclopentylethyl)-1H-pyrazol-4-yl)pyrimidin-2-ylamino)-*N*-(2-(pyridin-3-yl)ethyl)benzamide;
3-cyclopentyl-3-(4-(2-(3-(2-(methoxymethyl)pyrrolidine-1-carbonyl)phenylamino)pyrimidin-4-yl)-1 H-pyrazol-1-yl)propanenitrile;
3-(4-(1-(2-cyano-1-cyclopentylethyl)-1H-pyrazol-4-yl)pyrimidin-2-ylamino)-*N*-(2-methoxybenzyl)benzamide;
3-cyclopentyl-3-(4-(2-(3-(4-phenoxypiperidine-l-carbonyl)phenylamino)pyrimidin-4-yl)-1H-pyrazol-1-yl)propanenitrile;
3-(4-(1-(2-cyano-1-cyclopentylethyl)-1H-pyrazol-4-yl)pyrimidin-2-ylamino)-*N*-(1-(hydroxymethyl)cyclopentyl)benzamide;
4-(4-(3-(4-(1-(2-cyano-1-cyclopentylethyl)-1H-pyrazol-4-yl)pyrimidin-2-ylamino)benzoyl)piperazin-1-yl)benzonitrile;
*N*-(1-benzylpyrrolidin-3-yl)-3-(4-(1-(2-cyano-1-cyclopentylethyl)-1H-pyrazol-4-yl)pyrimidin-2-ylamino)benzamide;
3-cyclopentyl-3-(4-(2-(3-(4-phenylpiperazine-1-carbonyl)phenylamino)pyrimidin-4-yl)-1H-pyrazol-1-yl)propanenitrile;
3-cyclopentyl-3-(4-(2-(3-nitrophenylamino)pyrimidin-4-yl)-1H-pyrazol-1-yl)propanenitrile;
3-cyclopentyl-3-(4-(2-(4-nitrophenylamino)pyrimidin-4-yl)-1H-pyrazol-1-yl)propanenitrile;
3-cyclobutyl-3-(4-(2-(4-morpholinophenylamino)pyrimidin-4-yl)-1H-pyrazol-1-yl)propanenitrile;
3-(4-(2-(4-aminophenylamino)pyrimidin-4-yl)-1H-pyrazol-1-yl)-3-cyclopentylpropanenitrile;
3-(4-(2-(4-(1H-pyrazol-1-yl)phenylamino)pyrimidin-4-yl)-1H-pyrazol-1-yl)-3-cyclobutylpropanenitrile;
3-cyclobutyl-3-(4-(2-(4-(2-oxopiperidin-1-yl)phenylamino)pyrimidin-4-yl)-1H-pyrazol-1-yl)propanenitrile;
3-cyclobutyl-3-(4-(2-(4-(3-oxomorpholino)phenylamino)pyrimidin-4-yl)-1H-pyrazol-1-yl)propanenitrile;
3-cyclobutyl-3-(4-(2-(3-(oxazol-5-yl)phenylamino)pyrimidin-4-yl)-1H-pyrazol-1-yl)propanenitrile;
3-cyclopropyl-3-(4-(2-(4-morpholinophenylamino)pyrimidin-4-yl)-1H-pyrazol-1-yl)propanenitrile;
3-(4-(2-(4-(1H-pyrazol-1-yl)phenylamino)pyrimidin-4-yl)-1H-pyrazol-1-yl)-3-cyclopropylpropanenitrile;
3-cyclopropyl-3-(4-(2-(4-(2-oxopiperidin-1-yl)phenylamino)pyrimidin-4-yl)-1H-pyrazol-1-yl)propanenitrile;
3-cyclopropyl-3-(4-(2-(4-(3-oxomorpholino)phenylamino)pyrimidin-4-yl)-1H-pyrazol-1-yl)propanenitrile;
3-cyclopropyl-3-(4-(2-(3-(oxazol-5-yl)phenylamino)pyrimidin-4-yl)-1H-pyrazol-1-yl)propanenitrile; *N*-(4-(4-(1-(2-cyano-1-cyclopentylethyl)-1H-pyrazol-4-yl)pyrimidin-2-ylamino)phenyl)-2,6-(cis)-dimethylmorpholine-4-sulfonamide;
*N*-(4-(4-(1-(2-cyano-1-cyclopentylethyl)-1H-pyrazol-4-yl)pyrimidin-2-ylamino)phenyl)benzamide;
*N*-(4-(4-(1-(2-cyano-1-cyclopentylethyl)-1H-pyrazol-4-yl)pyrimidin-2-ylamino)phenyl)-1-(methylsulfonyl)methanesulfonamide;
*N*-(4-(4-(1-(2-cyano-1-cyclopentylethyl)-1H-pyrazol-4-yl)pyrimidin-2-ylamino)phenyl)-3,5-difluorobenzamide;
*N*'-(4-(4-(1-(2-cyano-1-cyclopentylethyl)-1H-pyrazol-4-yl)pyrimidin-2-ylamino)phenyl)-*N,N-*dimethylsulfamide;
*N*-(4-(4-(1-(2-cyano-1-cyclopentylethyl)-1H-pyrazol-4-yl)pyrimidin-2-ylamino)phenyl)-5-methylisoxazole-3-carboxamide;
*N*-(4-(4-(1-(2-cyano-1-cyclopentylethyl)-1H-pyrazol-4-yl)pyrimidin-2-ylamino)phenyl)isoxazole-5-carboxamide;
*N*-(4-(4-(1-(2-cyano-1-cyclopentylethyl)-1H-pyrazol-4-yl)pyrimidin-2-ylamino)phenyl)-3,5-dimethylisoxazole-4-carboxamide;
*N*-(4-(4-(1-(2-cyano-1-cyclopentylethyl)-1H-pyrazol-4-yl)pyrimidin-2-ylamino)phenyl)-1-methyl-1H-pyrazole-3-sulfonamide;
*N*-(4-(4-(1-(2-cyano-1-cyclopentylethyl)-1H-pyrazol-4-yl)pyrimidin-2-ylamino)phenyl)-2,5-difluorobenzamide;
3-cyclopentyl-3-(4-(2-(4-(1,1-dioxidoisothiazolidin-2-yl)phenyl)aminopyrimidin-4-yl)-1H-pyrazol-1-yl)propanenitrile;
*N*-(4-(4-(1-(2-cyano-1-cyclopentylethyl)-1H-pyrazol-4-yl)pyrimidin-2-ylamino)phenyl)-5-(2-methylthiazol-4-yl)thiophene-2-sulfonamide;
*N*-(4-(4-(1-(2-cyano-1-cyclopentylethyl)-1H-pyrazol-4-yl)pyrimidin-2-ylamino)phenyl)-6-methylpyridine-2-sulfonamide;
*N*-(4-(4-(1-(2-cyano-1-cyclopentylethyl)-1H-pyrazol-4-yl)pyrimidin-2-ylamino)phenyl)-5-(pyridin-2-yl)thiophene-2-sulfonamide;
5-chloro-*N*-(4-(4-(1-(2-cyano-1-cyclopentylethyl)-1H-pyrazol-4-yl)pyrimidin-2-ylamino)phenyl)thiophene-2-sulfonamide;
*N*-(4-(4-(1-(2-cyano-1-cyclopentylethyl)-1H-pyrazol-4-yl)pyrimidin-2-ylamino)phenyl)-6-morpholinopyridine-3-sulfonamide;
tetrahydrofuran-3-yl 4-(4-(1-(2-cyano-1-cyclopentylethyl)-1H-pyrazol-4-yl)pyrimidin-2-ylamino)phenylcarbamate;
tetrahydrofuran-3-yl 3-(4-(1-(2-cyano-1-cyclopentylethyl)-1H-pyrazol-4-yl)pyrimidin-2-ylamino)phenylcarbamate;
*N*-(3-(4-(1-(2-cyano-1-cyclopentylethyl)-1H-pyrazol-4-yl)pyrimidin-2-ylamino)phenyl)-1-methyl-1H-pyrazole-3-sulfonamide;
*N*'-(3-(4-(1-(2-cyano-1-cyclopentylethyl)-1H-pyrazol-4-yl)pyrimidin-2-ylamino)phenyl)-*N,N-*dimethylsulfamide;
*N*-(4-(4-(1-(2-cyano-1-cyclopentylethyl)-1H-pyrazol-4-yl)pyrimidin-2-ylamino)phenyl)-2-(pyrrolidin-1-yl)acetamide;
*N*-(4-(4-(1-(2-cyano-1-cyclopentylethyl)-1H-pyrazol-4-yl)pyrimidin-2-ylamino)phenyl)-2-(3-hydroxypyrrolidin-1-yl)acetamide;
*N*-(4-(4-(1-(2-cyano-1-cyclopentylethyl)-1H-pyrazol-4-yl)pyrimidin-2-ylamino)phenyl)-2-(4-hydroxypiperidin-1-yl)acetamide;
*N*-(4-(4-(1-(2-cyano-1-cyclopentylethyl)-1H-pyrazol-4-yl)pyrimidin-2-ylamino)phenyl)-2-(3-oxopiperazin-1-yl) acetamide;
*N*-(4-(4-(1-(2-cyano-1-cyclopentylethyl)-1H-pyrazol-4-yl)pyrimidin-2-ylamino)phenyl)-2-morpholinoacetamide;
*N*-(4-(4-(1-(2-cyano-1-cyclopentylethyl)-1H-pyrazol-4-yl)pyrimidin-2-ylamino)phenyl)-2-((tetrahydro-2H-pyran-4-yl)methylamino)acetamide;
*N*-(4-(4-(1-(2-cyano-1-cyclopentylethyl)-1H-pyrazol-4-yl)pyrimidin-2-ylamino)phenyl)-2-(2-(methoxymethyl)pyrrolidin-1-yl)acetamide;
*N*-(4-(4-(1-(2-cyano-1-cyclopentylethyl)-1H-pyrazol-4-yl)pyrimidin-2-ylamino)phenyl)-2-(cyclopropylmethylamino)acetamide;
*N*-(4-(4-(1-(2-cyano-1-cyclopentylethyl)-1H-pyrazol-4-yl)pyrimidin-2-ylamino)phenyl)-2-(1-methoxypropan-2-ylamino)acetamide;
2-(4-(5-methylisoxazol-3-yloxy)-1-(4-(2-(4-morpholinophenylamino)pyrimidin-4-yl)-1H-pyrazol-1-yl)cyclohexyl)acetonitrile;
3-cyclopentyl-3-(4-(2-(4-(morpholine-4-carbonyl)phenylamino)pyrimidin-4-yl)-1H-pyrazol-1-yl)propanenitrile;
4-(4-(1-(2-cyano-1-cyclopentylethyl)-1H-pyrazol-4-yl)pyrimidin-2-ylamino)-*N*-((tctrahydro-2H-pyran-4-yl)methyl)benzamide;
3-cyclopcntyl-3-(4-(2-(4-((3-cndo)-3-hydroxy-8-azabicyclo[3.2.1] octanc-8-carbonyl)phenylamino)pyrimidin-4-yl)-1H-pyrazol-1-yl)propanenitrile;
3-cyclopentyl-3-(4-(2-(4-(2-oxa-6-azatricyclo[3,3,1,1(3,7)]dec-6-ylcarbonyl)phenyl)aminopyrimidin-4-yl)-1H-pyrazol-1-yl)propanenitrile;
4-(4-(1-(2-cyano-1-cyclopentylethyl)-1H-pyrazol-4-yl)pyrimidin-2-ylamino)-*N*-(*cis*-4-hydroxycyclohexyl)-*N*-methylbenzamide;
4-(4-(1-(2-cyano-1-cyclopentylethyl)-1H-pyrazol-4-yl)pyrimidin-2-ylamino)-*N*-methyl-*N*-(tetrahydro-2H-pyran-4-yl)benzamide;
3-cyclopentyl-3-(4-(2-(4-(S*)-(4,4-dimethyl-2-oxo-1-oxa-3,7-diazaspiro[4.4]nonane-7-carbonyl)phenylamino)pyrimidin-4-yl)-1 H-pyrazol-1 -yl)propanenitrile;
3-cyclopentyl-3-(4-(2-(4-(4,4-dimethyl-1-oxa-7-azaspiro[4.4]nonane-7-carbonyl)phenylamino)pyrimidin-4-yl)-1 H-pyrazol-1-yl)propanenitrile;
3-cyclopentyl-3-(4-(2-(4-(4-methoxypiperidine-1-carbonyl)phenylamino)pyrimidin-4-yl)-1H-pyrazol-1-yl)propanenitrile;
*N*-((3*S*)-1-(4-(4-(1-(2-cyano-1-cyclopentylethyl)-1H-pyrazol-4-yl)pyrimidin-2-ylamino)benzoyl)pyrrolidin-3-yl)acetamide;
4-(4-(1-(2-cyano-1-cyclopentylethyl)-1H-pyrazol-4-yl)pyrimidin-2-ylamino)-*N*-(*cis*-4-hydroxycyclohexyl)benzamide;
3-(4-(2-(4-(4-acetylpiperazine-1-carbonyl)phenylamino)pyrimidin-4-yl)-1H-pyrazol-1-yl)-3-cyclopentylpropanenitrile;
(3*S*)-1-(4-(4-(1-(2-cyano-1-cyclopentylethyl)-1H-pyrazol-4-yl)pyrimidin-2-ylamino)benzoyl)pyrrolidine-3-carbonitrile;
3-cyclopentyl-3-(4-(2-(4-(3-methoxypyrrolidine-1-carbonyl)phenylamino)pyrimidin-4-yl)-1H-pyrazol-1-yl)propanenitrile;
4-(4-(1-(2-cyano-1-cyclopentylethyl)-1H-pyrazol-4-yl)pyrimidin-2-ylamino)-*N*-(1-methylpiperidin-4-yl)benzamide;
3-cyclopentyl-3-(4-(2-(4-(3-oxo-2,8-diazaspiro[4.5]decane-8-carbonyl)phenylamino)pyrimidin-4-yl)-1 H-pyrazol-1-yl)propanenitrile;
3-cyclopentyl-3-(4-(2-(4-(3-fluoropyrrolidine-1-carbonyl)phenylamino)pyrimidin-4-yl)-1H-pyrazol-1-yl)propanenitrile;
3-cyclopentyl-3-(4-(2-(4-(3-(dimethylamino)pyrrolidine-1-carbonyl)phenylamino)pyrimidin-4-yl)-1H-pyrazol-1-yl)propanenitrile;
Ethyl 4-(4-(4-(1-(2-cyano-1-cyclopentylethyl)-1H-pyrazol-4-yl)pyrimidin-2-ylamino)benzamido)piperidine-1-carboxylate;
4-(4-(1-(2-cyano-1-cyclopentylethyl)-1H-pyrazol-4-yl)pyrimidin-2-ylamino)-*N*-(1-(pyridin-2-yl)pyrrolidin-3-yl)benzamide;
3-cyclopentyl-3-(4-(2-(4-(3-(pyridin-2-yloxy)pyrrolidine-1-carbonyl)phenylamino)pyrimidin-4-yl)-1H-pyrazol-1-yl)propanenitrile;
1-(4-(4-(1-(2-cyano-1-cyclopentylethyl)-1H-pyrazol-4-yl)pyrimidin-2-ylamino)benzoyl)-*N,N-*dimethylpiperidine-4-carboxamide;
4-(4-(1-(2-cyano-1-cyclopentylethyl)-1H-pyrazol-4-yl)pyrimidin-2-ylamino)-*N*-(1-(dimethylamino)-1-oxobutan-2-yl)benzamide;
*N*-(3-(4-(1-(2-cyano-1-cyclopentylethyl)-1H-pyrazol-4-yl)pyrimidin-2-ylamino)phenyl)-2-(4-methylpiperazin-1-yl)acetamide;
*N*-(3-(4-(1-(2-cyano-1-cyclopentylethyl)-1H-pyrazol-4-yl)pyrimidin-2-ylamino)phenyl)-2-(3-hydroxypyrrolidin-1-yl)acetamide;
*N*-(3-(4-(1-(2-cyano-1-cyclopentylethyl)-1H-pyrazol-4-yl)pyrimidin-2-ylamino)phenyl)-2-(3-oxopiperazin-1-yl)acetamide;
*N*-(3-(4-(1-(2-cyano-1-cyclopentylethyl)-1H-pyrazol-4-yl)pyrimidin-2-ylamino)phenyl)-2-(4-hydroxypiperidin-1-yl)acetamide;
*N*-(3-(4-(1-(2-cyano-1-cyclopentylethyl)-1H-pyrazol-4-yl)pyrimidin-2-ylamino)phenyl)-2-(4-(2-hydroxyethyl)piperazin-1-yl)acetamide;
*N*-(3-(4-(1-(2-cyano-1-cyclopentylethyl)-1H-pyrazol-4-yl)pyrimidin-2-ylamino)phenyl)-2-(cyclopropylmethylamino)acetamide;
*N*-(3-(4-(1-(2-cyano-1-cyclopentylethyl)-1H-pyrazol-4-yl)pyrimidin-2-ylamino)phenyl)-2-morpholinoacetamide;
*N*-(3-(4-(1-(2-cyano-1-cyclopentylethyl)-1H-pyrazol-4-yl)pyrimidin-2-ylamino)phenyl)-2-(ethylamino)acetamide;
2-(4-(5-methylisoxazol-3-yloxy)-1-(4-(2-(4-(3-oxomorpholino)phenylamino)pyrimidin-4-yl)-1H-pyrazol-1-yl)cyclohexyl)acetonitrile;
2-(4-(5-methylisoxazol-3-yloxy)-1-(4-(2-(4-(2-oxopiperidin-1-yl)phenylamino)pyrimidin-4-yl)-1H-pyrazol-1-yl)cyclohexyl)acetonitrile;
2-(1-(4-(2-(4-(1H-pyrazol-1-yl)phenylamino)pyrimidin-4-yl)-1H-pyrazol-1-yl)-4-(5-methylisoxazol-3-yloxy)cyclohexyl)acetonitrile;
2-(4-(5-methylisoxazol-3-yloxy)-1-(4-(2-(3-(oxazol-5-yl)phenylamino)pyrimidin-4-yl)-1H-pyrazol-1-yl)cyclohexyl)acetonitrile;
3-(cyanomethyl)-3-(4-(2-(4-morpholinophenylamino)pyrimidin-4-yl)-1H-pyrazol-1-yl)cyclobutanecarbonitrile;
4-(4-(1-(3-(cyanomethyl)-1-(ethylsulfonyl)azetidin-3-yl)-1H-pyrazol-4-yl)pyrimidin-2-ylamino)benzoic acid;
4-(4-((1-(2-eyano-1-cyclopropylethyl)-1H-pyrazol-4-yl)pyrimidin-2-ylamino)benzoicacid; 3-cyclopropyl-3-(4-(2-(4-(4-hydroxypiperidine-1-carbonyl)phenylamino)pyrimidin-4-yl)-1H-pyrazol-1-yl)propanenitrile;
3-cyclopropyl-3-(4-(2-(4-((3-endo)-3-hydroxy-8-azabicyclo[3.2.1]octane-8-carbonyl)phcnylamino)pyrimidin-4-yl)-1H-pyrazol-1-yl)propancnitrilc;
3-cyclopropyl-3-(4-(2-(4-(pyrrolidine-1-carbonyl)phenylamino)pyrimidin-4-yl)-1H-pyrazol-1-yl)propanenitrile;
4-(4-(1-(2-cyano-1-cyclopropylethyl)-1H-pyrazol-4-yl)pyrimidin-2-ylamino)-N-(tetrahydro-2H-pyran-4-yl)benzamide;
2-(1-(ethylsulfonyl)-3-(4-(2-(4-(morpholine-4-carbonyl)phenylamino)pyrimidin-4-yl)-1H-pyrazol-1-yl)azetidin-3-yl)acetonitrile;
2-(1-(ethylsulfonyl)-3-(4-(2-(4-(4-hydroxypiperidine-1-carbonyl)phenylamino)pyrimidin-4-yl)-1H-pyrazol-1-yl)azetidin-3-yl)acetonitrile;
2-(1-(ethylsulfonyl)-3-(4-(2-(4-((3-endo)-3-hydroxy-8-azabicyclo[3.2.1]octane-8-carbonyl)phenylamino)pyrimidin-4-yl)-1H-pyrazol-1-yl)azetidin-3-yl)acetonitrile;
2-(1-(ethylsulfonyl)-3-(4-(2-(4-(pyrrolidine-1-carbonyl)phenylamino)pyrimidin-4-yl)-1H-pyrazol-1-yl)azetidin-3-yl)acetonitrile;
4-(4-(1-(3-(cyanomethyl)-1-(ethylsulfonyl)azetidin-3-yl)-1H-pyrazol-4-yl)pyrimidin-2-ylamino)-*N-*(tetrahydro-2H-pyran-4-yl)benzamide;
3-cyclopropyl-3-(4-(2-(4-(morpholine-4-carbonyl)phenylamino)pyrimidin-4-yl)-1H-pyrazol-1-yl)propanenitrile;
3-(4-(2-(4-(azetidine-1-carbonyl)phenylamino)pyrimidin-4-yl)-1H-pyrazol-1-yl)-3-cyclopropylpropanenitrile;
3-cyclopropyl-3-(4-(2-(4-(2-oxa-6-azatricyclo[3.3.1.1(3,7)]dec-6-ylcarbonyl)phenyl)aminopyrimidin-4-yl)-1 H-pyrazol-1-yl)propanenitrile;
3-cyclopropyl-3-(4-(2-(4-(4-methoxypiperidine-1-carbonyl)phenylamino)pyrimidin-4-yl)-1H-pyrazol-1-yl)propanenitrile;
(3*R*)-1-(4-(4-(1-(2-cyano-1-cyclopropylethyl)-1H-pyrazol-4-yl)pyrimidin-2-ylamino)benzoyl)pyrrolidine-3-carbonitrile;
3-cyclopropyl-3-(4-(2-(4-(3-methoxypyrrolidine-1-carbonyl)phenylamino)pyrimidin-4-yl)-1H-pyrazol-1-yl)propanenitrile;
3-cyclopropyl-3-(4-(2-(4-(3-hydroxypyrrolidine-1-carbonyl)phenylamino)pyrimidin-4-yl)-1H-pyrazol-1-yl)propanenitrile;
3-cyclopropyl-3-(4-(2-(4-(4-methylpiperazine-1-carbonyl)phenylamino)pyrimidin-4-yl)-1H-pyrazol-1-yl)propanenitrile;
*N*-((3*R*)-1-(4-(4-(1-(2-cyano-1-cyclopropylethyl)-1H-pyrazol-4-yl)pyrimidin-2-ylamino)benzoyl)pyrrolidin-3-yl)acetamide;
3-(4-(2-(4-(4-acetylpiperazine-1-carbonyl)phenylamino)pyrimidin-4-yl)-1H-pyrazol-1-yl)-3-cyclopropylpropanenitrile;
3-cyclopropyl-3-(4-(2-(4-(3-(dimethylamino)pyrrolidine-1-carbonyljphenylamino)pyrimidin-4-yl)-1 H-pyrazol-1-yl)propanenitrile;
3-cyclopropyl-3-(4-(2-(4-(3-fluoropyrrolidinc-1-carbonyl)phcnylamino)pyrimidin-4-yl)-1H-pyrazol-1-yl)propanenitrile;
Ethyl 4-(4-(4-(1-(2-cyano-1-cyclopropylethyl)-1H-pyrazol-4-yl)pyrimidin-2-ylamino)benzoyl)aminopiperidine-1-carboxylate;
2-(3-(4-(2-(4-(azetidine-1-carbonyl)phenylamino)pyrimidin-4-yl)-1H-pyrazol-1-yl)-1-(ethylsulfonyl)azetidin-3-yl)acetonitrile;
1-(ethylsulfonyl)-3-(4-(2-(4-(2-oxa-6-azatricyclo[3.3.1.1 (3,7)]dec-6-ylcarbonyl)phenyl)aminopyrimidin-4-yl)-1H-pyrazol-1-yl)azetidin-3-ylacetonitrile;
(1-(ethylsulfonyl)-3-4-(2-(4-((4-methoxypiperidin-1-yljcarbonyl)phenylamino)pyrimidin-4-yl)-1H-pyrazol-1-ylazetidin-3-yl)acetonitrile;
1-(4-(4-(1-(3-(cyanomethyl)-1-(ethylsulfonyl)azetidin-3-yl)-1H-pyrazol-4-yl)pyrimidin-2-ylamino)benzoyl)pyrrolidine-3-carbonitrile;
2-(1-(ethylsulfonyl)-3-(4-(2-(4-(3-methoxypyrrolidine-1-carbonyl)phenylamino)pyrimidin-4-yl)-1H-pyrazol-1-yl)azetidin-3-yl)acetonitrile;
2-(1-(ethylsulfonyl)-3-(4-(2-(4-(3-hydroxypyrrolidine-1-carbonyl)phenylamino)pyrimidin-4-yl)-1H-pyrazol-1-yl)azetidin-3-yl)acetonitrile;
2-(1-(ethylsulfonyl)-3-(4-(2-(4-(4-methylpiperazine-1-carbonyl)phenylamino)pyrimidin-4-yl)-1H-pyrazol-1-yl)azetidin-3-yl)acetonitrile;
*N*-(1-(4-(4-(1-(3-(cyanomethyl)-1-(ethylsulfonyl)azetidin-3-yl)-1H-pyrazol-4-yl)pyrimidin-2-ylamino)benzoyl)pyrrolidin-3-yl)acetamide;
2-(3-(4-(2-(4-(4-acetylpiperazine-1-carbonyl)phenylamino)pyrimidin-4-yl)-1H-pyrazol-1-yl)-1-(ethylsulfonyl)azetidin-3-yl)acetonitrile;
2-(3-(4-(2-(4-(3-(dimethylamino)pyrrolidine-1-carbonyl)phenylamino)pyrimidin-4-yl)-1H-pyrazol-1-yl)-1-(ethylsulfonyl)azetidin-3-yl)acetonitrile;
2-(1-(ethylsulfonyl)-3-(4-(2-(4-(3-fluoropyrrolidine-1-carbonyl)phenylamino)pyrimidin-4-yl)-1H-pyrazol-1-yl)azetidin-3-yl)acetonitrile;
Ethyl 4-(4-(4-(1-(3-(cyanomethyl)-1-(ethylsulfonyl)azetidin-3-yl)-1H-pyrazol-4-yl)pyrimidin-2-ylamino)benzamido)piperidine-1-carboxylate;
4-(4-(1-(3-cyano-1-(cyanomethyl)cyclobutyl)-1H-pyrazol-4-yl)pyrimidin-2-ylamino)benzoic acid;
4-(4-(1-(2-cyano-1-cyclopentylethyl)-1H-pyrazol-4-yl)-5-methoxypyrimidin-2-ylamino)benzoic acid;
3-(cyanomethyl)-3-(4-(2-(4-(morpholine-4-carbonyl)phenylamino)pyrimidin-4-yl)-1H-pyrazol-1-yl)cyclobutanecarbonitrile;
3-(cyanomethyl)-3-(4-(2-(4-(pyrrolidine-1-carbonyl)phenylamino)pyrimidin-4-yl)-1H-pyrazol-1-yl)cyclobutanecarbonitrile;
4-(4-(1-(3-cyano-1-(cyanomethyl)cyclobutyl)-1H-pyrazol-4-yl)pyrimidin-2-ylamino)-*N*-(tetrahydro-2H-pyran-4-yl)benzamide;
3-(cyanomethyl)-3-(4-(2-(4-(3-hydroxypyrrolidine-1-carbonyl)phenylamino)pyrimidin-4-yl)-1H-pyrazol-1-yl)cyclobutanccarbonitrile;
4-(4-(1-(3-cyano-1-(cyanomethyl)cyclobutyl)-1H-pyrazol-4-yl)pyrimidin-2-ylamino)-*N*-((5-methylisoxazol-3-yl)methyl)benzamide;
3-(4-(2-(4-(azetidine-1-carbonyl)phenylamino)pyrimidin-4-yl)-1H-pyrazol-1-yl)-3-(cyanomethyl)cyclobutanecarbonitrile;
3-(cyanomethyl)-3-(4-(2-(4-(4-methylpiperazine-1-carbonyl)phenylamino)pyrimidin-4-yl)-1H-pyrazol-1-yl)cyclobutanecarbonitrile;
3-(cyanomethyl)-3-(4-(2-(4-(3-fluoropyrrolidine-1-carbonyl)phenylamino)pyrimidin-4-yl)-1H-pyrazol-1-yl)cyclobutanecarbonitrile;
3-(cyanomethyl)-3-(4-(2-(4-(4-methoxypiperidine-1-carbonyl)phenylamino)pyrimidin-4-yl)-1 H-pyrazol-1-yl)cyclobutanecarbonitrile;
3-(cyanomethyl)-3-(4-(2-(4-(3-methoxypyrrolidine-1-carbonyl)phenylamino)pyrimidin-4-yl)-1H-pyrazol-1-yl)cyclobutanecarbonitrile;
1-(4-(4-(1-(3-cyano-1-(cyanomethyl)cyclobutyl)-1H-pyrazol-4-yl)pyrimidin-2-ylamino)benzoyl)pyrrolidine-3-carbonitrile;
3-(4-(2-(4-(4-acetylpiperazine-1-carbonyl)phenylamino)pyrimidin-4-yl)-1H-pyrazol-1-yl)-3-(cyanomethyl)cyclobutanecarbonitrile;
*N*-(1-(4-(4-(1-(3-cyano-1-(cyanomethyl)cyclobutyl)-1H-pyrazol-4-yl)pyrimidin-2-ylamino)benzoyl)pyrrolidin-3-yl)acetamide;
3-(cyanomethyl)-3-(4-(2-(4-((3-endo)-3-hydroxy-8-azabicyclo[3.2.1]octane-8-carbonyl)phenylamino)pyrimidin-4-yl)-1H-pyrazol-1-yl)cyclobutanecarbonitrile;
3-(cyanomethyl)-3-(4-(2-(4-(4-hydroxypiperidine-1-carbonyl)phenylamino)pyrimidin-4-yl)-1H-pyrazol-1-yl)cyclobutanecarbonitrile;
3-cyclopentyl-3-(4-(5-methoxy-2-(4-(morpholine-4-carbonyl)phenylamino)pyrimidin-4-yl)-1H-pyrazol-1-yl)propanenitrile;
3-cyclopentyl-3-(4-(5-methoxy-2-(4-(pyrrolidine-l-carbonyl)phenylamino)pyrimidin-4-yl)-1H-pyrazol-1-yl)propanenitrile;
3-cyclopentyl-3-(4-(5-methoxy-2-(4-(4-methylpiperazine-1-carbonyl)phenylamino)pyrimidin-4-yl)-1H-pyrazol-1-yl)propanenitrile; and
4-(4-(1-(2-cyano-1-cyclopentylethyl)-1H-pyrazol-4-yl)-5-methoxypyrimidin-2-ylamino)-*N-*(tetrahydro-2H-pyran-4-yl)benzamide, or pharmaceutically acceptable salt thereof.
and pharmaceutically acceptable salts of any of the aforementioned.

In a tenth aspect, the agent is selected from:
3-(4-(7H-pyrrolo[2,3-d]pyrimidin-4-yl)-1H-pyrazol-1-yl)-3-(3-hydroxycyclopentyl)propanenitrile; 3-(4-(7H-pyrrolo [2,3-d]pyrimidin-4-yl)-1H-pyrazol-1-yl)-3-(2-hydroxycyclopentyl)propanenitrile; and 3-(4-(7H-pyrrolo [2,3-d]pyrimidin-4-yl)-1H-pyrazol-1-yl)-3-(3-oxocyclopcntyl)propanenitrile; and pharmaceutically acceptable salts of any of the aforementioned.

In a eleventh aspect, the agent is selected from:
(R)-3-(4-(7H-pyrrolo[2,3-d]pyrimidin-4-yl)-1H-pyrazol-1-yl)-3-cyclopentylpropanenitrile maleic acid salt;
(R)-3-(4-(7H-pyrrolo[2,3-d]pyrimidin-4-yl)-1H-pyrazol-1-yl)-3-cyclopentylpropanenitrile sulfuric acid salt, and
(R)-3-(4-(7H-pyrrolo[2,3-d]pyrimidin-4-yl)-1H-pyrazol-1-yl)-3-cyclopentylpropanenitrile phosphoric acid salt.

At various places in the present specification, substituents of compounds of the invention are disclosed in groups or in ranges. It is specifically intended that the invention include each and every individual subcombination of the members of such groups and ranges. For example, the term "C₁₋₆ alkyl" is specifically intended to individually disclose methyl, ethyl, C₃ alkyl, C₄ alkyl, C₅ alkyl, and C₆ alkyl.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

At various places in the present specification, linking substituents are described. It is specifically intended that each linking substituent include both the forward and backward forms of the linking substituent. For example, -NR(CR'R")ₙ - includes both NR(CR'R")ₙ and -(CR'R")ₙNR-. Where the structure clearly requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

It is further intended that where a group is depicted in a certain direction or orientation, all other possible orientations are included. For example, it is intended that the defining groups of ring A and ring B are meant to include all orientations, such that when rings A and B are asymmetric they can be combined with the core structure in at least two possible orientations.

It is further intended with respect to the moiety -W¹-W²-W³-W⁴, that the bond(s) connecting each component (e.g., bonds between W¹ and W², between W² and W³, etc.) can be single, double, or normalized.

The term "n-membered" where n is an integer typically describes the number of ring-forming atoms in a moiety where the number of ring-forming atoms is n. For example, piperidinyl is an example of a 6-membered heterocycloalkyl ring and 1,2,3,4-tetrahydro-naphthalene is an example of a 10-mcmbcrcd cycloalkyl group.

As used herein, the term "acyl" refers to -C(O)-alkyl.

As used herein, the term "acylamino" refers to an amino group substituted with an acyl group.

As used herein, the term "acyloxy" refers to -OC(O)-alkyl.

As used herein, the term "alkenyl" refers to an alkyl group having one or more double carbon-carbon bonds. Example alkenyl groups include ethenyl, propenyl, cyclohexenyl, and the like. A linking alkenyl group is referred to herein as "alkenylene."

As used herein, the term "alkoxy" refers to an -O-alkyl group. Example alkoxy groups include methoxy, ethoxy, propoxy (*e*.*g*., n-propoxy and isopropoxy), t-butoxy, and the like.

As used herein, the term "alkyl" is meant to refer to a saturated hydrocarbon group which is straight-chained or branched. Example alkyl groups include methyl (Me), ethyl (Et), propyl (*e.g*., n-propyl and isopropyl), butyl (*e.g.,* n-butyl, isobutyl, t-butyl), pentyl (*e.g*., n-pentyl, isopentyl, neopentyl), and the like. An alkyl group can contain from 1 to about 20, from 2 to about 20, from 1 to about 10, from 1 to about 8, from 1 to about 6, from 1 to about 4, or from 1 to about 3 carbon atoms. A linking alkyl group is referred to herein as "alkylene."

As used herein, the term "alkylamino" refers to an amino group substituted by an alkyl group.

As used herein, the term "alkylaminocarbonyl" refers to a carbonyl group substituted by an alkylamino group.

As used herein, the term "alkynyl" refers to an alkyl group having one or more triple carbon-carbon bonds. Example alkynyl groups include ethynyl, propynyl, and the like. A linking alkynyl group is referred to herein as "alkynylene."

As used herein, the term "amino" refers to NH₂.

As used herein, the term "aminocarbonyl" refers to a carbonyl group substituted by an amino group.

As used herein, the term "aryl" refers to monocyclic or polycyclic (e.g., having 2, 3 or 4 fused rings) aromatic hydrocarbons such as, for example, phenyl, naphthyl, anthracenyl, phenanthrenyl, indanyl, indenyl, and the like. In some embodiments, aryl groups have from 6 to about 20 carbon atoms. A linking aryl group is referred to herein as "arylene."

As used herein, the term "arylalkyl" refers to alkyl substituted by aryl and "cycloalkylalkyl" refers to alkyl substituted by cycloalkyl. An example arylalkyl group is benzyl.

As used herein, the term "aryloxy" refers to an -O-aryl group. An example aryloxy group is phenoxy.

As used herein, the term "benzyloxy" refers to -O-benzyl.

As used herein, the term "carbocyclyl" groups are saturated (i.e., containing no double or triple bonds) or unsaturated (i.e., containing one or more double or triple bonds) cyclic hydrocarbon moieties. Carbocyclyl groups can be mono- or polycyclic (e.g., having 2, 3 or 4 fused rings). Example carbocyclyl groups include cyclopropyl, cyclobutyl, cyclopentyl, cyclohexyl, cycloheptyl, cyclopcntcnyl, 1,3-cyclopentadienyl, cyclohcxcnyl, norbornyl, norpinyl, norcarnyl, adamantyl, phenyl, and the like. Carbocyclyl groups can be aromatic (e.g., "aryl") or non-aromatic (e.g., "cycloalkyl"). In some embodiments, carbocyclyl groups can have from about 3 to about 30 carbon atoms, about 3 to about 20, about 3 to about 10, or about 3 to about 7 carbon atoms.

As used herein, the term "carbocyclylalkyl" refers to an alkyl moiety substituted by a carbocyclyl group. Example carbocyclylalkyl groups include "aralkyl" (alkyl substituted by aryl ("arylalkyl")) and "cycloalkylalkyl" (alkyl substituted by cycloalkyl). In some embodiments, carbocyclylalkyl groups have from 4 to 24 carbon atoms.

As used herein, the term "cyanoalkyl" refers to an alkyl group substituted by cyano. The carbon of the cyano group is typically not counted if a carbon count precedes the term. For example, cyanomethyl is considered herein to be a C₁ cyanoalkyl group.

As used herein, the term "cycloalkyl" refers to non-aromatic cyclic hydrocarbons including cyclized alkyl, alkenyl, and alkynyl groups. Cycloalkyl groups can include mono- or polycyclic (*e*.*g*., having 2, 3 or 4 fused rings) groups and spirocycles. In some embodiments, cycloalkyl groups can have from 3 to about 20 carbon atoms, 3 to about 14 carbon atoms, 3 to about 10 carbon atoms, or 3 to 7 carbon atoms. Cycloalkyl groups can further have 0, 1, 2, or 3 double bonds and/or 0, 1, or 2 triple bonds. Ring-forming carbon atoms of a cycloalkyl group can be optionally substituted by oxo or sulfido. Cycloalkyl groups also include cycloalkylidenes. Example cycloalkyl groups include cyclopropyl, cyclobutyl, cyclopentyl, cyclohexyl, cycloheptyl, cyclopentenyl, cyclohexenyl, cyclohexadienyl, cycloheptatrienyl, norbornyl, norpinyl, norcarnyl, adamantyl, and the like. Also included in the definition of cycloalkyl are moieties that have one or more aromatic rings fused (*i.e*., having a bond in common with) to the cycloalkyl ring, for example, benzo or thienyl derivatives of pentane, pentene, hexane, and the like. A cycloalkyl group containing a fused aromatic ring can be attached through any ring-forming atom including a ring-forming atom of the fused aromatic ring. A linking cycloalkyl group is referred to herein as "cycloalkylene."

As used herein, the term "cycloalkylalkyl" refers to an alkyl group substituted by a cycloalkyl group.

As used herein, the term "dialkylamino" refers to an amino group substituted by two alkyl groups.

As used herein, the term "dialkylaminocarbonyl" refers to a carbonyl group substituted by a dialkylamino group.

As used herein, the term "dialkylaminosulfonyl" refers to -SO₂-N(alkyl)₂.

As used herein, the term "halo" or "halogen" includes fluoro, chloro, bromo, and iodo.

As used herein, the term "haloalkoxy" refers to an -O-haloalkyl group. An example haloalkoxy group is OCF₃.

As used herein, the term "haloalkyl" refers to an alkyl group having one or more halogen substituents. Example haloalkyl groups include CF₃, C₂F₅, CHF₂, CCl₃, CHCl₂, C₂Cl₅, and the like.

As used herein, the term "halosulfanyl" refers to a sulfur group having one or more halogen substituents. Example halosulfanyl groups include pentahalosulfanyl groups such as SF₅.

As used herein, the term "heteroaryl" refers to an aromatic heterocycle having at least one heteroatom ring member such as sulfur, oxygen, or nitrogen. Heteroaryl groups include monocyclic and polycyclic (*e*.*g*., having 2, 3 or 4 fused rings) systems. Examples of heteroaryl groups include without limitation, pyridyl, pyrimidinyl, pyrazinyl, pyridazinyl, triazinyl, furyl, quinolyl, isoquinolyl, thienyl, imidazolyl, thiazolyl, indolyl, pyrryl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, benzothienyl, purinyl, carbazolyl, benzimidazolyl, indolinyl, and the like. In some embodiments, the heteroaryl group has from 1 to about 20 carbon atoms, and in further embodiments from about 3 to about 20 carbon atoms. In some embodiments, the heteroaryl group contains 3 to about 14, 4 to about 14, 3 to about 7, or 5 to 6 ring-forming atoms. In some embodiments, the heteroaryl group has 1 to about 4, 1 to about 3, or 1 to 2 heteroatoms. A linking heteroaryl group is referred to herein as "heteroarylene."

As used herein, the term "heteroarylalkyl" refers to alkyl substituted by heteroaryl and "heterocycloalkylalkyl" refers to alkyl substituted by heterocycloalkyl.

As used herein, the term "heteroaryloxy" refers to an -O-heteroaryl group. An example heteroaryloxy group is pyridin-2-yloxy or pyridin-3-yloxy.

As used herein, the term "heterocycloalkyl" refers to non-aromatic heterocycles including cyclized alkyl, alkenyl, and alkynyl groups where one or more of the ring-forming carbon atoms is replaced by a heteroatom such as an O, N, or S atom. Heterocycloalkyl groups include monocyclic and polycyclic (*e*.*g*., having 2, 3 or 4 fused rings) systems as well as spirocycles. Example "heterocycloalkyl" groups include morpholino, thiomorpholino, piperazinyl, tetrahydrofuranyl, tetrahydrothienyl, 2,3-dihydrobenzofuryl, 1,3-benzodioxole, benzo-1,4-dioxane, piperidinyl, pyrrolidinyl, isoxazolidinyl, isothiazolidinyl, pyrazolidinyl, oxazolidinyl, thiazolidinyl, imidazolidinyl, and the like. Ring-forming carbon atoms and heteroatoms of a heterocycloalkyl group can be optionally substituted by oxo or sulfido. Also included in the definition of heterocycloalkyl are moieties that have one or more aromatic rings fused (*i.e.,* having a bond in common with) to the nonaromatic heterocyclic ring, for example phthalimidyl, naphthalimidyl, and benzo derivatives of heterocycles. The heterocycloalkyl group can be attached through a ring-forming carbon atom or a ring-forming heteroatom. The heterocycloalkyl group containing a fused aromatic ring can be attached through any ring-forming atom including a ring-forming atom of the fused aromatic ring. In some embodiments, the heterocycloalkyl group has from 1 to about 20 carbon atoms, and in further embodiments from about 3 to about 20 carbon atoms. In some embodiments, the heterocycloalkyl group contains 3 to about 14, 4 to about 14, 3 to about 7, or 5 to 6 ring-forming atoms. In some embodiments, the heterocycloalkyl group has 1 to about 4, 1 to about 3, or 1 to 2 heteroatoms. In some embodiments, the heterocycloalkyl group contains 0 to 3 double or triple bonds. In some embodiments, the heterocycloalkyl group contains 0 to 2 double or triple bonds. A linking heterocycloalkyl group is referred to herein as "hctcrocycloalkylcnc."

As used herein, the term "heterocycloalkylalkyl" refers to an alkyl group substituted by a heterocycloalkyl group.

As used herein, the term "heterocyclyl" or "heterocycle" refers to a saturated or unsaturated cyclic group wherein one or more of the ring-forming atoms is a heteroatom such as O, S, or N. Heterocyclyl groups include mono- or polycyclic ring systems. Heterocyclyl groups can be aromatic (e.g., "heteroaryl") or non-aromatic (e.g., "heterocycloalkyl"). Heterocyclyl groups can be characterized as having 3-14 ring-forming atoms. In some embodiments, heterocyclyl groups can contain, in addition to at least one heteroatom, from about 1 to about 13, about 2 to about 10, or about 2 to about 7 carbon atoms and can be attached through a carbon atom or heteroatom. In further embodiments, the heteroatom can be oxidized (e.g., have an oxo or sulfido substituent) or a nitrogen atom can be quaternized. Examples of heterocyclyl groups include morpholino, thiomorpholino, piperazinyl, tetrahydrofuranyl, tetrahydrothienyl, 2,3-dihydrobenzofuryl, 1,3-benzodioxole, benzo-1,4-dioxane, piperidinyl, pyrrolidinyl, isoxazolidinyl, isothiazolidinyl, pyrazolidinyl, oxazolidinyl, thiazolidinyl, imidazolidinyl, and the like, as well as any of the groups listed below for "heteroaryl" and "heterocycloalkyl." Further example heterocycles include pyrimidinyl, phenanthridinyl, phenanthrolinyl, phenazinyl, phenothiazinyl, phenoxathiinyl, phenoxazinyl, phthalazinyl, piperazinyl, piperidinyl, 3,6-dihydropyridyl, 1,2,3,6-tetrahydropyridyl, 1,2,5,6-tetrahydropyridyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrazolyl, 6H-1,2,5-thia-diazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, xanthenyl, octahydro-isoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, quinazolinyl, quinolinyl, 4H-quinolizinyl, quinoxalinyl, quinuclidinyl, acridinyl, azocinyl, benzimidazolyl, benzofuranyl, benzothiofuranyl, benzo-thiophenyl, benzoxazolyl, benzthiazolyl, benztriazolyl, benztetrazolyl, benzisoxazolyl, benzisothiazolyl, benzimidazolinyl, methylenedioxyphenyl, morpholinyl, naphthyridinyl, deca-hydroquinolinyl, 2H,6H-1,5,2dithiazinyl, dihydrofuro[2,3-b]tetrahydrofuran, furanyl, furazanyl, carbazolyl, 4aH-carbazolyl, carbolinyl, chromanyl, chromenyl, cinnolinyl, imidazolidinyl, imidazolinyl, imidazolyl, 1H-indazolyl, indolenyl, indolinyl, indolizinyl, indolyl, 3H-indolyl, isobenzofuranyl, isochromanyl, isoindazolyl, isoindolinyl, isoindolyl, isoquinolinyl, isothiazolyl and isoxazolyl. Further examples of heterocycles include azetidin-1-yl, 2,5-dihydro-1H-pyrrol-1-yl, piperindin-1yl, piperazin-1-yl, pyrrolidin-1-yl, isoquinol-2-yl, pyridin-1-yl, 3,6-dihydropyridin-1-yl, 2,3-dihydroindol-1-yl, 1,3,4,9-tetrahydrocarbolin-2-yl, thieno[2,3-c]pyridin-6-yl, 3,4,10,10a-tetrahydro-1H-pyrazino[1,2-a]indol-2-yl, 1,2,4,4a,5,6-hexahydro-pyrazino[1,2-a]quinolin-3-yl, pyrazino[1,2-a]quinolin-3-yl, diazepan-1-yl, 1,4,5,6-tetrahydro-2H-benzo[f]isoquinolin-3-yl, 1,4,4a,5,6,10b-hexahydro-2H-benzo[f]isoquinolin-3-yl, 3,3a,8,8a-tetrahydro-1H-2-aza-cyclopenta[a]inden-2-yl, and 2,3,4,7-tetrahydro-1H-azepin-1-yl, azepan-1-yl.

As used herein, the term "heterocyclylalkyl" refers to an alkyl moiety substituted by a heterocarbocyclyl group. Example heterocarbocyclylalkyl groups include "heteroarylalkyl" (alkyl substituted by heteroaryl) and "heterocycloalkylalkyl" (alkyl substituted by heterocycloalkyl). In some embodiments, heterocyclylalkyl groups have from 3 to 24 carbon atoms in addition to at least one ring-forming heteroatom.

As used herein, the term "hydrocarbyl" refers to any moiety comprising only hydrogen and carbon atoms. Example "hydrocarbyl" groups include alkyl, alkenyl, alkynyl, cycloalkyl, cycloalkenyl, aryl, arylalkyl and arylalkenyl groups.

As used herein, the term "hydroxylalkyl" refers to an alkyl group substituted by hydroxyl.

The compounds described herein can be asymmetric (e.g., having one or more stereocenters). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms.

Resolution of racemic mixtures of compounds can be carried out by any of numerous methods known in the art. An example method includes fractional recrystallizaion using a chiral resolving acid which is an optically active, salt-forming organic acid. Suitable resolving agents for fractional recrystallization methods are, for example, optically active acids, such as the D and L forms of tartaric acid, diacetyltartaric acid, dibenzoyltartaric acid, mandelic acid, malic acid, lactic acid or the various optically active camphorsulfonic acids such as α-camphorsulfonic acid. Other resolving agents suitable for fractional crystallization methods include stereoisomerically pure forms of α-methylbenzylamine *(e.g.,* S and *R* forms, or diastereomerically pure forms), 2-phenylglycinol, norephedrine, ephedrine, N-methylephedrine, cyclohexylethylamine, 1,2-diaminocyclohexane, and the like.

Resolution of racemic mixtures can also be carried out by elution on a column packed with an optically active resolving agent (e.g., dinitrobenzoylphenylglycine). Suitable elution solvent composition can be determined by one skilled in the art.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H-, 2H- and 4H- 1,2,4-triazole, 1H- and 2H- isoindole, and 1Hand 2H-pyrazole. Tautomeric forms can be in equilibrium or sterically locked into one form by appropriate substitution.

Compounds of the invention further include hydrates and solvates, as well as anhydrous and non-solvated forms.

Compounds of the invention can also include all isotopes of atoms occurring in the intermediates or final compounds. Isotopes include those atoms having the same atomic number but different mass numbers. For example, isotopes of hydrogen include tritium and deuterium. the term, "compound," as used herein is meant to include all stereoisomers, geometric iosomers, tautomers, and isotopes of the structures depicted. All compounds, and pharmaceutically acceptable salts thereof, can be found together with other substances such as water and solvents (e.g. hydrates and solvates) or can be isolated.

In some embodiments, the compounds of the invention, and salts thereof, are substantially isolated. By "substantially isolated" is meant that the compound is at least partially or substantially separated from the environment in which is was formed or detected. Partial separation can include, for example, a composition enriched in the compound of the invention. Substantial separation can include compositions containing at least about 50%, at least about 60%, at least about 70%, at least about 80%, at least about 90%, at least about 95%, at least about 97%, or at least about 99% by weight of the compound of the invention, or salt thereof. Methods for isolating compounds and their salts are routine in the art.

The expressions, "ambient temperature" and "room temperature," as used herein, are understood in the art, and refer generally to a temperature, *e.g* a reaction temperature, that is about the temperature of the room in which the reaction is carried out, for example, a temperature from about 20 °C to about 30 °C.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The present invention also includes pharmaceutically acceptable salts of the compounds described herein. As used herein, "pharmaceutically acceptable salts" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention include the conventional non-toxic salts of the parent compound formed, for example, from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile (MeCN) are preferred. Lists of suitable salts are found in Remington's Pharmaceutical Sciences, 17th ed., Mack Publishing Company, Easton, Pa., 1985, p. 1418 and Journal of Pharmaceutical Science, 66, 2 (1977), each of which is incorporated herein by reference in its entirety.

The present invention also includes prodrugs of the compounds described herein. As used herein, "prodrugs" refer to any covalently bonded carriers which release the active parent drug when administered to a mammalian subject. Prodrugs can be prepared by modifying functional groups present in the compounds in such a way that the modifications are cleaved, either in routine manipulation or *in vivo,* to the parent compounds. Prodrugs include compounds wherein hydroxyl, amino, sulfhydryl, or carboxyl groups are bonded to any group that, when administered to a mammalian subject, cleaves to form a free hydroxyl, amino, sulfhydryl, or carboxyl group respectively. Examples of prodrugs include, but are not limited to, acetate, formate and benzoate derivatives of alcohol and amine functional groups in the compounds of the invention. Preparation and use of prodrugs is discussed in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987, both of which are hereby incorporated by reference in their entirety.

### Synthesis

Agents for use in the methods of the invention, including salts thereof, can be prepared using known organic synthesis techniques and can be synthesized according to any of numerous possible synthetic routes.

The reactions for preparing the agents can be carried out in suitable solvents which can be readily selected by one of skill in the art of organic synthesis. Suitable solvents can be substantially nonreactive with the starting materials (reactants), the intermediates, or products at the temperatures at which the reactions are carried out, *e.g.,* temperatures which can range from the solvent's freezing temperature to the solvent's boiling temperature. A given reaction can be carried out in one solvent or a mixture of more than one solvent. Depending on the particular reaction step, suitable solvents for a particular reaction step can be selected by the skilled artisan.

Preparation of the compounds can involve the protection and deprotection of various chemical groups. The need for protection and deprotection, and the selection of appropriate protecting groups, can be readily determined by one skilled in the art. The chemistry of protecting groups can be found, for example, in T.W. Green and P.G.M. Wuts, Protective Groups in Organic Synthesis, 3rd. Ed., Wiley & Sons, Inc., New York (1999), which is incorporated herein by reference in its entirety.

Reactions can be monitored according to any suitable method known in the art. For example, product formation can be monitored by spectroscopic means, such as nuclear magnetic resonance spectroscopy (e.g., ¹H or ¹³C) infrared spectroscopy, spectrophotometry (e.g., UV-visible), or mass spectrometry, or by chromatography such as high performance liquid chromatography (HPLC) or thin layer chromatography.

Agents for use in the method of the invention can be prepared according to numerous preparatory routes known in the literature. For example, compounds of Formula I, and embodiments thereof, can be prepared by the methods set forth in U.S. Patent Publ. No. US 20070135461, published June 14, 2007 (Appl. Ser. No. 11/637,545, filed December 12, 2006), and in U.S. Patent Appl. Ser. No. 12/137,892, filed June 12, 2008; U.S. Patent Appl. No. 12/138,082, filed June 12, 2008, each of which is incorporated herein by reference in its entirety. Compounds of Formula IV, and embodiments thereof, can be prepared by the methods set forth in U.S. Patent Publ. No. US 20060106020, published May 18, 2006 (Appl. Ser. No. 11/115,702 filed April 27, 2005), which is incorporated herein by reference in its entirety. Compounds of Formulas V and VI, and embodiments thereof, can be prepared by the methods set forth in U.S. Patent Publ. No. US 20060183906, published August 17, 2006 (Appl. Ser. No. 11/313,394, filed December 21, 2005), which is incorporated herein by reference in its entirety. Compounds of Formula VII, and embodiments thereof, can be prepared by the methods set forth in U.S. Patent Publ. No. US 20070149506, published June 28, 2007 (Appl. Ser. No. 11/524,641, filed September 21, 2006), which is incorporated herein by reference in its entirety. Compounds of Formula VIII, and embodiments thereof, can be prepared by the methods set forth in U.S. Patent Publ. No. US 20080188500, published August 7, 2008 (Appl. Ser. No. 11/961,424, filed December 20, 2007), which is incorporated herein by reference in its entirety.

Compounds of Formulas II and III, and embodiments thereof, can be prepared as set forth in U.S. Prov. Appl. No. 61/035,662, filed March 11, 2008, which is incorporated herein by reference in its entirety, and as detailed below. Compounds of Formula IX, and embodiments thereof, can be prepared as set forth in U.S. Prov. Appl. No. 60/988,606, filed November 16, 2007, which is incorporated herein by reference in its entirety, and as detailed below.

3-(4-(7H-Pyrrolo[2,3-d]pyrimidin-4-yl)-1H-pyrazol-1-yl)-3-(3-hydroxycyclopentyl) propanenitrile, 3-(4-(7H-pyrrolo[2,3-d]pyrimidin-4-yl)-1H-pyrazol-1-yl)-3-(2-hydroxycyclopentyl) propanenitrile, and 3-(4-(7H-pyrrolo[2,3-d]pyrimidin-4-yl)-1H-pyrazol-1-yl)-3-(3-oxocyclopentyl) propanenitrile, can be prepared as set forth in U.S. Patent Appl. Ser. No. 12/137,883, filed June 12, 2008, which is incorporated herein by reference in its entirety. (R)-3-(4-(7H-Pyrrolo[2,3-d]pyrimidin-4-yl)-1H-pyrazol-1-yl)-3-cyclopentylpropanenitrile maleic acid salt, (R)-3-(4-(7H-pyrrolo[2,3-d] pyrimidin-4-yl)-1H-pyrazol-1-yl)-3-cyclopentylpropanenitrile sulfuric acid salt, and (R)-3-(4-(7H-pyrrolo[2,3-d]pyrimidin-4-yl)-1H-pyrazol-1-yl)-3-cyclopentylpropanenitrile phosphoric acid salt can be prepared as set forth in U.S. Patent Appl. Ser. No. 12/137,892, filed June 12, 2008, which is incorporated herein by reference in its entirety. Example synthetic methods for preparing certain agents, for use in the methods of the invention, are provided in the Schemes below.

### Kits and Uses

The present invention also includes pharmaceutical kits useful, for example, in the treatment or prevention of a dry eye disorder comprising a pharmaceutical or ophthalmic composition comprising a therapeutically effective amount of an agent such as those described above; and instructions comprising a direction to administer the composition to a patient in need thereof. The direction may be for a physician to administer the agent to the patient or a direction for self-administration of the agent. The instructions may be in paper form, such as a product label or insert. Alternatively, the instructions may be stored electronically, such as on a website. Such kits can further include, if desired, one or more of various conventional pharmaceutical kit components, such as, for example, containers with one or more pharmaceutically acceptable carriers, additional containers, etc., as will be readily apparent to those skilled in the art. Instructions, either as inserts or as labels, indicating quantities of the components to be administered, guidelines for administration, and/or guidelines for mixing the components, can also be included in the kit.

In a further aspect, the present invention provides use of an agent as described in the embodiments above, for use in the preparation of a medicament for treatment of a dry eye disorder. In another aspect, the present invention provides an agent for use in treatment of a dry eye disorder in an individual.

The invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results. The compounds of the Examples have been found to be JAK inhibitors according to at least one assay described herein.

### EXAMPLES

### Section A: Animal Models and Assay Methods

### Example A: Animal Models for the Treatment of Dry Eye, Uveitis, and Conjunctivitis

Agents may be evaluated in one or more prcclinical models of dry eye known to those schooled in the art including, but not limited to, the rabbit concanavalin A (ConA) lacrimal gland model, the scopolamine mouse model (subcutaneous or transdermal), the Botulinumn mouse lacrimal gland model, or any of a number of spontaneous rodent auto-immune models that result in ocular gland dysfunction (e.g. NOD-SCID, MRL/lpr, or NZB/NZW) (Barabino et al., Experimental Eye Research 2004, 79, 613-621 and Schrader et al., Developmental Opthalmology, Karger 2008, 41, 298-312, each of which is incorporated herein by reference in its entirety). Endpoints in these models may include histopathology of the ocular glands and eye (cornea, etc.) and possibly the classic Schirmer test or modified versions thereof (Barabino et al.) which measure tear production. Activity may be assessed by dosing via multiple routes of administration (e.g. systemic or topical) which may begin prior to or after measurable disease exists.

Agents may be evaluated in one or more preclinical models of uveitis known to those schooled in the art. These include, but are not limited to, models of experimental autoimmune uveitis (EAU) and endotoxin induced uveitis (EIU). EAU experiements may be performed in the rabbit, rat, or mouse and may involve passive or activate immunization. For instance, any of a number or retinal antigens may be used to sensitize animals to a relevant immunogen after which animals may be challenged ocuarly with the same antigen. The EIU model is more acute and involves local or systemic administration of lipopolysaccaride at sublethal doses. Endpoints for both the EIU and EAU models may include fundoscopic exam, histopathology amongst others. These models are reviewed by Smith et al. (Immunology and Cell Biology, 1998, 76, 497-512, which is incorporated herein by reference in its entirety). Activity is assessed by dosing via multiple routes of administration (e.g. systemic or topical) which may begin prior to or after measurable disease exists. Some models listed above may also develop scleritis/episcleritis, chorioditis, cyclitis, or iritis and are therefore useful in investigating the potential activity of compounds for the therapeutic treatment of these diseases.

Agents may also be evaluated in one or more preclinical models of conjunctivitis known those schooled in the art. These include, but are not limited to, rodent models utilizing guinea-pig, rat, or mouse. The guinea-pig models include those utilizing active or passive immunization and/or immune challenge protocols with antigens such as ovalbumin or ragweed (reviewed in Groneberg, D.A., et al., Allergy 2003, 58, 1101-1113, which is incorporated herein by reference in its entirety). Rat and mouse models are similar in general design to those in the guinea-pig (also reviewed by Groneberg). Activity may be assessed by dosing via multiple routes of administration (e.g. systemic or topical) which may begin prior to or after measurable disease exists. Endpoints for such studies may include, for example, histological, immunological, biochemical, or molecular analysis of ocular tissues such as the conjunctiva.

### Example B: In vitro JAK Kinase Assay

Compounds herein are tested for inhibitory activity of JAK targets according to the following *in vitro* assay described in Park et al., Analytical Biochemistry 1999, 269, 94-104. The catalytic domains of human JAK1 (a.a. 837-1142), Jak2 (a.a. 828-1132) and Jak3 (a.a. 781-1124) with an N-terminal His tag are expressed using baculovirus in insect cells and purified. The catalytic activity of JAK1, JAK2 or JAK3 is assayed by measuring the phosphorylation of a biotinylated peptide. The phosphorylated peptide is detected by homogenous time resolved fluorescence (HTRF). IC₅₀s of compounds is measured for each kinase in the reactions that contain the enzyme, ATP and 500 nM peptide in 50 mM Tris (pH 7.8) buffer with 100 mM NaCl, 5 mM DTT, and 0.1 mg/mL (0.01%) BSA. The ATP concentration in the reactions is 90 µM for Jak1, 30 µM for Jak2 and 3 µM for Jak3. Reactions are carried out at room temperature for 1 hr and then stopped with 20 µL 45 mM EDTA, 300 nM SA-APC, 6 nM Eu-Py20 in assay buffer (Perkin Elmer, Boston, MA). Binding to the Europium labeled antibody takes place for 40 minutes and HTRF signal is measured on a Fusion plate reader (Perkin Elmer, Boston, MA). Compounds having an IC₅₀ of 10 µM or less for any of the above-mentioned JAK targets are considered active.

### Example C: Cellular Assays

One or more compounds herein are tested for inhibitory activity of JAK targets according to at least one of the following cellular assays.

Cancer cell lines dependent on cytokines and hence JAK/STAT signal transduction, for growth, are plated at 6000 cells per well (96 well plate format) in RPMI 1640, 10% FBS, and 1 nG/mL of appropriate cytokine. Compounds are added to the cells in DMSO/media (final concentration 0.2% DMSO) and incubated for 72 hours at 37 °C, 5% CO₂. The effect of compound on cell viability is assessed using the CellTiter-Glo Luminescent Cell Viability Assay (Promega) followed by TopCount (Perkin Elmer, Boston, MA) quantitation. Potential off-target effects of compounds are measured in parallel using a non-JAK driven cell line with the same assay readout. Compounds having an IC₅₀ of 10 µM or less with selectivity for JAK driven proliferation are considered active. All experiments are performed in duplicate.

The above cell lines can also be used to examine the effects of compounds on phosphorylation of JAK kinases or potential downstream substrates such as STAT proteins, Akt, Shp2, or Erk. These experiments can be performed following an overnight cytokine starvation, followed by a brief preincubation with compound (2 hours or less) and cytokine stimulation of approximately 1 hour or less. Proteins are then extracted from cells and analyzed by techniques familiar to those schooled in the art including Western blotting or ELISAs using antibodies that can differentiate between phosphorylated and total protein. These experiments can utilize normal or cancer cells to investigate the activity of compounds on tumor cell survival biology or on mediators of inflammatory disease. For example, with regards to the latter, cytokines such as IL-6, IL-12, IL-23, or IFN can be used to stimulate JAK activation resulting in phosphorylation of STAT protein(s) and potentially in transcriptional profiles (assessed by array or qPCR technology) or production and/or secretion of proteins, such as IL-17. The ability of compounds to inhibit these cytokine mediated effects can be measured using techniques common to those schooled in the art.

Compounds herein can also be tested in cellular models designed to evaluate their potency and activity against mutant JAKs, for example, the JAK2V617F mutation found in myeloid proliferative disorders. These experiments often utilize cytokine dependent cells of hematological lineage (e.g. BaF/3) into which the wild-type or mutant JAK kinases are ectopically expressed (James, C., et al, Nature 434:1144-1148; Staerk, J., et al. JBC 280:41893-41899). Endpoints include the effects of compounds on cell survival, proliferation, and phosphorylated JAK, STAT, Akt, or Erk proteins.

Certain compounds herein can be evaluated for their activity inhibiting T-cell proliferation. Such as assay can be considered a second cytokine (*i.e.* JAK) driven proliferation assay and also a simplistic assay of immune suppression or inhibition of immune activation. The following is a brief outline of how such experiments can be performed. Peripheral blood mononuclear cells (PBMCs) are prepared from human whole blood samples using Ficoll Hypaque separation method and T-cells (fraction 2000) can be obtained from PBMCs by elutriation. Freshly isolated human T-cells can be maintained in culture medium (RPM1 1640 supplemented with 10% fetal bovine serum, 100 U/ml penicillin, 100 µg/ml streptomycin) at a density of 2 x 10⁶ cells/ml at 37 °C for up to 2 days. For IL-2 stimulated cell proliferation analysis, T-cells are first treated with Phytohemagglutinin (PHA) at a final concentration of 10 µg/mL for 72h. After washing once with PBS, 6000 cells/well are plated in 96-well plates and treated with compounds at different concentrations in the culture medium in the presence of 100 U/mL human IL-2 (ProSpec-Tany TechnoGene; Rehovot, Israel). The plates are incubated at 37 °C for 72h and the proliferation index is assessed using CellTiter-Glo Luminescent reagents following the manufactory suggested protocol (Promega; Madison, WI).

### Example D: Murine Skin Contact Delayed Hypersensitivity Response Test

Compounds herein can also be tested for their efficacies (of inhibiting JAK targets) in the T-cell driven murine delayed hypersensitivity test model. The murine skin contact delayed-type hypersensitivity (DTH) response is considered to be a valid model of clinical contact dermatitis, and other T-lymphocyte mediated immune disorders of the skin, such as psoriasis (Immunol Today. 1998 Jan; 19(1):37-44). Murine DTH shares multiple characteristics with psoriasis, including the immune infiltrate, the accompanying increase in inflammatory cytokines, and keratinocyte hyperproliferation. Furthermore, many classes of agents that are efficacious in treating psoriasis in the clinic are also effective inhibitors of the DTH response in mice (Agents Actions. 1993 Jan;38(1-2):116-21).

On Day 0 and 1, Balb/c mice are sensitized with a topical application, to their shaved abdomen with the antigen 2,4,dinitro-fluorobenzene (DNFB). On day 5, ears are measured for thickness using an engineer's micrometer. This measurement is recorded and used as a baseline. Both of the animals' ears are then challenged by a topical application of DNFB in a total of 20 µL (10 µL on the internal pinna and 10 µL on the external pinna) at a concentration of 0.2%. Twenty-four to seventy-two hours after the challenge, ears are measured again. Treatment with the test compounds is given throughout the sensitization and challenge phases (day -1 to day 7) or prior to and throughout the challenge phase (usually afternoon of day 4 to day 7). Treatment of the test compounds (in different concentration) is administered either systemically or topically (topical application of the treatment to the ears). Efficacies of the test compounds are indicated by a reduction in ear swelling comparing to the situation without the treatment. Compounds causing a reduction of 20% or more are considered efficacious. In some experiments, the mice are challenged but not sensitized (negative control).

The inhibitive effect (inhibiting activation of the JAK-STAT pathways) of the test compounds can be confirmed by immunohistochemical analysis. Activation of the JAK-STAT pathway(s) results in the formation and translocation of functional transcription factors. Further, the influx of immune cells and the increased proliferation of keratinocytes should also provide unique expression profile changes in the ear that can be investigated and quantified. Formalin fixed and paraffin embedded ear sections (harvested after the challenge phase in the DTH model) are subjected to immunohistochemical analysis using an antibody that specifically interacts with phosphorylated STAT3 (clone 58E12, Cell Signaling Technologies). The mouse ears are treated with test compounds, vehicle, or dexamethasone (a clinically efficacious treatment for psoriasis), or without any treatment, in the DTH model for comparisons. Test compounds and the dexamethasone may produce similar transcriptional changes both qualitatively and quantitatively, and both the test compounds and dexamethasone can reduce the number of infiltrating cells. Both systemically and topical administration of the test compounds can produce inhibitive effects, *i.e.,* reduction in the number of infiltrating cells and inhibition of the transcriptional changes.

### Example E: In vivo anti-inflammatory activity

Compounds herein can be evaluated in rodent or non-rodent models designed to replicate a single or complex inflammation response. For instance, rodent models of arthritis can be used to evaluate the therapeutic potential of compounds dosed preventatively or therapeutically. These models include but are not limited to mouse or rat collagen-induced arthritis, rat adjuvant-induced arthritis, and collagen antibody-induced arthritis. Autoimmune diseases including, but not limited to, multiple sclerosis, type I-diabetes mellitus, uveoretinitis, thyroditis, myasthenia gravis, immunoglobulin nephropathies, myocarditis, airway sensitization (asthma), lupus, or colitis may also be used to evaluate the therapeutic potential of compounds herein. These models are well established in the research community and are familiar to those schooled in the art (Current Protocols in Immunology, Vol 3., Coligan, J.E. et al, Wiley Press.; Methods in Molecular Biology: Vol. 225, Inflammation Protocols., Winyard, P.G. and Willoughby, D.A., Humana Press, 2003.).

### SECTION B. COMPOUND EXAMPLES

### Example 67: (3R)- and (3S)-3-Cyclopentyl-3-[4-(7H-pyrrolo[2,3-d]pyrimidin-4-yl)-1H-pyrazol-1-yl]propanenitrile

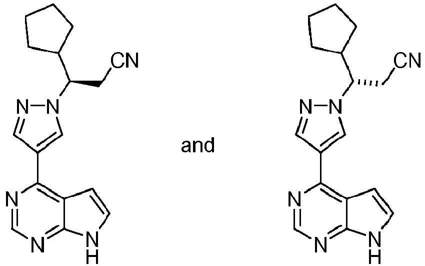

### Step 1. (2E)- and (2Z)-3-Cyclopentylacrylonitrile

To a solution of 1.0 M potassium tert-butoxide in THF (235 mL) at 0 °C was added dropwise a solution of diethyl cyanomethylphosphonate (39.9 mL, 0.246 mol) in THF (300 mL). The cold bath was removed and the reaction was warmed to room temperature followed by recooling to 0 °C, at which time a solution of cyclopentanecarbaldehyde (22.0 g, 0.224 mol) in THF (60 mL) was added dropwise. The bath was removed and the reaction warmed to ambient temperature and stirred for 64 hours. The mixture was partitioned between diethyl ether and water, the aqueous was extracted with three portions of ether, followed by two portions of ethyl acetate. The combined extracts were washed with brine, then dried over sodium sulfate, filtered and concentrated *in vacuo* to afford a mixture containing 24.4 g of olefin isomers which was used without further purification (89%).
¹H NMR (400 MHz, CDCl₃): δ 6.69 (dd, 1H, trans olefin), 6.37 (t, 1H, cis olefin), 5.29 (dd, 1H, trans olefin), 5.20 (d, 1H, cis olefin), 3.07-2.95 (m, 1H, cis product), 2.64-2.52 (m, 1H, trans product), 1.98-1.26 (m, 16H).

### Step 2. (3R)- and (3S)-3-Cyclopentyl-3-[4-(7-[2-(trimethylsilyl)ethoxy]methyl-7H-pyrrolo[2,3-d]-pyrimidin-4-yl)-1H-pyrazol-1-yl]propanenitrile

To a solution of 4-(1H-pyrazol-4-yl)-7-[2-(trimethylsilyl)ethoxy]methyl-7H-pyrrolo[2,3-d]-pyrimidine (15.0 g, 0.0476 mol) in ACN (300 mL) was added 3-cyclopentylacrylonitrile (15 g, 0.12 mol) (as a mixture of cis and trans isomers), followed by DBU (15 mL, 0.10 mol). The resulting mixture was stirred at room temperature overnight. The ACN was evaporated. The mixture was diluted with ethyl acetate, and the solution was washed with 1.0 N HCl. The aqueous layer was back-extracted with three portions of ethyl acetate. The combined organic extracts were washed with brine, dried over sodium sulfate, filtered and concentrated. The crude product was purified by silica gel chromatography (gradient of ethyl acetate/hexanes) to yield a viscous clear syrup, which was dissolved in ethanol and evaporated several times to remove ethyl acetate, to afford 19.4 g of racemic adduct (93%). The enantiomers were separated by preparative-HPLC, (OD-H, 15% ethanol/hexanes) and used separately in the next step to generate their corresponding final product. The final products (see Step 3) stemming from each of the separated enantiomers were found to be active JAK inhibitors; however, the final product stemming from the second peak to elute from the preparative-HPLC was more active than its enantiomer.
¹H NMR (300 MHz, CDCl₃): δ 8.85 (s, 1H), 8.32 (s, 2H), 7.39 (d, 1H), 6.80 (d, 1H), 5.68 (s, 2H), 4.26 (dt, 1H), 3.54 (t, 2H), 3.14 (dd, 1H), 2.95 (dd, 1H), 2.67-2.50 (m, 1H), 2.03-1.88 (m, 1H), 1.80-1.15 (m, 7H), 0.92 (t, 2H), -0.06 (s, 9H); MS(ES):437 (M+1).

### Step 3. (3R)- and (3S)-3-Coclopentyl-3-[4-(7H-pyrrolo[2,3-d]pyrimidin-4-yl)-1H-pyrazol-1-yl]propanenitrile

To a solution of 3-cyclopentyl-3-[4-(7-[2-(trimethylsilyl)ethoxy]methyl-7H-pyrrolo[2,3-d]-pyrimidin-4-yl)-1H-pyrazol-1-yl]propanenitrile (6.5 g, 0.015 mol, R or S enantiomer as isolated above) in DCM (40 mL) was added TFA (16 mL) and this was stirred for 6 hours. The solvent and TFA were removed *in vacuo.* The residue was dissolved in DCM and concentrated using a rotary evaporator two further times to remove as much as possible of the TFA. Following this, the residue was stirred with ethylenediamine (4 mL, 0.06 mol) in methanol (30 mL) overnight. The solvent was removed *in vacuo,* water was added and the product was extracted into three portions of ethyl acetate. The combined extracts were washed with brine, dried over sodium sulfate, decanted and concentrated to afford the crude product which was purified by flash column chromatography (eluting with a gradient of methanol/DCM). The resulting mixture was further purified by preparative-HPLC/MS (C18 eluting with a gradient of ACN/H₂O containing 0.15% NH₄OH) to afford product (2.68 g, 58%).
¹H NMR (400 MHz, D₆-dmso): δ 12.11 (br s, 1H), 8.80 (s, 1H), 8.67 (s, 1H), 8.37 (s, 1H), 7.60 (d, 1H), 6.98 (d, 1H), 4.53 (dt, 1H), 3.27 (dd, 1H), 3.19 (dd, 1H), 2.48-2.36 (m, 1H), 1.86-1.76 (m, 1H), 1.68-1.13 (m, 7H); MS(ES):307(M+1).

### Example 1. {1-(ethylsulfonyl)-3-[4-(7H-pyrrolo[2,3-d]pyrimidin-4-yl)-1H-pyrazol-1-yl]azetidin-3-yl}acetonitrile trifluoroacetic acid salt

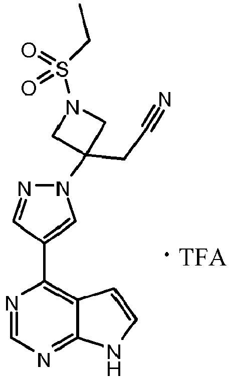

### Step 1. tert-butyl 3-(cyanomethylene)azetidine-1-carboxylate

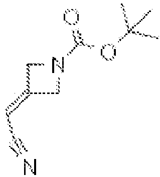

To a suspension of sodium hydride (60% dispersion in mineral oil, 0.257 g, 6.42 mmol) in tetrahydrofuran (32 mL) at 0 °C under a nitrogen atmosphere was added diethyl cyanomethylphosphonate (1.19 g, 6.72 mmol) (purchased from Aldrich). The reaction was then stirred for 45 minutes at room temperature. A solution of tert-butyl 3-oxoazetidine-1-carboxylate (1.00 g, 5.84 mmol) (purchased from Alfa Aesar) in tetrahydrofuran (8.8 mL) was introduced dropwise and the mixture was stirred for 16 hours. Brine and ethyl acetate were added and the layers separated. The aqueous layer was extracted with three portions of ethyl acetate. The combined extracts were dried over sodium sulfate, filtered and concentrated to afford product, used without further purification in Step 2 (1.12 g, 99%).
¹H NMR (300 MHz, CDCl₃): δ 5.38 (p, 1H), 4.73-4.68 (m, 2H), 4.64-4.59 (m, 2H), 1.46 (s, 9H).

### Step 2. tert-butyl 3-(cyanomethyl)-3-[4-(7-[2-(trimethylsilyl)ethoxy]methyl-7H-pyrrolo[2,3-d]pyrimidin-4-yl)-1H-pyrazol-1-yl]azetidine-1-carboxylate

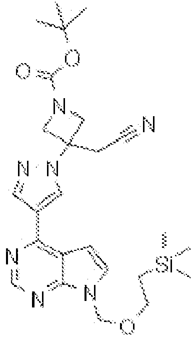

To a solution of 4-(1H-pyrazol-4-yl)-7-[2-(trimethylsilyl)ethoxy]methyl-7H-pyrrolo[2,3-d]pyrimidine (4.61 g, 14.6 mmol) (prepared according to the method of WO 2007/070514 in Example 65, Step 2) andtert-butyl 3-(cyanomethylene)azetidine-1-carboxylate (2.84 g, 14.6 mmol) in acetonitrile (100 mL) was added 1,8-diazabicyclo[5.4.0]undec-7-ene (2.19 mL, 14.6 mmol). The reaction was stirred at room temperature for 16 hours. The acetonitrile was *removed in vacuo* and the residue was dissolved in ethyl acetate. This solution was sequentially washed with 1N HCl and brine, dried over sodium sulfate, filtered and concentrated. The residue was purified by flash column chromatography, eluting with 80% ethyl acetate/hexanes to afford desired product (5.36 g, 72%).
¹H NMR (300 MHz, CDCl₃): δ 8.86 (s, 1H), 8.44 (s, 1H), 8.34 (s, 1H), 7.42 (d, 1H), 6.80 (d, 1H), 5.68 (s, 2H), 4.54 (d, 2H), 4.29 (d, 2H), 3.59-3.51 (m, 2H), 3.33 (s, 2H), 1.47 (s, 9H), 0.96-0.89 (m, 2H), -0.06 (s, 9H); LCMS (M+H)⁺: 510.2.

### Step 3. 3-[4-(7-[2-(trimethylsilyl)ethoxy]methyl-7H-pyrrolo[2,3-d]pyrimidin-4-yl)-1H-pyrazol-1-yl]azetidin-3-ylacetonitrile

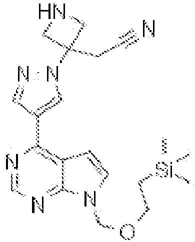

To a solution of tert-butyl 3-(cyanomethyl)-3-[4-(7-[2-(trimethylsilyl)ethoxy]methyl-7H-pyrrolo[2,3-d]pyrimidin-4-yl)-1H-pyrazol-1-yl]azetidine-1-carboxylate (5.36 g, 10.5 mmol) in 1,4-dioxane (100 mL) was added 4.00 M of hydrogen chloride in 1,4-dioxane (40 mL, 160 mmol) and the mixture was stirred at room temperature for 16 hours. The reaction was poured into saturated sodium bicarbonate solution sufficient to neutralize. The product was extracted with three portions of ethyl acetate. The combined extracts were washed with brine, dried over sodium sulfate, filtered and concentrated to afford product which was used without further purification (3.0 g, 69%).
¹H NMR (400 MHz, CDCl₃): δ 8.85 (s, 1H), 8.42 (s, 1H), 8.32 (s, 1H), 7.41 (d, 1H), 6.80 (d, 1H), 5.68 (s, 2H), 4.30 (d, 2H), 3.88 (d, 2H), 3.58-3.51 (m, 2H), 3.42 (s, 2H), 0.96-0.89 (m, 2H), -0.06 (s, 9H); LCMS (M+H)⁺: 410.2.

### Step 4. 1-(ethylsulfonyl)-3-[4-(7-[2-(trimethylsilyl)ethoxy]methyl-7H-pyrrolo[2,3-d]pyrimidin-4-yl)-1H-pyrazol-1-yl]azetidin-3-ylacetonitrile

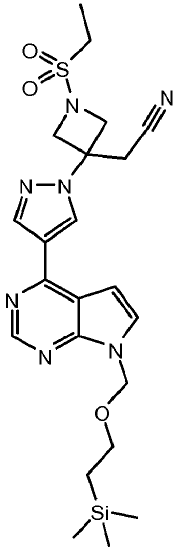

To a solution of 3-[4-(7-[2-(trimethylsilyl)ethoxy]methyl-7H-pyrrolo[2,3-d]pyrimidin-4-yl)-1H-pyrazol-1-yl]azetidin-3-ylacetonitrile (0.100 g, 0.244 mmol) in tetrahydrofuran (2 mL) containing N,N-diisopropylethylamine (0.085 mL, 0.49 mmol) was added ethanesulfonyl chloride (0.023 mL, 0.24 mmol). After stirring for 1.5 hours, the reaction mixture was poured into dilute HCl and extracted with three portions of ethyl acetate. The combined extracts were washed with brine, dried over sodium sulfate, decanted and concentrated to afford product, used without further purification in Step 5 (111 mg, 91%).
¹H NMR (300 MHz, CDCl₃): δ 8.86 (s, 1H), 8.63 (s, 1H), 8.35 (s, 1H), 7.45 (d, 1H), 6.83 (d, 1H),
5.68 (s, 2H), 4.63 (d, 2H), 4.26 (d, 2H), 3.54 (t, 2H), 3.42 (s, 2H), 3.09 (q, 2H), 1.41 (t, 3H), 0.92 (t, 2H), -0.06 (s, 9H); LCMS (M+H)⁺: 502.1.

### Step 5. 1-(ethylsulfonyl)-3-[4-(7H-pyrrolo[2,3-d]pyrimidin-4-yl)-1H-pyrazol-1-yl]azetidin-3-ylacetonitrile trifluoroacetate salt

To a solution of 1-(ethylsulfonyl)-3-[4-(7-[2-(trimethylsilyl)ethoxy]methyl-7H-pyrrolo[2,3-d]pyrimidin-4-yl)-1H-pyrazol-1-yl]azetidin-3-ylacetonitrile (0.111 g, 0.22 mmol) in methylene chloride (3 mL) was added trifluoroacetic acid (2 mL) and the solution was stirred for 1.5 hours. The solvents were removed *in vacuo* and the residue was dissolved in methanol (3 mL) and ethylenediamine (0.1 mL) was added. After stirring for 3 hours, the volume was reduced *in vacuo* and the product was purified by preparative-HPLC/MS, (SunFire C18 column, eluting with a gradient of MeCN/H₂O containing 0.1% TFA) to afford the product as the trifluoroacetic acid salt (50 mg, 47%).
¹H NMR (400 MHz, d₆-dmso): δ 9.03 (s, 1H), 8.83 (s, 1H), 8.56 (s, 1H), 7.77 (s, 1H), 7.22 (s, 1H), 4.59 (d, 2H), 4.26 (d, 2H), 3.71 (s, 2H), 3.25 (q, 2H), 1.24 (t, 3H); LCMS (M+H)⁺: 372.1.

Alternatively, the deprotection and sulfonylation steps could be performed in the reverse order, as in Example 2.

### Example 52. cis and trans-3-(cyanomethyl)-3-[4-(7H-pyrrolo[2,3-d]pyrimidin-4-yl)-1H-pyrazol-1-yl] cyclobutanecarbonitrile

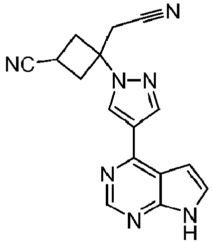

### Step 1. 3-oxocyclobutanecarbonitrile.

A mixture of water (40 mL, 2 mol) and 1,4-dioxane (100 mL, 1 mol), 3-methylenecyclobutanecarbonitrile (3.30 g, 0.0354 mol) (commercially available from Bepharma Ltd., China), and 0.2 M of osmium tetraoxide in water (0.7 mL) was stirred for 5 min, during which time the mixture became brown. While the temperature was maintained at room temperature, sodium periodate (15.9 g, 0.0744 mol) was added in portions over a period of 30 min. The mixture was stirred for an additional 1.5 h, then extracted with dichloromethane. The combined organic layers were dried over magnesium sulfate and concentrated to give a solid (2.04 g, 60.54%). ¹H NMR (300 MHz, CDCl₃): δ 3.58 (4H, m), 3.25 (1H, m) ppm.

### Step 2. 3-(cyanomethylene)cyclobutanecarbonitrile

To a solution of 1 M of potassium tert-butoxide in THF (67.4 mL) at 0 °C was added dropwise a solution of diethyl cyanomethylphosphonate (11.4 mL, 0.0706 mol) in tetrahydrofuran (100 mL, 1 mol). The reaction mixture was warmed up to room temperature and cooled to 0 °C again. To the resulting mixture, a solution of 3-oxocyclobutanecarbonitrile (6.10 g, 0.0641 mol) in tetrahydrofuran (20 mL, 0.2 mol) was added. The reaction mixture was allowed to warm up to room temperature and stirred for 2 hours. After quenching with water, the mixture was extracted with EtOAc. The combined organic layers were dried and concentrated. The residue was purified by flash silica gel column, eluting with 0-10% MeOH/dichloromethane to give the titled product (5.40 g, 71.26%). LCMS (M+Na) 141.3. ¹H NMR (400 MHz, CDCl₃): δ 5.30 (1H, m), 3.40 (2H, m), 3.14 (3H, m) ppm.

### Step 3. 3-(cyanomethyl)-3-[4-(7-[2-(trimethylsilyl)ethoxy]methyl-7H-pyrrolo[2,3-d]pyrimidin-4-yl)-1H-pyrazol-1-yl]cyclobutanecarbonitrile

3-(Cyanomethylene)cyclobutanecarbonitrile (120 mg, 0.0010 mol) was combined with 4-(1H-pyrazol-4-yl)-7-[2-(trimethylsilyl)ethoxy]methyl-7H-pyrrolo[2,3-d]pyrimidine (0.1 g, 0.0003 mol) in acetonitrile (2 mL, 0.04 mol) and 1,8-diazabicyclo[5.4.0]undec-7-ene (6 µL, 0.00004 mol) under nitrogen. The mixture was stirred at room temperature over the weekend. After evaporation to dryness, the crude mixture was purified by flash column, eluting with 0 to 10% MeOH in dichloromethane, to give the desired product. LCMS (M+H) 434.4.

### Step 4. 3-(cyanomethyl)-3-[4-(7H-pyrrolo[2,3-d]pyrimidin-4-yl)-1H-pyrazol-1-yl]cyclobutanecarbonitrile.

A 500 mL round bottom flask fitted with stir bar, condenser, and nitrogen inlet, was charged with acetonitrile (16.3 mL, 0.311 mol), water (1.4 mL, 0.078 mol) and 3-(cyanomethyl)-3-[4-(7-[2-(trimethylsilyl)ethoxy]methyl-7H-pyrrolo[2,3-d]pyrimidin-4-yl)-1H-pyrazol-1-yl]cyclobutanecarbonitrile (1.00 g, 0.00231 mol). The solution was homogeneous. After adding lithium tetrafluoroborate (2.21 g, 0.0231 mol), the resulting mixture was heated to reflux overnight, then charged with 7.2 M of ammonium hydroxide in water (1.2 mL) in portions over a period of 5 minutes at room temperature to adjust pH to 9-10. The reaction was stirred for 2 h at room temperature. Solid was removed by filtration and the filtrate was diluted with acetonitrile, water, and MeOH. The resultant mixture was purified on Waters XBridge HPLC column (C18, 30x100 mm, 5 µM), with injection volume 5 mL (∼50 mg/injection) and flow rate 60 mL/min, at gradient 10-28% B in 12 minutes (A = water with 0.15% NH₄OH; B = acetonitrile with 0.15% NH₄OH), to give the desired products as free bases. First peak retention time 0.826 min at Waters SunFire HPLC column (C18, 2.1x50 mm, 5 µM) with injection volumn 2 µL and flow rate 3 mL/min, at gradient from 2 to 80% B in 3 minutes (A = water with 0.025% TFA; B = acetonitrile). LCMS calculated for C₁₆H₁₄N₇(M+H)⁺: 304.1; Found 304.3. ¹H NMR (500 MHz, DMSO-d₆): δ 12.10(1H, br s), 8.82 (1H, s), 8.70 (1H, s), 8.44 (1H, s), 7.61 (1H, d, J = 4.0 Hz), 7.08 (1H, d, J = 4.0 Hz), 3.59 (1H, m), 3.57 (2H, s), 3.19 (2H, m), 2.86 (2H, m) ppm. Second peak retention time 0.864 min at the same SunFire column HPLC condition, LCMS calculated for C₁₆H₁₄N₇(M+H)⁺: 304.1; Found 304.3. ¹H NMR (400 MHz, CD₃OD): δ 8.67 (1H, s), 8.66 (1H, s), 8.40 (1H, s), 7.51 (1H, d, J = 3.6 Hz), 6.99 (1H, d, J = 3.6 Hz), 3.50 (1H, m), 3.42 (2H, s), 3.24 (2H, m), 3.00 (2H, m) ppm.

This application claims the benefit of priority of U.S. Patent Appl. No. 61/102,242, filed October 2, 2008, which is incorporated herein by reference in its entirety.

Various modifications of the invention, in addition to those described herein, will be apparent to those skilled in the art from the foregoing description. Such modifications are also intended to fall within the scope of the appended claims. Each reference, including all patent, patent applications, and publications, cited in the present application is incorporated herein by reference in its entirety.

### Reference Embodiments

1. A method of treating a dry eye disorder in a patient in need thereof, comprising administering to said patient a therapeutically effective amount of an agent selected from:
   (a) compounds of Formula I: 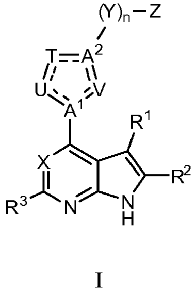 wherein:
      A¹ and A² are independently selected from C and N;
      T, U, and V are independently selected from O, S, N, CR⁵, and NR⁶;
      wherein the 5-membered ring formed by A¹, A², U, T, and V is aromatic;
      X is N or CR⁴;
      Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀ heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
      Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NR)NR^{c}R^{d}, NR^{c}C(=NR)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
      wherein when Z is H, n is 1;
      or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from -(W)ₘ-Q;
      W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c'}, NR^{c'}, NR^{c'}C(O)NR^{d'}, S(O), S(O)NR^{c'}, S(O)₂, or S(O)₂NR^{c'};
      Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a}, C(O)R^{b'}, C(O)NR^{c'}R^{d'}, C(O)OR^{a'}, OC(O)R^{b'}, OC(o)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
      Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
      R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
      R⁵ is H, halo, C₁₋₄ alkyl, C₂₋₄ alkenyl, C₂₋₄ alkynyl, C₁₋₄ haloalkyl, halosulfanyl, CN, NO₂, OR⁷,SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
      R⁶ is H, C₁₋₄ alkyl, C₂₋₄ alkenyl, C₂₋₄ alkynyl, C₁₋₄ haloalkyl, OR⁷,C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
      R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁹ and R¹⁰ are independently selected from H, C₁₋₁₀ alkyl, C₁₋₆ haloalkyl, C₂₋₆ alkenyl, C₂₋₆ alkynyl, C₁₋₆alkylcarbonyl, arylcarbonyl, C₁₋₆alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
      or R⁹ and R¹⁰ together with the N atom to which they are attached form a 4-, 5-, 6- or 7-membered heterocycloalkyl group;
      R¹¹ and R¹² are independently selected from H and -E¹-E²-E³-E⁴;
      D¹ and E¹ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
      D² and E² are independently absent or independently selected from C₁₋₆alkylene, C₂₋₆ alkenylene, C₂₋₆ alkynylene, (C₁₋₆alkylene)ᵣ-O-(C₁₋₆alkylene)ₛ, (C₁₋₆alkylene)ᵣ-S-(C₁₋₆alkylene)ₛ, (C₁₋₆ alkylene)ᵣ-NR^{e}-(C₁₋₆alkylene)ₛ, (C₁₋₆alkylene)ᵣ-CO-(C₁₋₆alkylene)ₛ, (C₁₋₆alkylene)ᵣ-COO-(C₁₋₆ alkylene)ₛ, (C₁₋₆alkylene)ᵣ-CONR^{e}-(C₁₋₆alkylene)ₛ, (C₁₋₆alkylene)ᵣ-SO-(C₁₋₆alkylene)ₛ, (C₁₋₆ alkylene)ᵣ-SO₂-(C₁₋₆alkylene)ₛ, (C₁₋₆alkylene)ᵣ-SONR^{e}-(C₁₋₆alkylene)ₛ, and (C₁₋₆alkylene)ᵣ NR^{e}CONR((C₁₋₆alkylene)ₛ, wherein each of the C₁₋₆ alkylene, C₂₋₆ alkenylene, and C₂₋₆ alkynylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆alkoxy, C₁₋₆haloalkoxy, amino, C₁₋₆ alkylamino, and C₂₋₈ dialkylamino;
      D³ and E³ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆alkoxy, C₁₋₆haloalkoxy, amino, C₁₋₆ alkylamino, and C₂₋₈ dialkylamino;
      D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
      R^{a} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
      R^{b} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, C₁₋₆ haloalkyl, halosulfanyl, aryl, arylalkyl, heteroaryl, heteroarylalkyl, cycloalkyl and heterocycloalkyl;
      R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
      R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
      R^{c} and R^{d} are independently selected from H, Cy¹, -(C₁₋₆alkyl)-Cy¹, C₁₋₁₀alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆alkyl, C₁₋₆haloalkyl, C₁₋₆haloalkyl,and halosulfanyl;
      or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆alkyl)-Cyl, OH, CN, amino, halo, C₁₋₆alkyl, C₁₋₆haloalkyl, C₁₋₆haloalkyl, and halosulfanyl;
      R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
      or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, halosulfanyl, C₁₋₆haloalkyl, aryl, arylalkyl, heteroaryl, heteroarylalkyl, cycloalkyl and heterocycloalkyl;
      or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      Rⁱ is H, CN, NO₂, or C₁₋₆ alkyl;
      R^{e} and R^{f} are independently selected from H and C₁₋₆ alkyl;
      Rⁱ is H, CN, or NO₂;
      m is 0 or 1;
      n is 0 or 1;
      p is 0, 1, 2, 3, 4, 5, or 6;
      q is 0, 1, 2, 3, 4, 5 or 6;
      r is 0 or 1; and
      s is 0 or 1;
      wherein when X is N, n is 1, and the moiety formed by A¹, A², U, T, V, and -(Y)ₙ-Z has the formula: 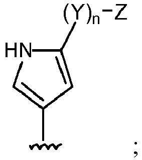 then Y is other than (CR¹¹R¹²)ₚC(O)NR^{c}(CR¹¹R¹²)_{q};
   (b) compounds of Formula II: 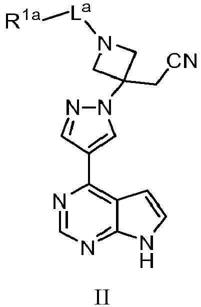 wherein:
      L^{a} is SO₂ or CO;
      R^{1a} is C₁₋₆alkyl, C₃₋₇ cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
      R^{2a} and R^{3a} are independently selected from H, C₁₋₄ alkyl, and phenyl; and
      R^{4a} is C₁₋₆ alkyl, phenyl, or benzyl;
   (c) compounds of Formula III: 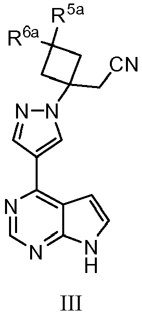 wherein:
      R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
      and pharmaceutically acceptable salts thereof;
      provided that compound of Formula I is not selected from 4-[5-(2-isopropyl-5-methylcyclohexyloxymethyl)-1,2-4-oxadiazol-3-yl]-1H-pyrrolo[2,3-b]pyridine; 4-[5-(4-tert-butylphenoxymethyl)-1,2-4-oxadiazol-3-yl]-1H-pyrrolo[2,3-b]pyridine; 4-[5-cyclopentylethyl-1,2-4-oxadiazol-3-yl]-1H-pyrrolo[2,3-b]pyridine; 4-[5-(2,6-difluorophenyl)-1,2-4-oxadiazol-3-yl]-1H-pyrrolo[2,3-b]pyridine; 4-[5-(1-tert-butyl-3-methyl-1H-pyrazol-5-yl)-1,2-4-oxadiazol-3-yl]-1H-pyrrolo[2,3-b]pyridine; 4-[5-(benzyloxymethyl)-1,2-4-oxadiazol-3-yl]-1H-pyrrolo[2,3-b]pyridine; 4-[5-(3-fluorophenyl)-1,2-4-oxadiazol-3-yl]-1H-pyrrolo[2,3-b]pyridine; 4-[5-(phenoxymethyl)-1,2-4-oxadiazol-3-yl]-1H-pyrrolo[2,3-b]pyridine; 4-[5-(4-methoxybenzyl)-1,2-4-oxadiazol-3-yl]-1H-pyrrolo[2,3-b]pyridine; 4-[5-(phenylthiomethyl)-1,2-4-oxadiazol-3-yl]-1H-pyrrolo[2,3-b]pyridine; 4-[5-(3-methylbutyl)-1,2-4-oxadiazol-3-yl]-1H-pyrrolo[2,3-b]pyridine; 4-[5-benzyl-1,2-4-oxadiazol-3-yl]-1H-pyrrolo[2,3-b]pyridine; 4-[5-(2,2-dimethylpropyl)-1,2-4-oxadiazol-3-yl]-1H-pyrrolo[2,3-b]pyridine; 4-[5-methyl-1,2-4-oxadiazol-3-yl]-1H-pyrrolo[2,3-b]pyridine; 4-[5-(formyl)-1,2-4-oxadiazol-3-yl]-1H-pyrrolo[2,3-b]pyridine; 4-[5-(furan-2-yl)-1,2-4-oxadiazol-3-yl]-1H-pyrrolo[2,3-b]pyridine; 4-[5-(1-methyl-1H-pyrrol-2-yl)-1,2-4-oxadiazol-3-yl]-1H-pyrrolo[2,3-b]pyridine; 4-[5-(sec-butyl)-1,2-4-oxadiazol-3-yl]-1H-pyrrolo[2,3-b]pyridine; 4-[5-cyclopropyl-1,2-4-oxadiazol-3-yl]-1H-pyrrolo[2,3-b]pyridine; and pharmaceutically acceptable salts of any of the aforementioned.
2. A method according to embodiment 1, wherein said dry eye disorder is aqueous tear-deficient dry eye.
3. A method according to embodiment 1, wherein said dry eye disorder is evaporative dry eye.
4. A method according to embodiment 1, wherein said dry eye disorder is Sjogren syndrome dry eye.
5. A method according to embodiment 1, wherein said dry eye disorder is non-Sjogren syndrome dry eye.
6. A method according to embodiment 1, wherein said treating comprises ameliorating a symptom selected from eye discomfort, visual disturbance, tear film instability, tear hyperosmolarity, and inflammation of the ocular surface.
7. A method according to any one of embodiments 1 to 6, wherein said treating comprises administering a pharmaceutical composition to said patient, said composition comprising said agent and a pharmaceutically acceptable carrier.
8. A method according to embodiment 7, wherein said pharmaceutical composition is an oral dosage form.
9. A method according to any one of embodiments 1 to 6, wherein said treating comprises administering an ophthalmic composition to said patient, said composition comprising said agent and an ophthalmically acceptable carrier.
10. A method according to embodiment 9, wherein said ophthalmic composition is a topical composition.
11. A method according to embodiment 9, wherein said topical composition is an aqueous formulation, an aqueous suspension, an ointment or a gel.
12. A method according to embodiment 9, wherein said ophthalmic composition is an ophthalmic insert.
13. A method according to embodiment 12, wherein said ophthalmic insert comprises microspheres.
14. A method according to embodiment 13, wherein said microspheres are injected to the posterior segment of the eye, in the chroidal space, in the sclera, intravitreally or sub-retinally.
15. A method according to embodiment 12, wherein said ophthalmic insert comprises collagen, gelatin, or a polymer, wherein said polymer is selected from polycaprolactone (PCL), an ethylene/vinyl acetate copolymer (EVA), polyalkyl cyanoacralate, polyurethane, a nylon, poly(dl-lactide-co-glycolide) (PLGA), or a copolymer of any of the aforementioned.
16. A method according to embodiment 12, wherein said ophthalmic insert is implanted under the upper eyelid, in the posterior segment of the eye, in the chroidal space, in the sclera, intravitreally or sub-retinally.
17. A method according to any one of embodiments 1 to 16, further comprising administering at least one additional therapeutic agent.
18. A method according to embodiment 17, wherein said additional therapeutic agent is fluocinolone acetonide, rimexolone, cyclosporine, riaminolone, dexamethasone, fluocinolone, cortisone, prednisolone, flumetholone, civamide, testosterone, ecabet sodium, 15-(s)-hydroxyeicosatetraenoic acid, 2S,3S,4R,5R)-3,4-dihydroxy-5-[6-[(3-iodophenyl)methylamino]purin-9-yl]-N-m ethyl-oxolane-2-carboxamide, gefarnate, cevilemine, doxycline, minocycline, oxytetracycline, voclosporin, rivoglitazone, lacritin rebamipide, pilocarpine, tacrolimus, pimecrolimus, loteprednol etabonate, rituximab, diquafosol tetrasodium, dehydroepiandrosterone, anakinra, efalizumab, mycophenolate sodium, etanercept, hydroxychloroquine, or thalidomide.
19. A method according to embodiment 18, wherein said additional therapeutic agent is sodium hyaluronate, hyaluronic acid, polyvinylalcohol, hydroxypropyl methylcellulose, glycerin, polyethylene glycol, or carboxymethyl cellulose.
20. A method according to any one of embodiments 1 to 19, wherein said agent is selected from compounds of Formula I: 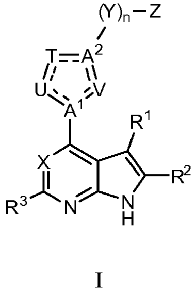 and pharmaceutically acceptable salts thereof.
21. A method according to embodiment 20, wherein n is 1 and Y is C₁₋₈ alkylene optionally substituted with 1, 2, or 3 substituents independently selected from D⁴.
22. A method according to embodiment 20, wherein n is 1 and Y is C₁₋₈ alkylene optionally substituted with 1, 2, or 3 halo, OH, CN, amino, C₁₋₄ alkylamino, or C₂₋₈ dialkylamino.
23. A method according to embodiment 20, wherein n is 1 and Y is C₁₋₈ alkylene optionally substituted with cyano.
24. A method according to any one of embodiments 20 to 23, wherein Z is cyclopropyl, cyclobutyl, cyclopentyl, cyclohexyl, or cycloheptyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.
25. A method according to any one of embodiments 20 to 23, wherein Z is cyclopentyl, which is optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}.
26. A method according to any one of embodiments 20 to 23, wherein Z is cyclopentyl.
27. A method according to any one of embodiments 20 to 26, wherein X is N.
28. A method according to any one of embodiments 20 to 27, wherein A¹ is C.
29. A method according to any one of embodiments 20 to 28, wherein A² is N.
30. A method according to any one of embodiments 20 to 29, wherein T is N.
31. A method according to any one of embodiments 20 to 30, wherein U and V are independently CR⁵.
32. A method according to any one of embodiments 20 to 31, wherein R¹, R², R³, and R⁴ are each H.
33. A method according to any one of embodiments 20 to 32, wherein the 5-membered ring formed by A¹, A², U, T, and V is selected from: 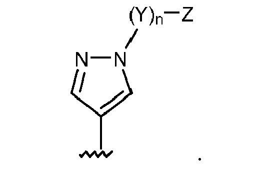
34. A method according to any one of embodiments 20 to 32, wherein said agent is selected from compounds having Formula II: 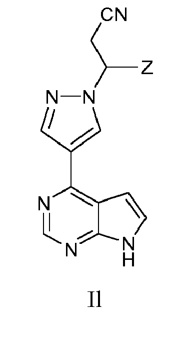 and pharmaceutically acceptable salts thereof.
35. A method according to any one of embodiments 20 to 32, wherein the moiety formed by T, U, V, A¹, and A² is not the following moiety: 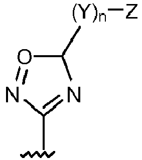
36. A method according to embodiment 20, wherein said agent is selected from 3-cyclopentyl-3-[4-(7H-pyrrolo[2,3-d]pyrimidin-4-yl)-1H-pyrazol-1-yl]propanenitrile and pharmaceutically acceptable salts thereof.
37. A method according to embodiment 20, wherein said agent is selected from (3R)-3-cyclopentyl-3-[4-(7H-pyrrolo[2,3-d]pyrimidin-4-yl)-1H-pyrazol-1-yl]propanenitrile and pharmaceutically acceptable salts thereof.
38. A method according to any one of embodiments 1 to 19, wherein said agent is selected from compounds of Formula II: 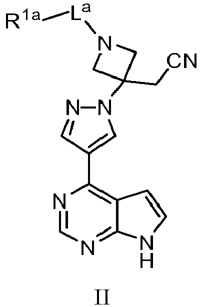 and pharmaceutically acceptable salts thereof.
39. A method according to embodiment 38, wherein L^{a} is SO₂.
40. A method according to embodiment 38 or 39, wherein R^{1a} is C₁₋₄ alkyl.
41. A method according to embodiment 38 or 39, wherein R^{1a} is ethyl.
42. A method according to embodiment 38, wherein said agent is selected from {1-(Ethylsulfonyl)-3-[4-(7H-pyrrolo[2,3-d]pyrimidin-4-yl)-1H-pyrazol-1-yl]azetidin-3-yl}acetonitrile and pharmaceutically acceptable salts thereof.
43. A method according to embodiment 38, wherein said agent is {1-(Ethylsulfonyl)-3-[4-(7H-pyrrolo[2,3-d]pyrimidin-4-yl)-1H-pyrazol-1-yl]azetidin-3-yl}acetonitrile phosphoric acid salt.
44. A method according to any one of embodiments 1 to 19, wherein said agent is selected from compounds of Formula III: 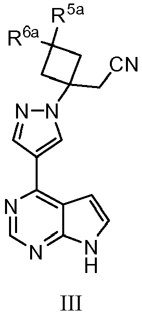 and pharmaceutically acceptable salts thereof.
45. A method according to embodiment 44, wherein one of R^{5a} and R^{6a} is H and the other is selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl.
46. A method according to embodiment 44, wherein R^{5a} and R^{6a} are independently selected from H and CN.
47. A method according to embodiment 44, wherein said agent is selected from 3-(cyanomethyl)-3-[4-(7H-pyrrolo[2,3-d]pyrimidin-4-yl)-1H-pyrazol-1-yl]cyclobutanecarbonitrile and pharmaceutically acceptable salts thereof.
48. A method according to embodiment 44, wherein said agent is selected from cis-3-(cyanomethyl)-3-[4-(7H-pyrrolo[2,3-d]pyrimidin-4-yl)-1H-pyrazol-1-yl]cyclobutanecarbonitrile and pharmaceutically acceptable salts thereof.
49. A method according to embodiment 44, wherein said agent is selected from trans-3-(cyanomethyl)-3-[4-(7H-pyrrolo[2,3-d]pyrimidin-4-yl)-1H-pyrazol-1-yl]cyclobutanecarbonitrile and pharmaceutically acceptable salts thereof.
50. An ophthalmic insert comprising a therapeutically effective amount of an agent selected from:
   (a) compounds of Formula I: 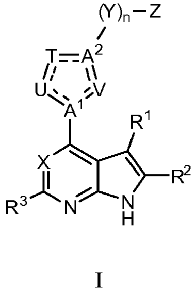 wherein:
      A¹ and A² are independently selected from C and N;
      T, U, and V are independently selected from O, S, N, CR⁵, and NR⁶;
      wherein the 5-membered ring formed by A¹, A², U, T, and V is aromatic;
      X is N or CR⁴;
      Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)ₚ-(heteroarylene)-(CR¹¹R¹²)_{q}, (CR¹1R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D ²-D³ -D ⁴;
      Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂ OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
      wherein when Z is H, n is 1;
      or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from -(W)ₘ-Q;
      W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c'}, NR^{c'}, NR^{c'}C(O)NR^{d'}, S(O), S(O)NR^{c'}, S(O)₂, or S(O)₂NR^{c'};
      Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b}, S(O)NR^{c'}R^{d'} S(O)₂R^{b'}, NR^{c'}S(O)₂R^{b'}, and S(O)₂NR^{c'}R^{d'};
      Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a''}, SR^{a''}, C(O)R^{b}, C(O)NR^{c''}R^{d}, C(O)OR^{a''}, OC(O)R^{b''}, OC(O)NR^{c''}R^{d''}, NR^{c''}R^{d''}, NR^{c''}C(O)R^{b''}, NR^{c''}C(O)OR^{a''}, NR^{c''}S(O)R^{b''}, NR^{c''}S(O)₂R^{b''}, S(O)R^{b''}, S(O)NR^{c''}R^{d''}, S(O)₂R^{b''}, and S(O)₂NR^{c''}R^{d''};
      R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
      R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
      R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
      R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁹ and R¹⁰ are independently selected from H, C₁₋₁₀ alkyl, C₁₋₆ haloalkyl, C₂₋₆ alkenyl, C₂₋₆ alkynyl, C₁₋₆alkylcarbonyl, arylcarbonyl, C₁₋₆alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
      or R⁹ and R¹⁰ together with the N atom to which they are attached form a 4-, 5-, 6- or 7-membered heterocycloalkyl group;
      R¹¹ and R¹² are independently selected from H and -E¹-E²-E³-E⁴;
      D¹ and E¹ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
      D² and E² are independently absent or independently selected from C₁₋₆alkylene, C₂₋₆ alkenylene, C₂₋₆ alkynylene, (C₁₋₆alkylene)ᵣ-O-(C₁₋₆alkylene)ₛ, (C₁₋₆alkylene)ᵣ-S-(C₁₋₆alkylene)ₛ, (C₁₋₆ alkylene)ᵣ-NR^{e}-(C₁₋₆ alkylene)ₛ, (C₁₋₆alkylene)ᵣ-CO-(C₁₋₆alkylene)ₛ, (C₁₋₆alkylene)ᵣ-COO-(C₁₋₆ alkylene)ₛ, (C₁₋₆alkylene)ᵣ-CONR^{e}-(C₁₋₆alkylene)ₛ, (C₁₋₆alkylene)ᵣ-SO-(C₁₋₆alkylene)ₛ, (C₁₋₆ alkylene)ᵣ-SO₂-(C₁₋₆ alkylene)ₛ, (C₁₋₆alkylene)ᵣ-SONR^{e}-(C₁₋₆alkylene)ₛ, and (C₁₋₆alkylene)ᵣ-NR^{e}CONR^{f}-(C₁₋₆ alkylene)ₛ, wherein each of the C₁₋₆alkylene, C₂₋₆ alkenylene, and C₂₋₆ alkynylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
      D³ and E³ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
      D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
      R^{a} is H, Cy¹, -(C₁₋₆ alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
      R^{b} is H, Cy¹, -(C₁₋₆ alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
      R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
      R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
      R^{c} and R^{d} are independently selected from H, Cy¹, -(C₁₋₆ alkyl)-Cy¹, C₁₋₁₀ alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆ alkyl, C₁₋₆ haloalkyl, C₁₋₆ haloalkyl, and halosulfanyl;
      or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆ alkyl)-Cy¹, OH, CN, amino, halo, C₁₋₆ alkyl, C₁₋₆ haloalkyl, C₁₋₆ haloalkyl, and halosulfanyl;
      R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
      or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
      R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, C₁₋₆ haloalkyl, aryl, arylalkyl, heteroaryl, heteroarylalkyl, cycloalkyl and heterocycloalkyl;
      or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
      Rⁱ is H, CN, NO₂, or C₁₋₆ alkyl;
      R^{e} and R^{f} are independently selected from H and C₁₋₆ alkyl;
      Rⁱ is H, CN, or NO₂;
      m is 0 or 1;
      n is 0 or 1;
      p is 0, 1, 2, 3, 4, 5, or 6;
      q is 0, 1, 2, 3, 4, 5 or 6;
      r is 0 or 1; and
      s is 0 or 1;
      wherein when X is N, n is 1, and the moiety formed by A¹, A², U, T, V, and -(Y)ₙ-Z has the formula: 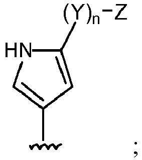 then Y is other than (CR¹¹R¹²)ₚC(O)NR^{c}(CR¹¹R¹²)_{q};
   (b) compounds of Formula II: 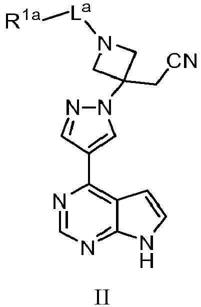 wherein:
      L^{a} is SO₂ or CO;
      R^{1a} is C₁₋₆ alkyl, C₃₋₇ cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
      R^{2a} and R^{3a} are independently selected from H, C₁₋₄ alkyl, and phenyl; and
      R^{4a} is C₁₋₆ alkyl, phenyl, or benzyl;
   (c) compounds of Formula III: 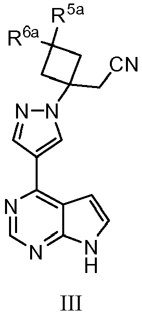 wherein:
      R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
      and pharmaceutically acceptable salts thereof;
      provided that compound of Formula I is not selected from 4-[5-(2-isopropyl-5-methylcyclohexyloxymethyl)-1,2-4-oxadiazol-3-yl]-1H-pyrrolo[2,3-b]pyridine; 4-[5-(4-tert-butylphenoxymethyl)-1,2-4-oxadiazol-3-yl]-1H-pyrrolo[2,3-b]pyridine; 4-[5-cyclopentylethyl-1,2-4-oxadiazol-3-yl]-1H-pyrrolo[2,3-b]pyridine; 4-[5-(2,6-difluorophenyl)-1,2-4-oxadiazol-3-yl]-1H-pyrrolo[2,3-b]pyridine; 4-[5-(1-tert-butyl-3-methyl-1H-pyrazol-5-yl)-1,2-4-oxadiazol-3-yl]-1H-pyrrolo[2,3-b]pyridine; 4-[5-(benzyloxymethyl)-1,2-4-oxadiazol-3-yl]-1H-pyrrolo[2,3-b]pyridine; 4-[5-(3-fluorophenyl)-1,2-4-oxadiazol-3-yl]-1H-pyrrolo[2,3-b]pyridine; 4-[5-(phenoxymethyl)-1,2-4-oxadiazol-3-yl]-1H-pyrrolo[2,3-b]pyridine; 4-[5-(4-methoxybenzyl)-1,2-4-oxadiazol-3-yl]-1H-pyrrolo[2,3-b]pyridine; 4-[5-(phenylthiomethyl)-1,2-4-oxadiazol-3-yl]-1H-pyrrolo[2,3-b]pyridine; 4-[5-(3-methylbutyl)-1,2-4-oxadiazol-3-yl]-1H-pyrrolo[2,3-b]pyridine; 4-[5-benzyl-1,2-4-oxadiazol-3-yl]-1H-pyrrolo[2,3-b]pyridine; 4-[5-(2,2-dimethylpropyl)-1,2-4-oxadiazol-3-yl]-1H-pyrrolo[2,3-b]pyridine; 4-[5-methyl-1,2-4-oxadiazol-3-yl]-1H-pyrrolo[2,3-b]pyridine; 4-[5-(formyl)-1,2-4-oxadiazol-3-yl]-1H-pyrrolo[2,3-b]pyridine; 4-[5-(furan-2-yl)-1,2-4-oxadiazol-3-yl]-1H-pyrrolo[2,3-b]pyridine; 4-[5-(1-methyl-1H-pyrrol-2-yl)-1,2-4-oxadiazol-3-yl]-1H-pyrrolo[2,3-b]pyridine; 4-[5-(sec-butyl)-1,2-4-oxadiazol-3-yl]-1H-pyrrolo[2,3-b]pyridine; 4-[5-cyclopropyl-1,2-4-oxadiazol-3-yl]-1H-pyrrolo[2,3-b]pyridine; and pharmaceutically acceptable salts of any of the aforementioned.
51. A kit for treatment of dry eye disorder, comprising an ophthalmic or pharmaceutical composition and instructions;
   wherein said composition comprises a therapeutically effective amount of an agent selected from:
   (a) compounds of Formula I: 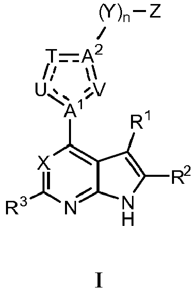 wherein:
      A¹ and A² are independently selected from C and N;
      T, U, and V are independently selected from O, S, N, CR⁵, and NR⁶;
      wherein the 5-membered ring formed by A¹, A², U, T, and V is aromatic;
      X is N or CR⁴;
      Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀ heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
      Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
      wherein when Z is H, n is 1;
      or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from -(W)ₘ-Q;
      W is C₁₋₈ alkylenyl, C₂₋₈ alkenylenyl, C₂₋₈ alkynylenyl, O, S, C(O), C(O)NR^{c}, C(O)O, OC(O), OC(O)NR^{c'}, NR^{c'}, NR^{c'}C(O)NR^{d'}, S(O), S(O)NR^{c'}, S(O)₂, or S(O)₂NR^{c'};
      Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a}, SR^{a}, C(O)R^{b'}, C(O)NR^{c'}R^{d'}, C(O)OR^{a'}, OC(O)R^{b'}, OC(O)NR^{c'}R^{d'} NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
      Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"},R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
      R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
      R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
      R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
      R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁹ and R¹⁰ are independently selected from H, C₁₋₁₀ alkyl, C₁₋₆ haloalkyl, C₂₋₆ alkenyl, C₂₋₆ alkynyl, C₁₋₆ alkylcarbonyl, arylcarbonyl, C₁₋₆ alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
      or R⁹ and R¹⁰ together with the N atom to which they are attached form a 4-, 5-, 6- or 7-membered heterocycloalkyl group;
      R¹¹ and R¹² are independently selected from H and -E¹-E²-E³-E⁴;
      D¹ and E¹ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
      D² and E² are independently absent or independently selected from C₁₋₆ alkylene, C₂₋₆ alkenylene, C₂₋₆ alkynylene, (C₁₋₆ alkylene)ᵣ-O-(C₁₋₆ alkylene)ₛ, (C₁₋₆ alkylene)ᵣ-S-(C₁₋₆ alkylene)ₛ, (C₁₋₆ alkylene)ᵣ-NR^{e}-(C₁₋₆ alkylene)ₛ, (C₁₋₆ alkylene)ᵣ-CO-(C₁₋₆ alkylene)ₛ, (C₁₋₆ alkylene)ᵣ-COO-(C₁₋₆ alkylene)ₛ, (C₁₋₆ alkylene)ᵣ-CONR^{e}-(C₁₋₆ alkylene)ₛ, (C₁₋₆ alkylene)ᵣ-SO-(C₁₋₆ alkylene)ₛ, (C₁₋₆ alkylene)ᵣ-SO₂-(C₁₋₆ alkylene)ₛ, (C₁₋₆ alkylene)ᵣ-SONR^{e}-(C₁₋₆ alkylene)ₛ, and (C₁₋₆alkylene)ᵣ-NR^{e}CONR^{f}-(C₁₋₆ alkylene)ₛ, wherein each of the C₁₋₆ alkylene, C₂₋₆ alkenylene, and C₂₋₆ alkynylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
      D³ and E³ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆haloalkyl, C₂₋₈ alkoxyalkyl, C₁₋₆alkoxy, C₁₋₆ haloalkoxy, amino, C₁₋₆ alkylamino, and C₂₋₈ dialkylamino;
      D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, ,S(O)₂R^{b}, NR^{c}S(O)₂R^{b}, C(=NOH)R^{b}, C(=NO(C₁₋₆ alkyl))R^{b}, and S(O)₂NR^{c}R^{d};
      R^{a} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
      R^{b} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, C₁₋₆ haloalkyl, halosulfanyl, aryl, arylalkyl, heteroaryl, heteroarylalkyl, cycloalkyl and heterocycloalkyl;
      R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
      R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
      R^{c} and R^{d} are independently selected from H, Cy¹, -(C₁₋₆alkyl)-Cy¹, C₁₋₁₀ alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆alkyl, C₁₋₆haloalkyl, C₁₋₆haloalkyl,and halosulfanyl;
      or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆alkyl)-Cy¹, OH, CN, amino, halo, C₁₋₆alkyl, C₁₋₆haloalkyl, C₁₋₆haloalkyl, and halosulfanyl;
      R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆haloalkyl, C₁₋₆haloalkyl, halosulfanyl, aryl, arylalkyl, heteroaryl, heteroarylalkyl, cycloalkyl and heterocycloalkyl;
      or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆haloalkyl, C₁₋₆haloalkyl, halosulfanyl, aryl, arylalkyl, heteroaryl, heteroarylalkyl, cycloalkyl and heterocycloalkyl;
      R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆haloalkyl, halosulfanyl, C₁₋₆haloalkyl, aryl, arylalkyl, heteroaryl, heteroarylalkyl, cycloalkyl and heterocycloalkyl;
      or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆haloalkyl, C₁₋₆haloalkyl, halosulfanyl, aryl, arylalkyl, heteroaryl, heteroarylalkyl, cycloalkyl and heterocycloalkyl;
      Rⁱ is H, CN, NO₂, or C₁₋₆ alkyl;
      R^{e} and R^{f} are independently selected from H and C₁₋₆ alkyl;
      Rⁱ is H, CN, or NO₂;
      m is 0 or 1;
      n is 0 or 1;
      p is 0, 1, 2, 3, 4, 5, or 6;
      q is 0, 1, 2, 3, 4, 5 or 6;
      r is 0 or 1; and
      s is 0 or 1;
      wherein when X is N, n is 1, and the moiety formed by A¹, A², U, T, V, and -(Y)ₙ-Z has the formula: 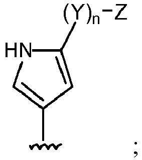 then Y is other than (CR¹¹R¹²)ₚC(O)NR^{c}(CR¹¹R¹²)_{q};
   (b) compounds of Formula II: 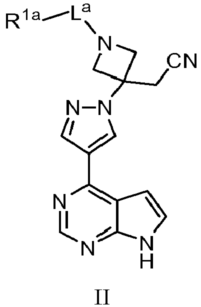 wherein:
      L^{a} is SO₂ or CO;
      R^{1a} is C₁₋₆alkyl, C₃₋₇ cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
      R^{2a} and R^{3a} are independently selected from H, C₁₋₄ alkyl, and phenyl; and
      R^{4a} is C₁₋₆ alkyl, phenyl, or benzyl;
   (c) compounds of Formula III: 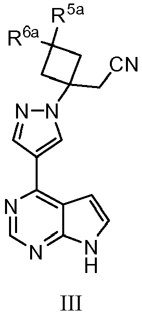 wherein:
      R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
      and pharmaceutically acceptable salts thereof;
      provided that compound of Formula I is not selected from 4-[5-(2-isopropyl-5-methylcyclohexyloxymethyl)-1,2-4-oxadiazol-3-yl]-1H-pyrrolo[2,3-b]pyridine; 4-[5-(4-tert-butylphenoxymethyl)-1,2-4-oxadiazol-3-yl]-1H-pyrrolo[2,3-b]pyridine; 4-[5-cyclopentylethyl-1,2-4-oxadiazol-3-yl]-1H-pyrrolo[2,3-b]pyridine; 4-[5-(2,6-difluorophenyl)-1,2-4-oxadiazol-3-yl]-1H-pyrrolo[2,3-b]pyridine; 4-[5-(1-tert-butyl-3-methyl-1H-pyrazol-5-yl)-1,2-4-oxadiazol-3-yl]-1H-pyrrolo[2,3-b]pyridine; 4-[5-(benzyloxymethyl)-1,2-4-oxadiazol-3-yl]-1H-pyrrolo[2,3-b]pyridine; 4-[5-(3-fluorophenyl)-1,2-4-oxadiazol-3-yl]-1H-pyrrolo[2,3-b]pyridine; 4-[5-(phenoxymethyl)-1,2-4-oxadiazol-3-yl]-1H-pyrrolo[2,3-b]pyridine; 4-[5-(4-methoxybenzyl)-1,2-4-oxadiazol-3-yl]-1H-pyrrolo[2,3-b]pyridine; 4-[5-(phenylthiomethyl)-1,2-4-oxadiazol-3-yl]-1H-pyrrolo[2,3-b]pyridine; 4-[5-(3-methylbutyl)-1,2-4-oxadiazol-3-yl]-1H-pyrrolo[2,3-b]pyridine; 4-[5-benzyl-1,2-4-oxadiazol-3-yl]-1H-pyrrolo[2,3-b]pyridine; 4-[5-(2,2-dimethylpropyl)-1,2-4-oxadiazol-3-yl]-1H-pyrrolo[2,3-b]pyridine; 4-[5-methyl-1,2-4-oxadiazol-3-yl]-1H-pyrrolo[2,3-b]pyridine; 4-[5-(formyl)-1,2-4-oxadiazol-3-yl]-1H-pyrrolo[2,3-b]pyridine; 4-[5-(furan-2-yl)-1,2-4-oxadiazol-3-yl]-1H-pyrrolo[2,3-b]pyridine; 4-[5-(1-methyl-1H-pyrrol-2-yl)-1,2-4-oxadiazol-3-yl]-1H-pyrrolo[2,3-b]pyridine; 4-[5-(sec-butyl)-1,2-4-oxadiazol-3-yl]-1H-pyrrolo[2,3-b]pyridine; 4-[5-cyclopropyl-1,2-4-oxadiazol-3-yl]-1 H-pyrrolo [2,3-b]pyridine; and pharmaceutically acceptable salts of any of the aforementioned;
      and wherein said instructions comprise a direction to administer said composition to a patient in need of treatment of said dry eye disorder.
52. Use of an agent for the preparation of a medicament for the treatment of an dry eye disorder; wherein said agent is selected from:
   (a) compounds of Formula I: 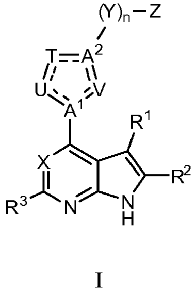 wherein:
      A¹ and A² are independently selected from C and N;
      T, U, and V are independently selected from O, S, N, CR⁵, and NR⁶; wherein the 5-membered ring formed by A¹, A², U, T, and V is aromatic;
      X is N or CR⁴;
      Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
      Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{C}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{C}R^{d}, C(O)OR^{a}, OC(O)R^{b}, OC(O)NR^{C}R^{d}, NR^{C}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, ,S(O)₂R^{b}, NR^{c}S(O)₂R^{b}, C(=NOH)R^{b}, C(=NO(C₁₋₆ alkyl))R^{b}, and S(O)₂NR^{c}R^{d};
      wherein when Z is H, n is 1;
      or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from-(W)ₘ-Q;
      W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c'}, NR^{c'}, NR^{c'}C(O)NR^{d'}, S(O), S(O)NR^{c'}, S(O)₂, or S(O)₂NR^{c'};
      Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, O(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
      Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"} , SR^{a"}, C(O)R^{b"}, C(O)NR^{c"}R^{d"}, C(O)OR^{a"} , OC(O)R^{b"}, OC(O)NR^{c"}R^{d"}, NR^{c"}R^{d"}, NR^{c"}C(O)R^{b"}, NR^{c"}C(O)OR^{a"} , NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
      R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
      R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
      R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
      R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁹ and R¹⁰ are independently selected from H, C₁₋₁₀ alkyl, C₁₋₆ haloalkyl, C₂₋₆ alkenyl, C₂₋₆ alkynyl, C₁₋₆alkylcarbonyl, arylcarbonyl, C₁₋₆alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
      or R⁹ and R¹⁰ together with the N atom to which they are attached form a 4-, 5-, 6- or 7-membered heterocycloalkyl group;
      R¹¹ and R¹² are independently selected from H and -E¹-E²-E³-E⁴;
      D¹ and E¹ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆haloalkyl, C₂₋₈ alkoxyalkyl, C₁₋₆alkoxy, C₁₋₆ haloalkoxy, amino, C₁₋₆ alkylamino, and C₂₋₈ dialkylamino;
      D² and E² are independently absent or independently selected from C₁₋₆ alkylene, C₂₋₆ alkenylene, C₂₋₆ alkynylene, (C₁₋₆alkylene)ᵣ-O-(C₁₋₆alkylene)s, (C₁₋₆alkylene)ᵣ-S-(C₁₋₆alkylene)ₛ, (C₁₋₆ alkylene)ᵣ-NR^{e}-(C₁₋₆alkylene)ₛ, (C₁₋₆alkylene)ᵣ-CO-(C₁₋₆alkylene)ₛ, (C₁₋₆alkylene)ᵣ-COO-(C₁₋₆ alkylene)ₛ, (C₁₋₆alkylene)ᵣ-CONR^{e}-(C₁₋₆alkylene)ₛ, (C₁₋₆alkylene)ᵣ-SO-(C₁₋₆alkylene)ₛ, (C₁₋₆ alkylene)ᵣ-SO₂-(C₁₋₆alkylene)ₛ, (C₁₋₆alkylene)ᵣ-SONR^{e}-(C₁₋₆alkylene)ₛ, and (C₁₋₆alkylene)ᵣ NR^{e}CONR^{f}-(C₁₋₆alkylene)ₛ, wherein each of the C₁₋₆ alkylene, C₂₋₆ alkenylene, and C₂₋₆ alkynylene is optionally substituted by 1, 2 or 3 substituents independently selected from halo, CN, NO₂, N₃, SCN, OH, C₁₋₆ alkyl, C₁₋₆haloalkyl, C₂₋₈ alkoxyalkyl, C₁₋₆alkoxy, C₁₋₆ haloalkoxy, amino, C₁₋₆ alkylamino, and C₂₋₈ dialkylamino;
      D³ and E³ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆haloalkyl, C₂₋₈ alkoxyalkyl, C₁₋₆alkoxy, C₁₋₆ haloalkoxy, amino, C₁₋₆ alkylamino, and C₂₋₈ dialkylamino;
      D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{C}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{C}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
      R^{a} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
      R^{b} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, C₁₋₆ haloalkyl, halosulfanyl, aryl, arylalkyl, heteroaryl, heteroarylalkyl, cycloalkyl and heterocycloalkyl;
      R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆haloalkyl, halosulfanyl, aryl, arylalkyl, heteroaryl, heteroarylalkyl, cycloalkyl and heterocycloalkyl;
      R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆haloalkyl, C₁₋₆haloalkyl, halosulfanyl, aryl, arylalkyl, heteroaryl, heteroarylalkyl, cycloalkyl and heterocycloalkyl;
      R^{c} and R^{d} are independently selected from H, Cy¹, -(C₁₋₆alkyl)-Cy¹, C₁₋₁₀alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆alkyl, C₁₋₆haloalkyl, C₁₋₆haloalkyl,and halosulfanyl;
      or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆alkyl)-Cy¹, OH, CN, amino, halo, C₁₋₆alkyl, C₁₋₆haloalkyl, C₁₋₆haloalkyl, and halosulfanyl;
      R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆haloalkyl, C₁₋₆haloalkyl, halosulfanyl, aryl, arylalkyl, heteroaryl, heteroarylalkyl, cycloalkyl and heterocycloalkyl;
      or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆haloalkyl, C₁₋₆haloalkyl, halosulfanyl, aryl, arylalkyl, heteroaryl, heteroarylalkyl, cycloalkyl and heterocycloalkyl;
      R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆haloalkyl, halosulfanyl, C₁₋₆haloalkyl, aryl, arylalkyl, heteroaryl, heteroarylalkyl, cycloalkyl and heterocycloalkyl;
      or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      Rⁱ is H, CN, NO₂, or C₁₋₆ alkyl;
      R^{e} and R^{f} are independently selected from H and C₁₋₆ alkyl;
      Rⁱ is H, CN, or NO₂;
      m is 0 or 1;
      n is 0 or 1;
      p is 0, 1, 2, 3, 4, 5, or 6;
      q is 0, 1, 2, 3, 4, 5 or 6;
      r is 0 or 1; and
      s is 0 or 1;
      wherein when X is N, n is 1, and the moiety formed by A¹, A², U, T, V, and -(Y)ₙ-Z has the formula: 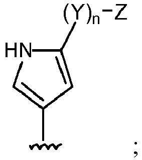 then Y is other than (CR¹¹R¹²)ₚC(O)NR^{c}(CR¹¹R¹²)_{q};
   (b) compounds of Formula II: 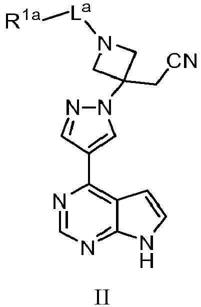 wherein:
      L^{a} is SO₂ or CO;
      R^{1a} is C₁₋₆ₐlkyl, C₃₋₇ cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
      R^{2a} and R^{3a} are independently selected from H, C₁₋₄ alkyl, and phenyl; and
      R^{4a} is C₁₋₆ alkyl, phenyl, or benzyl;
   (c) compounds of Formula III: 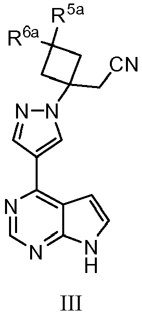 wherein:
      R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
      and pharmaceutically acceptable salts thereof;
      provided that compound of Formula I is not selected from 4-[5-(2-isopropyl-5-methylcyclohexyloxymethyl)-1,2-4-oxadiazol-3-yl]-1H-pyrrolo[2,3-b]pyridine; 4-[5-(4-tert-butylphenoxymethyl)-1,2-4-oxadiazol-3-yl]-1H-pyrrolo[2,3-b]pyridine; 4-[5-cyclopentylethyl-1,2-4-oxadiazol-3-yl]-1H-pyrrolo[2,3-b]pyridine; 4-[5-(2,6-difluorophenyl)-1,2-4-oxadiazol-3-yl]-1H-pyrrolo[2,3-b]pyridine; 4-[5-(1-tert-butyl-3-methyl-1H-pyrazol-5-yl)-1,2-4-oxadiazol-3-yl]-1H-pyrrolo[2,3-b]pyridine; 4-[5-(benzyloxymethyl)-1,2-4-oxadiazol-3-yl]-1H-pyrrolo[2,3-b]pyridine; 4-[5-(3-fluorophenyl)-1,2-4-oxadiazol-3-yl]-1H-pyrrolo[2,3-b]pyridine; 4-[5-(phenoxymethyl)-1,2-4-oxadiazol-3-yl]-1H-pyrrolo[2,3-b]pyridine; 4-[5-(4-methoxybenzyl)-1,2-4-oxadiazol-3-yl]-1H-pyrrolo[2,3-b]pyridine; 4-[5-(phenylthiomethyl)-1,2-4-oxadiazol-3-yl]-1H-pyrrolo[2,3-b]pyridine; 4-[5-(3-methylbutyl)-1,2-4-oxadiazol-3-yl]-1H-pyrrolo[2,3-b]pyridine; 4-[5-benzyl-1,2-4-oxadiazol-3-yl]-1H-pyrrolo[2,3-b]pyridine; 4-[5-(2,2-dimethylpropyl)-1,2-4-oxadiazol-3-yl]-1H-pyrrolo[2,3-b]pyridine; 4-[5-methyl-1,2-4-oxadiazol-3-yl]-1H-pyrrolo[2,3-b]pyridine; 4-[5-(formyl)-1,2-4-oxadiazol-3-yl]-1H-pyrrolo[2,3-b]pyridine; 4-[5-(furan-2-yl)-1,2-4-oxadiazol-3-yl]-1H-pyrrolo[2,3-b]pyridine; 4-[5-(1-methyl-1H-pyrrol-2-yl)-1,2-4-oxadiazol-3-yl]-1H-pyrrolo[2,3-b]pyridine; 4-[5-(sec-butyl)-1,2-4-oxadiazol-3-yl]-1H-pyrrolo[2,3-b]pyridine; 4-[5-cyclopropyl-1,2-4-oxadiazol-3-yl]-1 H-pyrrolo [2,3-b]pyridine; and pharmaceutically acceptable salts of any of the aforementioned.
53. An agent for use in treatment of a dry eye disorder in an individual; wherein said agent is selected from:
   (a) compounds of Formula I: 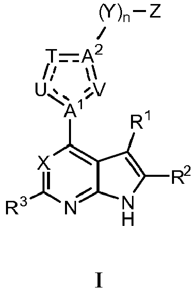 wherein:
      A¹ and A² are independently selected from C and N;
      T, U, and V are independently selected from O, S, N, CR⁵, and NR⁶;
      wherein the 5-membered ring formed by A¹, A², U, T, and V is aromatic;
      X is N or CR⁴;
      Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)ₚ-(heteroarylene)-(CR¹¹R¹²)_{q}, (CR¹¹R¹²)pO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴.
      Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{C}R^{d}, C(O)OR^{a}, OC(O)R^{b}, OC(O)NR^{C}R^{d}, NR^{C}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NR)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{C}R^{d}, C(O)OR^{a}, OC(O)R^{b}, OC(O)NR^{C}R^{d}, NR^{C}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, ,S(O)₂R^{b}, NR^{c}S(O)₂R^{b}, C(=NOH)R^{b}, C(=NO(C₁₋₆ alkyl))R^{b}, and S(O)₂NR^{c}R^{d};
      wherein when Z is H, n is 1;
      or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from-(W)ₘ-Q;
      W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c'}, NR^{c'}, NR^{c'}C(O)NR^{d'}, S(O), S(O)NR^{c'}, S(O)₂, or S(O)₂NR^{c'};
      Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
      Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"} , OC(O)R^{b"}, OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
      R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
      R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
      R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
      R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁹ and R¹⁰ are independently selected from H, C₁₋₁₀ alkyl, C₁₋₆ haloalkyl, C₂₋₆ alkenyl, C₂₋₆ alkynyl, C₁₋₆alkylcarbonyl, arylcarbonyl, C₁₋₆alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
      or R⁹ and R¹⁰ together with the N atom to which they are attached form a 4-, 5-, 6- or 7-membered heterocycloalkyl group;
      R¹¹ and R¹² are independently selected from H and -E¹-E²-E³-E⁴;
      D¹ and E¹ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
      D² and E² are independently absent or independently selected from C₁₋₆alkylene, C₂₋₆ alkenylene, C₂₋₆ alkynylene, (C₁₋₆alkylene)ᵣ-O-(C₁₋₆alkylene)ₛ, (C₁₋₆alkylene)ᵣ-S-(C₁₋₆alkylene)ₛ, (C₁₋₆ alkylene)ᵣ-NR^{e}-(C₁₋₆alkylene)ₛ, (C₁₋₆alkylene)ᵣ-CO-(C₁₋₆alkylene)ₛ, (C₁₋₆alkylene)ᵣ-COO-(C₁₋₆ alkylene)ₛ, (C₁₋₆alkylene)ᵣ-CONR^{e}-(C₁₋₆alkylene)ₛ, (C₁₋₆alkylene)ᵣ-SO-(C₁₋₆alkylene)ₛ, (C₁₋₆ alkylene)ᵣ-SO₂-(C₁₋₆alkylene)ₛ, (C₁₋₆alkylene)ᵣ-SONR^{e}-(C₁₋₆alkylene)ₛ, and (C₁₋₆alkylene)ᵣ-NR^{e}CONR^{f}-(C₁₋₆alkylene)ₛ, wherein each of the C₁₋₆alkylene, C₂₋₆ alkenylene, and C₂₋₆ alkynylene is optionally substituted by 1, 2 or 3 substituents independently selected from halo, CN, NO₂, N₃, SCN, OH, C₁₋₆alkyl, C₁₋₆haloalkyl, C₂₋₈ alkoxyalkyl, C₁₋₆alkoxy, C₁₋₆haloalkoxy, amino, C₁₋₆alkylamino, and C₂₋₈ dialkylamino;
      D³ and E³ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
      D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d,} S(O)R^{b}, S(O)NR^{c}R^{d}, S(O)₂R^{b}, NR^{c}S(O)²R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d,} S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
      R^{a} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
      R^{b} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, C₁₋₆ haloalkyl, halosulfanyl, aryl, arylalkyl, heteroaryl, heteroarylalkyl, cycloalkyl and heterocycloalkyl;
      R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
      R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      R^{c} and R^{d} are independently selected from H, Cy¹, -(C₁₋₆alkyl)-Cy¹, C₁₋₁₀alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆alkyl, C₁₋₆haloalkyl, C₁₋₆haloalkyl,and halosulfanyl;
      or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆alkyl)-Cy¹, OH, CN, amino, halo, C₁₋₆alkyl, C₁₋₆haloalkyl, C₁₋₆haloalkyl, and halosulfanyl;
      R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, halosulfanyl, C₁₋₆haloalkyl, aryl, arylalkyl, heteroaryl, heteroarylalkyl, cycloalkyl and heterocycloalkyl;
      or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      Rⁱ is H, CN, NO₂, or C₁₋₆alkyl;
      R^{e} and R^{f} are independently selected from H and C₁₋₆ alkyl;
      Rⁱ is H, CN, or NO₂;
      m is 0 or 1;
      n is 0 or 1;
      p is 0, 1, 2, 3, 4, 5, or 6;
      q is 0, 1, 2, 3, 4, 5 or 6;
      r is 0 or 1; and
      s is 0 or 1;
      wherein when X is N, n is 1, and the moiety formed by A¹, A², U, T, V, and -(Y)ₙ-Z has the formula: 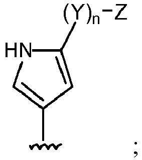 then Y is other than (CR¹¹R¹²)ₚC(O)NR^{c}(CR¹¹R¹²)_{q};
   (b) compounds of Formula II: 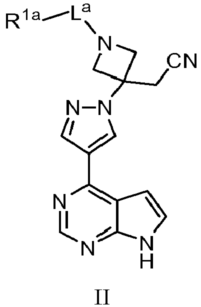 wherein:
      L^{a} is SO₂ or CO;
      R^{1a} is C₁₋₆alkyl, C₃₋₇ cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
      R^{2a} and R^{3a} are independently selected from H, C₁₋₄ alkyl, and phenyl; and
      R^{4a} is C₁₋₆alkyl, phenyl, or benzyl;
   (c) compounds of Formula III: 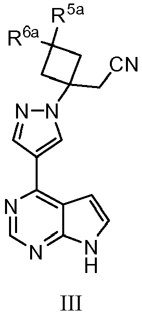 wherein:
      R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
      and pharmaceutically acceptable salts thereof;
      provided that compound of Formula I is not selected from 4-[5-(2-isopropyl-5-methylcyclohexyloxymethyl)-1,2-4-oxadiazol-3-yl]-1H-pyrrolo[2,3-b]pyridine; 4-[5-(4-tert-butylphenoxymethyl)-1,2-4-oxadiazol-3-yl]-1H-pyrrolo[2,3-b]pyridine; 4-[5-cyclopentylethyl-1,2-4-oxadiazol-3-yl]-1H-pyrrolo[2,3-b]pyridine; 4-[5-(2,6-difluorophenyl)-1,2-4-oxadiazol-3-yl]-1H-pyrrolo[2,3-b]pyridine; 4-[5-(1-tert-butyl-3-methyl-1H-pyrazol-5-yl)-1,2-4-oxadiazol-3-yl]-1H-pyrrolo[2,3-b]pyridine; 4-[5-(benzyloxymethyl)-1,2-4-oxadiazol-3-yl]-1H-pyrrolo[2,3-b]pyridine; 4-[5-(3-fluorophenyl)-1,2-4-oxadiazol-3-yl]-1H-pyrrolo[2,3-b]pyridine; 4-[5-(phenoxymethyl)-1,2-4-oxadiazol-3-yl]-1H-pyrrolo[2,3-b]pyridine; 4-[5-(4-methoxybenzyl)-1,2-4-oxadiazol-3-yl]-1H-pyrrolo[2,3-b]pyridine; 4-[5-(phenylthiomethyl)-1,2-4-oxadiazol-3-yl]-1H-pyrrolo[2,3-b]pyridine; 4-[5-(3-methylbutyl)-1,2-4-oxadiazol-3-yl]-1H-pyrrolo[2,3-b]pyridine; 4-[5-benzyl-1,2-4-oxadiazol-3-yl]-1H-pyrrolo[2,3-b]pyridine; 4-[5-(2,2-dimethylpropyl)-1,2-4-oxadiazol-3-yl]-1H-pyrrolo[2,3-b]pyridine; 4-[5-methyl-1,2-4-oxadiazol-3-yl]-1H-pyrrolo[2,3-b]pyridine; 4-[5-(formyl)-1,2-4-oxadiazol-3-yl]-1H-pyrrolo[2,3-b]pyridine; 4-[5-(furan-2-yl)-1,2-4-oxadiazol-3-yl]-1H-pyrrolo[2,3-b]pyridine; 4-[5-(1-methyl-1H-pyrrol-2-yl)-1,2-4-oxadiazol-3-yl]-1H-pyrrolo[2,3-b]pyridine; 4-[5-(sec-butyl)-1,2-4-oxadiazol-3-yl]-1H-pyrrolo[2,3-b]pyridine; 4-[5-cyclopropyl-1,2-4-oxadiazol-3-yl]-1H-pyrrolo[2,3-b]pyridine; and pharmaceutically acceptable salts of any of the aforementioned.
54. A method of treating conjunctivitis, uveitis, chorioditis, retinitis, cyclitis, sclieritis, episcleritis, or iritis; treating inflammation or pain related to corneal transplant, LASIK (laser assisted in situ keratomileusis), photorefractive keratectomy, or LASEK (laser assisted sub-epithelial keratomileusis); inhibiting loss of visual acuity related to corneal transplant, LASIK, photorefractive keratectomy, or LASEK; or inhibiting transplant rejection related to corneal transplant in a patient in need thereof, comprising administering to the patient a therapeutically effective amount of an agent; wherein said agent is selected from:
   (a) compounds of Formula I: 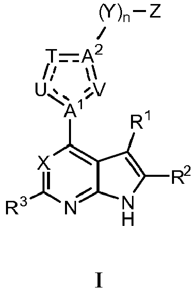 wherein:
      A¹ and A² are independently selected from C and N;
      T, U, and V are independently selected from O, S, N, CR⁵, and NR⁶;
      wherein the 5-membered ring formed by A¹, A², U, T, and V is aromatic;
      X is N or CR⁴;
      Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
      Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)²R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d,} S(O)R^{b}, S(O)NR^{c}R^{d}, S(O)₂R^{b}, NR^{c}S(O)²R^{b}, C(=NOH)R^{b}, C(=NO(C₁₋₆ alkyl))R^{b}, and S(O)₂NR^{c}R^{d};
      wherein when Z is H, n is 1;
      or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from-(W)ₘ-Q;
      W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c'}, NR^{c'}, NR^{c'}C(O)NR^{d'}, S(O), S(O)NR^{c'}, S(O)₂, or S(O)₂NR^{c'};
      Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
      Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
      R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{C}C(O)OR⁷, S(O)R⁸, S(O)NR⁹R¹⁰, S(O)₂R⁸, NR⁹S(O)₂R⁸, and S(O)₂NR⁹R¹⁰;
      R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
      R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
      R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
      R⁹ and R¹⁰ are independently selected from H, C₁₋₁₀ alkyl, C₁₋₆ haloalkyl, C₂₋₆ alkenyl, C₂₋₆ alkynyl, C₁₋₆alkylcarbonyl, arylcarbonyl, C₁₋₆alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
      or R⁹ and R¹⁰ together with the N atom to which they are attached form a 4-, 5-, 6- or 7-membered heterocycloalkyl group;
      R¹¹ and R¹² are independently selected from H and -E¹-E²-E³-E⁴;
      D¹ and E¹ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
      D² and E² are independently absent or independently selected from C₁₋₆alkylene, C₂₋₆ alkenylene, C₂₋₆ alkynylene, (C₁₋₆alkylene)ᵣ-O-(C₁₋₆alkylene)ₛ, (C₁₋₆alkylene)ᵣ-S-(C₁₋₆alkylene)ₛ, (C₁₋₆ alkylene)ᵣ-NR^{e}-(C₁₋₆alkylene)ₛ, (C₁₋₆alkylene)ᵣ-CO-(C₁₋₆alkylene)ₛ, (C₁₋₆alkylene)ᵣ-COO-(C₁₋₆ alkylene)ₛ, (C₁₋₆alkylene)ᵣ-CONR^{e}-(C₁₋₆alkylene)ₛ, (C₁₋₆alkylene)ᵣ-SO-(C₁₋₆alkylene)ₛ, (C₁₋₆ alkylene)ᵣ-SO₂-(C₁₋₆alkylene)ₛ, (C₁₋₆alkylene)ᵣ-SONR^{e}-(C₁₋₆alkylene)ₛ, and (C₁₋₆alkylene)ᵣ-NR^{e}CONR^{f}-(C₁₋₆alkylene)ₛ, wherein each of the C₁₋₆alkylene, C₂₋₆ alkenylene, and C₂₋₆ alkynylene is optionally substituted by 1, 2 or 3 substituents independently selected from halo, CN, NO₂, N₃, SCN, OH, C₁₋₆alkyl, C₁₋₆haloalkyl, C₂₋₈ alkoxyalkyl, C₁₋₆alkoxy, C₁₋₆haloalkoxy, amino, C₁₋₆alkylamino, and C₂₋₈ dialkylamino;
      D³ and E³ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
      D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)²R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d,} S(O)R^{b}, S(O)NR^{c}R^{d}, ,S(O)₂R^{b}, NR^{c}S(O)²R^{b}, C(=NOH)R^{b}, C(=NO(C₁₋₆ alkyl))R^{b}, and S(O)₂NR^{c}R^{d};
      R^{a} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
      R^{b} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, C₁₋₆ haloalkyl, halosulfanyl, aryl, arylalkyl, heteroaryl, heteroarylalkyl, cycloalkyl and heterocycloalkyl;
      R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
      R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      R^{c} and R^{d} are independently selected from H, Cy¹, -(C₁₋₆alkyl)-Cy¹, C₁₋₁₀ alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆alkyl, C₁₋₆haloalkyl, C₁₋₆haloalkyl,and halosulfanyl;
      or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆alkyl)-Cy¹, OH, CN, amino, halo, C₁₋₆alkyl, C₁₋₆haloalkyl, C₁₋₆haloalkyl, and halosulfanyl;
      R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      or R^{c}' and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, halosulfanyl, C₁₋₆haloalkyl, aryl, arylalkyl, heteroaryl, heteroarylalkyl, cycloalkyl and heterocycloalkyl;
      or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
      Rⁱ is H, CN, NO₂, or C₁₋₆alkyl;
      R^{e} and R^{f} are independently selected from H and C₁₋₆ alkyl;
      Rⁱ is H, CN, or NO₂;
      m is 0 or 1;
      n is 0 or 1;
      p is 0, 1, 2, 3, 4, 5, or 6;
      q is 0, 1, 2, 3, 4, 5 or 6;
      r is 0 or 1; and
      s is 0 or 1;
      wherein when X is N, n is 1, and the moiety formed by A¹, A², U, T, V, and -(Y)ₙ-Z has the formula: 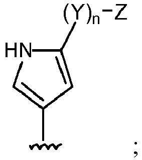 then Y is other than (CR¹¹R¹²)ₚC(O)NR^{c}(CR¹¹R¹²)_{q};
   (b) compounds of Formula II: 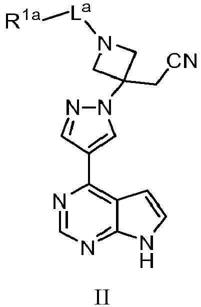 wherein:
      L^{a} is SO₂ or CO;
      R^{1a} is C₁₋₆alkyl, C₃₋₇ cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
      R^{2a} and R^{3a} are independently selected from H, C₁₋₄ alkyl, and phenyl; and
      R^{4a} is C₁₋₆alkyl, phenyl, or benzyl;
   (c) compounds of Formula III: 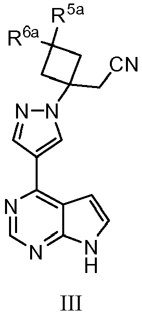 wherein:
      R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
      and pharmaceutically acceptable salts thereof.
55. The method according to embodiment 54, wherein said agent is administered preoperatively to a patient about to undergo a procedure selected from corneal transplant, LASIK, photorefractive keratectomy, and LASEK.
56. The method according to embodiment 54, wherein said agent is administered postoperatively to a patient who has undergone a procedure selected from corneal transplant, LASIK, photorefractive keratectomy, and LASEK.

## Claims

1. An agent for use in treating a dry eye disorder in a patient wherein said agent is selected from:
(a) compounds of Formula I: 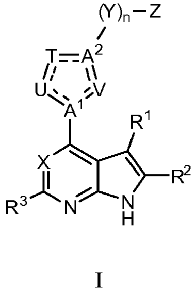 wherein:
A¹ and A² are independently selected from C and N;
T, U, and V are independently selected from O, S, N, CR⁵, and NR⁶;
wherein the 5-membered ring formed by A¹, A², U, T, and V is aromatic;
X is N or CR⁴;
Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ₋(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)²R^{b}, C(=NOH)R^{b}, C(=NO(C₁₋₆ alkyl)R^{b}, and S(O)₂NR^{c}R, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)²R^{b}, C(=NOH)R^{b}, C(=NO(C₁₋₆ alkyl))R^{b}, and S(O)₂NR^{c}R^{d};
wherein when Z is H, n is 1;
or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from-(W)ₘ-Q;
W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c}, NR^{c}, NR^{c}C(O)NR^{d}, S(O), S(O)NR^{c'}, S(O)₂, or S(O)₂NR^{c'};
Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"} R^{d"}, NR^{c"} C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ O(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁹ and R¹⁰ are independently selected from H, C₁₋₁₀ alkyl, C₁₋₆ haloalkyl, C₂₋₆ alkenyl, C₂₋₆ alkynyl, C₁₋₆alkylcarbonyl, arylcarbonyl, C₁₋₆alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
or R⁹ and R¹⁰ together with the N atom to which they are attached form a 4-, 5-, 6- or 7-membered heterocycloalkyl group;
R¹¹ and R¹² are independently selected from H and -E¹-E²-E³-E⁴;
D¹ and E¹ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D² and E² are independently absent or independently selected from C₁₋₆ alkylene, C₂₋₆ alkenylene, C₂₋₆ alkynylene, (C₁₋₆alkylene)ᵣ-O-( C₁₋₆alkylene)s, (C₁₋₆alkylene)r-S-(C₁₋₆alkylene)s, (C₁₋₆ alkylene)ᵣ-NR^{e}-(C₁₋₆alkylene)ₛ, (C₁₋₆alkylene)ᵣ-CO-(C₁₋₆alkylene)ₛ, (C₁₋₆ alkylene)ᵣ-COO-(C₁₋₆ alkylene)ₛ, (C₁₋₆alkylene)ᵣ-CONR^{e}-(C₁₋₆alkylene)ₛ, (C₁₋₆alkylene)ᵣ-SO-(C₁₋₆alkylene)ₛ, (C₁₋₆ alkylene)ᵣ-SO₂-(C₁₋₆ alkylene)ₛ, (C₁₋₆alkylene)ᵣ-SONR^{e}-(C₁₋₆alkylene)ₛ, and (C₁₋₆alkylene)ᵣ NR^{e}CONR^{f}(C₁₋₆alkylene)ₛ, wherein each of the C₁₋₆ alkylene, C₂₋₆ alkenylene, and C₂₋₆ alkynylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D³ and E³ are independently absent or independently selected from C₁₋₆ alkylene, C₂₋₆ alkenylene, C₂₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
R^{a} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
R^{b} is H, Cy¹, -(C₁₋₆alkyl)-Cyl, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, C₁₋₆ haloalkyl, halosulfanyl, aryl, arylalkyl, heteroaryl, heteroarylalkyl, cycloalkyl and heterocycloalkyl;
R^{a}' and R^{a''} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{b'} and R^{b''} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{c} and R^{d} are independently selected from H, Cy¹, -(C₁₋₆alkyl)-Cy¹, C₁₋₁₀ alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆alkyl, C₁₋₆haloalkyl, C₁₋₆haloalkyl, and halosulfanyl;
or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆alkyl)-Cyl, OH, CN, amino, halo, C₁₋₆alkyl, C₁₋₆haloalkyl, C₁₋₆haloalkyl, and halosulfanyl;
R^{c'} and R^{d}' are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
or R^{c}' and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{c''} and R^{d''} are independently selected from H, C₁₋₁₀ alkyl, C₁₋₆ haloalkyl, C₂₋₆ alkenyl, C₁₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₁₋₆ alkenyl, C₁₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, C₁₋₆ haloalkyl, aryl, arylalkyl, heteroaryl, heteroarylalkyl, cycloalkyl and heterocycloalkyl;
or R^{c}" and R^{d''} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
Rⁱ is H, CN, NO₂, or C₁₋₆ alkyl;
R^{e} and R^{f} are independently selected from H and C₁₋₆ alkyl;
Rⁱ is H, CN, or NO₂;
m is 0 or 1;
n is 0 or 1;
p is 0, 1, 2, 3, 4, 5, or 6;
q is 0, 1, 2, 3, 4, 5 or 6;
r is 0 or 1; and
s is 0 or 1;
wherein when X is N, n is 1, and the moiety formed by A¹, A², U, T, V, and -(Y)ₙ-Z has the formula: 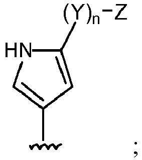 then Y is other than (CR¹¹R¹²)ₚC(O)NR^{c}(CR¹¹R¹²)_{q};
(b) compounds of Formula II: 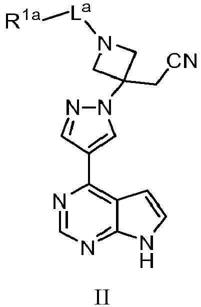 wherein:
L^{a} is SO₂ or CO;
R^{1a} is C₁₋₆alkyl, C₃₋₇ cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
R^{2a} and R^{3a} are independently selected from H, C₁₋₄ alkyl, and phenyl; and
R^{4a} is C₁₋₆ alkyl, phenyl, or benzyl;
(c) compounds of Formula III: 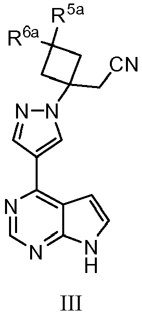 wherein:
R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
and pharmaceutically acceptable salts thereof;
provided that compound of Formula I is not selected from 4-[5-(2-isopropyl-5-methylcyclohexyloxymethyl)-1,2-4-oxadiazol-3-yl]-1H-pyrrolo[2,3-b]pyridine; 4-[5-(4-tert-butylphenoxymethyl)-1,2-4-oxadiazol-3-yl]-1H-pyrrolo[2,3-b]pyridine; 4-[5-cyclopentylethyl-1,2-4-oxadiazol-3-yl]-1H-pyrrolo[2,3-b]pyridine; 4-[5-(2,6-difluorophenyl)-1,2-4-oxadiazol-3-yl]-1H-pyrrolo[2,3-b]pyridine; 4-[5-(1-tert-butyl-3-methyl-1H-pyrazol-5-yl)-1,2-4-oxadiazol-3-yl]-1H-pyrrolo[2,3-b]pyridine; 4-[5-(benzyloxymethyl)-1,2-4-oxadiazol-3-yl]-1H-pyrrolo[2,3-b]pyridine; 4-[5-(3-fluorophenyl)-1,2-4-oxadiazol-3-yl]-1H-pyrrolo[2,3-b]pyridine; 4-[5-(phenoxymethyl)-1,2-4-oxadiazol-3-yl]-1H-pyrrolo[2,3-b]pyridine; 4-[5-(4-methoxybenzyl)-1,2-4-oxadiazol-3-yl]-1H-pyrrolo[2,3-b]pyridine; 4-[5-(phenylthiomethyl)-1,2-4-oxadiazol-3-yl]-1H-pyrrolo[2,3-b]pyridine; 4-[5-(3-methylbutyl)-1,2-4-oxadiazol-3-yl]-1H-pyrrolo[2,3-b]pyridine; 4-[5-benzyl-1,2-4-oxadiazol-3-yl]-1H-pyrrolo[2,3-b]pyridine;4-[5-(2,2-dimethylpropyl)-1,2-4-oxadiazol-3-yl]-1H-pyrrolo[2,3-b]pyridine; 4-[5-methyl-1,2-4-oxadiazol-3-yl]-1H-pyrrolo[2,3-b]pyridine; 4-[5-(formyl)-1,2-4-oxadiazol-3-yl]-1H-pyrrolo[2,3-b]pyridine; 4-[5-(furan-2-yl)-1,2-4-oxadiazol-3-yl]-1H-pyrrolo[2,3-b]pyridine; 4-[5-(1-methyl-1H-pyrrol-2-yl)-1,2-4-oxadiazol-3-yl]-1H-pyrrolo[2,3-b]pyridine; 4-[5-(sec-butyl)-1,2-4-oxadiazol-3-yl]-1H-pyrrolo[2,3-b]pyridine; 4-[5-cyclopropyl-1,2-4-oxadiazol-3-yl]-1H-pyrrolo[2,3-b]pyridine; and pharmaceutically acceptable salts of any of the aforementioned.

2. The agent for use according to claim 1, wherein said dry eye disorder is:
(a) aqueous tear-deficient dry eye;
(b) evaporative dry eye;
(c) Sjogren syndrome dry eye; or
(d) non-Sjogren syndrome dry eye.

3. The agent for use according to claim 1, wherein said treating comprises ameliorating a symptom selected from eye discomfort, visual disturbance, tear film instability, tear hyperosmolarity, and inflammation of the ocular surface.

4. The agent for use according to any one of claims 1 to 3, wherein said treating comprises administering an ophthalmic composition to said patient, said composition comprising said agent and an ophthalmically acceptable carrier, and wherein said ophthalmic composition is:
(a) a topical composition; or
(b) a topical composition which is an aqueous formulation, an aqueous suspension, an ointment or a gel.

5. The agent for use according to any one of claims 1 to 4, wherein said treating further comprises administering at least one additional therapeutic agent; optionally wherein said additional therapeutic agent is:
(a) fluocinolone acetonide, rimexolone, cyclosporine, riaminolone, dexamethasone, fluocinolone, cortisone, prednisolone, flumetholone, civamide, testosterone, ecabet sodium, 15-(s)-hydroxyeicosatetraenoic acid, 2S,3S,4R,5R)-3,4-dihydroxy-5-[6-[(3-iodophenyl)methylamino]purin-9-yl]-N-m ethyl-oxolane-2-carboxamide, gefarnate, cevilemine, doxycline, minocycline, oxytetracycline, voclosporin, rivoglitazone, lacritin rebamipide, pilocarpine, tacrolimus, pimecrolimus, loteprednol etabonate, rituximab, diquafosol tetrasodium, dehydroepiandrosterone, anakinra, efalizumab, mycophenolate sodium, etanercept, hydroxychloroquine, or thalidomide; or
(b) sodium hyaluronate, hyaluronic acid, polyvinylalcohol, hydroxypropyl methylcellulose, glycerin, polyethylene glycol, or carboxymethyl cellulose.

6. The agent for use according to any one of claims 1 to 5, wherein said agent is selected from compounds of Formula I: 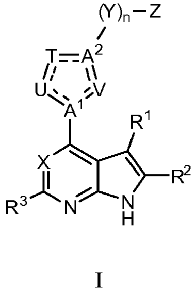 and pharmaceutically acceptable salts thereof; and wherein:
(a) n is 1 and Y is C₁₋₈ alkylene optionally substituted with 1, 2, or 3 substituents independently selected from D⁴;
(b) n is 1 and Y is C₁₋₈ alkylene optionally substituted with 1, 2, or 3 halo, OH, CN, amino, C₁₋₄ alkylamino, or C₂₋₈ dialkylamino; or
(c) n is 1 and Y is C₁₋₈ alkylene optionally substituted with cyano; and/or
(d) Z is: (i) cyclopropyl, cyclobutyl, cyclopentyl, cyclohexyl, or cycloheptyl, each optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}; (ii) cyclopentyl, which is optionally substituted with 1, 2, 3, 4, 5, or 6 substituents selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, and S(O)₂NR^{c}R^{d}; or (iii) cyclopentyl.

7. The agent for use according to claim 6, wherein:
(a) X is N; and/or
(b) A¹ is C; and/or
(c) A² is N; and/or
(d) T is N; and/or
(e) U and V are independently CR⁵; and/or
(f) R¹, R², R³, and R⁴ are each H.

8. The agent for use according to claim 6 or claim 7, wherein:
(a) the 5-membered ring formed by A¹, A², U, T, and V is selected from: 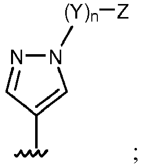 or
(b) said agent is selected from compounds having Formula I1: 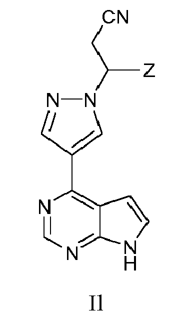 and pharmaceutically acceptable salts thereof; or
(c) the moiety formed by T, U, V, A¹, and A² is not the following moiety:

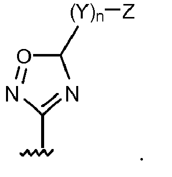

9. The agent for use according to any one of claims 1 to 5, wherein said agent is selected from:
(a) 3-cyclopentyl-3-[4-(7H-pyrrolo[2,3-d]pyrimidin-4-yl)-1H-pyrazol-1-yl]propanenitrile and pharmaceutically acceptable salts thereof; or (b) (3R)-3-cyclopentyl-3-[4-(7H-pyrrolo[2,3-d]pyrimidin-4-yl)-1H-pyrazol-1-yl]propanenitrile and pharmaceutically acceptable salts thereof.

10. The agent for use according to any one of claims 1 to 5, wherein said agent is selected from compounds of Formula II: 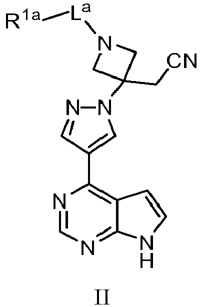 and pharmaceutically acceptable salts thereof; and wherein:
(a) L^{a} is SO₂; and/or (b) R^{1a} is (i) C₁₋₄ alkyl, or (ii) ethyl.

11. The agent for use according to any one of claims 1 to 5, wherein said agent is selected from:
(a) {1-(Ethylsulfonyl)-3-[4-(7H-pyrrolo[2,3-d]pyrimidin-4-yl)-1H-pyrazol-1-yl]azetidin-3-yl}acetonitrile and pharmaceutically acceptable salts thereof; or (b) {1-(Ethylsulfonyl)-3-[4-(7H-pyrrolo[2,3-d]pyrimidin-4-yl)-1H-pyrazol-1-yl]azetidin-3-yl}acetonitrile phosphoric acid salt.

12. A kit for treatment of dry eye disorder, comprising an ophthalmic or pharmaceutical composition and instructions;
wherein said composition comprises a therapeutically effective amount of an agent selected from:
(a) compounds of Formula I: 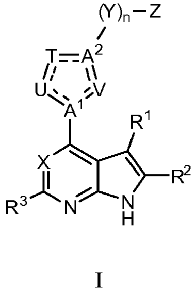 wherein:
A¹ and A² are independently selected from C and N;
T, U, and V are independently selected from O, S, N, CR⁵, and NR⁶;
wherein the 5-membered ring formed by A¹, A², U, T, and V is aromatic;
X is N or CR⁴;
Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹²R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
wherein when Z is H, n is 1;
or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from-(W)ₘ-Q;
W is C₁₋₈ alkylenyl, C₂₋₈ alkenylenyl, C₂₋₈ alkynylenyl, O, S, C(O), C(O)NR^{c}, C(O)O, OC(O), OC(O)NR^{c}, NR^{c'}, NR^{c'}C(O)NR^{d'}, S(O), S(O)NR^{c'}, S(O)₂, or S(O)₂NR^{c'};
Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b''}, OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)OR⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)OR⁷, S(O)R⁸, S(O)NR⁹R¹⁰, S(O)₂R⁸, or S(O)₂NR⁹R¹⁰;
R⁷ is H, C₁₋₆ alkyl, C₁₋₆ haloalkyl, C₁₋₆ alkenyl, C₁₋₆ alkynyl, aryl, cycloalkyl, heteroaryl, heterocycloalkyl, arylalkyl, heteroarylalkyl, cycloalkylalkyl or heterocycloalkylalkyl;
R⁸ is H, C₁₋₆ alkyl, C₁₋₆ haloalkyl, C₁₋₆ alkenyl, C₁₋₆ alkynyl, aryl, cycloalkyl, heteroaryl, heterocycloalkyl, arylalkyl, heteroarylalkyl, cycloalkylalkyl or heterocycloalkylalkyl;
R⁹ and R¹⁰ are independently selected from H, C₁₋₁₀ alkyl, C₁₋₆ haloalkyl, C₁₋₆ alkenyl, C₁₋₆ alkynyl, C₁₋₆alkylcarbonyl, arylcarbonyl, C₁₋₆alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
or R⁹ and R¹⁰ together with the N atom to which they are attached form a 4-, 5-, 6- or 7-membered heterocycloalkyl group;
R¹¹ and R¹² are independently selected from H and -E¹-E²-E³-E⁴;
D¹ and E¹ are independently absent or independently selected from C₁₋₆alkylene, C₁₋₆ alkenylene, C₁₋₆ alkynylene, arylene, cycloalkylene, heteroarylene, and heterocycloalkylene, wherein each of the C₁₋₆ alkylene, C₁₋₆ alkenylene, C₁₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D² and E² are independently absent or independently selected from C₁₋₆ alkylene, C₁₋₆ alkenylene, C₂₋₆ alkynylene, (C₁₋₆alkylene)ᵣ-O-( C₁₋₆alkylene)s, (C₁₋₆alkylene)-S-(C₁₋₆alkylene)s, (C₁₋₆ alkylene)ᵣ-NR^{e}-(C₁₋₆ alkylene)ₛ, (C₁₋₆alkylene)ᵣCO-(C₁₋₆alkylene)ₛ, (C₁₋₆alkylene)ᵣ-COO-(C₁₋₆ alkylene)ₛ, (C₁₋₆alkylene)ᵣ-CONR^{e}-(C₁₋₆alkylene)ₛ, (C₁₋₆alkylene)ᵣ-SO-(C₁₋₆alkylene)ₛ, (C₁₋₆ alkylene)ᵣ-SO₂-(d₁₋₆ alkylene)ₛ, (C₁₋₆alkylene)ᵣ-SONR^{e}-(C₁₋₆alkylene)ₛ, and (C₁₋₆alkylene)ᵣ NR^{e}CONR^{f}-(C₁₋₆alkylene)ₛ, wherein each of the C₁₋₆ alkylene, C₁₋₆ alkenylene, and C₁₋₆ alkynylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D³ and E³ are independently absent or independently selected from C₁₋₆ alkylene, C₁₋₆ alkenylene, C₁₋₆ alkynylene, arylene, cycloalkylene, heteroarylene, and heterocycloalkylene, wherein each of the C₁₋₆ alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆ alkoxy, C₁₋₆ haloalkoxy, amino, C₁₋₆ alkylamino, and C₂₋₈ dialkylamino;
D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NRⁱR^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
R^{a} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
R^{b} is H, Cy¹, -(C₁₋₆alkyl)-Cyl, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, C₁₋₆ haloalkyl, halosulfanyl, aryl, arylalkyl, heteroaryl, heteroarylalkyl, cycloalkyl and heterocycloalkyl;
R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{b'} and R^{b''} are independently selected from H, C₁₋₆ alkyl, C₁₋₆ haloalkyl, C₁₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
R^{c} and R^{d} are independently selected from H, Cy¹, -(C₁₋₆alkyl)-Cy¹, C₁₋₁₀alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆alkyl, C₁₋₆haloalkyl, C₁₋₆haloalkyl,and halosulfanyl;
or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆alkyl)-Cy¹, OH, CN, amino, halo, C₁₋₆alkyl, C₁₋₆haloalkyl, C₁₋₆haloalkyl, and halosulfanyl;
R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, halosulfanyl, C₁₋₆haloalkyl, aryl, arylalkyl, heteroaryl, heteroarylalkyl, cycloalkyl and heterocycloalkyl;
or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁₋₆haloalkyl, C₁₋₆haloalkyl, halosulfanyl, aryl, arylalkyl, heteroaryl, heteroarylalkyl, cycloalkyl and heterocycloalkyl;
Rⁱ is H, CN, NO₂, or C₁₋₆ alkyl;
R^{e} and R^{f} are independently selected from H and C₁₋₆ alkyl;
R¹ is H, CN, or NO₂;
m is 0 or 1;
n is 0 or 1;
p is 0, 1, 2, 3, 4, 5, or 6;
q is 0, 1, 2, 3, 4, 5 or 6;
r is 0 or 1; and
s is 0 or 1;
wherein when X is N, n is 1, and the moiety formed by A¹, A², U, T, V, and -(Y)ₙ-Z has the formula: 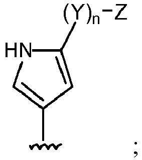 then Y is other than (CR¹¹R¹²)ₚC(O)NR^{c}(CR¹¹R¹²)_{q};
(b) compounds of Formula II: 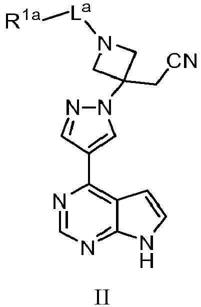 wherein:
L^{a} is SO₂ or CO;
R^{1a} is C₁₋₆alkyl, C₃₋₇ cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
R^{2a} and R^{3a} are independently selected from H, C₁₋₄ alkyl, and phenyl; and
R^{4a} is C₁₋₆ alkyl, phenyl, or benzyl;
(c) compounds of Formula III: 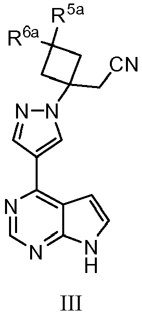 wherein:
R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
and pharmaceutically acceptable salts thereof;
provided that compound of Formula I is not selected from 4-[5-(2-isopropyl-5-methylcyclohexyloxymethyl)-1,2-4-oxadiazol-3-yl]-1H-pyrrolo[2,3-b]pyridine; 4-[5-(4-tert-butylphenoxymethyl)-1,2-4-oxadiazol-3-yl]-1H-pyrrolo[2,3-b]pyridine; 4-[5-cyclopentylethyl-1,2-4-oxadiazol-3-yl]-1H-pyrrolo[2,3-b]pyridine; 4-[5-(2,6-difluorophenyl)-1,2-4-oxadiazol-3-yl]-1H-pyrrolo[2,3-b]pyridine; 4-[5-(1-tert-butyl-3-methyl-1H-pyrazol-5-yl)-1,2-4-oxadiazol-3-yl]-1H-pyrrolo[2,3-b]pyridine; 4-[5-(benzyloxymethyl)-1,2-4-oxadiazol-3-yl]-1H-pyrrolo[2,3-b]pyridine; 4-[5-(3-fluorophenyl)-1,2-4-oxadiazol-3-yl]-1H-pyrrolo[2,3-b]pyridine; 4-[5-(phenoxymethyl)-1,2-4-oxadiazol-3-yl]-1H-pyrrolo[2,3-b]pyridine; 4-[5-(4-methoxybenzyl)-1,2-4-oxadiazol-3-yl]-1H-pyrrolo[2,3-b]pyridine; 4-[5-(phenylthiomethyl)-1,2-4-oxadiazol-3-yl]-1H-pyrrolo[2,3-b]pyridine; 4-[5-(3-methylbutyl)-1,2-4-oxadiazol-3-yl]-1H-pyrrolo[2,3-b]pyridine; 4-[5-benzyl-1,2-4-oxadiazol-3-yl]-1H-pyrrolo[2,3-b]pyridine; 4-[5-(2,2-dimethylpropyl)-1,2-4-oxadiazol-3-yl]-1H-pyrrolo[2,3-b]pyridine; 4-[5-methyl-1,2-4-oxadiazol-3-yl]-1H-pyrrolo[2,3-b]pyridine; 4-[5-(formyl)-1,2-4-oxadiazol-3-yl]-1H-pyrrolo[2,3-b]pyridine; 4-[5-(furan-2-yl)-1,2-4-oxadiazol-3-yl]-1H-pyrrolo[2,3-b]pyridine; 4-[5-(1-methyl-1H-pyrrol-2-yl)-1,2-4-oxadiazol-3-yl]-1H-pyrrolo[2,3-b]pyridine; 4-[5-(sec-butyl)-1,2-4-oxadiazol-3-yl]-1H-pyrrolo[2,3-b]pyridine; 4-[5-cyclopropyl-1,2-4-oxadiazol-3-yl]-1 H-pyrrolo [2,3-b]pyridine; and pharmaceutically acceptable salts of any of the aforementioned;
and wherein said instructions comprise a direction to administer said composition to a patient in need of treatment of said dry eye disorder.

13. Use of an agent for the preparation of a medicament for the treatment of a dry eye disorder; wherein said agent is selected from:
(a) compounds of Formula I: 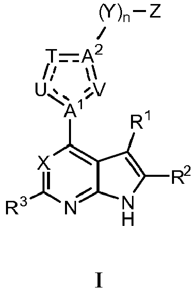 wherein:
A¹ and A² are independently selected from C and N;
T, U, and V are independently selected from O, S, N, CR⁵, and NR⁶;
wherein the 5-membered ring formed by A¹, A², U, T, and V is aromatic;
X is N or CR⁴_{;}
Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚS(CR¹¹R¹²)_{q}, (CR¹¹R¹²)ₚC(O)(CR¹¹R¹²)_{q}, (CR¹¹R¹²)ₚC(O)NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS(O)(CR¹¹R¹²)_{q}, (CR¹¹R¹²)ₚS(O)NR^{c}(CR¹¹R¹⁰)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
wherein when Z is H, n is 1;
or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from -(W)ₘ-Q;
W is C₁₋₈ alkylenyl, C₂₋₈ alkenylenyl, C₂₋₈ alkynylenyl, O, S, C(O), C(O)NR^{c}, C(O)O, OC(O), OC(O)NR^{c'}, NR^{c'}, NR^{c'}C(O)NR^{d'}, S(O), S(O)NR^{c'}, S(O)₂, or S(O)₂NR^{c'};
Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b}, C(O)NR^{c}R^{d}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c"}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{c}C(O)OR⁷, S(O)R⁸, S(O)NR⁹R¹⁰, S(O)₂R⁸, NR⁹S(O)₂R⁸, and S(O)₂NR⁹R¹⁰;
R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)0R⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)0R⁷, S(O)R⁸, S(O)NR⁹R¹⁰, S(O)₂R⁸, or S(O)₂NR⁹R¹⁰;
R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁹ and R¹⁰ are independently selected from H, C₁₋₁₀ alkyl, C₁₋₆ haloalkyl, C₂₋₆ alkenyl, C₂₋₆ alkynyl, C₁₋₆alkylcarbonyl, arylcarbonyl, C₁₋₆alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
or R⁹ and R¹⁰ together with the N atom to which they are attached form a 4-, 5-, 6- or 7-membered heterocycloalkyl group;
R¹¹ and R¹² are independently selected from H and -E¹-E²-E³-E⁴;
D¹ and E¹ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
D² and E² are independently absent or independently selected from C₁₋₆alkylene, C₂₋₆ alkenylene, C₂₋₆ alkynylene, (C₁₋₆alkylene)ᵣ-O-(C₁₋₆alkylene)ₛ, (C₁₋₆alkylene)ᵣ-S-(C₁₋₆alkylene)ₛ, (C₁₋₆ alkylene)ᵣ-NR^{e}-(C₁₋₆alkylene)ₛ, (C₁₋₆alkylene)ᵣ-CO-(C₁₋₆alkylene)ₛ, (C₁₋₆alkylene)ᵣ-COO-(C₁₋₆ alkylene)ₛ, (C₁₋₆alkylene)ᵣ-CONR^{e}-(C₁₋₆alkylene)ₛ, (C₁₋₆alkylene)ᵣ-SO-(C₁₋₆alkylene)ₛ, (C₁₋₆ alkylene)ᵣ-SO₂-(C₁₋₆alkylene)ₛ, (C₁₋₆alkylene)ᵣ-SONR^{e}-(C₁₋₆alkylene)ₛ, and (C₁₋₆alkylene)ᵣ-NR^{e}CONR^{f}-(C₁₋₆alkylene)ₛ, wherein each of the C₁₋₆alkylene, C₂₋₆ alkenylene, and C₂₋₆ alkynylene is optionally substituted by 1, 2 or 3 substituents independently selected from halo, CN, NO₂, N₃, SCN, OH, C₁₋₆alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
D³ and E³ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
R^{a} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
R^{b} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, C₁₋₆ haloalkyl, halosulfanyl, aryl, arylalkyl, heteroaryl, heteroarylalkyl, cycloalkyl and heterocycloalkyl;
R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{c} and R^{d} are independently selected from H, Cy¹, -(C₁₋₆alkyl)-Cy¹, C₁₋₁₀alkyl, C₁₋₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆alkyl, C₁₋₆haloalkyl, C₁₋₆haloalkyl,and halosulfanyl;
or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆alkyl)-Cy¹, OH, CN, amino, halo, C₁₋₆alkyl, C₁₋₆haloalkyl, C₁₋₆haloalkyl, and halosulfanyl;
R^{c'} and R^{d'} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{C} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, C₁₋₆ haloalkyl, aryl, arylalkyl, heteroaryl, heteroarylalkyl, cycloalkyl and heterocycloalkyl;
or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
Rⁱ is H, CN, NO₂, or C₁₋₆ alkyl;
R^{e} and R^{f} are independently selected from H and C₁₋₆ alkyl;
Rⁱ is H, CN, or NO₂;
m is 0 or 1;
n is 0 or 1;
p is 0, 1, 2, 3, 4, 5, or 6;
q is 0, 1, 2, 3, 4, 5 or 6;
r is 0 or 1; and
s is 0 or 1;
wherein when X is N, n is 1, and the moiety formed by A¹, A², U, T, V, and -(Y)ₙ-Z has the formula: 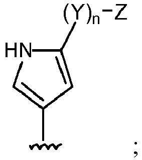 then Y is other than (CR¹¹R¹²)ₚC(O)NR^{c}(CR¹¹R¹²)_{q;}
(b) compounds of Formula II: 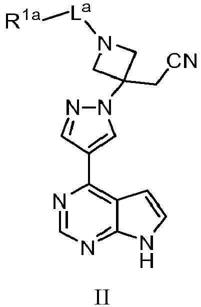 wherein:
L^{a} is SO₂ or CO;
R^{1a} is C₁₋₆alkyl, C₃₋₇cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
R^{2a} and R^{3a} are independently selected from H, C₁₋₄ alkyl, and phenyl; and
R^{4a} is C₁₋₆ alkyl, phenyl, or benzyl;
(c) compounds of Formula III: 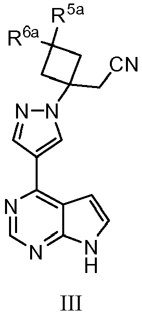 wherein:
R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
and pharmaceutically acceptable salts thereof;
provided that compound of Formula I is not selected from 4-[5-(2-isopropyl-5-methylcyclohexyloxymethyl)-1,2-4-oxadiazol-3-yl]-1H-pyrrolo[2,3-b]pyridine; 4-[5-(4-tert-butylphenoxymethyl)-1,2-4-oxadiazol-3-yl]-1H-pyrrolo[2,3-b]pyridine; 4-[5-cyclopentylethyl-1,2-4-oxadiazol-3-yl]-1H-pyrrolo[2,3-b]pyridine; 4-[5-(2,6-difluorophenyl)-1,2-4-oxadiazol-3-yl]-1H-pyrrolo[2,3-b]pyridine; 4-[5-(1-tert-butyl-3-methyl-1H-pyrazol-5-yl)-1,2-4-oxadiazol-3-yl]-1H-pyrrolo[2,3-b]pyridine; 4-[5-(benzyloxymethyl)-1,2-4-oxadiazol-3-yl]-1H-pyrrolo[2,3-b]pyridine; 4-[5-(3-fluorophenyl)-1,2-4-oxadiazol-3-yl]-1H-pyrrolo[2,3-b]pyridine; 4-[5-(phenoxymethyl)-1,2-4-oxadiazol-3-yl]-1H-pyrrolo[2,3-b]pyridine; 4-[5-(4-methoxybenzyl)-1,2-4-oxadiazol-3-yl]-1H-pyrrolo[2,3-b]pyridine; 4-[5-(phenylthiomethyl)-1,2-4-oxadiazol-3-yl]-1H-pyrrolo[2,3-b]pyridine; 4-[5-(3-methylbutyl)-1,2-4-oxadiazol-3-yl]-1H-pyrrolo[2,3-b]pyridine; 4-[5-benzyl-1,2-4-oxadiazol-3-yl]-1H-pyrrolo[2,3-b]pyridine; 4-[5-(2,2-dimethylpropyl)-1,2-4-oxadiazol-3-yl]-1H-pyrrolo[2,3-b]pyridine; 4-[5-methyl-1,2-4-oxadiazol-3-yl]-1H-pyrrolo[2,3-b]pyridine; 4-[5-(formyl)-1,2-4-oxadiazol-3-yl]-1H-pyrrolo[2,3-b]pyridine; 4-[5-(furan-2-yl)-1,2-4-oxadiazol-3-yl]-1H-pyrrolo[2,3-b]pyridine; 4-[5-(1-methyl-1H-pyrrol-2-yl)-1,2-4-oxadiazol-3-yl]-1H-pyrrolo[2,3-b]pyridine; 4-[5-(sec-butyl)-1,2-4-oxadiazol-3-yl]-1H-pyrrolo[2,3-b]pyridine; 4-[5-cyclopropyl-1,2-4-oxadiazol-3-yl]-1 H-pyrrolo [2,3-b]pyridine; and pharmaceutically acceptable salts of any of the aforementioned.

14. An agent for use in treating conjunctivitis, uveitis, chorioditis, retinitis, cyclitis, sclieritis, episcleritis, or iritis; treating inflammation or pain related to corneal transplant, LASIK (laser assisted in situ keratomileusis), photorefractive keratectomy, or LASEK (laser assisted sub-epithelial keratomileusis); inhibiting loss of visual acuity related to corneal transplant, LASIK, photorefractive keratectomy, or LASEK; or inhibiting transplant rejection related to corneal transplant in a patient in need thereof;
wherein said agent is selected from:
(a) compounds of Formula I: 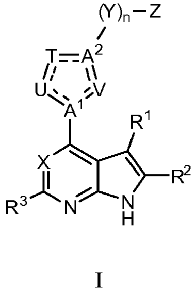 wherein:
A¹ and A² are independently selected from C and N;
T, U, and V are independently selected from O, S, N, CR⁵, and NR⁶;
wherein the 5-membered ring formed by A¹, A², U, T, and V is aromatic;
X is N or CR⁴;
Y is C₁₋₈ alkylene, C₂₋₈ alkenylene, C₂₋₈ alkynylene, (CR¹¹R¹²)ₚ-(C₃₋₁₀cycloalkylene)-(CR¹¹R¹²)_{q}, (CR¹¹R¹²)ₚ-(arylene)-(CR¹¹R¹²)_{q}, (CR¹¹R¹²)ₚ-(C₁₋₁₀heterocycloalkylene)-(CR¹¹R¹²)_{q}, (CR¹¹R¹²)ₚ-(heteroarylene)-(CR¹¹R¹²)_{q}, (CR¹¹R¹²)ₚO(CR¹¹R¹²)_{q}, (CR¹¹R¹²)ₚ-(CR¹¹R¹²)_{q}, (CR¹¹R¹²)ₚC(O)(CR¹¹R¹²)_{q}, (CR¹¹R¹²)ₚC()NR^{c}(CR¹¹R¹²)_{q}, (CR¹¹R¹²)ₚC(O)O(CR¹¹R¹²)_{q}, (CR¹¹R¹²)ₚOC(O)(CR¹¹R¹²)_{q}, (CR¹¹R¹²)ₚOC(O)NR^{c}(CR¹¹R¹²)_{q}, (CR¹¹R¹²)ₚNR^{c}(CR¹¹R¹²)_{q}, (CR¹¹R¹²)ₚNR^{c}C(O)NR^{d}(CR¹¹R¹²)_{q}, (CR¹¹R¹²)ₚS()(CR¹¹R¹²)_{q}, (CR¹¹R¹²)ₚS()NR^{c}(CR¹¹R¹²)_{q}, (CR¹¹R¹²)ₚS(O)₂(CR¹¹R¹²)_{q}, or (CR¹¹R¹²)ₚS(O)₂NR^{c}(CR¹¹R¹²)_{q}, wherein said C₁₋₈ alkylene, C₂₋₈ alkenylene, C₂₋₈ alkynylene, cycloalkylene, arylene, heterocycloalkylene, or heteroarylene, is optionally substituted with 1, 2, or 3 substituents independently selected from -D¹-D²-D³-D⁴;
Z is H, halo, C₁₋₈ alkyl, C₂₋₈ alkenyl, C₂₋₈ alkynyl, C₁₋₄ haloalkyl, halosulfanyl, C₁₋₄ hydroxyalkyl, C₁₋₄ cyanoalkyl, =C-Rⁱ, =N-Rⁱ, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
wherein when Z is H, n is 1;
or the -(Y)ₙ-Z moiety is taken together with i) A² to which the moiety is attached, ii) R⁵ or R⁶ of either T or V, and iii) the C or N atom to which the R⁵ or R⁶ of either T or V is attached to form a 4- to 20-membered aryl, cycloalkyl, heteroaryl, or heterocycloalkyl ring fused to the 5-membered ring formed by A¹, A², U, T, and V, wherein said 4- to 20-membered aryl, cycloalkyl, heteroaryl, or heterocycloalkyl ring is optionally substituted by 1, 2, 3, 4, or 5 substituents independently selected from -(W)ₘ-Q;
W is C₁₋₈ alkylenyl, C₂₋₈ alkenylenyl, C₂₋₈ alkynylenyl, O, S, C(O), C(O)NR^{c'}, C(O)O, OC(O), OC(O)NR^{c'}, NR^{c'}, NR^{c'}C(O)NR^{d"}, S(O), S(O)NR^{c'}, S(O)₂, or S(O)₂NR^{c'};
Q is H, halo, CN, NO₂, C₁₋₈ alkyl, C₂₋₈ alkenyl, C₂₋₈ alkynyl, C₁₋₈ haloalkyl, halosulfanyl, aryl, cycloalkyl, heteroaryl, or heterocycloalkyl, wherein said C₁₋₈ alkyl, C₂₋₈ alkenyl, C₂₋₈ alkynyl, C₁₋₈ haloalkyl, aryl, cycloalkyl, heteroaryl, or heterocycloalkyl is optionally substituted with 1, 2, 3 or 4 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy², CN, NO₂, OR^{a'}, SR^{a'}, C(O)R^{b'}, C(O)NR^{c'}R^{d'}, C(O)OR^{a'}, OC(O)R^{b'}, OC(O)NR^{c'}R^{d'}, NR^{c'}R^{d'}, NR^{c'}C(O)R^{b'}, NR^{c'}C(O)NR^{c'}R^{d'}, NR^{c'}C(O)OR^{a'}, S(O)R^{b'}, S(O)NR^{c'}R^{d'}, S(O)₂R^{b'}, NR^{c'}S(O)₂R^{b'}, and S(O)₂NR^{c'}R^{d'};
Cy¹ and Cy² are independently selected from aryl, heteroaryl, cycloalkyl, and heterocycloalkyl, each optionally substituted by 1, 2, 3, 4 or 5 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, CN, NO₂, OR^{a"}, SR^{a"}, C(O)R^{b"}, C(O)NR^{c"}R^{d"}, C(O)OR^{a"}, OC(O)R^{b"}, OC(O)NR^{c'}R^{d"}, NR^{c"}R^{d"}, NR^{c"}C(O)R^{b"}, NR^{c"}C(O)OR^{a"}, NR^{c"}S(O)R^{b"}, NR^{c"}S(O)₂R^{b"}, S(O)R^{b"}, S(O)NR^{c"}R^{d"}, S(O)₂R^{b"}, and S(O)₂NR^{c"}R^{d"};
R¹, R², R³, and R⁴ are independently selected from H, halo, C₁₋₄ alkyl, C₂₋₄ alkenyl, C₂₋₄ alkynyl, C₁₋₄ haloalkyl, halosulfanyl, aryl, cycloalkyl, heteroaryl, heterocycloalkyl, CN, NO₂, OR⁷, SR⁷, C(O)R⁸, C(O)NR⁹R¹⁰, C(O)OR⁷ OC(O)R⁸, OC(O)NR⁹R¹⁰, NR⁹R¹⁰, NR⁹C(O)R⁸, NR^{C}C(O)OR⁷, S(O)R⁸, S(O)NR⁹R¹⁰, S(O)₂R⁸, NR⁹S(O)₂R⁸, and S(O)₂NR⁹R¹⁰;
R⁵ is H, halo, C₁₋₄ alkyl, C₂₋₄ alkenyl, C₂₋₄ alkynyl, C₁₋₄ haloalkyl, halosulfanyl, CN, NO₂, OR⁷, SR⁷, C(O)R⁸, C(O)NR⁹R¹⁰, C(O)0R⁷, OC(O)R⁸, OC(O)NR⁹R¹⁰, NR⁹R¹⁰, NR⁹C(O)R⁸, NR⁹C(O)OR⁷, S(O)R⁸, S(O)NR⁹R¹⁰, S(O)₂R⁸, NR⁹S(O)₂R⁸, or S(O)₂NR⁹R¹⁰;
R⁶ is H, C₁₋₄ alkyl, C₂₋₄ alkenyl, C₂₋₄ alkynyl, C₁₋₄ haloalkyl, OR⁷, C(O)R⁸, C(O)NR⁹R¹⁰, C(O)0R⁷, S(O)R⁸, S(O)NR⁹R¹⁰, S(O)₂R⁸, or S(O)₂NR⁹R¹⁰;
R⁷ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁸ is H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl;
R⁹ and R¹⁰ are independently selected from H, C₁₋₁₀ alkyl, C₁₋₆ haloalkyl, C₂₋₆ alkenyl, C₂₋₆ alkynyl, C₁₋₆alkylcarbonyl, arylcarbonyl, C₁₋₆alkylsulfonyl, arylsulfonyl, aryl, heteroaryl, cycloalkyl, heterocycloalkyl, arylalkyl, heteroarylalkyl, cycloalkylalkyl and heterocycloalkylalkyl;
or R⁹ and R¹⁰ together with the N atom to which they are attached form a 4-, 5-, 6- or 7-membered heterocycloalkyl group;
R¹¹ and R¹² are independently selected from H and -E¹-E²-E³-E⁴;
D¹ and E¹ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆haloalkyl, C₂₋₈ alkoxyalkyl, C₁₋₆alkoxy, C₁₋₆haloalkoxy, amino, C₁₋₆ alkylamino, and C₂₋₈ dialkylamino;
D² and E² are independently absent or independently selected from C₁₋₆alkylene, C₂₋₆ alkenylene, C₂₋₆ alkynylene, (C₁₋₆alkylene)r-O-(C₁₋₆alkylene)ₛ, (C₁₋₆alkylene)ᵣ-S-(C₁₋₆alkylene)ₛ, (C₁₋₆ alkylene)ᵣ-NR^{e}-(C₁₋₆alkylene)ₛ, (C₁₋₆alkylene)ᵣ-CO-(C₁₋₆alkylene)ₛ, (C₁₋₆alkylene)ᵣ-COO-(C₁₋₆ alkylene)ₛ, (C₁₋₆alkylene)ᵣ-CONR^{e}-(C₁₋₆alkylene)ₛ, (C₁₋₆alkylene)ᵣ-SO-(C₁₋₆alkylene)ₛ, (C₁₋₆ alkylene)ᵣ-SO₂-(C₁₋₆alkylene)ₛ, (C₁₋₆alkylene)ᵣ-SONR^{e}-(C₁₋₆alkylene)ₛ, and (C₁₋₆alkylene)ᵣ-NR^{e}CONR((C₁₋₆alkylene)ₛ, wherein each of the C₁₋₆ alkylene, C₂₋₆ alkenylene, and C₂₋₆ alkynylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆alkoxy, C₁₋₆haloalkoxy, amino, C₁₋₆ alkylamino, and C₂₋₈ dialkylamino;
D³ and E³ are independently absent or independently selected from C₁₋₆alkylene, C₂₋₆ alkenylene, C₂₋₆ alkynylene, arylene, cycloalkylene, heteroarylene, and heterocycloalkylene, wherein each of the C₁₋₆alkylene, C₂₋₆ alkenylene, C₂₋₆ alkynylene, arylene, cycloalkylene, heteroarylene, and heterocycloalkylene is optionally substituted by 1, 2 or 3 substituents independently selected from halo, CN, NO₂, N₃, SCN, OH, C₁₋₆ alkyl, C₁₋₆ haloalkyl, C₂₋₈ alkoxyalkyl, C₁₋₆alkoxy, C₁₋₆haloalkoxy, amino, C₁₋₆ alkylamino, and C₂₋₈ dialkylamino;
D⁴ and E⁴ are independently selected from H, halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl)R^{b}, and S(O)₂NR^{c}R^{d}, wherein said C₁₋₈ alkyl, C₂₋₈ alkenyl, or C₂₋₈ alkynyl, is optionally substituted with 1, 2, 3, 4, 5, or 6 substituents independently selected from halo, C₁₋₄ alkyl, C₂₋₄ alkenyl, C₂₋₄ alkynyl, C₁₋₄ haloalkyl, halosulfanyl, C₁₋₄ hydroxyalkyl, C₁₋₄ cyanoalkyl, Cy¹, CN, NO₂, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)NR^{c}R^{d}, NR^{c}C(O)OR^{a}, C(=NRⁱ)NR^{c}R^{d}, NR^{c}C(=NRⁱ)NR^{c}R^{d}, S(O)R^{b}, S(O)NR^{c}R^{d}, S(O)₂R^{b}, NR^{c}S(O)₂R^{b}, C(=NOH)R^{b}, C(=NO(C₁₋₆ alkyl))R^{b}, and S(O)₂NR^{c}R^{d};
R^{a} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, halosulfanyl, aryl, arylalkyl, heteroaryl, heteroarylalkyl, cycloalkyl and heterocycloalkyl;
R^{b} is H, Cy¹, -(C₁₋₆alkyl)-Cy¹, C₁₋₆ alkyl, C₁₋₆ haloalkyl, C₂₋₆ alkenyl, C₂₋₆ alkynyl, wherein said C₁₋₆ alkyl, C₁₋₆ haloalkyl, C₂₋₆ alkenyl, or C₂₋₆ alkynyl is optionally substituted with 1, 2, or 3 substituents independently selected from OH, CN, amino, halo, C₁₋₆alkyl, C₁₋₆haloalkyl, C₁₋₆ haloalkyl, halosulfanyl, aryl, arylalkyl, heteroaryl, heteroarylalkyl, cycloalkyl and heterocycloalkyl;
R^{a'} and R^{a"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, halosulfanyl, aryl, arylalkyl, heteroaryl, heteroarylalkyl, cycloalkyl and heterocycloalkyl;
R^{b'} and R^{b"} are independently selected from H, C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl and heterocycloalkylalkyl, wherein said C₁₋₆ alkyl, C₁₋₆ haloalkyl, C₂₋₆ alkenyl, C₂₋₆ alkynyl, aryl, cycloalkyl, heteroaryl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
R^{c} and R^{d} are independently selected from H, Cy¹, -(C₁₋₆alkyl)-Cy¹, C₁₋₁₀ alkyl, C₁-₆ haloalkyl, C₂₋₆ alkenyl, C₂₋₆ alkynyl, wherein said C₁₋₁₀ alkyl, C₁₋₆ haloalkyl, C₂₋₆ alkenyl, or C₂₋₆ alkynyl, is optionally substituted with 1, 2, or 3 substituents independently selected from Cy¹, -(C₁₋₆ alkyl)-Cy¹, OH, CN, amino, halo, C₁₋₆alkyl, C₁₋₆haloalkyl, C₁₋₆haloalkyl, and halosulfanyl;
or R^{c} and R^{d} together with the N atom to which they are attached form a 4-, 5-, 6- or 7-membered heterocycloalkyl group optionally substituted with 1, 2, or 3 substituents independently selected from Cy¹, -(C₁₋₆alkyl)-Cy¹, OH, CN, amino, halo, C₁₋₆alkyl, C₁₋₆haloalkyl, C₁₋₆haloalkyl, and halosulfanyl;
R^{c'} and R^{d'}are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆ alkyl, C₁₋₆ haloalkyl, C₁₋₆ haloalkyl, halosulfanyl, aryl, arylalkyl, heteroaryl, heteroarylalkyl, cycloalkyl and heterocycloalkyl;
or R^{c'} and R^{d'} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁-₆haloalkyl, C₁₋₆haloalkyl, halosulfanyl, aryl, arylalkyl, heteroaryl, heteroarylalkyl, cycloalkyl and heterocycloalkyl;
R^{c"} and R^{d"} are independently selected from H, C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl and heterocycloalkylalkyl, wherein said C₁₋₁₀ alkyl, C₁₋₆ haloalkyl, C₂₋₆ alkenyl, C₂₋₆ alkynyl, aryl, heteroaryl, cycloalkyl, heterocycloalkyl, arylalkyl, heteroarylalkyl, cycloalkylalkyl or heterocycloalkylalkyl is optionally substituted with 1, 2, or 3 substituents independently selected from OH, CN, amino, halo, C₁₋₆alkyl, C₁-₆haloalkyl, halosulfanyl, C₁-₆haloalkyl, aryl, arylalkyl, heteroaryl, heteroarylalkyl, cycloalkyl and heterocycloalkyl;
or R^{c"} and R^{d"} together with the N atom to which they are attached form a 4-, 5-, 6- or 7-membered heterocycloalkyl group optionally substituted with 1, 2, or 3 substituents independently selected from OH, CN, amino, halo, C₁₋₆alkyl, C₁-₆haloalkyl, C₁-₆haloalkyl, halosulfanyl, aryl, arylalkyl, heteroaryl, heteroarylalkyl, cycloalkyl and heterocycloalkyl;
Rⁱ is H, CN, NO₂, or C₁₋₆ alkyl;
R^{e} and R^{f} are independently selected from H and C₁₋₆ alkyl;
Rⁱ is H, CN, or NO₂;
m is 0 or 1;
n is 0 or 1;
p is 0, 1, 2, 3, 4, 5, or 6;
q is 0, 1, 2, 3, 4, 5 or 6;
r is 0 or 1; and
s is 0 or 1;
wherein when X is N, n is 1, and the moiety formed by A¹, A², U, T, V, and -(Y)ₙ-Z has the formula: 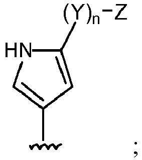 then Y is other than (CR¹¹R¹²)ₚC(O)NR^{c}(CR¹¹R¹²)_{q;}
(b) compounds of Formula II: 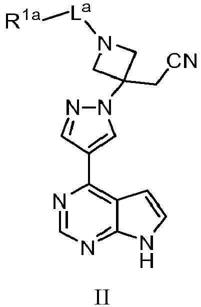 wherein:
L^{a} is SO₂ or CO;
R^{1a} is C₁₋₆alkyl, C₃₋₇ cycloalkyl, phenyl, 5- or 6-membered heteroaryl, indolyl, NR^{2a}R^{3a}, or OR^{4a}, wherein said alkyl, cycloalkyl, phenyl, or heteroaryl is optionally substituted with 1, 2, or 3 substituents independently selected from F, CN, and C₁₋₄ alkyl;
R^{2a} and R^{3a} are independently selected from H, C₁₋₄ alkyl, and phenyl; and
R^{4a} is C₁₋₆ alkyl, phenyl, or benzyl;
(c) compounds of Formula III: 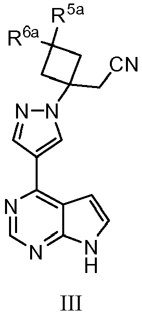 wherein:
R^{5a} and R^{6a} are independently selected from H, F, CN, OH, C₁₋₄ alkyl, benzyloxy, C₂₋₈ dialkylaminosulfonyl, and 5-membered heteroaryl, wherein said alkyl is optionally substituted by 1, 2, or 3 substituents selected from F, OH, CN, and C₁₋₄ alkoxy, and wherein said 5-membered heteroaryl is optionally substituted with C₁₋₄ alkyl;
and pharmaceutically acceptable salts thereof.

15. The agent for use according to claim 14, wherein said agent is administered (a) preoperatively to a patient about to undergo a procedure selected from corneal transplant, LASIK, photorefractive keratectomy, and LASEK; or (b) postoperatively to a patient who has undergone a procedure selected from corneal transplant, LASIK, photorefractive keratectomy, and LASEK.
